# EUROPEAN PATENT SPECIFICATION

(11) **EP 4 392 550 B1**
(45) Date of publication and mention of the grant of the patent: **20.08.2025**
(21) Application number: 23738642.0
(22) Date of filing: 29.06.2023
(51) Int. Cl.: C12N 7/00, A61P 31/04, C12N 15/10

(54) **TARGETING E COLI CELLS**
TARGETING VON E-COLI-ZELLEN
CIBLAGE DE CELLULES D'E. COLI

(30) Priority: 29.06.2022 GB 202209518; 06.03.2023 GB 202303242
(43) Date of publication of application: 03.07.2024
(73) Proprietor: SNIPR Biome ApS., 2100 Copenhagen (DK)
(72) Inventor: KRAUSE HAABER, Jakob, 2100 Copenhagen (DK); SEMSEY, Szabolcs, 2100 Copenhagen (DK); GROVE, Mette, 2100 Copenhagen (DK); DAMHOLT, Birgitte, 2100 Copenhagen (DK); JASINSKYTE, Dziuginta, 2100 Copenhagen (DK); GENÇAY, Yilmaz Emre, 2100 Copenhagen (DK)
(74) Representative: CMS Cameron McKenna Nabarro Olswang LLP
(86) International application number: PCT/EP2023/067906
(87) International publication number: WO 2024/003301

(56) References cited:
- WO-A1-2019/243373
- WO-A1-2021/092254
- KARIMI MAHDI ET AL: "Bacteriophages and phage-inspired nanocarriers for targeted delivery of therapeutic cargos", ADVANCED DRUG DELIVERY REVIEWS, ELSEVIER, AMSTERDAM , NL, vol. 106, 17 March 2016 (2016-03-17), pages 45 - 62, XP029786403, ISSN: 0169-409X, DOI: 10.1016/J.ADDR.2016.03.003
- GENCAY Y ET AL: "Engineered phage with antibacterial CRISPR-Cas selectively reduce E. coli burden in mice", NATURE BIOTECHNOLOGY, 4 May 2023 (2023-05-04), XP093087732, Retrieved from the Internet <URL:https://www.nature.com/articles/s41587-023-01759-y> [retrieved on 20231002], DOI: 10.1038/s41587-023-01759-y
- NATH A ET AL: "Phage delivered CRISPR-Cas system to combat multidrug-resistant pathogens in gut microbiome", BIOMEDICINE & PHARMACOTHERAPY, ELSEVIER, FR, vol. 151, 17 May 2022 (2022-05-17), XP087085088, ISSN: 0753-3322, [retrieved on 20220517], DOI: 10.1016/J.BIOPHA.2022.113122
- GIBB B ET AL: "The Many Applications of Engineered Bacteriophages-An Overview", PHARMACEUTICALS, vol. 14, no. 7, 30 June 2021 (2021-06-30), pages 634, XP093089657, DOI: 10.3390/ph14070634

## Description

### TECHNICAL FIELD

The technology described herein relates to compositions for targeting *E coli* cells and uses of such compositions, such as for treating or preventing an infection by *E coli* cells in human or animal subjects. The uses comprise administering to the subject a composition comprising a particle cocktail comprising a plurality of different transduction particles to *E coli* cells. The uses comprise administering to the subject a composition comprising a plurality of transduction particles that encode a nuclease for targeting the genomes of B2 phylogroup *E coli* cells.

### BACKGROUND

*E coli* infection has been identified as harmful or life-threatening in various settings, such as UTI infections, transplant patients, cancer patients and other patients that are immunocompromised or on immunosuppressant.

Nuclease targeting of *E coli,* such as by means of CRISPR/Cas systems, has been proposed with delivery using transduction particles that can target the nuclease to *E coli* cells for chromosomal or episomal cutting, thereby killing cells or reducing their growth or proliferation. Suitable transduction particles are phage or engineered particles (such as non-self-replicative transduction particles) comprising capsids that contain nucleic acid encoding at least crRNAs or gRNAs (or additionally a Cas nuclease) for targeting. Advantageously, selective targeting can be achieved which is not possible using conventional antibiotics, such as broad-spectrum antibiotics. Such selective targeting can avoid killing of beneficial species and strains in treated patients. Indeed, disturbances of the microbiome with broad-spectrum antibiotics is a risk-factor in the prophylactic management of cancer patients at risk of febrile neutropenia.

Bacteriophage (phage) therapy has been used prior to the broad availability of antibiotics, but has now re-gained interest due to the rise in bacterial antimicrobial resistance (AMR) combined with several successful individual case reports.

Bacteriophages (phages) are a phylum of viruses that infect bacteria, and are distinct from the animal and plant viruses. Phages can have either a "lytic" life cycle, a "lysogenic" life cycle that can potentially become lytic, or a "non-lytic" life cycle. Phages replicating through the lytic cycle cause lysis of the host bacterial cell as a normal part of their life cycles. Phages replicating through the lysogenic cycles are called temperate phages, and can either replicate by means of the lytic life cycle and cause lysis of the host bacterium, or they can incorporate their DNA into the host bacterial DNA and become noninfectious prophages.

The natural capability of phages to infect and kill bacteria, together with the specificity of the phage-bacterial interactions, is the basic phenomena on which the concept of phage therapy is built. Therefore, phages that possess lytic life cycle are suitable candidates for phage therapy.

International Patent Application No. WO 00/69269 discloses the use of certain phage strain for treating infections caused by Vancomycin-sensitive as well as resistant strains of *Enterococcus faecium,* and International Patent Application No. WO 01/93904 discloses the use of bacteriophage, alone or in combination with other anti-microbial means, for preventing or treating gastrointestinal diseases associated with the species of the genus *Clostridium.*

US Patent Application No. 2002/0001590 discloses the use of phage therapy against multi-drug resistant bacteria, specifically methicillin-resistant *Staphylococcus aureus,* and International Patent Application No. WO 02/07742 discloses the development of bacteriophage having multiple host range.

The use of phage therapy for the treatment of specific bacterial-infectious disease is disclosed, for example, in US Patent Application Nos. 2002/0044922; 2002/0058027 and International Patent Application No. WO 01/93904.

US20160333348 describes the use of CRISPR/Cas systems delivered to host bacterial cells using phage as vectors.

Amongst the several phylogroups of *E coli,* it has been observed that antibiotic-resistant (eg, fluoroquinolone (FQ)-resistant) and multi-drug resistant (MDR) strains are frequently found in the B2 phylogroup. B2 strains ST131 and ST1193 have been found that are associated with antibiotic resistance. ST131 is a globally dominant multidrug resistant clone associated with high rates in rUTI. ST131 is a major contributor to hospital- and community-acquired UTI, as well as *E coli* bloodstream infections and infections in companion animals and poultry. Originally identified in 2008, ST131 is associated with the worldwide spread of the CTX-M-15 extended spectrum β-lactamase (ESBL) resistance gene. ST131 is now strongly associated with multidrug resistance (MDR), including resistance to fluoroquinolones. Recent reports have also identified strains that are resistant to last-line carbapenems. Sequence type 1193 has recently emerged as a new, virulent and resistant lineage among fluoroquinolone resistant *E coli.*

Classic antibiotics, such as FQ and broad-spectrum antibiotics are not, thus, sufficiently effective for combatting such infections. There is, therefore, a need to find alternative means to address these infections.

WO 2021/092254 A1 (LOCUS BIOSCIENCES INC [US]) relates to phage compositions comprising crispr-cas systems and methods of use thereof.

WO 2019/243373 A1 (ELIGO BIOSCIENCE [FR]) relates to bacterial delivery vehicle, process of production and uses thereof.

Gencay Y et al (Nature Biotech., 4 May 2023) relates to engineered phage with antibacterial CRISPR-Cas selectively reduce *E*. *Coli* burden in mice.

Nath A et al: (Biomedical Pharmacotherapy., vol. 151, 2022) relates to phage delivered CRISPR-Cas system to combat multidrug-resistant pathogens in gut microbiome.

Gibb B et al (Pharmaceuticals, vol. 14, 2021, page 634) relates to an overview of the many applications of engineered bacteriophages.

### SUMMARY

The invention provides compositions for treating or preventing B2 phylogroup *E coli* infections in humans and animals by combining the use of selective killing with nucleases targeted using specific types of transduction particles. The particles of the composition of the invention target by adhesion to LPS, LamB and Tsx, which has surprisingly been found highly advantageous for killing and inhibiting growth of B2 *E coli* cells of many different strains (including the potentially lethal ST131 and ST1193 strains). As exemplified herein, surprisingly more than 10 different ST131 strains were killed (plaques formed) and more than 10 different ST1193 strains were killed (plaques formed).

The invention finds utility, for example, to treat or prevent potentially life-threating B2 phylogroup *E coli* infections in patients, such as in immunosuppressed, cancer, transplant and UTI patients, who are susceptible to infection by B2 strains (and often by multiple different B2 strains). As demonstrated in the Examples, the invention is useful for preventing *E coli* B2 phylogroup bacteraemia (infection of the blood stream by *E coli)* in a subject.

Any references to methods for treatment of the human or animal body by surgery or therapy are to be interpreted as references to the compositions of the present invention for use in a method for such treatment.

### STATEMENTS OF INVENTION:

The invention is as claimed in claims 1 to 15.

Any part of the description not within the scope of claims 1-15 and these statements does not form a part of the invention but serves in the understanding of the claimed invention.

### BRIEF DESCRIPTION OF THE DRAWINGS

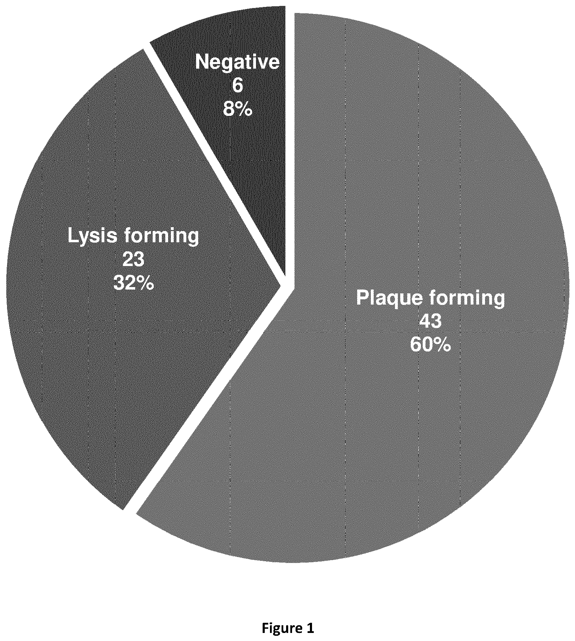

**Figure 1****:** Killing and growth inhibition activity of a composition of the invention when tested against *E coli* of several different phylogroups and different strains within each group *(E coli* taken from clinical samples); the composition was surprisingly highly effective at killing across many phylogroups (and particularly in the B2 phylogroup, where multiple strains - including those known to be potentially life-threatening and related with antibiotic resistnance - were killed or whose growth was effectively inhibited). The 6 negatives comprised non *E coli* and non FQ resistant samples.

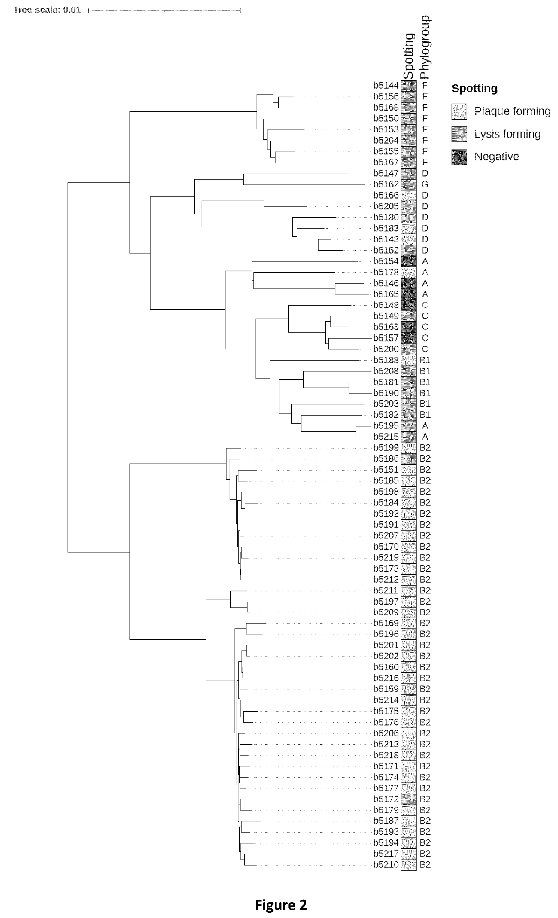

**Figure 2****:** Phylo-grouping of strains used in the study and showing particularly advantageous and extensive targeting of many clinical B2 phylogroup strains.

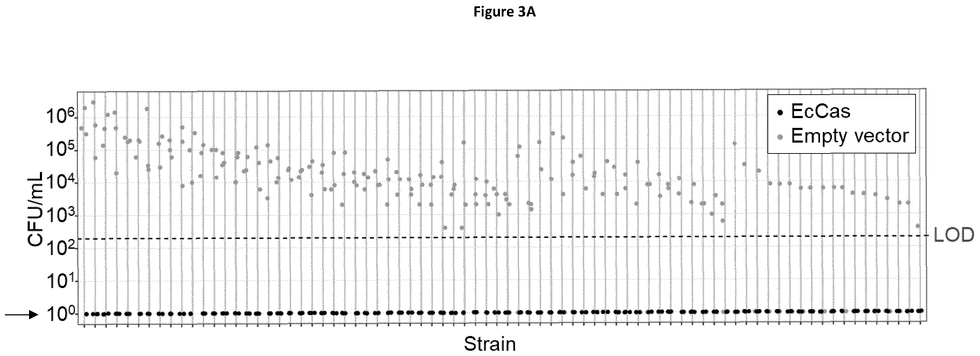

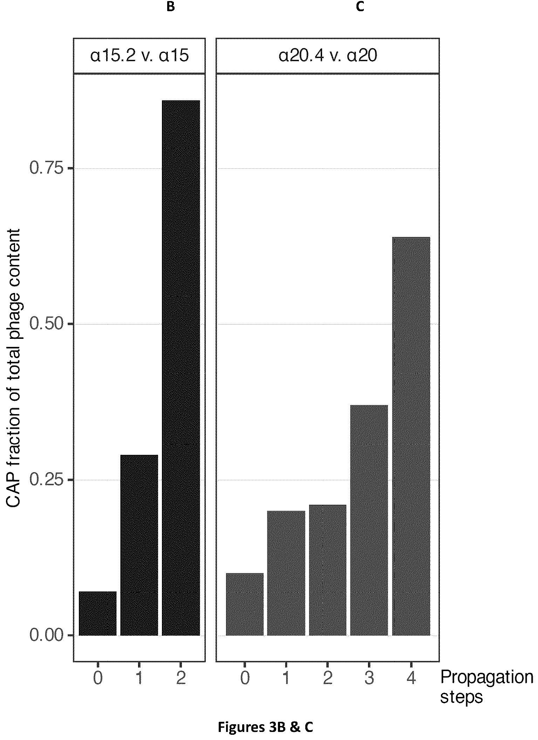

**Figure 3****:** A) CRISPR-Cas driven elimination of an abbreviated panel of 82 E. coli clinical isolates by conjugation of CGV-EcCAS and empty vector. The conjugation efficiency was determined by spotting a dilution series of the conjugation reaction on LB agar supplemented with antibiotics and calculated as CFU/ml. The limit of detection (LOD) of this assay was 200 CFU/mL. The experiment was carried out in triplicates and is indicated by dots. CGV-EcCAS conjugation experiments indicated by arrow; empty vector conjugations indicated above the dotted line labelled LOD. B) Fraction of CRISPR-armed phage^{™} (CAP) and WT phage during co-culture with a host strain susceptible to both phages. CAP α15.2 increases its relative abundance compared to the WT phage from 7% to 86% over two consecutive passages. C) CAP α20.4 outcompeted WT α20 by increasing its relative abundance during co-culture with the common target E. coli strain b230 from 10% to 68% over four consecutive passages. B-C) The ratio of CAP and WT phage during co-culture with a host strain (E. coli b230) susceptible to α15.2, α15, α20.4 and α20. CAP α15.2 increase its relative abundance compared to the WT phage from 7% to 86% (G) while CAP α 20.4 outcompeted WT α20 from 10% to 68% (H).

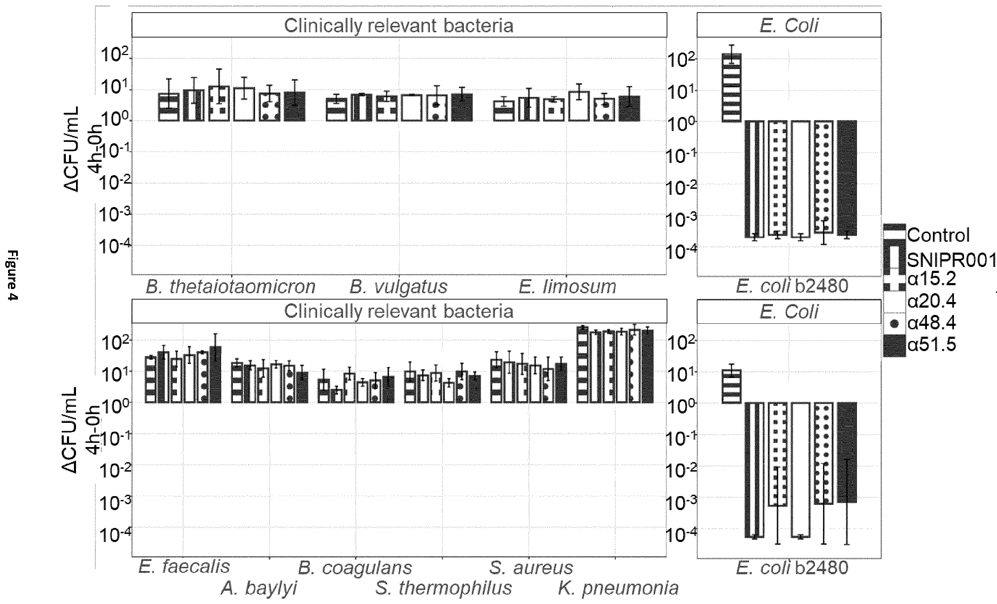

**Figure 4****:** Specificity evaluation of SNIPR001 and individual CAPs against a panel of clinically relevant bacteria and E. coli strain b2480 (positive control) showing no off-target effects. Positive values represent bacterial growth (absence of observed killing), while negative values indicate bacterial killing following phage treatment within the assessed time-period. All values indicated as means of four biological replicates with standard deviations, measuring growth over a 4-hour period, measured in CFU/mL.

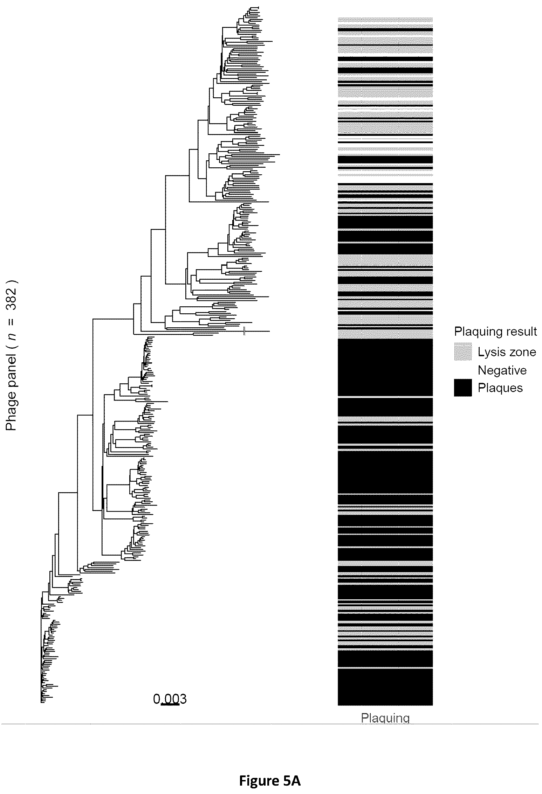

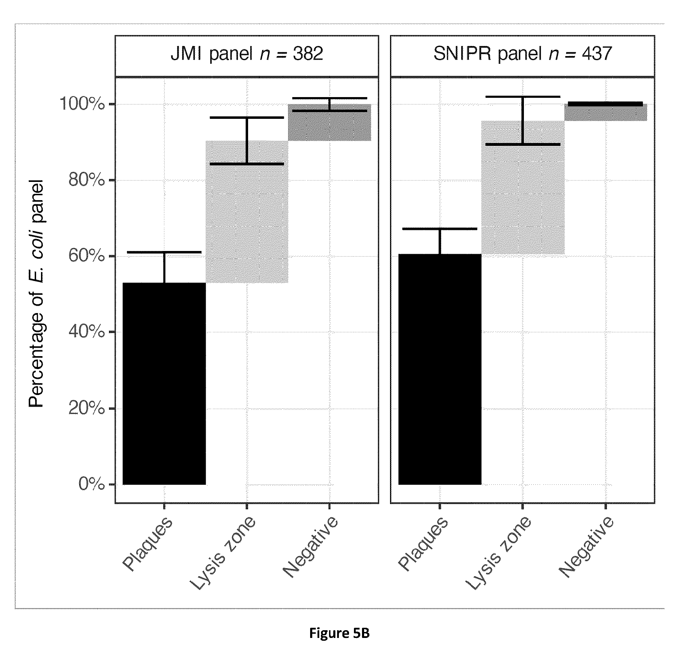

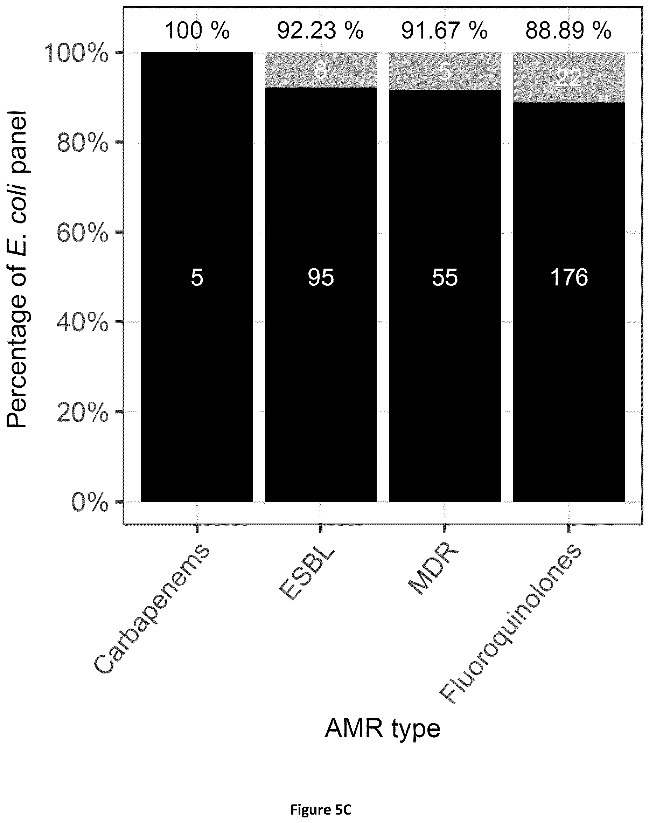

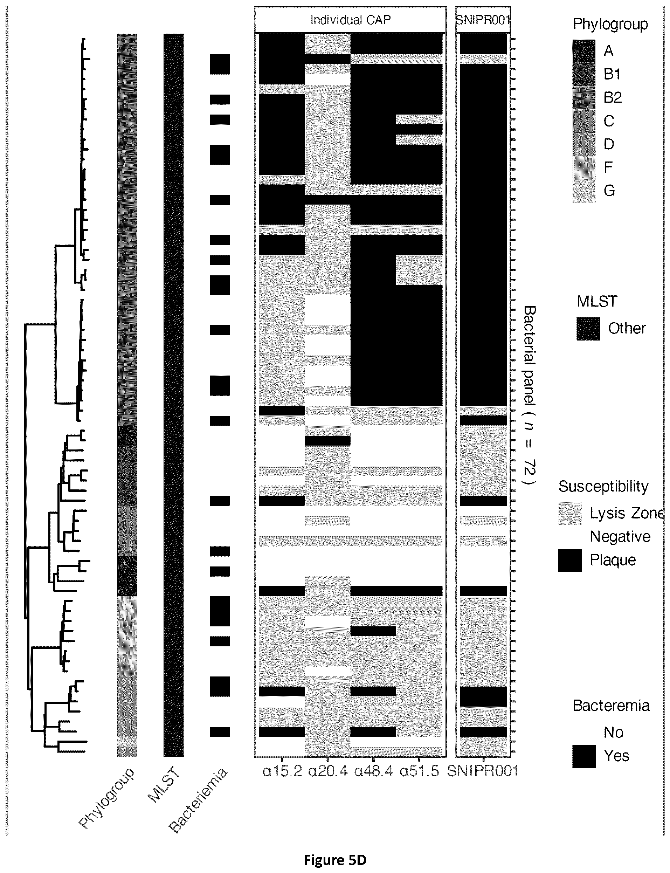

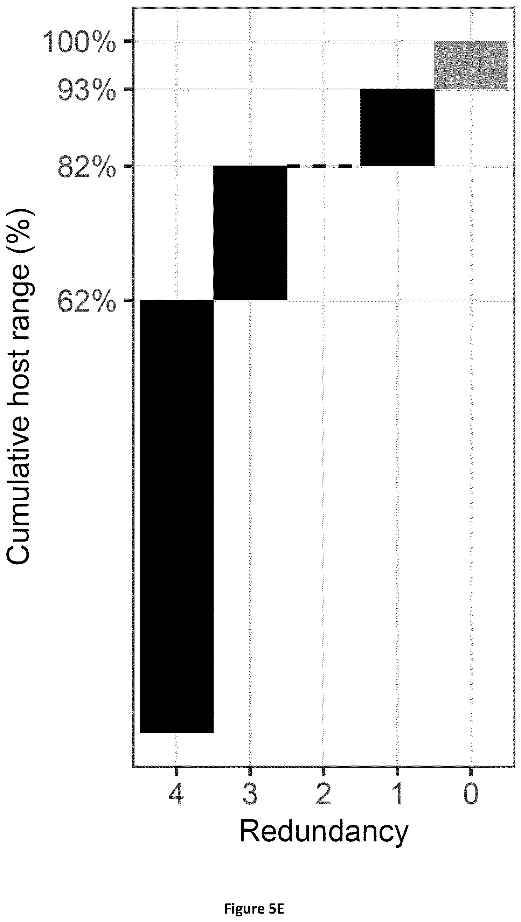

**Figure 5****:** A) An unrooted phylogenetic tree of the JMI strains displaying a clinical panel of 382 E. coli strains encompassing nine phylogroups and 118 MLSTs. Plaquing data reflects a single plaquing replicate. One strain, E. coli b4038, with a long branch has been truncated to 37% of the original length. Phylogenetic distance scale indicated below the phylogenetic tree, as computed by MASH. B) A spot assay was used to analyze the efficacy of SNIPR001 against the clinical panel of 382 E. coli strains (from JMI, North Liberty, IA, USA) isolated from bloodstream infections and the internal 429 E. coli strain panel. The spot assay was conducted as two independent experiments, with results presented as mean ± SD values. The standard deviation shown is based on the discrepancy of results between the two runs, calculated as the average absolute deviation of the mean prevalence of a given spotting type, normalized to the panel size. C) Coverage of SNIPR001 does not depend on antibiotic resistant phenotypes; consequently, SNIPR001 targets >90% of E. coli strains that are carbapenem-resistant, extended-spectrum β-lactamase (ESBL)-producing or multi-drug resistant (MDR), and 89% of fluroquinolone resistant E. coli strains. Numbers indicated in each green or grey bar indicates number of bacteria susceptible or resistant to SNIPR001, respectively, for each resistance category. D) A midpoint rooted phylogenetic tree of the 72 fluroquinolone resistant E. coli strains isolated from fecal samples of hematological cancer patients. 67 of the 72 strains are susceptible to at least one of the four CAPS in SNIPR001. E) Redundancy distribution showing 82% of the fluroquinolone resistant E. coli strains (n = 72) from panel D are targeted by at least 2 different CAPs.

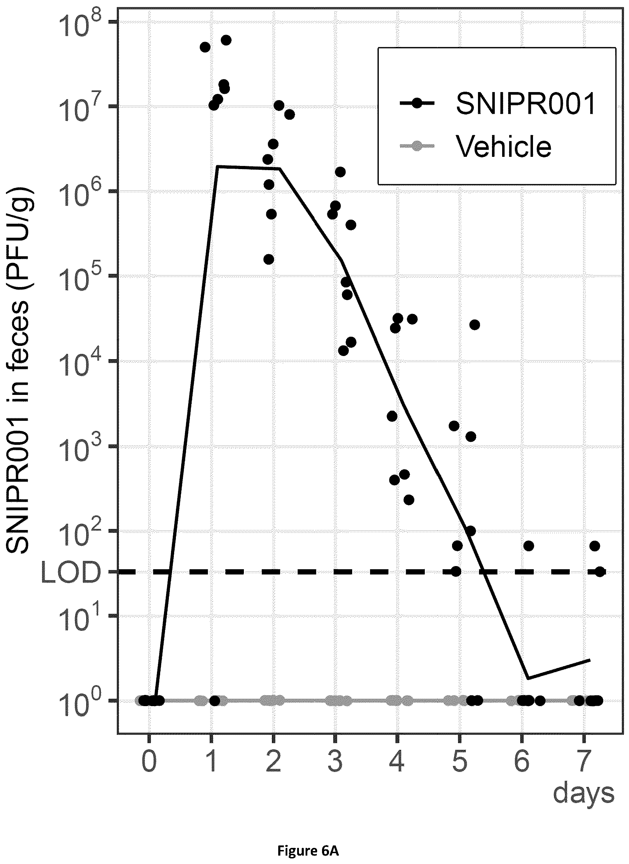

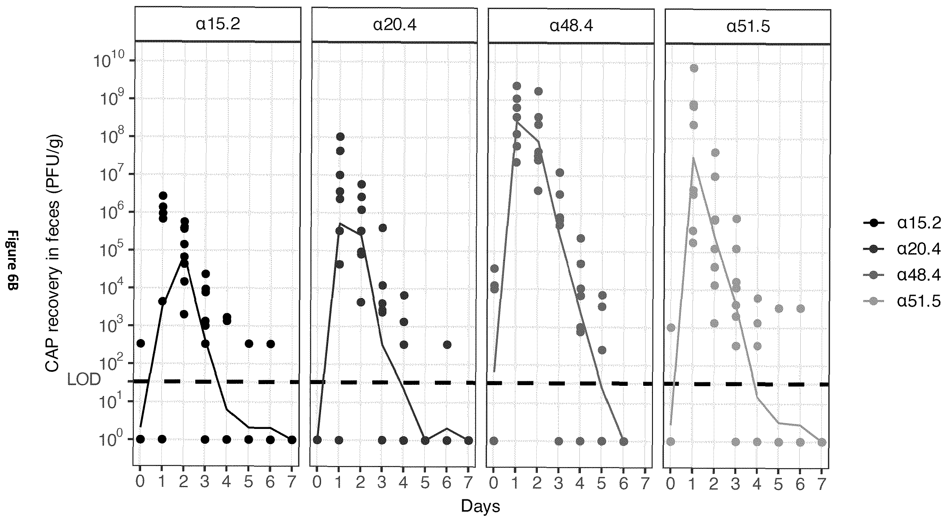

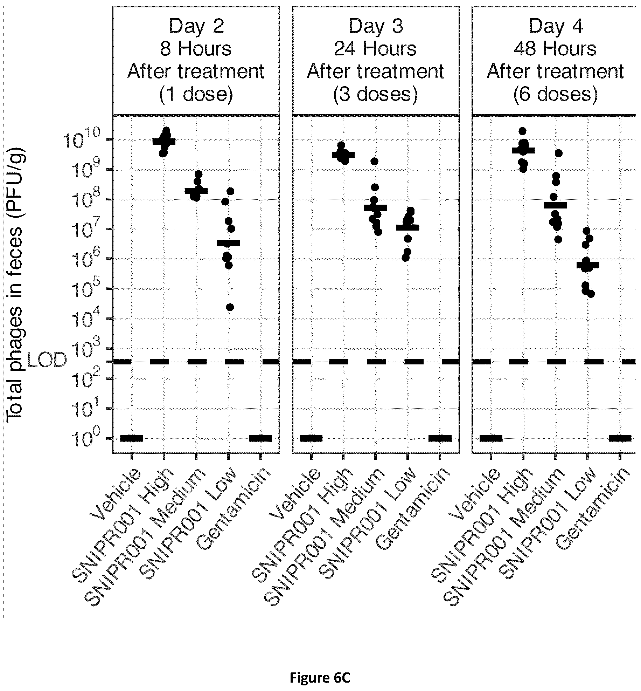

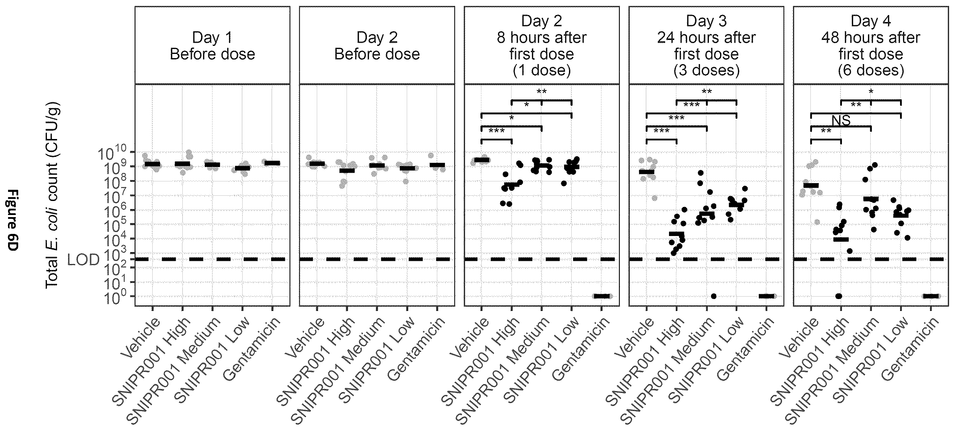

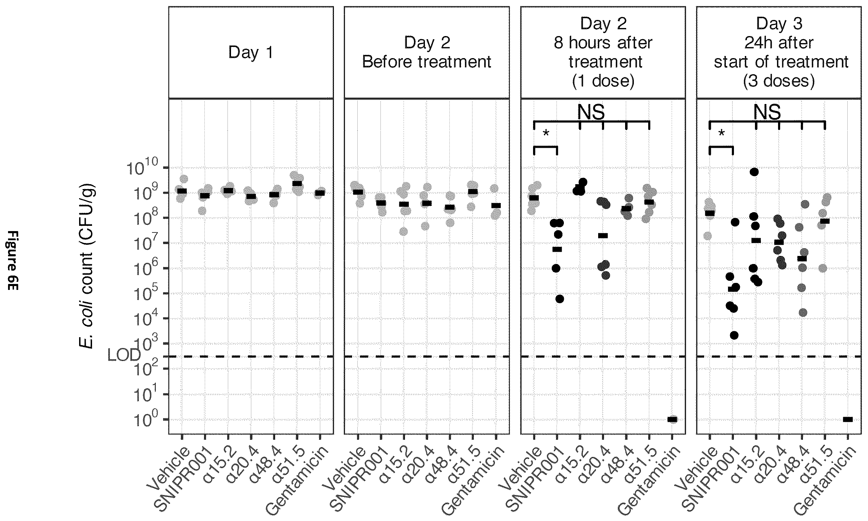

**Figure 6****:** A) CAP recovery in minipigs feces after a single p.o. dose of 2 x 10¹² PFU of SNIPR001 (n = 8, shown in green) or vehicle (n = 6, shown in grey) over one week with daily sampling. Trend lines indicate average recovered phage in PFU/g feces, dots indicate individual measurement points. LOD of 33 PFU/g feces indicated by the dotted line. B) CAP recovery in minipig feces after a single p.o. dose of 2 x 10¹² PFU of a single CAP (n = 8 minipigs received either α15.2, α20.4 or α51.5, n = 7 minipigs received α48.4) over one week with daily sampling. Trend lines indicate average recovery, while points indicate individual measurements. Recovery was measured in PFU/g feces. LOD of 33 PFU/g feces is indicated by the dotted line. C) CAP recovery in mouse feces 8h, 24h, and 48h after the start of treatment with three times daily administration of varying doses of SNIPR001 (n = 10 for low, medium, and high, indicated in green), vehicle (n = 10, indicated in grey), or gentamicin (n = 4, indicated in grey). Recovery is measured in PFU/g feces, LOD of 371 PFU/g feces is indicated by the dotted line. D) E. coli b17 recovery in mouse feces indicates increased SNIPR001 effect with increased dose. Statistical analyses were performed using two-sided Kruskal-Wallis tests for comparison of the SNIPR001-treated groups, two-sided Mann-Whitney U test for comparison of treated groups with vehicle. P<0.05=*,0.01=**,0.001=***, FDR corrected using Holm's method separately for each day. Recovery is measured in CFU/g feces, with a limit of detection of 371 CFU/g feces. Animals that have begun SNIPR001 treatment are indicated in green, others in grey. E) E. coli b17 recovery in mouse feces 8h and 24h after the start of treatment with three times daily administration of CAPs α15.2, α20.4, α48.4 or α51.5 and in combination as SNIPR001 confirming synergy of the CAPs.

### DETAILED DESCRIPTION

The invention finds application to combat harmful or life-threatening B2 *E coli* infections in various settings, such as UTI infections, transplant patients, cancer patients and other patients that are immunocompromised or on immunosuppressant.

Any reference herein to particles of the invention are to be understood as particles comprised by a composition of invention, in accordance with the claims.

Cancer treatment continues to advance and survival rates for people with hematological malignancies are increasing. However, this population is immunocompromised and chemotherapy regimens cause bone-marrow suppression and gastrointestinal mucositis with associated increased intestinal permeability. Translocation of gut bacteria, including *E coli,* from the gastrointestinal tract is a frequent cause of bloodstream infections (BSIs). The mortality-related to BSIs can be up to 50%; thus, antimicrobial prophylaxis is applied in people at risk of f1 1ebrile neutropenia. There are no approved therapies for the prevention of BSIs in patients with hematological cancers, yet fluoroquinolones are used off-label in the United States. This antibiotic prophylaxis practice is at odds with the emerging paradigm that maintaining a normal microbiome is important for upholding immunological tonus potentially benefiting the outcome of oncology treatments. Indeed, disturbances of the microbiome with broad-spectrum antibiotics is a risk-factor in the prophylactic management of patients at risk of febrile neutropenia. Beyond the side effects of fluoroquinolones, including safety warnings and precautions, bacterial resistance is rising and approaching 60% in USA.

In immunocompromised patients with hematological malignancies at risk of developing neutropenia, *E coli* is responsible for 25.1-30% of all bacteraemia cases with a 35.8% 90-day mortality rate. Moreover, up to 65% of *E coli* isolated as the causative pathogen from BSIs in patients with hematological cancers undergoing hematopoietic stem cell transplantation (HSCT) were resistant to fluoroquinolones. Accordingly, novel narrow-spectrum treatment and prophylactic options are needed to prevent infections in these vulnerable patients. The invention addresses this need.

To this end, the disclosure provides compositions, methods and uses
The invention provides a composition comprising at least 4 different types of transduction particles, wherein each of said particles comprises a nucleic acid and said particles are capable of contacting *E coli* cells and introducing therein the nucleic acid, wherein
(a) said nucleic acid of each particle comprises a nucleotide sequence encoding a product of interest (POI), wherein the nucleic acid is capable of expressing the POI in an *E coli* cell; and wherein the POI comprises:
   (i) a Cas nuclease for targeting and cutting the DNA of the *E coli* cell, thereby modifying or killing the cell; and
   (ii) at least one crRNA or guide RNA each comprising a spacer sequence that is complementary to a protospacer sequence comprised by a gene selected from B2 phylogroup *E coli* genes fimH, bolA, rpoH, lptA and murA and each of which is operable with the Cas nuclease for DNA targeting in the *E coli* cells;
(b) each particle comprises:
   (iii) an adhesion moiety for recognising and binding to a cognate moiety selected from a LPS and Tsx displayed on the surface of E coli cells; and
   (iv) a phage capsid containing the nucleic acid; and
(c) said at least 4 different types of transduction particles comprises
   A: particles which comprise a LPS adhesion moiety and a LamB adhesion moiety;
   B: particles which comprise a LPS adhesion moiety and a Tsx adhesion moiety; and
   C: particles which comprise a Tsx adhesion moiety but are devoid of LamB and LPS adhesion moieties;
and wherein the particles of the composition target all of *E*. *coli* genes fimH, bolA, rpoH, lptA and murA.

In a first example of the disclosure the claimed compositions comprising a plurality of transduction particles are for use in a method of treating or preventing an infection by *E coli* cells in a human or animal subject, wherein the method comprises administering the particles to the subject, wherein
(a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of phylogroup B2 *E coli* cells.

Optionally, the genomic DNA is chromosomal DNA of the cells. Additionally or alternatively, the genomic DNA is plasmid DNA of the cells.

Optionally, each particle comprises a nucleic acid encoding a nuclease for chromosomal targeting, wherein the said administered particles contact the cells and introduce therein the nucleic acid, wherein the nuclease is expressed in the cells and cuts the chromosomes of the cells, thereby killing the cells or reducing growth or proliferation of the cells in the subject.

The human may be a male or female. The human may be an adult or child. The human may be 18 years of age or older, eg, 40, 50, 60, 70 80 or older. The human may be younger than 18, eg, a teenager, eg, a baby, eg, up to 5 years of age, eg, up to 2 years of age. The animal may be be a livestock or companion animal eg, a dog or cat). The animal may be a bird (eg, a poultry bird, eg, a chicken, turkey or duck, preferably a chicken), cow, sheep, goat or pig (eg, a neonatal swine or a swine under 6 months of age).

The infection may be a bloodstream infection. The infection may be a nosocomial infection.

In an example, each adhesion moiety is a tail fibre protein. In an example, each particle comprises a phage tail fibre that comprises or is fused to a said adhesion moiety. In an example, each adhesion moiety is an antibody fragment, eg, an antibody single variable domain. In an example, each adhesion moiety is a nanobody. In an example, each adhesion moiety comprises an antibody binding site that is capable of binding to the cognate moiety. For example, each adhesion moiety comprises an antibody single variable domain (ie, a dAb), such as a nanobody. In an example, each particle comprises one or more phage tail fibres or spikes, each fibre or spike comprising a said adhesion moiety.

For example, the at least 4 different types of said transduction particle are administered to the subject and each type comprises one or a plurality of types of tail fibres comprising adhesion moieties, wherein the other particle types do not comprise said one or plurality of tail fibre types. In an example, each type of said plurality of tail fibre types differs from the other types by the type of adhesion moiety it comprises.

In Gram-negative bacteria, the peptidoglycan layer is relatively thin and is located inward of the outer membrane, the major component of the cell wall. These two layers are connected by Braun's lipoproteins. The outer membrane is a sophisticated structure composed of a lipid bilayer ornamented with proteins, polysaccharides and lipids; the latter two molecules form the LPS layer. LPSs are complexes that consist of three parts: lipid A, the core polysaccharide and the O-polysaccharide. Lipid A is, in general, composed of fatty acids attached to glucosamine phosphate disaccharides. The core polysaccharide is connected to the lipid A through a ketodeoxyoctonate linker. The core polysaccharide and the O-polysaccharide (O-chain or O-antigen) contain several units of sugar residues extending outward to the outer membrane. Cells that contain all three components of the LPS are denominated as smooth (S) type and those that lack the O-polysaccharide portion are distinguished as rough (R) type.

Optionally, the LPS is smooth LPS or rough LPS.

For example, the particles comprise at least one type of particle whose adhesion moiety is capable of binding to O-antigen of LPS.

*E coli* is a very versatile species for which diversity has been explored from various perspectives highlighting, for example, phylogenetic groupings, pathovars as well as a wide range of O serotypes. The highly variable O-antigen, the most external part of the lipopolysaccharide component of the outer membrane of *E coli,* is linked to the innermost lipid A through the core region of LPS of which 5 different structures, denominated K-12, R1, R2, R3 and R4, have been characterized so far. Phylogroups B2 and C strains are mainly dominated by the R1 type. Strains within phylogroup B2 may carry a K-12 core, eg, belonging to the complex STc131, one of the major clone of extra-intestinal pathogenic *E coli* (ExPEC) strains.

Preferably, the LPS comprises a R1 core region. In an example, the LPS comprises a R2 core region. In an example, the LPS comprises a R3 core region. In an example, the LPS comprises a R4 core region. In an example, the LPS comprises a K-12 core region.

Optionally, the LamB comprises the amino acid of SEQ ID NO: 1 or an amino acid sequence that is at least 70, 80, 90 or 95% identical to SEQ ID NO: 1. Optionally, the Tsx comprises the amino acid of SEQ ID NO: 2 or an amino acid sequence that is at least 70, 80, 90 or 95% identical to SEQ ID NO: 2. Optionally, the LamB is encoded by the nucleotide sequence of SEQ ID NO: 3 or an amino acid sequence that is at least 70, 80, 90 or 95% identical to SEQ ID NO: 3. Optionally, the Tsx is encoded by the nucleotide sequence of SEQ ID NO: 4 or an amino acid sequence that is at least 70, 80, 90 or 95% identical to SEQ ID NO: 4.

The Escherichia coli tsx gene encodes an integral outer-membrane protein (Tsx) that functions as a substrate-specific channel for deoxynucleosides and the antibiotic albicidin. In an example, the Nucleoside-specific channel-forming protein Tsx of *E coli* has a Uniprot Accession Number of P0A927 or is a homologue thereof. In an example, the maltose outer membrane porin (maltoporin) LamB of *E coli* has a Uniprot Accession Number of P02943 or is a homologue thereof. Homologue: A gene, nucleotide or protein sequence related to a second gene, nucleotide or protein sequence by descent from a common ancestral DNA or protein sequence. The term, homologue, may apply to the relationship between genes separated by the event of or to the relationship between genes separated by the event of genetic duplication.

In an example, the *E coli* cells comprise UPEC E coli. In an example, the *E coli* cells comprise intestinal pathogenic E coli (ExPEC) cells.

The method may be for treating or preventing infection of the subject by an *E coli* strain selected from the group ST131, ST1193, ST648, ST315, ST405, ST361, ST88 and ST453.

In a preferred example, the strain is ST1193. In another preferred example, the strain is ST131.

The method may be for treating or preventing infection of the subject by a plurality of different phylogroup B2 strains of *E coli;* optionally wherein the plurality comprises *E coli* ST131 and ST1193 cells.

E coli ST131 and/or ST1193 have been found to be virulent and associated with fluoroquinolone resistance. As shown in the Example section herein, the compositions of the invention are useful for treating or preventing infection by such strains. Optionally, said plurality of strains comprises E coli ST131 and/or ST1193 strains. Optionally, said plurality of strains comprises fluoroquinolone-resistant strains. For example, said plurality of strains comprises E coli ST131 and/or ST1193 fluoroquinolone-resistant strains.

*E coli* strains B2-ST73 (CH24-30); B2-ST73 (CH24-103); B2-ST131 (CH40-30); B2-ST141 (CH52-5); B2-ST372 (CH103-9); B2-ST404 (CH14-27); B2-ST404 (CH14-807) and B2-ST1193 (CH14-64) have been found in UTI settings. In an example (eg, wherein the subject is suffering from or at risk of UTI), the B2 E coli comprise one or more strains selected from B2-ST73 (CH24-30); B2-ST73 (CH24-103); B2-ST131 (CH40-30); B2-ST141 (CH52-5); B2-ST372 (CH103-9); B2-ST404 (CH14-27); B2-ST404 (CH14-807) and B2-ST1193 (CH14-64).

The subject may be a transplant or cancer patient (optionally a haematological cancer patient), or wherein the patient is suffering from or at risk of a urinary tract infection (UTI); and optionally wherein the transplant is a solid organ or stem cell transplant (optionally a haematopoietic cell transplant) or wherein the transplant is a transplant of a medical device.

For example, the subject is a haematological cancer patient suffering from neutropenia. For example, the subject is a haematopoietic stem cell transplant patient.

A suitable medical device may be, eg, heart device (eg, ventricular assist device, such as a left ventricular assist device (LVAD)), a catheter (eg, a biliary catheter) or a prosthesis (eg, a joint) prosthesis.

In an example, the patient is suffering from a sequestered B2 phylogroup *E coli* infection. In an example, the *E coli* are sequestered B2 phylogroup *E coli.*

For example, the subject is suffering from or at risk of acute bacterial sinusitis, pneumonia, urinary tract infections, chronic prostatitis or gastroenteritis caused by B2 phylogroup *E coli.* For example, the subject is a male human prostate surgery patient.

The method may be carried out prior to the subject receiving a transplant.

For example, the transplant is a solid organ or stem cell transplant (optionally a haematopoietic cell transplant).

The B2 *E coli* cells may comprise a strain of *E coli* that causes sepsis, septicaemia or diarrhoea in humans.

Enterohemorrhagic *Escherichia coli* (EHEC) serotype O157:H7 is a human pathogen responsible for outbreaks of bloody diarrhoea and haemolytic uremic syndrome (HUS) worldwide. Conventional antimicrobials trigger an SOS response in EHEC that promotes the release of the potent Shiga toxin that is responsible for much of the morbidity and mortality associated with EHEC infection. Cattle are a natural reservoir of EHEC, and approximately 75% of EHEC outbreaks are linked to the consumption of contaminated bovine-derived products. EHEC causes disease in humans but is asymptomatic in adult ruminants. Characteristics of *E*. *coli* serotype O157:H7 (EHEC) infection includes abdominal cramps and bloody diarrhoea, as well as the life-threatening complication haemolytic uremic syndrome (HUS). Currently there is a need for a treatment for EHEC infections (Goldwater and Bettelheim, 2012). The use of conventional antibiotics exacerbates Shiga toxin-mediated cytotoxicity. In an epidemiology study conducted by the Centers for Disease Control and Prevention, patients treated with antibiotics for EHEC enteritis had a higher risk of developing HUS (Slutsker et al., 1998). Additional studies support the contraindication of antibiotics in EHEC infection; children on antibiotic therapy for hemorrhagic colitis associated with EHEC had an increased chance of developing HUS (Wong et al., 2000; Zimmerhackl, 2000; Safdar et al., 2002; Tarr et al., 2005). Conventional antibiotics promote Shiga toxin production by enhancing the replication and expression of *stx* genes that are encoded within a chromosomally integrated lambdoid prophage genome. The approach of the compositions of the present invention relies on nuclease cutting of target cell genomic DNA. *Stx* induction also promotes phage-mediated lysis of the EHEC cell envelope, allowing for the release and dissemination of Shiga toxin into the environment (Karch et al., 1999; Matsushiro et al., 1999; Wagner et al., 2002). Thus, advantageously, the disclosure provides alternative means for treating B2 phylogroup EHEC in human and animal subjects. In an example, the subject (eg, a human) is suffering from or at risk of haemolytic uremic syndrome (HUS), eg, the subject is suffering from an E coli infection, such as an EHEC *E coli* infection.

The method may be for preventing haemolytic uremic syndrome (HUS), a UTI infection, sepsis, septicaemia or diarrhoea in the subject.

The composition or method may be for treating or preventing a blood stream infection by pathogenic B2 phylogroup *E coli* cells in the subject.

In an example, each particle of the composition comprises a phage capsid containing the nucleic acid wherein the capsid comprises capsid proteins of a T-even (optionally T4) or lambda phage.

As is known the to the skilled addressee, transduction particles are operable to infect their cognate host cells to introduce therein nucleic acid by transduction.

In an example, each particle of the composition is a phage (optionally a lytic phage) or packaged phagemid.

In an example, each particle type in the composition comprises a type of adhesion moiety or collection of adhesion moiety types that differs from the other types of particles.

In an example, 5 different types of transduction particle are administered to the subject. In an example, 6 different types of transduction particle are administered to the subject.

In an example, each particle of the composition comprises a phage capsid containing a said nucleic acid; wherein at least 4 different types of transduction particle are administered to the subject; and wherein a first type of transduction particle, a second type of transduction particle, a third type of transduction particle and a fourth type of transduction particle are administered to the subject, wherein the first type of particle comprises a first adhesion moiety that is capable of recognising and binding to LPS displayed on B2 *E coli,* the second type of particle comprises a second adhesion moiety that is capable of recognising and binding to LamB displayed on B2 *E coli,* the third type of particle comprises a second adhesion moiety that is capable of recognising and binding to Tsx displayed on B2 *E coli,* and the fourth type of particle comprises a second adhesion moiety that is capable of recognising and binding to LPS displayed on B2 *E coli,* wherein the adhesion moieties of said particles are different from each other.

In an example, each particle of the composition comprises a phage capsid containing a said nucleic acid; wherein at least 4 different types of transduction particle are administered to the subject; and wherein a first type of transduction particle, a second type of transduction particle, a third type of transduction particle and a fourth type of transduction particle are administered to the subject, wherein the first type of particle comprises a first adhesion moiety that is capable of recognising and binding to LPS displayed on B2 *E coli,* the second type of particle comprises a second adhesion moiety that is capable of recognising and binding to LamB displayed on B2 *E coli,* the third type of particle comprises a second adhesion moiety that is capable of recognising and binding to Tsx displayed on B2 *E coli,* and the fourth type of particle comprises a second adhesion moiety that is capable of recognising and binding to LamB displayed on B2 *E coli,* wherein the adhesion moieties of said particles are different from each other.

In an example, each particle of the composition comprises a phage capsid containing a said nucleic acid; wherein at least 4 different types of transduction particle are administered to the subject; and wherein a first type of transduction particle, a second type of transduction particle, a third type of transduction particle and a fourth type of transduction particle are administered to the subject, wherein the first type of particle comprises a first adhesion moiety that is capable of recognising and binding to LPS displayed on B2 *E coli,* the second type of particle comprises a second adhesion moiety that is capable of recognising and binding to LamB displayed on B2 *E coli,* the third type of particle comprises a second adhesion moiety that is capable of recognising and binding to Tsx displayed on B2 *E coli,* and the fourth type of particle comprises a second adhesion moiety that is capable of recognising and binding to Tsx displayed on B2 *E coli,* wherein the adhesion moieties of said particles are different from each other.

The nucleic acid of each particle of the composition may comprise a nucleotide sequence (N1) encoding said nuclease, wherein each particle is a synthetic T-even phage (optionally a T4 phage) comprising an insertion of N1 into the genome of the phage, wherein the region is between the *pin* (protease inhibitor) gene and the *iPII (*internal protein) gene.

Optionally, the phage is
(a) a synthetic T-even (eg, a T4) phage that comprises a deletion of DNA from, and/or an insertion of heterologous DNA into, a region of the genome of the phage corresponding to a region between coordinates
   (i) 1887 and 8983;
   (ii) 2625 and 8092;
   (iii) 1904 and 8113;
   (iv) 2668 and 7178;
   (v) 7844 and 11117;
   (vi) 8643 and 10313;
   (vii) 9231 and 13383;
   (viii) 9480 and 12224;
   (ix) 8454 and 17479; or
   (x) 9067 and 16673;
   wherein coordinates are with reference to wild-type T4 phage genome (SEQ ID NO: 5).

In an example, the nuclease is a Type I, II, III, IV, V or VI nuclease, optionally a Cas9 or a Cas3.

In an example,, at least 1 x 10⁷ PFU of particles are administered to the subject.

In an example, 1 x 10⁸ to 1 x 10¹³ PFU of particles are administered to the subject. In an example, 1 x 10⁸ to 1 x 10¹² PFU of particles are administered to the subject. In an example, 1 x 10¹⁰ to 1 x 10¹² PFU of particles are administered to the subject.

In an example, the particles are administered to the subject at an MOI (multiplicity of infection) of at least 0.01.

Optionally, the particles are administered to the subject at an MOI of no more than 1. Optionally, the particles are administered to the subject at an MOI from 0.001 to 1. Optionally, the particles are administered to the subject at an MOI from 0.01 to 1. Optionally, the particles are administered to the subject at an MOI from 0.1 to 1.

In an example, the strain or at least one strain is an antibiotic-resistant or MDR strain; and/or wherein the strain or at least one strain is a B2-I strain.

For example, the strain or at least one strain is a strain selected from B2-I (STc131), B2-II, B2-IX, and B2-VI.

For example, at least one MDR strain is resistant to fluoroquinolone and the strain is a beta-lactamase (ESBL)-producing *E coli.*

In an example, when the strain is an antibiotic-resistant or MDR strain, the antibiotic is fluoroquinolone (optionally levofloxacin), carbapenem or vancomycin; and/or wherein the *E coli* are beta-lactamase (ESBL)-producing *E coli.*

Optionally, the antibiotic is selected from ciprofloxacin (eg, Cipro^{™}), gemifloxacin (eg, Factive^{™}), levofloxacin (eg, Levaquin^{™}), moxifloxacin (eg, Avelox^{™}), and ofloxacin.

For example, the E coli produce CTX-M-15. CTX-M-15 is the most abundant enzyme in ESBL-producing E. coli causing human infections.

Preferably, the antibiotic is fluoroquinolone (FQ). For example, the FQ is levofloxacin. Levofloxacin, sold under the brand name Levaquin^{™} among others, is an antibiotic medication. It is used to treat a number of bacterial infections including acute bacterial sinusitis, pneumonia, urinary tract infections, chronic prostatitis, and some types of gastroenteritis. Levofloxacin prophylaxis is recommended to prevent gram-negative bloodstream infections (BSIs) in patients with prolonged chemotherapy-induced neutropenia. However, increasing fluoroquinolone resistance may decrease the effectiveness of this approach (eg, Clin Infect Dis. 2021 Oct 5;73(7):1257-1265. doi: 10.1093/cid/ciab404, "Colonization With Fluoroquinolone-Resistant Enterobacterales Decreases the Effectiveness of Fluoroquinolone Prophylaxis in Hematopoietic Cell Transplant Recipients, Michael J Satlin *et al*)*.* This study found that in the patients tested, nearly one-third of hematopoietic cell transplantation (HCT) recipients with pretransplant fluoroquinolone-resistant *Enterobacterales* (FQRE) colonization developed gram-negative bloodstream infections (BSIs) while receiving levofloxacin prophylaxis, and infections were typically caused by their colonizing strains. In contrast, levofloxacin prophylaxis was highly effective in patients not initially colonized with FQRE. The authors found that 23% of patients admitted for HCT were colonized with FQRE and *E*. *coli* was the predominant species. Patients with hematologic malignancies who receive intensive chemotherapy, including those undergoing hematopoietic cell transplantation (HCT), frequently develop severe neutropenia and gastrointestinal mucositis, placing them at high risk of developing bloodstream infections (BSIs) from gram-negative enteric bacteria *(Enterobacterales).* Neutropenic patients often suffer severe consequences from BSIs caused by *Enterobacterales,* with mortality rates as high as 15%-20%. Moreover, many fluoroquinolone-resistant Enterobacterales (FQRE) also harbor extended-spectrum β-lactamases (ESBLs); thus, breakthrough infections that occur despite fluoroquinolone prophylaxis may be resistant to first-line antimicrobial therapies for fever and neutropenia. Finally, adverse effects of fluoroquinolones have become increasingly apparent, including *Clostridioides difficile* infection, aortic dissection and rupture, dysglycemia, tendinopathy, QT interval prolongation, and mental status changes. Thus, fluoroquinolones should only be administered to patients when they are likely to provide clinical benefit to justify these potential adverse effects. Thus, Although fluoroquinolones may decrease the risk of gram-negative BSI in many patients, those who are colonized with FQRE may not benefit from fluoroquinolone prophylaxis.

The high rate and absence of risk factors for FQRE colonization suggest that FQRE are prevalent in the community. Indeed, a study of urinary isolates among outpatients in the United States demonstrated that 12% of *E*. *coli* isolates from young women and 29% of *E*. *coli* isolates from elderly women were fluoroquinolone resistant. A surveillance study of 1831 urinary *E*. *coli* isolates from 2017 found that one-quarter were FQ resistant. Furthermore, 13%-16% of men undergoing transrectal prostate biopsies were found to be colonized with fluoroquinolone-resistant *E. coli.* Nearly one-half of fluoroquinolone-resistant *E coli* isolates in the study were ST131, a common sequence type that has spread throughout the world and whose isolates are frequently fluoroquinolone resistant and ESBL producers.

Bacteraemia caused by extended-spectrum β-lactamase (ESBL)-producing *Enterobacteriaceae* (ESBL-E), such as *E coli,* is associated with inadequate empirical therapy and substantial mortality in neutropenic patients (see eg, "Colonization With Levofloxacin-resistant Extended-spectrum β-Lactamase-producing Enterobacteriaceae and Risk of Bacteremia in Hematopoietic Stem Cell Transplant Recipients", Satlin MJ et al, Clin Infect Dis. 2018 Nov 13;67(11):1720-1728. doi: 10.1093/cid/ciy363). The study found that HSCT recipients who are colonized with levofloxacin-resistant ESBL-E pre-transplant and receive levofloxacin prophylaxis have high rates of bacteraemia from their colonizing strain during neutropenia. In this single-centre study of 312 HSCT recipients, it was found that 10% of patients were colonized with ESBL-E prior to their transplant. Nearly one-third of patients with pre-transplant ESBL-E colonization developed subsequent ESBL-E bacteraemia while neutropenic after their transplant, compared to <1% of patients who were not initially colonized with ESBL-E. Furthermore, the bloodstream and gastrointestinal ESBL-E had identical MLST and PFGE profiles in all cases, suggesting that these patients developed bacteraemia from their colonizing isolates.

In an example, the composition or method is for preventing the translocation of B2 phylogroup *E coli* from the gastrointestinal tract to the blood stream of the subject, thereby preventing or reducing bacteriaemia in the patient.

In an example, the composition or method is for preventing the translocation of B2 phylogroup *E coli* from the urinary tract to the blood stream of the subject, thereby preventing or reducing bacteriaemia in the patient.

In an example, the *E coli* are comprised by the gastrointestinal tract of the subject. Optionally, in these examples, the composition is administered orally to the subject.

In another example, the *E coli* are comprised by the urinary tract of the subject. For example, the infection is a kidney, bladder or urethra infection. Optionally, in these examples, the composition is administered to the urinary tract of the subject, such as by a catheter.

In a second example, the claimed compositions are for use in a method for treating or preventing an infection by *E coli* cells in a human or animal subject, the method comprising administering to the subject a plurality of transduction particles, wherein
(a) each particle comprises a nucleic acid encoding a crRNA or guide RNA that is operable with a Cas nuclease for chromosomal targeting in the cells, wherein the said administered particles contact the cells and introduce therein the nucleic acid, wherein the crRNA or guide RNA is expressed and guides the Cas nuclease wherein the nuclease cuts the chromosomes of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

Each nucleic acid preferably encodes a plurality of different cRNAs comprising spacer sequences that target *E coli* chromosomal genes.

Each crRNA or guide RNA comprises a spacer that targets an *E coli* gene selected from the group fimH, bolA, rpoH, lptA and murA. Optionally, each nucleic acid encodes a plurality of different cRNAs or guide RNAs, wherein the cRNAs or guide RNAs target at least 2, 3 or 4 (or targets all of) *E coli* genes selected from the group fimH, bolA, rpoH, lptA and murA. Optionally, each nucleic acid encodes a plurality of different cRNAs or guide RNAs, wherein the cRNAs or guide RNAs target fimH and bolA. Optionally, each nucleic acid encodes a plurality of different cRNAs or guide RNAs, wherein the cRNAs or guide RNAs target rpoH and lptA. Optionally, each nucleic acid encodes a plurality of different cRNAs or guide RNAs, wherein the cRNAs or guide RNAs target fimH and murA. Optionally, each crRNA or guide RNA comprises a spacer sequence that is complementary to an *E coli* gene selected from the group fimH, bolA, rpoH, lptA and murA. Optionally, each crRNA or guide RNA comprises a spacer sequence that is at least 80, 90 or 95% identical to a nucleotide sequence selected from the group SEQ ID NO: 6-10.

Optionally, each nucleic acid encodes a first crRNA, second crRNA, third crRNA, fourth crRNA and fifth cRNA, wherein the cRNAs are different from each other and each crRNA targets a B2 phylogroup *E coli* gene. Optionally, each nucleic acid encodes a first crRNA, second crRNA, third crRNA, fourth crRNA and fifth cRNA, wherein the cRNAs are different from each other and each crRNA is complementary to a B2 phylogroup *E coli* gene. Optionally, each nucleic acid encodes a first crRNA, second crRNA, third crRNA, fourth crRNA and fifth cRNA, wherein the cRNAs comprise respectively SEQ ID NO: 6, SEQ ID NO: 7, SEQ ID NO: 8, SEQ ID NO: 9 and SEQ ID NO: 10. Optionally, each nucleic acid encodes a first crRNA, second crRNA, third crRNA, fourth crRNA and fifth cRNA, wherein the cRNAs comprise respectively a nucleotide sequence that is at least 80% identical to SEQ ID NO: 6, a nucleotide sequence that is at least 80% identical to SEQ ID NO: 7, a nucleotide sequence that is at least 80% identical to SEQ ID NO: 8, a nucleotide sequence that is at least 80% identical to SEQ ID NO: 9 and a nucleotide sequence that is at least 80% identical to SEQ ID NO: 10. Optionally, each nucleic acid encodes a first crRNA, second crRNA, third crRNA, fourth crRNA and fifth cRNA, wherein the cRNAs comprise respectively a nucleotide sequence that is at least 90% identical to SEQ ID NO: 6, a nucleotide sequence that is at least 90% identical to SEQ ID NO: 7, a nucleotide sequence that is at least 90% identical to SEQ ID NO: 8, a nucleotide sequence that is at least 90% identical to SEQ ID NO: 9 and a nucleotide sequence that is at least 90% identical to SEQ ID NO: 10. Optionally, each nucleic acid encodes a first crRNA, second crRNA, third crRNA, fourth crRNA and fifth cRNA, wherein the cRNAs comprise respectively a nucleotide sequence that is at least 95, 96, 97, 98 or 99% identical to SEQ ID NO: 6, a nucleotide sequence that is at least 95, 96, 97, 98 or 99% identical to SEQ ID NO: 7, a nucleotide sequence that is at least 95, 96, 97, 98 or 99% identical to SEQ ID NO: 8, a nucleotide sequence that is at least 95, 96, 97, 98 or 99% identical to SEQ ID NO: 9 and a nucleotide sequence that is at least 95, 96, 97, 98 or 99% identical to SEQ ID NO: 10.

In a third example, the composition comprising a plurality of transduction particles is for use in a method of treating or preventing an infection by *E coli* cells in a human or animal subject as described in the second example above, wherein
(a) each particle comprises a nucleic acid encoding a crRNA or guide RNA that is operable with a Cas nuclease for chromosomal targeting in the cells, wherein the said administered particles contact the cells and introduce therein the nucleic acid, wherein the crRNA or guide RNA is expressed and guides the Cas nuclease wherein the nuclease cuts the chromosomes of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.
Compositions described herein may be useful in a method of detecting the presence of a B2 phylogroup *E coli* in a sample, the method comprising contacting the sample comprising B2 phylogroup *E coli* with a composition comprising a plurality of transduction particles, wherein
(a) each particle comprises a nucleic acid encoding a nuclease for targeting the genomes of said *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject;
(b) each particle comprises an adhesion moiety for recognising and binding to a cognate moiety selected from a LPS, LamB and Tsx displayed on the surface of the phylogroup B2 *E coli* cells; and
(c) the method comprises detecting that B2 phylogroup *E coli* cells have been killed or the growth or proliferation thereof has been reduced.

Compositions described herein may be useful in a method of detecting the presence of a B2 phylogroup *E coli* in a sample, the method comprising contacting the sample comprising B2 phylogroup *E coli* with a composition comprising a plurality of transduction particles, wherein
(a) each particle comprises a nucleic acid comprising or encoding a detectable label, wherein the said administered particles contact the cells and introduce therein the nucleic acid, wherein optionally the label is expressed in the cells;
(b) each particle comprises an adhesion moiety for recognising and binding to a cognate moiety selected from a LPS, LamB and Tsx displayed on the surface of the phylogroup B2 *E coli* cells; and
(c) the method comprises detecting B2 phylogroup *E coli* cells comprising the label.

The compositions used in the methods of detection described above may comprise the features of the compositions described herein.

In the detection methods described above, the *E coli* may comprise one or more *E coli* strains selected from the group ST131, ST1193, ST648, ST315, ST405, ST361, ST88 and ST453.

In the detection methods described above, the sample may be a patient sample (eg, blood, urine, stool or saliva sample), wherein the subject is a transplant or cancer patient (optionally a haematological cancer patient), or wherein the patient is suffering from or at risk of a urinary tract infection (UTI); and optionally wherein the transplant is a solid organ or stem cell transplant (optionally a haematopoietic cell transplant).

In the detection methods described above, the nucleic acid of each particle may comprise a nucleotide sequence (N1) encoding said nuclease, or comprising or encoding the label wherein each particle is a synthetic T-even phage (optionally a T4 phage) comprising an insertion of N1 into the genome of the phage, wherein the region is between the *pin* (protease inhibitor) gene and the *iPII (*internal protein) gene.

In the detection methods described above, at least 1 x 10⁷ PFU of particles may be contacted with the sample.

In the detection methods described above, the particles may be contacted with the sample at an MOI (multiplicity of infection) of at least 0.01.

Labelling for detection methods is routine for the skilled addressee. The label may, for example, be a fluorescence label, eg, GFP. The sample may be a blood, spit, sputum or cell sample.

The disclosure also provides the fourth and fifth examples, which are fully supported by Example 2. The fourth and fifth examples can be combined with any other features disclosed herein.

Patients with hematological malignancies frequently develop bloodstream infections due to translocation of E. *coli* and other bacteria from the gut. Antibiotic treatment to prevent these infections has detrimental effects on the microbiome and immune tonus and is further hampered by increasing antibiotic resistance, in particular towards fluroquinolones. As described in Example 2, compositions of the Concepts may target bacteria in biofilms, reduce the emergence of phage-tolerant *E. coli* and out-compete their ancestral WT phages in co-culture experiments. Compositions are provided with broad host-range across the E. *coli* phylogeny, including multi-drug resistant strains (B2 phylogroup). Compositions comprising different particles as per the Concepts may surprisingly reduce E. *coli* load in the murine gut better than individual constituent particles. The compositions, methods and doses of the Concepts find utility to selectively kill E. *coli* which may cause fatal infections in hematological cancer, transplant or UTI patients. The compositions, methods and doses also find utility for treating or preventing *E coli* blood stream infection.

In a fourth example, the claimed compositions comprising a plurality of transduction particles are for use in a method of treating or preventing an infection by *E coli* cells in a human or animal subject, wherein the method comprises administering the particles to the subject, wherein
(a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx (optionally selected from LPS and Tsx) displayed on the surface of the phylogroup B2 *E coli* cells.

In a fifth example, the claimed compositions comprising a plurality of transduction particles are for use in a method of treating or preventing an infection by B2 phylogroup *E coli* cells in a human or animal subject, wherein the method comprises administering the particles to the subject, wherein (a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject; and
(b) each particle comprised by the composition is a T-even phage capsid; optionally a capsid of a T2 phage, T2-like phage, RB69 phage or a RB69-like phage.

The following features are optional features that are combinable with any other feature herein.

Each particle of the claimed compositions comprises a phage capsid containing the nucleic acid. In an example, the capsid comprises capsid proteins of a T-even (optionally T2) or lambda phage.

Optionally, the capsid of each particle comprised by the composition is a T-even phage capsid; optionally a capsid of a T2 phage, T2-like phage, RB69 phage or a RB69-like phage.

Optionally, each particle is a phage (optionally a lytic phage) or packaged phagemid.

According to the invention, the composition comprises at least 4 different types of transduction particles. In an example the composition has 4 (but no more than 4) different types of transduction particles.

Optionally, the nucleic acid of each particle comprises at least one nucleotide sequence (N1) encoding said POI, wherein each particle is a synthetic T-even phage comprising an insertion of N1 into a Modification Permissive Region (MPR) of the genome of the phage, wherein the MPR is immediately after *gene* 49 and to *gene E* when compared to a reference wild-type T2 phage. The nucleic acid may comprise a DNA deletion of phage DNA in the MPR. The insertion may comprise up to 5000, 6000, 7000 or 8000bp of DNA and/or the deletion comprises up to 5000, 6000, 7000 or 8000bp of DNA. The MPR may comprise contiguous DNA between said *gene* 49 and *gene E,* wherein the contiguous DNA is at least 1000bp in length; or wherein the MPR comprises at least 100bp of DNA between said *gene* 49 and *gene E.*

Optionally a T-even phage herein is a phage selected from T2, T4 or T6 phage; or comprises a genome that is at least 95% identical to the genome of a said selected phage. This percentage may be at least 96, 97, 98 or 99%.

In an example, the synthetic phage genome comprises said insertion between coordinates 9000 and 21000, wherein the coordinates are the nucleotide positions counted from the nucleotide (coordinate number 1) immediately after *gene* 49 towards *gene E* when compared to a reference wild-type T2 phage. Optionally, the insertion is between coordinates 10300 and 19800, eg, between 10359 and 19810 with reference to the T2 genome.

Optionally, the insertion is in a window selected from the following windows (numbers being coordinates with reference to the T2 genome):-
9000-21000; 10000-21000, 10100-21000, 10200-21000, 10300-21000, 10400-21000, 10500-21000, 11000-21000, 15000-21000;
9000-20000; 10000-20000, 10100-20000, 10200-20000, 10300-20000, 10400-20000, 10500-20000, 11000-20000, 15000-20000;
9000-19500; 10000-19500, 10100-19500, 10200-19500, 10300-19500, 10400-19500, 10500-19500, 11000-19500, 15000-19500;
9000-19000; 10000-19000, 10100-19000, 10200-19000, 10300-19000, 10400-19000, 10500-19000, 11000-19000, 15000-19000;
9000-18000; 10000-18000, 10100-18000, 10200-18000, 10300-18000, 10400-18000, 10500-18000, 11000-18000 and 15000-18000.

Optionally, the Cas nuclease is a dsDNA nuclease, ie, is capable of cutting dsDNA. Optionally, the Cas nuclease is a nickase, eg, a Cas9 nickase.

The nuclease may be a Type I, II, III, IV, V or VI Cas nuclease, optionally a Cas9 or a Cas3.

In a preferred example, the nucleic acid comprises (optionally in 5' to 3' order) a *cas3* gene (*ygcB*) and a cognate cascade gene complex comprising *casA* (*ygcL, cas8e), casB* (*vgcK, casll), casC (ygcJ, cas7), casD* (*ygcI, cas5),* and *casE (ygcH, cas6),* and optionally a CRISPR array or nucleotide sequence encoding a guide RNA that targets an *E. coli* genome.

The POI comprises at least one crRNA or guide RNA that is operable with a Cas for DNA targeting in *E coli* cells. Optionally, each crRNA or guide RNA comprises a spacer sequence that is complementary to an *E coli* protospacer sequence; optionally a protospacer of a B2 phylogroup *E coli* cell or an *E coli* cell of a strain selected from the group ST131, ST1193, ST648, ST315, ST405, ST361, ST88 and ST453. According to the invention, the particles of the composition target all of *E coli* genes *fimH, bolA, rpoH, lptA* and *murA.*

Each nucleic acid preferably encodes a plurality of different cRNAs comprising spacer sequences that target *E coli* chromosomal genes. For example, each nucleic acid preferably encodes a plurality of different cRNAs comprising spacer sequences that target 2, 3 or 4 *E coli* chromosomal genes selected from fimH, bolA, rpoH, lptA and murA. In an example, the composition comprises a type of transduction particle that targets *E coli* genes *bolA, rpoH and fimH.* Additionally or alternatively, the composition comprises a type of transduction particle that targets *E coli* genes *lptA* and *murA.* Optionally, the composition comprises a first type of transduction particle that targets *E coli* genes *bolA, rpoH and fimH;* and a second type of transduction particle that targets *E coli* genes *lptA* and *murA.* Optionally, the first type of particle is according any other first type of particle herein which comprises a LPS adhesion moiety (eg, comprises LPS and Tsx adhesion moieties). Optionally, the second type of particle is according any other first type of particle herein which comprises a Tsx adhesion moiety. Optionally, the second type of particle comprises a LPS adhesion moiety (eg, comprises LPS and LamB adhesion moieties).

The protospacer sequence may comprised by a gene selected from *E coli* genes *fimH, bolA, rpoH, lptA* and *murA.*

According to the invention, the particles of the composition target all of *E coli* genes *fimH, bolA, rpoH, lptA* and *murA.*

The nucleotide sequence encoding the POI may comprise a stress-phase active (SPA) promoter for expression of the POI in *E coli cells.* The promoter may be an *E coli bolA* promoter. The promoter may comprise SEQ ID NO: 13.

In an example, the *E coli* cells comprise a strain of *E coli* that causes sepsis, septicaemia or diarrhoea in humans or animals.

Optionally, the *E coli* cells are GI tract cells. Optionally, the *E coli* cells are comprised by a gut microbiome. Optionally, the *E coli* cells are comprised by blood of the subject. Optionally, the *E coli* cells are comprised by a urinary tract of the subject. Optionally, the E coli cells are comprised by a microbiome selected from a GI tract (eg, stomach), blood, urinary tract, mouth, nose, eye, ear, skin, anus or hair microbiome.

In an example, the composition is for use in a method of treating or preventing infection of by *E coli* cells in a human or animal subject, wherein the method comprises administering the particles to the subject.

Optionally, the method is for preventing the translocation of B2 phylogroup *E coli* from the gastrointestinal or urinary tract to the blood stream of the subject, thereby preventing or reducing bacteriaemia in the subject.

Optionally, the method is for preventing *E coli* infection in a subject at risk of febrile neutropenia. The subject may be a cancer patient, such as a haematological cancer patient. The infection may be a bloodstream infection in the subject. Thus, in an example, the method is for preventing a bloodstream *E coli* infection in a subject at risk of febrile neutropenia, wherein the subject is a human cancer patient, such as a haematological cancer patient.

There is provided:-
A method of treating or preventing sepsis, septicaemia or diarrhoea in a human or animal subject, the method comprising administering to the subject the composition described herein, wherein the *E coli* cells comprise a strain of *E coli* that causes sepsis, septicaemia or diarrhoea in humans or animals.

A method of treating or preventing infection of by *E coli* cells in a human or animal subject,
wherein the method comprises administering the composition described herein to the subject, wherein the infection is treated or prevented.

The infection may be reduced or eliminated. The infection may be reduced by at least 20, 30, 40, 50, 60, 70, 80 or 90%.

The subject may be a transplant or cancer patient (optionally a haematological cancer patient), or wherein the patient is suffering from or at risk of a urinary tract infection (UTI). Optionally, the transplant is a solid organ or stem cell transplant (optionally a haematopoietic cell transplant) or wherein the transplant is a transplant of a medical device. The subject may be suffering from a haematological cancer, eg, leukaemia. The subject (eg, cancer patient) may be at risk of neutropenia or may be suffering from neutropenia. Preferably, the subject is suffering from a haematological cancer, eg, leukaemia, and is at risk of neutropenia or suffering from neutropenia.

Optionally, the method is carried out prior to the subject receiving a or said transplant. The transplant may be a stem cell transplant, eg, when the subject is a cancer patient.

The composition or method may be for preventing haemolytic uremic syndrome (HUS), a UTI infection, sepsis, septicaemia or diarrhoea in a human subject.

In the composition for use in a method, or any method described herein, at least 1 x 10⁷ PFU of particles are administered to the subject. Also provided is a dose of a composition described herein, wherein the dose is a dose of at least 1 x 10⁷ PFU of said particles. For example, the dose is at least 1 x 10⁷, 1 x 10⁸, 1 x 10⁹, 1 x 10¹⁰ or 1 x 10¹¹ PFU of said particles. For example, the dose is 1 x 10⁷, 2 x 10⁹ or 2 x 10¹¹ PFU of said particles. For example, the dose is from 1 x 10⁷ to 2 x 10¹¹ PFU of said particles. For example, the dose is 1 x 10⁷ to 2 x 10¹¹ PFU of said particles per gram body weight of the subject. In the method, *E coli* may be reduced by at least a 3 or 4 log10 CFU/g of body weight of the subject.

The dose may be comprised by a medical device or container, eg, for oral or intravenous administration. The device may be an IV device, syringe or comprise an injection needle. The device or container may be sterile.

Optionally, the particles are administered to the subject at an MOI (multiplicity of infection) of at least 0.01. For example, the MOI is at least 0.1 or 1.

Preferably, the *E coli* cells comprise at least one strain that is an antibiotic-resistant or MDR strain; and/or at least one B2-I strain. Optionally, the antibiotic is fluoroquinolone (optionally levofloxacin), carbapenem or vancomycin; and/or wherein the *E coli* cells comprise beta-lactamase (ESBL)-producing *E coli.*

According to the disclosure, there is provided:-
A method of detecting the presence of *E coli* (optionally B2 phylogroup *E coli* cells) in a sample, the method comprising
(a) contacting the sample with a composition described herein; and
(b) detecting that *E coli* cells have been killed or the growth or proliferation thereof has been reduced.

According to the disclosure, there is provided a method of detecting the presence of *E coli* (optionally B2 phylogroup *E coli* cells) in a sample, the method comprising
(a) contacting the sample with a a composition described herein; wherein particles of the composition comprise a nucleic acid comprising or encoding a detectable label, wherein the said particles contact the cells and introduce therein the nucleic acid, wherein optionally the label is expressed in the cells; and
(b) detecting that *E coli* cells comprising the label.

According to the disclosure, there is provided a method for modifying the genomes of *E coli* cells, the method comprising contacting the cells with a composition described herein, wherein nucleic acid encoding the POI is introduced into the cells thereby modifying the genomes of the cells.

Where the POI is a nuclease, the nuclease is expressed in the cells and cuts genomic DNA of the cells, thereby killing the cells or reducing growth or proliferation of the cells in the subject.

According to the disclosure, there is provided a method for treating or preventing an infection by E *coli* cells in a human or animal subject, the method comprising administering to the subject a composition described herein.

Optionally, the sample is a patient sample (eg, blood, urine, stool or saliva sample), wherein the subject is a transplant or cancer patient (optionally a haematological cancer patient), or wherein the patient is suffering from or at risk of a urinary tract infection (UTI); and optionally wherein the transplant is a solid organ or stem cell transplant (optionally a haematopoietic cell transplant).

The method may be carried out *in vitro.* The method may be carried out *ex vivo.*

Optionally, at least 1 x 10⁷ PFU of particles are contacted with the sample. Optionally the PFU of particles described above is contacted with the sample.

Optionally, the particles are contacted with the sample at an MOI (multiplicity of infection) of at least 0.01.

Optionally, each particle comprised by the composition comprises a T-even phage capsid; optionally a capsid of a T2 phage, T2-like phage, RB69 phage or a RB69-like phage. Each particle may comprise a capsid of a *Tevenvirinae* phage. Each particle may be a modified *Tevenvirinae* phage that comprises a genomic insertion of a nucleotide sequence encoding POI or said nuclease (and optionally a cognate crRNA when the nuclease is a Cas).

Each particle may be a phage or packaged phagemid. Preferably, the phage is a lytic phage. In another example, the phage may be a non-lytic phage. In another example, the phage may be a temperate phage.

Optionally the phage is a modified T2 phage, T2-like phage, RB69 phage or a RB69-like phage. Preferably the phage is a T2- or RB69-like phage. Preferably, each particle is a modified first phage, wherein the genome of the first phage is at least 95, 96, 97, 98 or 99 % identical (by nucleotide sequence identity) to the genome of wild-type T2 or RB69. Preferably, said percentage is at least 95%. Preferably, percentage identity between phage genomes is determined by Mash analysis using a kmer size of 17 and 1000.

The cells comprise B2 phylogroup *E coli.* The cells are preferably pathogenic cells. The cells are preferably pathogenic to the subject. The cells may mediate a disease or condition in the subject.

In an example in the compositions of the disclosure
(i) the nuclease is a guided Cas nuclease for targeting the DNA of an *E coli* cell, wherein the nuclease is capable of being expressed in the cell for cutting the DNA of the cell, thereby killing the cell;
(ii) each particle comprises a phage capsid containing the nucleic acid; and
(iii) the particles of the composition target a plurality of different *E coli* genes, optionally selected from essential and virulence genes.

The nuclease is a Cas nuclease and the nucleic acid encodes at least one crRNA or guide RNA that is operable with the Cas for DNA targeting in *E coli* cells. Optionally, each crRNA or guide RNA comprises a spacer sequence that is complementary to an *E coli* protospacer sequence; optionally a protospacer of a B2 phylogroup *E coli* cell or an *E coli* cell of a strain selected from the group ST131, ST1193, ST648, ST315, ST405, ST361, ST88 and ST453. Each protospacer sequence may be comprised by a gene selected from *E coli* genes *fimH, bolA, rpoH, lptA* and *murA.* According to the invention, the particles of the composition target all of *E coli* genes *fimH, bolA, rpoH, lptA* and *murA.*

Optionally, the particles of the composition target at least one virulence gene and at least one essential gene. According to the invention, the particles of the composition target all of *E coli* genes *fimH*, *bolA, rpoH, lptA* and *murA.*

Expression of the POI or nuclease or crRNA may be under the control of a stress-phase active (SPA) promoter. Optionally, the promoter is an *E coli bolA* promoter or comprises SEQ ID NO: 13.

Additionally or alternatively, expression of the POI or nuclease or crRNA may be under the control of an *E. coli* promoter PJ23100 (SEQ ID NO: 14), for example a promoter having a nucleotide sequence that is at least 80, 90, 95, 96, 97, 98 or 99% identical to SEQ ID NO: 14. For example, the promoter has the nucleotide sequence of SEQ ID NO: 16.

A spacer herein may be adjacent a direct repeat sequences in the nucleic acid comprised by the particles. For example, the repeat has the sequence of SEQ ID NO: 15 (or said sequence with up to 5, 4, 3, or 2 nucleotide changes compared with SEQ ID NO: 15).

In an example, expression of the POI or nuclease or crRNA may be under the control of a first promoter and expression of the adhesion moiety/ies is under the control of a second promoter that is different from the first promoter.

An adhesion moiety herein is on the outer surface of the cognate particle. In an example, the moiety is comprised by a tail fibre, spike or capsid of the particle, preferably a tail fibre.

In a sixth example, the claimed compositions comprising a plurality of transduction particles are for use in a method of treating or preventing an infection by *E coli* cells in a human or animal subject, wherein the method comprises administering the particles to the subject, wherein
(a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

In a seventh example, the claimed compositions are for use in a method for treating or preventing an infection by *E coli* cells in a human or animal subject, the method comprising administering to the subject a plurality of transduction particles, wherein the method comprises administering the particles to the subject, wherein
(a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The method may be for treating or preventing infection of the subject by an *E coli* strain selected from the group ST131, ST1193, ST648, ST315, ST405, ST361, ST88 and ST453.

The method may be for treating or preventing infection of the subject by a plurality of different phylogroup B2 strains of *E coli;* optionally wherein the plurality comprises *E coli* ST131 and ST1193 cells.

The subject may be a transplant or cancer patient (optionally a haematological cancer patient), or wherein the patient is suffering from or at risk of a urinary tract infection (UTI); and optionally wherein the transplant is a solid organ or stem cell transplant (optionally a haematopoietic cell transplant) or wherein the transplant is a transplant of a medical device.

The method may be carried out prior to the subject receiving a transplant.

The B2 *E coli* cells may comprise a strain of *E coli* that causes sepsis, septicaemia or diarrhoea in humans.

The method may be for preventing haemolytic uremic syndrome (HUS), a UTI infection, sepsis, septicaemia or diarrhoea in the subject.

Each particle may comprise a phage capsid containing the nucleic acid and wherein the capsid comprises capsid proteins of a T-even (optionally T4) or lambda phage.

Each particle may be a phage (optionally a lytic phage) or packaged phagemid.

At least 4 different types of transduction particle are administered to the subject.

The nucleic acid of each particle in the composition may comprise a nucleotide sequence (N1) encoding said nuclease, wherein each particle is a synthetic T-even phage (optionally a T4 phage) comprising an insertion of N1 into the genome of the phage, wherein the region is between the *pin* (protease inhibitor) gene and the *iPII (*internal protein) gene.

The nuclease may be a Type I, II, III, IV, V or VI Cas nuclease, optionally a Cas9 or a Cas3.

At least 1 x 10⁷ PFU of particles may be administered to the subject.

The particles may be administered to the subject at an MOI (multiplicity of infection) of at least 0.01.

The strain or at least one strain may be an antibiotic-resistant or MDR strain; and/or wherein the strain or at least one strain is a B2-I strain. Optionally, the antibiotic is fluoroquinolone (optionally levofloxacin), carbapenem or vancomycin; and/or wherein the *E coli* are beta-lactamase (ESBL)-producing *E coli.*

In an eighth example, the claimed compositions are for use in a method for treating or preventing an infection by *E coli* cells in a human or animal subject, the method comprising administering to the subject a plurality of transduction particles, wherein
(a) each particle comprises a nucleic acid encoding a crRNA or guide RNA that is operable with a Cas nuclease for chromosomal targeting in the cells, wherein the said administered particles contact the cells and introduce therein the nucleic acid, wherein the crRNA or guide RNA is expressed and guides the Cas nuclease wherein the nuclease cuts the chromosomes of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The method may be for preventing the translocation of B2 phylogroup *E coli* from the gastrointestinal or urinary tract to the blood stream of the subject, thereby preventing or reducing bacteriaemia in the patient.

The compositions described herein may be useful in:-
A method for treating or preventing an infection by *E coli* cells in a human or animal subject, the method comprising administering to the subject a plurality of transduction particles, wherein
(a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The compositions described herein may be useful in:-
A method for treating or preventing an infection by *E coli* cells in a human or animal subject, the method comprising administering to the subject a plurality of transduction particles, wherein
(a) each particle comprises a nucleic acid encoding a crRNA or guide RNA that is operable with a Cas nuclease for chromosomal targeting in the cells, wherein the said administered particles contact the cells and introduce therein the nucleic acid, wherein the crRNA or guide RNA is expressed and guides the Cas nuclease wherein the nuclease cuts the chromosomes of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The compositions described herein may be useful in:-
A method for treating or preventing *E coli* bacteriaemia in a human or animal subject, the method comprising administering to the subject a plurality of transduction particles, wherein
(a) each particle comprises a nucleic acid encoding a nuclease for targeting the genomes of cells of said *E coli,* wherein the said administered particles contact the cells and introduce therein the nucleic acid, wherein the nuclease is expressed in the cells and cuts genomic DNA of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The compositions described herein may be useful in:-
A method for treating or preventing *E coli* bacteriaemia in a human or animal subject, the method comprising administering to the subject a plurality of transduction particles, wherein
(a) each particle comprises a nucleic acid encoding a crRNA or guide RNA that is operable with a Cas nuclease for chromosomal targeting in cells of said *E coli,* wherein the said administered particles contact the cells and introduce therein the nucleic acid, wherein the crRNA or guide RNA is expressed and guides the Cas nuclease wherein the nuclease cuts the chromosomes of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The compositions described herein may be useful in:-
Use of a composition comprising a plurality of transduction particles in a method for treating or preventing an infection by phylogroup B2 *E coli* cells in a human or animal subject, the method comprising administering to the subject the composition, wherein
(a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject; and
(b) each particle comprises an adhesion moiety for recognising and binding to a cognate moiety selected from a LPS, LamB and Tsx displayed on the surface of the phylogroup B2 *E coli* cells.

The compositions described herein may be useful in:-
Use of a composition comprising a plurality of transduction particles in a method for treating or preventing an infection by phylogroup B2 *E coli* cells in a human or animal subject, the method comprising administering to the subject the composition, wherein
(a) each particle comprises a nucleic acid encoding a crRNA or guide RNA that is operable with a Cas nuclease for chromosomal targeting in the cells, wherein the said administered particles contact the cells and introduce therein the nucleic acid, wherein the crRNA or guide RNA is expressed and guides the Cas nuclease wherein the nuclease cuts the chromosomes of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The compositions described herein may be useful in:-
Use of a composition comprising a plurality of transduction particles in a method for treating or preventing phylogroup B2 *E coli* bacteraemia in a human or animal subject, the method comprising administering to the subject the composition, wherein
(a) each particle comprises a nucleic acid encoding a nuclease for targeting the genomes of cells of said *E coli,* wherein the said administered particles contact the cells and introduce therein the nucleic acid, wherein the nuclease is expressed in the cells and cuts genomic DNA of the cells, thereby killing the cells or reducing growth or proliferation of the cells in the subject; and
(b) each particle comprises an adhesion moiety for recognising and binding to a cognate moiety selected from a LPS, LamB and Tsx displayed on the surface of the phylogroup B2 *E coli* cells.

The compositions described herein may be useful in:-
Use of a composition comprising a plurality of transduction particles in a method for treating or preventing phylogroup B2 *E coli* bacteraemia in a human or animal subject, the method comprising administering to the subject the composition, wherein
(a) each particle comprises a nucleic acid encoding a crRNA or guide RNA that is operable with a Cas nuclease for chromosomal targeting in cells of said *E coli,* wherein the said administered particles contact the cells and introduce therein the nucleic acid, wherein the crRNA or guide RNA is expressed and guides the Cas nuclease wherein the nuclease cuts the chromosomes of the cells, thereby killing the cells or reducing growth or proliferation of the cells in the subject;
(b) the *E coli* cells are cells of *E coli* phylogroup B2; and
(c) each particle comprises an adhesion moiety for recognising and binding to a cognate moiety selected from a LPS, LamB and Tsx displayed on the surface of the phylogroup B2 *E coli* cells.

The compositions described herein may be useful in:-
A composition comprising a plurality of transduction particles for use in a method of treating or preventing an infection by *E coli* cells (optionally B2 phylogroup *E coli* cells)in a human or animal subject, wherein the method comprises administering the particles to the subject, wherein (a) each particle comprises a nucleic acid encoding a nuclease for targeting the genomes of *E coli* cells, wherein the said administered particles contact the cells and introduce therein the nucleic acid, wherein the nuclease is expressed in the cells and cuts genomic DNA of the cells, thereby killing the cells or reducing growth or proliferation of the cells in the subject; and
(b) each particle comprised by the composition is a T-even phage capsid; optionally a capsid of a T2 phage, T2-like phage, RB69 phage or a RB69-like phage.

The compositions described herein may be useful in:-
A method of treating or preventing sepsis, septicaemia or diarrhoea in a human or animal subject, the method comprising administering to the subject the composition described herein, wherein the *E coli* cells comprise a strain of *E coli* that causes sepsis, septicaemia or diarrhoea in humans or animals.

The compositions described herein may be useful in:-
A method of treating or preventing infection of by *E coli* cells in a human or animal subject, wherein the method comprises administering the composition described herein to the subject, wherein the infection is treated or prevented.

Those skilled in the art will recognize, or be able to ascertain using no more than routine study, numerous equivalents to the specific procedures described herein.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" or similar as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

The present invention is described in more detail in the following non-limiting Examples.

### EXAMPLES

### EXAMPLE 1: Particle Composition for surprisingly targeting a plurality of different E coli strains, including B2 phylogroup

### Overview

Patient samples containing various different *E coli* strains were challenged with a particle composition. The composition comprised a plurality of transduction particles bearing adhesion moieties that are able to bind to LPS, LamB or Tsx present on *E coli* cells. The particles comprised capsids comprising phage capsid proteins, with the capsids containing nucleic acids encoding CRISPR/Cas systems for chromosomal targeting in *E coli.* Each nucleic acid encoded a plurality of different cRNAs comprising spacer sequences that target *E coli* chromosomal genes. The ability to kill or reduce growth of *E coli* in the samples was determined using a plaque assay as described below. Whole genome sequencing and genome assembly were used to assign susceptible *E coli* strains to phylogroups. It was surprisingly seen that use of such a particle composition was able to very effectively and extensively target a plurality of different *E coli* strains (these strains being clinically relevant from actual patient samples). Furthermore, advantageously a large number of different strains of the B2 phylogroup were targeted and killed. This is significant, because B2 *E coli* strains often display antibiotic resistance (such as MDR), eg, resistance to fluoroquinolone, that causes potentially life-threating infections in patients, such as in cancer, transplant and UTI patients. In addition to the B2 group, we surprisingly also were able to successfully kill or inhibit the growth of multiple strains of *E coli* phylo groups B1, D, F and G.

### Patient Sampling

The samples tested (n=71) were obtained from two prospective observational studies enrolling adult (≥18 years) patients who were admitted to hospital for an autologous or allogenic hematopoietic cell transplantation (HCT) and who received levofloxacin (a fluoroquinolone, FQ) prophylaxis starting the day before the transplantation (Day -1) (Satlin 2021 and Satlin 2018). Trimethoprim-sulfamethoxazole (TMP-SMX) was administered to allogenic HCT recipients from 2 to 4 days prior to the HCT. The antibiotic treatments do not eradicate all *E coli* in the patients; for example, fluoroquinolone-resistant *E coli* would persist and thus sampling obtained *E coli* from patients. *E coli* isolates were derived from either anal swabs or fecal samples obtained upon admission for transplantation. The timing of sampling varied from Day -7 (7 days before transplantation) to Day 0 (the day of transplantation).

### General Plaque Assay: Spotting for Coverage

This procedure describes the method to assess the coverage of phage particle lysates on a panel of bacterial strains.

| Section | Procedure | |
|---|---|---|
| Step 1 | Prepare bacterial indicator strains by inoculating 5µl of a frozen stock into 250µl of LB broth on a 96 well plate. Incubate overnight at 37°C and 250 rpm. This step is done the day before the assay is performed. | |
| Step 2 | Pre-acclimatize the LB plates to room temperature. | |
| | Melt and acclimatize the top agar to 55°C and add CaCl₂ and MgSO₄ to a final concentration of 5 mM. | |
| Step 3 | In a culture tube mix 100 ml of the overnight indicator strain with 3 ml of pre-warmed top agar. When working with many strains, the top agar can be distributed to culture tubes and kept at 60 °C before use. | |
| Step 4 | Pour the mixture on top of the room temperature LB plate and distribute evenly by swirling. | |
| | Let plates solidify in a biosafety cabinet or lab bench for 5-10 minutes. Keep the lids closed. While solidifying avoid stacking plates to keep the layer of the top agar even across the plate surface | |
| Step 5 | In the meantime, dilute the sample(s) or the stock of phage to the required dilutions (10-fold dilutions). Use PBS for dilutions. | |
| Step 6 | Spot dilutions 0 to -9, 5 µl of each on top of your overlays and let them dry for 20 minutes (or until the spots are completely absorbed by the agar) on a bench with the lids open. Incubate plates upside down at 37°C overnight. | |
| Step 7 | Evaluation of the results: | |
| | | 1. If visible plaques appear, they are counted, and phage concentration is calculated. The result is recorded as YES. |
| | | Calculation of phage concentration: number of plaques x 200 (to make it into ml) x dilution where you counted. i.e: If counted 5 plaques on dilution - 6: 5 x 200 x 1e6 = 1e9 pfu/ml |
| | | 2. If there are no visible plaques, but inhibition of growth is observed the result is recorded as LZ (lysis zone) and the lowest dilution of inhibition is noted. |
| | | 3. If no plaques or inhibition is observed, the result is recorded as NO |

Spotting was performed according to the above general plaque assay. Bacterial strains were prepared by inoculating 5µl of a frozen stock into 250µl of LB broth in a 96 well plate. The plate was incubated at 37°C and 250 rpm overnight. Next day, in a culture tube 100 ml of the overnight strain was mixed with 3 ml of pre-warmed top agar (at 55°C) containing 5mM CaCl₂ and 5mM MgSO₄. The mixture was poured on top of pre-acclimatized LB plate and distributed evenly by swirling. The plates were left on the lab bench for 5-10 minutes to solidify. In the meantime, the particle composition was diluted in PBS buffer from 10⁰ to 10⁻⁹ and 5 µL of each serial dilution were spotted on top of the overlays. Plates were left on the bench with the lid open for 20 minutes or until the spots are completely absorbed by the agar, and then incubated upside down at 37°C overnight.

### Evaluation of the results:

If visible plaques appeared, the results were recorded as positive, plaques were counted, and phage concentration was calculated. Calculation of phage concentration: number of plaques x 200 x dilution where plaques were observed. i.e: If counted 5 plaques on dilution - 6: 5 x 200 x 1e6 = 1e9 pfu/ml
If there were no visible plaques, but inhibition of growth was observed, the result was recorded as lysis zone and the lowest dilution of inhibition is noted.

If no plaques or inhibition was observed, the results were recorded as negative.

### Whole genome sequencing

DNA extraction was performed using Omega Bio-tek, Mag-Bind Bacterial DNA 96 Kit. The protocol was followed, and samples were eluted in 100µL Elution Buffer.

Sequencing libraries were generated using Illumina Nextera XT, and sequencing was performed with paired ends on an Illumina MiSeq instrument with a V2 flow cell (300 cycles). The average sequencing depth for all samples was 48x (range: 31-72x)

### Genome assembly and phylogenetic tree reconstruction

Raw data was trimmed for adaptor sequences and low-quality bases using fastp 0.22.0 (Chen et al 2018). Genomes were assembled using SKESA 2.4.0 (Souvorov et al 2018). The phylogroup of each sample was determined using EzClermont 0.7.0 (https://github.com/nickp60/EzClermont). Genomic distances were estimated using Mash 1.1 (Ondov et al 2016) with a kmer size of 17 and 1000 sketches. A neighbour joining tree was constructed using rapidnj 2.3.2 (Simonsen et al 2008). The final tree visualization was generated in Interactive Tree Of Life (iTOL) version 6.5.3 (Letunic et al 2021). Strain phylotyping was carried out in silico using the method disclosed in Microb Genom. 2018 Jul; 4(7): e000192, Published online 2018 Jun 19. doi: 10.1099/mgen.0.000192, PMCID: PMC6113867, PMID: 29916797, "ClermonTyping: an easy-to-use and accurate in silico method for Escherichia genus strain phylotyping, Johann Beghain *et al.* The set of primer sequences described in Table S1 of that reference (available in the online version of this article) was used.

### Results

It was surprisingly seen that use of such a particle composition was able to very effectively and extensively target a plurality of different *E coli* strains (these strains being clinically relevant from actual patient samples), see Figure 1. Furthermore (see Figure 2), advantageously a large number of different strains of the B2 phylogroup were targeted and killed. This is significant, because B2 *E coli* strains often display antibiotic resistance (such as MDR), eg, resistance to fluoroquinolone, that causes potentially life-threating infections in patients, such as in cancer, transplant and UTI patients. More than 10 different ST131 strains were killed (plaques formed) and more than 10 different ST1193 strains were killed (plaques formed). Sequence type 1193 has recently emerged as a new, virulent and resistant lineage among fluoroquinolone resistant *E coli.* Escherichia coli ST131 is a globally dominant multidrug resistant clone associated with high rates of rUTI. Uropathogenic *E coli* (UPEC) are the primary cause of urinary tract infection (UTI), being responsible of ~90% of all cases. UPEC strains largely belong to the *E coli* phylogenetic groups B2 or D and are often clonal, with the most common sequence types (STs) isolated worldwide being ST69, ST73, ST95 and ST1312. The recently emerged and globally disseminated ST131 clone is a major contributor to hospital- and community-acquired UTI, as well as bloodstream infections and infections in companion animals and poultry. Originally identified in 2008, ST131 is associated with the worldwide spread of the CTX-M-15 extended spectrum β-lactamase (ESBL) resistance gene. Most ST131 strains are now strongly associated with multidrug resistance (MDR), including resistance to fluoroquinolones. Recent reports have also identified strains that are resistant to last-line carbapenems.

Significantly, we included clinical samples from patients that went on to develop *E coli* bacteraemia (despite pre-treatment with FQ/TMP-SMX). The composition could kill or reduce growth of *E coli* strains in these samples (and this is indicative of the possibility of using the composition for prophylaxis of bacteraemia in subjects). This included strains of the following Multi-Locus Sequence Typing (MLST) types (see Figure 2): ST648, ST315, ST405, ST361, ST88, ST453, ST1193 and ST131.

In addition to the B2 group, we surprisingly also were able to successfully kill or inhibit the growth of multiple strains of *E coli* phylo groups B1, D, F and G.

### References

- Shifu Chen, Yanqing Zhou, Yaru Chen, Jia Gu; fastp: an ultra-fast all-in-one FASTQ preprocessor, Bioinformatics, Volume 34, Issue 17, 1 September 2018, Pages i884-i890, https://doi.org/10.1093/bioinformatics/bty560
- Alexandre Souvorov, Richa Agarwala and David J. Lipman. SKESA: strategic k-mer extension for scrupulous assemblies. Genome Biology 2018 19:153. doi.org/10.1186/s13059-018-1540-z
- Ondov, B.D., Treangen, T.J., Melsted, P. et al. Mash: fast genome and metagenome distance estimation using MinHash. Genome Biol 17, 132 (2016). https://doi.org/10.1186/s13059-016-0997-x
- Martin Simonsen, Thomas Mailund and Christian N. S. Pedersen. Rapid Neighbour Joining. Proceedings of the 8th Workshop in Algorithms in Bioinformatics (WABI), LNBI 5251, 113-122, Springer Verlag, October 2008. doi:10.1007/978-3-540-87361-7_10
- Letunic I and Bork P (2021) Nucleic Acids Res doi: 10.1093/nar/gkab301 Interactive Tree Of Life (iTOL) v5: an online tool for phylogenetic tree display and annotation
- Michael J. Satlin et al, Colonization with Fluoroquinolone-resistant Enterobacterales decreases the effectiveness of fluoroquinolone prophylaxis in hematopoietic cell transplant recipients. Clinical Infectious Diseases 2021.
- Michael J. Satlin et al, Colonization with Levofloxacin-resistant extended-spectrum β-lactamase-producing Enterobacteriaceae and risk of bacteremia in hematopoietic cell transplant recipients. Clinical Infectious Diseases 2018.

### EXAMPLE 2: Phage-derived CRISPR therapeutic designed to reduce E. coli in hematological cancer patients

### ABSTRACT

Patients with hematological malignancies frequently develop bloodstream infections due to translocation of E. *coli* and other bacteria from the gut. Antibiotic treatment to prevent these infections has detrimental effects on the microbiome and immune tonus and is further hampered by increasing antibiotic resistance, in particular towards fluroquinolones. We screened a library of 162 wild-type (WT) phages identifying 8 phages with broad coverage of E. *coli.* Selected phages were engineered with novel tail fibers and a CRISPR-Cas machinery targeting clinically relevant E. *coli.* Engineered phages target bacteria in biofilms, reduce the emergence of phage-tolerant *E. coli* and out-compete their ancestral WT phages in co-culture experiments. SNIPR001 comprises four engineered bacteriophages with broad host-range across the E. *coli* phylogeny, including multi-drug resistant strains. SNIPR001 is well-tolerated in animals and reduces E. *coli* load in the murine gut better than its constituent components. SNIPR001 represents a novel CRISPR-Cas-therapeutic designed to selectively target E. *coli* which may cause fatal infections in hematological cancer patients.

### INTRODUCTION

Cancer treatment continues to advance and survival rates for people with hematological malignancies are increasing¹. However, the chemotherapeutic regimens that are frequently used in this immunocompromised population cause bone marrow suppression and gastrointestinal mucositis with associated increased intestinal permeability²⁻⁴. Translocation of gut bacteria, including E. *coli,* from the gastrointestinal tract is a frequent cause of bloodstream infections⁵.

The mortality related to bloodstream infections caused by enteric bacteria such as E. *coli* is 15-20%⁶; thus, antimicrobial prophylaxis is applied in people at risk of febrile neutropenia⁷. There are no approved therapies for the prevention of bloodstream infections in patients with hematological cancers, yet fluoroquinolones are used off-label in the United States based on two randomized trials that demonstrated that they decrease bacterial infections during neutropenia^{7,8,9}. Beyond the side effects of fluoroquinolones, including safety warnings and precautions, bacterial resistance is rising in oncology patients and approaching 60% in E. *coli* bloodstream infections in the USA¹⁰. In immunocompromised patients with hematological malignancies who develop chemotherapy-induced neutropenia, *E. coli* is responsible for 25.1-30% of all bacteremia cases^{11,12}. Moreover, up to 65% of E. *coli* isolated as the causative pathogen from bloodstream infections⁵ in patients with hematological cancers undergoing hematopoietic stem cell transplantation were resistant to fluoroquinolones¹³. Accordingly, novel narrow-spectrum prophylactic options that would also cover fluroquinolone resistant E. *coli* are needed to prevent infections in these vulnerable patients.

Bacteriophage therapy has been used prior to the broad availability of antibiotics¹⁴, but has now re-gained interest¹⁵ due to the rise in bacterial antimicrobial resistance combined with several successful individual case reports¹⁶⁻¹⁸. Still, few clinical trials with wild-type (WT) phages have been conducted¹⁹⁻²² and, although several have been directed towards E. *coli,* these have failed to produce convincing results in larger randomized controlled trials likely due to incomplete coverage of the target strains by the phage cocktail²³. Recent efforts have involved more extensive characterization of phages (n = 41) targeting *Klebsiella pneumoniae* strains *(n* = 17)²⁴ and phages *(n* = 248) targeting *Vibrio* strains *(n =* 294)²⁵ suggesting that better coverage of target strains can be achieved with large scale systematic screening. Synthetic biology has also been used to engineer T3 phage tailfibers augmenting the spectrum of strains targeted by the engineered phage²⁶. Finally, clustered regularly interspaced short palindromic repeats (CRISPR)-Cas systems can contribute to efficacy as a complementary killing modality to the lytic activity of the phage. CRISPR-associated nucleases (Cas) and a CRISPR-RNA form a complex, which, in some CRISPR-Cas systems, can bind to a homologous DNA target sequence and result in DNA degradation^{27,28}. Since prokaryotes lack error-prone non-homologous end-joining and rely solely on homologous recombination to repair DNA damage, they are prone to cell death following DNA degradation. This vulnerability has been exploited by using CRISPR-Cas as an antimicrobial modality for several bacteria, including *Staphylococcus aureus, E. coli* or *Clostridium difficile* ²⁹⁻³⁵.

To address the significant unmet medical need for new prophylactic agents for patients with hematological malignancies we report on the development of SNIPR001. Our research process for designing SNIPR001 includes several steps **(****Fig. 3****).** In short, a library (n = 162) of WT phages were tested *in vitro* on a panel of phylogenetically diverse *E. coli* strains representing the biology of the target bacterium E. *coli.* WT phages with the broadest and most complementary target strain coverage were selected for further engineering. Selected WT phages were subjected to both tail fiber engineering and CRISPR-Cas-arming to create a library of CRISPR-Cas-Armed Phages (CAPs). The CAP library was assessed for manufacturability, *in vitro* stability, spectrum of efficacy, *in vivo* pharmacokinetics, and efficacy. A combination of four CAPs was selected to create the development candidate SNIPR001, which has now entered clinical development (ClinicalTrials.gov ID NCT05277350).

### RESULTS

Wild-type lytic phages α15, α17, α20, α48 and α51 (all members of *Tevenvirinae)* were the starting point for phage engineering to produce synthetic phage types (CRISPR-armed phage; CAPs) *α15.2, a20.4, α48.4 or α51.5.* Tail fibre specificities for cognate *E coli* surface ligands TSX, LPS and LamB were as follows:-
α15.2 binds LPS and Tsx
α20.4 binds LPS and LamB
α48.4 binds Tsx
α51.5 binds Tsx

A cocktail comprising these phage types targeting LPS, LamB and Tsx was made (herein the cocktail termed SNR001).

### CRISPR-Cas-arming of phages to target E. coli

To CRISPR-Cas-arm the selected lytic phages and generate a library of CAPs, the type I-E CRISPR-Cas system of *E*. *coli³⁹* was engineered to target phylogenetically diverse *E*. *coli* strains. A CRISPR-Guided Vector (CGV^{™}) was generated, containing the *cas3* gene (*ygcB*) and a downstream cascade gene complex composed of *casA (ygcL, cas8e), casB (ygcK, cas11*)*, casC* (*ygcJ, cas7), casD* (*ygcI, cas5),* and *casE (ygcH, cas6),* and a CRISPR array targeting a plurality of different genes of the *E. coli* genome (CGV-EcCas). To evaluate the killing efficiency of the CRISPR-Cas system, the CGV-EcCas was conjugated to *E*. *coli* strain b52, showing an average reduction of 3.5 log₁₀ CFU/mL, compared to the empty vector. As expected, no effect was observed after conjugating the CGV-EcCas to a non-target *E. coli* strain. The killing efficiency of CGV-EcCas was further assessed on an abbreviated panel of 82 *E. coli* strains. Conjugative delivery of the empty vector was accomplished in 75% of the isolates. For all strains where the CGV-EcCas was delivered, bacterial counts were reduced below the limit of detection (LOD, 200 CFU/mL) corresponding to a reduction of 1-6 log₁₀, highlighting the potent CRISPR-Cas-mediated killing **(****Fig. 3A****).**

In addition to promoter P*_{bolA},* the CRISPR-Cas systems was engineered to express from a synthetic constitutively expressed *E. coli* promoter (P_{J23100}). CRISPR arrays were designed to target multiple virulence (spacer 1, 2 and 3) or essential genes (spacer 4 and 5) **(SEQ ID NOs: 6-10),** as targeting multiple regions has been shown to prevent resistance evolution⁴³. To confirm the CRISPR-Cas activity in the CAPs, we measured the *cas3* transcripts in samples obtained at 5-, 15- and 30-min following a synchronized infection with the equal MoI of CAP α15.2 in comparison to WT α15 using RT-qPCR and observed increasing levels of *cas3* RNA only upon CAP α15.2 infection. Next, we extended this assay to all four CAPs (α15.2, α20.4, α48.4, α51.5) and demonstrated increasing levels of *cas3* transcripts highlighting that the CAPs expressed the CRISPR-Cas system during infection of a target strain.

To demonstrate the competitive superiority of the CAPs, we performed competition experiments in which CAPs (α20.4 and α15.2) and their WT ancestral phages were co-cultured with *E*. *coli* strain b230, serving as target for both competing phages. Approximate initial ratios of 1 CAP to 9 WT phages were co-cultured and passaged four times on fresh target cells in liquid cultures. After each passage, the relative abundance of CAP and WT phage particles was evaluated. Both CAPs outcompeted their WTs within four rounds; CAP α20.4 reached 68% after four rounds, and CAP α15.2 reached 86% after two rounds **(****Fig. 3B-C****),** demonstrating an improved fitness compared to the WT phages.

### CAP cocktail SNIPR001

The activity of CAPs was tested against the *E*. *coli* panel *(n* = 429) using the growth kinetics assay. To maximize our coverage, we combined CAPs, resulting in a composition (SNR001) comprising phage types α15.2, α20.4, α48.4, and α51.5.

The ancestors of CAPs α15.2, α20.4, α48.4, and α51.5 are classified under the *Tevenvirinae* subfamily. Specifically, ancestors α15, α48, α20 and α51 have sequence similarity to *E. coli* phage T2, T4 and RB69 phages as shown below (as determined by Mash analysis). *In silico* analyses of the genomes of SNIPR001 showed that the CAPs encode no known transposase or integrase genes, indicating that the phages are not temperate, and thus not predicted to be capable of inserting their DNA in bacterial cells.

### Similarity Matrix:

| | **T2** | **T4** | **RB69** |
|---|---|---|---|
| **a15** | 96.56% | 96.41% | 82.60% |
| **a20** | 82.60% | 83.20% | 96.70% |
| **a48** | 96.74% | 96.57% | 81.82% |
| **a51** | 96.23% | 96.13% | 81.86% |

This similarity was calculated wherein the distance between genomes was calculated using Mash (v 1.1) with a k-mer size of 21 and a sketch size of 10 000.

See Ondov, B.D., Treangen, T.J., Melsted, P. et al., "Mash: fast genome and metagenome distance estimation using MinHash", Genome Biol 17, 132 (2016). https://doi.org/10.1186/s13059-016-0997-x for further discussion of Mash

### SNIPR001 does not affect other gut associated bacteria

Ideally, a phage-based therapy should not disturb the non-targeted genera of the microbiome, thus the specificity of SNIPR001 towards E. *coli* was assessed by investigating its effects on a panel of strain which includes non-E. *coli* species which are *E*. *coli* relatives, as well as a range of families associated with the commensal bacterial community in the gut bacteria (and *E*. *coli* as a positive control). The bacteria were cultured without CAPs, with the SNIPR001 cocktail, or with individual SNIPR001 CAPs *(n* = 4). The growth in CFU/mL was evaluated over a 4-hour period (DCFU/mL₄ₕ₋₀ₕ). In parallel, *E*. *coli* b2480 was grown under the same conditions as a positive control (Fig. 4). We observed no significant effect (p>0.05, Student's t-test, FDR-corrected with Holm's method) of the SNIPR001 cocktail or any of the SNIPR001 CAPs on non-E. *coli* strains, while the growth of *E*. *coli* was significantly inhibited (p<0.05, Student's t-test, FDR-corrected with Holm's method). Thus, SNIPR001 is not expected to impact the gut microbiome beyond the target *E. coli.*

### In vitro host-range of SNIPR001 in clinical target population

To understand the potential effect in strains relevant to hematological cancer patients the coverage of SNIPR001 was tested against our internal *E*. *coli* panel (429 strains) and a set of 382 clinical *E*. *coli* strains (JMI Laboratories, North Liberty, IA, USA). These JMI strains originated from patients with bloodstream infections hospitalized in hemato-oncology units across four different regions from 2018-2020 (Asia-Pacific 54 isolates, Europe 161 isolates, Latin America 26 isolates, and North America 141 isolates). The genotypic distribution of *E*. *coli* strains in the patient population was determined using whole genome sequencing and was found to be diverse, representing nine phylogroups and 118 multi-locus sequence types (MLSTs) (Fig. 5A). Using a spotting assay, we recorded phage infectivity against the panel of JMI strains. Visible single plaques were differentiated from lysis zones in cases where single plaques could not be verified. All spotting assays were run in duplicates. We observed overall coverages of 90.4 ± 1.6% of SNIPR001 in the 382 JMI *E*. *coli* panel, and of 95.6 ± 0.3% of SNIPR001 on the internal *E*. *coli* panel (429 strains). Furthermore, we observed plaques in 53.1 ± 7.7% and lysis zones in 37.3 ± 6.1% of the JMI panel strains, and similarly plaques in 60.5 ± 6.6% and lysis zones in 35.1 ± 6.3% of the internal panel strains (Fig. 5B). SNIPR001 showed 100% coverage in the B2 phylogroup, representing 53% of the JMI panel. This phylogroup is correlated with multi-drug resistance and virulence. Additionally, we observed that SNIPR001 covered 91.7% *(n* = 55) of strains classified as multi-drug resistant 100% *(n* = 5) of carbapenem-resistant strains, 92.2% *(n* = 95) of extended-spectrum β-lactamases producing strains, and 88.9% (n = 176) of strains that are resistant to fluroquinolones, such as ciprofloxacin and levofloxacin **(****Fig**. **5C****).**

Finally, we validated SNIPR001 on a clinical panel *(n =* 72) of fluroquinolones-resistant *E. coli* strains that were isolated from either a fecal sample or a perianal swab from hematological cancer patients. This population represents the expected clinical target patient population being pursued (SNIPR001 has been designated fast-track status by the FDA). A subset of these strains gave rise to a bloodstream infection (Fig. 5D). 82% of the *E*. *coli* strains *(n =* 72) were susceptible to at least two or more of the CAPs in SNIPR001, and 93% of the strains were susceptible to the whole SNIPR001 cocktail (Fig. 5E). This data demonstrates the benefit of SNIPR001 compared to the individual CAPs with regards to improving the spectrum of efficacy.

### Tolerability and gastrointestinal recovery of SNIPR001 in minipigs

The tolerability and gastrointestinal recovery of SNIPR001 were evaluated in Göttingen minipigs. Blood and feces were sampled over 7 days following oral administration of 2 x 10¹² PFU of SNIPR001 or vehicle. No CAPs were recovered from plasma, indicating no systemic exposure, while CAPs were recovered in the feces up to 7 days after SNIPR001 administration with a peak of 2 x 10⁷ PFU 24h post-dosing (Fig. 6A). The minipigs exhibited no clinical signs and no significant changes were observed in hematology or biochemistry parameters, in particular, no changes were seen in any immune cells, compared to vehicle treatment, supporting that SNIPR001 was well tolerated. Similar recoveries were obtained with the individual CAPs (Fig. 6B). In conclusion, SNIPR001 appears to be well tolerated in Göttingen minipigs with gastrointestinal recovery.

### Efficacy of SNIPR001 and constituent CAPs in a mouse colonization model

To assess the *in vivo* efficacy of the four selected CAPs in reducing *E. coli,* we adapted a mouse gut colonization model from Galtier et al.⁴⁴ for *E. coli* strain b17. Streptomycin was administered for 3 days to reduce Gram-negative bacteria from the mouse gastrointestinal tract, after which streptomycin administration was stopped and animals were inoculated once perorally with E. *coli* b17 (1 x 10⁷ CFU). This allowed stable colonization for 3 to 4 days. Aiming at assessing the efficacy of CAPs on an established colonization, treatment was started 2 days after inoculation and the study was terminated on day 4 after inoculation, as the colonization starts to drop. To ensure maximum exposure of CAPs, mice were treated with 3 daily doses, administered 8h apart, for a total of 6 doses over 2 days.

Mice were treated by oral gavage with a high, medium, or low dose (2 x 10¹¹ PFU, 2 x 10⁹ PFU, 1 x 10⁷ PFU, respectively) of SNIPR001, vehicle (negative control), or gentamicin (positive control). CAP recovery in the feces ranged from 3 x 10⁷ PFU/g in the low dose to 1 x 10¹⁰ PFU/g in the high dose, confirming successful GI passage (Fig. 6C). These levels of CAPs were associated with a significant (p<0.05, Mann-Whitney U test, FDR corrected) dose-dependent reduction in the target *E. coli* population compared to vehicle treated mice, after 24h of treatment (Day 3). At the high dose, SNIPR001 led to a 4 log₁₀ CFU/g reduction (Fig. 6D). Despite an increased variability in bacterial recovery on day 4, possibly due to clearance of the colonizing strain as illustrated in the vehicle group, similar reductions were observed after 2 days of treatment (Day 4). While the medium dose did not reach statistical significance (p<0.05, Mann-Whitney U test), there was nevertheless a numerical reduction in comparison to the vehicle group. Subsequently, the efficacies of the individual CAPs were compared to the SNIPR001 cocktail in this model. In this experiment a greater reduction in the colonization of the target strain was observed with SNIPR001 compared to any single CAP (which showed a numerical, but not statistically significant reduction) highlighting a benefit in efficacy from the combination (Fig. 6E). We also assayed the resistance profile of randomly sampled surviving bacteria and found no isolates that were resistant to the SNIPR001 cocktail. Overall, this data demonstrates the ability of SNIPR001 to decrease the target *E. coli* in the GI tract of colonized mice.

### DISCUSSION

Here we describe the development of SNIPR001 designed to target gut *E. coli* that frequently translocate in the bloodstream to cause bloodstream infections in patients with hematological cancers who are neutropenic. While fluoroquinolones are being used off-label, these patients continue to have a high morbidity and mortality. The use of traditional antibiotics has yielded important health benefits over the past century. However, in parallel we are now experiencing significant bacterial resistance development, and the number of deaths attributable to bacterial antimicrobial resistance in 2019 has been estimated to 1.27 million, with E. *coli* being the leading pathogen⁴⁵. Accordingly, new antibiotic modalities are needed to address both the unmet medical need of antimicrobial resistant infections in this vulnerable population. In this study we describe the development of SNIPR001 a novel development candidate with the potential to address these challenges.

Confirmation of the efficacy of SNIPR001 on a large and clinically relevant strain panels supports the clinical potential of the SNIPR001. The observed 4 log₁₀ reduction of E. *coli* in our *in vivo* model is a clear improvement over the previous studies⁴⁹⁵⁰.

SNIPR001 is an orthogonal antimicrobial approach as it has shown activity in multi-drug resistant strains. In addition, there is emerging evidence that maintaining a normal microbiome is important for upholding immunological tonus and potentially benefiting the outcome of oncology treatments⁵¹, and this has also been recognized in the most recent guidance on prophylactic management of patients at risk of febrile neutropenia⁷. In this context *in vitro* studies with SNIPR001 have shown specificity towards E. *coli* with no off-target effects toward any of the tested non-E. *coli* strains, thereby having a less detrimental effect on the microbiome. In the future, individualized combinations of narrow-spectrum antibiotics such as SNIPR001 may be used first line rather than use in addition to broad-spectrum antibiotics such as fluoroquinolones.

A clinical study to evaluate the ability of SNIPR001 to ascertain safety and its ability to reduce *E. coli* in the gut without perturbing the overall gut microbiome is currently ongoing in the US (NCT05277350). We believe that SNIPR001 exemplifies a potentially significant therapeutic advance in the field of antimicrobials for high-risk patient populations and can serve as a blueprint for narrow-spectrum therapies for other life-threatening antimicrobial resistant pathogens in high-risk patient populations.

**Table 1a: Overview of the four CAPs making up SNIPR001. The E. coli genes targeted by the five individual spacers and the sequence are listed in Table 1b.**

| |
|---|
| α15.2 comprises spacers 1, 2, 3 |
| α20.4 comprises spacers 4, 5 |
| α48.4 comprises spacers 4, 5 |
| α51.5 comprises spacers 4, 5 |

**Table 1b: The E. coli genes targeted by the five individual spacers comprised by SNR001 CAPs, and the spacer sequences used in the CAPs.**

| spacer | Target gene | Spacer Sequence |
|---|---|---|
| 1 | *bolA* | AGTGGGAAGGGTTGCAGGACACCGTCTTTGCC |
| 2 | *rpoH* | CCGATGTTACCTTCCTGAATCAAATCCGCCTG |
| 3 | *fimH* | CGAATGACCAGGCATTTACCGACCAGCCCATC |
| 4 | *lptA* | TGATTGACGGCTACGGTAAACCGGCAACGTTC |
| 5 | *murA* | GCTGTTAACGTACGTACCGCGCCGCATCCGGC |

### METHODS

Phage isolation was carried out by using *E. coli* strain panels. In brief, 100 µL of overnight cultures of each E. *coli* strain were mixed with 100 µL of each phage cocktail or wastewater samples. Following 6 minutes incubation at RT (in this period infection should occur), 3 mL of pre-warmed top agar containing Ca²⁺ were added to the *E. coli*/*phage* or wastewater mixtures and poured immediately on a LB plate. Alternatively, 10-fold dilutions of each cocktail were spotted on lawns prepared with isolation strains. After drying, plates were incubated at 37 °C overnight. Plaques were picked from each plate and resuspended in 500 µL of SM buffer, vortexed and stored at 4 °C. Ten-fold dilutions were spotted on the isolation strain which the plaque was originally picked from. To increase the likelihood of obtaining plaques corresponding to single phages, the procedure was repeated at least three times. Lysates were prepared from single plaques picked at the previous round of propagation, DNA was extracted, and their genomes were sequenced.

### E. coli panels and isolation procedures

Three *E. coli* panels, one in-house panel and two clinically relevant panels were included in this study. The in-house panel consisted of 429 phylogenetically diverse E. *coli* strains, isolated from the blood of patients with bloodstream infections and urinary tract infections, from feces of humans with no known disease, from animals and the environment. The strains cover seven different phylogroups (A, B1, B2, C, D, E, F), 114 multi-locus sequence typing (MLST) groups, serotypes (K- and O-type), antibiotic resistance profiles, and different geographical locations of isolation.

The JMI panel comprises of 382 strain E. *coli* clinical collection obtained from JMI Laboratories (North Liberty, IA, USA). These strains were isolated from patients with bloodstream infections hospitalized in hematology and oncology units across four different regions (Asia-Pacific 54 isolates, Europe 161 isolates, Latin America 26 isolates, and North America 141 isolates), sourced through the SENTRY Antimicrobial Surveillance Program (2018-2020), which is composed of a network of more than 150 medical centers in more than 28 countries worldwide (https://www.jmilabs.com/sentry-surveillance-program).

Finally, the panel comprising of 72 fluoroquinolone-resistant *E. coli* strains are isolated from either fecal samples or perianal swabs of hematological cancer patients hospitalized for hematopoietic cell transplantation^{53,54}.

*E. coli* strains were cultivated at 37 °C in lysogeny broth (LB) at 250 rpm in liquid media or on agar plates containing 1.5% (w/v) agar. When necessary, cultures were supplemented with ampicillin (100 µg/mL), kanamycin (50 µg/mL), gentamicin (15 µg/mL) or amikacin (50 µg/mL). All media for the growth of conjugation donor E. *coli* JKE201⁵⁵ and its derivatives were supplemented with 1,6-diaminopimelic acid (DAP) (80 µg/mL) to complement their auxotrophy.

Both *E. coli* strain b52, which was used to produce α15.2, α48.4 and α51.5, and *E. coli* strain b2479, which was selected to produce α20.4, belong to phylogroup A. Strain *E*. *coli* b17 was used as colonizing strain in the *in vivo* efficacy models as the strain is susceptible to all SNIPR001 CAPs and is part of the SNIPR Biome strain bank.

### Phage screening by growth kinetics

*In vitro* susceptibility of the intin-house *E*. *coli* panel *(n* = 429) to the 162 WT phages was evaluated using a growth kinetics assay. The assay measures the metabolic activity of a bacteria by tracking the reduction of a tetrazolium dye to a purple compound that aggregates during bacterial growth. The colorimetric reading was recorded every 15 min over a 24-h period by using the OmniLog^{®} (Biolog, Hayward, CA, USA) - adapted from Henry *et al.,* 2012⁵⁶. The inhibitory area under the curve (iAUC) was calculated from the kinetic curves over the course of the experiment and was defined as the ratio between the normalized AUC of the phage-treated bacterial growth curve and the bacteria-only control. Susceptibility was defined at iAUC values ≥0.2.

### Calculation of bacterial growth inhibition using iAUC

The growth inhibitory effect of SNIPR001 was determined using growth kinetic curves constructed using the OmniLog^{®} apparatus. To limit technical variability in measurement between timepoints, a cubic smoothing spline function was applied to the data in Scala using the "umontreal.ssj.functionfit" package. To identify appropriate ρ and weight variables, every combination of ρ and weight 0.1 and 0.5 was applied in 0.1 increments (i.e., 0.1, 0.2, ... 0.5). The spline with the lowest mean absolute error was chosen for AUC calculation. The initial cumulative amount of fluorescent dye at the initial timepoint varies slightly from well to well, leading to an artificial inflation of the AUC of certain wells. Using the best smoothed square spline, the mean signal for the first 1.5 h, prior to any measurable growth, was removed from all growth curves to approximate a zero-growth signal intercept. The total incremental AUC (iAUC) was calculated as the sum of the Riemann midpoint sums for each timepoint along the smoothed square spline. Lastly, we calculated the iAUC as iAUC=1-AUC_{Sample}/AUC_{Control}, where AUC_{Sample} is the AUC of the spline created by a given bacteria and SNIPR001, while AUC_{Control} refers to the AUC of the spline created with a given bacteria without a given phage or CAP, or a combination of those. Thus, iAUC values usually lie between 0 and 1, where 0 indicates no growth inhibition and 1 indicates complete growth inhibition. Some biological and technical noise does result in iAUC values outside these bounds on occasion but is considered negligible.

Host-range was calculated as the fraction of a panel that had an iAUC<0.2 for each repeat. Reported standard deviations were calculated as the deviance in the number of strains with an iAUC<0.2, and then normalized to the size of the panel, by dividing the standard deviation with the size of the panel.

### CRISPR-Cas-arming phages to target E. coli

Phages were CRISPR-Cas-armed by using homologous recombination. We inserted the payload in the region immediately after *gene* 49 and to *gene E* when compared to a reference wild-type T2 phage. Thus, the the synthetic phage genomes comprised the insertion between coordinates 9000 and 21000, wherein the coordinates are the nucleotide positions counted from the nucleotide (coordinate number 1) immediately after *gene* 49 towards *gene E* when compared to a reference wild-type T2 phage. Recombination was carried out in bacterial cells during phage propagation. Cells carried a plasmid that served as a recombination template. Recombination template plasmids carried the sequences that were aimed to be inserted into the phage genome between ~200-700 bp flanking sequences that were homologous to the phage sequences at the insertion site. For each phage, we inserted the Type-I-E CRISPR-Cas system endogenous to *E. coli* (Genbank CP032679.1), i.e., the *cas3* gene (*ygcB*) and the downstream genes encoding the cascade complex, *casA (ygcL), casB (ygcK), casC (ygcJ), casD (ygcl)* and *casE (ygcH),* as well as a CRISPR array targeting selected *E. coli* sequences. For all CAPs selected, the *cas* genes originating from *E. coli* are identical. Insertion of the CRISPR-Cas system resulted in deletion of ~7 kbp deletion of phage DNA in the *gene 49 - gene E.* The sequences of the resulting CAPs were verified by NGS (BaseClear, Leiden, The Netherlands).

### Transduction of CGVs in biofilms

*E. coli* b52 cells were grown in 96-well plates, and biofilms were allowed to develop on peg lids. Each well contained 180 µL M9 medium (Sigma, M6030) supplemented with 20 mM Glucose, 2 mM MgSO₄, 0.1 mM CaCl₂, 0.1% Amicase (Sigma), and 0.1% mannitol. Wells were inoculated with 1 µL of overnight b52 culture. The peg lid was inserted, and the microtiter plate was incubated static for 24 hours at 37 °C. Next, the peg lid was transferred to a new plate with fresh media without washing, and the plate was incubated for an additional 24 hours. After incubation, a new plate was prepared with 100 µL media and 100 µL of CGV transducing particles (~10⁸ particles) in each well (3 replicates). Biofilms grown on the pegs were rinsed 3 times in sterile H₂0 (200 µl) before transferring them to the new plate. The plate was incubated static for 5 hours at 37 °C.

To assay the metabolic activity of cells in the biofilms, lids were rinsed three times in sterile H₂0 (200 µL) before placing them in a plate with 20 µL Alamarblue stain (ThermoFisher) and 180 µl media in each well. Plates were incubated for 1.5 hours at 37 °C and moved to a microplate reader (Synergy H1, Biotek). Fluorescence (excitation: 560 nm; emission 590 nm) and absorbance (600 nm) was recorded for each well.

The metabolic activities of the biofilms treated with CGVs carrying the promoter **P***_{bolA}* were reported relative to the metabolic activities of biofilms treated with a CGV not carrying a promoter transcribing the *cas* genes.

### Plasmid and strain construction

Plasmids were constructed by InFusion HD cloning using PCR-generated DNA fragments. To construct CGV-EcCas, *cas3* and cascade genes from *E. coli* were amplified and cloned into a ColE1-type plasmid, pZE21⁵⁷. Additionally, a 3-spacer array targeting genes in *E*. *coli* was included in the vector under the control of constitutive promoter J23100. The array contains nucleotides from the genome of *E*. *coli* per target locus separated by direct repeats (repeat sequence, SEQ ID NO: 15). The protospacer adjacent motif (PAM) is located adjacent to the selected target sequences in the genome of *E*. *coli.*

### Transformation assays

Overnight cultures were diluted (1:100) in fresh LB medium and grown to mid-exponential phase (OD₆₀₀ ≈ 0.6). Subsequently, cells were prepared for electroporation and concentrated 50-fold in ice-cold MilliQ water. Cells were then electroporated with appropriate plasmids, allowed to recover for 1 h at 37 °C in super optimal broth (SOB), and plated on LB plates supplemented with antibiotics.

### Conjugation assays

Conjugation experiments assessing the transfer and killing efficiency of CGV-EcCas were established using *E. coli* JKE201 as the donor and *E. coli* clinical isolates as recipients (including target and non-target and *E*. *coli* strains as controls). Plasmids were conjugated into *E*. *coli* recipients by liquid mating. Briefly, overnight cultures were diluted (1:100) in fresh LB medium, grown to OD₆₀₀ ≈ 0.4, washed, and suspended in fresh LB to OD₆₀₀ ≈ 0.25. 125 µl of donor and 25 µl of recipient cell suspensions were mixed for 5:1 mating in a 96-well microplate and incubated for 16 h at 37°C. The conjugation efficiency was determined by plating a dilution series of conjugation reactions onto LB agar supplemented with antibiotics (to select for the transconjugants). The specific killing efficiency was quantified by plating 90 µL of the conjugation reactions on selective plates. The CGV-EcCas plasmid encodes kanamycin, gentamycin, and amikacin resistance to enable selection for transconjugants. Viability was calculated by counting CFUs on the plates, and data were recorded as viable cell concentration (CFU/mL).

### Synchronized CAP infection and cas3 expression assay

An overnight culture of the test strain in LB was 100-fold diluted and incubated to stationary phase in LB at 37 °C with shaking, and 10-mL aliquots were subsequently separated into 50-mL falcon tubes. Each aliquot was then seeded with 50 µL of high-titer lysate of the individual CAPs, and incubation was continued under the same conditions. Additionally, a mock 10 mL LB volume for each CAPs were also seeded with 50 µL of CAP lysates and used for 0 min phage enumeration. At 5 min, 15 min and 30 min post-seeding, aliquots were collected for total RNA extraction and phage enumeration. Phage enumeration aliquots were syringe filtered (0.2 µm, Sartorious, Göttingen, Germany) and subjected to an efficiency of plating assay. For total RNA extraction, 1 mL aliquots of individual cultures were centrifuged at 13.3k × *g* using a table-top centrifuge for 15s, and supernatants were discarded. Then, pellets were immediately resuspended in cold RNA Later (Thermo Fischer Scientific, AM7020) and stored at -20 °C until extraction. Total RNA was extracted using a GeneElute Total RNA kit (Sigma-Aldrich, St. Louis, MO, USA) following the manufacture's protocol for extraction of RNA from bacteria. After first elution, 1 µL of Dnase I (1U/µL) was added and incubated overnight at 37°C. The reaction was terminated by incubation at 70 °C for 15 min. The RNA was re-purified on a GeneElute column and eluted in 35 µL of kit elution buffer. Total RNA concentration was estimated on a NanoDrop instrument (Thermo Scientific, One/OneC), and 0.5 to 2 µg of RNA was added to a cDNA synthesis reaction containing SuperScriptIII RT enzyme (Thermo Fisher, Waltham, MA, USA) and random decamers to prime synthesis in a 20-µL reaction volume. The cDNA reaction was diluted to 100 µL in water. Real-Time PCR was conducted in triplicate using 5 µL of cDNA as template, 10 µL of Power SYBR Green PCR Master mix (Thermo Fisher) and 0.2 µM of each PCR primer. PCRs were performed on an AB QuantStudio5 system (Applied Biosystems, Foster City, CA, USA) using the standard two-step thermocycling protocol for Power SYBR Green PCR Master Mix with 60 °C annealing/extension. The forward and reverse primers for *gapA* (reference gene) were 5'-cgctaacttcgacaaatatgctggc-3' (SEQ ID NO: 17), and 5'-aggacgggatgatgttctgggaa-3' (SEQ ID NO: 18), and for *cas3* were 5'-caagtatgctaccaacggctaaag-3' (SEQ ID NO: 19) and 5'- ccaatcaaaatcaacgtcgagtga-3' (SEQ ID NO: 20). Single PCR products were confirmed for these primer pairs by melting curve analysis. Relative levels of transcripts were estimated using 10-fold dilutions of purified PCR products as standards, and values were expressed as the ratio of *cas3* to *gapA* transcripts.

### Phage competition assay

Lysates of the two phages were mixed at 9:1 (WT:CAP) ratio and the phage mixtures were added to 10 ml 2xYT medium containing 10 mM CaCl₂ and 20 mM MgCl₂, and 100 µL overnight of *E*. *coli* strain b230, serving as target for both competing phages. After 2h incubation in a 37 °C shaking incubator, the cultures were centrifuged and 1 µL of the supernatant was added to a new b230 culture. The same steps were repeated twice.

The ratio of phages was assessed by PCR with three primers, resulting in two specific products, one for the WT phage and one for the CAP. PCR products were separated on a 1% agarose gel and DNA bands were stained by SYBRsafe and visualized and quantified by the ChemiDoc XRS+ System (Model 1708265, Biorad). The background-corrected intensity of the band corresponding to the WT phage was divided by the intensity of the band corresponding to the CAP in the same lane, to obtain the ratio of the two band intensities (WT/CAP). The fraction of CAP compared to the total phage content (WT+CAP) were determined based on the calibration curve, which was made by using a set of different mixtures of the two phages, and fitting a curve to the measured band intensity ratios (WT/CAP). The estimated error of the reported values is less than 20%.

### Lawn killing assay

An overnight culture of the test strain in LB was adjusted to 10⁹ CFU/mL. Hundred µL aliquots of CFU/mL adjusted strain was mixed with 100 µL of 10⁹ PFU/mL to achieve a multiplicity of infection of 1 of either CAP α15.2 or WT α15 in 15 mL falcon tubes, mixed with 3 mL of molten and pre-tempered top agar and spread on LB plates. After lawns were solidified, plates were incubated at 37 °C overnight, and the total number of surviving colonies were counted for CAP α15.2 or WT α15 groups the next day. Assays were performed as independent biological duplicates where each experiment comprised often technical replicates. Statistical significance was established using both replicates using a Mann-Whitney U test.

### Generalized transduction assay

The transduction ability of each CAP was evaluated via the generalized transduction assay. Briefly, transducing lysates were prepared by propagating each CAP on *E*. *coli* MG1655 *lamB::Cm.* This strain was modified from the WT MG1655 (Catalog number 700926, American Type Culture Collection, Manassas, VA, USA) to carry a chloramphenicol selection marker. Experiments were conducted in parallel with the well characterized lytic T4 phage (negative control), and its transducing mutant T4GT7⁵⁸ (positive control). Following this step, the WT *E*. *coli* MG1655 strain was infected at an OD₆₀₀ of 0.3 with each transducing lysate at a MoI of 0.5, 0.1 and 0.01, and spread on LB plates containing chloramphenicol. Next day, the number of transductant colonies were recorded for each CAP, controls and different MOIs. The frequency of transduction was calculated as the number of transductants divided by the titer of the transducing lysate.

### Sequence analysis of CAPs

Sequences of the individual SNIPR001 CAPs were analyzed for the presence of antibiotic resistance, virulence genes and lysogeny associates genes (transposases and integrases) using databases **(Table 2).** Furthermore, phage samples were analyzed using whole genome sequencing. This typically results in >1000x coverage of the whole phage genome. Assemblies are constructed by down-sampling the data to 1000x average coverage for the phage and assemble using SKESA. To detect differences between samples and to detect non-majority mutations the raw reads were mapped back to the assembly using BWA (version 0.7.17).

**Table 2: List of databases used for the analysis of SNIPR001 CAP sequences**

| **Database** | **Source** | **Analysis** |
|---|---|---|
| ResFinder 4.1 | https://cge.cbs.dtu.dk/service s/ResFinder | Identification of acquired genes and/or chromosomal mutations mediating antimicrobial resistance |
| VirulenceFinder-2.0 | https://cge.cbs.dtu.dk/service s/VirulenceFinder/ | Detection of virulence genes including exotoxins |
| PHASTER Prophage/Virus DB | https://phaster.ca/ | Detection of potential transposases and integrases |
| | | |

### Phage specificity assay using liquid killing assay

SNIPR001 CAPs (α15.2, α20.4, α48.4 and α51.5) and SNIPR001 killing specificity was evaluated via a biopotency assay against a panel of human-relevant, aerobic (*n =* 6) and anaerobic (*n =* 3) bacterial strains. An *E. coli* strain b2480, was included as positive control for phage-mediated killing (Table 3).

In brief, overnight cultures were adjusted to 10⁶ CFU/mL in LB broth. SNIPR001 CAPs or SNIPR001 (in which each CAP was combined in equal ratio) were added at a MOI of 1 prior to incubation for 4 h. Untreated bacteria were cultured in parallel as controls for bacterial growth. CFU counts were recorded at 0 and 4 h post phage-treatment, and data are represented as Δlog₁₀ CFU/mL by subtracting the initial inoculum (0 h) from the assay end point CFU/mL (4 h).

**Table 3: Panel of bacterial strains (Aerobic: n = 6, Anaerobic: n = 3, Aerobic/Anaerobic: n = 1) tested via a biopotency assay, showing Gram-type classification, growth conditions and source/ID**

| Strain/name | Gram type | Growth condition | Source/ID |
|---|---|---|---|
| *Acinetobacter baylyi* | Negative | Aerobic | ATCC 33304 |
| *Klebsiella pneumonia* | Negative | Aerobic | SNIPR Biome ID b2951 |
| *Enterococcus faecalis* (Andrewes and Horder) Schleifer and Kipper-Balz | Positive | Aerobic | ATCC 47077 |
| *Streptococcus thermophilus* Orla-Jensen | Positive | Aerobic | ATCC 19258 |
| *Bacillus coagulans* Hammer | Positive | Aerobic | ATCC 7050 |
| *Staphylococcus aureus* subsp. *Aureus* Rosenbach | Positive | Aerobic | ATCC 12600 |
| *Eubacterium limosum* Eggerth | Positive | Anaerobic | ATCC 8486 |
| *Bacteroides vulgatus* Eggerth and Gagnon | Negative | Anaerobic | ATCC 8482 |
| *Bacteroides thetaiotaomicron* (Distaso) Castellani and Chalmers | Negative | Anaerobic | ATCC 29148 |
| *E. coli* | Negative | Aerobic/Anaerobi c | Takara Cat. #636763 |

### Spotting assay and efficiency of plating (EoP)

For counting of phage titers, phage lysates or the equal volume mix of SNIPR001 CAPs were serially diluted 10-fold in SM buffer or PBS, respectively. Bacterial lawns were prepared by adding 100 or 300 µL of bacterial overnight culture to 3 or 10 mL of 0.5% top agar (containing Ca²⁺ and Mg²⁺), which was vortexed briefly and poured onto a round or square LB plate. Five µl of the dilution series of test phages were then spotted onto lawns and left to dry at room temperature with an open lid prior to incubation at 37°C overnight. The strains b52, b2479 and b17 were used as controls of the assay and included in each round of assays.

The next day, results were assessed (Table 4). In this assay, a susceptible strain is defined as one producing plaques that are countable in PFU/mL as well as one without visible plaques but demonstrating impairment of bacterial growth (i.e., lysis zones). Coverage defines the percentage of the total number of susceptible strains. Images of all plates were recorded. Figures illustrating efficiency of plating results first had titers log₁₀ transformed and then standard deviances and averages were calculated subsequently. The clinical panels and control strains were tested in two independent experiments.

**Table 4 Criteria used to evaluate results of the spot assay and define strain susceptibility following standards^{46,59}**

| **Spot assay categories** | **Observation** | **Strain susceptibility to SNIPR001** |
|---|---|---|
| Plaques | Visible plaques were counted, and PFU/mL was calculated by multiplying with volume and dilution | Susceptible strain |
| Lysis zone | Impairment of bacterial growth observed as lysis zones. No plaques are visible; the highest dilution of visible zones is recorded | Susceptible strain |
| Negative | Neither plaques nor lysis zones are Jetected | Non-susceptible strain |

### Animals and housing

Mouse studies were performed with female CD-1^{®} IGS mice (approximately 6-7 weeks of age upon arrival) from Charles River (Freiburg, Germany). The animals were housed in groups of 3 to 5 mice per cage within a climate-controlled room (temperature 20-23°C; relative humidity 30-70%) under a 12:12 h light-dark cycle (illuminated 07:00-19:00). Standard pelleted chow and tap water were available *ad libitum.* Animals were allowed an acclimatization period of at least 7 d before the start of the experimental procedures. 30 female Göttingen minipigs (approximately 4-7 months of age upon arrival) from Ellegaard Göttingen minipigs A/S, Denmark was used for tolerability and kinetic studies. Animals were allowed an acclimatization period of at least 14 days before the start of experiments. Pigs were housed in groups of 2 to 3 animals and given standard pig diet twice daily and tap water was available *ad libitum.* All procedures were conducted in accordance with guidelines from the Danish Animal Experiments Inspectorate, Ministry of Environment and Food of Denmark and in accordance with the institutional license (BioAdvice, animal license no. 2015-15-0201-00540).

### Mouse gut colonization model

The mouse gut colonization model was adapted from *Galtier et al.* (2016)⁴⁴. Briefly, pre-treatment with streptomycin (5 g/L) in the drinking water was given 3 days prior inoculation with *E*. *coli* b17 to decrease the level of native bacteria. On day 0, an inoculum of 3 x 10⁷ CFU of *E*. *coli* b17 was prepared from a frozen glycerol stock and administered to all mice in 0.25mL by oral gavage.

Treatment was administered three times daily for 2 days starting 2 days after inoculation. Right before each administration, the 4 CAPs were mixed in a 1:1:1:1 ratio to form SNIPR001 at a high, medium, or low concentration resulting in dose levels of 2 x 10", 2 x 10⁹ and 1 x 10⁷ PFU. At the time of treatment, mice were administered 0.1 mL of 10% sodium bicarbonate by oral gavage followed by the oral administration of 0.3 mL of SNIPR001, saline (vehicle) or 43.5 mg/kg gentamicin.

### CAP recovery and tolerability studies

Göttingen minipigs were first given a cocktail of antibiotic comprising neomycin (60 mg/kg, orally, once daily for 4 days) and cefquinome (2 mg/kg, intramuscular once daily for 3 days) before SNIPR001 or single CAP administration to decrease the level of Gram-negative bacteria in the GI tract and therefore limiting phage replication. Animals were then fasted overnight and lightly sedated before administration of a single CAP, or SNIPR001 cocktail, once orally at 2 x 10¹² PFU in 100 mL, following an oral administration of 50 mL of 10% sodium bicarbonate. Fecal samples were collected daily for CAPs quantification by plaque assay. In addition, for the tolerability study, blood samples were collected for hematology and blood chemistry analysis, including C-reactive protein, and plaque assay. Animals were closely monitored following SNIPR001 administration, and their body temperature was recorded regularly.

### Quantification of E. coli b17 and CAPs in feces

Fecal samples were homogenized and serially diluted in SM buffer. Triplicates of 10 µl of each dilution were then spotted on McConkey agar plates (Sigma, M7408) supplemented with streptomycin (1 mg/mL) and incubated for 12 to 16h at 37 °C for *E*. *coli* enumeration.

Plaque assays were performed for enumeration of CAPs in feces samples. Briefly, homogenized samples were centrifuged at 10,000g for 10min, and the supernatant was serially diluted. Triplicates of 10 µl of each dilution were spotted on an *E*. *coli* b17 overlay and incubated for 12 to 16h at 37°C.

To quantify the presence of *in vivo* resistors, three colonies from each mice fecal sample in the medium dose group at 3 different time points were picked from the McConkey agar plates. Colonies were incubated for 12 to 16h at 37 °C in LB broth and used to make top agar overlays on LB agar plates. Then, plates were dried for 15 minutes in the LAF bench. The SNIPR001 cocktail, as well as the 4 individual CAPs, were spotted as a dilution series from 1 x 10⁵ PUF/mL stocks. As a control, a top agar overlay of colonization strain *E*. *coli* b17 was spotted in the same way. Plates were left to dry in the LAF bench with the lid on and subsequently incubated upside down for 12 to 16h at 37°C.

### Whole Genome Sequencing of E. coli strains from JMI

Total genomic DNA was extracted and purified using the KingFisher Cell and Tissue DNA kit (Thermo Scientific, Waltham, MA, USA) in a robotic KingFisher^{™} Flex Magnetic Particle Processor (Thermo Scientific) workstation.

Total genomic DNA was used as input material for library construction. DNA libraries were prepared using the Nextera XT^{™} library construction protocol and index kit (Illumina, San Diego, CA, USA) and sequenced on a MiSeq Sequencer (Illumina) using MiSeq Reagent Kits v3 (600 cycle).

### Resistance Phenotype Definitions

The extended-spectrum β-lactamase (ESBL)-phenotype was defined for *Escherichia coli* as a minimum inhibitory concentration (MIC) value ≥2 mg/L for ceftriaxone, ceftazidime and/or aztreonam (https://clsi.org/).

Carbapenem-resistant *Enterobacterales* (CRE) was defined as any isolate displaying imipenem, doripenem, and/or meropenem resistance with MIC > 2 mg/L (https://clsi.org/).

### Assembly of whole genome sequencing data

Raw sequencing reads were trimmed using Trimmomatic⁶⁰ (version 0.39) with the settings "LEADING:3 TRAILING:3 SLIDINGWINDOW:4:15 MINLEN:36". Trimmed reads were assembled using SPAdes⁶¹ (version 3.14.1) with default settings. Contigs shorter than 500 bp or with a sequencing depth below 2x were removed from the final assemblies.

### Comparative genomic methods for clinical E. coli strains

Multi-locus sequence typing (MLST) was performed using MLST2⁶² on the assembled genomes of the *E. coli* bacteria using default settings, with the MLST database downloaded on July 1, 2021, from the MLST2 repository (https://bitbucket.org/genomicepidemiology/mlst_db/src/master/). Phylogroup classification was conducted using ClermonTyping⁶³ on the assembled *E*. *coli* genomes using default settings. Distance matrices for phylogenetic tree construction were generated using MASH⁶⁴ with a k-mer size of 21 and 10,000 sketches per genome. Sketches were then compared to create the MASH-distance in a pairwise manner to create a distance matrix of *E*. *coli* genomes.

### Phage synteny analysis

To generate the synteny plot, wild type sequences of the four phages included in the final cocktail, plus the two closely related and well-known reference phages (RB69 AY303349.1, and T2 NC_054931.1) were annotated with RAST to extract predicted protein sequences. All protein sequences for each phage were queried again all other phage genomes using tblastn (v 2.12.0), with an E-value cutoff of 1e-10. The synteny plot was then generated using a custom Python script (see Data Availability), using the drawSvg library (v 1.9.0). The plot shows the phage genomes in order of similarity and displays all tblastn hits as synteny blocks shaded by their protein identity. The proteins of the two reference phages were manually classified as belonging to each of the functional groups "DNA metabolism", "Structure" or "Other", and colored accordingly.

### Data processing and visualization

Figures and key statistics were generated using R version 4.1.0. For figure generation the following packages were used: RcolorBrewer v. 1.1-2, ape v. 5.5, ggsignif v. 0.6.2, ggpubr v. 0.4.0, matrixStats 0.59, reshape2 v. 1.4.4, ggimage v. 0.3.0, here v. 1.0.1, purr v. 0.3.4, ggtree⁶⁵ v. 3.0.2, systemfonts v. 1.0.2, Cairo v. 1.5-12.2, cowplot v. 1.1.1, reaxxl v. 1.3.1, and ggplot2 v.3.3.3. Averages and standard deviations are calculated after transforming the values to the scale shown on a given figure, e.g. when a log₁₀ scale is used, the averages and standard deviations are calculated after log₁₀ transformation.

### DATA AVAILABILITY

Data and results that were generated during this study is deposited at https://github.com/sniprbiome/SNIPR001_paper. Phage genome sequences are deposited at Genbank under access numbers OQ067373 - 76

### CODE AVAILABILITY

All code needed to produce this study is available at https://github.com/sniprbiome/SNIPR001_paper.

### REFERENCES

1. Pulte, D., Jansen, L. & Brenner, H. Changes in long term survival after diagnosis with common hematologic malignancies in the early 21st century. Blood Cancer J 10, 56 (2020).
2. Riley, M. et al. Epidemiology and Burden of Mucosal Barrier Injury Laboratory-Confirmed Bloodstream Infections in Bone Marrow Transplant and Hematology-Oncology Units. Am J Infect Control 45, S38 (2017).
3. Dandoy, C. E. et al. Incidence, Risk Factors, and Outcomes of Patients Who Develop Mucosal Barrier Injury-Laboratory Confirmed Bloodstream Infections in the First 100 Days After Allogeneic Hematopoietic Stem Cell Transplant. Jama Netw Open 3, e1918668 (2020).
4. Velden, W. J. F. M., Herbers, A. H. E., Netea, M. G. & Blijlevens, N. M. A. Mucosal barrier injury, fever and infection in neutropenic patients with cancer: introducing the paradigm febrile mucositis. Brit J Haematol 167, 441-452 (2014).
5. Girmenia, C. et al. Incidence, Risk Factors and Outcome of Pre-engraftment Gram-Negative Bacteremia After Allogeneic and Autologous Hematopoietic Stem Cell Transplantation: An Italian Prospective Multicenter Survey. Clin Infect Dis 65, 1884-1896 (2017).
6. Klastersky, J. et al. Bacteraemia in febrile neutropenic cancer patients. Int J Antimicrob Ag 30, 51-59 (2007).
7. Taplitz, R. A. et al. Antimicrobial Prophylaxis for Adult Patients With Cancer-Related Immunosuppression: ASCO and IDSA Clinical Practice Guideline Update. J Clin Oncol 36, JCO.18.00374 (2018).
8. Cullen, M. et al. Antibacterial Prophylaxis after Chemotherapy for Solid Tumors and Lymphomas. New Engl J Med 353, 988-998 (2005).
9. Bucaneve, G. et al. Levofloxacin to Prevent Bacterial Infection in Patients with Cancer and Neutropenia. New Engl J Medicine 353, 977-987 (2005).
10. See, I., Freifeld, A. G. & Magill, S. S. Causative Organisms and Associated Antimicrobial Resistance in Healthcare-Associated, Central Line-Associated Bloodstream Infections From Oncology Settings, 2009-2012. Clin Infect Dis 62, 1203-1209 (2016).
11. Trecarichi, E. M. et al. Current epidemiology and antimicrobial resistance data for bacterial bloodstream infections in patients with hematologic malignancies: an Italian multicentre prospective survey. Clin Microbiol Infec 21, 337-343 (2015).
12. Marín, M. et al. Factors influencing mortality in neutropenic patients with haematologic malignancies or solid tumours with bloodstream infection. Clin Microbiol Infec 21, 583-590 (2015).
13. Averbuch, D. et al. Antimicrobial Resistance in Gram-Negative Rods Causing Bacteremia in Hematopoietic Stem Cell Transplant Recipients: Intercontinental Prospective Study of the Infectious Diseases Working Party of the European Bone Marrow Transplantation Group. Clin Infect Dis 65, 1819-1828 (2017).
14. Mi 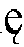 dzybrodzki, R. *et al.* Bacteriophages. 1-31 (2018) doi:10.1007/978-3-319-40598-8_31-1.
15. Mi 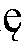 dzybrodzki, R. *et al.* Bacteriophages. 921-951 (2021) doi:10.1007/978-3-319-41986-2_31.
16. Schooley, R. T. et al. Development and Use of Personalized Bacteriophage-Based Therapeutic Cocktails To Treat a Patient with a Disseminated Resistant Acinetobacter baumannii Infection. Antimicrob Agents Ch 61, (2017).
17. Dedrick, R. M. et al. Engineered bacteriophages for treatment of a patient with a disseminated drug resistant Mycobacterium abscessus. Nat Med 25, 730-733 (2019).
18. Jennes, S. et al. Use of bacteriophages in the treatment of colistin-only-sensitive Pseudomonas aeruginosa septicaemia in a patient with acute kidney injury-a case report. Crit Care 21, 129 (2017).
19. Jault, P. et al. Efficacy and tolerability of a cocktail of bacteriophages to treat burn wounds infected by Pseudomonas aeruginosa (PhagoBurn): a randomised, controlled, double-blind phase 1/2 trial. Lancet Infect Dis 19, 35-45 (2018).
20. Sarker, S. A. et al. Oral Phage Therapy of Acute Bacterial Diarrhea With Two Coliphage Preparations: A Randomized Trial in Children From Bangladesh. Ebiomedicine 4, 124-137 (2016).
21. Leitner, L. et al. Intravesical bacteriophages for treating urinary tract infections in patients undergoing transurethral resection of the prostate: a randomised, placebo-controlled, double-blind clinical trial. Lancet Infect Dis 21, 427-436 (2020).
22. Sabino, J., Hirten, R. P. & Colombel, J. Review article: bacteriophages in gastroenterology-from biology to clinical applications. Aliment Pharm Therap 51, 53-63 (2020).
23. Górski, A., Borysowski, J. & Mi 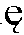 dzybrodzki, R. Phage Therapy: Towards a Successful Clinical Trial. *Antibiotics* 9, 827 (2020).
24. Federici, S. et al. Targeted suppression of human IBD-associated gut microbiota commensals by phage consortia for treatment of intestinal inflammation. Cell 185, 2879-2898.e24 (2022).
25. Kauffman, K. M. et al. Resolving the structure of phage-bacteria interactions in the context of natural diversity. Nat Commun 13, 372 (2022).
26. Yehl, K. et al. Engineering Phage Host-Range and Suppressing Bacterial Resistance through Phage Tail Fiber Mutagenesis. Cell 179, 459-469.e9 (2019).
27. Westra, E. R. et al. CRISPR Immunity Relies on the Consecutive Binding and Degradation of Negatively Supercoiled Invader DNA by Cascade and Cas3. Mol Cell 46, 595-605 (2012).
28. Sinkunas, T. et al. Cas3 is a single - stranded DNA nuclease and ATP - dependent helicase in the CRISPR/Cas immune system. Embo J 30, 1335-1342 (2011).
29. Bikard, D. et al. Development of sequence-specific antimicrobials based on programmable CRISPR-Cas nucleases. Nat Biotechnol 32, 1146-1150 (2014).
30. Citorik, R. J., Mimee, M. & Lu, T. K. Sequence-specific antimicrobials using efficiently delivered RNA-guided nucleases. Nat Biotechnol 32, 1141-1145 (2014).
31. Selle, K. et al. In Vivo Targeting of Clostridioides difficile Using Phage-Delivered CRISPR-Cas3 Antimicrobials. Mbio 11, e00019-20 (2020).
32. Neil, K. et al. High - efficiency delivery of CRISPR - Cas9 by engineered probiotics enables precise microbiome editing. Mol Syst Biol 17, e10335 (2021).
33. Edgar, R. & Qimron, U. The Escherichia coli CRISPR system protects from λ lysogenization, lysogens, and prophage induction. J Bacteriol 192, 6291-4 (2010).
34. Hsu, B. B. et al. In situ reprogramming of gut bacteria by oral delivery. Nat Commun 11, 5030 (2020).
35. Yosef, I., Manor, M., Kiro, R. & Qimron, U. Temperate and lytic bacteriophages programmed to sensitize and kill antibiotic-resistant bacteria. Proc National Acad Sci 112, 7267-7272 (2015).
36. Ochman, H. & Selander, R. K. Standard reference strains of Escherichia coli from natural populations. J Bacteriol 157, 690-3 (1984).
37. Oechslin, F. Resistance Development to Bacteriophages Occurring during Bacteriophage Therapy. Viruses 10, 351 (2018).
38. Dunne, M. et al. Salmonella Phage S16 Tail Fiber Adhesin Features a Rare Polyglycine Rich Domain for Host Recognition. Structure 26, 1573-1582.e4 (2018).
39. Brouns, S. J. J. et al. Small CRISPR RNAs Guide Antiviral Defense in Prokaryotes. Science 321, 960-964 (2008).
40. Knight, D. & Girling, K. Gut flora in health and disease. Lancet 361, 1831 (2003).
41. Christensen, S. K., Mikkelsen, M., Pedersen, K. & Gerdes, K. RelE, a global inhibitor of translation, is activated during nutritional stress. Proc National Acad Sci 98, 14328-14333 (2001).
42. Vieira, H. L. A., Freire, P. & Arraiano, C. M. Effect of Escherichia coli Morphogene bolA on Biofilms. Appl Environ Microb 70, 5682-5684 (2004).
43. Uribe, R. V. et al. Bacterial resistance to CRISPR-Cas antimicrobials. Sci Rep-uk 11, 17267 (2021).
44. Galtier, M. et al. Bacteriophages to reduce gut carriage of antibiotic resistant uropathogens with low impact on microbiota composition. Environ Microbiol 18, 2237-2245 (2016).
45. Antimicrobial Resistance Collaborators. Global burden of bacterial antimicrobial resistance in 2019: a systematic analysis. Lancet 399, 629-655 (2022).
46. Hyman, P. & Abedon, S. T. Bacteriophage host range and bacterial resistance. Adv Appl Microbiol 70, 217-48 (2010).
47. Lemay, M.-L., Renaud, A., Rousseau, G. & Moineau, S. Targeted Genome Editing of Virulent Phages Using CRISPR-Cas9. Bio-protocol 8, e2674 (2018).
48. Yehl, K. et al. Engineering Phage Host-Range and Suppressing Bacterial Resistance through Phage Tail Fiber Mutagenesis. Cell 179, 459-469.e9 (2019).
49. Selle, K. et al. In Vivo Targeting of Clostridioides difficile Using Phage-Delivered CRISPR-Cas3 Antimicrobials. Mbio 11, e00019-20 (2020).
50. Lam, K. N. et al. Phage-delivered CRISPR-Cas9 for strain-specific depletion and genomic deletions in the gut microbiome. Cell Reports 37, 109930-109930 (2021).
51. Lee, K. A. et al. The gut microbiome: what the oncologist ought to know. Brit J Cancer 125, 1197-1209 (2021).
52. Gencay, Y. E., Ayaz, N. D., Copuroglu, G. & Erol, I. Biocontrol of Shiga Toxigenic Escherichia coli O157:H7 in Turkish Raw Meatball by Bacteriophage. J Food Safety 36, 120-131 (2016).
53. Satlin, M. J. et al. Colonization With Fluoroquinolone-Resistant Enterobacterales Decreases the Effectiveness of Fluoroquinolone Prophylaxis in Hematopoietic Cell Transplant Recipients. Clin Infect Dis 73, 1257-1265 (2021).
54. Satlin, M. J. et al. Colonization With Levofloxacin-resistant Extended-spectrum β-Lactamase-producing Enterobacteriaceae and Risk of Bacteremia in Hematopoietic Stem Cell Transplant Recipients. Clin Infect Dis Official Publ Infect Dis Soc Am 67, 1720-1728 (2018).
55. Harms, A. et al. A bacterial toxin-antitoxin module is the origin of inter-bacterial and inter-kingdom effectors of Bartonella. Plos Genet 13, e1007077 (2017).
56. Henry, M. et al. Development of a high throughput assay for indirectly measuring phage growth using the OmniLogTM system. Bacteriophage 2, 159-167 (2014).
57. Lutz, R. & Bujard, H. Independent and Tight Regulation of Transcriptional Units in Escherichia Coli Via the LacR/O, the TetR/O and AraC/I1-I2 Regulatory Elements. Nucleic Acids Res 25, 1203-1210 (1997).
58. Young, K. K., Edlin, G. J. & Wilson, G. G. Genetic analysis of bacteriophage T4 transducing bacteriophages. J Virol 41, 345-347 (1982).
59. Gibson, S. B. et al. Constructing and Characterizing Bacteriophage Libraries for Phage Therapy of Human Infections. Front Microbiol 10, 2537 (2019).
60. Bolger, A. M., Lohse, M. & Usadel, B. Trimmomatic: a flexible trimmer for Illumina sequence data. Bioinformatics 30, 2114-2120 (2014).
61. Bankevich, A. et al. SPAdes: A New Genome Assembly Algorithm and Its Applications to Single-Cell Sequencing. J Comput Biol 19, 455-477 (2012).
62. Larsen, M. V. et al. Multilocus Sequence Typing of Total-Genome-Sequenced Bacteria. J Clin Microbiol 50, 1355-1361 (2012).
63. Beghain, J., Bridier-Nahmias, A., Nagard, H. L., Denamur, E. & Clermont, O. ClermonTyping: an easy-to-use and accurate in silico method for Escherichia genus strain phylotyping. Microb Genom 4, e000192 (2018).
64. Ondov, B. D. et al. Mash: fast genome and metagenome distance estimation using MinHash. Genome Biol 17, 132 (2016).
65. Yu, G. Using ggtree to Visualize Data on Tree - Like Structures. Curr Protoc Bioinform 69, e96 (2020).
66. Datsenko, K. A. & Wanner, B. L. One-step inactivation of chromosomal genes in Escherichia coli K-12 using PCR products. Proc National Acad Sci 97, 6640-6645 (2000).
67. Hantke, K. Compilation of Escherichia coli K-12 outer membrane phage receptors - their function and some historical remarks. Fems Microbiol Lett 367, (2020).

### SEQUENCES

Amino acid sequences are written in N- to C-terminal direction and DNA sequences are written in 5' to 3' direction.

### PROTEIN

**>LamB (SEQ ID NO: 1)** 
MMITLRKLPLAVAVAAGVMSAQAMAVDFHGYARSGIGWTGSGGEQQCFQTTGAQSKYRL
GNECETYAELKLGQEVWKEGDKSFYFDTNVAYSVAQQNDWEATDPAFREANVQGKNLIEW
LPGSTIWAGKRFYQRHDVHMIDFYYWDISGPGAGLENIDVGFGKLSLAATRSSEAGGSSSFAS
NNIYDYTNETANDVFDVRLAQMEINPGGTLELGVDYGRANLRDNYRLVDGASKDGWLFTA
EHTQSVLKGFNKFVVQYATDSMTSQGKGLSQGSGVAFDNEKFAYNINNNGHMLRILDHGAI
SMGDNWDMMYVGMYQDINWDNDNGTKWWTVGIRPMYKWTPIMSTVMEIGYDNVESQRT
GDKNNQYKITLAQQWQAGDSIWSRPAIRVFATYAKWDEKWGYDYNGDSKVNPNYGKAVP
ADFNGGSFGRGDSDEWTFGAQMEIWW
**>Tsx (SEQ ID NO: 2)**

MKKTLLAAGAVLALSSSFTVNAAENDKPQYLSDWWHQSVNVVGSYHTRFGPQIRNDTYLE
YEAFAKKDWFDFYGYADAPVFFGGNSDAKGIWNHGSPLFMEIEPRFSIDKLTNTDLSFGPFK
EWYFANNYIYDMGRNKDGRQSTWYMGLGTDIDTGLPMSLSMNVYAKYQWQNYGAANEN
EWDGYRFKIKYFVPITDLWGGQLSYIGFTNFDWGSDLGDDSGNAINGIKTRTNNSIASSHILA
LNYDHWHYSVVARYWHDGGQWNDDAELNFGNGNFNVRSTGWGGYLVVGYNF

### DNA

**>LamB (SEQ ID NO: 3)** 
ATGATGATTACTCTGCGCAAACTTCCTCTGGCGGTTGCCGTCGCAGCGGGCGTAATGTCT
GCTCAGGCAATGGCTGTTGATTTCCACGGCTATGCACGTTCCGGTATTGGCTGGACAGGT
AGCGGCGGTGAACAACAGTGTTTCCAGACTACCGGTGCTCAAAGTAAATACCGTCTTGG
CAACGAATGTGAAACTTATGCTGAATTAAAATTGGGTCAGGAAGTGTGGAAAGAGGGCG
ATAAGAGCTTCTATTTCGACACTAACGTGGCCTATTCCGTCGCGCAACAGAATGACTGGG
AAGCTACCGATCCGGCCTTCCGTGAAGCAAACGTGCAGGGTAAAAACCTGATCGAATGG
CTGCCAGGTTCCACCATCTGGGCAGGTAAGCGCTTCTACCAACGTCATGACGTTCATATG
 
ATCGACTTCTACTACTGGGATATTTCTGGTCCTGGTGCCGGTCTGGAAAACATCGATGTT
GGCTTCGGTAAACTCTCTCTGGCAGCAACCCGCTCCTCTGAAGCTGGTGGTTCTTCCTCTT
TTGCCAGCAACAATATTTATGACTATACCAACGAAACCGCGAACGACGTTTTCGATGTGC
GTTTAGCGCAGATGGAAATCAACCCGGGCGGCACATTAGAACTGGGTGTCGACTACGGT
CGTGCCAACCTGCGTGATAACTATCGTCTGGTTGATGGCGCATCGAAAGACGGCTGGTTA
TTCACTGCTGAACATACTCAGAGTGTCCTGAAGGGCTTTAACAAGTTTGTTGTTCAGTAC
GCTACTGACTCGATGACCTCGCAGGGTAAAGGTCTGTCGCAGGGTTCTGGCGTCGCGTTT
GATAACGAAAAATTTGCCTACAATATCAACAACAACGGTCACATGCTGCGTATCCTCGAC
CACGGTGCGATCTCCATGGGCGACAACTGGGACATGATGTACGTGGGTATGTACCAGGA
TATCAACTGGGATAACGACAACGGCACCAAGTGGTGGACCGTTGGTATTCGCCCGATGT
ACAAGTGGACGCCAATCATGAGCACCGTGATGGAAATCGGCTACGACAACGTCGAATCC
CAGCGCACCGGCGACAAGAACAATCAGTACAAAATTACCCTCGCACAACAATGGCAGGC
TGGCGACAGCATCTGGTCACGCCCGGCTATTCGTGTCTTCGCAACCTACGCCAAGTGGGA
TGAGAAATGGGGTTATGACTACAACGGCGATAGCAAGGTTAACCCGAACTACGGCAAAG
CCGTTCCTGCTGATTTCAACGGCGGCAGCTTCGGTCGTGGCGACAGCGACGAGTGGACCT
TCGGTGCCCAGATGGAAATCTGGTGGTAA
**>Tsx (SEQ ID NO: 4)** 
ATGAAAAAAACATTACTGGCAGCCGGTGCGGTACTGGCGCTCTCTTCGTCTTTTACTGTC
AACGCAGCTGAAAACGACAAACCGCAGTATCTTTCCGACTGGTGGCACCAGAGCGTTAA
CGTTGTCGGAAGCTATCACACCCGTTTCGGACCGCAGATCCGCAACGATACCTACCTTGA
GTACGAAGCATTCGCTAAAAAAGACTGGTTCGACTTCTATGGTTATGCGGATGCGCCGGT
ATTCTTCGGCGGTAACTCCGATGCAAAAGGTATCTGGAACCACGGTTCTCCGCTGTTTAT
GGAAATCGAACCACGTTTCTCCATCGACAAGCTGACCAATACTGACCTTAGCTTCGGTCC
GTTCAAAGAGTGGTACTTCGCGAACAACTACATTTACGACATGGGTCGTAATAAAGATG
GTCGCCAGAGCACCTGGTACATGGGTCTGGGTACCGATATCGACACTGGCCTGCCGATG
AGCCTGTCCATGAACGTCTATGCGAAATACCAGTGGCAGAACTATGGCGCAGCGAACGA
AAACGAGTGGGACGGTTACCGTTTCAAAATTAAATACTTTGTGCCGATTACCGATCTGTG
GGGCGGTCAGCTGAGCTACATCGGCTTCACCAACTTCGACTGGGGTTCCGATTTAGGGGA
TGACAGCGGTAACGCAATCAACGGTATTAAGACCCGTACTAATAACTCTATCGCTTCCAG
CCATATTCTGGCTCTGAACTACGATCACTGGCACTACTCTGTCGTAGCTCGTTACTGGCAC
GACGGTGGTCAGTGGAACGACGATGCAGAACTGAACTTCGGCAACGGCAACTTCAACGT
TCGCTCTACCGGCTGGGGTGGTTACCTGGTAGTAGGTTACAACTTCTGA
**>NC_000866.4 Enterobacteria phage T4, complete genome (SEQ ID NO: 5)** 
AATTTTCCTTATTAGGCCGCAAGGGCCTTCATAGTTTTAGCGATTTGGGAAACTTCATCATCACTTAAAG
AGTTGCGATAACCGATGAAGTCGGAAACAATACGGAATTTCTTGGTAAACTCAGCAACCATTTTATCACT
GTTTTTTGAAGCATTATTTGATAATACATCAAAAAGATTAGTTACTGTCCAAATGTCATGACCGATGGTA
TCTTTTCCACCATTAAAATATACACCCTGTAATGAACTAACCATATTAGCGAGTCGTGTATATTCTTCAG
AAACTTCATCTATACTGAAGTACTTCATCATAAAATCTAACTCAGGATACTTGATAATTTTATCAATATA
TCGTTTAGCTGAACTTGAATAACCTACATACTTATCATAATCTACATCATCAAAAGCATCTACATATAAA
TCACGCAAAGCTTCAAAAATACATTGGCACTGACCGAGTTCTTTTACCTTTTTCTGTAAAAGCGGACGAA
TAACATAAAATTCATTAATGCCAATAAGATTAGCCATACGAATCAAAATATTCATAGATGGATGACAAAG
AGATGTAGTACCATCCATAGAGAAAATATCAGAACGATGCATATACGCTACATAACCAGTAATTTCATCT
GCTTCTGATGTGAGGCGTAAATAATTCCTCTTTTCCCAGCGCCCGTCTTTAATTTCAAACTTAAACGCTG
TAGCAGCTTTAGGACGAGGAGCTTTACTTTTAACTACCTTTGGAATATAACTTTTTACTAAAGCTTCAAT
TTCTGACAAATAATGAATGTTAACTTCATCACTTTCAAACATCGCCATAATATCAGGAAGCAAATCAATC
TGCGATTCTACTTCTGGATTAATAAACAGAAGACGTTCGTTATGATGAATATTCAAAGTGTTATTAAATT
CACTATCATCTAACGCACGTGCTAATCCACGGACAATATTAACACGATTTTTAATATTATCAATAACGAT
ATTAATTTTTGTTGTATTAATACCAAACAGACGATAACTTGATGCAACGGCTGAAGTTTCATGACTTTGC
TTAATGCGCTTCAGTCGAGGGTCAAGATTTACTTCATACACAACTCCCGCGTTGCATAACTTACTGTCAG
GTTCAAACATGCTCTGCATCTTTTTATATGACAGATTTTTAGTCGTGAATTTGACTGAATTACTAATCAT
ATAATCTCGAGCAGAATACCCCATCTTCATCAATTCACGATATGTGTGACGAGGAGATGTAGATTCTTTA
AATCGTTTTACATCTTCATTAAATGCTTTCTCACTGAGTTCTTTAACTCGTTCAATAATATTTTTACGAG
TGCGATCATCCAGTGAAAGAGCCTCGCGAGATGGAGCAATATCAAGTGAACCCATTGGAAACTTAATGTA
ATTCACTTCATTGCGAATGCTTAGCCAGTTACGGTCTCTAATAACACCATCGATAGGATAAACAATACCA
CCGTAGATAGCATATAATCCACCACGATCAGGCCAGTATCTTTCTGGATTTACACCGTAATAGTCATCAA
AATCCGGAAAATAATCAATTTCGCGGTCAAGACCATTAATGATAGCCAAATCTTTGAACGGTCGCATGAT
ATAAGAAACTTCATAAGCAAAGTTTCTAAAGTCTTTTTCTTCAACTGGAACTACGATTTCAATACCAGTT
TTATCATCTGGACCCATTTCTTTTACGAATGTAGGTTTAATCTGTGGACCATCACCATCCATGTAAGCTA
CATAACCACGAATTTCACCTTTATGATACGAAGTAATACTAAACGTATCAGTATAACTAAACGGAGATTT
AGAACCTAAACCAAATCCGCCAATAAAGTCATTAGATTCAGCTTTAGATGAACTGAAGTATGAATTATAC
AACCCAGGAGAATTATCATCACCTTGAATATCAAAATCACTCATACCCGGACCAAAATCTCGACAAACAA
ATCGTGGGTCTAAACGTCCAGGAACTTGTATGATAAATTTTTCAGGATTTCCATTAAGTGCATGAGCATC
AATCATGTTAGTAATCAATTCACGGACTACTGCGCGAATCTTGTTTGTATACAAATCAGATGACAGAATT
TTAAATACTTTAGGAGATGCTGTGATGCTAAATGCTTTTGATTTAGAACCATTACCAAGAATTGTTTCTT
TTTCAGTGGTGATAATCATAATTTCCTCATTAATTCATATTACGCTTAATAACTTCAGCAACTTCTAGTA
GTTCATCTTTAGTTGCGGTGTCGGATTGAATTTTATCTCTAATATCTTTAAAGCGGGTTTTAAATTCTTC
GGCTTCTCCCATATCGAAAAAGCGTTGAATGATTCTATATTCTCGATGAACTGCTTTATCAAAAAGTTCT
AAATTTACTTTATATGATTTCATTTCAATATCCTCATTTGCCCAATTAATTATACCACATCCTTGTGGTA
AAGTAAACTACTGGCTCATCCATTCTTTACGAAGGTCAGCATTATCTCCCATGAGCATTTCAAAAAGCTC
TTTCCAGTTCTCAGGAAGTTTAACAACATCATATACTGGGTTTTGAATCATCTCACGATATTCAGATTTT
TCCAAAGAGCCAAGTCCCTTAATATAACGGATGCTATGTTTAGGTAGAGCATCTTTGGCACTCTCATATT
CAGCGACTGTATAAAACCATTCTTGTTTTTTACCGACCTGAGCGATGATTACAGGAGTTTTGACAAAGCG
 
AATTCGTCCTTGCTCAAACAATTCTGGCCAATTACTAAAAAATCCGAGCAGAGAAGGATAAATAGAACCT
AATCCAATAATCTCTTAATTATGAGGTATTTCTATAGATAGCCCGAAGGCTATCCATCGTGATCTGCGTC
TGTCATAATAGCGACATTCGCATAGTTCATTGAAGAGGATTTAATAGAACGACGAACATTGTTCTGCAAT
TTATATTTTTTCATATCAACGCTAGAAGAATCAATTTTTACAAATTTCATTATACACCTCATAGAACTTT
TCATCAGGAATCCAACCGCGTTTAAATTCATTAAATGCTCGGCCGAATAATTTTGAATTCACAGTTATAT
TATTAACTGATTTCCATTTAGCAACTCCCGTTCGTTTATAATGATCGGGGTCATATTTCGTGACGTACCA
TTCATATAAATTTGGTATTAATTTTACAGCCTCTGGATTTGTCGCTGATTTATTATACCATGGTTTCGCT
TTTTCAAGTTCAGCTGCTTTTGCAGTAGCAGCAACAGTATTTCCAACACCAACTAATTTTTCAGTTTTAA
TGCGTGGATCATTAACATGAAGACGAAAAACTTCGCCCTTTTCATTTTTAAAGCATGTCATGCCTTTAAT
TCCAGGAAGCTTAACACCTTTATTAACACCGCATCATTCCTTTGGGTTAAATGATCCTTTAATTAATAAG
GCGCATTTACCCGATTTAACTACTTCTCATTCAACAACTTTATCTTTCATAACGTTTTTTGACCATTCAG
ATACTGCTCTTTGATGGCTAAATTCTAGCAATTTCACTATAATTTGCACTAGAACGTAAACTATTTTTCA
GTGTTTCAACATTCTATTCATCGCATATGCCATTTCACGATTACGATGAATTTTATATAGTAGAAAATAG
TGCTAAAAAGTGTTCACGAAAAGTCATGTTTCACCAAATTTTCGTTATCATCAGAACCTCCCATTGATCT
TGGAAGGATATGATGAATTTCACCCTTAAATTTAGAAACGCGGGTTTTACCCCGCACTATTAAGTCATTA
TAGATTTTTTCGTAATTCACCTACTGTTATCCATTTACCATTAATCTGTACTTCATCATTTTCATTTACG
ATAATTGTATCGCCATTTAGCTCGAAAGTAAACCACTCGCCATCTTCTTTTTCTTCAAACGCTTTTTCAC
CGAGAACTAGACCAGTGATTGCGCAAATATCAAATAGTTCTTTGTTTTTAAGCATATCTGCATAAGACAT
ACCCCAACTGTTGAGAACTTTACCACGCAATGGATAACCACCGTGAAGTTCTTTATCACGAACATCAATA
AGATATCCGATAGCCGAATCACCCTCAGTCAAGAAAAGAGTAGTATCAGCATCTTTACCGCAAAGATTCG
CTTTGATATGTTTATGAACCTTAGCTTTAGAAGCCTTTTTAGCTGCTTTAGTTTCTGCTGCTTTTTCTGC
CGCCAATTTACGAGCCAAAGCAGCTTCAATAATCGGCATTAGAATTGCTTCATTATTTAGAATATCACGT
GAAATCTTTTTAGCATCAAGTTGAATATGACTACGAATTTCGCCAAATGGAGAAGTCAAACGCTCTTTAG
TTTGACGAATCAATCGCATGTTTTTCATATCACGAACAAACATAACGATAGTCAAACATTCTTTGACACG
TGCTTTAGTCACATCAATTTTGAACTTACGTTTGATTTGTGGAATAAGGTCTTCACAAATATCATCCATA
GCGCAGTCAATGTGATGGCCACCATTCTTAGTATGAATGTTATTGACGTATGTTAATTGACGAAAACCAT
CCGGTGAACGACCAACCGCAATAGAACAATTTTCTTGCTCTTGAACAATAGCATGTTCATCATACTGCCG
TGCATATTTCTTAAAATTGCCCTGAACCTTTTTACCATTAAAGGTAAATTGAATATCAGGATAAACTACA
GCAAGTGTCTGGAGACGATCCAGTGTAATGTCAAGATAAACTTGGGACAGCTCATTAGTTTCAAATGACA
TAAAATCAGGAATGAAAGTAACACGAGTTCCTTTCCATTTTCCAGGAATATCTTCCCATGATTTATTTTC
CATGCCATTTGAACAACGAACTACAATATTATTTTGACCGTCGCCAGTTTCACCGACAAACATCACAGAA
AAAATGTTTGTCAAACTAGAACCAACACCGTTCATACCGCCGGTGACGCGTTCTTTATCATCACCAAAGT
TACCACCTGCTTTTGGAATAGTCCATGCGGCAACAGGACCAGGAATTTCTTCACCGGTAGGTGTTTTAAC
CATCGCTTGTGGAATACCGCGACCGTTATCTTCAACTGTTACTTGATTGTTTTTAATAGTAACATTAATT
TTATTCGCGAATTTAAACTTAGTACGAATACCTTCATCTACTGAGTTATCGATAATTTCATCAATAAGCT
TAACAAGACCAGGTACATACTGAACACTTTCCCATTTACCAAACATAAAGCGCTCATGCGTTTCATTAGC
AGAAGAGCCAATGTACATGCCACTACGCTTTTTGATATGTTCAATATCGCTCAGAATTTTAATTTCATTC
TTAATCATCACTTATCCTCGTTTGGTTTCGGGAATATTATACTCCGGTAATCATAAAGCTAAAGGCCCGA
AGGCCTTTTATTTAAAACGAATAGTTGAATCCTTAAAGAACAGCCCAGAACATACTGTTCCTTCTACTTT
CTGCCCGGTAGGTCCAATAGCACGAAATCCAGTATGCTGGAAATCATTTTCAGAGCAACCGAACCAATTA
TATCCAGTGATTTCAATATTAGTAAAACCACTTGAAGACAAAACTTTGGTTGCATTAATCAGCATCAGTA
 
CTATTAATTAAAGACACTGCTAATACTAATGCTGCAATTGAACGACTAATATATTTCATAACTACCCTTT
AAGCAAGTCGTAAAATCCATTATTCCCATGCTTAGGAAGCGGAAACTAACCGAACAGCCAGCCGATGACA
ATCAGGACATACACCAGTATCTCTTCCAGAAATTTTCTTGATTTTTTCGTATTCTTTTGCACAGTCTTTG
GATTGACATTTATAATCATAAAGCGGCATAATTATTCCTTAAAGTAAGCTTTCAACATCTGATATAAAGA
CCACGCCTGATCATTATTTTCAATAGTAACTTTCATGACTGGGAATTCTGTGAAATCTTCTATTTGTTCT
TGCTCTTTCTCTTCCTGCTCTTGCTCTTCAACCGCCTGATATGGATTTTCCACTTCATCAAAGAACCCAG
CTTCGTTAGTAGAGAGCCAGATAAAGTTTTCGTCAAGGATATCACCGCCGGCACAACGTTTGAGTACACC
TATGGATGTCATAATTTTAGTAGGACGCCCAAGATAATCAGCATCTAAAATTTTAAAAGGCTCCATACCT
AAACGTCGTGCATAGATTCCGTTATCAGTATGGTCTTTAATAAAATTTTCTTGAGCTTGTTTATTTTTAA
ATTGATACCATTTATTAACTTCAAATTTAATAGCCATTAATAAATTTCCTTCCAGTAAGTTGTGCCGTCT
TCAGTAATTTCACGAAATACACCATAAATTGGCTGTTTATCACCGACTTTCTCATACACATAAACAGAAG
TCAAGTGAGTAAACTTGCTAGTATGTTCCTTTTGAACTACTACCAAATTTGGATCAAATAATACATCTTC
AAATTCATCATTAGTGCAATTCTGAACAATTTTACGTTTCATTACAATTTCCTCATTAATTGAACAGTGG
AGCGATACGTTTCAGAAGAGTATCAACACCTTTAGCGAATTTTCCATTTTATTCTCCAAGTTGTTTTCTG
TATCAGTAGTTGATATTGATATAGTACCATAATCAACTACTGATGTATATAGTTTTATGAAAAATTTAAA
CTTTATGCATAGAGAGCATTGCTATAGTGTTTAATCCAACTTTCAGGAATGACTTTGTATGTTCCTAAAA
ATACCACGTTGTACAACTTAACACCATCTTCTACCCATTGATCGGTAATGTATCCACACATAGCGCGAGT
ATAAACAACCCTTCCATCATCTTTAATAAAGTTAAATTCACAAGGAGCAATGAACTTGATAGCCTGACCG
AGTTTCCACTTAAAGTCTACACCTACATGCGAAGTATCAATCGTTTCAATTCCTTTAGCAGGAACAGCTT
TTAAAAACGCAGACTCAAGAAATTTCGCACGAACATAGCCAAACTGGGGTTTAGACTTTCCATCTTTAGG
AATGATACGCACTTTTACTTCAGAATCTTCATCTTTAACACCATGCTTAAGCTGAATGCTTACAACTTCG
ACCAATTTTCCTGCTGCTTTAGAACGGGATTTATCAGATACACGAGCTAATTCACCAATATTAATAATCA
TAGTTATCTCTCACTTGTTAAAAAGATTTTATACTCCACGGGACCATTATACTCTGGTCCCAAGAGTTTG
TAAACTATTAATTCAAAATAGCTACCACTGCACTACGAGGAACTACGGAGTACTCTCCAGCATGAACTAC
GTTCAGAAGTTCAACGCCATCTTCCAATCCATTGGTCAGTTACCCAACCACCAATCGGATTCGCAAATGG
ACGACGAATGTAAACTGCCTTACACAACAAATCAGTCGGGTCTTCAGGTTTTTCAATTTCTGTCAATAGA
TTAAAATCTTCTTCATAGATGATGAATGATGATACCCTTCCATAATAGTTTCTAATTTCCATGTACACTG
TACCAATGAGAATTCCACTATTAACACTAATGACAGTAAAAGGATATCCGCCAGTTGTACCAAGAAGTTC
CCAAAATTTGCTATCAGTCGTATTCGACATTGTCTGAAAATCAATTTCAGATGGTTTTTTCATTTGATAC
GCAGTATTAATTTTAATCATAATTTTCTCTTTAGTTTAAGGTAATAAAGCCTTTTAGTTCGGCATAGGAT
TTACGGAACATTACTTGATGCCCGCCAATGATAACTTGGTCATCTGGTACTTCGTATACAGCAAGATAAA
ATCCTTTCGAAGCTAATTCTTCACGCTCTTCTCGTGTGAACCATTTCATCATATCATATTCGCTAGCAAA
AGCAAAATGATAAAGAGCTACAAACCATCCGGGAATATGATATTCTACTCCAACATAATCTTTCTTGAAC
TTAGTATTAATTACGATATTAGCATTTTTAACTAATAGTTTGTCTTCGTGCGGCACAGGAATTCTTTTAT
TATCATTACTATGATGCATAAAATTAGGTCTGTCATAACCTACATGTAATAACCACTCTTCACTCCATGA
ATCTATTATACTTCTATACGGCGTTATTTGAACACAAAGATCTCGGCGTATTGTTATAGCGTCTTCATAA
TTAAGAATACTAAACGATGATTCAACACGATAAATTTTCATTTTATTATCCTCAGTAGCTATGGTGTTAT
AGTACCACAACTAACCGAGGAAGTAAACAACTTTTTATCGTTTTGTTGGAAGAGATAGAGGATCGCATTC
TTCCTCTGATGGAGCATCTTCAAGACCCATAGCATATCGCAAAGCATACTTCATCATCAGGATGTCTTTC
GCACAGTCATGAATAGAATCATGTGCAACGAATCCATCTAAAGTTCCCTTTGGAAGAGGACACGTTGTCA
TATCACGAACAAGCAGAAGTGCTTCAATTCTAGTACGAATATCACGCTGATTCCAAAATTTACAAGGTTC
 
TAACTTAAATGTATCAAGCTCATTCTCGGAAACGCCGTTAAGACGTTGAATATCGCGAATAAGATCGACT
AAAATTGGAAAATCAAACGACATTCCACGGCACCAGCCTTGAGATTTCCAAGGATCGATATTATGTGCAT
TGATGTAATCATTAAATTTTGCAATACCGTCGATAGTGCTTACATCTTCATCGGATGGTGCAATATTTTT
TCGAGCTTCAGGAGATTGATTCTTCCACCATTCGATAGTACTTTTAGTAAAAAGACGGTGTCCTTTTTGG
CTTTTTAAATCAAATTTGATTTTAATGCCACGTGAAACTAATTCATCGAATGTTTCAACTACTTCTGGAT
TAGGGTCAAAAGCAATTACAGCCAAATCAATAACCGCTGCTTTTTCACCACTTCCCATTGTTTCAAAATC
TATAATAAAATCAAACATTAAATTTTCCTCGCTAAATCACGAATTTGACCTACAGTATAGTCTTGAATAT
AAACTTTATTAATAGGCTCATCAATAAATTTTGCCATAGATTCAATATCTTTTTGTATTTCTTCAAGACT
GTATACTATCTTTGAAGCTTTTTCGCGAATAGTGATATTTTCAGGACCCGGATTTTCTTGAATGACAACT
TTAACATTTGTCATAAGAGATTTAAACTGGTACCAACTTAATTCAATCATTAATAATCGCCTCATAAAGA
TAGCTAATTTCGCCTAAAACATAATCATTGATTGTAACAGTTTTAACTTCACCGCAAAAGAATTCTAACG
CAATTAAATCTCGTTCAATTTCTTCTAATTGAAGCATCAACTTACTAGATTCAATTTTTACAGTTTCACG
ATTTTTGCTATAAGCTATTTCATAAATTTCGCTTACTTTATCTTGAAGAAGATAAAACTGATCTTTAGTT
ATTTCCACGAATAGCTTCCTCAAATTTAATCATACATAAAACACATCATAACGACCACGGGTGACACCAA
CATAAAGAAGTTGTTGAGCTAATTCAACATCTGCATAATGAATACAAGGCGTATAAATGAAAGCACGGTC
TACAGACATACCCTGCGCTTTATGGAATGTTGATGCAGGAAGTGCTTTCACTTTACTAAACTGTGATTTA
GCATCCCAAAAATCACTCCACGGAGCTTTTCCGCCTTTGTTCCAATTTTTATAAGTTTCTGCTGTTTTAG
CTAAAAATAGGTTAAACTTATACAATTCTTCGTCAGATGAAATTATTTTAATCTTTTCACGATAATATTC
ATCATCGCCATAAGTTTCTACTGTTAAATCCCAATGACGAATTAGATATTCTCCAGGAACACCACGGGCT
TTAACAAACGTTGATGTATACTCTGCTTCTATAATACGAACTAATTGTCCGTTATTAAAAATAATTTCTG
ACACAGGCTTTCCATCAATTTTATATGTTTTAAATAATGGTTCCTGCATTACAATAATTTCACCGACAAT
AAAATCTTTATCAGTTTCAAAAATCTTTTTACGAATAATGCTATTTAACTTGTCAACAGATTTATTCGTA
AATGCCATTACGCGATTTTCAAACAAATCATCTAGTGATTTGACGATTGAAAAATAATTTACCATAAAAT
CGCGTAAAGCGGTATCACCAGTAAATCCACGTACTCCATGCCCGTCAACAACTTTATCATAATTCCACTT
ACCGTTGCGAACGTCAGTAGCTACATCAATAATAGGAGCATTACTGCGTTTAACTTCAGTGAGTTCACAC
TGATAAAAATCTTTATGTGTAAAGAATGGACTGATATAAGCAGTATTTTCTCCTGGTTCAACAGGTCTGA
TTTGCTTATTATCCCCTATTCCAATTATAGTACACCAAGGTGGAATAGTTGAAAGCAGAATTTTAAATAG
CTTTCTATCATACATTGACACTTCGTCGCAGATTAATACTCTGCATTTGGCTAAATCAGGTACTTCTTTT
TGTTCAAAAAGAACATTTTCTTCATATGTTACTGGGTTAATTTTAAGAATACTATGAATAGTACTCGCTT
CTTTCCCTGATAGTTTTGAAAGAATCTTTTTAGCTGCATGTGTAGGAGCTGCTAAAATAATACCAGTTCC
ACCCGTAGATATTAAAGCTTCAATGATGAACTTAGTAAGAGTAGTCTTACCGGTACCAGCAGGTCCATTA
ATAGTTACATGATGTTTCTTTTCTTTAATAGCCTTCATAACAATGTTAAAGGCATTTTTCTGGCCTTCGG
TCAAATCATCAAATGTCATCGTAAATTCCCTGCAATTGGTATACTAACAATACGCCCAGTATCTAAAATT
CGCTGATATAATCTTTGCGTGTCTACGTCAGGCTTAACATGTTTAACTTCTATTTTATTAAACCAAAATT
TACGTGGAGTCTCAACTAATCTTGGAATTCCCTTACCTAAAGCTAATCGATACTGCTCTTTAAGAGTGGT
AAATACTTTATCAGCAATCTTCCATTCAAAAAATACAGCAGGACGATGTTCATCAAGCGGAACTGGCGCT
GTAAATCCGTCTTTGTCTCGGTAAACTATCGCATATACATAAACCATATTATCCTCGGATAAGTTTAAAA
ATTGAACAATTTAGCGGATATCCTCTTTTCAGTTTAAGTTTATCAATAAAAGACAAATTTTGATACCGCT
CTACACCTTGAATAATTTTATCACACATATCATATTGCATTTCTGCTTCTGACAACTTTTTCACAATTTT
CCAATCCGAGCCTTTAAGAAGAACGTTCAATTTAACAACTTCAGCGCCTTCTGCTATGCGAGAACCATCA
ATACGTGCTTTAAGTGCTATAATTCTCAGCTTAATGTCAGAGGTCTGTTTTGATTTAGAAAGCTGAGAAA
 
TGTGTTCAATTCGATTTTCACGTTTTTTCTGTATAGCTTTAATTTGATTATAAGTCTTTTTGATTTTAGC
CCATTTCTTTTCATCTAAATTTAGTTTATGAACTTTTTTCGCAGATGAACGACCAATTCGCAAAGCAAAT
AAATCACGCTTTTCAATCAACTCTTCTAAAGTATAATCAGAACGAAATGTATTATACTTTTTCTTTACTG
CAATAACATTCCCTTTAATGTATCCAACGTTATTATCAAAACGTTCTAATGATAATTTCTCTCCTTCAAT
ACGATTATCAAAAGGTTCTCCCGAGTAAGCACAAACTTTTTGATCTAAAATGTTCTTAATGTAATTGAAG
TCTAAGTTAAAATCTTTAGAACGTCTTTTTGCAGATGCCTGAGTATGCTCTAAACGACGTTTAATTTTAC
GAATTTGGTTATTAGACAGCTTCATATTTTTCTCACATCTTACGGACGGTTAACTACTTATACTATAACA
TTTTTACTTTAACTTGTAAACAACTTTATGAAAAATGCTTTAAAACTTTCATGGTATAATGAATCTAAGT
CCTTCCATTATAGATTAAATCCTTCAAAATCAAGAGTATAGATAGTGTATGTTGAACACTTTTTATACTC
ATATCTATCTGCAATTCTAAATACACTTCCAGCTGGTATCATTACTTCTTGTTCATCTGAAACTAATTCC
ATATTACGATAACGATGACTATCCGGAAACTTAAAGTTTGGATTGTATTCTTTACAGCGTAGAGCTTTTA
TAGCATACTCCTGGAAATTGAATACCATAGGAGCTTTGAATTCAAAAATAACTTGTGTGTTATACTCTAA
ACCAGAAGCAAAATGTAGAGCTATATTTTTATCATATGAAGCTGATACGACTTTATCAAATGTAATAATA
TCAATTCCTTGATTTAATACTTGTTTAGTCTCAGCTGGAACACCTCTCCAAAGAGGTTTATCGTTTGGAA
CCAAACGAGATTTGATTATTTCATTTAACCAAGAATGGTCATCTGGTTTATTAGTAATACAATGAATTAA
AAGTTCAATTTCAGATAAATTAAACCCTTCAGAAAGTAATTCTTCACGAATAGAAGCACGCACCGATGCA
TCCATTGATTTTATTTTAAAATCTTTTAGTTGCATTACTGAGTATTTCATTCAACTACCTCAATATCATA
AACTTTAAATGTTCCAAATGAATCGTGTAATTTTTCTTTTGAAATAGAAGTTATTTTATACTTTCCAATT
GGAATCATCCATTCTTGTTCACGCACAATCATCATTAAGTTATCAGTACGCTCTGAATCTAATCCATCAG
TATCTTCATACGTGTACTTAAACTCAGTATTAGGAGAAGAAAGTATAATATCGCTGATATGGTCAGAATA
ATTAAAAGCTTTATCAGTTTTTAAACGAAGTATTGTTTCAGTGAAATATTCAGCATAAGAAAAAGAACAC
GCTGTATGCAAACTAGTAGTAAATGAATCTACCCTGTTCGTTGAAAACACTTCTCCAACTTGTAAATCTT
TAATGAGTTCTTTTGTCGATTTTGATATACCACGATATAATTGATAAGGCGATTTAGTTAAATGCTTTTT
AATGATTTCATTTAAATGCTTATGAAGAGCTTCATTCTTTTTGGCTTCCATACATTGCCAAAGAACAGAC
TGCTCAAAGTCAGTAAATTTTTCACAGACCTTTTTATACATATCATATTGAAAATCAACGCTTTCAGCTT
TTATAGATAACTGTTCAACATCTGCAAGATTAATAATCATGATAGCCTCCGTATACTTCAGAAGCTATCA
TATCATCGTTAGAAAGGAAAGTAAACAACTTTTTGAATTATTTTGCCCAGGGAGCCCAAGGCGGAGGGTC
AAGATGGTATGAAGCTAGTTCTTCTAGAAGAGCATCTGGGGCTTCAATTCCATAATTCTGTAATACTATA
CGGTACTCTTTCTTATAATCACTAGAATCATTCTGGTTATTCGTAGAATGATTATCTTCTAACATCTCAA
ATAAATCCATATTAATTCCTAGCGATAAAAACCAAATTTACGATTAGTTTCAATGATCTTTCTTTCTTCT
TCGGACATTCTCCAGCGTAGTCCAACATCAAAATGAGCCCAGACCATCCTAACAAATGCATTTATATCTT
TAATATCTTCAATAATGAATGTTTTACTTTTAGAAGGTTGACTCGCTAATTTAACTTTATCGTCTTCAAA
CATGTCAAAAAGACCATATTCATGAGCTCTTTTATAGCCTTTAATAGTTAAAGCTTCAGAAGAAGAAAAT
GGTGAATCTGTATTCTGCAAAATATCTTCAGTATGCTTTATAATTAGAATAATATTTTCTGGATATTTTC
TTTCTTTTATATCTTTAATTAAAAAATCCGGATTTTCTGCTAAAGGAATAATTAATGAGCAATTTTTATC
AAGTGTATTTACTGATTTACCTTCTTTAGACATAAATTCTATTGAATATAATTTTGCTACTTCAATCATG
TGATTTCCTTTTGCCTACTAATGGACCGTCAGGAATTTTATTTTCCTGGATATATTTCTCATTTTCTTCC
ATCATTTTACTGCCAATTTTAAGAAGCAAATCCATTGCTTCATTTGCTTTTGCTTTAGCTTCTTCTAACG
TCATATCTTTGTTCATGATTTATCACCATAGATGTCTCTCATCAATTTAAGCGCTGAGCGTTCTAGTTTC
TTTTCTTTCTCAGCACTAATCATTGATTTCATCCATTCTTCTGATTCATTCTGCATTTCTTTATTTGCTT
GTTCAACCCAACCGTCATCAATATACATTGAGTTTGGTCTATTGAACCATTCAAGCATCTTCTTCAGAAC
 
TTTCATTCGTTTTACCTAAAACAATAGTAGGAGCATCGTCAAATTTATGAATTTTTAGCAAATTTGGATT
TAAATTATTCCATAAAGAGGTAATAAAATATGATAGCGCACTTTCGTCCGTAATTATAAATTTATTTCCT
TTATCTATTTTCCAATCATATATTGAATCATATGAATAGAAGGATAACGGTTTATTATTATAATATGCTT
CAGCAACATCAATATAGTTAGATTTAGTAAATGCTTGAATTGCCATAAATGGAGAATTTTGTACAGTTTC
AATAATTCCGATCTTTTTAAGTTTTATTTCAACATCTTCTGGAATTGGCATTGAAATAAAATCTTCTACT
AGATACATATTATTGTCATATCTTTTATCAATCATTACTGCTACAGTAATTGGAACATCCTTGACAAACG
CCATACTAATACTATTGATAGACATTTCAAACAAAATTGCTTCCATAATTTTCCTCAATCACAAGATGTA
GATGAACAACTAGAATCACAAGAACTTCCACATGAATCACCTGCCCATACATGAACAGGAACATTAGTAT
CATATGAATCAGAACTAGACTGTGTATTCTGTGTGTTAGATGATGTAGTAGGTGTTGACCAGCGCCAAGG
ATTTTTATAATATTCTTGGGCTTCTTCATAAGTCATAGTAACTGCTTCTACTGTTCCATCTCCCATATAA
ACATATTCAACTACAGTTAAAGGAAGGTAGTCATTTGAAATAGGAACTACACCTTCCCCAGGAGTTGTAG
AGAAAAAATCCGTAAAGAAACTTTTAAACCAATTAAAGATAAACATTACAAAAAGCCTCTTTTGAATTCG
ACTTGCTTCTCACCATAATCATATCGAATCTCTACATTAAATTCGACAGAACCATCTGCGTACATCATAA
ATGAATGCACAACAACTTCTGTAGACCATGGTTGTAGTTCATATTTCTTCATTACATGTCGTGAAATGAT
AATATCTAAATCTTCATTTGGTTTAATCCAACGATTTAACATAGTACTCTCCTCTATAAGATAATTCTAT
TATACCATACTCATTTTGGAAAGTAAACCATTTAAATGAAAAAAGGACTCCCGAAGGAGTCCTTGAGTTA
TTAACCAGTTACTTTCCACAAATCTTCATTTGCAGCAATCCATTCAGTACGTTGATTTTCTTCATATACT
GTAGAATATGCTGCTTTTTCTGAAGGGAATGTCTGGTAATGAGCGCCAGAAATTTTGTGAACGTCAGAAT
AAAGAGGGAAAGCTACAGAAATTTCCTTTCCTTCAATTTTCTTATTTCCATCTAATTTCTGCTCAAATGT
TTTGATATTAACATAACCGCGAGTACTAGCCATGTAATTCTCCTTTATTTAAATTACATGATTATTTATA
CATCTTCTTTTCTGAATAAGTAAATTAAATTCTTAAGAGCCGAACTTGTTACATCATATTTTCCTTTAAG
CGCCTTTACAACCGGGCCTGTTGCTGGTTTACCTAAAGAAACCCATAACTCGTGTATTTCGCTTTTAAGT
GGTTCATGCCAATGCGGTGCTTTTCTTTGCGCCCTTGAAGTTCCTTCTGAAATCTTTTTATTTCCGCCAT
TCGAATAAAACTTTTTCATTACTTCAGATTGCCGAGCTTTTCTTTCTGCCCTATTTTGGGCTATACGTTG
TGAATTCTTCATCCGAGTTTTTGTTTCAGGATTGTTTAATCTAAGTTTATGTTCTAATCGTTGTTGCTCA
GTCCATTTTCTTCCTTCTCCACCCTGACCACCAGGAGAAATATTAATGCAAGTATCTGGGTGTTTACGTT
TTAATGCAGATATTAGCTCACGTTCAACTTCATATGATTTTTCTCTAGAACCATGGCATTTTGACCATCG
TATTTTATAATTAAATCCATACTTACGATATATGTTCCATAGTATTTTACCTGAACCCGGATATTTATCA
TTATATGGATTTACAATAAATGATTCATGTTTCCCAGCGTACCAAAAGACGCCTTTCGGCGTCCTAACTT
TTACTATATATGTTCTATAAAATTTTTGTTTAATATCCATTATCTTGACGTTCAAAATTATGTTTGTTCT
TCAGATAATAAAGTTTAAAGATTTCTTCCGCATTCATTCCAAGGCCAACAAACATATTTAATACGAAATG
AAATATATCCACAAGCTCAAATTTAATTTCGAGCTGGTCTTCGGGGGACATTTCATCAATGCGTTTTTCT
TGCGCTTCAATATAACGTGCTTTCCATTTTTTCCATACAGCAGAAGCTTCTTTTTCACCACGTGACATTT
CACCAAGAGAAGTCAGAAGTTCGCGGAATTCATCATCAATACAGTCTTTTTGTTCACGCATCCAAGAAAC
AACATCACCGGCAGTTTCTAATTTATCTGGATGATAGCAGTATTCGCGGACATTAGCCAAACGAATCTGT
AAAAACCGCTGCATATCAAGCATAACTTGCAGCGGATCTTTTTCATCACCGAGAATATCCCAGTATTCAT
TTTGAGCTTTATCAACACCTTCGATCAAATGAGCACATTCATTAAAGTGAGCCATTAGTTTTCCTTTCAA
TTCATTAATAAGTTAAATAATTATATCATTTGAGTATGTAAGCAATTAATTAAAAATATATACTTCATCA
GTTCCATTCTTTTCTTTGGAATGATATATGTTAAAGACGTATTTTTTATTAAGATGCTTTACATTATATT
TTTTAGACCATTCTTTAAGAAGAGTGTTTTCCTTTCCGTGGTGTTCTAAAACATTCGACTGCCCAAATTT
TATTCCTCTATCATTTAAAGAATCTAAAAGATTTAAAAGGTCTTTTTCTTCATCTTCTGACCAAAATTTA
 
TTATAATCAGCAACTGTTATGAGATACGGAGGATCTACATATACAAAATCGCCGTCTAAAATTTTAACAT
CTTTAAAATGCAATGAACTAAAGATTATTTTATCACAATTTTGTTTAAAGTGATTATATTGTTTTTCACT
ATTTTTGTTTATAGTTCTTTTTCCAAACGGAGTAGTAAAATTTCCTTTATCGTTTATACGAATCATATTA
CTAAATCCGTGAAAATGAAGAACATAAAGTAAAAGAGGATCTCTAGTTTTATTATAATCTTCACGTAATT
TCAAAAACTCTTCTTTTGATGTTTTTGATAGTTTGTATTGCTTTATTACTTTTAAAACGTCATCCCATGA
TACATTAATAAGACGCTTATACATTTCAATAATTGGTTCTTGAATATCATTGGCCAATACAGGGCCATTA
ACATTCAAAGACACTGATAAACCTCCACAAAATAAATCCACGAATCTGTTATATTTTGGAAAGTGAGATT
TGAGTTCAGGTAATAATGATTGTTTATTACCTGTATACGCGATAGCTCCTAGCATTATATTCTCTCATTT
ATTGCAGCAAAAATGAATTATACAATTCTTCATCATATGTATTTGATAGTAATACTAACACGTTTTGCGA
TAAATATTAATTTAAAGGAGGATATATATGGTACAAAAATTAATGGCACTTGTTAATGCCATAAAAGGTA
ATAAGAAACGTATAGCTTTTACTATTTCTACTATGGTAGGAATTTTACTCTGGAACTTTATTTTATCACC
TGTTGCAATTGCACATGGTGTTAATATTCCAGTAGTTACTCTTGATACATTCGTAGATTTAGCATTTGCT
TTAGTTGGGTTAATTTAAATCTTAGCATATTTAGATAGCCGCATTTTAGCCATTAACCCCTGGGCAATAT
TATTTTTCATATATTCCATAATTTGTTCAGGGGTTGCACCTTCCTTTCTAATCATATCATTAACATCTTT
TGATTTCCAGGGAGATTTATCCCAAAACATAACCCTTTCTCCTGCATCAACTAATTTAGTCATTCGTTTA
ATAGTGTCAGGGTGACGAGGTTCATTATCTAAGACCCACACACGTCTATCTTTAAATGGAACAACTTCTA
GGTCTAATTGACCGCCCGTAATAGCTATACCATTTTCAATAAAAAGTGAATCTATAGGTCCTTCTAGAAC
ATATACATCACCATCTTTAACTCGTTCGACTCCATAGATTTTTGTTGCCTCAGGATAAGCTTCGATGGTG
ATATATTTTTGAGGAGCATCTTTCTTTAATGCACGTCCTTGAAAAGACTCAGCTTTTCCATTAGCATTAT
AAATTGGAATAACAAGACGAGGCTCAGAAATTTCCTTTTTGTATGTTCCCGGTGCTATGCTATTAACTAA
TTTAGGCCATTCGGTTGTAAACCAAAGATATTTCCATTTATCCTTTGGAATACAACGAGCTTTTACGTAT
TTTATAATTGGATGGTCTTCCGCCAGTTTATCTAATCTAACACATGACGGAAGAGATTTAATTATTTTCT
TCTCGGGTTGTTTAGGAAGTTCTTTAGGTTTTTCTATTGGACGACTTTTACCTTTTTCTTTTCTTATTTC
AAAGATATACTCACGATATAAATCGGGTTCAAACTCCTTTAAATATATTCCGATTGGTGCATGATAGTTA
CAGTTATAACAATGAATATTTCCTTCATTATTATCACCATAATACCATCCACGGGCTTTATTCTGGTCGG
TTTTTGAATCTCCACAAACAGGGCATCTAAACCGTAATTTAAAAGTTGAACTATTATTTACTTGTGTGAA
TTTAGGTAAATGAGCTAATGCACGGTATGCAAACTCATTATCAATCCAAGGTATTGATGACATTTTTACT
CTTCTTTTTCTTTAGATTCCTCTTTTTTCTTTTTAGGAATCTGTTCAGGACCTTTATTTACTACAGCGCC
TGATGTTGTTCCAATAGAGATATTTTCAGGATTACCACCTGAATCTCCAGCGACCATATCTTCTTTGATA
AATTCTTTAAATGTTTTCATATTAACCTCTATTCATAAAAGCATTAAAAATTTGGTCATCAATAGAAACA
TTTACTTTAGGCTGTTTTTCAGATGGCAATTCATATCCACATATTACAATTTTATGATCAATATCAAAAT
ACACAGAAGCAATATGATTAATGATATTTTCAGTAAAGTCTAAATCAACATCAATATCTTTTTGACCAAA
GCCCAAAGGATAAATAATGCGAGTAATTCGATTATCTTTAACAAAGATTCCACCGACATACTCTGTGCTG
CGTTTAAAGTCTACACGCCGACGAAATGAAAAATATTCAGGCTCTTTATGAGCTCGGCTCATAGGACACA
ACGAATAACTAGAATAAGAGATGTCAAATCCTACGCCTTCAATATGAACTAAATTGTCATGATTAAACCA
ATTATAATCATATGCCAAGTCCATTAGATTGTCATATGTGAAAAGCACCGGATTAACATCATTGGTCACA
AGCATATAATTAGCAACTGCTATAATTTCATTATTTTTAACGAATACAAACCGTGATTGATGCGAATGGC
CTGGACCTGCGTTTTCACGATTAATGATATAACACTGGGCACCTTTATATTTGTACGTGTCTTTACACTT
GTGCATTTGATAAAGCATTATTCACCTACCACTTCAGCGATGATATTTTTGTTATTAAAGTTTTTATCGC
AATACAGAACATAATTATACTGCATTACACCACCAGACTTAAGCTGTTTTTGCACTTCAGCTTTCATTTC
AGGACGATCACGCTTAACGATATTCATAATATCTGCTTCAATTTGAGTTTCAACCTCAGTCTGATCCGCA
 
GTCATAGACCATTCGCACAAATCTTTATCATAACCTGCCATAGCAGGCTGAGCAGCACAAGAAGCTAAAG
CAAAAATTGTAGCAAAGATGAATTTTTTCATGATAATCTCCTCAGTAGTTTATGTTTATATAGTATCTCA
ATTTCCAACAAAAGTAAACAGTTATTTTAAAATTTCTGCGTAATCACATGTTACAAACTGTTTCTCTAAC
TTGACGATTTTACGAAAGTATCTTTTGCATTGGCGAATCTGCCTCTTCGTAGGGCGAACAGCAAACTTAA
TAAATTCCACTCGACCAAATGGAGGGCTTTCTTCTGCTGGAATATCTAACACCAATTCCCACGTATCTGC
AATAAGTGCTTTGAATTGCGTATTTTTCCTGACGTTATACGGAGTAGGTTTAAATAAAACAATATGCATA
TTATCCTCGGCAATCCACTTCACATACTTTCTTGTCATCAATGAAAGCTTTAACTAATGCTTTATTAACT
TCAGCATATTGAGTAGTAGCCCATTGAACGTCATCTTTCATCATTGTGATTTCTTTAGTAAACATGCTTT
CATTCTTAAACCACCCCATAAAAACTACCTTTACCAATTCCATAACAATCTCCTCATTTAACCGACAAGA
CTACTATACCATAGTCTTGTCAGCTTGTAAACTAAAATTTTAATTCATTCGCCAAAGCATCTAACTGAGC
TCGAGTCGATTCATTTCTTTGATAGCGATTCTGCTCAGCCTGAATCTGTTGTGAACCTGCTACTTCGTTC
ACTTCAGTTGGAGTAGAATCTTGTTCAATTTCTACCCATTTTTGATTTCCTTTTTGAACACCCATCAAAA
ACTTATTCCACTTATTCTTATCACCATATCGTGATTTGATTTGCTTAATGAGTTGTTGTTCAGCAGCTGC
TAGCTCCTCGGTTTCAATGACCGCAAGCATAAAATCGGCTGTTGCTGGAAGACCGGCAGATTCTGCAATA
TCGCTCATGTTAACATCGGAAGAGTCCCAAGCTTGTTTACCAACCTGTGCTGCAGTCCAAAGAACAGTTT
CGGTTTCAACAGCCAGAGCACGTAATTCCTCTGCAATAGCTTTAACAGTTGTGTAACTATTTTCTGAATA
AACTCTAATGCGGCAAGATTTACAAATACCTAGATAGTCGACAATAATGATTGTTGGAACAAAATTCTTC
TTGAGCTTCAATTCGTTTAAAAGTGATCGAAATGTATTAGCGTCTGCTCCACCAGTAGGATACTGTTTAA
CGATTAAACGACCAAGAGTAGATTTCTCACGCCATTTTTCCATTTTTCCTTTATACTCAGCGTAAGAAAT
ATGCCCATCATCAATGTCATCAAGAGAAACATCAAGCATATTAGCGTCAATACGTTTAGCACAGACTTCT
TCTGCCATTTCCATGGAAATGTAAAGAACATTATGTCCGAGCTGTAAATAATCTGCCGCTAATGAACACA
ATCCTAATGATTTACCAACGTTAACGCCAGCCATTAAAACGTTCAGTGTTCCAGTTTCAGCTCCGCCTTT
CGTAATTTTGTTCAGAATTCTGAGTTTAAATGGAACCTTACGAGCTTTATTCATATAAGATAGCCAACGT
GCTTCGTAGTCATCCATCCAATCATGACCAACGTAACTATCAAATGAAATTGATAATGCCTGGCGCATGA
TGTCAGGAATAGCACCAACATCCGGCATTTTCTTATTTCGTTTTTCCGGAGGAAGCTCAGCATTAGTTTG
AATTTCGATTATTTTAGACGTAGCATTAAACATCGCCCTTTGCTGAACATATTTTTCTGTTTCTTTTACT
AACCAGCTGTGGTCTTCCGGAGAATCAGCCAGTTTTGAAATAAGTGTTTTTACACCAGAATATTCTGTTT
CAGTAAATGAACTATTTTCTAATGCAACATTTAACGCATTAATAGATGGAACGCTATGGTACTCATTAAC
ATGAGATTTAATTAATTTGAATGTATTTTTAGCTGGACCACTTTCAAAATATTCTGAATCCATATATGGC
CAAACTTTTGAAAAATAAGCTTGATCAAATATGAGATGAGAAAGAATAATTTCTACCACACTTACTCCTT
AAAAGAATTTAAATTTTTTCTTTGACCTTTTATTAAATGCATCTTGCAGTTGCATTGTAATACATTTTTC
TACATGAGGAGCTAACTCAGCTTTTCTTTCTTGGTCAAGAACAGCAAAGTCCATTACAACCTTTCCATCA
ACCCAATCCAGTTTAGTTACATACACTATATGCGTAGAACCATCTTCTAGTTTAATGACAATCTCCTGGA
TAACATTTTCCATAGCAGATTTAATTATCTTAAGAGACTCATTAAAAAGACGTTCTTTTCTTTCTTCTTC
CCCCTCCGAAGAGGGGGATTCATCGATAATTTCTAGATCTAAATCTAAATCATCTTTATTCATTAAATTC
TTCCATATCACTTAGCTGTTCGAGGTCAGTTTCTAAATCAGCAGCTGATTTACTTTTACTTTCTGGAGAT
TTAAATTTTTCAACCTTTGAGTTAATCAATTCATCAACTTCAGCTTCAACAATTTCATTACTATCAATAG
CACCTAACTGATAAGCACGTTTAATAGCATCTCGGAATGGTTGATGCTTAAATAAAGGACCCCAGAATGT
AGTGCAGTTGGTATCTTTTGCACGCCAAGATTTTTCTTCGCGAATCATCTCGCCAGTTTCTTCGTCAAGA
AATTCACGAGCATACCAGCCATTTTTAGGTTTTACCACGAATCCTAATTCTAGAGCCATATCTAACAATC
CAGAATAAGGATCGATACCACCGTCAAATTTAACATCAATAAAGAATTTACTTTTTTCTTTAACGGTACG
 
AGATTTTTCTACATTTAGAACAAATTGATACCCCTGAAGATCAGAACCATCTTTAATCTGGCGTTTACCG
ATAATGAATACGGTATCAGCCGAATACATCGGTCCAGTACCACCTCCCATAACTGTTTTACTAAACATTT
CTTGTGTTTCGTATGTATGGTTAATAGCAATACATGGAATATTTTTAGTACTAAAATAGGGAGTTACGAT
ACGAAATAAGCTTTTCATTGTTTTAGCTCTAGTCATATCACTAACAACTTTTTCATTTAAAGCATCTTCA
GTTTCTTTCTTAGAAGCTAAGTTACCAAGTGAATCGATAAAAACGACTACCTTTTCGCCGCGTTCAATTG
CATCCAATTGATTAACCATGTCAATACGTAATTGCTCAAGTGATTGAACCGGAGTATGAATTACTCGTTC
TGGATCGACTCCCATAGACCGCAAATAAGCAGGAGTAATACCAAATTCACTATCATAAAACAAACATACT
GCATCAGGATATTGACGCATGTAAGATGACACCATTGTTAATCCAAAGTTTGATTTAAATGATTTTGATG
GACCTGCCAAAATTAACAGACCAGATTGCATACCACCAGTAATTTCACCAGAAAGTGCAATATTCATCAT
AGGAATTTTTGTTCGAACTACATCTTTTTCATTAAAGAATTTAGATGCTGTTAATTCTGCAGTCAATTTA
GAAGTAGAAGCTTTAATCAAACGAGATTTTAAATCAGACATATAATACCTTATAGTAGTGTTCTTGTTCC
ACGAAATACTTCGAGCATTCTTGCATAAGATTTATTTGGAAATCCAGCTTCAATTGCTAATTTTTTAAGC
TTAATAGCACCGGATTTTCCAGAGTTTTCCCATATTTCTTTAATCTTTTCAATGTTATCCCACATTATTG
GGTCACGTTTATGTGATGGTTTATTTCTATTATAACCATATGGATTATTAAGCAAAGCTTCTTTTCGTTT
TGCTTTAGTTTCTTCTGACTGCTTTTTGCCTAGCTGGGCTTTCCGGCAAACATCACTAAATCCAAGATGT
TTTGGTTTTCCTTTTTGAGCTTTTGAAATTTTTTCTTTGGTTTCTGATGAAACTCTATAACCAATTCTTC
TACCACCCTCTCCACCAATTTTTAAATTATAAGTCATAGGATCATTTACCACATCTATTGTTACTAATTC
TCTTTCAGCATCACGGGCTGATTTAAAATCTTTATAAAACCCAAGAATGCTTAAATTAAAATTTTTCTTA
CCATACTTTTTCTTGGCTTGTGCTAATAAAGTTCCTGATCCCATATAACCATCATTTAAATCATCGGTTG
AATGAGTTCCATAGTAAATTTTATTATTAACTAAATTAGTTATAACATAAGTATAATTATATTTCTTTTC
TTTATGTCTTTTCATGTCATTAATCAACTGCTATTCGATGAACTTCTCTCTTTTCTAATCAATATAACAA
AAGAAGTCCCAAAAAGCAAACAGACCTAAACCGATAATAAGCAATAAAGGTCCTAACATTTATTCCACCG
GTTAAAGATAAATAACTTTCTAATAATAGTTCATAATTTTTATAAATCAATAGCTTTTTTGAACGCATCT
TGCCATTCGGCTTTCTTTGCACGGGTTTTATTTAAAATATCATGTTGAATAGAAAGCATCTCTTTACGCA
AAACATCACTGTGTTTTAACTCATTGACTCTATCAATGAGTTCAGCACGATTATTTACATAAAAACGAGC
ATCATTAATAATTCGATGTTTGGTATCAAATTCTTCGTCAATTAGCATCACTGCATCAGATGCCATTGTT
TCCCAGACGCGTAAGGTAATAAAGTTGTCATTATAATTCTTGTCACCAATAATTAATGCAGCAATAGCTT
GACTATTCTTTTCAGATACCATGTTCATAGGAATTTTTCCAGTGAACACCGGAGCTTTGGTCCAAGGATA
TTTAGGATTTTTAAACTGTTTTTCTCGTGCATTGCCAAAAAACTCAATATTTAAACCGGTGTCAAATAAG
AATTCTACCATCTTGGATTCGCGTTGACCGGACCGAAATGAACCGCCATAAATAACATCCAAAGTTTTCT
TGGTAGGCTTAGATAATTGAAAATCGTTCATATGAATTTTATATTGTTCAATAGGAAAATATTCAAATTC
AATAACATTATCAACTTTCTTATGCGCAGCCTTAGCAATGTCTAAATTTATACCTTGGGAAATCACTTTA
ATTGGTGATTTAATTAATAGCTCTTCTTCAGTGTACAAATATGCCCATGGTCTATTTTTAACATTTGGCC
AAGACTGCGAAAACGGCAAACGTATATCTGTAAATAAATAATAAATTTTACTTTTGTATTTTGCCATAAA
TTTTTGCGCAGATAAAATTGCTAAATTAGGTTTACCGCCAAAAAAGTTAATAGAAGAATTAACAACTATC
AAACGGTCATAATCATTAACATCTACTTCATCAAAAGATTTAGTGTAAACACCATTTTTAAGAGAAATAA
TGTCGACATTAAGACCCATTTCAGAAATAACTTTAAAAAGATAAATAGTTTCAGAAGATGGAACAGTTTT
AAAATTAATAACATTATTACCCATATTAATTATAGCAATTTTCATATTATTCCTTTTATGTTAAACGATT
AAGCGTATTTTCCTACATAATCTTTTTTCGAAATATGTGTTTCGCCAGTTTTCCACCAATGATCAACCAA
ATAAAAATGACGAGAATACACATGTAGGCTTCCAACATTCCATATAATGGAACCTGCTTTATACTGGCGA
GTTGAATCACCTGCATTCAAATCAGATACTAATTTATCTAATACGTATTTTTGCCATGCATAATCATTAC
 
GGAATCCGAAGACCACGTCATTTGAGCGCATGTTAACAACCGCATTGATTTTCTTGTCACGAATCAGGTA
TTGTACTGTATTCGTGCACATGAAATCTGACATACCATCTTTATTATAGTCAAACTGCATAGATGGACGA
GTATAAATCATGATACCACGTCGAGAATCAGGATTTTGACCAAGTTCAGCTAAACACATGTCATACTGAG
CATAGTTATCTTCTGACCAGATAGCCCAACCATAATTCGAGTTAATTTCACCTTTAGAAGATGCTACTTG
TTGCCAAATCTTCGGTGTTTCACCCGGAATATCTTTAACGAACAAGCTTTTAGATTTATACCATTCAAGT
TCACGCTGAATGTATTCATCATTAAGAGCGCCAAAAATAAACGGTTCATCTGCTACAAATGATGCGCCAA
TAATTTCAATAGTTTTAACACCTGTTTTATCAACTACGAAATCTTTTTCTTTTAATGCAAGCCCCAAATG
AAGACGGATTTCTTCAACTGTCATAGAGTCACTAATCATTTAAACCTCAATTGATACATTCATATTTAAC
TTGTAACAGTAATAAACTCCAACCTAAAATAATAGTTGGAATCATAAGAGGAACCGTTACACTATAGTAT
ATACTTATTATAATCATCAAGATTAAAAGCAATGCTGCTATAATTTTGCTTTTCATTCCTTCTCTCTGAT
GATAATTACCTGATTTGGCTGCGCAGACTTTTTAGTTTCACCTGCAATTGACCAAATAAATGTAATAAAC
CAACCAATAATTGACCAGTTAAACAGTAAAGATGCGAAAAAGATTCCTACTGTCGATTTTGACCCACGCA
TCAAGGCGATAAACCATGGAAGCATGTATATAATAATAGCCAACACGCCTGAAACTAAAACCATAAAAAT
TGAACCTGCTACTAAAGTTTCCATGTTTTCCTCACTTAGGTCAAATTTTTTACACATGAATTATAAGAAT
TCACTACATACTCCATCGGAGCGTTTTTACCTGTACGCCACTGGTAATTATTAGCCCAATTTGCCCAAAG
CTCAGCGCAGTAGTTTTCAATTTTTTCTTCGCGTGTAATTACATCTGAATTACGATATGCTTGAGCAGAT
TCATCTGGACGAATAGCTTCGTCAAAATTCGCCTGCATTTGTTCTACTGTCTGTTTTGGAGCTTCTTTAT
AACACTTGACATTAGGATTATAAAATTTGCTTGAACAGTTTACAATTTTTCCTACATCAGACTGATTTAC
TACCGGTCCTTGAGCTACACAACCAGCAAGACCTAATGCAATAACCAAAATAGCGATTTTCATTTCATTC
TCCAAATCCGTATCAGTAGTTGATAGTTGTATAGTACCATGGAAGAACAGTCTTGTAAACAGTTTTGTGA
AAAAATTTTTAGGGAATCCAAAGGGTCCAGAATCATCTCTTTTTCATAAGTATAGATTTATATTACTTGT
ATGAAAAAGGGACCTGGAGGTCCTAGATTTATTCTATCAGCCAAACAGGAAGTCTAACGAAGCTTTTTCT
TCATAGTCCATGCCAGCCGATTCACACATACCCGCAAGCGGTTTAACAAACGATTTTTGGAACAAAGTTG
AGTGGTCAATCCAAGATAGCACATCAGAACGAATTTCTTTTGGAAGTTCTGTACCCGATGGCCAAGCAAT
GCACTTGTCACCAAATGGATTTCCTTCACGTAATGGAAGAACCATTACTTTATTTCCATCCAAAATTGGA
GCTACACCTAAACCGCTAACAGCTCGACGATAAGTTAGCACACCACGAATATGGAACGGGCATTTAAATC
CTGGCCAACCTTTATCATCATATTTCGCTATATCGTTCGCAGTTTTTACTTCAGCAATAACTTTATAGTC
AAGTTGACGATATTCTTTCTCGAAGTTCTTGTAGTATTCTTGGACAGACTCTTCACCTTCCTGAAGAATA
CGACGAATACTTTCTTCGAGAGCTTCTTGCACTGCTTTTGGTGTTGAACTCTGCTGAGTTTCCATACCCA
TGATTTTTAGATGCGGTTCAGCAAATCGCTTATCTTCCATATCATAAACGTTCAGAGCATAACGCTTTTT
CGCTTTCCAAAATCCACCAACGCCCTTTGAACCAAGCGGAGGGCAAGAAATAGCTTCACGGTCCATATGC
ATCAGATGCTCGCGGTTATTCATATAATCACATAACTCACGATATGCAACATCAATCATAGGTTCCATCT
TTTTCTTACCGAACTGATTCATGAATTCAACCAAATCGTTCTGCTCTTTGAATCGGTCAAGACCAACTTT
TTCAATAACTTTATCTACGCAAACATATACCGAATCAGTATCACCTGCTGCAATGAAATCTTCATCATTA
GTTCCGCATACTTTATTCAGATATTCATTAATTTTACGAGCAATCCACTGAATACCGACTTGGCCGAAAA
TTGTGATAGCAGTAGCATTTCGCAAATCATAGTAACGGAAATGAATATTACCAAGAGCACCATAAAGACT
GTTAATGAGAATTTTACGGTTCAGCTGATTTGTATTAGCAAGTGTAGCTGCTTTTTCACATTCTTCAATC
AGACTATTGAGAACAGATTCGGTGTAATTCGATAGTTCATTTAAGAAATCATCACTGAACTTAACATATC
GTTCAACTTCTGGTTTAGTTGAACAAGACCCTGCGCCTTTCATAATAATCTTTTTAATAGCTTCGGCATT
CATTTCTTCAGCGAACATTTTCTTTTTCCAGTCTTTACGCTGGAAAAATACTTTAGCGATTTCCTTTGGA
ATGATACCTTCTTGATGTTTATCATACATCCATCCATTCGGAGAACAAGAATATTCATCACTCGGTTTAG
 
GAGCTGTTCCTGCGATATATTCATGAATTGGATGAACTTTAAACTGACCACGAATAGTTTCAGGACTAAT
GTTAACCTGGCGAATAATGCTCGGATACAGAGACGTCAAGTCAAAACTCATAATGTATCGACGTGCAATT
GGTTTAGGTTCAAACACAAATGCACCCGGAAAACTCTGTTTAACGTGCGAACCTTGTTGAGGAATAACCT
TATGTTCACCTTTCAATGAGTTAAAAATAATAGCATCCCAAGTTTTAATAGGACTCATTACACCAGAAAA
AGGCATTTTAGCGTAATAAGACATACTTAAAACTAGATCGATAAACCCACGAATTTTATCGATTGCTTGA
ACTGATTCTACGTCAATGATGTTATAACTAATGTATCGTTGATGATTAGTCTCACGAAGTTTATTAATAG
GACCGTCGTATGGTAATTTACCTTTTTTGGTTTCATGTTGAGCAACTGATTCCAAAGAGAATGACGGCAA
ATTAGTAAAAGCGAATTTCTTGTACAAATCTAAATAATCAAGAATAGATACGCCATCAATAGAATAAATT
TCTTTGCTACCGTACATATTTTGAATTAGTTTAGATTTTACCCGACCGATTGGAGAGAAACGTTTCATAC
TACGTTCACCCAGAATCATTTTAACACGATTCATGATATACGGAACGTCAAACCCCTCAATATTCCAACC
AGTAAAAATAGCAGGTCGTTTCTGTTCCCAAAGATTGATATATTCCATGAGCATATCACGCTCATTATCG
AATGGCATATAAATTACTCGGTCAAGAATTTCTTGAGGAACTTCATCACCACCTTCACAGTCAAGCTTAG
CAGCTAACTTTGCATCCCATTTTGATACTGAACCGTACATTGAATTCAAAAGGTCGAAAACATAAAAACG
ATCGTCAATTGAATCGTAATGAGTGATAGCATCAATTTCATATTCTGCTTTCATTGGGTCAGGAAATTTA
TCACCAGTAACCTCAATGTCACAGTTAGCTACACGAACAAATTTTCGGTCATAAACAATTTCTGAACCAT
ATGTATCACTTATATAAGCGAGTTTAAAATCGTTCATACCGAGAGCTTCGAGACCGATGTCTTCCATTCG
CTTCATCCAATCTCGAGCATCTTTCATTGATGGAAATTTTTGAGGAGCGCAGTTTTTACCATAGATGTCT
TTGTATTTTGACTCTTCCTTACAATGCCTAAACATAGTTGGAAGATATTCTACTTCACGGGTACGTTCCT
TTCCATTTTCATCAATATAACGTTCAACAATGTTATTTCCGACTGTTTCAATAGAGATATAAAATTCTTT
CATAGATATTCCTTAGTTTATAGCCCGAGTTATTAGGCTCTTGATATATTATACTCCAAATAAGGGGCCG
AAGCCCCTTGCTTAATTACCAATCGTATATTTAGGAACGAGCTTCCATTCATGTTTTTGTTTAAAAGAAA
TAACTCGGAAGTTATTAGTTAAATCTTTCATAAAAGTTCTTTGACCAGGAACGATTTCAATTAGTCCCCA
ATCTTCTAATAGCCATGCAATCGAATCACGACGAACTTCATCTTCTTCTGTCATTTCAACTTGACGACCA
TCCATACGAAGCATTTCTTTAAAATGAACGATATAGTATAGTCCTTTTTTCTGAAGAATATGACAGGACT
GATATAGAACTTTATCTTTATTATTAGCAATTCCCATACGAGTCAAAGTTTCTTTTACTTTCAGAAAATC
TTCAGGTTTTTTAAGAGTAATTTCAATCATTTTACCATTCCAATGCTAGTTTTTTGAGTTGTTTCTGTTC
TTTTACGTTCTTAGTCACTTCTTTCAAAAAATCATCCGTGACTAAACCTTTTAGTTCTTTTAATACTAAA
GGAAGTTTTCCATTTTTAGTAAGAATTGATTTATAGTTAATTGCATCATTTGTATTAACTTGATACCGCT
TAGCAAGTAACTTAATAATCAATACTTCGGTGGAATCTTCAACCAGTTTTGCCCATTTACCATATCTTTT
ACCACGAGGAACTGCAGCCATTAGATAATTAAAATGAGCTTCATCACTTAAGCCTGATCCAATTAAATTC
ATAGCATATACAGCTGGCATACACTCTGGAAATTGTGATAATGCATTTTCAACCATGAATTTTGAATAAT
CTTTTTGAGCAATAGAGCATTTAGTTTTATTATTAATAGCTCCAATTATTTCAAAAAATTCATTTTCTGC
TTTTTCTTTAAAAGAATCAGCAGCGGATTGGACAGCTGTCCAATCTTTTGAATACCAAGCAACTTGATGC
TCGTTTAATTGAATATCATCTTTAAATAAGCTCATATCACTTCCACTGCATTTCGCATGCTAATTGAATG
AAAAGATAAGCTAAATGCAATTCAGTATTAGCTGCAATACCATGATACTGATTATTTTCGCCGACAATTT
CGTACATACGAATAATACTTTGTGGAGTTACACGTGAATAGATTTCTTCGGCAAGTTTACCCACGAACCA
CGAATAATCAGCCGCATATTTTGGTGCTAAAGCTCTGAGTTGTTTAACATCTTTATTTTTGAGAGACTCA
AGAACATCATCAATAGCACCACGATCGTTAGTAACCAGTGATAAAATACCAGCATCCAAAACACCTTTAG
ACGAATAACTATCGAGCTCGCCAATAGTTTTACGAAAATCAGGAAAATTCTTTTTAACCAAAGCTGCTAC
AACTTTCATATCAGCTATAGCAATTCCTTCATGCTTGCAGATTTCAGTCAATCGACGAATCATCTGCTTC
ATCATTTCAATTTTATCTTCATCAGTTGGTTGACCGAATGTAATAACTCGGCAGCGTGACTGAAGCGGTT
 
TAATAATACCATCAATATTATTAGCAGTAATAATAATACTACAGTTTGAACTATAAGCTTCCATAAAGGA
ACGAAGATGTCGCTGAGACTCTGCTAACCCTGAACGGTCAAATTCATCAATAACGATTACTTTTTGACGA
CCATCAAATGAAGCGGCGCTGGCAAAATTAGTCAAAGGACCACGAACGAAATCAATTTTACAATCTGACC
CATTCACAAACATCATATCAGCATTTACATCATGACATAATGCTTTTGCTACAGTTGTTTTACCTGTTCC
TGGAGAAGGAGAATGAAGAATAATATGTGGAATCTTACCTTTACTTGTAATAGATTTAAAGGTTTCTTTA
TCAAAAGCGGGAAGAATACATTCATCGATAGTAGATGGACGATATTTCTGTTCAAGAATGTGTTCTTTTT
CATTTACAGTAATCATAATTTCCTCATTCAAGTTTTAGTGTAAATTATAAAGGCCGAAGCCCTCTATTAA
AAATCGTGGGTAGAATCAGCTTCAAGAGCTACCACATAATTCGCGTGTTCACCTTCAAATTTAGCAGCAC
CTTGTTTACCTTTTGCCCAAAGCAGAAGTTTATAATTTCCTGGTTGCATTTTCATATTTGCCATATTGAT
AATGAAATTAAATGTATTTTCACCATCATAATCACCAAGAGTCAAAGAATATTTAACACGGGTCAGAGCA
GAATCTTCTACTTTATTAAAACCGTTAATTACGATTTTACCTTCTTTTACCGTGATAGCAATTGTATCAA
TTTGCAGACCACGAGATACACGCAACAGCTGTTGAAGGTCTTCAGCTTTAATTTCAGTAACAGCAGATGC
TACCGGGAATGGAATTGGTTTATTAGGAGCAACTACTGTACTCGGATCGGCTGCTGGCCAAAAAATTGTT
GAGCGGGCATCAGCAATTTTAATATTTCCATCTTCTGACTGGGAAATTTCTGCATCATCATTAACTAAAG
ACAGAATACCGAGAAAACCGTTCAAATCGTAAATTGCTACATCAAAATCAATAACGTCAGAAATATTTGC
TTCCGCATAAGTTGTACCATTAACTGCGCGAGTCATAATAAATTGACCGGATTTAAGCATAATACCAGAG
TTAATAGTAGCGAAATTTTTAAGCAGAGCAGTAGTATCTTTAGACAGTTTCATGTAATTTCCTTCAATTC
AAATGAGATTTAATTTTATAACTAATTTAATAAAGCAATTAACGATTAAAATCAGCCGCAATTGTTTCCG
CAACAATTTGAGCAGCAACAATTAGACGTTCATCTGCATTACCGCAATAATCATCTTCAAGGCGTTCACC
ACATGAAGTCATAATAAATTTAGCACCGGCGTTTAGGGATTCTGTAGTATGTTTGCGCATTAGTTCAATC
CATTTATTACTTACTTCACGATCGATAGCTTCATAATACGCATGACGAGCAGGTGCAGATTTAATTTTGT
TCTGAATAACTTCCATTGCGTTATCAGAAAGAGACAAAACCCATGCTCGACGAATTTTATTTTGGTTTTG
TGGATTTGATTCAGAACGCACGTGTTTTGGCTGAATATCTTTTACATCAACAGTATAATTCACAGTAATT
TTAGTCATAATACACCTTTAGTCATAATAATCAGTAACAGTCCAAGCTTCATTTCTATTGGACATTATTT
TTGTATATTCTGCTTTAAATGCATTCCTAAGCATAGATTCAGTAACTATATGCTCTTCATTAGAAAAATT
ATTTCTCAGAATATATCGTTTTATTTCAGGAATAGTTAATAGATGCTGTCCAGTTGAATATTCCATGTTT
TTCCTCCATAGAGATTATACTCTAATAAATTAAAGCATAATCTCTTATAAATTAAACCATTACAGTAAAT
CGACCAACTTTCTTCATTTGAAGATGCTGACCATATTCTTGCGGGTCATGGTCTTTATGCGAAATTATAA
AAACGTTAGTGTTTTTCATTGAATTTATAATATTAGCTACACCTTTAATACCTTCGGCATCAAATGACCC
ATCAAACACTTCATCAAGAATTAATGTACTAATACTAACACCAGATACGATAGAAGCAATATCACGCCAA
GTAAATAAAAGAGCAATATCGATTCGTGCCTTTTCACCTTCACTAAATGAAGCATAACTAAAATCTTCAC
GACCACGGGATTTAATTGTCTCATTAAATTCTTCATCTAATGTAAACACATAATCCGCTTCCATTATTTT
AAGATAATGGTTAATCTGCTTATTAAATAATGGAATGTACTTTTTAATAATAGCACCTTTAATACCAGAA
TCTTTGAGCATATCAGTCAAAATTCCTCGGTGGTATTTTTCCATTACTAAATTAGTTTTTGTCTTAACAA
TTTTATCAAGTTCTTCTTGAAGCAGTGCTATTTCATCAGCATGGTCAATAAACTCAGAAGATGCTTTTTC
TATAGCCGCTTTAACTTTTTTAGCTTTATCTACTGCTGCGATCAGAGATTGCTTTTTATTGCGAATATCA
TTTGCCAACGACTGCTGGGTTTTAATATTATCTCGGTATTCATCAACAAGAACTTTTAAATTATCACGAT
GTGTTGAAAGCTGTTCAAACGAATGTGTGCATTCAGAAACTTTATCTTTAATTTTAGAAACAACTTTATC
ACCGGAACTCAATTGTGACAAACAGGTTGGACATAATCCACCTTCGTGATACATATTAATGACTTTATTA
TACGAGTCAATTTTTGATTTAATTAAAAATGCTTCTTGACCGATTTTATTAAATGCATCAGTCGGGTCTT
CGTCCAAAACAATATTAACTAATCTTTCGTTAGCTTCTTCTATTTCCGATTTTAGCGTTCTAGCTTCTTT
 
TGCCAAATCATCATACATATTTTGTAGACGAGTAAGGTTGTCACCCGTTAATTTTTTCTGGCGTTCAACA
TTATCATTATATATTTTAATTTGTTGGATAATACTATCTTTTTTAACATCAAGCACTTGGTTCTGCGAAT
TTAATTCACGTATTAGTGCTTTATTAAGCTTATCCATTTCAGCTAATGTTCCTACCTCAAGCAGGTCTTC
CACAAGCTTTCTTCGCGCAGGGGTCGACAAACCCATGAAAGGGGTATACCCTGCTGTACCAAGGACAACA
ATCTGCTTGAAACTGGCATATGACATTCCGATAAGCTGTTCAAATTCTGCTTGGAAATCTTTACTGCTGG
CAGATTCATTAAGACGTGTACCGTTAACGGTGATTTCGAAAACGTTTGGTTTTTGTCCTCTTTTGATATA
GTACTTTTTCTCATCATATTCCATCCACAGTTCAACTAAAAGTTCTTTCTTATTTGTGCTGTTTATTAAT
TGACCTTTCTTTACATCGCGAAATGGCTTACCAAAAAGCCCAAATGTGATGGCTTCTAGCATAGTAGACT
TACCACCGCCATTTCGTCCAGTAATAAGAGTTTTTTGAACCTTATCTAATTGAATGTCAATCCCATTTTG
ACCAACTGACATTATATTTTTATATTTTACTCTATTAAGTTTAAAATTCTTCACAAAAGATTCCTTTTAA
TGTATCTTTTAGACCATTCTATCATATCATCATAATCTAAAAAGTATTCATCAAATTCAGCCATGCAAAC
AACGCCTTGTGCTGTTTTTGATGTGATATAAATTATTCCAACATATCTAGAATCTTCTTCGGTATAATCA
ATATTTGCTATGAATTCATCATTAATGTCAAATGTCGAAAACTTCACAGTATGCATCCTTAATACAAGAT
ACAGCCATATCTCGTAATGATTTAGGTGTGTCATTATCTAAAATGTTGAAGTTAAAAGATACAGCCCAGT
CAGTGCAGACAGTAACCTTTTTAATACAATTATCTTCAACCTCTAACGGTTCAAACCAGTATTCAATAAT
TTCTTTATGACCAATATCATCTTTTACTTCACATTTAAAATGCTGACTCATCATAACATTTTTAAATTCA
TCAAAAGTCATTGTGTTGCCTCTACATATAGCTGATTTGCATATTGAATAAGTGCTTCACGGTCAGAATC
AGTGATGTCTGGAATTGCATTAATATACTCTTCCATTAATGTCTGAAGCGATTGAACTTCAACTTCTTCA
CTGTCATCTGACTCGACAGAGTTATCAATCTTTGACACAACTCGTAATGAATGCACAACTTTTTCTAGTT
CAGATTCGAACTTCGTCAGATTTTTGTCTACTTCAGTTACTATAACACGTACTGATAGATTTGTAAAATC
TTTATAGTCAATTTTTCCTTTAAATGGATAATGAATTCTACGATGCCAGGTAGTATTGTTTGGAATAAAT
TCCGTTCGTTCTGTTTCTGTATCAAACATCCAGAACCCACGAGGGTCATTCTCGTCACCTGCGGTTAGTG
TCCATGGTGTCCCAATATATCTGACGTTTGCAGCCTCAGAAATAGTATGGAAGTGACCAGACCACACTTC
TTTATAAGTCTTAAGGAAATCGGGTTCAAGACCATGAGATTTCATTCCTTTATAAAAATAAAATCCATTC
AGTTCCCAGTGACCAACACAAAAAGAAGCAGATGAAGTTTTGATATGCTCAAGAATTTCACCAGTATTTT
CTTCGCACATCCAAGGAATCAAATCAATCAAACACCCGTCAAAATCTACTGTAGTAGGCTTATCATACAC
TTTAACATTAGGATATTTAGCCAAAAGCTCAGTAGAAGCATTTGGATGCATTACATTTTTATAGTGGAGA
TCGTGATTTCCTACAATAGTGTGTAATGTAATTCCAGCATCATCAAGCGTTTGAACTATTTCACGGGCAA
ACTCCATAGTTTTATGTGTGATCGCTTTTCGCACATCAAAAATATCACCGTATTGAATCCAGGTAGTAAT
TCCATTTTTCTTAGAATATTCTATCGCTTGCTTAATTCCATCAATTTGAATACCGCGAATCCACTCATCA
TCAGCTTTAACGCCTAAATGCCAATCACCTAAATTTAAAATTTTCATATATCAAGAACCGTCATTGAAAT
GCAAAATAAAATTATTGAAATAAATCCATCTGGAGTGCTAAAGAACCCAATCCAACATGCTCTAGTGAAT
AGATAAAATGCAAGAAAAAGTATCACATATCCAAGAAATATCATTATATCAAACTCCGTATAAAGCTAAA
GGGCCGAAGCCCTTTATTTTGTAATAATGTCAAACTGTTCTTTAAAGCAGAAGCTTGAATCTTGATGCTG
ATACAAAAATTCATATGCTTTTTCTCGCTCACGGTCATAAAGAGCTCGGTCAGATGACAGTTCTTTAATA
CGTTCAAATGTTGATTCCATATCATTTTCATCAAACCAAATGATACCGCTATCATGCGAGGTCAAAGGAG
TATTATCAACACGGAATTTTAAATTTTCGCCAGTAGATTTCCAAAATACCGGAATTGTTCCACATGCACC
AAGCTCGAGATGAGTATATTCGAGTGAGCGTTGTAAGTATTTCTGGTTAAGTTTACTCAACTGATATCCA
AAGCCAGATTTACTCATTCGTTCAAGCATTTCACTATTAATATAACAATCTAGGATTTGTGCCGGTTGAT
TCGGCGCGAGATTCATTTTATCAATCTCACGATTACCGTAATATTCATACGGAATACCTTTTTCCTTAAT
TGCAATAAAAGCAGGGGAACGTTCCAGACCTTCCATTACAGTGGATTTACCAGCAGGTTTTAAGAATTTT
 
TCATGAAAATCAAACATCTGGTAAAAACCTTTCCATGTAGTCGTACGACCAATCCAACGGTTGATATTCA
TGTTAATTTCAGAAACATCTTTCCAATAAGTTGACCGAACCTTCACAATATCCATAGGAGGCTGAAAATT
ATATACTGTCGGTGCTTCTTCAATATCATCAAACAGAGAAACAGTTTCTGGATACCATTCTTTCATCAGA
ACTTTATTAAAATCACCATTATCAGAATGGCTAAAAATAACATCAGCTCGACGAACAGTTTCTTCTAATC
CCAAATTTCGACGCAAAGAAAGAACAGAATGATCATGCTGATAAACTACAACACGAATAGAAGGTTTAAT
ATTATCTAAAAGTTTTTTATAGTTATTAATCGTAGCTTCTTGAACGGAAGTAGCAGGAACAGAATTAATA
ATTAGAATATCACAATCATTTACTAGCTTAAGTGCTTTATCGTATTCTTTAGCTAAAATAACTGGAATTG
AAAATGATTTGTGGTCATGAGAACTTGTACGAGTAAATGATTTATCTTTAGCATAAACCAAAGTTACTTC
ATGACCATTTTTAATAAACCAATCACGTTGCTCGAGTGAGAATTTTGTTACACCACAACCTTCAAGACCT
CGAGCCATAAAAATGCAAATACGCATAGTTTTCCTCTTTTCATTTAATAAATCATGTAAATAATATTTTA
TTTTCTATAAAACGCTACGAATAGGCCCAAACATATCCATAAGCAATTTTGCTTTTTCCAGATATGCACT
TTTTAATATTAGACATGCATCCTTTAACGGATACAGCTGCGTCTTTAATTCTAGGAAATACTCGTAATAA
TTTTCCAGTAGTATCATATTGAAATACTGGCACATTTCTTTTCTGAGTTATCCCACGTTTAGATTTAGAC
ATTTTTTGTTTAGTTGCATTAGACATCCTAGAGGGCTTTCCTATGTTATCAGAATAATAATATTTCCATT
GAAATCCTCCAGCGGTTTTCCTTTTACCATCTACACATTGTTTAATTGAAGTTGAACAGCTATATGACAT
ATCTTCTGCAGCATCTGTAATACATCTATATTTGCGAATAAAATTTCCATTTAAATCATATTGATAAATT
GGTTTTCCAGCATTTCGTCTAGCGTTAGACATGTATTTTTTATTATCATCATCTTTCCAGAACTCTTTCA
TGCTTTCCGATATTGAAGAAGATACAGCTGTTTTAATCCATCCATACATTTTATTATTTAGTCTTGTTCC
GTCAGAACTATAACACATCATACGAATAGCTAAAGCCAATTTAGGAAGTCTATAAATTTTAAATAATAAT
AAATGCGCGGTAAAATGTTCTTCTGGTGTCAAAAGAACTAAATTAGTTTTATCATCTGTACCACCCATAC
ATCTAGGAATTATATGATGTGTTTCAGTATAGTATGTCAAAAGACTTTTATCATTGCCTCTGTTTAGTCC
TTTTTCGATCAGTAAATTATATATGTTTAAATAATTCATTTTAGTTTATTTTACCAAAAAATTTATAAAG
CAATATAGGAGCCGAAGCTCCTATCCACATAATACGCCATACAGAGGCTCGTTAGAACTTTTAAATTTTA
TGCGCTTATATGTTATAGTTCCTTCTGCTTTAGCTTTATCATGAGCCTCTTTAAAGCGTCTCATCATTTC
CTCTCTAGAGGAACGAATTTTATTATAATCTATTTCAGAAGTCGGATGTTCATCTTTCACAGTTGCCACC
ATTTTTTGACTTGATAAGAATCAACCCACACTTTCATATTAGGGTCTGCTCTTACAATAGGAGGATTAAC
TTCTTTGATTGAACTATCACGGTATTCTTTTTCAGAAATTTTCACGCAAAGATGACCATTCAAAGATTCA
GTAAATCCTGCATTAATTTTAAGACGCTTGAATTTTACGTGTGTAAGCATCAATCATATCCTCAATCTGC
GATCTAGTAGTCTTCCAAAGAATACTGATGAGTTCATCGTTATATGGCTGTTTTAGAATATCCCGACTTT
TCTTGATAGCATATTCGTATTGAGCAAAATTATTATTTTCAGCTGCGATCTGAGCGTGCTTATAAAGACG
ATTCAGTTCGCGTTTGTTTTTAGACAATAACTTATTTGCTTTTCTTTCTGCTTCAAGACGGTTCTTTTCT
TCAATAGAAGAAATAAGCTTTTCCACTTCATCATTAATTTCGGGTTTATCAGTCATATTATTTCTCTAAT
ATAAAATAAAAATCATCATCTGTTAAATGATACCGATAGTTTAATTCTACACCATTAGATTTAAAAGCGG
TATCATACGGATTTTCTGGATCAATATCAATGTCAAGAGCTAAAACTTCCCTGAGATACATTTTAAGTAA
ATAGGGAATAGCTTCAACTTCAGGTATTTCTTCCAAGAATCCGGAGAGGTTAATCGTTAGCCTCATATAA
AAAATCCAAACTAGGAGAATCATCTACAACACTTTTCTTTTCAGCCCCCGGTGTTCTATAGGTTGATTCT
TCGTAATGCGTCATTTTATCATAGATGTCTTGAATAAAAGTTTCATCTACTAACGCAACCATATCGTCGT
CACGGCTGTCATAGACATTGTGAACGAAGTAACTATATTTCTTTGCAACTTCCTTACGTTCTTTTTTAAT
ACGTTGGACGAATGCATTAAAACAAGCTTGAGTTATATACGCATGTGGGTTTTTATATTTCGTTTCATCA
AAATTGTGAAGCCCCTTAATAGAAGCTTCTATACCATCTGCAATCATTTCTTGTTTCCAAGACTGGGTGT
ATCCTGAAAAGTTGAAACGTTTAGATAAGCCTTCTGCAATAAGCATAATGGCTAATCCGATAGTATCATT
 
CTGACGAACTACTTTATTTGGGTCTTTATTATTTGCTAATTCTGTTTTCCAATCAATAATAGCTTGTAAA
AGCTCTTTATTGTTTACGTAATTATATTTAGGCTTAGTTTCTGACATTTTCACCTCTTAGCTCAATTCAT
AGATCTATTATATCATAATATTTGAAGACCTATCTTAAAGCATAGAGGATGAATCAATTTCAAGCACTTC
ATCAGATTAGCCGCTCCAAGAGCTGCATCTAGTGAATCAAATTGGTCAACATATTCAATTAATTCGCCGT
AATTAGCGTATAACCACCATTGGCTAAATTCATACTCAAGGATGAATCCATTTCCTTCAATTTGAGTTAA
ACCAATGCCATTTGTATTTACTTCATACCCAGCGAGACGTAAATCGTTAATAAGAGCTTCGTTCATAATT
ATACCTTAGTAATTTTCAGGTCTGCAAATTTTTTCTTGCGTTGATTTTTCATGCGACGAATAGTTTTATC
GGAAATTTCATGCTTTTGATAAGCTTTAGATTCTACACCAAAAGCTTTAACATCAAATTCTGACAAGATA
TATTGAACCAACAATTCACGGACAGTATTGCGTCCAATCTTCTGATCATTCTGTTTCATCGTTTGATGAA
GCTCTTTTTCCCATTTATCCAAAATTTGAGGAGTTACAATATCGCCTTTTTCTAGCAAAGAAACTACTTT
ATCATATGCGTAAGAATTGATGGCGTTTTTAATTTGAATAGTCATACATTATCCTCAATTACGTTAAAAT
TTTATTATCCAAAAAGGCCGAAGCCCTTAGGCTAAACTTTTTGGCACCCTTCCAGCCTTCGTACATCATT
GCGACTGACAATGACAAAGCTCCTTCACATGCTGCATACTTATTATTCCAGAACCAATTTAGAAAAACTT
CATCCTCAATACCGTTGTGTTTCATGTTCTGGAAAAATTTGGCGCGTTCTTCAGCAATTCGCTGCGATAA
TTTAGATTCAGGATTCATTTAAATTTCCCAATTGCCATTTTCATCAATAAATTTAATCCAGTCATTTACT
GACCACTTGGTCGTATCGCCTTTTGGAGTAACATTTAAAGTGTATAGCCCTTGCTTAAAAAGCATGCGTT
TGATATTCATATTTTCCTCAGCTGTAACGATAACACTCGTTTGATTTACGTTTAGCAACTCGTTGAGAAG
TATTATAATCAAAATCATCATCAATGTAAACTGATTTTTTCAACTTTCTTACTTCACCGCGTAATTGACG
ATTTAACTCATCTTTAACTTCTGAATCAATACCTTTCATTCTACGCCATTTATCTGAGCGAAAAATGTTT
TCAACCATATCTTTATGACTTACCCCATCAGGAGCTTTACGCTTTCCGAAATAGTCATAATCACGCACTT
TTAAGTCTTTACGACGATACGTTTTACCCATGGAGTTTAATTTCCTTAGCAACTGAACTAAATACAGCAC
GATCACAAATCATACGTTTATGTAACTTGAGTATAAGATAGTAAACATCAAAACCATTTACATAAGTAAC
ACGAACAACATTACCATTCACGAGATAATACTGTCCCTTTTTAATCTCTTTATCAACCACAACCATATCA
ATTCCTCAAAGGTAATTCATATGTTAATAATACCACGGTTTGAACTTGTTGTAAACAACTTTGTGAAAAA
TATTTTAGGGAATGATAAGAAAGGAACGATAGCTTAGAATGGTAATATACAGAATGTGAGAAAGAAAGGC
CCAGAGGGCCCGTCTTAATCTTCTATGATATCTCTATCATATCCAAGTGAAATGAGAGTTTCTTTGAAGT
GTTTAATGTTCTTTTGTCTAGAATCATTAATGAAAATGACTGGATAACGAATGTTAAGAGATGTGAATCC
AGCGCGTTTAGCAAGAGATACAATCAGCGGACGATCATACTCAATCTTACCATTATTTGTAAGAACTTTA
TAGAAAGTAAAAGGAGCATTGAGCTCCTTTAGAAGTTTTGTAACTGATTGACATCCAGGACAACGACCTA
CTTCATCTGGAATTCCATAGACTTCAATCTTATTTTGTTTCACAACTATTCCTTACTAAGAGCAGCATTC
AGCTTGTTAGTAATTTTATCCAGACGCTCATTGAATTCACTAGAAGATAAGCCCTTTTCTGGAGAAATCA
AACTAATCACGAAAAATATAGCAATAAAAGGAATAAGGAAAATAGCTCCAACTGCCATAAACAGAAAGAA
TGTTACAGTTGTAAGAAAATCAGCTAAACCTTTACGAAATTTATACATTTACATTTACCTTTAATTGATT
AACCAAGCATTGATAAGCACTAAACTATACTGCGAATAAAATTCTGGACCAAAATGAAAATCATATCATT
TATAGTATCCATAATGTAATTCAATTTAATCATGTTTCCACACCCCATCGGTATTTGACCAAAGTCGCTG
ATTATCTGATCCTCGCCACAGCTTTTTGGTCGGAAGATTTTTCTCATACTTCCCATCAATAATAACATCA
ACATATTTAAGCATTTCTAGTTGTTTAATATCTTCAAACTTATATCCTGTCCACAACCAGATATCTTTCT
CCGGAAATCTTGCTTTAACCCAAGAAACTAAATTTGAAATCTCTTCTCGGTTCTGTGGATAAAGTGGGTC
ACCGCCGGTTAAGGTAAGGCCTTGGATATACGATTTGCTTAAATGGGACGCAAGTTCTTTAACGGTATTC
ATAGTGAATAACTGTCCGTTACGAGCATTCCAAGTACTACGATTATAACAACCTTCGCATTTATGCAAAC
ATCCAGTGACGAAGAGAACGACCCTACATCCAGGGCCGTTAACGAAATCGCATGGATAAATTCTATCATA
 
ATTCATCTTTATATTTCCAAGTATATCCCTTATGAATAGATTGAATTCCTTTGCAACATTTATAAACAGC
AGAATGAAAAAATCCTGCATTTCTTATTTCAGTTGCTCCAGTTAGTTCTATTGTATTTCCGTCTGGTGAA
TAGCCAATAACCGTTTTGGTGTTAAAGCGATTTCTAGAACTATTTTTGATATGCTCTTTATGATTAGTAG
ACAAAGTTCTTCCTGTTAGCCCATCAGATATCCTTTTTCCAAAATCATCAGGAAGAGTTACTTTAGACCT
AGCTATGCTCATTAGCTTCTTAGTTTTATCGCTTCTTTTTGCGCCACGCTGATTTTCAAATTTCTTAGCT
TTTGCATACGCGTATTGTGAAGAAGTAACTTTTCTATGCTTACTATTACTCATTATCCAATATGCATCAT
ACATCTTTCCACCATATATTTTAGCCAATAAATGGTGAGCAGTATAGTGGGCCTTATAAGTTAAAAATAC
TAAGTTGTCTAAATCATCTGAACCACCCATACTTCTTGGAATTATATGATGAAGTTCATACCCGGCTTTA
GTTTGACGGGGCTTAGATGGGTGTTCAGCAGAATTAACTAGATTTGAATAGATTCTGTCATAATTCATTG
GTGCTTAACCCTATGCATGATTTCTTTATTTTTACCGAGATTAAATCCGCGTTCGTTCGGATTTCCCAAA
TAACCACATGTTCTTCTTATGGTATTCATCTTTTTAGGATCAGTTTCTCCACAAATAGAACAAACAAATC
CGTTTTCAGTAGGAGTCATTTCATGGGTACTTCCACATGTAAAACATTTATCTACTGGCATATTAACACC
AAAATAATCTAAATGCTGTGCAGCATAATCCCACACGGCCTCAAGACCTTTTAAGTTATTTTTCATATCA
GGAAGTTCAACATAAGAAATGTGACCACCTGTCGCAATGAAATGATATGGCGCTTCACGAGAAATCTTTT
CAAACGGAGTAATATTTTCTTCTACTGAAACATGGAAACTGTTAGTGTACCAGCCTTTATCGGTAACATC
TTTTACGCTTCCATATTTTTCTGTATCGAGTTTACAGAAGCGATAACACAGGTTTTCAGCAGGAGTCGAA
TATAAACTAAAAGCAAATCCGGTTCTTTCAGTCCACTGTTTAAGATGAGCATTCATTTTAGTTAAAATTT
CTCGTCCAATATCACGACCGACAAGAATATTCAATTCGTGAATACCAATGTATCCTAAAGACACTGAACT
TCTACCGTTTTTAAATAACTCAATTATGTCGTCATCAGGTTTAAGACGAACCCCGAATGCACCTTCTTGG
TAAAGAATAGGAGCAACAGTAGCTTTAACTCCTTTTAAGGAACTAATTCTACACATCAAAGCTTCAAAAC
ATAAATCCATTCGTTCATTAAATAGCTCAACAAATTTCTGTTCATTGAACTGTGTTCCAATATAAGAATC
TAACGCGATGCGAGGAAGATTCAGTGTTACAACACCAAGATTATTGCGTCCATCAAGAATTTCATTGCCA
GTCGAATCTTTCCATACGCTCAAGAAACTACGGCAACCCATCGGAGAAACAGGAACAGATGAACCAGTGA
TAGCTTTATTGTTCTTAGCTGAAATAATATCAGGATACATCCTTTTGCTTGCGCACTCTAGAGCAAGCTG
TTTAATATCATAGTTCGGATCGTCTTTATAAAGATTAACACCTTCTTCAACGAACATAACAAGCTTAGGG
AAAATAGGAGTTATCCCATCACGACCAAGACCTTTAATACGATTTTTCAGAATTGCTTTCTGAATCATTC
GTTCAGTCCAGTCAGTTCCCGTACCAAATGTAATTGTTACAAAAGGAGTCTGTCCGTTTGAACTGAATAA
CGTGTTCACTTCATCAATGTGTTCAGTAGAGTTCGCTACTTCTCTACCCGTTTATATGAAACAATGTAAT
TTGGATAATTGTCATCTAAACACAAATCTTTAATACGACTTGGGTGGAGTTTTAAATCTTTAGCAGCATC
CACAAATGATTTATAGATATTGTCATTTATTTTAACATATTCTGGGACGGGATGAATTGTTATTTTTATA
TCTATAACATTTGGATGATTACTAACCTGTTCTTCAGAGCATTTAAGAAATTTTGCAGCTGATTTAAAAC
TTCTAAATTTATTTCCGGATTTTAAAGCTATAGAAACAGTCTTTTTAGCCGTTCTACTACCAATATTATT
TTTAACATGAGCTTCACTTCTACTATTATTTTTATAATGCTCAATCAATTTCTCTCGTATCTTATCTTTA
TGTTTTAAAGATAAGATTTTACCTTTATGGGCATTACTAAGTTTTTGCTTATGTTCTTCTGAATCCGGAT
ATTTGTTAAATTTATATCCACCTATAGATTTATTAAGAATAAATTCGTTATTAAAATATTTCCTAATAAG
CATTTCTTCATGTTTAAGGGCCGATTCATAAGAATCAAAAACTTGAAGAATTATCCACTTAGCTTTATAA
TCTTTAAGCTTTTCTTTAACAAGCTTAGATGACGAATTGTATTCTTTCCAATTTGTATCTTTACCATATA
TAGTTTTGAATTTTTTAAAACCTATATAAAAAGACTTATCAGGAAATCTTACCATATAGGTAAATGCTAC
AGAATTGGCAATTTTTAAGCTTTTTCTTAATTTCCATTTCATCGTTTCACCTCGTATTCATATTTATACG
AGGATAAACAGCTGCATGTCACCATGCAGTTCAGACTATATCTTCAACTCTTAGAGTTGTCTGCCGTTTC
GGGTCGCTTGACCCTACTCCCTTACATTCATCAGGGATAGTCGTTGGGCATTTACAGCTACTGCTGATTT
 
AGCAACGGATTGTCTCAGTGAGAGTTTCCCGTTTTAGGCAGATTTTACATGAGCTTGACTTACGTTAACT
CATAAGCTTGGAATGCATCGTATACGTCTTTTTCTGTTTTAGATTGAGCATAATTCAACGCATCAGCGAT
TTGCCATTTTTCTGCATCCTCAATATGTTTTGCATAGGTGCGTTTAACATAAGGAGAAAGTACTTTATCT
ACATTCGCAAAAGTCGTTCCGCCGTATTGGTGAGAAGCAACTTGCGCAGTAATTTGTGCCATAATTGCAG
TAGCAACTCCAATTGATTTAGGAGTTTCAATCTGCGCATTACCAAGCTTAAATCCGTTTTCAAGCATTCC
TTTTAAATCTACTAAACAGCAATTAGTAAATGGAAGAGCAGGGGAATAATCAATATCATGCACGTGAATA
ATTCCGCTTTCATGCGCTTTCATAATAAAAGACGGGACCATATTTTTGGCAATGTGTTTAGACACAATAC
CAGCCATAAGGTCCCGTTGAGTTGGAAAAACACGAGAATCTTTATTAGCATTCTCGTTTAAAAGGTCTTT
ATTAGTTTTATGAATTAATCCTTCAATTTCTTTTTCAATTGTCATTTTAAACTCTTTCTAAGCTGCTTCT
TGAATGAAGCTATTAATTGTGTTTTGGTGTCAGATTCATTATATTCAAATCCTCTTTGAAGCATCTCGGC
CATCATTTCCTCTTTTCCTAAACGAGAAAATTCCTTTGATTTATCTCCAACAAAGTTAGGGTGAATATTA
TTTTGGGTGTAATCGGATTTTAAATAAGTAAGTAAATTTTCTAACCATTCAAGATAATCAACACCTTGTC
CCTTTAAGCCAGAACGATTAAATTTATGCTTCATTTGACCTTCTGCAGCATTGCATAGATTACAAAGCAA
TCCACGCACCTTTCCTGCTTTTGGTCCATTTAATTCATGGTCATGGTCGAGGTGATTAGCTTGAACATCA
GGATTTAGTTCTCGTTGGCAAATTAAGCATTTACCGTTTTGTGCATCATAAAATTTCTGTTTTTCTTCTT
TGTATAATTTGCCAGTCAATAACATAATAAACCCTTACCTTAAATAGATAAGGGTATTTATTATTTTCAA
GTATTGTAAAACATTTGATGCAATCGCTTATATTGCTGAATCATTCGGTCAGAAAAAGAAATTTGAGTTT
CAAGCCATTCAATGTACTCTGCGGCAGCTTGCATTAAATTTCCTTCATAGCCATCGTTATTTTCTTGTGC
AGCTAATTTAGCTAATGCGTATGAAATACGTTCACCTTGAAAATCGGCTTTAGGCTTCTGAACAACTTGA
TTAGTTCGCTCTACAACTTCTTCAATTTCGCCATTTTCTACTGATTCAGTATTCCACAAACACCAATACG
TAATTGGCTTATCGTAGATGTTAATAATCTTTCCATCAGAAAGTTCAATTTCAATAATTCCAGTGTCAGG
CTCTATATCATCTTCACATTCTTTTACAAGTTCACGAACTTTAAAGACAGTACCTGCACTAAGTTCCGGC
CAGTAATTACACAGCCCTGTATCAGCACGATTAATTCTAAACCATTTATCTACTGTAATCATGTCCCATC
TCCATATCAATTAAGTCATTTATCGTTGGTTCATTATACACCGTTTCTTCATCAGTGTAAACCGGTTCTT
CCGGCTCTGGCTCTACAGTTTCCCATCTAGCCGCCCACCAAGGTTTTATAGCGTAATCCATTCTCGTACT
GTCTGAGTTACTACACGTTCTCGAAGCTCAACCAGTTCAACAGTAGGAACTGCATAATACCAGTCAGAAT
GGTAAGAACCTGAGCGAGATTCATTTACAGCCACATGTACATTATGTTTTGGACTAAAATAAACACACTG
ACGATATTGGCATTTACCATCTTGCACCCAGTCTTCTATTTCGATAGGTTTAAGATAGTCGGAAAAATCG
AAATCATAGTTTTCCGAAAATGCATCATGGTCTTCAATGATTTCGCTCAGAACTGCATCAACATCTAATT
TATTTTCAATATTCATTTTTCACACCACCAACTGCTTACTTCAAAATCGGGTTCGCCATGTTCCCAGAAC
CAGGGGCCGATAGGAATCCACTTACCTTCATCGGTGACGTCATATTCTTCGGATTTAAGCCCAACGTATC
GCCATTCGACTTCATAAGCGGTACCACCGATTACAACTGTGTTCTCGCCATACGGGTTAGACACAACAAA
GTCTATACCTTCTGCTTTAGCTAACCAAATCATGTATTCATAGAGTTTATATTCCAGGTCGCCTTCTGTT
CCATCGCCTAGAAAAATAATTTGTTCATTTAATTCTATCATTTAAAGTATTCCCGCAATTGGTCAAATCC
ACCAATATGACTTCCATCAGGAGCAAATACCTGAGGCATTGTTAAGCCGATTTGAGTATCACGACCTAGT
TTAGTCAGAAGCTCAGCAATTTTCTCATCATCAAAAACACCTTTTTCCGGCATAATGTTGATAAATTCAA
ACGGCTGTTTCTTCACAGTCAAAAGACGTTTTGCATTATCGCAATACACACATTTATGAATGTTGCTATC
ATAACCATATACTTTAAACATATTATTCCTTAATTCCTAGTACTTGTTTAAAAGTCTCGTCGTAATCAAG
ACTTTGGCCCGTTTGTTCTTTATGCTTGTATATAATATCACTTACTTCTGATAGCATATTTTTATATGAA
CGAGTTAAAGCAGATTTAAGCACGCTGTATCTATCAGGAAATTTACCGTGTTCATTATAATAAGCTATTG
CTAATTCACGGACTGCCTTTTCAGCAGTCTCCATATATTCTTTACGCTTTGTCATTTTCTTCTCGGTCAA
 
ATCGGTGTTTACAATGGCGACATTTATAACGAAGATTATTAGTCTGCCAATGGACCAATTGCACCTGTTC
AGTTCCACATTCAGGGCAATTAGGAACGTTTTTAGAAGCCCTTTCGCGACGTTCAACCATGACCATTACA
GAATCCCAATTAACAGGAGGGCTATAATCATCACAACCATAAATCTTTCCACGCATTTCCAGATCGTCTT
CTTCACCAGCCATAATAATTTTAATTAGACTAGAATTACTTGAAGCTGCAATGTCTTCTAATAGACGCTT
TTTCATTTCAATACCTCAATAGCATTACGTAAACCATTTGCTTTGGCGGTTAAATCCTTAAGAACTTTAG
TATGCTCTTCAATCTGTTTTTCAACTTCAATCAAACGGGTACTGAGATATTCGCGTTCTTCTTTCATAGT
ATCATTCCCATGATTGGGCTTTGGCGTAGGTTTAAATTTATTAGGGTCCTTTAACTTAATAACAACTTTA
GTTTCAAACAGTGAAAGACCATAATTCGTATTATCATCAAAGGATTCAACTACGTCCATTTTAGACAATA
ACGTACGAAGTTTATAAGTCAAAGAACGAATCGTTTTATTATGTTTATTAGCTTCACATGGTTTCATATA
TGGAAGATTTTTATATATTTCATGCGGAGCACAAACAATAATTAACAATTCAAATGGTCCATTCTGAGAT
GATGGAATGAATTTAGCCGTAACCCAATCTTTTACATTTACATTAATAGTACGATCATCGCCATGACAAA
ACAGTCTACGAGAATGCTTAAAAAGCATATTCACATTATTATTAAATTCGTGTGAAAACTCACTGGCAAA
TTTTTCTTTACTATCTTTAGAAAACCATTCTTCTAAATAAGTTCTTTTCACGTCGTTAATAATATTAAAT
GTAACAACATTATCATTTGATACATTAGTCTCAATGTAGCTTGGATACATAAATTTTTTAACATTATTAA
CAGCTATCACAGAAGATAGTAATCGGGAACGTTTGAACCACTCGAGCAAAGAACCAAGAGAATGTGAATA
CGCATTTTGTGTATCTTTACAAATATGGTTTTCCCATCCAATATAGTCTAAAAAATCACGAAGAATATTA
TTGATTACTTCTCTATGTCCTTTCTGATAATCACGATGTTTAGTAATAAGACTGTCAAAATAATCAATAT
AATGTTTACGAGTTTTCATGTTCTTCTCACTTGGTTAATGATTTATACTCCGAGCCATCCTTGGCTTTAA
ATTTTACTTAATTAACTGCAAAGCTTGTTCTAGACGATCAAGACGATTAACGGATTCTTCCCAAATCTTT
TTAGCCTGCTCATATTCTTTCTGCGCTTTATTAGAAATTTCTAGAACTTCTTTATAAGCTTTTTCTAGTG
CAATAACTTCTGGACGAATTTCTATCGGTTCAGGTGAATCATCAAGACATTCCATTAGTTCCTCAAGGGT
AGTTTCTTCTTTAGGAGTATTCACAATTTCATCACATTTTTGTTGGTAAATTTCTTTACTAGTAGGTGAA
TAAGCACATTTCACTTCACGAAGGCTAATTACGAGTTTATATCCTTCCCAACCGCATATATTCTTAAAAG
GATATGTATGAATATTTTCTACTGTTTCCATACAAAGTAATGCGGTCTCTAATTGACGAGTTATAGTGCT
AGCAATTGAGAAAAATTTATTAATATTCTTTTGGTTAGTTTCTGGTGTAAAAAATCCATAATTCACAAAA
ATAGATACTTTATTTTTATCAAGTTCATATTCTTTTATGATAATCATATCAGAAGCCAAAGGATGAATTT
GACGATAATCGCCATAACATAATTTAGAAGCTGATTTTAGAATTTGCTTCTTGAAAAGTCTGAAATTACT
AATCCAACGACGAGTAAAAATATTCTCAGGGTCTTCTTTATTATTGAGATGATAAGAATTAACATCACCG
AACCAATTATATCCTACTACATCTTTAGTTCGTTTAATTTCTTTCCCAAAATTACCAAGAATATCCCGAT
TAACCAAATATGAAAGAAGACGAGACTCTTTAAAGGTTTTCTTGATAACATCTTTATCTACACGGCTAGA
AATTTTACGAATTTCATGAATAAATTTAGTAGAAGAAACAATACTTGCATCAACACTATTGAGCCACTGA
TTGATAATAGAAAGAACGCTATTTTGGCCAAACATCGGTATAGCTTTATCATCAATAACGCTATTGAATG
ATTTGATATATTCGTTACGAGTCATATTAATCTCCTCAGTAGAAAGTAAGAACATTATACCACATCCTTG
TGGCAAAGTAAACTAGTTCAGTGCATTTAGTGCATTGTTCAGTTTAGAACGTTGCTTTGTCAGATTTTGA
ACCCTTGACTGAGCTTGTTCTAACGCCTTTTCAGCTTCTAGCACTTCATTGGTCGCTTTAACTAATTCAG
CATCTACTGCCTTAAGAGACTTCTCAATCGCATCCGCGTGCCATTTTTCAACAGGTTTAAGACTTGGATT
TTCAACAGGAAGAAAATTCACTTTATTGAATTTCCATGCATCTTTATTACCTGAGCTATACATCCAAAAT
TTAGGATCGTTTGATAAGTAATTAGATAAATTTAAGTTATCTTGCACTTCTTGCTTTTTCTCTTGTGTGA
CTTCGTCCTTTCTCAAAAATTCATATTTAAATGAAACGATCATATCAAGTTCATATGATGTTGTATCTAA
CTTAAATCGTTCAAAACGAGGTAAAATCTTAGATTGAACAGCAGCAACAACATCCATATACTTAAATGCT
TCTGTCAACTGCGATTTAAGGCATTCGCAAATTGAAAGAGAATTTTTTGTATTAGGTTTAAAGCTAATTC
 
GTGCAGTTCTATTATTTTCTTTTAACGGTCTTACTTCCATCTGAAGAGTATAACCATCAAATTTCAGATT
CTTTAAGTTAATGTCAGAGCCTTTTAATCGACTAGCAGTAGACAAAATTTGCTTTAATTGTCTACGGAAC
AGAGCAATAAATCTCCATTCAATACGATAATGATTTGGATTGAAAAATCCAGATGATAAATCGACCTTAC
TTTGACCAACGCCTTGAACAAATAGAGGATTTTTATAATCAATAGTTTTACAGAAATCGGTGAGCTGTTC
ACGAGCAGTCATTTGAGTAATATACCCTGCTTTACTAAAATTACGCACCCATTCACTGCTATTCGTATAC
TTAAAGTGATGAATAACACGATTAACTTTATCAGGGTCTAAATTATTTTCACACAAAAATGTCATAACAT
CACGAGTATAGCTGGCATTACGAACCATATCTTCAATTTGAGAACGAGTTTTCATGGTGTTCCTTAAGAT
TTAAGTAAATCAACAATTTTAATTAACTTTTCACGCTCAGATTTAGCTTTACTACTCAATCCAGATAGTC
TGAAAATTTCATCATCATATTGTTGAATAGAAATATCAGCTCTTCAATTTGCTTATTAAAATAATCAATT
TGTTCAGAATGTTTTTCGTTACTACGAACTGGTACAGGTTTTGTAGGCAATTTAGTTGAACTGGATTCAT
TCGGGCGATAAATTAAAATGCAATTTGAACCAATCGGGCATTTAGCACCAGATGAATATTCTAACGTTCC
AGCTTCTTTTAAAACTTCAAAAGCTAAACAGAGATGATGCCCCATATTAACATAATCAGTAGAGCGAGCT
CTGATGTAATAATCATCTCCACGAGTGCTAAATTTAAAGCATTTAAGGTCTTCTGTATTATTAGTTTTAA
AAATCATTTGTTTATCTAGACCTTTAGCCAATCGAGCACCTAATGCTAATAATCGTCGTTGATTTTCCCA
CAAATCTGCGATCATTTGGTCAAATGATAATTTCGGCATTAGCCGACCATAAAGGTCATATCCTTTATTA
TAATTGCGAAGGATTTCACTCGCATTAATACGAGACAACGCTCCAATTTTATTAAGGGCTTTCATAATAC
GATTAGATAAACTCATTCCAGACCCGTTTGTTCGCTGTAAATGTTTTTCTAATCCAAATCCAATATCAAC
TTTAAATTTATCTAAAATTTCAGCATGAACATCTCTGTCAATAACATTCAAATCCAAAGTTGGGTTAAAT
CTATGAAAAAATTTATCTGGCTCTCCACGACGTAAAACAGTCCATTCATTCATCATTTTTTTATTAACTA
AAGATTTAATTACTGCGTTGACATTATTAATTACTACTGACATATTTTCCTCACTCAATTTTACCAATTA
CGCGGAATAAGATTGAACAGACTATATAAGTACCACATATAGATTGAACTAATGCCATTCCAAAGAACCA
AACAATATTATCAAACCATGTCTGTTTTACGTCGAAAGGACGTAAACTTACAGTAATATTATCACCTTTT
TCTATTGAAGAATACGTCTCTGGGGAAATATATTCACTAAATCTATAACCGTCTTTGAGTTCATATACAG
CAATAAACGATAAACTAGACCCCTTTCCTTGAGTTCCTGTAAGGGTATTAACTACAGTAACATCATAATC
TTTATAATGCATATAATCATTAATTGCGTAATAACCATATGCAATTACTATACATAAACAACATATCAAT
AAATTCAATCTTTTAATTATCAACTGTTTCATAATAATCTCAATTAAAAGGGCTTAGAACCATTATACCA
TCCTTGGTATAAAGCGGTTATGCGAGTACCGTATTTAACCGTTCTTCAAACTTCCGAAGAGTGTTCTGGC
GTTCAGCTCTTTGCTTTTTGTAAGTTTCAATACGCTCTGAAATGAGAGTGTATCGTTCATTTACTGATTC
TTTCATAAAATCAGGAATTTCTCGAGAAGCTTTAATCTCGTCAAATTTATCAATAACAGCTTGCTCTTCA
GCAATTAAGTTATCATACATCAAAATATCTTTCTTGATGAACTCAATATCTTCTTGAGTTACACGAGATA
ATTTAGATGCTTTATCCTTTTTGTACTGTTCGTTAGTATCACGAGACCAGTGTAATGTACGATTTTTATT
CGTATTCTTGTAAATTTCTACAATACCAATCTCATCGATAATAACGATCCAATTCCAACGGGATTTGTAA
ATTTGTCCTCCATTAACAGTGATTTCACCTCCGATTGAGATGTCATTAAAGAACTTGCTTTGTGCTTCTG
ATTTAAATTTACCATCGTTGTAATTTACCAGGTTGAAAATATCTTTAGCGTTCATTTTGTGTTCCTCCGT
AGTTGATAGTTGTATAGTACCACAGAGGAACGGTCTTGTAAACAACTAAAAGAAACTTCTTTCACAATTT
TTTCCACTGAACCAAGCGCTCACTGCTTTCTTAGTTTCAGGAGCAGTGTTATCCATAAACCATTCAAAGG
CAGCCTTTTTATGATTCTGGAGGGCTTCTCGGGCTTTAATCTGCTCACGGTCTATTAACACTAACATATG
AGCCTTTCTTGTCACCAGGGGCTTCTTATGATTTTTTGAATACTCCCAATCATTTGTCCATCGCATCGTT
GTTGCGAATTGAAATACAGCTTCTTTAATCTTAGTTTCGTAAATTTCACGAGCCTTTGAGTATAACATCA
TTACCTCCATTTACCAGTTTAATTCTAGTCATCTTTTTGATGGCAGTCCATATAATCTATTTCTGAACTG
CCTTTTTGTCTTAGAAGGCCTCTTATGAATTTATTTCAGAAGAGTAACCCGTAGCGATTTCTTCCCAACC
 
GTTTTTGTCGGTCATAATAAAGTCAGCAAGATAAAGTGCAGTACGCAGTGAAACATTACGTAAACGGTTA
ACATTGACTTTCATCCATGATAATGCTTTATAAGTTTCTTCATCAGAAAGACCACGCTTTTGCATCATGT
CGGTTGAAAGAATAACATCTTCAACCCTGACCATAATTTCTTCATTAGTGTGAACACCCAAATCCAAATA
AACTGAGCGGGACACTAATGCTTGTAAATGTGGAGCAAGTTTAGTACCACGGTCTAATTCGCGGTCAATG
TCAACGTTTGTGATAAAAACAATTGTTCCTTTAAATTCGAGCTCACGCTCAATGCCTTTTTCTTCTAAGT
AAGAAGATGCAGTGCTCCAGCAGACTTTACGGGTCTCTCCAGTGTCCAGAGCAGCTTTCAGAAGATTAAG
AATGTCCATATCAGAGAAAACATCCACATCATCAATCAAAAGGACAGAATTCTCTTCACGATTATTCCAA
AGCTGTTCATAAAGACCGATACCAGAGATTTTACCGTTAATGCTTTTATACTCAATGTATCCAATATCAT
TTGCTTTATTCAAAGCTTTATCTAAAGAATATGTTTTACCAATACCCGCCGCACCAGAGATAATTAATGA
ACGAATATTTCCGTTAATAATACCATTCGTCATCATTCCCATAACATTAAATCTTTTATTAATGCGGGTT
TTCATATCTTCATATGATTCTTTAACTTCTTCAACTTTTACACCATCATATGAAATGTCTGATTTGTAAA
CCCAAACACCGCGACGTTTACCGTCAATTTCAACAAAAACTTTACCATCTCCTTGTGCATCTACCGGAGC
ATTATCAGGGAACCATTCACCTAAGAGCTCAAAAGTTCCAGAGATTTCTTTACCGAAGTACATACCCTTA
TTGATAGTTACAGTTTTCATTTTATTCTCCAAATCCGTATCAGTTGATAGTTGTATAGTACCATAAAGCT
TTATGCTTGTAAACCGTTTTGTGAAAAAATTTTGAAATAAAAAAGGGAGCCCGAAGGCTCCCTATCATTT
ATAATAACTTCGATGGTTTTCAAGATAAACCCTCTCAAGGAAGTCATCCCAGAAACTCATGTCTACTTTT
TGCTGCATACCGTTCTTAGAAGCTTCAGTAGATGCTGCTTCTACTTGATCGACCACATCTTCCAAAAACT
CTTGAACGGTTTTAAATGGATGCTTACCCAACTTCACGTCGAGAATAAATGGAGCATCTTGGAGTGGATA
AACCAAGTCACCAGTTTTGTAAATTTCCAATAGTTGAAGTCCACCACGACAAGCATGGCTCAGAGCTTTC
CAGTCAATGCCTTCATTGGCTTCGGCCTTACGAGCACGTTCGCCGTATTCAGCATCTAATTTGTTCAGTG
ACTGCTTAAGCTCAATAAGAGAAAGCGTTGTCTGATATTTACGACCCAACACTGTGTAAAACGTTTGTGG
GCCTGTTTTCTCATGATTATGGAACACCCATTCACAGAATTCGTTTTCTGGAAGACGATGCTTAATATCT
TCAACTTTAGTACGACGCTGCTTAATGGAACCATCTTCTTGGTAATCAACCCATTGCTCAGGGATTTGAT
TAACTACTTTCAATACATCGCGTAATGCAGCCAAACGAGAACCCTTGACGCCGTATTTAGAAGCTTGCTT
GCGGACATATCCTAAATATGATTTCATGTTAGTCGTATAAAAACGAGAACGGTTGTCTTGAATAAACTTC
CAMACATCAGGCAAATCGGATTTAACCACTAGTTCAGGTGGAGTGTGAAGCATATCCAATGCTACAGGTT
CACCATCTGCTGCTAATTTAAAGAAATACTTAAGACTATACAATTCGTGGTCAATATTATCTTTAGTGTT
TTTAGATGATGTGTTGTTGGTATTTTTACTCATGTGCTCTTTGACGTTTCCAATAAGAATATCGCGAGCA
GGAGGAACAAAGATTTCTTTAAAATCTACATCAGATTCTGGAGTAGAAGTTCCATAAAGATGACTACCAA
AATAAGACTTAACTACAGTTTTCATTATTAGACCTTTCATAATCTTCATTAAATTGTACAATCAATCGAT
GATAATTAGATTTTGGATATCCTAATTTACTTATATAAGTTCCGAATGACCCACGTTTAGGTCTATTTAA
TTTAATCCATAACTTATAAAGTAAGTCATAGTCTTGCCAATGTTTACCAGTTCTTTGTCTAATTTTAGAT
GAATTTGATTGTTTCTTTTTAGCTTCAAAATTATTCAAAGATTTTTTGACTGCAGCAGAGTGTTTTTCTT
TAACTCCCGGTCGTTTAAAAGCAGCTTTCACCCCTTTAGATATTTTTTCTTTAACTTCCGGTTTGTTTTG
TGCTTCTAACTGGGATTCTGAAATTTTAGCCCTTATTTCAGGAAGACTTAATGTTATCGAACGTTTTTCT
CTGTATTCATTAGACTTCCACATTTCTTTCATTGTATTAGAATGTATTTTTGAAAATTTTTCTCTAACTA
ACTGATATCCTCTTGAAGTTAATTTGAGATTTCTTCCTAAAGAATCTTCTCCAAAATTATAAAATGACCA
CCATGCATAAATTAATCCAGGCGAATTGTAATGAATTTTAGCTAAAAGCCAATGGGCTATAAAATGCTCT
CTAGCTGTTAATAAAACTAGATTATCAGAATCATCATTACCACCAATACAAGATGGAATAATATGATGCT
TTTCCGTATAAAAATTTAATTTAGATTTATCTAATTTTCTGAGTTTTCCTTTCTTAATTAAATTATTATA
TACTTTAGTATAATTCATTGGTTCTTCGTCTCATTTAATTTTGCTTTGCATTTATAACACATGTCTGTTC
 
CACTTGAAATAAACATATTTCCTCACTTTGAAATCATAGTTGGAATAACAGAATCAAGATAAGTCTTTAG
TGCAATAGCTTCCTCTTTCTTTAATGTAATGATATGTGATCGAAAATCATCAATTTGACGAATAGATACT
ACATCTCCCTCTTCATAGCATTTTGAAACATTTAAAATAGTTTCATCATTCTGATTACAGGAGTTAGTAA
TAATAGCATTACATTTTTTAAACCATTTTAAATTATGTTTTCTTTTAGTAGAATCATAAAAATATTTAAT
GTTAGTTATAAAATTATCCCAATTATTAATTGATAAGCACATTGACTCGCTTTTAATATTAAATCCTGGG
CATGAAGAATAAAAATGAATTTTATGCTCATCATTAATGCTTACAATTTTATCAGTGTAAGCATATTCAA
TTTGGGTTAAACGAACAATTTCGCTAGGCGTAAAATATAACATGTCATCTTCTTGCGTCAAACGATACAT
GTTATTTACTTTTTCTATAGCCAATTCACCAAAAAGTGGACTAACTTCTAATACTAGTCGTTTCGCCTCG
CTCATCATTACATACTCCTCTGAATCATATTAATAATGTTATTCACCAGATTATAAGTAAACATTGGGTA
ATTATATTGAATCATCACATATATAACAAACAAAACTTTCATTCTCTTCTCCTTGGCAGTTGACAAGATT
ACTATACCATAATCTTGTCAACTTGTAAACCATTAAATGACGTTTTCGATAAAATTTTGAAGCTTTGTAT
GAGCATCAACCATGATTTTCATTTCTTCCTTGGAAAAGCTGTGCCTCTTTCCCGAGAAGAACATTCATAG
TCAGAAACTGCATTAGCATATTCTTCAATTAGCTTCATTAAAAACATCTGTTTTTCAGTTTTCATTATTC
CACCTAATCATTTCAAGATATTGAACTAACTTAGCTTTGGATTTATCCAAATCCCTTTTAGCTGCTTCTA
TACCGTCGTATGAATATCCTTCACAATGCTCAACTGCTAATTGATATGAATCTATTTCAATATCATGCGC
TAATTTAATGATTTTTTCAAACTGTTCGCGTGTTAGCATACTTAAACTCTCGTATTATGATCGATAATTT
CATCAAGAAACATATCTAACGCTTCTACAGCATTATTAACTTTAGCTTCAAATTCTTCAATACCTGATAA
GGCAAAGTTAATGCGTTCCTCATCTGCTTTACGAATTAAAGCTACCAATTCCTTAATTTTATCCGCTTGT
TCGATACTAATCATTATTCCACCACATATGAAAGAGAGAATATTGCACACGCCATGTGAGTTGCAACTTC
ATCACACATATTATAACGTTTCTTAAGAAGTTCTACAAGTTCTTCACTAGTAACTTCATCCATGTCGACG
AAAAAATCACCATTAATGATGACGTAGATATTTCCTTCTTGATTGAGTGCTTCAATTTTCATGATGTTCT
CCTCTTTATCCGATGGTTGTATAGTACCACAGCTCAAACGGAAAGTAAACCGGTAAAATGAAAAAAAGTC
TCCCGAAGGAGACTAATGTTATTCGAGGGAAAGAAGATACTTACTCTGGTAAAACATTCCAGTAATATCA
TCTATCGTGCTTTGGATGGCTGGAGGCATTTCTTTATAAATGCTGTTAGATTGGTCTAGTATGCGATCAA
TCATTTTAATTGTGTCGGTAGGAAGTTTACTGGCATCTGGAATTGAAGGCGTGTATTTTCGACCAGAATA
CCCCAAATATTGCTCACCAAATTTATCAATCAAATCTGGCAACTCGGAAAAAATAAAATCGTATGCTTTG
TGTCTAGCATAACTTTTAGTTTCAAAATGTGCAGAATGAAAATAAGCTTGTGCAGCCATTAATAAACCTA
AGTATTCATCTGCCTTTGAAGGTTTTCCACTTTGTGAAAAGTCGCTGAATTTCATTCAGTCTCCAATTTA
ATGTTCATAATTCTAGCGTATGATTGTGCCATCTCCGCGCCTCGCTCTATACATTCAAAATCAGAAGAGC
ACGGGTCATTTTTATAGGTCGTTCTCATAAAACTATAGAATTGTTCAGACGATTCTACGCTTTTATTTTC
AAAAAGCATATAAACGTGCCTAATACCAGATTCCATAAATTTATCAAAATGAGGATCGACATTCGCTTCA
ATCGATGGAGATAAAACAAATGACAATCCTAGCATGGCAAAAAGTGCTGTTGCTTTTAAGGCCATAAAGG
CCTCCTATCATTTTTGTCCTGTATTTACTTTGTGCCGATGCACGGCCTTAACTTTATCAAGGTATTTTTC
AAAATTTCGCAATCTAGTATAGTCTGCCGGAGATTGGTTGAGTGATACTTCTCGACGCAAAGCTGAAATG
ATATTTCCAACTTCCCTACGAATTTCATCTAATTGAAGAACAGTAAGATTGCGAAGTTGCTTTTCAGTTA
ATTGTAGCATATATACCCCTTTAGTTAGATAAACCTATTTATAACTTTTGCACTAACCGAGCTTTTTAGT
TAATTCATTCCAATGTTTTCTACACAAAGAAACATAAATTTCATCACCAATACAAATTTGATTACCTTCT
TTAACTGGTGTTCCATCTTCCATTAATCGAGCTGTCATAATCGCTTTTTTACCACAATGACAAACTGCTT
TTAGTTCAATAAGTTTATCTGCAATCGCTAAAAGTTCTTTAGAACCTTCAAATAATTTTCCAGCGAAATC
AGTCCTTAGCCCATAAGCCATAACAGGAACATTATATGTATCAACAATTCGGCTCAATTGATGCACCTGT
TCAGTTTTTAAAAACTGAGCTTCATCTACAAATACGCAATGAATATCTTTTTGTGCTTCAGCCCATTTAT
 
AGAACTCGAAAATATCCATATCATCTGTAATAATATTCGCTTCCTGCTTAATTCCAATGCGAGAAACGAC
TTCACAGACAGAATCGCGAGTATCAATAGCAGGCTTAAGAACTAATACACTCATTCCACGTTCTTTATAA
TTATGTGCAGCAATCAAAAGAGAAGCAGATTTTCCAGCATTCATTGCTGCATAAGTAAAAATTAAACTCG
CCATCTTAGTCCTTAGTTAAATTTTCTAAATATGTTTCTAAATCATTTTCAGCTTTATCGATAGATTTTA
CTAATTCGTAATATGTTTCGGCATCTCCATATTCAGAAGATATTTCAAAAGACAAATCCTTTTCTAAACT
AATAATTTCACCAACTAAAAATAATATTTCGTTCTTTTGTTCGCGAGTAATCATAAGGAATTTATATAAT
CAATGAGTTCTTGTTCTTTATTATCGAATTCTTTAGAAAGTTCTTCGTACTCGTTTGCGCTAAAAGGACC
GCATTCATTACAAACTTTTTCCAATTCACTATTTTTATCCATAACTTCGTGGATAAGAGAAAAGAGTGTG
TCTTTTTGTTCTTTGCTTAAACTCATAACCATGTCACCTTTAAGCAGTATTCTTCTACATGCTGTTTACG
ACCTTTCTTATCAATAAAGGTATATTCAACGAATGTTCCAATGTAGTCTTTATCTACATCATGTGGACTA
TTAATTGGACATTTAGTGCGACAAATGCGTTCCCATTGACGAATAATTACTGCCTTATTCTTTGGGTCAT
ATGGATGTGGATAATGTATATTCATAATAATGGTTCCCAATCAACAATCACAATTTCTAATTTAGAGGAA
TATGTATCTAAAATCCCCTCAATAATATCCCAGTTCCCTTTACCTATGCCTGCACCAATCCTAGGCATAT
AGATTGTAGGTTTAATCAGTTTATTTTCACCAAACTCATTTAATTCTAACATACAATTCATTAAAGCGGA
ATACTCAAAATTTGGCCCTGGTTGAAATTGAGTATAAAGATTGAAGCAGTAAGCTTTATGAGTCCTAAAG
TATTTTTCATAGACTGAGTAAGAACCGAGTTTAGTTACATCACCCCATTCAGTCTGTAATTTATCAGCTT
CCAAAATTTTAGGGAAAGCTTTGGTTAATTGACCCGCTACGCCTGAACCCATAGTATGAAAACAATTACA
TCCATGTGCAATATTTTTACCTTCAGCGAAAAGGGCGACAATATCGCCCTTGATATATTTTACAATCATC
TAGTACTCAATCCTCGATTATAAGAATCTACCAAACGGTCAACCATTGAATGACAAGCGGCTTTATCTTT
CTCCTCCGCAACTGAACATTCTAAGGTATTCCACTTTTTAGCATATCGTTTTAACAATGTATCGTTTTTG
TATCTGCTTGATTTATCTCTTTCTCCGTCTTTATATGCATATATTAATTTCTGTGCAAATTCAGCTTGGC
ATGCCTTATTTTTCCCACAATAATCTGCCGCAGTGCGGTTTACATATTCTCTAATTTCAGTATATGATGT
ATCTGCTGACGCAGAAGCAGAAAATGAAATTAATCCTATACATAAAACCAAAATTTTAGTCATTTACTAT
TTCCAAAAGTTTATTATTTTTAAGGTAATTAGCCTTTTCTAGGACTTCAGAAGCATATTTAGAACCTGCT
TTAACATTCCATCCCGAATTATAAGAGGATATTGCTTTTCTTATATCGCCCTTATGTATATTTAACCAAT
AAGAAAGTTCAATGTACGCCCAGGAAGCTGAATTGGATCGTTTATTCAACATTCTTTTTATTTCAGCATC
GGTCATATTATAACCAAGTTCCTTAACTCTTGCTCGCATAGTAGGCAAATAATTTTGGAACATTCCGTAG
GCGTGATGCTTTGGTTTAGATTTTAAATTAACTCCGCCAGAGCTTTCTTGCCATAAAATGGCAGCCATTA
TATGACCTAATCCGCTCTTGTGGATATTTTTGTGTGTTTTATATTTTCCATCCTTAGAAAATTGTTCCCC
GAATTGATACGCGTAACGCATGTTATCGAGTTGGACATTACTGAAAGTATGCTCGGAGCTATGTGCCATC
ATTGAAATGGCCAATAGACCAGCGAGTAGTGCTTTTCTCATGCTTACCTCATTGAGTTTTAATTACTGCT
TTAGAAGCCTTTCCTGGTAAACGACGACTGTTGATAATTGCCATCCTACATTGAAGTGACGGGTCTTTGA
ACTTCTCGTTAGGTTTACAAACTGTAAATCCAAGCCAAAGATTTCCATCTGTGATTTCTAAACGTCCAGG
ACGATATTCAACCCCATCAATAAAATCCTCGTCAATGTCAGGACGCGGAGGCATACTCAGGAATTCATTA
ACTTCTAAAACATGGTCTTTTATTTTATGGAATAATTCAAAAACGTATGTCTCATCAATCTCCCGTTGAA
TTGCGCGATCAAGAAGATGTTGAGAATATTTTAGATGAAACGATGAGACTCCTGCTGCTTTTGATGCCTC
ACGAATCTCATTGTTAATTTGACGAAACTCCGACTCAAAGTGACGACGAAGCTTATTTCGACGGATAAAA
ACTTCTGTATTGATAGTCATGTTATTCTCCTCTTAACTGATAGAAAAATTATACCACAGTCAAGAGGAAA
AGTAAACAGTTATTCTTTAAATCTAATCAATTTATTCATAGACTTTGAAACTTCGGCACGAACCTCATGT
AGATTTTTGAGCTGTTCAAGACGCTGCGTATAGTAAGCAATTTCATCTTCTTCGAGACAGTCCTGCGAAT
CTTCTTTAAGATAACGTGCATAGTCCTGGAAAGCGTTACGGACTACTTCCTGGAAGTCATCAAGACTTTG
 
AATTTTCTTAGGAGCAACAGATACACGACGAGGGGCAGTATAATACTCATAACCAAACCCTGCGCTTAAT
TGAGCCATTAGTATTTTTCCTCTGGTTGGAACACTGCACGACAAGCCCACATACTGGCTTCTTTGAGTTT
CGTTTTAGCAATAGTTAACTGATCGAGACTTTCAGCATAATTCTTCGCGAATTCACAGTCTTCGCAATTA
TCTAGTGCTTCCCAGAATTCATCATATAAAGCATCAAAGATAAGTCCTAAACGAACTTCAGCGTCTTTAA
TAGCATTTACTTTACCGATTTTCTCTTCAGTATGTGGTTTATAACCCTTAATATCTTCAATCATATTTGA
CTTCCTCACCAGTACATAATACGTATTCAACTAAACGAATAGGTTCATGAATGCCATAGCCTTGAACAGA
AATTTCTGTCGTAGGATAAATTCCATCAATATCACCCATATTCCACGCTTCATTAAATTGCTGTTCGCCT
GAGTTACTAAACCACTCGCGAAAGCATTTAGCACATCTTCAGAACCTTCAATAATTATCTTTGCCATTAC
AAACTTTCAGTAAAGGTACGAGCGATAACGTCGCGCTGCTGTTCCGGAGTCAGAGAGTTAAAGCGAACTG
CATAACCGGATACACGGATGGTCAGCTGCGGATATTTTTCCGGATGCTTAACTGCATCTTCCAGAGTTTC
ATGACGCAGAACGTTAACGTTCAGGTGTTGACCACCTTCAATTTTAACTGTAGGTTGTGGCTCAATTTCA
ATTTCACGGGCATGCAAACCATAGAAAATTTCTGGGTCTACAAAAGAGTCCTCTTTAAAGGTTTTAGAGA
CAATAATTCGTGCTTGAATACCATCTTCAAAATAAATAGTACCTTTATGTGTGCCTTCAAGAATTTGATA
TGCTTTCATATAAACCTCAATTAGAAAATAAATTTATCCAAGATTGTTCTTTAATTAAAAATGGCTCAGA
ATCATATGCCATTAAACTTTGCGTAATTAATCCTTTAAAAGGTCCATCAATAAATTCCATGGTAAAATAT
GGAATTTTATTCATTAGCCGTGCATTAGGAGCAGTGCACAAAACTCTGCATCCTTTGAATACGCCTTTTT
GTAATTTGTATTGCTTGGGATAAAATTCGCTCAAAATGTTATTTTTTGCCAAAATTTCAAAATGATTCAC
CAATTTATTTTTAATAGTTTTTGGCGAAAAATAAAGATATTCGAAAAGCTGAGTGTCTGTCATCATTGCA
TTCCGATTACGAAAAACTGTGGACGAGTAATACCACCAATGCAACATTTACTATTACAGCAGTAGTGTAC
GGTGTCAATATGGACACTATAAATCTTATCCATATCAGGAGATTTGACAGGCTCATCAATTATATACAAA
ATTCGCGAAAGCTTTAAACCTCTGAACTTGCTTCCTTTATTACCAATAAAACTGCGTACAGAATCAGTAA
ATAAACGAAAACGTATATCATCATTAGAATAACGCGAAAATTCCTTTTTGATGTTATTTGCAGAAATTTT
AGCATAAGCTGAAGTATTAGAAAGAACAATAACTGTTCCGCCATCATACAACCAATTAGCAGCAAAGTTA
GTCACAGCAATTGATTTACCGGATTGACGTCCACCATCTAGTCGAAGTGTACAATACTGTTTAAGTAAGT
CTTCAAATGGCGGGATATATTCGTTTTTACAAATTTCTTCTACTCTAGCATCAGAATGGTGTGTAAAAGC
ATTCATCAGGGATAGATAAGGACCAGTTAAAAATGTTCTCATTTCTTCTCTATAAGCTCTATAAGTTTGG
GCCATTCCGTGGCACATGAATTGTCCATTTCTGTATTTACCCATTACCGCGCTTGGGCTCGACCTTATTA
CAGGTTGGCGGGAATCCCTCATATAATCATGAGGTCCAGGTTGTTCCCTTATGCATAAATCGCCTTACCG
TAGTATTTGTACCAAGTAGGACGTTGTGCAATTTTTTCATCTAAACGAGCTTGTGATATAGCAATAGAAG
CTTCATGGGGAATATAATCACCACGGAATTCCTGAGGAATATCACTAATATCCTGGACTGTAGTATCCTT
GATATTAAAACCACGTTTTAAACATTCAGCTATAAGCTCAATTTGACGTTTACGTAAGAACTCGAGCTTA
TCGTAAAAGAATGTAACATGACCTGCGCCAAGGATAAAAGTAGGACTGATTTTAAAATCACGAACACGTT
TACCGTTAGCAACATGCTTACGAACTGCACCAAAAACACGCGGCAATTCACGATATTCAGCCATTAAGTG
TTGGTCAGCCAATTCAGATACTAAAGTAAGGTTGATACGAGTCATTTTAGTGTTCTCCTGTAGTTGATAG
GTCTATAGTATCATACCTACAGGAGATGTAAACTGTTATTTATCTTTAATTGCTTTAGCTGCTTCGATAG
CCGCTTGCTGAAGGTCATCCATAGACATGCCGAACTTAGAAGCAAAGTTATCAATTTTCTTTTCGACGGC
ATTCAAAGGCTTGGCCTGTTTACCTTCATTAGCGCCAGGAAGAGCGAGAATACGCTCTCTGTCAATATAA
AGGCTTACAAGTTTCTTACGGTCTTTATCGGGCAAATCATGAAATGAATGGGCCTTTTTATTTACAGCGG
CTTCTAATTTACCTGCGCCCACTCGCGCTTCGGCAATAAATTCTTGATATGTTTTCATATGTTTCCTTTA
AATGTAAATATTTTTATTATTCTATCCTAGAATTGTGATAATATATTCACAATTCTAGGAGTTGTAAACT
GCTTTTATTTAAGCGTCCCAAGTATAAGCTTTATTAAGAATTACCACGGGCTGCATTAGCAACGGCGTAA
 
GCGTACTGAATATTAGCGTCTTTAAACTTACCTTTAGAGGTATCTATTTCTGCCTTAAAGCCGCCTTTAG
TCATAACATCGGCAAATTCTTTACGGAAAGCCATTGCATCAAGACCTTTCCATGATGATTTATGCTTGGC
AAATTCAAGACCTGCGAAGTTGACAGCTTTAGCCAATTTATTATCAATGACCCATTTACCGGCTTTAGGC
ACAAACTTCGGGCCTTTCTGTTTAGAGAACAGTTTTAAATTCCAGCGGCGAAGGTCTGCATCAGCTTTAA
CAAATTCTGAGTCTAAGTTACTAGCAACAACATCTTCAATATGAGCAAATTTAAGTCCGTCAACTTCAAT
ATTTAAATCGGTACGCCAGCGAAGTCCTTCCCAAGCGAAGGCTTTAAAGTCGGAAGCCTTTGTAGCTAAG
TACCGTTCAATAGGAGCTGTTTTAGGGTCAAAGCCGTTTCCTGATCGGTAGGTCCACTCATCTTTGTTAA
TGCCTTTGGCCTTTACTACAGAAGCTTCGGCAATAAATTCTTGATATGTTTTCATATGTTTCCTTTAAAT
ATTTTAATTAGTAATTGTCTATTCAAGTAATTGTGAATATACTATCACAATTCCAAGAGAAAGTAAACAG
CTTTATAGATTTTTATACGCGTCCCAAGTGCCAGTTCTAAACGTTGTAATGACTCGTTTTGCGCGATTAG
GTGTTTGATTATACCATATACTTTTAGCTAAGTTAACTGCTGCTTCATCCCAGCGTTTTTGTTGAAGCAT
ACGTAAAGAGTTAGTAAATCCTGCCACACCGGTTTCTCCCATTTGGAAAACCATATTAATCAATGCACAG
CGACGAACCGCATCAAGAGAATCATAAACCGGTTTTAATTTAGCATTTCTCAGAATTCCGCGAACAGCAG
CATCAACATCCTGATTAAAGAGTTTTTCAGCCTCATCTTTTGTAATTACACCATTGCAATTACGCCCAAT
AGCTTTATCTAATTCAGATTTAGCAGCATTAAGTGATGGACTTTTTGTAAGCAAATGACCGATGCCAATA
GTGTAATAGCCTTCTGTGTCTTTATAGATTTTAAGTCTAAGACGTTCATCTATACGTAACATTTCAAATA
TATTCATAATACCTCCTAAGTATTTATAGAAGGTATTTATAAAATTAAAAGAGGTTGTTCATTATTCGGT
AAAGTGAAGGACCCATCACATATTGCCACTGAGTACGAGGAATAAGAGCAAAAGCGTCCATCTCTGGAAT
CATAACGCCATCTTTATTTTCAAAATAAGACTCGCAACGGCAATTTCTGAACATCTCATGCTCTACTGGA
ATCGTGTAATAAAATAACTGTAGGTCTTTATTACTAGAATATTTAAATACACCTAGGTCTTCTAGAAGGT
CTGGATTATAATTGCTAAAACCAGTCTCTTCTAAACATTCTCTTCGTGCTGCATCTAATGCGCTTAAATC
AGAATTTTCTACACGGCCCTTTGGAATATCCCAACGATGTGCCATCATTCCAGTCTTACGAGAACCAGTA
ACCCGACCCATAAATAAATCTTTATCTTCTGTCATAAAGATAATACCAGCTGATAATGTTTTCATTTTAA
TTTCCTGCATTCAGTGATAAAGTTATTTAAATTTTGAGCATATTTCTTTTCATCAAAAATCTTTTGCTGT
CTGCGTAACCGCCATGGCATTTCAATGAACATACGCCATATCCCTAGATAATACCGCTGCTGTAAAAATA
TTAACAAGTATAGTTAAAAGAATCCAATCGCCTATTCTGTCCATTGGATTTTTATAAAAAAGTAAAATAC
GAATGATGATATAGGAAGACTAATGATATACCACAGAAGAACCTTCTTATCTGTGAACCAATCAGCATTC
GTTAACTTAGCGCGACCATTTTGAATACACACGAATTTATCATCTGTTACAGTAAATGGCTTAGCTGCTT
GATATCCCATTCTAAACTCCCTAATTAATCGTTTCTTTGTATCTTCGGAACAACCATTCCAATCAACTCT
ATCAACTGGAATGCCATCATCCCCATCATCTAAATCATACCAGCGAGTTTTTAAAATCATTTAATTTTCC
TGCAATCAATCACAAACTCTTTCATTGATTCATTTTCAATATAAGACATGTAGCTATTATATTCTTTTAA
TTGTATTTTGTAATCCTTTTTTCTTTGCCAATTTATTTTAAAATTATCATAATGAAAATATAAAATGATA
CCAAAGAATGAAAACAATGAAATAATTTTAGTATAACAAAGCTCGCTCCAAACTTCTATTATATCTACTG
TACCACTGATTTTTAAAATAAAACAGTCAATTAATAGTCCAATAAGACTACCTGTAAGAGCTGCAGCCAA
CGCCACAGCAAAAATTAAAAATGACTCAGAAAACGAATATTTGACTTTATTTAGCTTTGGCTTTTGCATC
GTGATTCCTTAACAAATTTCATAATTTCATTAAATTCATACTCAGCAAGTTTAAGCTGGTGTTCCTTTTT
AATCTTTTTGCACTGGGCTTTCCAATCACGTACGCGTTTACGATAATGTCTTCCTTGATACCAGTATCCT
ATCCAATTTACGGGTACTAATAAAAATGGAACTACCAATGGAAGAGTTAGCATTAACATAATTATTACGC
CAGAATCAATATCAGTCATAACATCTAAAACACCTCCAATAATCAATAGAATTACAAATGATATAGCTAT
CACAGGACCTATTAATACATCAGTAGAAATTATCTGGCGCTTTAATTCATACTTCAAAGGTTTACTTGGA
AGGTATAGTGATGGCTTTGACATATTCTCTACATTCCTTAACAAATTTTTCTAGTAATAAATCACTTTCA
 
AAATTAGGATTTTCCACTAATTTATCAAAAAGATCATCAACAATATTCAAGATATTTCTTTTACTAAGAA
TACGTTTATTTTCATGCTTCGTTTCAGAATCAACTATAAGAGTAAAGAAATATTTCTTTCCCTGAAATTT
TACCGTAGTATCAATATAAAATAAATTTGACTTTTGTAAATTACGTTTAAACCATGCGTCACTTAAACTA
TAAACACCGAGATAATCAAAATCGTCGTTTAAATAACAAACTGACCATTCAGGAGAAATGAAATCAGTAA
ATTCAACATCAAAATCACATGTCAATGAATGAATTGATTCAATACTGTTAATAAGTATTCCAGGACGTAT
TAAAGACTTTTTACCTCTGGAAAATCTTCCAGAAAGACTTTCATCAGTTTCATATGAAGAACCCCAATAA
TAATTACGTCCTTTTGCCATATGTTTAAGAGCATTTAGTAATTGGTCTGGAACATCAACGTGTCTTTGGA
ACTCTTCAAACATTGAATTGAAATCACTTTGCATTTTCATTCCTATTTACTCCAAGTAATAGGGGCCGAA
GCCCCTTATCATTATTTCAGAGAATTAATATATTCCTGAACATCGGCAGAGGTAGTTTCAACCCCAGAAA
TATTACCATTAAAGGTTTCAACTCGAGCAAGAGTATCTTCAATATCAACCTTAGTCAGTGCTGCAATTTC
AACTACATCATCAGCAGTACTAATTCCAAGGGCATTTGCTGCACGAGTTTCACGGATATATTCCAATTTA
ACTGCAAGTTCTTGGCGAGCATCATCTAACTCAACTACTTTCTTGGCGATTTCAATTCGCATTTCAGCAT
AACCATCAGCTTTAGTAGTCAGCTGTTCAGCTGTTCGACGATATAGCAAGCCGAGTTTAGCATGCATTGT
TACATCTTGACCTTCGGAAAGAAGCTTGCGAATTTCACGCTCTTTTGATTCGGCCTGTTTATTCTTTTCA
ACAATAAGTTCACGAATACGTTTTTCTTCATTAATAGATTTAACAGAAGCAGTTTTTAGGTCTTTAATTT
TATCAAGTAGTTTTGCTGCTGCAGCAGTATACTGTTCTTCAACAGATAGATTTTTAGCCATTGCAGAACC
AAGTTTAGTGCGAATAAACTCAACAATTTTCTTCAGTGTGTTCATAGTATTTCCTTAGGTTGGTATAATT
AGATAATATAATATCACGTTTCTAATAGATTGTAAACTTATTCTTCGTCTAGCTCGTCGATAAAGGCGTT
GATGGCCTCGATAATGGCATCATTGATAGCCAATAAAATAAAATCATCGTCAGTACCTTTAGAAGATTCT
AAAGCATTGATATATGCTTGGTTGACGAGTTCCCAAGCCTTTTTAAAATAAGGAGCTTCATCATCAGGAC
AAATATCCCGCACGCCTTCAAAGATACGTTTGGCATAATCTAACACCCATTGTACAGGCATGTTTTGAGA
ACGTTCGTTAAACTCTTTAAAGTCCTTAGATTCAAAAAGCTCTTCAGGATAATTATTTCTATTACAAAAA
GCTTTACTAAAGTTACGTTTCATAATGTTTTCCTCATTTGTATAGGCTCATAATATCTCAATCATSAGCC
TATGTAAACTTATTTCATATTATTGAAATATTCTTCTGCGATTTCGTCGTTATCATGGTAAACTTTAGAA
GACAGTTTAACATAACTTTCAGCAGTGAACATGTTAATCACAACCTTTACAGTATACCACTGACCGTCTT
CATTACCCATTACTGCGTAAGTTTCAAACATCGGATGGTCAGGACCGATAACTTTAATATCATTCACCGT
ACGACCGAAATCTTCTGAAACACATTTCATAAAGAAGTTGAAAAGTTCACCGTAATTATCCATTTTATTC
TCCAAGTTATTTTCTGTATCAGTAGTTGATAGTTGTATAGTACCATGGAAGAACAAGGATGTAAACAGTT
TTGTGAAAAAATTTTTAAAAAGTTTTAGGGAATTCTAGGGCGGAGAGGGGCAATTAAAAGATAGGATAAT
ATATTATAAAGGGTATAAACTAAATGATGCCTAGAGAGGTCTGGAAAGGCTTAGATACCAAAAAGCCCCA
ACCTTTCGGTCGGGGCTAACCGTTGCGGCAACCTTGTCGGGGTTCCACCTGCCAAGGCAAGTGTTTGTAC
GAAACGCCGGGATTCGAACCCGGTTATTAAGTAGTTGACGCTACTCAATATTTTTAAAAGGCCATATCTC
AACCATATCCGAACGTTCCGTCAAAAACGCTACTCGGCTTACGGCAAAGATATTTCCTCGAATCGATAAT
TTGGTGCGCCGTTTCTGCTGTGATGTAAGAGGGCATCAATAAACGCAAAGATTATTAACGCAATTCCTTA
CTCAGGGAACCATCAGTCCGACGACTTACCGGTAGCGACCCGGTTTCTCATTTGGTATCCCGCCCTGGGA
TCGAACCAGGACCGCAAACTTAGAAGGATCGTATGCTATCCATTACACCAGCGGGACGTAATTTAAAATT
TCATTTTTCGACCTTTAAACCATCCTTCTGGAATTGGGTCAGTTTTCTTAATACGTTTAGAAACTTTTTC
ATCTAATGAATGAATCCACATCATACCGAATTGGGAATTCTTTTCACCTTTCTGGTGATTATTTTTGGCG
TGAGATTCTTTCATTTTATTAATAGTTTCAGGAGTATGATGCTTATTTAGAAATCTGCTATTATTTAAAA
ATTTTTCCCTGTATTCAGGAGTTGACCACAAACGTTTAAATACATTTGAACCAATTTTACGATATTTTTC
TTGAAGTAAAATATCATTTTCAAAACGTGACTTAAACGATTTAGCTCCTTTTAAGCTAGCATCTTTCTTC
 
TGGTTTAGCATTCCAGGAATATTTACATGATCCCATCCACCTTCACCGCCAAGTTTTAAATTATACACAT
CTGGTCTATTTAAAAACTCTTCTGTGACAATATTTTTCTCGGCTTCAAGCATAGATTCTTTATCGTCAAA
ATACTCTAATATTTCTTTAGAAAAATTTTCTATACCATATTTATCTTGGGCTCTTTTTAATAATTTACCA
GAACCCATATATCCATCATCTAAATTTTCGGTAGAATGCACACCAATATAAATTTTATTATTAATTTTAT
TTGTTATTTTATAAGTGTAATAGAACATAAATATCTCCTATTTCTAAGAGTATTTATGTTCTCAAAATAT
GACCCAGACCAGATTTGAACTGGTAACCTTTCCCTTATGAGGGGACTGCTGCTAACCATTGAGCTACAGG
GCCTTGGTGCTGATTGACGGAATCGAACCGCCGACATCCTCATTACAAGTGAGGTGCTCTACCTACTGAG
CTAAATCAGCAAAATTACGGAGGCGATAGGATTTGAACCTATGAGTCGCCGGAGCGACTGCCGGTTTTCA
AGACCGGTGCATTAAACCACTCTGCCACGCCTCCAGTCTCCATACAAGGATTTGAACCTTGGACCTCCTG
ATCCCAAATCAGGCGCTCTACCAAACTGAGCTACACGGAGTAAATTAAATTGGAGCGGATAATGAGAATC
GAACTCACATCATCAGATTGGAAGTCTGAGGTAATACCATTATACGATATCCGCAAATTTGGTGCGAGAA
GTGGGACTCGAACCCACAAGGAAATCATTCCGCAGCATTTTAAGTGCTGTGCCTTTACCAATTTGACCAT
TCTCGCGCTGGGAATAAAGGACTCGAACCTTTGCATCTAGCAGTCAAAGTGCTATGCCTTACCAACTTGG
CTAATTCCCAATTATTAACAAAGGCTCTCTAACAAGAACCCTTGATGATAGAGGGTATTAATCAGTGCGG
TATGAGTTAATAATAACAAATAATTCTTAAAGCATATTTACCATTTATGATGATACGTATTTACGATACA
TTCAAGACCCAAAGGATTCTTGAAAATATCATATTCAAGAGGACCTTTTTCTGTTTCAATAAAGAAATCA
AAATTTACTGTATTAAATTTACGGTCTTCCTTTACTAATTTAACTTGAGAAGATGAACGATCAATGTAAA
CCTTTTCAACTTCAAAACACGTTAAAATGCCATAATCATCAATCAAGGCTTTAGCTGCTTCTTGATCATA
TTTATATCCATTTTCAACGGATGATACTTTCGCATAAAGAATCATCATCAACCTCTATCAACAATAGCAT
GAGTATGGGCATTTACGATTTGCCACCAGTCGAAACGATTGGAACCATAATCTGGTTTATTTTCATTTTC
TTTAATGATATCACGCAGTTTATCTTCTGTTTCAGCATACGCAATTAAATCATCATATCCACCACAAGGA
TAATAATTATCACCTGCGAATAAAAGAAAATTTACCTTAGATGGATTTACGTAATAATGGTCTTTAGGAT
ATTTAGTTCCTCTCCAATCAGTTACTTCAACATAACGGTAAGAAAATCCATTTTTACTTTCAATCCAACT
CCACGCTTCAAAAGGAGTATTAAAAACTTTATCAGGTATTAAATTACCTTCAAAATGAGAAGGATTTGCA
TAATCCCCGGCATAAACATAATATTCGTTAATACTCATTTATTCACCTTTAGAAATTTTATCCATAACGA
TAGCAATTAAACCAATTAAAAATGCTACTACAAGTGAAAACACATTTTCTGCTGTAGTTAATAATCCGCA
TATAAATCCAACAAACATTGAAAAACTAAAAGCAGAAGCAGAAATTGCAATAGCAACATTTCGAATTAAT
TCACAGCGTTTCATTTTATTCTCCTCAGTAGTTGATAGGGTAATAGTATCACAGCTAAAACCCTATGTAA
ACAACTTTGTGAAATATTTATTACAAAAGATTTTTAGCAATAATCTTGAGATGTGCCGCAGAAATGTGTT
TAGCTTTAAACAACGCAGTTTCTTCAGCAGGAGAGATAACGATTGTAGCACCATCCTTTTTAGCAGACCA
CCCATCACCTAGGTAAACAGTACCTTTGATTTCTTCGCCATCAACCAGACTAATCATTGGTTTACCTTCT
CGTCCTTTATTTGCTTTAATAACTTCAGAAGTAAGAGTAGCTTCGGTAATGGTAGAAACCGGGGTAGTTG
TAGAAGTAAATTCTTTAAATGTTTTCATTTTTATTTTCCTAATTAATTTTGATGAGGTAATAGTATCACT
ACCTCATCAGTATGTAAACAACTTTGTGAAATTATTTTAAATCATCTGCCCAATCGAGTTTAAGAGGCTC
TTTGTATTCACGATCTAATACGACCGGAATTTGTACATCACCGCTAAATGATAAGGGCCCAACATTATAA
GACAATGTTATATGCGGTGTGTAATCATCAAAATCATGTGTAGCACCTAGTGCCCGCGCATACATGTGTC
GACAGCGCAGATATTCAGAATCTAGCACAAGTACAAGAGTCGATCCATCTTGTGTTTTCCATACTTCTAA
ATGTCCAGAAGAAGCTACTTCAAAACTTCCACTCGATGGAACATATGGAACATTTACTCTTGAATAACAT
ATAGTCGAATGAATTTTTTCTCTAGGAACTGGATTAGGAACACGTAAAGAGCGCTGAAGTTCTTCCAGCG
CATCAAGTGTTAATTCTGAAAACTTAGCTGCTACATAAAGACCCGTTGAAAAGTCTTTAAATTCCATCAT
TCTTCATCTTTTGCTTCATCTGCAGATTCAGCAGTAAGATTTTTGACAGCTTCAACGATTTCTTCAACTT
 
TGATAGTATCGCCAGTGATACCTACTGCACGAGCAATTTCAGCCAAAGTTCCTTGCAGAATTTTGGATTC
TTCCATCAGACGAGCAGCTTGATCCTGCGTATCAAGAATGCGAGATTTCAGAGTTACGATTTCAGCAGAC
AGTTTTTGTTCAACAGTTTGTTCAGACATTATAGTACCTTTAGTGTATTTTTAATTTTAGAAAAAAGTTC
TTCAAGAGAACCATCGTTTGTAATTACTAAATCGCCATCACGAATTGGCAATCCAGCTTCTGTAATATGT
GTATCATTGGATTTTTGACCAGGACGAACTACATGAATTACTGTAGCACCCATCGCCCTAGCCGCATCCA
TTTCATGATCTTGACGGGTATCAGGAACGATATAATAATCATAACCTGAGTTAAATTTATCAAGATAATC
TAAAGCAAATAATTTTACCCAGTACATGCGGTCGAAGTTATTAACAATCAAATCCGTACCTAGGGCTTGC
ATCAGACGACGGACTGACCATTGATCTTCAATATTATTTATAACGTCAGTAATCTTATTAAATGCTACGA
AATTAACTGATTCTTTTCCTTCGTCATCAAAAACAAACACACCTTTAATTGGGCTTTTACCATTAAGATA
ACAAAATGCTTGTTCCATAATCGTGATTACTTCTAATTTAGTCAGATTTAAATTAGTCTCACGATCATAG
TCAATTCCTTCAAACTCTTTACGAGTTAAGCAAGGATAGTCAGTGTTTGCTGCAAATACTCCCCATGCAT
AAGCCAATGCATCCTTAATAGGACCAGCAAGTTGGTATTTAACTGCAGAATAATTGCTCATGATAAAATC
AGCAGTAGTATCTTTTCCACTACGCTTTACACCGCTTAAAAAGATTAGTTTCATGTGTTTCTCCTCAAAT
TTAATTAAGATTATAACACACAAAACTGAAGCATTAAACTTCTGCTATAATTTTACCATCTTTTTCTACT
TGAAAATAGGTGTAAGGAATTGTTGCAGTACATACTAAAGCCGGGTCTGAATCTTCCGTGTAGCTAAATT
CTACTTCAGATAGGTCAGAAACCCAAGGCTTATAAAAATTTATTGACATCACGATTTCAGTTTTGCTATT
ATCTAAGATGTAAAGCGTAATGTACTCAGGACCTGTTTTTTGGGCAGTATTTTCACCTGTAAGATAGTTG
CTAGTTCCTAGCATCCATTCATACATTCCTATCCACGACTTAAGTTCTTCATCAACTATAAATCTCACAA
TGAGTGGATCATACTCAAATGTAACACCTGGACGTTGTGCTCGGCCCAGTCCAAACGGCCCAGTCACGGT
ATCAGTAACAGGTATTCTAATTCCTGGAATAGGAACTGACTGAGCATTTAAAGTAAAAGCAGATGTAGTA
TTACTATGTGGTATTGATACTACAAAGTTAGTTGTATTTGCTTGGTTAAAAATTTGTTGCAGAGCTTGCG
ACATATATTCCTCATAATGCTTTATAACTGTTGGTGGTATAATGGGTCTAAGTCCCTTCCATTCAATTCC
ATTTAGAACAAACAACAGAAAAGAATGGAAGATAATAGAATTAGATATTTGACCAGACTTTGTTTGCAGA
GAAACGTTTTCCTTTTGAAACGAACTGCTGAAGTGGCATCAACACAACGTTCGCCCAGTCTTTCGGGGCG
ATTTCAACAAGGCTACCCATAATATTACCAGGTATATATGCCTTAATCATTTGGTCTGCACCCCTAAATC
CTTTCACTTGACTCCAATCAATTTTTAATTTCGTTTTATTAGTAATAGTAGGTGTATTTGCATATTGCTT
TAAAAGCTCTTCTAGGAATTGCTGACGAGCTTTAGGTGGAATATAGTGCAAGTTTAATCCGTACATTAAA
TTATGCTTACCTAAACCAAGGTAAATTATCAAAGGAAATTTATCCCAGTAAGGAAGAGTTTCCTTGTGTT
TAGCATCATAAGCAAAAGCATATATTCGTCCCGGCTGCGGGCGAACAACTTTATGTCCTTTTACTTGCTT
AATAGTTTCAGCAAACCACTTTCTGGTTTTATTATTAATTGCTGCGCCTTCATTACGAATTTTATCACGC
AATGTTTGTCTGAATGAATTTATCATAAGCAGTTGTCTTTCTTGCTTATTGAGTTTATTCATTGGTTTTG
ATTCAAGTTTTTGAATCTTTTCAGCCGTTTTAATTCCTGAAGCATATTTTGACATTGCTGAAGTAAACGT
AGAGTATTTGATTCCTCTTTCTTCAGCAAATTGCTTTCCTGTCATTCCTTTTGCTTTGGCCTTTTTATAT
TCAAGACCTATCTGAATCCATTTCTTTTCGTTTAATGATTGCTTAACCTTTGGAACTTGGGGAGTGCTTT
CATTAATTATTTGAAAAATAGCCATTATGCCCCCTTAAAGCCAAGAGCTCGTAATCCATCTTCTGTTAGA
ATTCTAAATTTTATTCCACGCTTTTCAGCTAAAGATTGTGCTGCTTTCCATTTGTCAGTGTTCACAGACC
AGGTATAAATTTCATTCATAAATCTTTTCTTCGCTGCGGTCGTTAGATGTGCTGGTTTAACTGGTGGTTG
TGTTTCTTTTTTAGGTTTTATTTCAATAAAAAATTCTTGTCCAGAAGAATCTTTCATCCAAATATCCATG
AAGTATCTACGTTTTTTCCCTTCTGCATTACAAAAATAAGGAATTACTGCTGTTTCACTACCCCATGCAA
TAATTTCTGGATTTTTATCTAACCATTCAAAAAAGAATTTTTCCCAATTTGATCTATACGTAATTTTTTT
AGGGTCACCTCTATACTTTGATATATTTTTAGGAACCCATTTTCCAGAATATGCCATTGGATTCTCCTTA
 
TAAATAGATAATATATTTATAAACAGGAGGGCCCATGCTCTTTACATTTTTTGATCCGATTGAATATGCG
GCCAAAACGGTGAATAAAAACGCGCCGACTATTCCTATGACAGATATTTTTAGAAACTATAAAGACTATT
TTAAACGCGCTCTTGCGGGATACCGCTTACGTACTTATTATATTAAAGGTTCACCACGCCCGGAAGAATT
AGCAAATGCTATATATGGAAATCCACAGCTGTATTGGGTTTTATTGATGTGTAATGATAATTATGACCCG
TATTATGGATGGATTACTTCGCAAGAAGCTGCTTATCAAGCATCTATACAAAAATACAAAAACGTAGGTG
GAGACCAAATAGTATATCATGTGAATGAGAACGGTGAAAAATTTTATAATTTAATATCATACGATGATAA
TCCATATGTTTGGTATGATAAAGGCGATAAAGCTAGAAAATATCCTCAATATGAAGGAGCGCTTGCTGCG
GTCGATACGTATGAAGCTGCTGTTCTTGAAAATGAAAAACTTCGTCAAATAAAAATAATAGCAAAATCAG
ACATCAATTCATTTATGAACGACCTTATACGTATAATGGAGAAATCTTATGGAAATGATAAGTAATAACC
TTAATTGGTTTGTCGGTGTTGTTGAAGATAGAATGGACCCATTAAAATTAGGTCGTGTTCGTGTTCGTGT
GGTTGGTCTGCATCCACCTCAAAGAGCACAAGGTGATGTAATGGGTATTCCAACTGAAAAATTACCATGG
ATGTCAGTTATTCAACCTATAACTTCTGCAGCAATGTCTGGAATTGGAGGTTCTGTTACTGGACCAGTAG
AAGGAACTAGAGTTTATGGTCATTTTTTAGACAAATGGAAAACTAATGGAATTGTCCTTGGCACGTATGG
TGGAATAGTTCGCGAAAAACCGAATAGACTTGAAGGATTTTCTGACCCAACTGGGCAGTATCCTAGACGT
TTAGGAAATGATACTAACGTACTAAACCAAGGTGGAGAAGTAGGATATGATTCGTCTTCTAACGTTATCC
AAGATAGTAACTTAGACACCGCAATAAATCCCGATGATAGACCGCTATCAGAGATTCCGACCGATGATAA
TCCAAATATGTCAATGGCTGAAATGCTTCGCCGTGATGAAGGATTAAGATTAAAAGTTTATTGGGATACC
GAAGGATATCCGACAATTGGTATTGGTCATCTTATCATGAAGCAGCCAGTTCGTGATATGGCTCAAATTA
ATAAAGTTTTATCAAAACAAGTTGGTCGTGAAATTACAGGAAATCCAGGTTCTATTACAATGGAAGAGGC
GACGACTTTATTTGAGCGTGATTTGGCTGATATGCAACGGGACATTAAATCACATTCTAAAGTAGGACCA
GTCTGGCAAGCTGTCAACCGTTCTCGTCAAATGGCGTTAGAAAATATGGCATTTCAGATGGGTGTTGGTG
GTGTAGCTAAATTTAACACAATGTTAACTGCTATGTTAGCAGGAGATTGGGAAAAAGCGTATAAAGCCGG
TCGTGATTCATTGTGGTATCAACAAACAAAAGGCCGTGCATCCCGTGTTACCATGATTATTCTTACGGGG
AATTTGGAATCATATGGTGTTGAAGTGAAAACCCCAGCTAGGTCTCTATCAGCAATGGCTGCTACTGTAG
CTAAATCTTCTGACCCTGCTGACCCTCCTATTCCAAATGACTCGAGAATTTTATTCAAAGAACCAGTTTC
TTCATATAAAGGTGAATATCCTTATGTGCATACAATGGAAACTGAAAGCGGACATATTCAGGAATTTGAT
GATACCCCTGGGCAAGAACGATATAGATTAGTTCATCCAACTGGAACTTATGAAGAAGTATCACCATCAG
GAAGAAGAACAAGAAAAACTGTTGATAATTTGTATGATATAACCAATGCTGATGGTAATTTTTTGGTAGC
CGGTGATAAAAAGACTAACGTCGGTGGTTCAGAAATTTATTATAACATGGATAATCGTTTACATCAAATC
GATGGAAGCAATACAATATTTGTACGTGGAGACGAAACGAAAACTGTTGAAGGTAATGGAACTATCCTAG
TTAAAGGTAATGTTACTATTATAGTTGAAGGTAATGCTGACATTACAGTTAAAGGAGATGCTACCACTTT
AGTTGAAGGAAATCAAACTAACACAGTAAATGGAAATCTTTCTTGGAAAGTTGCCGGGACAGTTGATTGG
GATGTCGGTGGTGATTGGACAGAAAAAATGGCATCTATGAGTTCTATTTCATCTGGTCAATACACAATTG
ATGGATCGAGGATTGACATTGGCTAATATACTTCCAATGAGCGCTGATTTAGGAGAATCCATGGAAGGTT
CTTCTATCGACGTCACCTTTACCGCTCAATTAGAAACAGGTGAAACGTTAGTATCTATAAATATAACTAG
TTACGAAGAAACTCCTGGGGTTTTAGTAGAAGAAAATCGCTTATATGGAACATATGAATCTGTATTTGGT
TTCGGAAATGACGCGTTGAAATATCGTTTAGGCGATGAATTTAAAACTGCTGCTTCATGGGAAGAACTTC
CTACTGATTCTGATACTCAGTTGTATTTGTGGAAAGCTCCTCAAAACCTCCAGAAGACATTCACTTACGA
AGTAACATTAATATATGACTACCAAGAACAAAGTGAATCTGGGGGTTCTGGCAGTAATTCTAGGTCATCT
TCTGATACTACTGAACCGACAGATCCTCCTGCTCCAGTAAGAAAAACTCTAGTTAAAAATTATACTAAAA
CTATAGTTGGAAATTGGAGTCGTTGGGCTAATAAACTGAGAAAATATGCCTATGCAAGACCATAAATATT
 
TTTATTTGTATTCAATAACTAATAAAACAACAGAAAAAATTTATGTAGGCGTCCACAAAACTTCAAATTT
GGATGATGGGTATATGGGTTCTGGCGTTGCCATTAAAAATGCCATTAAAAAATATGGCATAGATAATTTT
TATAAGCATATTATAAAATTCTTTGAATCTGAAAAAGCTATGTATGACGCAGAGGCAGAAATAGTCACAG
AGGAATTTGTTAAATCTAAGAAAACTTATAATATGAAACTAGGCGGTATCGGTGGCTTCCCAAAACATAA
CACAGCGGGTGCTAAAAATGGATTTTACGGTAAATCTCATTCGCGTGAAACTAGATTGAAAATTAGCATT
AAATCGTCTAGAAAAAGAGGGCCTAGAGGGCTAGAGGTAAAACTCTGAAGATGTGTGGCGCCAATAACCC
AAGGTATGGCAAAATAGCCCCTAATGCTAAATCTGTTATTATCAACGGCGTTTTATATAAAAGTATTAAA
ATCGCAGCTAAAGCTCTTAATATAAATTATAGTACCTTAAAGGGGCGAGTTAAAGCGGGGTATTATAAAT
GTCAGGATTAAGTTATGATAAGTGTGTTACTGCTGGCCATGAAGCGTGGCCTCCAACAGTTGTGAATGCT
ACACAAAGTAAAGTATTCACTGGAGGAATTGCTGTTCTCGTAGCAGGCGATCCAATTACAGAACATACAG
AAATTAAAAAGCCGTATGAAACACATGGCGGAGTGACACAACCTAGAACTTCTAAGGTATATGTCACTGG
AAAGAAAGCTGTTCAAATGGCTGATCCAATATCATGCGGTGATACTGTGGCTCAGGCATCATCTAAAGTA
TTCATTAAATAGGATTTAAAATGGCAAATACCCCTGTAAATTATCAATTAACAAGAACAGCAAATGCTAT
TCCCGAGATATTCGTCGGGGGTACATTTGCTGAAATAAAACAAAACCTCATTGAATGGCTTAATGGCCAA
AATGAATTTTTGGATTATGATTTTGAAGGCTCAAGATTAAACGTTCTGTGTGACCTTTTAGCTTATAATA
CATTATACATTCAGCAGTTTGGTAATGCTGCTGTGTATGAAAGCTTTATGCGTACTGCTAACTTACGAAG
TTCAGTTGTTCAAGCTGCACAAGATAACGGATATTTACCTACTTCAAAATCCGCTGCGCAGACCGAAATT
ATGTTAACATGCACTGACGCATTGAATAGGAATTACATTACTATTCCTCGCGGAACTCGCTTTTTAGCAT
ATGCAAAAGATACTTCTGTTAATCCATATAACTTCGTTTCTAGGGAAGACGTTATTGCTATTCGTGATAA
AAATAACCAATATTTTCCGCGTTTAAAATTGGCCCAGGGACGTATAGTAAGAACTGAAATCATTTATGAT
AAATTAACACCTATTATCATTTATGATAAAAATATTGATAGAAACCAGGTTAAATTATACGTTGATGGAG
CGGAATGGATTAACTGGACGAGAAAGTCAATGGTTCATGCTGGTTCAACATCAACGATTTACTATATGCG
TGAAACTATTGATGGAAACACTGAATTCTATTTTGGTGAAGGTGAAATTTCTGTTAATGCTTCTGAAGGA
GCTTTGACCGCTAATTATATCGGAGGTCTTAAACCTACTCAGAACTCTACGATTGTTATTGAGTACATTA
GTACTAATGGTGCTGACGCGAACGGAGCAGTCGGATTTTCATACGCAGATACATTAACAAATATAACTGT
CATCAATATTAATGAAAATCCAAACGATGATCCAGATTTTGTTGGGGCAGATGGAGGCGGTGATCCAGAA
GATATTGAGCGTATTCGCGAATTGGGTACTATTAAACGCGAAACCCAACAACGATGCGTAACTGCGACTG
ACTATGATACATTCGTTTCAGAGAGATTTGGTTCTATTATTCAAGCTGTTCAGACTTTCACTGATTCTAC
TAAACCTGGGTATGCATTTATTGCTGCTAAACCTAAATCAGGATTGTATTTAACTACCGTACAGCGTGAA
GATATTAAAAATTATCTCAAAGACTATAATTTAGCTCCTATTACGCCATCAATTATTTCTCCTAATTATC
TTTTTATTAAGACTAATTTAAAAGTCACATATGCTTTAAATAAACTGCAAGAATCCGAACAGTGGCTTGA
AGGTCAAATAATTGATAAAATAGATCGCTATTATACCGAAGATGTAGAAATTTTTAACTCGTCTTTCGCT
AAATCTAAGATGTTGACATATGTAGATGATGCAGATCATTCTGTCATTGGTTCATCAGCGACTATTCAAA
TGGTTCGTGAAGTACAAAACTTCTATAAAACGCCTGAAGCGGGTATTAAATACAATAATCAAATAAAAGA
TCGTTCTATGGAATCTAATACGTTTTCATTTAATTCTGGACGAAAGGTTGTAAATCCTGATACTGGTTTA
GAAGAAGATGTATTATATGACGTTCGTATAGTATCAACAGACCGAGATTCTAAAGGAATTGGTAAAGTTA
TTATTGGTCCATTTGCTTCTGGCGATGTTACAGAAAATGAAAACATTCAGCCGTATACAGGCAACGATTT
TAACAAATTAGCAAATTCTGATGGACGCGACAAATACTATGTTATCGGTGAAATAAATTATCCAGCTGAT
GTGATTTATTGGAATATCGCTAAAATTAATTTAACATCTGAAAAATTTGAAGTTCAGACCATTGAATTAT
ATTCTGACCCAACCGATGATGTTATCTTTACTCGCGATGGTTCACTGATTGTATTTGAAAATGACTTACG
TCCACAATACTTAACTATCGATTTGGAGCCTATATCACAATGACAGTAAAAGCACCTTCAGTCACTAGTC
 
TCAGAATTTCCAAGTTATCCGCAAATCAGGTGCAAGTACGCTGGGATGACGTTGGTGCTAATTTCTACTA
TTTTGTAGAAATCGCTGAGACAAAAACAAACTCGGGGGAAAATCTCCCGAGTAATCAATATCGTTGGATT
AATTTAGGATATACAGCAAATAATAGTTTCTTTTTTGATGATGCTGATCCATTAACAACATACATTATTA
GAGTAGCCACAGCTGCGCAAGATTTTGAGCAGTCTGATTGGATTTATACCGAAGAGTTTGAAACTTTTGC
TACAAATGCTTATACATTTCAAAACATGATTGAAATGCAATTAGCCAATAAATTCATTCAGGAAAAATTT
ACTCTTAATAATTCTGATTATGTTAATTTTAATAATGATACTATAATGGCTGCATTGATGAATGAATCAT
TCCAATTCAGCCCATCGTATGTTGATGTTTCATCAATAAGTAATTTTATTATTGGTGAAAATGAGTATCA
TGAAATACAAGGTTCTATTCAGCAAGTATGTAAGGATATTAACCGAGTTTATTTGATGGAATCAGAAGGA
ATTCTATATCTTTTTGAGCGCTATCAACCTGTAGTTAAAGTATCCAATGATAAAGGACAAACCTGGAAAG
CTGTAAAGCTCTTCAATGACCGTGTAGGATATCCTTTATCTAAGACAGTATATTACCAATCTGCGAACAC
AACATACGTTCTAGGATACGACAAGATTTTCTATGGCCGCAAATCTACTGATGTTAGATGGTCAGCCGAT
GATGTCAGATTTAGTTCTCAGGATATAACATTTGCTAAACTTGGCGACCAATTACATCTAGGATTTGATG
TAGAAATTTTTGCCACTTACGCGACTTTACCAGCGAATGTATACCGCATTGCAGAAGCTATTACTTGCAC
CGATGATTACATTTACGTTGTCGCCAGAGACAAAGTTAGATACATAAAAACGAGTAATGCACTTATAGAT
TTTGATCCATTATCTCCAACATATTCGGAAAGACTTTTTGAACCTGATACCATGACTATAACCGGAAATC
CTAAAGCAGTATGCTATAAAATGGATTCTATCTGTGATAAAGTTTTTGCTCTTATTATTGGTGAAGTTGA
AACATTAAATGCTAATCCTAGAACATCAAAAATAATTGATTCCGCTGATAAAGGAATATATGTTTTAAAT
CATGACGAAAAAACATGGAAAAGAGTTTTTGGTAATACCGAAGAAGAAAGAAGACGTATTCAACCCGGAT
ATGCGAATATGTCAACTGACGGTAAATTAGTTTCTCTGTCTTCGAGTAATTTTAAATTTTTAAGTGATAA
TGTTGTTAATGACCCTGAAACTGCAGCAAAATATCAGTTAATTGGCGCTGTTAAATATGAATTTCCTCGT
GAATGGTTAGCTGATAAGCATTATCATATGATGGCATTTATAGCGGATGAAACATCTGATTGGGAGACTT
TTACTCCTCAACCAATGAAATACTACGCAGAACCATTCTTTAACTGGTCTAAAAAATCTAACACACGTTG
TTGGATAAACAACTCTGATAGAGCTGTGGTAGTTTATGCTGATTTAAAATACACTAAAGTTATAGAAAAT
ATTCCGGAAACATCACCAGATAGATTAGTTCATGAATACTGGGATGATGGTGATTGCACTATAGTAATGC
CAAATGTCAAATTCACTGGATTTAAAAAATACGCATCAGGAATGCTTTTCTATAAAGCCTCCGGTGAAAT
AATTTCTTACTATGATTTTAACTATCGTGTGAGAGATACAGTAGAAATTATTTGGAAGCCAACTGAAGTA
TTTTTAAAAGCATTTTTACAAAACCAAGAGCATGAGACTCCTTGGTCACCAGAAGAAGAGCGTGGATTAG
CTGACCCTGATTTAAGACCATTAATTGGCACAATGATGCCTGATTCTTATTTGTTACAGGATTCGAATTT
TGAGGCATTTTGCGAAGCATATATTCAGTATCTTTCTGATGGATATGGAACTCAATACAATAATTTACGA
AATTTAATTCGTAACCAATATCCACGAGAAGAGCACGCATGGGAATATTTGTGGTCAGAGATATATAAAA
GAAACATTTATTTAAATGCTGATAAACGCGATGCTGTTGCGAGATTCTTTGAATCACGTAGCTATGATTT
TTATTCTACTAAAGGAATTGAAGCATCATACAAGTTTCTTTTTAAAGTTCTTTATAATGAAGAAGTTGAA
ATTGAAATTGAATCTGGGGCTGGTACTGAATATGATATAATCGTTCAATCTGATTCTTTGACTGAAGATT
TAGTAGGACAAACGATTTATACGGCAACAGGAAGATGTAATGTTACTTATATAGAAAGAAGCTATTCTAA
TGGTAAATTGCAATGGACCGTAACTATTCATAATCTTTTGGGACGATTAATTGCTGGTCAAGAAGTTAAA
GCAGAAAGACTCCCTAGTTTTGAAGGCGAAATTATTCGTGGGGTTAAAGGAAAGGATTTGCTTCAAAACA
ATATAGACTATATTAATAGAAGTAGATCATACTATGTAATGAAAATTAAATCCAATTTACCTTCTTCCCG
CTGGAAATCTGACGTTATTCGTTTTGTTCATCCAGTAGGATTTGGATTTATAGCAATTACCCTTTTAACA
ATGTTTATTAATGTTGGTTTAACTCTTAAACATACAGAGACTATAATTAATAAATACAAAAACTATAAAT
GGGATTCTGGATTGCCTACTGAATATGCCGACAGAATAGCTAAATTAACTCCAACCGGTGAAATTGAGCA
TGATTCAGTAACAGGCGAAGCAATTTATGAGCCTGGCCCAATGGCTGGTGTAAAATATCCTCTTCCTGAT
 
GACTATAATGCTGAAAATAATAATTCAATATTTCAAGGTCAATTGCCGTCTGAACGACGTAAATTAATGA
GTCCTTTATTTGATGCATCTGGAACAACATTTGCGCAATTTAGAGATTTAGTTAATAAACGTCTAAAAGA
TAATATAGGAAATCCAAGAGACCCTGAAAATCCAACACAGGTTAAAATAGATGAATGATTCAAGTGTTAT
CTATCGTGCGATAGTTACTTCAAAATTTAGAACAGAAAAAATGTTGAATTTTTATAATTCAATTGGAAGT
GGTCCGGATAAAAACACTATCTTTATCACATTTGGAAGATCAGAACCGTGGTCATCAAATGAAAATGAGG
TGGGCTTTGCCCCACCTTATCCAACCGATTCTGTATTAGGCGTAACTGACATGTGGACGCATATGATGGG
AACAGTAAAAGTTCTTCCATCAATGCTTGATGCTGTTATTCCTCGCAGAGATTGGGGAGATACTAGATAT
CCGGATCCATACACATTTAGAATTAACGATATTGTAGTGTGTAACTCAGCTCCTTACAACGCTACTGAAT
CAGGCGCTGGTTGGTTAGTGTATCGTTGTTTAGATGTTCCTGATACCGGAATGTGTTCAATAGCATCTTT
AACTGATAAAGATGAATGCCTTAAGTTAGGTGGAAAATGGACTCCTTCTGCTAGGTCAATGACTCCGCCT
GAAGGTCGAGGAGATGCTGAAGGAACAATTGAACCCGGAGACGGGTATGTGTGGGAATATCTATTTGAGA
TTCCGCCTGATGTATCTATAAATAGATGCACGAATGAATATATCGTGGTTCCTTGGCCTGAGGAATTAAA
AGAAGACCCGACTAGATGGGGATATGAAGATAATCTCACTTGGCAACAAGATGATTTTGGATTAATTTAC
CGTGTTAAGGCAAATACTATCCGTTTTAAAGCATATTTAGATTCAGTTTATTTTCCTGAAGCTGCATTAC
CAGGAAATAAAGGATTTAGACAAATATCAATAATCACGAATCCTCTTGAAGCTAAAGCTCATCCAAATGA
CCCAAACGTTAAAGCTGAAAAGGATTATTATGACCCAGAAGATTTAATGAGGCATTCGGGTGAAATGATT
TATATGGAAAATAGGCCACCTATTATTATGGCAATGGATCAAACAGAAGAAATCAATATTCTGTTTACAT
TTTAAATTAAGGGAGCCCATGGGCTCCCTTTTTCTTTATAAATACTATAAACTCATAAGGAAACCGCTAT
GTTCATTCAAGAACCAAAGAAATTGATTGATACCGGCGAAATTGGTAACGCTTCTACTGGTGATATCTTA
TTCGACGGTGGTAATAAAATTAATAGTGATTTTAACGCAATTTATAATGCGTTTGGCGATCAGCGTAAAA
TGGCAGTAGCAAATGGCACTGGAGCAGATGGTCAAATTATCCATGCTACTGGATATTATCAAAAACACTC
TATTACAGAGTACGCAACTCCAGTGAAAGTTGGCACTAGACATGATATTGATACCTCTACTGTAGGTGTT
AAAGTTATCATTGAAAGAGGCGAACTCGGCGATTGTGTTGAATTCATTAACTCTAATGGATCAATATCAG
TTACTAATCCTTTGACAATTCAAGCTATTGATTCAATTAAAGGTGTTTCAGGTAATTTAGTAGTAACTAG
CCCATATAGTAAAGTTACTTTACGCTGTATTTCATCTGATAATTCTACGTCGGTTTGGAATTATTCTATT
GAAAGTATGTTTGGACAAAAGGAATCACCAGCTGAAGGTACATGGAATATTTCTACATCTGGATCAGTTG
ACATTCCATTATTTCATCGTACTGAATACAATATGGCTAAATTGCTAGTTACGTGCCAATCGGTAGATGG
AAGAAAAATTAAAACAGCAGAAATAAATATTCTTGTGGATACTGTTAATTCAGAGGTAATTTCTTCTGAA
TATGCTGTCATGCGAGTTGGGAATGAAACCGAAGAAGACGAAATCGCTAATATTGCATTTAGTATTAAAG
AAAATTATGTAACGGCGACTATAAGTTCTTCAACTGTCGGTATGAGAGCAGCAGTTAAAGTTATCGCTAC
GCAGAAAATCGGGGTGGCTCAATAATGAAACAAAATATTAATATCGGTAATGTTGTAGATGATGGTACCG
GTGACTACCTGCGTAAAGGTGGTATAAAAATAAATGAAAACTTTGATGAGCTTTATTATGAACTCGGTGA
TGGTGATGTTCCATATTCAGCCGGTGCCTGGAAAACTTATAATGCTTCATCAGGACAAACATTAACAGCA
GAATGGGGAAAATCATACGCTATTAATACATCTTCTGGAAGAGTGACTATAAATCTTCCAAAGGGTACAG
TTAATGATTACAACAAGGTAATTAGAGCTAGAGACGTATTTGCTACATGGAACGTCAACCCAGTTACACT
AGTAGCTGCTTCCGGCGATACGATTAAAGGGTCTGCAGTACCAGTTGAAATTAATGTTCGATTCAGCGAT
TTAGAACTAGTGTATTGTGCCCCAGGACGTTGGGAATATGTCAAAAATAAACAAATTGACAAAATTACCA
GTTCAGACATTAGTAATGTAGCTCGCAAAGAATTTTTAGTTGAAGTTCAAGGACAAACAGACTTTTTAGA
TGTTTTCCGTGGAACTAGTTATAATGTAAATAACATCAGAGTAAAACATCGTGGTAACGAATTGTATTAC
GGCGATGTGTTTAGCGAAAACAGCGATTTTGGCTCTCCAGGCGAAAATGAAGGAGAACTGGTTCCTCTTG
ATGGATTTAACATTCGATTAAGACAGCCTTGTAATATTGGTGACACTGTTCAAATTGAAACATTTATGGA
 
TGGTGTATCACAGTGGAGAAGTTCATATACAAGACGTCAAATTAGATTGTTAGATTCAAAATTAACGTCA
AAAACTTCTTTAGAAGGAAGCATTTACGTTACTGATTTATCAACAATGAAATCAATTCCATTTTCTGCTT
TTGGATTAATTCCAGGAGAACCTATTAATCCTAACTCTCTTGAGGTTCGTTTTAACGGGATTTTACAGGA
ATTGGCTGGCACAGTTGGAATGCCATTATTTCATTGTGTTGGTGCCGATTCAGACGATGAAGTAGAATGC
TCTGTTTTAGGTGGAACTTGGGAACAATCTCATACCGATTATTCAGTTGAAACTGATGAAAACGGCATAC
CAGAAATTTTACATTTCGATAGCGTATTTGAGCATGGTGACATTATCAATATCACCTGGTTTAATAATGA
TTTGGGTACATTATTAACAAAAGATGAGATTATTGATGAAACTGATAATCTCTATGTATCGCAAGGACCT
GGAGTAGATATTTCTGGTGATGTAAATTTAACAGACTTCGATAAAATTGGTTGGCCAAATGTAGAAGCAG
TTCAATCTTATCAACGCGCATTTAATGCTGTTTCAAATATCTTTGATACGATTTATCCTATTGGAACTAT
ATATGAAAACGCTGTTAATCCAAATAACCCTGTTACATATATGGGATTCGGCTCATGGAAATTATTTGGG
CAAGGAAAAGTTTTAGTTGGATGGAATGAAGATATTTCGGACCCTAACTTTGCTCTAAATAACAACGATT
TAGATTCGGGTGGAAATCCTTCACATACCGCAGGTGGAACAGGTGGTTCTACTTCTGTTACATTGGAAAA
TGCTAATCTTCCTGCAACTGAAACAGATGAAGAAGTTCTAATAGTTGATGAAAATGGATCAGTCATTGTT
GGTGGGTGTCAATACGATCCAGATGAATCCGGTCCAATTTACACTAAATACCGTGAAGCTAAAGCATCTA
CTAACTCTACTCACACTCCGCCAACATCAATAACTAACATTCAACCATATATTACAGTTTATCGTTGGAT
AAGGATTGCATAATGAGTTTACTTAATAATAAAGCGGGAGTTATTTCCCGCTTAGCCGATTTTCTTGGTT
TTAGACCTAAAACTGGCGACATTGATGTAATGAATCGTCAATCAGTCGGGTCAGTGACAATATCTCAATT
AGCGAAAGGATTTTATGAACCAAACATAGAATCAGCTATTAATGACGTTCATAATTTTTCTATAAAAGAC
GTTGGCACAATTATTACTAATAAAACTGGTGTTTCTCCTGAGGGTGTTTCTCAAACTGATTATTGGGCAT
TTTCTGGAACTGTAACAGACGATTCTCTTCCTCCGGGTTCTCCTATTACGGTATTAGTATTTGGTCTTCC
AGTTTCAGCAACAACTGGAATGACGGCAATTGAGTTTGTTGCAAAAGTTCGCGTTGCACTACAAGAAGCT
ATTGCGTCATTTACTGCTATCAATTCATATAAAGACCATCCAACTGATGGTAGTAAATTAGAAGTTACTT
ATTTAGATAATCAAAAACATGTATTAAGCACATATTCTACATATGGAATAACTATTTCCCAAGAAATTAT
ATCTGAGTCTAAGCCTGGCTATGGTACATGGAATTTATTGGGCGCACAAACTGTAACTTTAGATAATCAG
CAGACTCCTACAGTATTTTATCATTTTGAGAGAACAGCATGAGTAATAATACATATCAACACGTTTCTAA
TGAATCTCGTTATGTAAAATTTGATCCTACCGATACGAATTTTCCACCGGAGATTACTGATGTTCACGCT
GCTATAGCAGCCATTTCTCCTGCTGGAGTAAATGGAGTTCCTGATGCATCGTCAACAACAAAGGGAATTC
TATTTATTCCCACTGAACAGGAAGTTATAGATGGAACTAATAATACCAAAGCAGTTACACCAGCAACGTT
GGCAACAAGATTATCTTATCCAAATGCAACTGAAACTGTTTACGGATTAACAAGATATTCAACCAATGAT
GAAGCCATTGCCGGAGTTAATAATGAATCTTCTATAACTCCAGCTAAATTTACTGTCGCCCTTAATAATG
CGTTTGAAACGCGAGTTTCAACTGAATCCTCAAATGGTGTTATTAAAATTTCATCTCTACCGCAAGCATT
AGCTGGTGCAGATGATACTACTGCAATGACTCCATTAAAAACACAGCAGTTAGCTATTAAATTAATTGCG
CAAATTGCTCCTTCTGAAACCACAGCTACCGAATCGGACCAAGGTGTTGTTCAATTAGCAACAGTAGCGC
AGGTTCGTCAGGGAACTTTAAGAGAAGGCTATGCAATTTCTCCTTATACGTTTATGAATTCATCTTCTAC
TGAAGAATATAAAGGCGTAATTAAATTAGGAACACAATCAGAAGTTAACTCGAATAATGCTTCTGTTGCG
GTTACTGGCGCAACTCTTAATGGTCGTGGTTCTACGACGTCAATGAGAGGCGTAGTTAAATTAACTACAA
CCGCCGGTTCACAGAGTGGAGGCGATGCTTCATCAGCCTTAGCTTGGAATGCTGACGTTATCCAGCAAAG
AGGTGGTCAAATTATCTATGGAACACTCCGCATTGAAGACACATTTACAATAGCTAATGGTGGAGCAAAT
ATTACGGGTACCGTCAGAATGACTGGCGGTTATATTCAAGGTAACCGCATCGTAACACAAAATGAAATTG
ATAGAACTATTCCTGTCGGAGCTATTATGATGTGGGCCGCTGATAGTCTTCCTAGTGATGCTTGGCGCTT
CTGCCATGGTGGAACTGTTTCAGCGTCAGATTGTCCATTATATGCTTCTAGAATTGGAACAAGATATGGC
 
GGAAACCCATCAAATCCTGGATTGCCTGACATGCGTGGTCTTTTTGTTCGTGGTTCTGGTCGTGGTTCTC
ACTTAACAAATCCAAATGTTAATGGTAATGACCAATTTGGTAAACCTAGATTAGGTGTAGGTTGTACCGG
TGGATATGTTGGTGAAGTACAGATACAACAGATGTCTTATCATAAACATGCTGGTGGATTTGGTGAGCAT
GATGATCTGGGGGCATTCGGTAATACCCGTAGATCAAATTTTGTTGGTACACGTAAAGGACTTGACTGGG
ATAACCGTTCATACTTCACCAATGACGGATATGAAATTGACCCAGAATCACAACGAAATTCCAAATATAC
ATTAAATCGTCCTGAATTAATTGGAAATGAAACACGTCCATGGAACATTTCTTTAAACTACATAATTAAG
GTAAAAGAATGACAGATATTGTACTGAATGACTTACCATTCGTTGACGGCCCTCCTGCAGAGGGCCAGAG
CCGCATTTCCTGGATTAAAAACGGCGAAGAAATATTAGGAGCTGACACACAGTATGGAAGTGAAGGCTCA
ATGAATAGACCTACGGTTTCTGTACTAAGAAATGTTGAAGTTCTTGATAAAAACATTGGAATACTTAAAA
CATCTTTAGAAACCGCAAATAGTGATATTAAAACAATTCAGGGCATCTTAGATGTATCTGGTGATATTGA
AGCTTTGGCCCAAATAGGTATCAATAAAAAGGATATTTCTGACCTCAAAACGCTAACCAGTGAACACACA
GAAATATTAAATGGAACTAATAATACGGTTGACAGTATTCTTGCCGATATTGGTCCATTTAACGCCGAGG
CCAACTCTGTATACAGAACGATCAGAAATGATTTACTGTGGATAAAGCGTGAACTTGGACAATACACTGG
TCAAGATATTAATGGTCTTCCTGTTGTAGGAAATCCTAGTAGTGGAATGAAGCATCGCATTATTAATAAT
ACTGATGTCATCACTTCGCAGGGAATACGTTTAAGCGAATTAGAAACAAAATTTATTGAATCTGATGTAG
GTTCTTTGACCATTGAAGTTGGTAATCTTCGTGAAGAGCTTGGACCGAAACCACCATCATTTTCACAGAA
CGTTTATAGTCGTTTAAATGAAATTGACACTAAACAGACAACAGTTGAGTCTGACATTAGTGCTATTAAG
ACCTCAATAGGATATCCAGGAAATAATTCGATTATCACGAGTGTTAATACAAACACTGATAATATTGCAT
CTATTAATTTAGAGCTAAATCAAAGTGGAGGTATTAAACAGCGTTTAACCGTTATTGAAACTTCCATTGG
TTCAGATGATATTCCTTCGAGTATTAAAGGTCAAATCAAAGATAATACAACTTCAATCGAATCTCTAAAT
GGAATCGTCGGTGAAAACACTTCATCTGGCTTAAGAGCGAATGTTTCATGGTTAAACCAAATTGTTGGAA
CTGATTCTAGCGGTGGACAACCTTCTCCTCCTGGGTCTCTTTTAAACCGAGTTTCTACAATTGAAACTTC
TGTTTCAGGCTTGAATAACGCTGTTCAAAACCTACAAGTAGAGATTGGTAATAACAGCGCAGGAATTAAA
GGGCAAGTTGTAGCGTTAAATACTTTAGTAAATGGAACTAATCCAAACGGTTCAACTGTTGAAGAGCGCG
GATTAACCAATTCAATAAAAGCTAACGAAACTAACATTGCATCAGTTACACAAGAAGTGAATACAGCTAA
AGGCAATATATCTTCTTTACAAGGTGATGTTCAAGCTCTCCAAGAAGCCGGTTATATTCCTGAAGCTCCA
AGAGATGGGCAAGCTTACGTTCGTAAAGATGGCGAATGGGTATTCCTTTCTACCTTTTTATCACCAGCAT
AACATGGGGCCGCAAGGCCCCAAAGGATTTTAAATGTCAGGATATAATCCTCAGAATCCAAAGGAACTCA
AAGATGTCATTCTAAGACGTTTAGGGGCTCCAATTATTAATGTTGAGTTAACACCCGATCAAATTTACGA
TTGTATCCAGCGTGCCCTAGAATTATACGGTGAATACCATTTTGATGGACTCAATAAAGGTTTTCATGTT
TTTTATGTAGGGGATGATGAAGAAAGGTACAAGACCGGAGTCTTCGATTTAAGAGGTTCTAACGTATTTG
CAGTAACCCGCATTTTACGCACAAATATTGGGTCAATAACATCTATGGATGGAAACGCTACATATCCGTG
GTTTACTGACTTTCTTTTAGGAATGGCTGGTATTAATGGCGGAATGGGAACGTCTTGTAATAGATTTTAT
GGACCAAATGCCTTTGGAGCTGATTTAGGATATTTTACCCAGCTTACCAGTTATATGGGAATGATGCAAG
ATATGCTCTCTCCTATTCCAGACTTTTGGTTTAATTCAGCAAATGAACAGCTCAAAGTCATGGGAAACTT
CCAAAAATATGATTTAATTATCGTAGAAAGCTGGACTAAATCATACATTGATACAAACAAAATGGTTGGA
AATACAGTAGGATATGGAACAGTCGGTCCACAAGATAGCTGGTCATTATCTGAACGATATAATAACCCAG
ACCACAATTTAGTAGGTCGTGTTGTCGGCCAAGATCCGAATGTTAAACAGGGTGCTTATAATAATCGTTG
GGTGAAAGACTATGCAACAGCTTTAGCTAAAGAATTGAACGGTCAAATTTTAGCACGCCACCAAGGTATG
ATGCTTCCGGGCGGTGTTACAATTGATGGGCAGCGCTTAATAGAAGAAGCCAGATTAGAAAAAGAAGCAC
TGCGCGAAGAATTATACTTACTTGATCCTCCATTTGGAATTTTGGTAGGTTAATATGGCTACTTATGATA
 
AAAATCTTTTTGCTAAATTGGAAAACCGCACAGGTTATTCTCAGACCAATGAAACTGAAATATTAAATCC
TTATGTAAATTTCAATCATTATAAAAACAGCCAAATATTAGCTGATGTATTAGTAGCTGAAAGCATTCAA
ATGCGAGGTGTAGAATGCTATTATGTTCCAAGAGAGTATGTTTCCCCTGATTTGATATTCGGCGAAGACT
TAAAAAATAAATTTACTAAAGCTTGGAAATTTGCTGCATATTTAAATTCATTTGAAGGATATGAAGGAGC
TAAATCGTTCTTTAGTAACTTTGGTATGCAAGTACAAGACGAAGTGACTTTATCTATTAACCCAAATTTA
TTTAAGCATCAAGTTAACGGAAAAGAACCCAAGGAAGGTGATTTGATATATTTTCCTATGGATAACAGCT
TATTTGAAATTAACTGGGTTGAACCATATGATCCATTTTATCAATTAGGCCAAAACGCTATTCGTAAAAT
TACGGCAGGTAAATTCATTTATTCTGGAGAAGAAATTAATCCAGTTCTACAGAAAAATGAAGGAATTAAC
ATTCCAGAATTTAGTGAATTAGAATTAAATGCTGTTCGCAATCTTAACGGTATTCATGACATTAATATTG
ATCAGTATGCTGAAGTAGATCAAATTAATTCTGAAGCTAAAGAATACGTTGAACCTTATGTTGTTGTCAA
TAACAGAGGCAAATCTTTCGAATCTAGCCCATTTGACAATGATTTCATGGATTAATAAATATTATAAACT
AATTAAAGCCCGGATTAGGAGAAGTCATGTTTGGTTATTTTTATAATTCGTCTTTTAGACGATATGCTAC
CTTGATGGGCGATTTGTTTTCAAATATCCAAATCAAACGTCAGTTAGAATCTGGTGATAAGTTTATACGT
GTTCCTATTACGTATGCATCAAAGGAACACTTTATGATGAAATTGAATAAATGGACATCAATAAATTCAC
AAGAAGATGTAGCTAAAGTTGAAACTATTCTACCTCGTATAAATTTACATTTAGTTGATTTTAGCTATAA
TGCTCCATTTAAAACAAACATTTTAAATCAGAATTTACTGCAAAAAGGTGCAACTTCTGTAGTATCGCAG
TATAATCCATCTCCTATTAAAATGATTTATGAATTGAGTATCTTTACTCGCTATGAAGATGATATGTTTC
AAATAGTTGAACAGATTCTTCCATATTTTCAACCTCATTTTAATACAACTATGTACGAGCAGTTTGGAAA
TGATATTCCATTTAAAAGGGATATTAAAATTGTACTGATGTCTGCTGCTATAGACGAAGCTATAGATGGG
GATAATTTATCTCGTCGTAGAATTGAATGGTCATTAACATTTGAAGTAAATGGATGGATGTATCCTCCAG
TAGATGATGCAGAAGGATTAATTCGTACTACTTATACAGATTTTCACGCCAATACAAGAGATTTGCCTGA
CGGCGAAGGTGTTTTTGAATCTGTCGATAGCGAAGTTGTTCCTCGAGATATTGACCCAGAAGACTGGGAT
GGAACAGTAAAACAAACTTTCACTAGTAATGTAAATAGACCAACACCGCCAGAACCTCCTGGCCCAAGAA
CATAGAGGTTATTATGGAAGGTCTTGATATAAACAAACTTTTAGATATTTCTGACCTCCCCGGAATTGAC
GGGGAGGAAATCAAAGTGTATGAACCTCTGCAATTAGTAGAAGTTAAAAGCAATCCACAAAACCGTACTC
CAGACTTAGAAGATGATTATGGAGTAGTTCGTCGAAATATGCATTTTCAGCAACAAATGCTAATGGACGC
TGCCAAGATTTTTCTTGAGACAGCAAAGAATGCTGATTCTCCTCGTCACATGGAAGTATTTGCAACTCTT
ATGGGGCAAATGACTACGACGAACAGAGAAATACTGAAGCTTCATAAAGATATGAAAGACATTACATCTG
AGCAGGTTGGCACCAAAGGCGCTGTTCCTACAGGTCAAATGAATATTCAGAATGCGACAGTATTCATGGG
TTCACCAACAGAATTAATGGACGAAATTGGTGATGCTTACGAGGCTCAAGAAGCTCGTGAGAAGGTGATA
AATGGAACAACCGATTAATGTATTAAATGATTTCCATCCGTTAAATGAAGCTGGAAAAATTTTAATAAAA
CACCCAAGCTTAGCGGAAAGAAAAGATGAAGATGGAATTCATTGGATAAAATCTCAGTGGGATGGAAAAT
GGTATCCTGAAAAATTCAGTGATTACCTTCGTCTACACAAAATAGTAAAAATTCCAAACAACTCTGATAA
GCCTGAATTATTTCAAACTTATAAAGATAAGAATAATAAAAGATCTCGGTATATGGGTCTTCCTAACTTG
AAACGAGCTAATATTAAAACACAATGGACTCGTGAAATGGTTGAGGAATGGAAAAAATGCCGAGATGATA
TTGTTTATTTTGCAGAAACATACTGTGCCATTACTCATATTGACTATGGTGTCATAAAGGTTCAATTACG
TGACTATCAGCGTGATATGCTCAAAATAATGTCATCTAAACGTATGACTGTTTGTAATCTATCGCGCCAG
CTCGGTAAAACCACCGTAGTAGCTATTTTCCTTGCACACTTTGTATGTTTTAACAAAGATAAAGCTGTAG
GTATTCTTGCACACAAAGGCTCAATGTCTGCGGAAGTTTTAGACCGTACTAAGCAAGCAATTGAACTGCT
TCCTGACTTTTTACAACCAGGAATTGTTGAATGGAATAAGGGTTCAATTGAACTAGATAATGGTTCTTCA
ATTGGCGCTTATGCTTCCTCTCCTGACGCAGTTCGTGGTAACTCGTTCGCAATGATTTACATTGACGAAT
 
GTGCGTTTATTCCAAACTTCCATGATTCCTGGCTTGCTATTCAACCAGTAATTTCATCTGGTCGTCGTTC
GAAAATTATTATTACTACGACTCCTAATGGATTAAATCATTTTTATGATATTTGGACTGCTGCTGTCGAA
GGTAAATCTGGATTTGAACCATATACTGCTATTTGGAATTCAGTTAAAGAACGTCTTTATAACGATGAAG
ATATTTTTGACGATGGATGGCAATGGAGCATACAAACCATTAATGGTTCTTCATTAGCTCAATTCCGTCA
AGAACATACTGCAGCGTTTGAAGGGACTTCTGGTACATTAATTTCAGGAATGAAATTAGCTGTTATGGAT
TTTATTGAAGTAACACCAGATGATCATGGTTTTCACCAATTTAAAAAACCTGAACCAGATAGAAAATATA
TTGCAACTCTAGATTGCTCAGAAGGTCGTGGGCAAGATTACCACGCTTTGCATATTATTGATGTTACTGA
TGATGTGTGGGAACAGGTTGGTGTTTTGCATTCAAACACTATTTCTCATTTAATTCTACCTGACATCGTT
ATGCGTTATTTAGTAGAATACAATGAATGCCCAGTTTATATTGAATTAAATAGTACTGGTGTGTCAGTTG
CAAAATCGCTTTATATGGATTTAGAATACGAAGGTGTTATCTGCGATTCATATACTGATTTAGGAATGAA
ACAAACTAAACGCACGAAAGCAGTAGGATGTTCCACGCTAAAAGACCTTATTGAAAAAGATAAGCTTATT
ATTCATCACCGAGCGACTATTCAAGAATTTAGAACGTTTAGTGAAAAAGGCGTGTCTTGGGCGGCTGAAG
AAGGTTATCATGACGATTTAGTAATGTCTTTAGTGATTTTTGGATGGTTATCAACGCAGTCAAAATTTAT
TGATTATGCGGATAAAGATGACATGCGATTAGCATCTGAAGTATTTTCAAAAGAGCTTCAGGATATGAGC
GACGACTACGCGCCAGTTATATTTGTGGATTCGGTTCATTCTGCTGAGTATGTTCCAGTATCTCATGGTA
TGTCAATGGTATAAATATATTAAAGCATATTAAAGAGGATTAAAAATGACTTTATTATCTCCGGGCATTG
AGCTCAAAGAAACTACGGTTCAAAGCACCGTAGTTAATAACTCTACTGGTACAGCAGCTTTGGCCGGTAA
ATTCCAGTGGGGTCCTGCTTTTCAGATTAAACAGGTTACAAATGAAGTAGATTTAGTTAATACTTTTGGT
CAACCAACCGCTGAAACTGCTGACTATTTTATGTCTGCGATGAATTTCTTGCAGTACGGAAATGACTTAC
GAGTAGTTCGTGCTGTTGATAGAGATACCGCTAAAAACTCATCGCCAATTGCTGGTAATATTGATTACAC
AATTTCTACCCCAGGTAGTAACTATGCGGTTGGAGATAAAATCACGGTCAAATATGTTTCAGATGATATT
GAAACTGAAGGTAAAATTACTGAAGTAGACGCAGATGGAAAAATTAAGAAAATTAATATTCCTACTGGCA
AAAATTACGCTAAAGCGAAAGAAGTCGGTGAATATCCAACACTAGGTTCTAACTGGACTGCGGAAATTTC
TTCATCTTCCTCTGGTTTAGCTGCAGTAATAACTCTTGGAAAAATTATTACTGATTCTGGTATTTTATTA
GCTGAAATTGAAAATGCTGAAGCTGCTATGACAGCGGTTGACTTTCAAGCAAATCTTAAAAAATATGGAA
TTCCAGGAGTAGTAGCGCTTTATCCAGGCGAATTAGGCGATAAAATTGAAATTGAAATCGTATCTAAAGC
TGACTATGCAAAAGGAGCTTCTGCATTACTCCCAATTTATCCAGGTGGTGGTACTCGTGCATCTACTGCT
AAAGCAGTGTTTGGATATGGACCGCAAACTGATTCACAGTACGCTATTATAGTTCGTCGTAATGATGCTA
TTGTTCAAAGCGTTGTTCTTTCAACTAAGCGTGGTGAAAAAGATATTTACGATAGTAACATCTATATCGA
TGACTTTTTCGCAAAAGGTGGTTCAGAATATATTTTTGCAACTGCACAAAACTGGCCAGAAGGCTTCTCT
GGAATTTTAACTCTGTCTGGTGGATTATCATCAAATGCTGAAGTAACAGCAGGAGATTTGATGGAAGCTT
GGGACTTCTTTGCTGACCGTGAATCTGTTGACGTTCAACTGTTTATTGCAGGTTCTTGTGCCGGTGAATC
TTTAGAAACAGCATCTACTGTCCAAAAACACGTCGTTTCAATTGGGGATGCTCGCCAAGATTGCTTAGTA
TTGTGCTCTCCTCCGCGTGAAACTGTAGTTGGAATTCCTGTAACCCGTGCTGTTGATAACCTAGTCAATT
GGAGAACTGCGGCAGGTTCATACACTGATAATAACTTTAATATCAGTTCAACCTATGCAGCAATTGATGG
TAACCATAAGTATCAGTATGACAAATATAATGATGTGAATCGTTGGGTTCCATTAGCAGCTGATATTGCT
GGTTTATGCGCAAGAACTGATAACGTATCTCAGACTTGGATGTCTCCAGCTGGTTATAATCGTGGTCAGA
TTCTTAACGTTATTAAACTTGCTATTGAAACTCGCCAGGCTCAGCGCGACCGTTTATACCAAGAAGCTAT
CAACCCGGTAACCGGTACAGGTGGTGATGGTTACGTATTGTATGGTGATAAAACAGCTACTTCTGTTCCT
TCTCCATTTGATCGTATTAACGTTCGTCGTCTGTTTAATATGTTGAAAACGAATATCGGACGTAGTTCAA
AATATCGTTTGTTCGAATTAAACAACGCGTTTACTCGTTCATCATTCCGCACAGAAACTGCCCAGTACTT
 
ACAAGGGAATAAAGCTCTCGGTGGAATTTATGAATATCGTGTAGTTTGCGATACAACAAATAACACTCCG
TCAGTAATTGATAGAAATGAGTTTGTTGCAACATTCTACATCCAACCGGCTAGAAGCATTAACTACATTA
CCTTAAACTTCGTAGCAACTGCTACTGGTGCAGATTTCGATGAGTTAACTGGTCTTGCTGGTTAATACGG
TGCATTCTAAAGGCCTGTTTCGGCAGGCCATATAAATACACTATATCCTTAATTCTTTAATTCTATATGC
CCTAGGTTAAACATAGGGATATAAATACTACAGAGGCTAATATGTTTGTAGATGATGTAACACGAGCGTT
TGAATCTGGTGATTTTGCTCGACCTAACTTATTCCAAGTAGAAATTTCTTATCTTGGACAAAATTTTACG
TTCCAATGTAAAGCTACTGCTCTACCAGCTGGTATTGTAGAAAAAATTCCAGTCGGATTTATGAACCGTA
AAATTAACGTAGCAGGCGATCGTACATTCGATGACTGGACTGTTACAGTAATGAACGATGAAGCTCATGA
TGCTCGTCAGAAGTTCGTTGATTGGCAAAGCATTGCTGCGGGGCAAGGAAACGAAATTACTGGTGGAAAA
CCTGCAGAGTATAAAAAGAGCGCTATCGTTCGTCAATATGCTCGTGACGCTAAAACAGTAACAAAAGAAA
TTGAAATTAAAGGTCTGTGGCCTACTAACGTGGGTGAACTTCAATTAGATTGGGATTCAAACAATGAAAT
CCAAACTTTTGAAGTAACTCTTGCTCTCGATTATTGGGAATAAAATGAATGGGGAGAAATCCCCATCCTG
CTTAAAGCAGAGAAGTCCATTATAAATATAACTATAATTCCCATTTGGAGAATACAATGAAATTTAATGT
ATTAAGTTTGTTTGCTCCATGGGCTAAAATGGACGAACGAAATTTTAAAGACCAAGAAAAAGAAGATCTT
GTTTCCATTACAGCCCCAAAGCTTGATGATGGAGCAAGAGAATTTGAAGTAAGCTCGAATGAAGCTGCTT
CTCCTTATAATGCTGCATTCCAAACAATTTTTGGTTCATATGAACCAGGAATGAAAACTACTCGTGAGCT
TATTGATACATATCGTAATCTCATGAATAACTATGAAGTAGATAATGCAGTTTCAGAAATCGTTTCAGAT
GCTATCGTCTATGAAGATGATACTGAAGTCGTAGCGTTAAATTTGGATAAATCTAAATTTAGCCCAAAAA
TTAAAAATATGATGTTAGATGAATTTAGTGATGTATTAAATCATCTATCGTTTCAACGAAAAGGTTCTGA
TCATTTTAGACGTTGGTATGTTGATTCAAGAATTTTCTTTCATAAAATCATTGATCCAAAACGTCCAAAA
GAAGGCATAAAAGAATTACGTAGATTAGACCCTCGCCAAGTTCAGTATGTTCGTGAAATTATAACAGAAA
CTGAAGCTGGCACAAAAATAGTTAAAGGTTACAAAGAATATTTTATATATGATACTGCCCATGAGTCATA
TGCATGTGATGGTAGAATGTATGAAGCTGGCACAAAAATAAAAATTCCTAAAGCTGCCGTCGTTTATGCC
CATTCTGGATTAGTCGATTGTTGCGGTAAAAATATCATCGGGTATTTGCATCGTGCTGTTAAACCTGCTA
ACCAATTAAAATTATTAGAAGATGCTGTAGTCATTTATCGCATTACTCGTGCTCCTGACCGTCGTGTTTG
GTATGTAGACACAGGTAATATGCCTGCTCGTAAAGCTGCTGAGCACATGCAACATGTTATGAACACGATG
AAAAACCGTGTAGTATATGATGCATCAACAGGTAAAATAAAAAATCAACAGCATAATATGTCTATGACCG
AAGACTATTGGTTGCAGCGCCGTGATGGTAAAGCTGTGACAGAAGTTGATACTCTTCCTGGTGCTGATAA
TACTGGCAATATGGAAGATATTCGTTGGTTTAGACAAGCTCTTTATATGGCATTACGTGTTCCTCTTTCA
CGCATTCCGCAAGACCAACAAGGCGGTGTGATGTTTGATTCTGGAACTAGCATTACACGTGATGAATTAA
CGTTTGCTAAATTTATTCGTGAGTTACAGCACAAGTTTGAAGAAGTTTTCCTAGATCCGCTTAAAACAAA
TCTTTTGCTTAAAGGTATAATCACAGAAGATGAGTGGAATGATGAAATAAATAATATTAAGATAGAATTT
CATCGGGATAGCTACTTTGCTGAGCTCAAAGAAGCAGAAATTTTGGAACGAAGAATTAATATGCTAACCA
TGGCAGAACCATTTATTGGTAAATATATTTCTCACAGAACTGCTATGAAAGACATTTTGCAGATGACTGA
TGAAGAAATAGAACAAGAAGCCAAGCAAATTGAAGAAGAGTCTAAAGAGGCTCGTTTCCAAGACCCCGAC
CAAGAACAAGAGGATTTTTAATGGAAGGTTTAATTGAAGCTATTAAATCAAACGACCTCGTAGCCGCTCG
TAAATTATTTGCTGAAGCCATGGCTGCAAGAACGATTGATTTAATTAAAGAAGAAAAAATCGCTATCGCT
CGCAATTTCTTAATCGAAGGTGAAGAACCTGAAGACGAGGATGAAGATGAAGATGACGAAGATAGTGATG
ATAAAGACGACAAAAAAGACGAAGACTCTGACGAAGACGAGGATGATGAATAATGCTTCTGATCCCTGAA
ACTCATGAATTAGTTCTCGAGAATGTCGAAGCACTTATTCCTGAAGCACAGGGTCGCTTTGACGAATTGT
CTTCTGCTTTAAATAAAGACGATATAAATACAATTGTCGAGAATATGCTTGATGATGAAACTGATTTAGC
 
GGTTGCATTAGCTTCTATTAATGAAAATATGCCGTTAAATGAATTCATCGTTAAACATGTTTCTGCCCGT
GGTGAAATTACTCGCACTAAAGACCGCAAAACGCGTGAACGAAATGCATTTCAAACCACTGGGCTGTCTA
AAGCAAAACGTAGACAAATTGCTCGTAAAGCTACCAAAACGAAGATTGCCAATCCAGCAGGTCAATCTCG
TGCTCAGCGTAAGCGTAAAAAAGCTCTTAAACGCCGTAAAGCATTAGGATTAAGCTAATGAATGAACCCC
AATTACTAATTGAAACTTGGGGTCAACCTGGCGAAATTATTGATGGCGTACCAATGCTTGAATCTCATGA
TGGAAAAGACTTAGGTTTAAAACCGGGTTTATACATCGAAGGAATATTCATGCAAGCGGAAGTCGTCAAT
AGAAATAAACGTCTTTATCCAAAACGTATATTAGAAAAAGCGGTAAAAGACTATATTAATGAGCAAGTTT
TAACTAAACAAGCTCTCGGAGAATTAAATCATCCTCCACGCGCTAATGTTGACCCGATGCAAGCCGCTAT
CATTATAGAAGATATGTGGTGGAAAGGAAATGACGTATACGGACGAGCTCGTGTTATTGAAGGTGACCAT
GGTCCTGGAGATAAATTAGCAGCTAATATTCGTGCCGGATGGATTCCAGGAGTTTCTTCTCGTGGATTAG
GTTCATTGACTGACACAAATGAAGGTTATCGTATCGTAAACGAAGGATTCAAATTAACTGTAGGTGTTGA
TGCAGTATGGGGTCCAAGTGCTCCAGATGCATGGGTAACTCCTAAGGAAATTACCGAATCACAGACGGCG
GAAGCCGATACAAGTGCCGATGACGCCTATATGGCTCTCGCAGAGGCCATGAAAAAAGCGTTATAAATAT
TATTATCTAAACAACAGGACTACAAAATGCTTAAAGAACAACTGATTGCCGAAGCGCAGAAAATTGATGC
TTCCGTTGCTCTTGATAGTATTTTCGAATCAGTTAATATTTCTCCGGAAGCAAAAGAAACTTTCGGCACT
GTATTCGAAGCTACCGTCAAGCAGCACGCCGTTAAATTAGCTGAATCTCATATCGCTAAAATTGCTGAAA
AAGCAGAAGAAGAAGTAGAAAAAAATAAAGAAGAAGCCGAAGAAAAAGCTGAGAAGAAAATCGCTGAGCA
AGCTTCTAAATTCATTGACCATCTTGCAAAAGAATGGCTCGCTGAAAATAAATTAGCAGTTGATAAAGGC
ATCAAAGCCGAACTGTTTGAATCCATGCTTGGTGGATTAAAAGAGCTCTTTGTTGAACACAACGTTGTTG
TTCCAGAAGAATCAGTTGATGTTGTAGCTGAAATGGAAGAAGAGCTGCAAGAACATAAAGAAGAATCGCC
TCGTCTGTTCGAAGAACTGAATATGCGCGACGCATATATCAATTATGTGCAGCGTGAAGTGGCATTGAGC
GAAAGTACTAAAGATCTGACTGAGTCTCAAAAAGAAAAAGTCTCTGCTCTGGTCGAAGGTATGGATTATT
CAGATGCATTCTCAAGTAAATTGAGTGCAATCGTAGAAATGGTGAAGAAATCTAATAAAGATGAAAGCAC
TATTACTGAGAGTATAAATACTCCTGATACTGAAGCAGCCGGACTGAATTTCGTCACTGAAGCTGTAGAA
GATAAAGCTGCACAGGGTGCAGAAGATATTGTAAGTGTATATGCGAAAGTCGCATCTCGTTTCTAATTTT
AAAGGTTAACACAAATGACTATCAAAACTAAAGCTGAACTTTTGAACAAATGGAAGCCATTACTGGAAGG
TGAAGGTTTACCGGAAATTGCTAATAGCAAACAAGCGATTATCGCTAAAATCTTTGAAAACCAGGAAAAA
GATTTCCAGACAGCTCCGGAATATAAAGACGAAAAAATTGCTCAGGCATTCGGTTCTTTCTTAACAGAAG
CTGAAATCGGTGGTGACCACGGTTACAATGCTACCAACATCGCTGCAGGTCAGACTTCTGGCGCAGTAAC
TCAGATTGGCCCAGCTGTTATGGGTATGGTACGTCGTGCTATTCCTAACCTGATTGCTTTCGATATTTGT
GGTGTTCAGCCGATGAACAGCCCGACTGGCCAGGTATTCGCACTGCGCGCAGTATATGGTAAAGACCCAG
TGGCTGCCGGTGCTAAAGAAGCATTCCACCCAATGTATGGTCCAGATGCAATGTTCTCTGGTCAGGGTGC
TGCTAAGAAATTCCCAGCTCTGGCTGCTAGCACACAAACCACAGTAGGTGATATCTATACTCACTTCTTC
CAGGAAACTGGTACTGTATATCTGCAAGCTTCTGTTCAAGTAACAATCGATGCTGGTGCGACTGATGCTG
CTAAATTAGATGCTGAAATTAAGAAACAAATGGAAGCTGGTGCACTGGTAGAAATCGCTGAAGGTATGGC
TACTTCTATCGCTGAACTCCAGGAAGGTTTCAATGGTTCTACCGATAACCCATGGAATGAAATGGGCTTC
CGTATCGATAAGCAAGTTATCGAAGCTAAATCTCGTCAGCTGAAAGCTGCTTACTCTATTGAATTAGCAC
AAGACCTCCGCGCTGTTCACGGTATGGATGCTGATGCTGAACTGTCTGGTATTCTGGCTACAGAAATTAT
GCTGGAAATCAACCGTGAAGTTGTTGATTGGATTAACTACTCAGCTCAGGTTGGTAAATCTGGTATGACC
CTGACTCCGGGTTCTAAAGCTGGTGTATTTGACTTCCAGGACCCAATTGATATTCGTGGTGCTCGCTGGG
CGGGTGAATCCTTTAAAGCTCTGTTGTTCCAGATTGACAAAGAAGCAGTTGAAATTGCTCGTCAGACCGG
 
TCGTGGTGAAGGTAACTTCATTATCGCTTCCCGTAACGTAGTTAACGTTTTGGCTTCAGTTGATACCGGC
ATTTCTTATGCTGCACAGGGTCTGGCTACCGGCTTTAGCACTGATACTACCAAGTCAGTATTTGCTGGTG
TTCTGGGTGGTAAATACCGCGTATATATCGACCAGTATGCTAAACAGGATTATTTCACTGTAGGTTATAA
AGGTCCGAACGAAATGGATGCTGGTATTTACTATGCTCCATATGTAGCTCTGACTCCGCTGCGTGGTTCC
GATCCGAAGAACTTCCAACCGGTAATGGGATTCAAAACTCGTTACGGTATCGGTATCAACCCATTTGCAG
AATCCGCTGCTCAGGCTCCGGCTTCTCGCATCCAGAGCGGTATGCCTTCTATTCTGAATAGCCTTGGTAA
AAACGCTTACTTTAGACGTGTATATGTTAAAGGTATCTAATCTCTAACGATAGAAACACAATTTTAGGGA
ACCTTCGGGTTCCCTTTTTTCTATTTTATACGATAGCAATCAGGCATATCATCCGCATTTATCCAATTGC
GAATAGTTTTAGGACTAACTTTAAAATGCTCCGCTGCGTAATCAGGATTATCAAATTTAACGCCCTTTAT
ACATATTGGAATAAATTTTTTAATACCACCAAGTTTTTCAGAAATAGCTTTACGATGTGAAATCGATATA
GGTTTGTTTTTTCGTGGATGAACATGTGTTTTATAATATTCATTGCGCCCTTTAACTCGCTTCGCAATAG
TTTCATCAGATTGCTTAACGCCTGTTTTTGCCTTTGATATTTTTCGTTTAGCTTCCACAGTCATTCCTTC
TTTTGTTCGTATTGATAACATATTACGATATGAAGGATCTTGCAAATGAACTATAACTGGATTTCCTCTG
CCACCAATAGCAGCATTATAGGTATCAGTTCTCATAACGAATTCCTCATTAACTAGTAAAGCTTCCATTT
TATACATCTCCTCAGATGAGGAGAAAGAATAAAGAATTTCTTTTTTAAAGTTATGAATACCATATTTTTT
GATGGATTTTTTGATGTTTACGCCAGAACCCATATAACCATCGTTTTCGTCAAGAGTAGCATGAGCTCCG
ATGTAAATTTTTCCATTGATGATATTAGTAATTTGATATATTAAATATTTCATTTTAAACATCACTCCGT
TTGTATATGATTATAATATCATATTACTTTGGTCTTGTAAATAACTTTATAAATAGTATTATATTTCAAC
AAGGAAAATACAATGGCTAAAATCAACGAACTTCTGCGCGAATCAACCACAACGAATAGCAACTCAATCG
GTCGCCCAAATCTCGTTGCTTTGACTCGCGCTACCACTAAATTAATATATTCTGACATTGTAGCAACGCA
AAGAACTAATCAACCTGTTGCTGCTTTTTATGGTATCAAATACCTTAACCCAGACAACGAATTTACATTT
AAAACTGGTGCTACTTACGCTGGCGAAGCTGGATATGTAGACCGAGAACAAATCACAGAATTAACAGAAG
AGTCTAAATTAACTCTCAATAAAGGCGATTTATTCAAATATAATAATATCGTTTATAAAGTATTAGAAGA
TACTCCATTTGCTACTATCGAAGAAAGTGATTTAGAATTAGCTCTTCAGATTGCAATCGTTCTTTTAAAG
GTTCGTCTATTTTCTGACGCAGCGTCAACAAGCAAATTTGAAAGCTCTGATAGTGAAATTGCGGATGCTA
GATTCCAGATTAATAAATGGCAAACTGCAGTTAAATCTCGTAAACTTAAAACTGGCATCACAGTTGAATT
AGCGCAAGATTTAGAAGCAAATGGATTCGATGCTCCTAATTTCTTGGAAGATTTGCTTGCAACTGAAATG
GCAGATGAAATCAATAAAGACATTCTGCAGTCTTTGATTACAGTGTCAAAACGCTATAAAGTTACAGGAA
TTACTGATAGTGGATTCATCGATTTGAGTTATGCATCTGCTCCTGAAGCTGGTCGTTCATTATACCGAAT
GGTATGTGAAATGGTTTCGCATATCCAAAAAGAATCAACTTATACAGCAACGTTCTGTGTTGCTTCAGCT
CGTGCCGCTGCGATTCTTGCTGCATCAGGCTGGTTAAAACATAAACCAGAAGATGACAAATATCTTTCAC
AAAATGCCTACGGGTTCTTAGCTAATGGTTTACCGCTTTATTGCGATACTAACAGCCCATTAGATTATGT
AATCGTTGGCGTAGTAGAAAATATCGGTGAAAAAGAAATTGTTGGATCAATTTTCTATGCTCCGTATACA
GAAGGTCTCGACTTAGATGACCCTGAACATGTAGGTGCATTTAAAGTTGTTGTTGATCCAGAAAGCTTAC
AACCATCTATCGGTTTATTAGTTAGATATGCTTTATCAGCAAATCCTTATACTGTAGCAAAAGATGAAAA
AGAAGCAAGAATAATTGACGGTGGAGACATGGATAAAATGGCAGGTCGTTCAGATTTGTCTGTTTTATTA
GGTGTTAAGCTACCAAAAATTATCATTGATGAATAAAACAAAGGGACCTTTCGGTCCCTTTTTATTTAAC
TTACCAACTCAATCCAAGCTGGACGAAGTACATCTTGTACCATTTTAACTAATTCCTTTTTAATCAAAGA
AGGATTATCTGCTTGAGTTAGAGTAATACCTTCACGAGAAGTTTCTTCCAAAATATCTTGAACAGTTAGC
CCCATCACCTTTCCAAAATCCTTTGGACCAATTTCGCCAATTTTAGAAATAACGTTATTTACGCGGTTCA
GTGTAACGTAACAAGCTAAAATTCCCACCAATTTGTTATCAGCTTCTGATAGCTCAACTTTAGCTTTAAT
 
AGGCTTATCAGACTTTTTCTTTTCACTAAATTTAGAGTTCTTGCATTTAATCGCTACACGATTTCCATTA
CGAAGCCAAGAAGGATAACAAGGTTTCAATACATATCCTTCAGCAGTAAATACTTCGCCTTTTGCTTCGG
CATTCCAAACGCATTTATTTGCATCAACTAATCCAGCATGGTCTACTGTAAAATTATAATCTTGGACGAC
AGAATCTAAATCATTTGGCAATTTAATAAGCTCTTCAAATTTACCGCGACCTAAAAGTGGAGCCATTTTA
AATTTAAATGTATTACAGAATGATTCCATCATATAATCATCTACATAAGTCACATCACCGCTTTCTGTAG
TAACAATAATGTCAAATACATAAAAATCTTTATCACAATAATCAACATTCTTCTGAATGCCAGGTCCAGC
GAATTCGCCAAAGACTTGATAAGATACAACCGCTGAGGTTTCCATAATATCTTGTACAGCTTTAATGGAA
TCAGCATAATTCTTCAAAATAATTTCATACCCAAAGAAATCTTCAGCAGGAAGAATCGGTCCAGTGCGTT
TAGCGCAAGTCACTTTATCACGCTCAATAATCAATGAGAAATTTGTGCCGTGAATCTTTTCACGAGCTAC
CCACTCCCCACCAGTCAATCCCAAGCTATAAAGTTTTTCAATAAATTTAGAGTTGTAATGATTTTCAAGA
CTGCTATACTTTTTAAACATAATTAATCCTCAAAATGTAATTTCTAACCAATCACCATCACGCTGATCAC
TATTGACTTTAAAGCTGAATCCTTCTTTTCTCAGCCAATCACCAATTTCTTCTGTAATCAATTTATCACG
AGCAATACAATAATAATTAAAATGTGTTTTACCTTGTTCAGCTGCTTTATTAGCAAGTTCTGAAAAATCT
TTAATAAAACACTCTAGCTTAAACTGTTTACTTTTTAATGCTTTTTCGCGTAATTGATTAGCAAAAGATT
CATTTTCATAAAGATCATACTGTTCCATTTTTCACCTTTTTATTGATATGTCTTTTTCTATAGACAACTT
TTTCTCGAGCCCATAATACAGCCACTTCTTTTGCCTGTAAGTTTAATTCACGAGCAATTTCAATGAATGA
CTTTCCAGACTCATGAAGAGTAAACACCACAACCTCAGTTCTCATAATCAATCTCATGTTATCGAGTTGG
TGCCATTATATACATCATTTTCTGATTGTGTTTTGTGTGCTTTCAAAATGAAGAAAGGGGCCGAAGCCCC
TTATGATTATGGATAGGTATAGATGATACCAGTTTCTAAAGCAGTTTTATGAATGATGTATCCATTACGC
GATTCTTGGACATCAACTTCTGGATAGTCTTTCATCATCTTCTGGAGAGTGTAACGATGCAGGTAATATT
TACTATCTGGGTCGTCAGTTTTCCAATCTTTACCTTCTTCGGTCATTTTTTGGATTTCATCCATAACCCA
CCAACCGCACCAGATGTAAGCTGAGCTACGGTGTGGAAGAGGATGAACATAAGGTAATTCACCTTCTGGT
TCTGGAGCAACTTTAGCCGTGACTGTTACATTACCAGTTTTGGTAACGGTTACAGGGTTATAATCTGCAG
CAGTAACAGTTGCAGTAACTTCAATAGTTTGACTTCCAACAGATGAGGTATCGACAGTATATACGTTAGT
TGACCCTTCTACAGGAGAAGAATCTTTCTTCCATGAGTAAGTAATTTGTGCTTCTTCTGGAGCACCCGTA
ACATTAGCCGTAAATGTAGCCGAAGCATCTTGCTGAACATTAATAGAAGGAGGAGTCAATGTAACCTGTG
GATTCATTGTCTTCTTATTAACCGTTAATGATACTTCATTAGAAGTAACGCTTAGTGCATCATAATCTGT
CGCGGTTACTTGGGCTACGCATTTAATTCTTTTTACTCCACTTGTAGTTGGAGTATAGCTAAATGTAGAG
TTAGTTTCTCCACCAACTTGTGAATCATCTACATACCACTGATACGTAGCAGATGCTCCATCAGGTTGAG
AAGCTAAGGCAGCAGTAAATTGAACTGGGGTTCCAATCACTCCAGCCGCAGGACTAGCAGGAGTTACGGC
TAAGGTAGTCGTCTGTGTCTTATTTTTAACTGTGATAGTTGTTGTCGCTTCAGCCGTTTCCGGGCCTCCT
TCAGAAAGTGTATTTGTTGCAACTACTTTAATAGTCTTTTGACCGGCAGGTCCTTTTAGTACATAACTAA
AAGTTGCTTCAGCTCCATCTTGTGGAACATTATCTACGCTCCAAGCATATGTAATAGTTCCGCCTCCAGT
TTGACCACTGGGTGTAGCAGTAAACTGCTTAGTTTCATCAATAACCCCTGTAGGTGTTTTAGGAGTTATA
TCAACTGTAAAAGTCATAAGTTATCCTTATTTTAATGTTACGAAAGAAGAGTTGCGTGTTTCACGAATTA
AAACTGATCCATCGCGATTAATGTAATAAATTAAGCTAAATAAAGTTTGGTGTGCTGACGCATGTTCAAA
ACTAGTTGGGTGAGATTTCCAATCAGGAGTTTCAGCAATCCATTGATAAATCCACCAAGGAACAGTACAG
AATCCTAGATTTTTTCCAATCAGTTGAAGATTCGGACTAAAGTTTTCCGGAAGAGTAAATACAGACGGCT
TTTCAGATTCAATAATCTCAGCTACAGCCTGTTCGAATTTTTCTTCAACAAAAGGAGTATCTTCAATCAA
AACATCGGTATTTTCAGGAATTTTATCCGTTTCTACTACTTCAATTTTAATGTCAGATTTAATCGGAGAA
TCAATCAGAAGTGCTGCTTCTGGATTGACTTCTTCATCGTCATATTTTAATCCCTCTGCGGCATCAGCAG
 
CATCAATTAAGTCTTTAATAGATAACCCATCAGTCTCTGGCATAGGTTCACTAGCGAGCTTCTGGAGGGC
TTCTTCAATATCAACAACGATATTATCAAAAGATTTATTCTTTTTGACCTTTATACCAAACTGTTCAGCA
TATTCAGCTAATTTAGCTTTAGCTTCTTTGTTATCATCAAGAGCCTTCAGCTCTGCAATATAATCTTTAT
CTATCATAATATTTCCTCAGTATAAATATAGATATATTTATTACTCGGAAAATAGTATGTACCACTTTGT
ATATGAAACAACAAATCTAATAAATGGTAAAAAGTATATAGGAAAGCACTCTACTGATGACTTGAATGAT
GGTTACCTTGGTTCCGGTAAGGCAATTCAGCAGGCTATAAAGAAATATGGTGAAAACAATTTCTCTAGAA
CAATACTAAAAGAGTTTAAAACTTCCGAAGAAGCGTACATGTATGAAGAAGAAATTATAACTCCTGAACT
AATAAAAAGCAAAAATTATTATAATATGAAACCTGGTGGAATTGGTGGAATTGTTATGACTACAGATGTT
ATAGCAAAGATGAAAGAATCTTCCGCTAAAAGATTTGAAAACTCACCGGGCACGGTATTAGGTAAAACTT
GTTATACTAATGGAACTAAAAATATTTTTATTAAACCTGGAGAACTTGTTCCAGAAGGATTTGTAAAAGG
GATGGTTCATCCTAATAGAAAGTCCAGAAAAGGATGTAAAGTCAAACCGACTACCACAGGAACTTTTTGG
GTCAATAATGGCGCAATAAATAAATTAATACAACCAGACGGTATTATTCCCGACGGATTTATTAAAGGTC
GTCTCATGAAAAGAGATTCTAAAGGCAAATTTAGTAAGGCATAATTATGGATATTAAAGTACATTTTCAC
GACTTCAGTCATGTACGCATCGATTGTGAAGAGAGCACGTTCCACGAATTAAGAGATTTCTTTTCGTTTG
AGGCCGATGGATATAGATTTAATCCTCGCTTCAGATATGGCAACTGGGATGGACGAATCCGTCTTTTAGA
TTATAATCGTCTTCTTCCATTCGGCTTAGTCGGGCAAATTAAAAAATTCTGTGATAATTTTGGCTATAAA
GCCTGGATTGACCCACAAATTAACGAAAAAGAAGAATTATCAAGAAAAGATTTTGATGAATGGCTTTCTA
AATTAGAAATCTATTCAGGAAATAAAAGAATTGAACCGCACTGGTATCAAAAAGATGCAGTGTTCGAAGG
ATTAGTTAATCGTCGTAGAATTCTTAATCTTCCAACATCTGCAGGTAAATCTTTAATTCAAGCTTTGCTT
GCGCGATATTATTTGGAAAATTATGAAGGTAAAATTCTTATCATTGTTCCAACAACTGCTCTGACAACTC
AGATGGCTGATGACTTCGTCGACTATCGTTTATTCAGCCATGCAATGATAAAGAAAATTGGTGGCGGAGC
ATCAAAAGATGATAAATATAAAAATGATGCACCAGTCGTTGTTGGTACATGGCAAACTGTAGTAAAACAA
CCGAAAGAATGGTTCTCACAGTTTGGAATGATGATGAATGATGAATGCCATCTTGCTACAGGAAAAAGTA
TTTCATCTATCATATCAGGTTTAAATAACTGCATGTTCAAATTCGGTTTGTCTGGTTCATTACGTGATGG
CAAAGCCAATATCATGCAGTATGTTGGAATGTTTGGTGAAATATTTAAACCAGTAACGACTTCTAAATTA
ATGGAAGATGGACAAGTAACTGAGCTAAAAATTAATAGTATTTTTCTTCGCTATCCCGATGAGTTCACTA
CTAAATTAAAGGGAAAAACTTACCAAGAAGAAATAAAAATTATTACTGGGCTTAGTAAAAGAAATAAATG
GATCGCTAAATTAGCTATTAAGCTTGCGCAAAAAGATGAAAACGCTTTTGTCATGTTTAAACATGTATCG
CATGGTAAAGCTATTTTCGATTTAATTAAAAATGAATACGATAAAGTTTATTACGTATCAGGGGAAGTTG
ATACCGAAACCCGCAATATAATGAAAACCTTAGCTGAAAATGGTAAAGGAATAATTATAGTAGCTAGTTA
TGGTGTATTTTCTACTGGTATTTCAGTTAAAAATCTGCATCACGTTGTTTTAGCGCACGGTGTTAAATCA
AAAATCATTGTATTACAAACAATCGGTCGTGTATTACGTAAGCATGGTTCTAAGACAATAGCAACAGTCT
GGGACCTCATAGATAGCGCAGGCGTCAAGCCAAAATCTGCTAATACGAAAAAGAAATATGTTCATTTGAA
CTATCTTTTAAAACACGGCATTGATCGTATTCAGCGCTACGCAGATGAAAAATTTAATTACGTAATGAAA
ACAGTTAATTTAATAAGCTTCGGCCCTTTGGAGAAAAAGATGTTACTAGAATTTAAACAATTTCTTTATG
AAGCTTCTATTGATGAATTTATGGGTAAAATTGCCTCTTGTCAAACATTAGAAGGTTTAGAAGAACTTGA
AGCTTATTATAAGAAAAGAGTCAAAGAAACTGAATTAAAAGATACTGATGACATCTCTGTGAGAGATGCT
TTGGCAGGAAAAAGAGCTGAATTAGAAGATTCAGACGATGAAGTAGAAGAAAGCTTTTAAATTAAAAAAG
GCCCAACCAAAAAGGAAGGGCCAAAACTATAGACTAAAGGTCACACTATAGCAAAAGTTGTGTTTCATTT
AATTGTTCTTCCGAACTTTCTGAAACTGGTAGTTCTTTAATGTAATTATAGCAAGGCCCAGGATGTACAG
GACCTTTGTCTGTTTCAACAACCAATGCAGAATCGATTGGAGTTTTACAGACAACACAAATCTTATCTGA
 
CATGATTGTCTCCTCTGAATTATATCTATTTATACAACTCTCATATGCATATCAATGCCCATATCTTTAG
AATAAAAATATTCATCAAGATATCCGGCAAATTTTCCTTTAATATAAAGGACATCTTCACCACACGGGTG
GTCGGCCAGGATACGAATATCCTGACGCTTAAGATTATGCTTTTTCATTAAGAATTGAATTTCCGTTTCA
AATTCTTCTTCATAATTAAAAGCATCATCAATGCTATATCTCATTATTTTCCAGCCTCAAATGCTCGCAT
GTCTTGAATATGCTTAATAGCAAATCCACGTGATTTAATAGCATCAAGAGCTCCGCTACAGAAATCTAAT
AAAATCCCCCAATACTGCAACGAGGTATCAACCTTTAAAACATCCTTATCCGCTGATAGAACTGTCTTCA
TTTCTGATTTCTCGTAACGATCCATACTAAATTCATCACCATCTCCTCGTCCCGAGTAGTAGTCTAATCT
AGCTTTAAGAGCAACTTTTTTCTGTGCTTCAATTCTAAGCATTTCCTTTTTAATACTTGAATGCTTATTA
AGCCATTTACTATATAACATCACATTATTAGCTGCTTCATACTGTAATTTAGTCGAATCTATAAACACAT
CTTTCTTCAATTCTTCTTGAAGATCTTCTAATCTCATATTGTTCTCTATTCAATTGTTATTGGTTGTTAT
TGGATGGACTTAGATTCATTATACCACGTTTTAACGTGAAGCATTATACTCTATTACTGGAAGCCAGCTG
CAGTTTTATCTGCTCAATATCATCAGGATTATCGATGACCGAAAAGCGTATTTCTACTATCAGAGTATAA
TCGTCATAAACGGGTATCACATTAACTGCTAATTTATCAATACGTGGCTCATAGTTTCTTACTGCGCTTT
CGATATTGCGTTCAACCGTGTCAGCAGTAAGAGGAGTCATATTTTCAAAAAGCTGGTCTGATAAATCACA
TCCAAATTCAGGGTCAAACGGTCTTGAACCTTTTCTTGTTGTAATAATTCCCAAAAGACTGTTTTTAATT
GACCTTAATCCAAGCGATCTGGAAACGTCTTTGTTCCAATCCATTTTCATTTCCGGGTCAATATCAGAAT
AAAGCTTATTAATATTTGCCATTATAGTAACTCAAAGAACTCTTTGAGGCCTCTTATTACGTGAGCATGG
GTTTTTCCACACTCTGGACACTTAATTGGAACAGCCAAATAAACGGTAGGCTTTAAAAGCATATCTTTTA
TAGCTACAATATCTGACTCTGTGATGATAGAATATAAATCTTCTAGTTCCTTTTCATTTAAGTCTTCAAC
TGGAATGCTTTCCCCGTTAGCATGAATCGTTTCTATACATGATACTATCATGTGGGCTATATTTTTATCA
TCAAAAATTTTAGGGTATCGGAATTTAATTTTAATGTCACCTAGTGTATACCAGAGGTCTTCTGGTGCAT
CTATTTGTGTATGTAATAGATTTATATGGGTTGGTATTTCAGTTCCACAGGTGCACTTCCAGGAGTTTTC
GTGATTAACTTCACCGAGAGAATGTGCCCATAAATGAATCAACAATAGTTCTGATTCTTGGCGGTTTAAA
TCTTTTGCATTTGTGCAGTCTTTGATTAGCTTTTTAACAATTACTTCTACGGAACCATTATTTTTGGCAG
TAATAAGTTCTAGATATTCTTTAAGCGTGAATGCGCGACAATTGATTATTTTAGAACCAACTCTCACATC
AAATTTGTATTCATACATATTTAGCTCCTTTATTTATCATATTTATAAATAGAATAAAAGGAGCATCTAT
GGCAAACATTATTCGTTGTAAATTACCAGATGGTGTTCATCGTTTTAAACCATTTACGGTAGAAGATTAT
CGAGATTTTTTGTTAGTTCGAAACGATATAGAACATCGGTCACCACAAGAACAAAAGCAAATAATTACTG
ATTTAATTGATGATTATTTTGGAGACTATCCGAAGACTTGGCAACCATTTATATTTTTGCAGGTATTTGT
AGGGTCAATAGGTAAAACTAAAAGTACGGTCACATTTATATGTCCAAAATGTAAAAAAGAAAAGACAGTT
CCATTTGAAATATATCAAAAAGAATTAAAGGACCTTGTTTTTGATGTAGCTAATGTTAAAATTAAATTAA
AGTTTCCTTCTGAGTTTTATGAAAATAAAGCAAAGATGATTACTGAAAATATTCATTCTGTTCAAGTAGA
TGAAATATGGTATGATTGGAAGGAAATTAGCGAGTCCAGTCAAATAGAACTAGTTGACGCCATCGAGATA
GAAACATTAGAAAAAATTCTCGATGCAATGAATCCTATTAATTTAACTCTACACATGTCATGCTGTAATA
AGTACATTAAAAAATACACTGATATAGTAGACGTGTTTAAGCTATTAGTTAACCCAGATGAGATATTTAC
TTTTTATCAAATTAATCACACACTCGTAAAAAGTAATTATAGCTTAAATTCAATAAGTAAAATGATTCCT
GCCGAGCGCGGATTCGTATTAAAACTGATTGAGAAGGATAAACAATAATGAGTATGTTGCAACGCCCCGG
ATATCCAAATCTCAGCGTTAAATTATTTGATAGCTACGACGCTTGGAGTAATAATAGATTTGTTGAATTA
GCTGCTACTATTACCACATTAACTATGCGGGATTCTCTTTATGGCCGAAATGAAGGAATGCTGCAGTTTT
ATGATTCTAAAAACATCCATACAAAAATGGATGGAAATGAAATAATTCAGATTTCTGTAGCTAATGCAAA
TGATATTAATAATGTTAAAACACGAATTTATGGATGTAAGCATTTTTCCGTGTCAGTAGATTCAAAAGGT
 
GATAACATCATTGCTATTGAATTGGGAACTATTCATTCTATAGAAAATCTTAAATTTGGTAGACCATTTT
TCCCTGATGCAGGTGAATCTATAAAAGAAATGCTTGGTGTCATTTATCAGGATCGCACATTATTAACTCC
AGCAATAAATGCTATAAATGCTTATGTTCCTGATATTCCATGGACTAGCACATTTGAAAACTATTTGTCA
TATGTAAGAGAAGTTGCTCTAGCTGTAGGAAGCGACAAATTTGTATTTGTATGGCAAGACATCATGGGCG
TTAACATGATGGACTATGATATGATGATAAATCAAGAACCATATCCAATGATTGTCGGTGAGCCATCTTT
AATAGGTCAATTCATCCAAGAATTAAAATATCCATTAGCATATGATTTCGTTTGGTTGACTAAATCGAAT
CCTCACAAACGTGACCCAATGAAAAACGCTACTATCTATGCGCATTCATTTTTAGATTCTTCAATACCAA
TGATTACTACAGGAAAGGGTGAAAACTCTATTGTGGTGTCAAGGTCAGGTGCTTATTCTGAAATGACTTA
TAGGAATGGATATGAAGAAGCTATTCGTCTTCAAACTATGGCACAATATGACGGCTATGCTAAATGTTCT
ACTATCGGTAATTTTAACTTGACTCCTGGTGTTAAAATTATTTTTAATGATAGTAAAAACCAATTTAAAA
CAGAATTTTACGTTGATGAAGTTATCCATGAATTATCCAATAATAATTCAGTAACTCATCTATATATGTT
CACTAATGCAACGAAACTGGAAACAATAGACCCAGTTAAGGTTAAAAATGAATTTAAATCTGATACTACC
ACTGAAGAAAGTAGTTCTTCCAATAAGCAATAAAGAAGTTTCTATTCCTAAAATGGGTCTTAAACATTAT
AACATTTTAAAAGATGTTAAAGGTCCTGATGAAAATTTAAAACTTCTCATTGATTCTATTTGTCCGAATT
TATCACCGGCAGAAGTTGATTTCGTTTCTATTCATTTATTGGAATTTAATGGAAAGATTAAATCTCGTAA
AGAAATAGATGGTTATACTTATGACATTAATGATGTTTATGTATGCCAAAGATTGGAATTTCAATACCAA
GGAAATACATTTTATTTTAGACCTCCTGGAAAATTTGAACAATTTTTAACGGTGAGCGATATGTTATCTA
AATGCTTACTTAGGGTCAACGATGAAGTTAAAGAAATTAATTTTCTTGAGATGCCAGCATTCGTTTTAAA
ATGGGCAAATGATATTTTTACAACTTTAGCAATTCCTGGCCCTAATGGTCCAATAACTGGAATTGGCAAT
ATTATTGGATTATTTGAATGAAAAAGCCACAAGAAATGCAAACGATGCGTAGAAAAGTTATTTCAGATAA
TAAACCAACACAGGAAGCGGCTAAATCCGCTTCTAATACTTTATCTGGGCTTAATGACATATCTACGAAA
TTGGATGATGCTCAAGCTGCTTCTGAATTAATAGCTCAAACTGTCGAAGAAAAATCGAATGAAATAATTG
GAGCAATTGACAATGTAGAAAGCGCAGTGAGTGATACATCTGCCGGTTCTGAGTTAATTGCTGAAACTGT
CGAAATTGGCAACAATATTAATAAAGAAATCGGTGAATCGCTCGGAAGCAAATTAGATAAATTAACAAGT
TTACTAGAGCAAAAAATCCAGACAGCTGGAATTCAACAGACTGGAACTAGTTTAGCTACGGTTGAAAGCG
CTATTCCTGTTAAAGTCGTTGAGGATGATACTGCTGAATCTGTGGGTCCTTTATTACCAGCTCCTGAAGC
AGTTAATAATGATCCTGACGCTGATTTTTTCCCTACCCCTCAGCCAGTTGAGCCAAAGCAAGAATCACCA
GAAGAAAAACAGAAAAAAGAAGCATTTAACTTAAAATTATCTCAAGCTTTAGATAAATTAACGAAGACTG
TTGATTTTGGATTTAAAAAATCCATTTCAATTACTGATAAAATATCAAGCATGCTATTTAAGTACACCGT
CAGTGCTGCTATTGAAGCTGCTAAAATGACTGCAATGATATTGGCTGTTGTTGTTGGAATAGACCTTTTG
ATGATTCACTTTAAATACTGGTCAGATAAATTTTCAAAAGCCTGGGATTTGTTTAGTACAGACTTTACCA
AATTCTCTAGCGAAACCGGAACTTGGGGTCCTTTATTACAGAGCATCTTTGATTCTATTGATAAAATTAA
ACAACTTTGGGAAGCGGGAGATTGGGGTGGATTGACAGTAGCTATTGTTGAAGGGCTTGGAAAGGTTCTT
TTTAATTTAGGTGAACTTATTCAATTAGGTATGGCTAAATTATCTGCAGCAATTCTTCGAGTCATTCCTG
GTATGAAGGATACTGCTGATGAAGTAGAAGGAAGAGCATTAGAAAATTTCCAAAATTCTACTGGAGCATC
TCTCAATAAAGAAGACCAAGAAAAAGTAGCAAATTATCAAGATAAACGAATGAATGGAGACCTTGGCCCA
ATAGCAGAAGGACTAGACAAAATCTCTAACTGGAAAACTCGTGCATCTAACTGGATTCGTGGTGTAGATA
ATAAAGAAGCGCTGACTACCGACGAAGAGCGTGCGGCAGAAGAAGAAAAATTAAAGCAACTTTCACCGGA
AGAAAGAAAAAATGCTTTAATGAAGGCTAATGAAGCTCGTGCTGCGATGATTCGTTTTGAAAAATATGCC
GATTCAGCTGATATGAGTAAAGACTCAACGGTTAAATCAGTTGAAGCTGCCTATGAAGACCTTAAAAAAC
GGATGGATGACCCGGATTTAAATAATTCACCGGCAGTTAAAAAAGAACTTGCTGCTAGATTTTCTAAAAT
 
TGATGCTACTTATCAAGAGCTCAAGAAAAATCAGCCTAATGCCAAACCTGAAACTTCTGCTAAATCACCA
GAAGCGAAACAAGTCCAGGTGATTGAAAAGAACAAAGCACAGCAAGCTCCTGTTCAACAAGCATCTCCTT
CGATCAATAATACTAATAATGTTATTAAGAAAAATACTGTCGTTCATAATATGACACCTGTAACGAGCAC
GACTGCTCCTGGTGTATTTGATGCGACTGGAGTTAATTAAGGAATAATATGGCAATTGTTAAAGAAATAA
CTGCTGATTTAATTAAAAAGTCCGGTGAGAAAATTTCAGCCGGACAGAGTACTAAATCAGAAGTAGGAAC
TAAAACATACACAGCCCAGTTTCCAACTGGGCGTGCTAGTGGTAATGACACTACAGAGGACTTCCAGGTA
ACAGATCTATATAAGAATGGATTATTATTTACTGCATACAATATGTCATCTAGGGATTCTGGAAGTCTTA
GATCGATGAGATCTAACTACTCTTCTTCATCTTCGAGTATTTTACGTACAGCTAGAAACACTATTAGTAG
TACAGTATCAAAACTATCAAATGGATTAATATCAAATAATAATTCAGGAACAATAAGTAAATCTCCTATC
GCAAACATTCTTTTACCGAGATCTAAATCTGATGTTGATACATCATCACATAGATTTAATGATGTTCAAG
AAAGCCTTATCAGTAGAGGCGGAGGTACTGCTACTGGTGTGCTAAGTAATATTGCTTCAACCGCAGTATT
TGGGGCACTGGAAAGTATAACACAAGGTATAATGGCTGATAATAATGAACAGATTTATACGACAGCCAGA
AGTATGTATGGTGGTGCTGAAAATAGAACTAAAGTGTTTACATGGGATTTGACTCCACGTTCAACAGAAG
ATTTAATGGCTATTATTAATATCTATCAATATTTTAACTATTTTTCTTATGGTGAAACGGGTAAATCTCA
ATATGCTGCTGAAATAAAGGGGTATTTAGATGATTGGTATCGTTCTACGTTAATTGAACCTTTATCTCCG
GAAGACGCAGCTAAAAATAAAACACTATTTGAGAAAATGACATCGAGTTTAACTAACGTTCTAGTAGTTT
CAAACCCGACAGTTTGGATGGTGAAAAACTTTGGCGCAACATCTAAGTTTGATGGAAAAACGGAAATATT
TGGTCCATGTCAAATACAGAGCATTAGATTTGATAAAACACCTAATGGTAACTTTAACGGATTAGCTATT
GCTCCAAACCTCCCTAGTACATTTACTCTCGAGATTACTATGAGAGAAATTATCACGTTAAACCGTGCTT
CTTTATATGCGGGGACTTTTTAATGTATTCTTTAGAGGAATTTAATAATCAAGCAATAAACGCAGATTTC
CAACGTAATAATATGTTTAGCTGCGTTTTTGCGACAACTCCATCAACTAAAAGCTCTTCGTTGATAAGTT
CAATTAGCAACTTTTCTTATAATAACTTGGGCCTAAATTCAGATTGGTTAGGATTAACTCAAGGTGATAT
TAATCAGGGAATTACCACGCTAATTACAGCTGGCACACAAAAACTGATAAGAAAATCAGGAGTCAGTAAA
TATCTTATTGGTGCCATGAGTCAACGTACAGTTCAAAGTTTATTAGGCTCATTTACAGTTGGTACATATT
TAATTGACTTCTTTAACATGGCATATAACTCATCTGGATTGATGATATACTCTGTAAAAATGCCAGAGAA
TAGATTATCCTATGAAACTGACTGGAACTATAATTCTCCTAATATTCGTATAACCGGAAGAGAATTAGAC
CCTTTGGTTATTTCATTTAGAATGGATTCAGAAGCTTGTAACTATCGTGCAATGCAAGACTGGGTTAACT
CCGTTCAAGACCCAGTAACTGGACTGCGTGCTTTGCCACAAGATGTCGAGGCAGATATTCAGGTTAATCT
TCATTCTCGCAATGGATTACCTCATACTGCGGTGATGTTCACGATGCATTCAATATCAGTGAGCGCTCCT
GAGTTATCATATGATGGAGATAACCAAATAACTACATTTGATGTTACTTTTGCGTACAGAGTGATGCAGG
CTGGAGCAGTTGATAGGCAACGTGCGCTTGAATGGCTTGAATCTGCTGCTATAAATGGTATTCAAAGCGT
TCTCGGAAATAGTGGAGGTGTTACTGGACTATCTAATTCGCTTTCACGACTTAGTAGATTAGGGGGAACT
GCAGGAAGCATTTCAAACATTAATACTATGACAGGAATTGTCAATTCGCAGAGTAAAATATTAGGAGCAA
TATAACAATGGGGACCGAAAGGTCCATATTTTTATTTACGGAATGAAATGAAAGCAGCAACTGAAGCAAC
TAAACTGTCTTCAATATAAACTTCAATTTTTACAGGAGCTTCTGACTCAAATTTACCTGTTACTACACCC
TGAAAAATACTTTCAGTCTGTTCTGGCTTTGAAAAATTTTCAGAAGGAAAAATTCCGAACTTTTTATCTG
TTCCAAAAATTTTGATAAATTCATCGTAAACCGCTTCGTTAAAAGCATTATCAGCAGGAATAACGCCTTC
AACTACAAGTTCTTGACCTAAAAAGCGTAAAGAAGATTTCATTTTGTGTTCCTCATGTTATGTTAGTAAG
ACTACTATAACACAACACGAGGGACTTGTAAACTACATTTTGAACTTTTTAGTACGCGTAATAGGCATGC
GTCGATTTTATACTGTTTCATTGTTTGAAGAGCAGTATCAAAAACAGCATTAATGACACCAATTGGATTT
CCACCCAAGTTTTTAAGCTTAACTAATGATAGTTTCTCATTAACACCTATCATTACGATATGCATCATTT
 
TATCGCCTGGTTTAACGTTTTATTTGTATCACCGCCAGAGGTGTATGTACAAAATCTAAATCCAGGAAGT
ACACTTTCTTCACCGGCTTGAATAGCAAAAATTTGTGGTATTTTATGCTTAGGATTTAATGTAACAACCG
GCGCCGAAGCGCCGTCAAATAATTCTGTAATAAGTTCCATGATTTATCCTTGAACGAACTTGTAAGGCAT
GTTTGCAATATCTATGCAAGACGCAATAATTCCAAGAGATGATTCTACTTTCTGTTTAACATTTGATGAA
ACAAATGATGCAAAGTCAACTCTATCTTCTATATCACCTGTCATTGTAACCAATTCACCAGTTTCCATTA
AATGGTCTCCGTCATAGATTACCGATTCGGTGAGTTCTTCTGTGGTCATAACTTCAGCTTGAATAAGCTT
GTTGTTAGTTTTAACTGTTCCATCATTGTAAGATGCATCGGTTATTTTATTAACTTTGACCATTAATCCA
CGTGGCAAAATGACTTCCATTTCATTTGAAGGCGCTAAACTTCCACCGGGTAAAACAACATTGACCTTAT
CAGCCCCAGTAATAACCCATCCAATTCCAACTAAATTATCGCTAGAATTTACAAGTCCTTCATCAGTTTT
ATCAATAGAAACGCTTAAACGCTTTTCGTCTGGTAAAACACCTATAGATGAATCAGTCATCCAAGTACCA
AAAATATTTGGATATAATGATGTTGACACAAAGTTTCTAAAATAAAAAACTCGATTTTTTACCATTGCTT
CGTATATTGAAGGTAACATTCGTTGTGAACGATACAAAGTAATACCTTTTGGTAATCGTTCACCATTTTT
AAAGGCTGAATCTAAATTATCAATAGCTTTTTCTATGTCAGATGCTGTCAAAATACTTGTACGCTCATCT
GGATTATATAATCCCAAAAGAGCATTATTTATGTCTACATATCCTGAACCTACGTATTCACGAATTCCGC
GTTTTTGCGCTGGTGTGTATTTAGATGAATCTTTATTTTCGACTATAGGATGTAATGACCATCCAGCGGT
AAGAGCATATCCACGTAATTCTCTTCGAATAATCTTTGTTTTTATTGCATTCCAAGAATTTTGTACTAAC
TCATTTGCAGCATCTTGGTTTAAATGCGAATGTTTGTTAATATTTCTTTCAAACCAAGCACCCTTATATT
TTTCTAAAGTATCATCGACGATAGAAGCAATAGTTTCTAAGGTTTTTATTGAAGTAATAGATTCTTTTCG
TAATCTTTCTAAAGCTTTATTTTTTATTTCTGCGTTAATAATGGATTCTTTATCTTCAGGAATTATAGAA
GCTTCTCTGAATGCCATCCCAGCTGTAACACTGCTAAGAGCATTCTCTAGTGCAAATCCTGAAGTAGAAA
TTACCGTTAATTCATTAGAATCAGAAATTAAAGGCGCGTCCGCTGGTTTATTTAATTCGGCCGCTGAAGC
CTCAAATTTTTGAAACATTGGTGTTTCAAATCTAGAAGATTCCAATGACTGACTTTGCGCAATTGCTCTA
CGGGAAATTTTAACTTTAACGATTACAGCTTGGTCAGAACGTTTATCATTTTCTTGCGCAATAGATGCTG
CAATTGCCTCATTTTTAGTTACTTGAGCCCCAGTATCTTTATTGATATAAACATCACCGACCTTCGATTC
AACTTTAGTAAAGAGCTCGGTACTAATTTCCGGAACTCCTGGAATGTCTTCTAGTGATACATTTTTGCGA
TGTATAAGAATATATGCATACTTTTTATCGTAATCCCAGAGTTCCTTAAGAAGGACGTATCTACCACCTG
AACGACTACGGATAAGTCTATCAGCAATAACTTGAATTTGTCGAGCTTGGCCAGCAGTTTTAGACTTAAG
AATACGGAGCATACAGGCATCAATTTTATACTGGCGCATTGTTTGCATTGCAACAGTAAAAACTGAATTG
ATATAATTAATTGGGCTTGGACCAAGACCTTTTAATTTAGCAATTGAACCTTTAGCAGTTAATGTAAAAG
GAACAATATGCATCATTTTATCGCCCATCTTTAAATCACGATTAGTATCACCTCCAGATGTATAGGTACA
TAAACGAAAGCCTGGTTGTTCAATTGCATCATCAACATGAACTGAAAAAATTTGCGGTATTTTCTTCTTT
GGATATAAGTTTGTAATTGGAAGAGTAGTATCTTCGTCAAATAATTCTGTAATAAGTTCCATCATATCCT
CTCTAGTGTTTATTCTATTCTATTTATAAAATTAAAGGCCCGAAGGCCTTTAATAATCTATTGGTAAGAG
AGTACGATATATTTCAAACTTTGGACCTTTTTCATAAGCATCAAATGTTTCTGTGAATTTATTATAAGCA
TATGCATCTATAAATTCAATCATGATTTGTGATACAGAAGTAGAAACATCTCCACCTTCTTTTTGAGCCA
CGACAATTGTTTCTAAGTAAGCTTTCATAGACCAGTTACCTCATGAAAATCACCAAATACATCTTCGAAT
GTATTAGCTTTAGTTTTATCTTCACGTAAACGAATCGCAATCGGAAGAAATAATTTAACGTAATCAGTGC
GGCCATCAGATTTTAACCAACCGTTGCATTCGCACTCTAGAATTTTTCCAATATAATAATTTTGGTTTTC
CATAATGCGAGTACGGTCAAGTTCATGCGATTTTACACCGGCTTTATCTTTTAAGCCTGAACCAGCATTT
ACCTTAATTTTTCCACACTCTGACTCAAGAATAAATCCACCCGCTTTAGTAGGGTCTTTACGGTGAGGAT
AAATTCCTACAATTTTTAAATCAACATCAATTACTTCTTTAAATTTATAAAGATTTTTTGAACGAGCATT
 
TTCCCATAATCCATCGATATTTTTGAGAATAATACCTTCAAGACCTTGGTCAATATACTTTTTATAAATT
ACCTTAGCTTCATCTAGGTTATTTACTACCTGGTTTTCAATTAAAATTACTTTATCATATCCAGATGTCA
TTTGTTCTAGTTTAGAAAAACGTACATCATATTTCAAACGAAATGCAGGAAGACTGTATATTTCTACCAA
CGGGACATAATCCCAGACCTGAAACTTCATGCATTGTGCTTCTTTTTCAGAAATGGTTCCCTTTAAAGAT
TTATTGGCGATTCCATTAGAAGCAGTACGTGATTCAGCTACTTCGGCGAATTCTTTAGCTTTACTGTTTT
CAGGATAAGCATCAAAAAGAAAATCTAGGCCTTCTGGCTCCTTTTTAACTTGCTCATGGTATACCAATTC
GCCATCAATCAACACACCTTCTGGATGAATCTGGCGGGCTTCAGCGGTCATTTTAATTAACTCTTCCTTA
AGAAGATCTAATCCTAGATATTCATTACCAGCTCGTGATAAAAGACGAACATCATCTAATTCATCACCTC
TAACTTCAGCAAAACACCGAGCTCCATCAGCTTTTAACTGAGCAAAGGCTGGAAATTTGATATTCTTATT
AATGCCTTTTTCATCATAAGAACTTGCGAGCATTTGAGGTTGTTCAGGAATTAAACCTGGCCAAACTTTG
TTTGCAATAGATACTGAAGCACCACATTCAAGGTCTCGCATCATCACTCGACGCAAAACTTCAACATCAT
CTTTTTTACCATCGGTGATATATCCAGTTAATTCCTCAATTGCTGCATTTCCAGTCAATTTCCGAGTAGC
TAATGTGAATTCAATGAAGTCAAGCATATCGGTAAGAGTCAACATTCCAAAACTCTGGGTAGCAATACCA
GGTTTAGGCCATTTCTTGATATAATACTGTAACCCACGAGAATAAGTCAGACGATATACTCGTTTAAGCA
ATTCATTATCTTTATTCTTTTCAAGAATTGCTTGCTTCTGTTTAGTTGAACCAATAGATGCTATTTCGTT
CAGAATTTTAAGAATCATTGTTCATCCTTTAGAGTTTGGTTTACAGCTCTATTATAAATCAATTCATCAT
TAAGCTCAGTCAAAGACCTGTGGTACGTGGTTCTAACTTTATTTCCTTGCATCCAGTGCTTGATATAAAT
GAAACCTTGCTCTACACATTTTTTAAAAATTCGTTCGTCTTTTTGAGCTCGGAATTCTGGATTGCATCTA
AAAAATTGATTTACGTGACCGTAATCACGTGTAGTATTACCTTCATTTTCATAAATAGTGTGAACAACAA
ACATTAGAATGCTCCTTGGAAAATATTATCACCACAAGTAGGTCTATTATACAAATACTCTATACCGCCG
GGCTTAATATAGTTCCATGTCTGAAACGGATGCGTCTGATATGGATGATATGGATTATAAGGATTAAATC
CAGGAGTTCTCCAGGTAGTGTTCCAAGGAAAAGAATCGTTTTTAATCATCTTTTCAATTACATCTTTTAT
AGCTTTTTCACTATCAAAACTTTCTTTATTTTCCTTTGGTGAAAAAAGCTTATTCTCTACATCGTTCCAT
GTATAAACTCGCTGAGCAGTTTTTGGAATATTGTCACGCTCTCCTCGAGCCATCCAATACACAGGAACAC
GTAATATTTCACTCGCGTGATCGCAGTGGTGAGCGAGATCGTCAATATAACAAATTACGTTATATTTCTC
TTTTGCTTTTTTGAACAACTCTTCTTTTGAAGAATCATGACTACACATCAGTACTTCTGAGAAAGCACCA
GGAAAAAGAGCATTCAAATTAAATTGACGATTTAATAGAGCGTCAATAGAATCACCCAATGCTGTAACAG
CAACAAAATTATAATCTTCTTTTAATTTGTTAATTACACACAGAGCATCTTTATATGGAGACAAGTAACG
AATAAAATCCGAACGATTGTATTTTTCAATTAACTTGACGCCAAGTTCTTCATCACAGTTAAAGAGTTTA
CCAGGAGAAATAAATTTCTCATCTTGGATCATTTTTAAAATATGTTCTAACGGAAGATTATATTTCTGAG
CAAAATAAGGAAGGCCTGATTGCCAGCTTAAACATACTCCATCAATATCAGTTAAAATAGTAGGCTTCAT
AGAGAGTCTCTTAATAGGTTTAACACATCAATAAATTCAGCTTCGGTTAGTATTGTATCATCTTTTGTTA
GACCACTAGCAATGCTGTGCTTCAAAACTTTTCCTTTCGAGGCTTGTAATGCATCACGAAAGCCCTTGTT
TTGAATCGCTGCTTCAAAATATGCATTTGTGTATAATTCTTTCCACGCCGGGGAGTATCTTGAAAACGGA
ACTCCAAGCCAAAAGAGGGTCCCACGGTCCTGAGCTCTAGCATAAGACCTTCCAGCTTGTTGGGCGGCAA
GCCCGGATAACCCAAATATACGACGTTGTTGTTCAACATTTTTCACCTTACACCCTTGGAGGAATCCTTC
AAGACCTCCAAATTGAATACCATCCATAACGAAAGGCCATTGGGCGAAATTACTTAATGCACATGATGGC
CACCTAAAATTGCTTCTAATCTCTAACTCAGACATTTTCAATGCTTATAATTTCAACATCAGCCCAATGA
CCATAGCAAGGAAGACGAAATTCAACTGGCCAGTTAGGGTCCCTTTCTAATATAATAGACTCAACTTGTG
GTTCTTCGTGTTCATCAGTATACGGATTTTTATCTGTTACTTTATATGTGACCTTAATGTACTGAATTCC
AAAAATCTTATTAATTATATTCATACTAATTCCTTTAATCCGTAGATAGGAGATAATTCATCACCCATAC
 
GAAGGTCTTCATTTCCATCTACCCAGGAAACAATATAAGCCTCTTTTATTTGAATACCACTCCATTTAAA
TGGAGGTAGAACCTTAGAAATTAATCCAGGTATACCAACTCCCTTTAATTCAACAGTTTGACCTAAAAAG
AATTTCATTAGAACCTCATCTGAAAACCGTGCGATTTAACATTACCGCCGCCAATATCAGAAATGTTAAT
TTCACGCGCAATTGAAGGGTCAATGTCAATTTCACGATTGAGTTTAGTGATAGCTAAAGTATCACGCCCA
TTTACAGTACGGAACTCTAAAGGACAAACCACATCAACGTAGTTTTTAATCGATTCGCTAGTAAGTTGCA
AATGTGCAGGAAGGTCTTTAGGTGCCTTTGAGAAAACCACTTCACAGAAATTTTTGCGGGTATCAAAATA
AGTAGTCATAAACATAATATTTTCCTCAGTAAGGGGCTGAAGCCCCTCATTTTATTTTAAATATCAAATT
CATTAAGAACTACATCAAAGATTGCTTCAAGATGCTCAGGTTTAGCTCTGTTACTCAGAATATGACGAAT
CCAAGTTTTAACTAAGAGTTTACGATTAGCACCATTCCAGCAAGGATGAGTCCCTAAATCGCGTTGGCGG
AAATCATCATCCAGAGCGATTTTGAAGTTGGAACCTTTCATCGTGATTGAAACCGTGATACCGTTTTCAA
ATCGCATATAAACGTAGTTAGGAGTCATATACTGTTCAATTTCGCATACTGATCCATTTTGATGTTTCCA
GAGGCAAATAGTATCAATAGAACCTGCAATACCATTAGAAACATATTTACGTTCAAAGTTAATGTAGTTC
ATTTTTATTCTCCGAGATGTTTAATTGCGGTACAGGTATATAATATCATATCCTGTACCAAAGTAAACAA
TTATTTTACTACTTTCCAATGCTGCATGTCAAGTTTACCAACTTTTTTCATCTTCTCAATTAAGCGTTCT
GCACGTTGGCGAGCTGTAACATAATGCCATTCGCCTAATTCATTTTGTTCAATTTTTCCAACGATTACTG
TATTCAATTCATAAATCCAACCAGTAAAGAAATTATGAACTTGAATTGTAAAGGTGAAATCTGTTCCCAT
ACCTTCTGTTGTTTCTACTTCAATAATATCACCTTCAACTGCCATTAAGAACCACATAGTTTCATCATAT
TTACCATTGAAGCATTTAGTTTTAACTGCAGCGTTCAGATTAATCGTTTTCATTTTATTCTCCTTTGTTT
GTGTAAGATAATACTATCACAAAGGAACTATACTGTAAACAACTTTGTGCAATCTTTGGAAAATAAAAAA
GGACTCCCGAAGGAGTCCTCAACTTATGCTTTCTGCTTACCAAAACGAGAAGCATCATCTCGAAGAACCG
CACGTGCTCGGCGCATGATCTTCTCAACAGTTTGATTGATACGAGAGTTCGACCCACGCTTGTAGCCAGC
GCGTTTAGAATCACCAACTTTCTTTTCAACTGCTTTCTTTGCTTTAGCTTGTTTTGCCATTATAAATTCT
CTTTTAAATGAAAATGCAGGACTTATTGGCATTGCCTGCGCAAGCCCTCAAGGGGAACATAGGTTTTTGG
ATATTTAACGACCAGGATAACCATAAACCCGTCATCATTCACATTCAAGAGGTACACCGTAAAACTGTCG
GGGTCTTAAAACTATAATGATTCGCAAATCATTAATCAGACAGTTCGACGGCTCCTCGATTTAGCTCACA
CTAAGGCAGTGAATCTCCAATAAATTACTTCAGTGTTACCACAAAGTGACGAACTGCTTTTCGTGCAGCA
GAAGCCAGAGGCTTAGCATATTTAAGTTCATCTTTTTCCTGAAGCTCAGCAGCTAATGCAGTTTGAGCAG
GATTCAGATGTTTGAAATAACGCAGGATTTCAAGAGCTTCGGCTTCAACATCAATAGATGCGCCATAGTT
TTCGTGACCATTATTCCATGCGTTTCGTTGCAGTTCAAGAGCGTGTTGTAATTGTTTAATCATTTAAAAA
TTCTCGTTAGAGATTAAAACTCGGTAGTCACGTTCTTCTGAATTTCATCTTCTTTCGACAGATCTCTCAG
TTGTAGACTACCACATAGAATTGTTCGGTTAACTTATTATTCCGACACCCAATTCATATTATTATTTATA
TCACTTATAAAGACACGGAATAGCTTTATAGTGACAGGTAACGAATTTTTGTTTAATTTCTTTTGGCTGC
TTAAGACCCAGAGCTACAAAAGGATGCGGAACATTTCGAATTTGACCAACTGGAAGAGAAGTCAAATCAC
CAACTTCGCAGAAACCTTCAGGAACATCAGGACCGACAGAGTGAACTACACACAGTTCAGGAACTTCACC
TTGAACACGTTTACCGATAATAAGTCCTGATTCTGTAACTTCTTCATCACCGGCTTGTGCAGGTTCAGAA
ACTAAAATAACATATTCACCGACAGCACGAATTGGTAGCTGTTGTACTTCAGACATCGTTTTTCCTTTTT
GTTAACAGATGAATTAATAATAACAAATAGTTCTTAAAGCATTTATTTACCAATAAATTGAAGCAAATGC
TCAACTTTCATACCATTAACGGAAATCAATTTGTCAATAGAAAAACCTCGCCACGCACCAAGCTCAACAT
CAAATACTGGAATCATGTCAGTAGATTCTTTCCGAGTAGATTCAGTCAATTTGCCAGTTTGCATGGTTGG
CATAAAGTCTGCATCACGAGTACCTTTCATAGTACGAATAGTACCATCAGACTTTTCAAAAACTACGTTT
GAAACACCCATGGACAATTTAGTTTTCAAAATTTCACGAATTGCTACTTTCTGCTCAGTTGTCAGTTTCA
 
TTTATTTACCTATTACAGTTTTAATATGAGTTGTTCCACGTTCTTTAAGGGTGGAAAGTAATTTTTGGCA
TTTTTCTAAATCAGATTTCCAACTATATGGTCTATCAATACAAACCCAATTTGTCTTATAATACTGTTTC
CATTTAGAGAAAAAATATTTCTTATACTCTACAGCAAATGAGATGTTCTCGTTAGAATAAGAACTAATTG
CTGTGAGTTTTACCAAACGAAATTTCATTATTCACCACAGAATTCGTTGATATTTTCCCAGTTTAACTTA
TTCAAGTTTTTCTTAGGAACATTAAACACTTCAATACCTGCATTTCGCAGAATATCATCCCAACCGGGTT
TATTTTTGTCGTATGTTTCACAATAAACCAGCTTTTTAATACCAGATTGAGCTATCGCTTTTGCGCAATC
TGGACAAGGAGAAAGTGTTACATACATAGTAGCACCTTCAATAGAAGAACCATTTCGTGCAGCAAACAAA
ATTGCATTTAGTTCAGCATGAATTTCATTTTTAGATGACCATTCCGAGTGAGCACTACGATGTTCTTTCG
CCAAAACAAAACGATCAGTTGAACCAAATGATACGCATTCAGGCTTATGACCTTGAATGATAGCATGTTT
AGGCTTATTCAACAACCATCCTTGCTCAGCAGCATAATCACAACAGTTCACACCCCCTGCGGGTGAACCA
TTATACCCAGTAGAAATAATACGTCCATTCTTTTCAATTACTGCTCCTACCTTCCAGGAGCAACATTTTG
ATTCCTGCGATACTAAATATGCAATTTGAAGTACTGTACTCGCTTTCATTTCATAATCACCAGATAAGCA
GATTTAGCAGTTTCAACACGATAAATTTCGTGACGAAGTTTAGTTATACTTTTAATAACAGAACTAATTA
TATTCTGCCCATCTTTAAAGCGGTTTTTCTTATCAATAAAAACTGCGCCAGTCATCTTTTTGTGAAGCTC
AACTGGATACTTCGTCACAATAATAGCATCATACACAGAAGGATGAATACTATTCACCAGAGTATCATTC
ATTAAAGTTATTCTAATGAACTGTGCTGTTTCAGAATCAAGCGCTCTATGATCGCCAGTATCATTTTCAA
GACAATTATCAATTATATCAGTTAAATTCATCATAGTACGCCATACACCCTTTGTGCTTCAACTAATCCA
TCAAAATCCAGTTTAAGATGCGATATTTGATCGCCATCACCTGGATTCACAATTACTAATACTGAACGAG
GAGTTTCGGTAATAACACGAACCGATGTTTCAGGAAATCGTTCAGAAACCTTATTTACTAATTCCTGCGC
AAATAGTTTAACTTTTTCTTGGAATTCCTTTAACAGTAATCGGTTTTTCACTTAGCAACATTTTGTTTTC
CTCATTTGTTTTGGTAGAGCTATAATATCACAACTCTACCGTAAAGTAAACCATTAAATCGCTTTGAATT
CCGCAGTTTGAGATTCAAAGCGAATATCGCCTTTGATAACAAGCTCAGCATCAAGACCAAATACGACAAT
GATATGCGCGGAACCTGGATACAGTGTAATGGCAATAGAATCCACCTGGTCTGGAAGCAAAGTGTTCAAT
ACATGAGTCACTTGAGCATGGATTCGAAGCTCAGCTGCGTTATCAAGTTTTTCAAACATATTATTAGCGA
TAATTTGGCTAAACACTACTTCTACGATTTTAGAGTAAGTCGGAAACATATTTACCTCACATAATTTTCT
TCAAGCCAATCAATAACATCCAACGCATTATCAAAAGTTGAACCATCTACTCTGTCTTCTGTTTCATAAT
CAAGAACATCTAGGCCTACTCTTCCGTCAACAATAGGCCATAGACAAAATAGATATTTCTTTTCTTTTTC
AATTTTATCACAAAGACGATAAATCTTTTCTAGGTTATTCATAAGTTTTCCATGGTAAAGGCAGTTTAGT
TTTCTTTACTACTAGTTCAACATCGGGATTCTTTTCTCTTAATTTAAGACATTCCTCCCATGCTCTATTT
TCACTAGTAAATACACAAAATTGCCCATTACTAGTACCAACTAAACCGCTATTTACAATAACAATAGCCC
AAGTTTCATGGTGCCAAGCCATTAAAAATCTCCCGAAGCGACTTGCCAGCATTCAACACCGATACGACGC
CACATTTCAACTACTTGAGTTCGGTCATCAATAGCTAATTTCACGTCAAAATGCGGTGCAATGTGTTTCC
AGAAAATTTCTTCTTTAACTACATCGTCTTTACGGGTATCGCCTTGTTCGCGCTGACATTGCATAACTAA
TGGAACGCCAGCAATGTCCTCAACCCATTTACGGGTCATACGATAATATTTCGTTGGGTCTTCTTTAGTT
CCACTTTCACGACCTGAAACGACTACGATTTGATAACCCATAAGAGCATACATCTTAGACAGTTCAACAA
CCATAGGATTGATAACATCGGTATCGCATTTTTCAAGGTCATAAGGACCACGACCATTCATTTTAGCTAG
TGTACCATCAACATCAAAAATAACTGCTTTTGGTTTACCAGGAGTCCCATTATATACTGGAAGACCGAGA
TACTCTCGCATGCTTTTATACATTGAACGTAAAACATCAATTGGTACTGCTTTAGTTCCGCGTTTTGAGT
TACGTTTAACCAATTCAGTCCAAGGAACATCAAACACTTTATGTTCAACTTTCCAGCCGTATTCTTTGGC
AAAAGTTTCCCATGCTAGGCGACGTTCAGGATTCAGGTTAGTATCTGAAATGATTACTCCCTTAACAGAA
TCGCCACCGTACAGAATACTTTTAGCTGTATCAAACTGCATACCAGTTACGATACCTTCTTTCTTTTTGG
 
TATACTTGTACTCATCGCGTTCTTCATGCGCCATAATAGATTGGCGATAGTCATCACGATTGATATTATA
AAACCCGGGATTCTTAGCAATAAATTCACGAGCCCAAGTACTCTTACCAGAACCAGGACAGCCAATAGTC
AAAATAATCTTTTTCATTTATTTTTTCTCAACTAATGATTGAATATAATCATGTAGGTCTTTAGATGCTT
TACCCCACTTATTTTGATATTCATTTTTGAGATTAGCACGTGATTGAGCTAATAAAACATCATTAGTTGG
AGGTAAAGATTCTAACCGCTGAATCTGGCGTCCATAAATCATTGCAGCCATCTCGGATTCATAAATCAAT
CCTTTGAGATGTTCAAATTGATGCCATGAAATCATTTACATTTATCCTCTTTTAGCTCTTGACGATAATA
ACATRTCATAGTTTTCTGGTCATGTACATATCGTTTTACATCATTAAGCCAAATACGAAATTCCTGAGAA
TCTTCAAATGGCATACCGACCCAGGCTTTACCATCAATAACTTTAACTTGCCAAGATAGTTTAGCTTCAT
CATATGACTTTATTTGCACAGGCCAATTAGGATGAACTGTTTCTTTCTTTACTTCTAGAGGCTTTGTCGA
ACAACCAACTAGAAGACCAATAGATAATATTACTGCTGATAGTTTAATCATTTAGAAAGGTCCTGGATGT
CTTCTGCGAACTTGTTGAAGGAGTTGTTGATTTGTTTTTCAACCAATCCTGGCTTATGAGCCACCACATC
CGCCTTCTTTGCATCTTTGCGCAGTTTTTCATTTTCACGCTCAATAGCAGCAATTGCCTCACGATTTTTA
TTATTCATCGCATCAATATAATTATACTGAATTCGCAAATTATTTAATGCTAAGGCGTTTTCATTGGCCG
TTTTTGTAATTTCAGTAACTGATGTTTCTAATCTTTCTACCTTATGTTTTAAAATAATAGATGTTCCGCC
AAATGCTATTACAAGTAATAGCAATCCAGCTGTAAAATTACTTACATGCATAAAGTTTTAATAACCTCTA
CAATATCGTCTTGAGAAAGACCGTTAATTAAAATATGATGTTCAGCTGGAGATTTAGAAATTTTAAAGCA
TGCCTCAACATCTTCTGCCATATCCGATGCACTACGATTTGGATTACTAATACCAAGGCGATGTTTTCCC
GTCAAAGGATTAACGATGATATAGCATTTGCAGCTATTGATATGAACATTGGGTTGAGTCTGATTAATGA
ACACTTCACAATCATACTTAGCAAGTTGATTTTCTAAAAAGACTTTCATCTCTTCAACCGCATCAGGAAG
CATATCACGGGCTTGCTCAAGACGACGATTTCGATATTCTTTAATGGTCGTTTTCCGCTTGACTTGCTTA
GCTAAATCTTTCTTAAGACCAGTGATATATCCAACTCGACGATTTCCTTTGAATACAGAAATCCCATCTG
TAGTATCACCGTATGCTTCAACGACCATTTCAGTAGTAATAAGTTGTAAATCCATCATAAAGTCCTCATG
TTATGTCAGTAAGACTACTATAACACAACACGAGGGACTTGTAAACAACTTAGTATCCTTCTGGGATAAA
TTTTTTATAATTTTTCAAAAAATTCTGTTCGATTTCACACATGACCTTTTCTTGACTATCGTACCCCTGG
TATAAGCTCATGATGATACCGAACAGGTGATCCATTCCAGCACCTTTAGCAACACCTTGTGCTTCCATTG
CATAAGTCTTTCTATCCTTACCGCAATGCTTATTATGACAGTCAAGAACTAAAAACAGAGCTCGGTCTAA
GTACTTCAGATAAGTCGTTTCAAATGCTTCAATTTTTCTGTATGAATATTCATCGTCAGCATACATTGCT
TTAAGATCATCTGATGCACCATCAATAATAGTCTTAAACAATTTTTCTGGATTATCTAATGAACTTTTTG
TACTATGAAGAGACACGTACCAGTCAGACTTAATTTTAAAATGAGAACCATCTTTCATCACAGCAACATA
GCCTTCGATGTTTTCTGCATTTTTAGCTTCTTCTATCCATTTAGGGCTATCGATTTCGTATCGTTCAACT
AGATACGGACGAAGAGTAGCATCTTTATAAATATCATCGTATGAAATGTATTCACCCGTTTCGTTTTCAC
GAACATTCAGTAAAATAATTTTCATCTCTTGATAAGCAAGAACGATTCTATTCGTCGGGGCAACGAATTC
GAAGTTAGCAGTAAATCCATCTTCAGCTAATTCTTTAAGTCTATCACGCAACCGATGGTGATTAATATTC
ATCAAAATTCCATTAGCCATTAAAGCCTGCTCAGATTTGATTGAACCCTTTGATTTGAACAGAATTTCAT
CACCGTCTAAATAAGTTGATACCAAAGACCCGTCTTCTTTTGTTAGAATATAATCAACATCGTTTAAATC
GATATTCATCGTGAACGGATTTTCATTCAAGTTAAAAAACTTTTCCATAGGACGAGAAGCAATTCTTACT
GGTTTTTCTCCATCCATTTCAAACATAATTCCACGACATTCTAGTGCATCTGGAAGTAACCAATCAGAAT
AAGATGCATAATTATATGAGAAAATTCTGTAAGTTCTTCCAGATGCACTTACATCATCTGAGTAAAAAAA
CTTACGCTGCGAATCCTTACATAGTTCCATTAAATTGTTAAAAAGTTCTTGCATTGTGTATCCTCTTTTG
TGTTTTGAATATAGTACCACACTCCATGTGGAAGCATCATTTTTTCTTGTGTTGAATATTCCAAGGCGGG
TTAAACAGTTTAATGAATAGAGGCTCCTCTAAGTCAATCGTTGCGATTGTCATTGTACCTAACTCATTTG
 
TCATAGAAAGATTAAAACATTGGCGGGCGTAAAATTCAACTTTGCTTCCTTCCTTTAGCGCAGAATGAAT
TAATGCAGATTTAGTAGAATCAGACGTTTTGTCTTTGCGGTTAATAGCAGTTCTATAATAGTTTATTCTT
TTACGTAAATTTTTAGTTTTTCCAATATAAACAAGCTCATCATTTATAGCAATAGCATAAATTACGTTAT
ACTTGTTTGGAATAGATAATTGTTTTATACTTCCATTGTCGTCTAATTCTAGCTCAGTATATTTAATAAA
TGAATATTCTGTTGCAATTTCTTTCATAATAAAATGGGCCTTGCGGCCCACTCCTTAAAAGTATTTTTTA
AAACTCATCATAACTTTATCATCAACATCATTATCAATCTGTGCAACAAGGTAAGATGACAGTTCTACTT
CTTGCGGCGCGGATTGAACATTATCAGAATTAAGATATTCACGAATCCAAGGATATGGATGTTTAACCGG
AGCATCGGTAATTGGGCATGGAAGACCGCACTGTTTCATACGAGATACAGTTAAGTAATCAATAAAGCTC
CACATGCTATTTGTATTTAATCCAGGAACATCACCATCTTTAAATAAATGAACTGCCCAGTCTTTTTCCT
GGCGGTTAACTTCCATGAAAATATCAACTGCTTCTTGTTCACACTCTTGGGCAATTTTAACCCATTCATC
ACCATCAGTACCGGATTGAAGTTGACGAATAATATATTGGGTGCCTTTAAGGTGAAGCTGTTCATCACGT
GCAATGAACTTCATAATCTTGGCATTACCTTCCATGATTTCCATGTTCTTATGGAAGTTAAAGGTACCGT
ATGTTCGATTGAGCTCGCTAATTCTCAACCCGTTCTCTTATGAACTGCTGCATGTTACCATGCAGTCCAG
ACTATATCACAATCCCGGAGGGATTCTCCCCATTTCGAGTCGCTTGACCCTACGTCCGAAGACTAGTCGT
TGAACCTTGTTTTGCAATTGTCGCCGTGCCATCGTTTATATGTTGATGGTGATAAATCTTTCTTATCACA
ATATGGACAATTCAATTGAATTCTATTTGGATTTAATTTACATCTGTCATTATGTTTTAGATAAAATCCA
GGACCTTTACCAATGTGACCACAGAAATCACATGTGATTTCTTTTTGTGCAGGATGGGTCCCGTTCTTAA
CCATTTCTAATGTTTTTGCTGATGTTCTTTTCTTATGTTCTTCCATCAGAATCTTTAGGTATAGGTCCGT
TAGCATCTATCCAAATTTTTCTATAGTTCAATTGTGCATCCTCTTAAAAGTATAATCATATTTATATTAT
ACTAATTAAAGGTGCAAGCAAAACCTTGGCTGCTAGTTTTCCATAAAGGACTTTCCAGCAATTAAAGGAG
TTTTCGATAAACGTTACCGTTTAAAGGCGCATTTTACGCAAAAGATACATAAAAACGAATAGCTTCTAAG
GCATTAATAACGTGCAAACAGAGGTAAAGAGATTTCATCAGATCTCGTTTAGCTCTTTGCTCAACGTCTT
TATCAGCTAGAATTAGACCTTGGTCTTTATAATATTCAACCATGTCTTTAGCATTTTCCCATTCACGGGT
TTTAACCAAGACATCATCGTAATATCGCCCAATGGACTCAGCACGTTTCATAATAGCTTCATCTAATACA
ATCTCATCAAACACCTTCGATGGATCAGTATAAAGATTTCGCATGATATGAGTATATGAACGACTGTGAA
TAGTTTCACTAAAAGTCCATGTAGCAACCCATGTATCAAGGCTTGGGTCTGAAATTAATGACATAAGTAC
AGCAGATGGCGCACGACCCTGAATGCTATCCAAAAGTGATTGATACTTCAGGTTGTTAGTAAAAATATTT
TGCTGATACTGAGGAAGCTTATTAAATTGCGCAGCATCCATCATTAAGTTTACTTCTTCAGGACGCCAAA
AAAAACTGATCTGCCGCTCAATGAGTTCTTCAAATACTCGATGTCGTTGAATATCATATCGAGCTAAACC
TAATCCAGAACCAAAGAACATCGGTTCATTCAAAACATCAACTGGATTTGTATTAAAAACTGTACTCATT
TAGAATCCTTAAATTTACATTTATCATAATGCCATCTTAAAGCATTGCCTTTATTAACTTTCTTACCACA
GTGCGGGCAAGGTGGATACTCTGCTCCTTGTTTAGTTCTCAAAGAAATCAAATCACGGGTTTCTTTAGTA
TGAGGAACATCTCTTGTCGGAGAAATTGTACCATACATCGGATTAAGCACGCCGACTTTAGCAGCAGCTA
TATTCTTTTTAGCTTCTTCAGTTTTAGGCTTTCTCATCTTTTTCTTAGGTTTCTTCGCTTTTAGCCCTTC
CTTTGGTTGCCGCCGATATTTTGTGTTTGGTTTCTTCTGTTAAACGCAGACCTGTTTTCGCTTTTTGACA
TTTTCAGTCTCGTTTCTTCAGAAATGACCGTACCTTTGCGAAATTGCGAATTTAACTTCTTTGCATGGGC
ATATATTTTAGAATGAACTTTATAATGACGTTTCTTAGTTCCTTTCATATTGCACATCATGAAAAATGCG
AATATAACAGATTTTACCGGATAAATCTTTGATAAAATAGCATGCGCTATAAAATGCTCTCTAGCTGTTA
ATTCAACTAAATTTTCTTTATCATCAGAACCTCCCATGCATCTAGGGATTATATGATGTGTCTCTTTATA
TTCGGATAAAGGTTCCCGAGCCTGAGCTCGGGAAATTAGGTCGTTATAGATTTTTTGATAATTCATTACA
ATTTACACGCTGCACAATCATCGGCTTTAGGAGTTTCTATTTCATAATCATCAGTACCAGAACCATCACG
 
GGTATTATGATAATAGAAATTTTTAATGCCATAATACCATCCGTATAGCATGTCATCAATCATTATTGAC
ATTGGAACCTTTCCTTTTGGAAAAATCTGCGGGTCATAATATGTATTCGCTGAAGCTGATTGACATACCC
ATTTCAGCATAATAGCTACCTGCGTAAGATAAGGTTTATTACCTTTCTTAGCTAATTTCCATGTATAATC
ATAGAGGTCTATGTTATGCTCAATATTGGGCACGACTTGATTAAAGGAACCCTCTTTTGATTCTTTAACA
CTTACCGGTCCACGTGGAGGCTCGTAGCCGTTTGTACTGTTAGAAACTTGGGAAGATGACTCACATGGCA
TAAGTGCTGATAATGTGCTATTACGGATGCCAAATAGCTTAAGGTCTTCCCGCAGCGCCGACCAGTCACA
AACGTATTTTGGAGCTGCGATTTGGTCAATCTTTTTATTGTACCAGTCGATAGGTAATTCGCCTCGAGAC
CAACGAGTGTCTGAATAATATTCCGAAGGTCCTTTTTCTTTGGCGAGCTTAATGGATGCTTTAATGAGTC
CATACTGTAATCTCTCAAATAGTTCATGTGTTAAATCGTTAGCATCTTCATAAGAAGCAAAGTTACTTGC
CAGCCAAGCTGCATAGTTGGTAACACCTACACCGAGGTTACGACGCTTTTTAGCTTTTTCTGCTTCAGGA
ACCGGATATCCTTGGTAATCCAACAGATTATCAAGAGCACGAACTTGAACTTCTGCCAATTCATTAATTT
TATCTTGGTCTTGCCAGTCAAAATTATCTAATACGAATGCAGAGAGAGTACACAATCCAATTTCAGCATC
AGGGCTATTCACATCATTTGTTGGAATAGCAATTTCACAGCACAAGTTACTCTGACGAATAGGTGCCTTT
TCACGAATAAACGGAGTATAGTTATTCGTATTATCAATGAACTGCACATAAATCCTTGCTGTTCCTGAAC
GTTCAGTCATGAGCAATTCAAATAGTTCACGGGCTTTAATACGCTTTTTACGAATATTAGGGTCTTTTTC
TGCTGCTTCGTATAATTCACGGAAACGGTCTTGGTCTTTAAAATAAGAATAATAAAGCTCGCCACCCATT
TCATGCGGACTGAACAAAGTAATGTAATCGTTTTTTCCAAAACGTTCCATCATCAAATCATTCAGTTGAA
CACCATAATCCATATGACGAATGCGGTTTTCTTCTACGCCTTTGTTATTTTTCAAAACGAGAAGATTTTC
AACTTCCAAATGCCAAATAGGATAATAAGCAGTAGCAGCGCCGCCACGAATTCCACCCTGTGAACATGAT
TTAACTGCAGTCTGAAAATGTTTCCAAAAAGGAATAACACCAGTATGGCGTACTTCACCCATGCCAATCT
TAGAACCTTCGGCACGAATCATACCAACGTTAATACCAATTCCAGCGCGTTTAGAGATATATTCAACAAT
TGAAGCAGAAGCCTTATTGATAGACTTCAATGAATCTCCTGCCTCAATAACAACACATGAACTAAACTGT
CGAGTCGGAGTACGACAACCAGCCATAATAGGAGTTGGCAATGAAATCTGTCGAGTTGATACTGCTTCAT
AAAAACGGATAACATGTTTTAATCTATCAACAGGTTCATCTTGATGCAGTGCCATTCCAATAGTCATAAA
TGCAAACTGTGGAGTTTCATAAATTTGACCAGTGGTTTTATCTTTAACTAGATATTTTTCTTTTAATTGC
ATTGCCCCGGAATAAGTAAATTCCATATCCCGTTCGTGCTTAATTTTTGATTCTAAAAATGTAATTTCTT
CTGCTGAATATTTTGACAATAATTCAGGGTCGTATTTACCTGCATTTACACAATAAGAAATATGGTCAAT
AAATGAACGTGGTTCATACTGCCCATAAACATGCTTACGAAGAGCAAACATTAAACAGCGTGCAGCTACA
TATTGATAATCAGGTTCTTCAACCGAAATAGAATTCGCAGCAGCCTTAATGACAATAGTCTGAATATCAT
CAGTTGTCATTCCATCACGGAGATAAGATTTAATATTTTCATATAATTCATAAGGATCTACTGATGTTCC
TTCAGCTGCCCAAGATAAAACTTTAATAATTTTTTGTGGGTCAAAGCTCTGAGAAACACCACTACTTTTG
ATAACATTAATTAATTGCATAAGTCCTCAACTTGAAAATCGTCTTTAAACAATCGGTTAACTATATGAGC
TATTATATCACCATGACACGGCTTTGGTTTACATGTGCATCCTAGCCTCATTCCACGTAAAGGCTCTAAA
TGTGCTTTAGTTATTTCTCCGGATTTAATTCGACGTATAAAATCTTTTTTGAATAATTCAATGGCAGCCT
CCCGGCTGCCAGCATCTTTACCGACGTAATTTCCCCAAAATGTACCGCGGTGAATATTAACATCAAAGTC
GGATTTATATTTATTCACTACCCGGCATAGACGGCCCACGCTGGAATAATTCGTCATATTGTTTTTCCGT
TAAAACAGTAATATCGTAGTAACAGTCAGAAGAAGTTTTAACTGTGGAAATTTTATTATCAAAATACTCA
CGAGTCATTTTATGAGTATAGTATTTTTTACCATAAATGGTAATAGGCTGTTCTGGTCCTGGAACTTCTA
ACTCGCTTGGGTTAGGAAGTGTAAAAAGAACTACACCAGAAGTATCTTTAAATCGTAAAATCATATATCC
TCGCAATAATAAAATTACACCGCCATCTTTCCTTTAATAGGAGGGTGTGATACATAGTTGTTAAGAACGA
AATCTTTAGGCCTAAGTTTAAGAACATATTTTAATTGTTCTTTAGTAGAAAGATATCGGAATTTATAAGG
 
TAGACCACTTATTACCAGCTCACAAAGCTCTTTAGGTTCACGCCTCAAAATTTCTTTACATTGTTCTACG
TGATTCATATAGATATGAGTATTACCACCAGAAAATATCAAATCCCCTGGAATAAGATTACACATCTTAG
CTACAATATGAACTAACGTAGCATATGACGCAATATTAAACGGTAGCATTATGTTCAGATAAGGTCGTTA
ATCTTACCCCGGAATTATATCCAGCTGCATGTCACCATGCAGAGCAGACTATATCTCCAACTTGTTAAAG
CAAGTTGTCTATCGTTTCGAGTCACTTGACCCTACTCCCCAAAGGGATAGTCGTTAGGCATTTATGTAGA
ACCAATTCCATTTATCAGATTTTACACGATAAGTAACTAATCCAGACGAAATTTTAAAATGTCTAGCTGC
ATCTGCTGCACAATCAAAAATAACCCCATCACATGAAATCTTTTTAATATTACTAGGCTTTTTACCTTTC
ATCTTTTCTGATATTTTAGATTTAGTTATGTCTGAATGCTTATGATTAAAGAATGAATTATTTTCACCTG
AACGATTTCTGCATTTACTACAAGTATAAGCAGAAGTTTGTATGCGAACACCGCACTTACAAAACTTATG
GGTTTCTGGATTCCAACGCCCGTTTTTACTTCCGGGTTTACTGTAAAGAGCTTTCCGACCATCAGGTCCA
AGTTTAAGCATCTTAGCTTTAACAGTTTCAGAACGTTTCTTAATAATTTCTTCTTTTAATGGATGCGTAG
AACATGTATCACCAAACGTTGCATCAGCAATATTGTATCCATTAATTTTAGAATTAAGCTCTTTAATCCA
AAAATTTTCTCGTTCAATAATCAAATCTTTCTCATATGGAATTTCTTCCAAAATAGAACATTCAAACACA
TTACCATGTTTGTTAAAAGACCTCTGAAGTTTTATAGAAGAATGGCATCCTTTTTCTAAATCTTTAAAAT
GCCTCTTCCATCTCTTTTCAAAATCTTTAGCACTTCCTACATATACTTTATTGTTTAAAGTATTTTTAAT
CTGATAAATTCCGCTTTTCATAAATACCTCTTTAAATATAGAAGTATTTATTAAAGGGCAAGTCCTACAA
TTTAGCACGGGATTGTCTACTAGAGAGGTTCCCCGTTTAGATAGATTACAAGTATAAGTCACCTTATACT
CAGGCCTCAATTAACCCAAGAAAACATCTACTGAGCGTTGATACCACTGCAAATCCAAATAGCCATTACG
CACATTAAACTGATAGAACATATGACAAGGCGGTAATGCCATATATTTAAGTTCAGCTGGATTCCATGCA
GAAACAATTTGACGCCTATCATTTGGCAGTTTTTTAATACGATCAATAACTTCTATAATTTGGTCTACAC
CACCAAAATCACGCCACTGTTTTCCATAAATTGGACCAAGTTCACCGCTATGGTATCCTAAATCTTTTGC
TTGATTTTCGTAATTTTCATCCCAGACTGTTTTGCCTTGGATTAACGAATCGTGTTGAATTAATCGTAAA
TCATTGACATTTGTGCTTCCTGATAAAAACCATATTAGCTCAGCAATGCAAGCTTTCCAGGCGAGCTTCT
TAGTTGTTACCGCAGGAAAACCTTTAGTTAAATCCCAGCGTAATTTAGATCCGAACAGAGCAATTGTTCC
TGTGCCTGTACGATCATCGGTTTCATAACCATTTTCAAAAATGTCTTTAATTAAATCTTGGTATTGTTTC
ATTTATATACTGATTCCGTAAGGGTTGTTACTTCATCTATTTTATACCAATGCGTTTCAACCATTTCACG
CTTGCTTATATCATCAAGAAAACTTGCGTCTAATTGAACTGTTGAATTAACACGATGCCTTTTAACGATG
CGAGAAACAACTACTTCATCTGCATAAGGTAATGCAGCATATAACAGAGCAGGCCCGCCAATTACACTTA
CTTTAGAATTCTGATCAAGCATAGTTTCGAATGGTGCATTAGGGCTTGACACTTGAATTTCGCCGCCAGA
AATGTAAGTTATATATTGCTCCCAAGTAATATAGAAATGTGCTAAATCGCCGTCTTTAGTTACAGGATAA
TCACGCGCAAGGTCACACACCACAATATGGCTACGACCAGGAAGTAATGTAGGCAATGACTGGAACGTTT
TAGCACCCATAATCATAATTGTGCCTTCAGTACGAGCTTTAAAATTCTGGAGGTCCTTTTTAACTCGTCC
CCATGGTAAACCATCACCTAAACCGAATGCTAATTCATTAAAGCCGTCGACCGTTTTAGTTGGAGAATAA
CGGAATACCAATTTAATCATTACGTAAATCCTATTTTAATTGAAAACGAATGCTTACTTGGATAATTTCA
ATGACATACATAATATTTTCCTCAAACAGACTTTTTCACAATTTTCCAATCAGCTTTAAACTGCTCGACG
TCAGAATGGTAAATCCAAAATCCTGCGCTTTCTCCGTCTTCATAAAGAGGACATCCATCGCATTCATCTT
CCCATCCCATATCACGTAAAAGATGTTCAGCTTTTTCAACAAGTTCAGAATCTTTACCGATGATATTAAA
ATACCATTTACCTCTAACTTCTGAATCTTTGATGCTCTGGCGTTGTAATCTCATTTTATTCTCCTTAGCA
AGCTTTAATCAAAAGATATAAACAGACCAACATAACTGCTGCCATAATATAAGGTGCGAACATTTTCTTT
TCTCCATTAGTTTTGATAGGGTAATAGTATCACACTACTACCCTGATGTAAACTACTTTTTGAAAGTTTT
TCGCAAAAGTTCAATGATTTCATCTACATTGTTTTCGTCAACAATGCAGTGAATTTTTGTTACGCCAGAA
 
ACCTTGTCTTTGACTTCATCTTCTTCAGAAGTCGGTTCTTTATATTCGCGGAAACAATAAAACTCTTCTT
CACTAAGTTCAAAATAATCATCACCCATACCATCATCATTATAGATTTCACCATTAGCACAAATGATTTC
GGTTACATAATCAAAGCCATCTAAACTTGATATTGATTTAACTTCAAACCAACCGCCATTTTCTTGAATG
ATGCCGACCATACTAGCATTTGATGAACTAATATCAATGAAAGATTTAATACGATGTGGATTTAACTCGT
ATTTTTTGCCGATTTCCATTTTGATTTTCCTCATTTTAATAGGGGCTTGATAGCCCCTTGATAATTATTG
TTCAATCAGTCCCATGTAAAATTCTGCGTCTTCAGAATCCATGCCATCACAATATTCATTAGCCATAAAG
CGGGTGAGGTCTTCAAGAGGACCTTCAATAACGATTTGAATACTCCAAAACTTAGAATCTTGCACGCTTG
TGATACTAAGTTCAGGATAACGATTACGAATAATCTCTTCAATATATTCAAAATCAACGATGTCAATATC
AACTTTAGCCATATTATTTTCCTCTTTAATTATTAGCAGTATTGCCGATAGTTGTATAGTACCATAAAGC
TTTATGCTTGTAAACCGTTTTGTGAAAAAATTTTTAAAATAAAAAAGGGGACCTCTAGGGTCCCCAATTA
ATTAGTAATATAATCTATTAAAGGTCATTCAAAAGGTCATCCAGGTCCGTGTCATCAGCACTAGATGAAC
TACCAGAGCTTGAGCTCATAAAATCATCTTCAGTTTTTGTATTGAAGTCATCAACATTGAATGCATCCAA
ATCATCAGCCACTTTATCAGCTTTCTTAGCAGCAGTTGCAGCAGCACCGCCCATCACAGCAGTTCCCATA
ACTTGACCGAATTTAGTATTAAGTTCTTCAAACGATTTGAATTTATCTTTAGAAGTCATTTCAGAAAGGT
CAACCATTTGTTCGAACAGTTCTTTCTGGAAAGATTCATCGTCAATGTTTGGAATCGCAGATTGATTCAG
GAATTTAGATTCATCGTAGTTACTAAATCCAGAAACTTGTTTAACTTTCAGTACAAAGTTAGCACCTTCC
CACGGACAAGTTACATCAACTGGAGTTTCACCCATTTCAACATCAACCGCAATCATTGCATTGATTTTAT
CCCAGATTTTCTTACCAAAGCGGTATTTAAATACTTTACCTTCGTTTTCTGGAGCAGCTGGGTCTTTTAC
TACAAGAATGTTAGCCCAGTAAGAAGTTTTACGTTTAACAAGACTGTACTCTTTATTGTCAGTGTTGTAT
AGATCATTTTTACTGATGTATTGACATACTGGGCAAGAATCGTAATCACCATGGGTAGATGAACATGTTT
CAATATACCATTTACCATTTTTCTTGAAACCGTGATTTACAAGAATTGCGAATGGTGCTTGTTCATCATT
TTTAGACGGAAGAAAACGAATTACTGCTTGACCGTTACCCGCATTATCGAGTTTCAGTTTCCACTCGCCT
TTATCTTCAGAAGAAAAACCTTTATTGCCATTCAGTTTAGCCATTTGTGCAGCGAGTTCAGCAGTAGATT
TACGTTTAAACATTTTTATTTCCTTTTTAATTTAATTTAATTAACAGTTGGTGCTATGACACTTTACCTC
ATAGCTGGCATAATTCGCAATACTCTGGGTCTTCGAGAGGTATCCAACCTGAGTTGAAATACTTTACCAT
CGATTTAGCAGTTGTATCAGTTATATTTATATTACCTTTAACTCTTCGCCATCCAGGAGTTTTACCGTAC
AGATTAGAGGATAATAATAACACATAATTCTCGTAAGCAATATGAGATAATTTCCAAGACTCTATATTAG
CTCGTGATGTTTTCCAAGGTCTAAAATCGTCACGGTTCATATAATTAGCCAATCTCATATGCTCTCTAAC
TTCCGGGTCTTTGGCTGGATGAGTTTCACCACTCACACCAAATCCACCACCAGCATATACCAGATTAAAA
TAGTCTGGATTATCTCTGGCATTTACTTCAAGTTGGTATTTTCGTTCTGCTTCAATAACATCCAAGTCAT
CATCAATTTGAATTATTTTAACGCTCGGTTTTTGAAGCATTAGCGCATTCAAAAATCTTTTTTGTTTACA
TGAGCTCCAGTATTCCTTTCCGGAAGAGTCATATATTATTCCGTTCTCAAATGAGCAATTTAATTTACTA
CCGATATAGTAGTATGGCGGAGTCTTATTTTTGACACGGTCTTCAAATGTAAACCAATATACTATATTCA
TATCAATACTTGCAAGATTTCACAGTTTCAATGAAAACATTTTTAGCTTTCTGTGAATCAATATTTAAAA
TTTTTCTATAAGCCTTTAACTTTATAGAATAATTATTCCAGACTAAATTATCAGTCTGTTCATCATGTTT
ATCAATTATATTTAAAAACGAATCAAGCAAGATAAACGTCTCAAACGAAATTATGTTCGATTGCAGAAGT
TTAAAAATATAACTTGATTGAACTTTTGGATTATACTCAAAAATTTCTTTAAAAGCAGAAACTTCAACTT
TTTTACTAAAATAATAAATGTTGCGAATATCTTCTTCAAACTTAAATTTAATTTGCTTTAAGCGTCCGAT
ATATTCACGATAAAACACAAGTGCATCAGCGTCAGAGATGTCACCAATCCAAGCATCTTGGTTAGCAACC
AAATTGCTTATAAAGATTAAAGCAAGTTCCTTTAATTTATATTTTTCTGATAACTTCTGGAAAAAATACT
TATCCCTTCGCTTTTGATAAGCGGCATCAGACACCCGCATGCACCAATTATACTTAATTACATCATACTT
 
TCCATTCATATGTTGTTTTATCATTAAGTATAATTTATAAACTGATTTACCATCAATGTATCTTTCACCA
CCAGCAGGCATGCGGAGTTTAATCATAGTAGAAAATCTAATGTATTAGTTTTTTCACAACGAACAACAGA
AGGACGTAAAAGATTTTCGTCAATAGCTTCTGACTGAATTTTTTCAATTATACCCGAAGGAATAAATTTA
GCAAATTGAGTTTCAGGAATAGAATTTTCTTCTAAGAATGCTGTTGTAGCTTCAAGATAACTCATTCCAA
ACTCTTCTACCATTTTTTCAATAATAAATCCATTTTCTTGGCGGTCAAGAAGCTTTGCTATTTCATCCTT
TTCTTTCTTAATTGAAAGTTCTTTTTCTGAAAGACCGGTCTCATCGACCGGACGAATATCATTTAGAGAA
AACTGTGTCATAAAGTTCAACTACCTCTTCAGTTTCAGCTTCAAACACATCACGGTTATCTTTATGATAC
AAAGCTAATAGACGATTAAACATCTTACCATCAACGCCAAGTTCATCTTTAGCACGAATTCGAATATCTT
TAATCAGTTCATTATAACCGGAAATTTTCAGTTTATGATCAGATGCTTCTTTAATAAATTTAGCCAAGTC
TTCGCCATGGATAGCTTCATCAAATTCAACCATTTCTTTTTTAGCCATTATTCACCTCAAAATTCATTAA
TGCTATTAGTTAATTTAGAAAGACCCGCTTTTACAAAATATGAATAAATTTTGCCACGCGGTGGTAATTT
ATATGAATTATAGTAATTCACAATGTTTGAAGCAATATTATCAGGAATATAATCAAAATCAATTAGAACT
AAATTTTCTTTATAACGATTATATTCAGATTCAGTGAGAAGCACCTTAGCTTGCTCACGGTCATTAGCAA
TAGCTTCAACGATTGAAGTTTTCATTGAAGGAGTTCGTTCACCTTCAACTCTGGTAAACCAAAAGTCAGA
TCGTACTTTAACTGAAGCAACGTTATCCTTTTTGTCGCCTTTAAGGATTTTAGTCATACAGTCAATTTCA
GCAGAACCGCTTTTAATTTTAACCCATTTCTTATGCATCGGAGACCATTGCTTAACATTTGGATATTTGT
GAAGCTGAGTAAAGTCACCATCTGACGAAATGATTAAAATCTTATGTCCTTCTAAAGAGAACTTTTTAAC
AAGAACAGCAATGTGGTCATCTGCTTCATACTTATCAATATCCATAACAATGTATGGCATATAAGCTTTC
AATTCATCTATAACTTTATGGCTGGATTCAAAATAACCTTCCCAGTCCCAAGTAGATTCTTCTCGTGCTT
TTCCACGGTTTTTCTTATAATAATAAGCGAAATCACGACGCCAATATCCAGATTTCGCGTTATCAATACA
CAGTACAATTTTAGTGTATCCAAGCGTTTTTGCTTTTTTGACATTAAACTTAATTGAGTTCAATATCAAA
TGACGAACCATTGATAAATTAATTTTTTCTTTATCTGGGAAGTTTACCAAAGCAGTTGAAAGCGCAATTT
GACTAAAGTCAATTAAGCAGATTCCTTCTTTGTAATCTTCATCCAACATCATTTCTAAATCCATATGAAC
CTCGTTCAATTAGTGAGATTTCTATTATATACCATCCAAATCTTAAAGTAAACAAGTATAAATACTTATT
ATTGAAAACACAATAGGAGCCCGGGAGAATGGCCGAGATTAAAAGAGAATTCAGAGCAGAAGATGGTCTG
GACGCAGGTGGTGATAAAATAATCAACGTAGCTTTAGCTGATCGTACCGTAGGAACTGACGGTGTTAACG
TTGATTACTTAATTCAAGAAAACACAGTTCAACAGTATGATCCAACTCGTGGATATTTAAAAGATTTTGT
AATCATTTATGATAACCGCTTTTGGGCTGCTATAAATGATATTCCAAAACCAGCAGGAGCTTTTAATAGC
GGACGCTGGAGAGCATTACGTACCGATGCTAACTGGATTACGGTTTCATCTGGTTCATATCAATTAAAAT
CTGGTGAAGCAATTTCGGTTAACACCGCAGCTGGAAATGACATCACGTTTACTTTACCATCTTCTCCAAT
TGATGGTGATACTATCGTTCTCCAAGATATTGGAGGAAAACCTGGAGTTAACCAAGTTTTAATTGTAGCT
CCAGTACAAAGTATTGTAAACTTTAGAGGTGAACAGGTACGTTCAGTACTAATGACTCATCCAAAGTCAC
AGCTAGTTTTAATTTTTAGTAATCGTCTGTGGCAAATGTATGTTGCTGATTATAGTAGAGAAGCTATAGT
TGTAACACCAGCGAATACTTATCAAGCGCAATCCAACGATTTTATCGTACGTAGATTTACTTCTGCTGCA
CCAATTAATGTCAAACTTCCAAGATTTGCTAATCATGGCGATATTATTAATTTCGTCGATTTAGATAAAC
TAAATCCGCTTTATCATACAATTGTTACTACATACGATGAAACGACTTCAGTACAAGAAGTTGGAACTCA
TTCCATTGAAGGCCGTACATCGATTGACGGTTTCTTGATGTTTGATGATAATGAGAAATTATGGAGACTG
TTTGACGGGGATAGTAAAGCGCGTTTACGTATCATAACGACTAATTCAAACATTCGTCCAAATGAAGAAG
TTATGGTATTTGGTGCGAATAACGGAACAACTCAAACAATTGAGCTTAAGCTTCCAACTAATATTTCTGT
TGGTGATACTGTTAAAATTTCCATGAATTACATGAGAAAAGGACAAACAGTTAAAATCAAAGCTGCTGAT
GAAGATAAAATTGCTTCTTCAGTTCAATTGCTGCAATTCCCAAAACGCTCAGAATATCCACCTGAAGCTG
 
AATGGGTTACAGTTCAAGAATTAGTTTTTAACGATGAAACTAATTATGTTCCAGTTTTGGAGCTTGCTTA
CATAGAAGATTCTGATGGAAAATATTGGGTTGTACAGCAAAACGTTCCAACTGTAGAAAGAGTAGATTCT
TTAAATGATTCTACTAGAGCAAGATTAGGCGTAATTGCTTTAGCTACACAAGCTCAAGCTAATGTCGATT
TAGAAAATTCTCCACAAAAAGAATTAGCAATTACTCCAGAAACGTTAGCTAATCGTACTGCTACAGAAAC
TCGCAGAGGTATTGCAAGAATAGCAACTACTGCTCAAGTGAATCAGAACACCACATTCTCTTTTGCTGAT
GATATTATCATCACTCCTAAAAAGCTGAATGAAAGAACTGCTACAGAAACTCGTAGAGGTGTCGCAGAAA
TTGCTACGCAGCAAGAAACTAATGCAGGAACCGATGATACTACAATCATCACTCCTAAAAAGCTTCAAGC
TCGTCAAGGTTCTGAATCATTATCTGGTATTGTAACCTTTGTATCTACTGCAGGTGCTACTCCAGCTTCT
AGCCGTGAATTAAATGGTACGAATGTTTATAATAAAAACACTGATAATTTAGTTGTTTCACCTAAAGCTT
TGGATCAGTATAAAGCTACTCCAACACAGCAAGGTGCAGTAATTTTAGCAGTTGAAAGTGAAGTAATTGC
TGGACAAAGTCAGCAAGGATGGGCAAATGCTGTTGTAACGCCAGAAACGTTACATAAAAAGACATCAACT
GATGGAAGAATTGGTTTAATTGAAATTGCTACGCAAAGTGAAGTTAATACAGGAACTGATTATACTCGTG
CAGTCACTCCTAAAACTTTAAATGACCGTAGAGCAACTGAAAGTTTAAGTGGTATAGCTGAAATTGCTAC
ACAAGTTGAATTCGACGCAGGCGTCGACGATACTCGTATCTCTACACCATTAAAAATTAAAACCAGATTT
AATAGTACTGATCGTACTTCTGTTGTTGCTCTATCTGGATTAGTTGAATCAGGAACTCTCTGGGACCATT
ATACACTTAATATTCTTGAAGCAAATGAGACACAACGTGGTACACTTCGTGTAGCTACGCAGGTCGAAGC
TGCTGCGGGAACATTAGATAATGTTTTAATAACTCCTAAAAAGCTTTTAGGTACTAAATCTACTGAAGCG
CAAGAGGGTGTTATTAAAGTTGCAACTCAGTCTGAAACTGTGACTGGAACGTCAGCAAATACTGCTGTAT
CTCCAAAAAATTTAAAATGGATTGCGCAGAGTGAACCTACTTGGGCAGCTACTACTGCAATAAGAGGTTT
TGTTAAAACTTCATCTGGTTCAATTACATTCGTTGGTAATGATACAGTCGGTTCTACCCAAGATTTAGAA
CTGTATGAGAAAAATAGCTATGCGGTATCACCATATGAATTAAACCGTGTATTAGCAAATTATTTGCCAC
TAAAAGCAAAAGCTGCTGATACAAATTTATTGGATGGTCTAGATTCATCTCAGTTCATTCGTAGGGATAT
TGCACAGACGGTTAATGGTTCACTAACCTTAACCCAACAAACGAATCTGAGTGCCCCTCTTGTATCATCT
AGTACTGGTGAATTTGGTGGTTCATTGGCCGCTAATAGAACATTTACCATCCGTAATACAGGAGCCCCGA
CTAGTATCGTTTTCGAAAAAGGTCCTGCATCCGGGGCAAATCCTGCACAGTCAATGAGTATTCGTGTATG
GGGTAACCAATTTGGCGGCGGTAGTGATACGACCCGTTCGACAGTGTTTGAAGTTGGCGATGACACATCT
CATCACTTTTATTCTCAACGTAATAAAGACGGTAATATAGCGTTTAACATTAATGGTACTGTAATGCCAA
TAAACATTAATGCTTCCGGTTTGATGAATGTGAATGGCACTGCAACATTCGGTCGTTCAGTTACAGCCAA
TGGTGAATTCATCAGCAAGTCTGCAAATGCTTTTAGAGCAATAAACGGTGATTACGGATTCTTTATTCGT
AATGATGCCTCTAATACCTATTTTTTGCTCACTGCAGCCGGTGATCAGACTGGTGGTTTTAATGGATTAC
GCCCATTATTAATTAATAATCAATCCGGTCAGATTACAATTGGTGAAGGCTTAATCATTGCCAAAGGTGT
TACTATAAATTCAGGCGGTTTAACTGTTAACTCGAGAATTCGTTCTCAGGGTACTAAAACATCTGATTTA
TATACCCGTGCGCCAACATCTGATACTGTAGGATTCTGGTCAATCGATATTAATGATTCAGCCACTTATA
ACCAGTTCCCGGGTTATTTTAAAATGGTTGAAAAAACTAATGAAGTGACTGGGCTTCCATACTTAGAACG
TGGCGAAGAAGTTAAATCTCCTGGTACACTGACTCAGTTTGGTAACACACTTGATTCGCTTTACCAAGAT
TGGATTACTTATCCAACGACGCCAGAAGCGCGTACCACTCGCTGGACACGTACATGGCAGAAAACCAAAA
ACTCTTGGTCAAGTTTTGTTCAGGTATTTGACGGAGGTAACCCTCCTCAACCATCTGATATCGGTGCTTT
ACCATCTGATAATGCTACAATGGGGAATCTTACTATTCGTGATTTCTTGCGAATTGGTAATGTTCGCATT
GTTCCTGACCCAGTGAATAAAACGGTTAAATTTGAATGGGTTGAATAAGAGGTATTATGGAAAAATTTAT
GGCCGAGTTTGGACAAGGATATGTCCAAACGCCATTTTTATCGGAAAGTAATTCAGTAAGATATAAAATA
AGTATAGCGGGTTCTTGCCCGCTTTCTACAGCAGGACCATCATATGTTAAATTTCAGGATAATCCTGTAG
 
GAAGTCAAACATTTAGCGCAGGCCTCCATTTAAGAGTTTTTGACCCTTCCACCGGAGCATTAGTTGATAG
TAAGTCATATGCCTTTTCGACTTCAAATGATACTACATCAGCTGCTTTTGTTAGTTTCATGAATTCTTTG
ACGAATAATCGAATTGTTGCTATATTAACTAGTGGAAAGGTTAATTTTCCTCCTGAAGTAGTATCTTGGT
TAAGAACCGCCGGAACGTCTGCCTTTCCATCTGATTCTATATTGTCAAGATTTGACGTATCATATGCTGC
TTTTTATACTTCTTCTAAAAGAGCTATCGCATTAGAGCATGTTAAACTGAGTAATAGAAAAAGCACAGAT
GATTATCAAACTATTTTAGATGTTGTATTTGACAGTTTAGAAGATGTAGGGGCTACCGGGTTTCCAAGAG
GAACGTATGAAAGTGTTGAGCAATTCATGTCGGCAGTTGGTGGAACTAATGACGAAATTGCGAGATTGCC
AACTTCAGCTGCTATAAGTAAATTATCTGATTATAATTTAATTCCTGGAGATGTTCTTTATCTTAAAGCT
CAGTTATATGCTGATGCTGATTTACTTGCTCTTGGAACTACAAATATATCTATCCGTTTTTATAATGCAT
CTAACGGATATATTTCTTCAACACAAGCTGAATTTACTGGGCAAGCTGGGTCATGGGAATTAAAGGAAGA
TTATGTAGTTGTTCCAGAAAACGCAGTAGGATTTACGATATACGCACAGAGAACTGCACAAGCTGGCCAA
GGTGGCATGAGAAATTTAAGCTTTTCTGAAGTATCAAGAAATGGCGGCATTTCGAAACCTGCTGAATTTG
GCGTCAATGGTATTCGTGTTAATTATATCTGCGAATCCGCTTCACCCCCGGATATAATGGTACTTCCTAC
GCAAGCATCGTCTAAAACTGGTAAAGTGTTTGGGCAAGAATTTAGAGAAGTTTAAATTGAGGGACCCTTC
GGGTTCCCTTTTTCTTTATAAATACTATTCAAATAAAGGGGCATACAATGGCTGATTTAAAAGTAGGTTC
AACAACTGGAGGCTCTGTCATTTGGCATCAAGGAAATTTTCCATTGAATCCAGCCGGTGACGATGTACTC
TATAAATCATTTAAAATATATTCAGAATATAACAAACCACAAGCTGCTGATAACGATTTCGTTTCTAAAG
CTAATGGTGGTACTTATGCATCAAAGGTAACATTTAACGCTGGCATTCAAGTCCCATATGCTCCAAACAT
CATGAGCCCATGCGGGATTTATGGGGGTAACGGTGATGGTGCTACTTTTGATAAAGCAAATATCGATATT
GTTTCATGGTATGGCGTAGGATTTAAATCGTCATTTGGTTCAACAGGCCGAACTGTTGTAATTAATACAC
GCAATGGTGATATTAACACAAAAGGTGTTGTGTCGGCAGCTGGTCAAGTAAGAAGTGGTGCGGCTGCTCC
TATAGCAGCGAATGACCTTACTAGAAAGGACTATGTTGATGGAGCAATAAATACTGTTACTGCAAATGCA
AACTCTAGGGTGCTACGGTCTGGTGACACCATGACAGGTAATTTAACAGCGCCAAACTTTTTCTCGCAGA
ATCCTGCATCTCAACCCTCACACGTTCCACGATTTGACCAAATCGTAATTAAGGATTCTGTTCAAGATTT
CGGCTATTATTAAGAGGACTTATGGCTACTTTAAAACAAATACAATTTAAAAGAAGCAAAATCGCAGGAA
CACGTCCTGCTGCTTCAGTATTAGCCGAAGGTGAATTGGCTATAAACTTAAAAGATAGAACAATTTTTAC
TAAAGATGATTCAGGAAATATCATCGATCTAGGTTTTGCTAAAGGCGGGCAAGTTGATGGCAACGTTACT
ATTAACGGACTTTTGAGATTAAATGGCGATTATGTACAAACAGGTGGAATGACTGTAAACGGACCCATTG
GTTCTACTGATGGCGTCACTGGAAAAATTTTCAGATCTACACAGGGTTCATTTTATGCAAGAGCAACAAA
CGATACTTCAAATGCCCATTTATGGTTTGAAAATGCCGATGGCACTGAACGTGGCGTTATATATGCTCGC
CCTCAAACTACAACTGACGGTGAAATACGCCTTAGGGTTAGACAAGGAACAGGAAGCACTGCCAACAGTG
AATTCTATTTCCGCTCTATAAATGGAGGCGAATTTCAGGCTAACCGTATTTTAGCATCAGATTCGTTAGT
AACAAAACGCATTGCGGTTGATACCGTTATTCATGATGCCAAAGCATTTGGACAATATGATTCTCACTCT
TTGGTTAATTATGTTTATCCTGGAACCGGTGAAACAAATGGTGTAAACTATCTTCGTAAAGTTCGCGCTA
AGTCCGGTGGTACAATTTATCATGAAATTGTTACTGCACAAACAGGCCTGGCTGATGAAGTTTCTTGGTG
GTCTGGTGATACACCAGTATTTAAACTATACGGTATTCGTGACGATGGCAGAATGATTATCCGTAATAGC
CTTGCATTAGGTACATTCACTACAAATTTCCCGTCTAGTGATTATGGCAACGTCGGTGTAATGGGCGATA
AGTATCTTGTTCTCGGCGACACTGTAACTGGCTTGTCATACAAAAAAACTGGTGTATTTGATCTAGTTGG
CGGTGGATATTCTGTTGCTTCTATTACTCCTGACAGTTTCCGTAGTACTCGTAAAGGTATATTTGGTCGT
TCTGAGGACCAAGGCGCAACTTGGATAATGCCTGGTACAAATGCTGCTCTCTTGTCTGTTCAAACACAAG
CTGATAATAACAATGCTGGAGACGGACAAACCCATATCGGGTACAATGCTGGCGGTAAAATGAACCACTA
 
TTTCCGTGGTACAGGTCAGATGAATATCAATACCCAACAAGGTATGGAAATTAACCCGGGTATTTTGAAA
TTGGTAACTGGCTCTAATAATGTACAATTTTACGCTGACGGAACTATTTCTTCCATTCAACCTATTAAAT
TAGATAACGAGATATTTTTAACTAAATCTAATAATACTGCGGGTCTTAAATTTGGAGCTCCTAGCCAAGT
TGATGGCACAAGGACTATCCAATGGAACGGTGGTACTCGCGAAGGACAGAATAAAAACTATGTGATTATT
AAAGCATGGGGTAACTCATTTAATGCCACTGGTGATAGATCTCGCGAAACGGTTTTCCAAGTATCAGATA
GTCAAGGATATTATTTTTATGCTCATCGTAAAGCTCCAACCGGCGACGAAACTATTGGACGTATTGAAGC
TCAATTTGCTGGGGATGTTTATGCTAAAGGTATTATTGCCAACGGAAATTTTAGAGTTGTTGGGTCAAGC
GCTTTAGCCGGCAATGTTACTATGTCTAACGGTTTGTTTGTCCAAGGTGGTTCTTCTATTACTGGACAAG
TTAAAATTGGCGGAACAGCAAACGCACTGAGAATTTGGAACGCTGAATATGGTGCTATTTTCCGTCGTTC
GGAAAGTAACTTTTATATTATTCCAACCAATCAAAATGAAGGAGAAAGTGGAGACATTCACAGCTCTTTG
AGACCTGTGAGAATAGGATTAAACGATGGCATGGTTGGGTTAGGAAGAGATTCTTTTATAGTAGATCAAA
ATAATGCTTTAACTACGATAAACAGTAACTCTCGCATTAATGCCAACTTTAGAATGCAATTGGGGCAGTC
GGCATACATTGATGCAGAATGTACTGATGCTGTTCGCCCGGCGGGTGCAGGTTCATTTGCTTCCCAGAAT
AATGAAGACGTCCGTGCGCCGTTCTATATGAATATTGATAGAACTGATGCTAGTGCATATGTTCCTATTT
TGAAACAACGTTATGTTCAAGGCAATGGCTGCTATTCATTAGGGACTTTAATTAATAATGGTAATTTCCG
AGTTCATTACCATGGCGGCGGAGATAACGGTTCTACAGGTCCACAGACTGCTGATTTTGGATGGGAATTT
ATTAAAAACGGTGATTTTATTTCACCTCGCGATTTAATAGCAGGCAAAGTCAGATTTGATAGAACTGGTA
ATATCACTGGTGGTTCTGGTAATTTTGCTAACTTAAACAGTACAATTGAATCACTTAAAACTGATATCAT
GTCGAGTTACCCAATTGGTGCTCCGATTCCTTGGCCGAGTGATTCAGTTCCTGCTGGATTTGCTTTGATG
GAAGGTCAGACCTTTGATAAGTCCGCATATCCAAAGTTAGCTGTTGCATATCCTAGCGGTGTTATTCCAG
ATATGCGCGGGCAAACTATCAAGGGTAAACCAAGTGGTCGTGCTGTTTTGAGCGCTGAGGCAGATGGTGT
TAAGGCTCATAGCCATAGTGCATCGGCTTCAAGTACTGACTTAGGTACTAAAACCACATCAAGCTTTGAC
TATGGTACGAAGGGAACTAACAGTACGGGTGGACACACTCACTCTGGTAGTGGTTCTACTAGCACAAATG
GTGAGCACAGCCACTACATCGAGGCATGGAATGGTACTGGTGTAGGTGGTAATAAGATGTCATCATATGC
CATATCATACAGGGCGGGTGGGAGTAACACTAATGCAGCAGGGAACCACAGTCACACTTTCTCTTTTGGG
ACTAGCAGTGCTGGCGACCATTCCCACTCTGTAGGTATTGGTGCTCATACCCACACGGTAGCAATTGGAT
CACATGGTCATACTATCACTGTAAATAGTACAGGTAATACAGAAAACACGGTTAAAAACATTGCTTTTAA
CTATATCGTTCGTTTAGCATAAGGAGAGGGGCTTCGGCCCTTCTAAATATGAAAATATATCATTATTATT
TTGACACTAAAGAATTTTACAAAGAAGAAAATTACAAACCGGTTAAAGGCCTCGGTCTTCCTGCTCATTC
AACAATTAAAAAACCTTTAGAACCTAAAGAAGGATACGCGGTTGTATTTGATGAACGTACTCAGGATTGG
ATTTATGAAGAAGACCATCGCGGAAAACGCGCATGGACTTTTAATAAAGAAGAAATTTTTATAAGTGACA
TTGGAAGCCCGGTTGGTATAACTTTCGATGAGCCCGGCGAATTTGATATATGGACTGATGACGGTTGGAA
AGAAGACGAAACATATAAGCGAGTTTTAATTCGTAATAGAAAAATTGAAGAATTATATAAAGAGTTCCAA
GTTTTAAATAATATGATTGAAGCTTCTGTCGCCAATAAAAAGGAAAAATTCTATTATAAAAACCTTAAGC
GGTTCTTTGCTCTTTTAGAAAAGCATGAGCATTTAGGTGGTGAATTCCCTTCATGGCCTGAAAAAGAACA
GAAGCCTTGGTATAAGCGTTTATTCAAGCATTACGTATAAATATCTTAAAAGGAGGGTCTATGGCAGCAC
CTAGAATATCATTTTCGCCCTCTGATATTCTATTTGGTGTTCTAGATCGCTTGTTCAAAGATAACGCTAC
CGGGAAGGTTCTTGCTTCCCGGGTAGCTGTCGTAATTCTTTTGTTTATAATGGCGATTGTTTGGTATAGG
GGAGATAGTTTCTTTGAGTACTATAAGCAATCAAAGTATGAAACATACAGTGAAATTATTGAAAAGGAAA
GAACTGCACGCTTTGAATCTGTCGCCCTGGAACAACTCCAGATAGTTCATATATCATCTGAGGCAGACTT
TAGTGCGGTGTATTCTTTCCGCCCTAAAAACTTAAACTATTTTGTTGATATTATAGCATACGAAGGAAAA
 
TTACCTTCAACAATAAGTGAAAAATCACTTGGAGGATATCCTGTTGATAAAACTATGGATGAATATACAG
TTCATTTAAATGGACGTCATTATTATTCCAACTCAAAATTTGCTTTTTTACCAACTAAAAAGCCTACTCC
CGAAATAAACTACATGTACAGTTGTCCATATTTTAATTTGGATAATATCTATGCTGGAACGATAACCATG
TACTGGTATAGAAATGATCATATAAGTAATGACCGCCTTGAATCAATATGTGCTCAGGCGGCCAGAATAT
TAGGAAGGGCTAAATAATTATTTGTTCGTATACATCTCTAGATATCGATATACACCCTCAAAACCCTCGT
TGAATTCGTCGATGAGGGTTTTCTTATCTTCTTGAGTTAATTCAGAAACAATTTTACGGAATGAATTTTG
ATTTAACTTTCTACCTTCATGCGTTACTCCAATCTCATTTAGAAATGCAATAAAATTAGCACGATTCTCA
ACAATATCTTCTCTGGAAAATTTAATCAAAATAGATGCAACAGTAATAATTTCACGAACTGTATCAATGT
TTTTATTCATTAACTATACCACTCAATTAGTTGACTTTGTTATAATATCATCAGACGCTTGATTTGTAAA
CTGGTCTGTGTAATTTTCTTCAAAAATTTTTTCTACGAATTCCTTGAACGATTCACGTTCCTGAGCTACA
TTATGCTCGATTACCTTTTCAAGATTATGACTCATTCGAAATAATCTTCAATTTCATAATCATGGACATA
AATCATTATAGTTTTTAATACATCATCAATATTTTTTCCTGGAGCTGGAATTACGTAAAAATACCCTGCT
TTTGAGAGGTCTTTATAAGTTCCAATCAAGAAATCATTATTCTCAAGATGTAACTCTTCAACTAATTCAT
TGACAATTGAATGGTATAGGTTTGGCAGAAACTTATATAGCTTTTCTAGAATATCAATTTTGAATGTATA
TTGAACCACGGACTGAGAATCAATAATCATAGACCTTCCCCTTATGTTTCTGTTTGCGATTAGATTCTTT
AAACGCTTTCTTCTTATCCTTATGAACAGAAGCTTTATTAAAATTATGCTTTGCGACTAAATTGTTCATA
GTGCTGAATTACCTCTCTTAAACATTTGCATGTGAATGAAAACTTTTTAGCTACACCACATTCAAATATA
TGTTCTCTTAAATCGCGTGTATCGGTATATCCCATCTCAACAATAAAATGCCGTATTAGATTTTTATCTT
TATCGTTTAGAGAATTAAAATAATCAGATTTTGAATTAATTTCCCTGGCCAAATTGAATCACCTTCAGTT
GACGTTTTAACTCTTTTATCATCTCTTCGTTCATCGCAATATAAAGATCGCGTAGAGCAGGTTTTAGCAT
TCCATTTACTGGAGAACTAAATGGACATACATAATCTTTTCCTACGAGCTTTTTAGTGAATTCCATATCA
CAGAACTGAAATCCCGGCTCATTGGTATAAATTCCCCAATTAGTTGACATCATTTTATTGGCATATTCCA
GTGCCTGGATTTGATTCATAATTCCATCAATTTGAAACTTTTTAATATTCATTAGTAAAGGTCCTCAGAG
TAAAGTTCTTTTTCACTACCACGTTCAATACTTACTTGTCCAGCGTAAGTTGCAATAATCATTGCTTCTT
CACGTGTCCAATAATTACTATATTGGTCAATAAACCCTTGGTCATCATCACAAACTTGCTGAGTAACTAA
TTGAGGTTTAACTACATCTAAAACTTCTGCCATATCTTTAGAATAATGACGAGCACCTGGAATAATAAGA
GTTCGTCCATCTTTTAATTTAAAACGGTTGGCTGCGCAAACAATTCGTCGTTGATACTTTTGGTTTTCAT
CCCAGTACGCAGTCTGCCAACAGATTTCAGGAACTTCTTTCAGAATATCTTCTTCTGTGCATTTATAACC
ATGCGCTTTAAATTTTTCAACAAGACTTTCTGGAGTTTCACGAGATAAAGGAACATTCAGCATTTTTAAA
CGATTGATAAATGGGTTCATTTAAACCATCCTTTAATACGCTGCCACAAAGTTTTCTGTTGAGCTTTGTT
GACGCCAATTGAGCGAATAACCGGTTGAGATTCCTGGAATTCTTTATAATCAGCAAGGTAAATTTCGTAA
GCTGCATCCGTAAATGAACTTATCGCTGCCATAAAATTATTGCGAATACCTACTGGAGCATCTTTACTTT
CACGAATGATCATGTATTTACCAGTCTTAATCTTTACGATAGTTCCAAGATAAGCTCCATGGTACCAAAT
ATCCCAACCCTCTTGAGTAGGTTCTGCGCAACGACGAAGTTCATTGACAATTTCTAACTTGTTTATTATT
TATTCCTCACAGTTCAGATGCTACAGTGATTACAGCTTCAATGTTTTCTGCCGAGCGTTTAATGTCAAGA
TACACATTACCGTTTTTAGCGATTTTACATGACATTCCGATGTCAGTAAATTTCTGAATATGATGTTCCA
TCATTTTGTATCCAAAAATTCGCATATTTCCATTGTTATTAATTTCAAAATTACGAATTCCGTTAGTGCG
TTTTTCTAAAATAGCAAGATAATTACTACGATAAATTTCAACCTTTTTAAGAACAAATCCATTTTTATCT
AAAAGTTTTAACATGAGGTCTTTATCTTCTTCCATATCGGAAGTAATCTCGCGAGCTTTACGAGTTGCTC
GTTTTTTCAGCAGTTCCGGAGCATTTTCCTGTGCATATAAAGTTGCTGCATTTGAAATAATATCCTGAGC
TTCACCAGTAATGATTAATCCATCACCAGATTTCTCCACCAGGCCTTTTTTAATCAATACCCCAATATTA
 
CTATTAACTACTGCGTTACCTAAATCTGGATGCACCTCACGAACTTCTGCAGCTGTAATGAAATCTTTCT
TAGCAATGGTAATTAAAATCGTAGCAGTTTTTTCATTCAGAACATCGTTAGAAGCTTTGATGATGTAAGT
TACTTTAGACATTTTCTAATCTCCGTAATTCTGTATCAGTAGTTGATAGTTGTATAGTACCACAGTATGC
TTTGGTTGTAAACCGTTTTGTGAAAAAATTTTTAAAATAAAAAAGGGAGAGCCTCGGCTCTCCCTAAAAT
TACTGCATGACTGTGATAACTGTCATGATAACACGTTGAATTCCGAACGCAAGAAGACCTCCTGCTACGG
CTGGAACAACGCCTAAACCCGCCAGTAAAATGCTACCAGATACTAATGCAGCGCTTGTAATACCAATGAA
TGGACTCATTTGATTTCCTCTAAATCTTTGGTGTATTCAGTAACTACATCAGTAGTTTTCCAATATTCGT
TTTCTTCTTTTTTAGCTTTAGCTTCTTCAGCAAGTTTCTTTGCTTCATCGGAAGTCATATGAAAAATATT
CATTCCAACTAGTTTATCAACATAAGAAGAATACATATCGATTTTCGAAAGTTCTTCGGTCAGTTCTTTA
CGAGTTTTACCCTGTACAACAATTTCACCTGAAATTACTTTCTTAATGAAATGTGCTTTGGCAAAGGCTA
AACGAAAAGCTGACTCAGTTTCTTTAATTTTGTTATCAATTCGTTTTTGGACATAAGTTTTACGAACTTC
AACAAAGTCTTTAATTAAATCAACTACGTTATCGTAAACTTGCAGCTTTCCTTTCTCATTAATAACCGTA
ATATTCTGGGAACGACGCTCAATCAGTCCGAAGTCTTTCATAATTTTTGCATGGCGTTCTTCTTCGTTAT
CGCTCAAAGAATATTCTTTGCGGAATTTAACTTTGAAGCCAAAACCATGCTCACCACAAGCATCATCCCA
TGTAATGAAGCCTTTATTTTCAAGTGGGTCTAAGATTTTACTCACATAAGTTTCACGATCATACTTATAC
GGAATCTCAGTGATATGCATTTGAGTTCGTGAAGTAAACTTATATGTTCCACGAATTTCATATTGCCCAT
CAATTTCAACGACTTCACCACGAAATTCTGGGAATTCTACTTTCGGTTTAGTTACTTTCTTTCCTTGAAG
AGCTTGCAGTACAGCTTTCTTGACAGAAGAAACACTATGAGGAAGAATGTAAGTTGCATAACCAGTTGCA
ATACCGGAAACGCCATTAAGAAGAACAGTAGGAATAATAGGCAAATAGAAAGCAGGCGGAATGTGTTCTT
TATCTTGATGTACCGGAGCATATTCAGTATCTTTATATACGTTATAGAAATTTTTACTTACACGAGCAAA
AATATAACGACTTGCCGCTGCCTTTTGGACAGTACGAGAACCAAAGTTTCCTTGACCATCTAACAGAGGA
AAGTTATTATTCCAAGTATTAGCCATCAAAGCACCTGCGTCTTGTGCAGAGTTTTCACCATGATGATATC
CAAGGTCCGCTACACCACCTGCAATAGAAGCGAGTTTGTGAAACTTATCTTTATTTCCTCGTGCCAAATC
AAGAGCTCGAGCAATAACAAATCGTTGAACTGGCTTAAATCCATCAATCATATTTGGGATAGCACGATTT
TCAACCGTGTACATAGCATAAGCCAATGCTTCATTATCAATGATACTTTTTAAATCGCGATTATTCAGTT
GCATAAATTTACCATACTAGTGAATGTAGTGCCATAATAACATCAGAAATGAAAAGCACGACTTGAATTA
ATCCGAACATTACTCCGTAATATAGTGCTACCAATAAAGCAGCAAGGGCTAATGAATAGCCCAAGATTTT
CTTAATCATTAGATAACAACACAAATGTTAAATATGCACACATACCCTGGGCTAAAGCTTGTGAAAACAC
ACTGCTAGCATCGATACAGATAGTTAAAACACATGCTACTATCCAACAAATAAATGAAATAACTCCTAAT
AATTTTGCAATATTCATATTTTCCTCACTGGCGTCCGAAGACGCCTTTAGTTTTAAGATTGTTACGATAG
AACTGCATCACGTGTTCGTTATGGAAATTACTCATTAATATGCCTGTAAAACAAATTTAAAGTTATCAGC
CAACATACGGTTCATTTCTTCGAGTGTTTGATACTCAGAATGATGATTACGAGTAAACGCCAAAGCTAGC
TGACCTTTTCCAAATCCCGTCGTTAGAGGTTTCATCTTAGAAGCAGGCAGATAAAACACTGTGTATGGAA
CATTGTTATTTGCAATAGTACGCGCAAGTTGAGACCGGCGTTGACGAATATGACTTAAAACCGCACTGAA
TCCTTGCTTAGAACGCTGATTACCTACATAAAATCGTGCAGATACGCATGGATTACTAAATGGACCACCG
AGTTTACTCACTAAAAAGTAAAATCCAGGTTTAGATAAAATATCTTTATGCGGAGTTCCTAAAAACCATT
CACCACCCTTGATTGTACCAATAACAGTAGCGCCTGCATCATTCAGATCAGTAACAGTCATATATTTCAT
ATTAATTTCCTCTAAATTATTTTCTACTCCAAGGCCGCATGAATACACACGGCCATTAAATTACTCGTCG
CAGTCGACGCTCAATTCCCAAAACTCTTCTACAGTATAAGTTTCAGTATCATTTTCAATACAGAAACGTT
CATTACTATTATTTGCTAAAGTAGCATTAACTGTCATTTTTTCGCTAGTGCTCTTAAGAGGTGAAATACG
AATTAACTGATCACCGTTATCTAAACAAAAAATTTCACCAACTTTTACATCTTTAAAACTTTTCATAATT
 
CACCTCAAGGAGTATAAAATCCAAATGCAGTTGTTGACCATCCCATCCAATATGGAAAATTTACACCAAT
GTAAAACATAAGAATATAAAACCAACCGCTCAGCAAATTCATCATTTTACACCATTCCAAATTGTTTCAA
CCACGGATTTTAAACCATTTTGATGAATATCCATTCCTACTACCGCCATCAAATAAATTCCAACTACAAC
TGAACCTAAGGCAAAAATCAGCATGAAAATGAATAAAGCCGGAAAAATATTATCGAAAAACCATTCAATA
AATGTAAAAGCACTGCGTTTACGTTCATATTTTCCTCACATAAATCCAAAGTAAACGTTTAATACATCAA
TCATTAAAACGATTGGGAATATACTCAAAACTATTAGTATTATAACTACATTCCATATAGCTTTAATAAT
CTTTTTCATTTTCTGTTCCTCCATAGTTGATAGGGTAATAGTACCACGGAAGAACAGTCTTGTAAACAAC
TTTTTTAAAAATATTCGTAATAAATGTGAATACCAACTACTACCGCTGAAACCTGTGCAACCCACCACGC
ACAAGCAATAAGTACAGAATTCAAAATTTTCATAATAACCTCATTACAAAAGTAAATGTTAAACAAATTA
CTGGAATACTAATTAACCAAACAAAACACCACCATAATGAACTCATAGTTCAATCTCAGCGATTTTCATT
TTATTCTCCAAATCCGTATCAGTAGTTGATAGTTGTATAGTACCACGGTCCTTGTGGTATGTAAACTGTT
TTGTGAAATTTTTTAAATGGAAAGATACCATCCGTTGTAGTTGCTTTTTCTTACAACTTTACGAAGGTCT
TCTCTGTCACCGATGAACTTCGGAGTGTACTGGATGACACCTGGATGAATTTCTTTAGTGTTGAATATAA
TTATACAGTCAGCGACTTGATGATTTAGAATGGGCCCTAGATTTATTCCAGAACCATATGGATACTCTCC
GCTGCATCCCGTTGTTACCGAAATCCAACGTGAGTCAGTTTGATGTGTCTTAACTTCTACACGAAGCCCG
CAGTATTTTGGATGAGCCAATACATCCCATGCATATGTGTACGGATCATCGACATCCTCTTGGCCTTTAT
TGACATATCCACTCAACCAATCTGCCACAAAAAACTCTGCGTACACAGCGATACGGCATCTTTCGATAAC
TTCTGCCTTATCTTGATTTGGGTTTTGTTTTAAAGAGTATCTTGCAGTATCAGCAATTTTGACCTTCATT
TCACAGGTCAAGTCACTGTTCGATAGGGTAAATGTCGGAATCTGAAATAGTCTCTGTAACCCAGGATTCG
TTTTCTGCATTTAAACTTTCCTTTATGTCGGAATCACCGATATTCATATAAATCATAATTTCTCTTAAAA
CAAAAGGCCGAAGCCCTTTATTTTACTTGAATTGTGCAATTCTTTTCTCTAGACATTCAGCATAAGATTT
CATTGAGATGAACTGCGAAAGTAGCAGTTCTTGCTCAACTGCGCTAACTGTTAGAAACTTTGCGCTTTCT
AAAAATTTGCTCAGTGCATTAATTTTGAGCATTAATTGATCGTATTCTTCTTTTACTCGTGCTTGATAAG
CTAACATAATTTTCCTTAGTTAAGGGCCGAAGCCTTATTTAAATTGTTCAGTAACGTCTTCAACTACTTC
ATATTGGCAGGTACGCATTTTAGCATCGTTGTAATCAATCGGAATTGATACTACATCGCGAGGATGAACT
TTAACTTTTACAACTCGGCTGGTTGAACTACCAAAGTGACGAATATAAGATTTAGAACACACATGCAAAC
CACGAGAACAAGTTTGTGTATCATCGTCATTCACACGAGTACGTGGCATTTTAACTACTTTACCCGGACT
GTTATCAAAGGTGTTTGAGTGACAGTCAAAGTAATTGCTGCGAACTACTTTCCAAGCATAGAAGTAACCA
TCTTCTGTAATTTCAATATCGTTTGCTACCAAGAAATCAAAGAGTCGAGATACCGCTTTTTGGCTTGGGT
TTTCCAACAGATTTTCCAAGAACGGAAAATAAAATTCAAAGTTTTCGCCTTTTTCCATCGAGTCAAGAAT
ACGATCAACCAAACCAGACCGCAATTCAATATTTTGATAGAACAAGCTTCCACCTTCAATTCGAACATCG
CCGGAAATATATTTTTCAACAGCACGACGAACATTAATTTTTTGTGCCGCTTCTTCCAACTTATCCGCTA
CAAGCAGATTAAGAATTTCCTGGAAGTTTGAATGAGTATTAGGAGTTGCGTTATAAGTTACGCCATCAAC
AGTAATTGAAATGAATTTTTTAGATGCATTCCAAATAATGTCAGATTTAGCAACTGGAGCAATAACTGCA
TCGCTATTAACTTTAACTGTAATATCACCGCTAATAGTAACTTTAGGGCGTTTAGCTTCTTCAGCATTTT
TCAAAACACGACGGATTGTGTCAACCGATACACCTTGCCAATCAGCCAATTCCTGTTGGGTGTAATTACC
ACTTGAATACAGTTTAACAATTTCAGCTTGTTCGTTTTTGGTCAGGCATTTAATATTGTACAT
**Spacer targeting *E coli* fimH (SEQ ID NO: 6)**
   CGAATGACCAGGCATTTACCGACCAGCCCATC
**Spacer targeting *E coli* bolA (SEQ ID NO: 7)**
   AGTGGGAAGGGTTGCAGGACACCGTCTTTGCC
**Spacer targeting *E coli* rpoH (SEQ ID NO: 8)**
   CCGATGTTACCTTCCTGAATCAAATCCGCCTG
**Spacer targeting *E coli* IptA (SEQ ID NO: 9)**
   TGATTGACGGCTACGGTAAACCGGCAACGTTC
**Spacer targeting *E coli* murA (SEQ ID NO: 10)**
   GCTGTTAACGTACGTACCGCGCCGCATCCGGC
**Escherichia phage T2 DNA, complete sequence (NCBI Reference Sequence: NC_054931.1) (SEQ ID NO: 11)** 
AATTTTCCTTATTAGGCCGCAAGGGCCTTCATAGTTTTAGCGATTTGAGAAACTTCATCATCACTTAAAG
AGTTGCGATATCCGATGAAGTCGGAAACAATACGGAATTTCTTGGTAAACTCAGCAACCATTTTATCACT
GTTTTTTGAAGCATTATTTGATAATACATCAAAAAGATTAGTTACTGTCCAGATGTCATGACCAATGGTA
TCTTTTCCACCATTAAAATATACACCCTGTAATGAACTAACCATATTAGCGAGTCGTGTATATTCTTCAG
AAACTTCATCTACACTGAAGTACTTCATCATAAAATCTAGCTCAGGATACTTGATAATTTTATCAATATA
TCGTTTAGCTGAACTTGAATAACCTACATACTTATCATAATCTACATCATCAAAAGCATCTACATATAAA
TCGCGTAGAGTTTCAAAAATACATTGGCACTGACCGAGTTCTTTTACCTTTTTCTGTAAAAGCGGACGAA
TAACATAAAATTCATTAATGCCAATAAGATTAGCCATACGAATCAAAATATTCATAGATGGATTACAAAG
AGATGTAGTACCATCCATAGAGAAAATATCAGAACGATGCATATACGCTACATAACCAGTAATTTCATCT
GCTTCTGACGTAAGTGTAAATAGTTCCTCTTTTTCCCAGCGCCCGTCTTTAATTTCAAACTTAAATGCTG
TAGCAGCTTTAGGACGAGGAGCTTTACTTTTAACTACCTTTGGAATATAGCTTTTAACTAAAGCTTCGAT
TTCTGACAAATAATGAATGTTAACTTCATCACTTTCAAACATTGCCATAATATCAGGAAGCAAATCAATC
TGCGATTCTACTTCTGGGTTAATAAACAGAAGACGCTCATTGTGATGAATATTCAAAGTGTTATTAAATT
CACTATCATCTAACGCACGTGCTAATCCACGGACAATATTAACACGATTTTTAATATTATCAATAACAAT
ATTAATTTTTGTTGTATTAATACCAAACAGACGATAACTTGATGCAACGGCTGAAGTTTCATGACTTTGC
TTAATGCGCTTCAGTCGAGGGTCAAGATTTACTTCATACACAACTCCTGCATTGCATAACTTACTATCAG
GTTCAAACATACTCTGCATCTTCTTATATGACAGATTTTTAGTCGTGAATTTGACTGAATTACTAATCAT
ATAATCTCGAGCAGAATACCCCATCTTCATCAATTCACGATATGTGTGACGAGGAGATGTAGATTCTTTA
AATCGTTTTACATCTTCATTAAATGCTTGCTCACTGAGTTCTTTAACTCGCTCAATAATATTTTTACGAG
TACGATCATCAAGTGAAAGAGCCTCGCGAGATGGAGCAATATCAAGTGAACCCATTGGAAACTTAATGTA
ATTCACTTCATTGCGAATGCTTAACCAGTTGCGGTCTCTAATAACACCATCAATAGGATAAACAATACCG
CCATAGATAGCATATAATCCACCACGATCAGGCCAGTATCTTTCTGGATTTACGCCGTAATAATCATCAA
AATCCGGAAAATAGTCAATTTCACGGTCAAGACTATTAATGATAGCCAAATCTTTGAACGGCCGCATGAT
ATAAGAAACTTCATAAGCAAAGTTTCTAAAGTCTTTTTCTTCAACTGGAACTACGATTTCAATGCCAGTT
TTATCATCTGGACCCATTTCTTTTACGAATGTAGGTTTAATCTGTGGGCCATCACCATCCATGTAAGCTA
CATAACCACGAATTTCACCTTTATGGTATGAAGTAATACTAAACGTATCAGTATAACTAAACGGAGATTT
 
AGAACCTAAACCAAATCCACCAATGAAATCATTAGATTCAGCTTTAGATGAACTGAAGTATGAATTATAC
AGCCCAGGAGAATTATCATCACCCTGAATATCAAAATCACTCATACCCGGACCAAAATCTCGACAAACAA
ATCGCGGATCTAATCGTCCTGGAACTTGAATGATAAATTTTTCAGGATTTCCATTGAGAGCATGGGCATC
AATCATGTTAGTAATCAATTCACGGACTACTGCGCGAATTTTGTTTGTATACAAATCAGATGACAGAATT
TTAAATACTTTAGGAGATGCTGTGATGCTAAATGCTTTTGATTTAGAACCATTACCAAGAATTGTTTCTT
TTTCAGTGGTGATAATCATAATTTCCTCATTAATTCATATTACGCTTAATAACTTCAGCAACTTCTAGTA
ATTCATCTTTAGTTGCGGTGTCGGATTGAATTTTATCTCTAATATCCTTAAAGCGGTTTTTAAATTCTTC
GGCTTCTCCCATATCGAAAAAGCGTTGAATGATTCTATATTCTCGATGAACTGCTTTATCAAAAAGTTCT
AAATTTACTCTATATGATTTCATTTCAATATCCTCATTTGCCCAATTAATTATACCACATCCTTGTGGTA
AAGTAAACTACTGGCTCATCCATTCTTTACGAAGGTCAGCATTATCTCCCATGAGCATTTCAAAAAGCTC
TTTCCAGTTCTCAGGAAGTTTAACAACATCATATACTGGATTTTGAATCATTTCACGATATTCAGATTTT
TCCAAAGAGCCAAGTCCTTTAATATAACGGATGCTATGTTTAGGTAGAGCATCTTTGGCACTCTCATATT
CAGCGACTGTATAAAACCATTCTTGTTTTTTACCGACCTGAGCGATGATTACAGGAGTTTTGACAAAGCG
AATTCGTCCTTGCTCAAACAATTCTGGCCAATTACTAAAAAATCCGAGCAGAGAAGGATAAATGCTTCCT
AGACCATCATGGTCAGCATCAGTCATAATAGCAATATTATGATAATTCAAGTTTTCAGCTTTTTCACCAA
GAACTAGACCAGTGATTGCGCAAATATCAAATAGTTCTTTGTTTTTAAGCATATCGGCATATGACATACC
CCAGCTATTAAGAACTTTACCACGCAATGGATAACCACCATGAAGTTCTTTATCACGAACATCAATAAGA
TATCCGATAGCAGAATCACCCTCAGTCAAGAAAAGAGTAGTATCAGCATCTTTACCACAAAGATTCGCTT
TGATATGTTTATGAACCTTAGCTTTAGAAGCTTTTTTAGCTGCCTTTGTCTCTGCTGCTTTTTCCGCCGC
CAATTTACGAGCTAATGCTGCTTCAATAATTGGCATTAAAATTGCTTCATTATTTAGAATAGCGCGTGAA
ATCTTTTTAGCATCAAGTTGAATATGACTACGAATTTCACCAAAAGGAGAAGTAAGTCGTTCTTTAGTTT
GAGAGTCAAATCGCATGTTTTTCATATCGCGAACAAACATAACGATAGTCAAACATTCTTTAACACGTGC
TTTAGTTACATCAATTTTGAATTTACGTTTGATTTGTGGAATAAGGTCTTCACAAATATCATCCATAACA
CAGTCAATATGATGGCCACCATTCTTAGTATGAATGTTATTGACGTACGTCAACTGACGAAAACCATCCG
GTGAACGACCAACCGCAATAGAACAATTTTCTTGTTCTTGAACAATAGCATGTTCATCATACTGTCGTGC
ATATTTCTTAAAATTGCCCTGAACCTTTTTACCATTAAAGGTAAATTGAATATCAGGATAAACTACAGCA
AGCGTCTGGAGACGATCAAGTGTAATGTCAAGATAAACTTGGGACAGCTCATTAGTTTCAAATGACATAA
AATCAGGAATGAAAGTAACACGAGTTCCTTTCCATTTTCCAGGAATATCTTCCCATGATTTATTTTCCAT
GCCATTTGAACAACGAACTACAATATTATTTTGACCATCGCCAGTTTCACCGACAAACATCACAGAAAAA
ATGTTTGTCAAACTAGAACCAACACCATTCATACCACCGGTGACGCGTTCTTTATCATCACCAAAGTTAC
CACCTGCTTTTGGAATAGTCCATGCAGCAACTGGACCAGGAATTTCTTCACCAGTAGGTGTTTTAACCAT
CGCTTGTGGAATACCACGACCGTTATCTTCAACTGTTACTTGATTGTTTTTAATAGTAACATTAATTTTA
TTTGCTAATTTAAACTTAGTACGAATACCTTCATCTACTGAGTTATCGATAATTTCATCAATAAGCTTAA
CAAGACCAGGTACATACTGAACACTTTCCCATTTACCAAACAGAAAGCGCTCATGCATTTCATTAGCAGA
AGAGCCAATATACATGCCACTACGCTTTTTGATGTGTTCAATATCGCTCAGAATTTTAATTTCATTCTTA
ATCATCACTTATCCTCGTTTGGTTTCGGGAATATTATACTCCAATAATCATAAAGCTAAAGGCCCGAAGG
CCTTTTATTTAAAACGGATAGTCGAATCTTTGAAGAATAAACCAGAACATACTGTTCCTTCTACTTTCTG
CCCGGTAGGTCCAATAGCACGAAATCCAGTATGCTGGAAATCATTTTCAGAGCAACCAAACCAATTATAT
CCAGTGATTTCAATATTAGTAAAACCACTTGAAGACAAAACTTTTGTTGCATTATCAGCATCAGTACATC
CTACTAAAGACACTGCTAATACTAATGCTGCGATAGAACGATTAATATATTTCATAATTTTCACTTAAAT
TTAATGGCTTGAAGAAGACTAATAATTCTCAAGCGACTTCTTTCATCTAACTGTAAAAGAAAGAGGGTCA
CCAGATTTCATAGTAATAGTACATTCAAAATCAAAATCTTCGGAAACTTCTTTGAAGAAGTCAAATTCTT
CATCATAAATTAGAACATTACTCTGACAACTATGAAGAATTTTTCCATCATTTCCAGATGCTGTACTAAT
CATTGTAACATTATGACCTTTCATATCTTCAACGATAAATTCACTAGTCAATATTACAGCATTAATAGAA
 
CTATTCCTATTACTAGCAGAAAGTAGATATTTCTGTTCTTCACCTTCACGAATGCAATATTTCTTACCGA
TTTTAAACATAATTACCCTTTAAGTAAGTCGTAAAAACCACCATTCACATGCTTAGGAGCAGAAGGCCGA
CGAGTAGACAAACGATGACATTTAGGGCAAACATCATTTTCTCGTTCAGAAATCTTTTTAATTTTTTCAT
ATTCATGCCCGCAATCTTCGGATTGGCATTTATAATCATACATTGGCATAATTATTCCTTAAAGTGTGCT
TTCAGCATTTGATACAAGGACCATGCCTGTTCATTATTTTCAATAGTAACTTTCATTACTGGGAATTCTG
TGAAATCTTCTATTTGTTCTTGCTCTTTCTCTTCCTGCTCTTGCTCTTCAACTGCCTGATATGGATTTTC
CACTTCATCAAAGAACCCAGCTTCGTTAGTAGAAAGCCAGATAAAGTTTTCGTCAAGGATATCACCGTGT
GTTGCATATCCAACTGTGCCAGTAGATGTCACAATTTTAGTAGGACGCCAAAGATAATCAACATCTAAAA
TTTTAAAAGGATGCATACCTAAACGGCGTGCATAGATTCCGTTATCAGTATGGTCTTTAATAAAATTTTC
TTGAGCTTGTTTATTTTTAAATTGATACCATTTATTAACTTCAAATTTAATAGCCATTAATAAATTTCCT
TCCAGTAAGTTGTGCCATCTTCAGTAATTTCACGAAATACACCGTAAATTGGTTGTTTATCACCGACTTT
CTCATACACATAAACCGAAGTCAAGTGAGTAAACTTGATGGTGTGTTCCTTTTGAACTACTACCAAATCT
GGATCGAATAATACATCTTCAAATTCATCATTAGTGCAGTTCTGAACAATTTTACGTTTCATTATAATTT
CCTCATTAATTGAACAGTGGAGCGATACGTTTCAGAAGAGTAGCAGCACCTTTAGCGAATTTTACATCAT
GAAAAACAGTTTCTTCTTCTGTAACAATTCGAACTTTAATATTTTCACAATCCCAATTTTTGCGAACAAT
CTTCAAAAGCTGCAGTAAATCTTTACATTCAGCAATATCGATAACACATCCACGAGAATCTTCCATTATC
GCACGATAACCATAAGCCATACCATAATCGTTGAAAGATTGCTCGATGATAATTAAGTTTTCCATTTTAT
TCTCCAAGTTGTTTTCTGTATCAGTAGTTGATATTGATATAGTACCATAATCAACTACTGATGTATATAG
TTTTATGAAAAAATTTTTAAACTTTATGCATAGCGAGCTTTGCTATAGTGTTTAATCCAGCTTTCAGGAA
TGACTTTGTATGTTCCTAAAAATACCGCGTTATATAACTTAACGCCATCTTCTACCCATTGATCAGTAAT
GTATGCACACATAGCGCGAGTACGGTTAACAACCCCTTCCTCACCTTCAGTAAATTCAAACTCATAAGGA
GCAACGAACTTAATGGCCTGACCGAGTTTCCACTTAAAGTCTACACCTACATGCGAAGTATCAATCGTTT
CAATTCCTTTAGCGGGAACAGCTTTCAAAAACGCAGGCTCAAGAAATTTCCATCGAACATAACCAAACTG
AGGTTTAGACATTCCATCTTTAGGAATGATACGCACTTTTACTTCAGAATCTTCATCTTTAACACCATGT
TTAAGCTGAATGCTTACAACTTCAACCAATTTTCCTGCTGCTTTAGAACGGGATTTATCAGATACACGAG
CAATTTCACCGATATTAATATTCATAATTATCTCTCACTTGTTAAAAAGATTTTATACTCCGCGGGACCA
TTATACTCTGGTCCCAAGAGTTTGTAAACTTAATTTAATTCACTAAACGATGAATTCAACACGATAAATT
TTCATTTTATTATCCTCAGTAGCTATGGTGTTATAATACCACAACTAACCGAGGAAGTAAACAACTTTTT
ATCGTTTTGTTGGAAGAGATAGAGGATCGCATTCTTCCTCTGATGGAGCATCTTCAAGACCCATAGCATA
TCGCAAAGCATACTTCATCATCAGGATGTCTTTCGCACAGTCATGAATAGAATCATGCGCAACGAATCCA
TCTAAAGTTCCTTTTGGAAGAGGACACGTGGTCATATCACGAACAAGCAGAAGTGCTTCAATTCTGGTAC
GAATATCACGCTGATTCCAGAATTTACATGGTTCTAACTTAAATGTGTCAAGCTCATTCTCAGATACACC
ATTAAGGCGTTGAATATCACGAATGAGATCGACTAAAATTGGAAAATCAAATGACATTCCACGACACCAG
CCTTGAGATTTCCAAGGATCGATATTATGTGCATTGATGTAATCATTAAATTTTGCAATACCATCGATAG
TGCTTACATCTTCATCTGATGGTGCAATATTTTTTCGAGCTTCAGGAGATTGATTTTTCCACCATTCGAT
AGTGCTTTTAGTAAAAAGACGATGTCCTTTTTGGCTTTTTAAATCAAATTTGATTTTAATGCCACGTGAA
ACTAATTCATCGAATGTTTCAACGACTTCTGGATTAGGGTCAAAAGCAATTACAGCCAAATCAATAACAG
CTGCTTTTTCACCACTTCCCATTGTTTCAAAATCTATAATAAAATCAAACATTAAACTTTCCTCGCTAAA
TCGCGAATTTGACCTACAGTATAGTCTTGAATATAAACTTTATTAATAGGCTCATCAATAAATTTTGCCA
TAGATTCAATATCTTTTTGTATTTCTTCAAGACTGTATACTATCTTTAAAGCTTTTTCGCGAATAGTAAT
ATTTTCAGGACCCTGATTTTCTTCAATGACAGCTTTAACATTTGTCATAAGAGATTTAAACTGGCACCAA
CTTAATTCAATCATTAATAATCGCCTCATAAAGATAGCTAATTTCGCCTAAAACATAATCATTAATTGTA
ACAGTTTTCACTTCGCCACAAAAGAATTCTAACGCAATTAAATCTCGTTCAATTTCTTCTAATTGAAGCA
TCAACTTACTAGATTCAATTTTTACAGTTTCACGATTTTTACCATGAGCAATTTCATAAATTTCGCTTAC
 
TTTATCTTGAAGAAGATAAAACTGATCTTTAGTTATTTCCACGAATAGCTTCCTCAAATTTAATCATACA
TAAAATACATCATAACGACCACGGGTAACACCAACATAAAGAAGTTGTTGAGCCAATTCAGCATCTGCAT
AATGAATACAAGGTGTATAAATGAAAGCACGGTCTACAGACATACCTTGCGCTTTATGGAATGTTGATGC
AGGAAGTGCTTTCACTTTACTGAACTGTGATTTAGCATCCCAAAAATCACTCCATGGAGCTTTTCCACCT
TTGTTCCAATTTTTATAAGTTTCTGCTGTTTTACCTAAAAATAGGTTAAACTTATATAGTTCTTCATCAG
ATGAAATTATTTTAATCTTTTCACGATAATATTCATCATCGCCGTAAGTTTCTACTGTTAAATCCCAATG
ACGAATTAAGTATTCTCCAGGAACACCACGAGCTTTAACAAACGTTGATGTATACTCTGCTTCTATAATA
CGAACTAATTGTCCGTTATTAAAAATAATTTCTGACACAGGCTTTCCATCAATTTTATATGTTTTAATTA
ATGGTTCCTGCATTACAATAATTTCACCAACAATAAAATCTTTATCAGTTTCAAAAATCTTTTTACGAAT
AATGCTATTTAACTTATCAACAGATTTATTCGTAAATGCCATTACGCGATTTTCAAACAAATCATCTAAA
GATTTGACGATTGAAAAATAATTTACCATAAAATCGCGTAAAGCGGTATCACCAGTAAATCCACGTACTC
CATGCCCGTCAACAACTTTATCATAAATCCACTTACCGTTACGAACGTCAGTAGCTACATCAATAATAGG
AGCATTACTGCGTTTAACTTCAGTGAGTTCACACTGATAAAAATCTTTGTGTGTAAAGAATGGACTGATA
TAAGCAGTATTTTCTCCTGGGTCAACAGGTCTAATTTGCTTATTATCGCCTATTCCAATTATAGTACACC
ACGGCGGGATAGTTGAAAGCAGAATTTTAAATAGCTTTCTATCATACATTGACACTTCATCGCAGATTAA
TACCCTGCATTTAGCTAAATCTGGTACTTCTTTTTGTTCAAAAAGAACGTTTTCTTCGTATGTTACTGGG
TTAATTTTAAGAATACTATGAATAGTACTCGCTTCTTTCCATGATAGTTTTGAAAGAATCTTTTTAGCTG
CATGAGTAGGAGCTGCTAAAATAATACCAGTTTCACCCGTAGATATTAAAGCTTCAATGATGAACTTAGT
AAGAGTAGTCTTACCGGTACCAGCAGGTCCATTAATAGTTACATGATGTTTCTTTTCTTTAATAGCCTTC
ATAACAATGTTAAAGGCATTTTTTTGACCTTCGGTCAAATCATCAAATGTCATCGTAAATTCCCTGCAAT
TGGTATACTAACAATACGCCCAGTATCTAAAATTCGCCGATATAATCTTTGTGTGTCTACATCAGGCTTA
ACATGTTTAACTTCTATTTTATTAAACCAAAACTTGCGTGGAGTCTCAACTAATCTTGGAATTCCCTTAC
CTAAAGCTAGTTGATACTGCTCTTTAAGAGTGGTAAATACTTTATCAGCAATCTTCCATTCAAAAAATAC
AGCAGGACGATGTTCATCAAGCGGAACTGGCGCCGTAAATCCGTCTTTGTCTCGGTAAACTATCGCATAT
ACATAAACCATATTATCCTCGGATAAGTTTAAAAATTGAACAATTTAGCGGATATCCTCTTTTCAGTTTA
AGTTTATCAATAAAAGACAAGTTTTGATACCGCTCTACACCTTGAATAATTTTATCACACATATCATATT
GCATTTCTGCTTCTGACAACTTTTTCACAGTTTTCCAATCCGAGCCTTTAAGAAGAACGTTCAATTTAAC
AACTTCAGCGCCTTCTGCTATACGAGAACCATCAATACGAGCTTTAAGTGCTATAATTCTTAGCTTAATG
TCAGAGGTCTGTTTTGATTTAGAAAGCTGAGAAATGTGTTCAATTCGGTTTTCACGTTTTTTCTGTATAG
CTTTAATTTGATTATAAGTCTTTTTGATTTTAGCCCATTTCTTTTCATCTAAATTTAGTTTATGAACTTT
TTTCGCAGATGAACGACCAATTCGCAAAGCAAACAAATCACGTTTTTCAATTAACTCCTCTAAAGTATAA
TCAGAACGAAATGTATTATACTTTTTCTTTACTGCAATAACATTCCCTTTAATGTATCCAACGTTATTAT
CAAAACGTTCTAATGATAATTTCTCTCCTTCAATACGATTATCAAAAGGTTCTCCCGAGTAAGCGCAAAC
TTTTTGGTCTAAAATGTTCTTAATGTAATTGAAGTCTAAGTTAAAATCTTTAGAACGTCTTTTAGCAGAT
GCCTGAGTATGCTCTAAACGACGTTTAATTTTACGAATTTGATTATTAGACATATTATGCAGCTTCATAT
TTTTCTCACATCTTACGGACGGTTAACTACTTATACTATAACATTTTTACTTTAACTTGTAAACAACTTT
ATGTAAAAATGCTTTAAAACTTTCGTGGTATAATGAATCTAAGTCCTTCCATTATAGATTAAATCTTTCA
AAATCAAGAGTATAGATAGTGTATGTTGAACACTTTTTGTACTCATATCTATCTGCAATTCTAAATACAC
TTCCAGCTGGTATCATTACTTCTTGTTCATCTGAAACTAATTCCATATTACGATAACGATGACTATCCGG
AAACTTAAAGTTTGGATTGTATTCTTTACAGCGTAGAGCTTTTATAGCATACTCCTGGAAATTGAATACC
ATAGGAGCTTTGAATTCAAAAATAACTTGTGTGTTATACTCTAAACCGGAAGCAAAATGTAGAGCTATAT
TTTTATCATATGAAGCTGATACTACTTTATCAAATGTAATAATATCAATTCCTTGATTTAATAATTGTTT
AGTCTCAACTGGAACACCTCTCCAAAGAGGTTTATCGTTTGGTTCCAAACGAGATTTGATTATTTCATTT
AACCAAGTATGGTCATCTGGTTTGTTGGTAATACAATGAATTAAAAGTTCAATTTCAGATAAATTAAACC
 
CTTCAGAAAGTAATTCTTCACGAATAGAAGCACGCACCGATGCATCCATTGATTTTATTTTAAAATCTTT
TAGTTGCATTACTGAGTATTTCATTCAACTACCTCAATATCATAAACTTTAAGTGTTCCAAATGAATCGT
GTAATTTTTCTTTTGAAATAGAAGTTATTCTATACTTTCCAATTGGAATCATCCATTCTTGTTCACGCAC
AATCATCATTAAGTTATCAGTACGCTCTGAATCTAGCCCATCAGTATCTTCATACGTGTACTTAAACTCA
GTATTAGGAGAAGAAAGTATAATATCGCATATGGTCAGAATAATTAAAAGCTTTATCAGTTTTTAAGCGA
AATATTATTTCAGTGAAATACTCAACATAAGAAAAACCACATGCTGTGTGCAAACTGGTAGTAAATGAAT
CTACTCTGTTCGTTGAAAACACTTCTCCAACTTGTAAATCTTTAATAAGTTCTTTTGTTGATTTTGATAT
ACCACGATATAACTGATAAGGTGATTTAGTTAAATGCTTTTTAATGATTTTATTCAATTGCCGATGAAGA
GCTTCATTCTTTTTGGCTTCCATACATTGCCAAAGAACTGACTGTTCAAAGTCAGTAAATTTTTCACAAA
CCTTTTTATACATATCATATTGAAAATCAACGCTTTCAGCTTTTATAGATAACTGTTCAACATCTGCAAG
ATTAATAATCATGATAGCCTCCGTATACTTCAGAAGCTATCATATCATCGTTAGAAAGGAAAGTAAACAA
CTTTTTGAATTATTTTGCCCAGGGAGCCCAGGGCGGAGGGTCAAGATGGTATGAAGCTAGTTCTTCTAGA
AGAGCATCTGGGGCTTCAATTCCATAATTCTGTAATACTATACGGTACTCTTTCTTATAATCACTAGAAT
CATTCTGGATATTCGTAGAATGATTATCTTCTAACATCTCAAATAAATCCATATTAATTCCTAGCGATAA
AAACCAAAATGACGATTAGCTTCAGCAATCTTTCTTTCTTCTTCGGACATTCTCCAGCGTAGTCCAACAT
CAAAATGAGCCCAGACCATTTTAACAAATGCGTGTATGTCTTTAATATCTTCAATAATGAATGTTTTACT
TTTCGAAGGTTGACTCACTAATTTAACCTTATCATCTTCAAACAAGTCAAAAAGACCATATTCATGAGCT
CTTTTATAGCCTTTAATAGTTAAAGCTTCAGAAGAAGAAAACGGTGAATCAGTATTTTGCAAAATATCTT
CAGTATGCTTTATAATTAGAATAATATTTTCTGGATATTTTCTTTCTTTTATATCTTTAATTAAAAAATC
CGGATTTTCTGCTAAAGGAATAATTAATGAGCATTTTTTATCAAGTGTATTTACTGATTTACCTTCTTTA
GACATAAATTCTATTGAATAGTGTTTTGCTACTTCAATCATGTGATTTCCTTTTGCCTACTAATGGACCG
TCGGGAATTTTGTTTTCCTGGATATATTTCTCATTTTCTTCCATCATTTTACTGCCAATTTTAAGAAGCA
AATCCAGTGCTTCATTTGCTTTTGCTTTAGCCTCTTCTAACGTCATATCTTTGTTCACGATTTATCCCCA
TAGATGTCTCTCATCAATTTAAGCGCTGAGCGTTCTAATTTCTTTTCTTTCTCAGCACTAATCATTGATT
TCATCCATTCTTCCGATTCGTTCTGCATTTCTTTATTTGCTTGTTCAACCCAACCATCAATGTACATTGA
GTTTGGTCTATTGAACCATTCAAGTATTTTCTTCAGAACTTTCATTTGTTTTGCCTAAAACAATAGTAGG
ATTATCATCAAATTCATGAATTTTGAGTAAATTTGGATTTAAACTATTCCATACAGAAGAAATAAAGTAT
GATAAAGCATCTTCACTCACAATTATAAACTTATTTCCTTTATCTATTTTCCAACTATATACTGAATCAT
ATGAATAGAAAGATAAAGGTTTATCATTATATTTTTCTGCGCTAACATTAATGTATTCTGATTTAATAAA
TGCTTCAATTGCTCTAAATGGAATATTTTGTACAGTTTCAATAATACCAATCTTTTTAAGTTTTATTTCA
GCATCTTCTGGAACTGGCATTGAAACGAAATTTTTTACGAGATACATTGGGTCACAATAACCCTTATCAA
TCATTACAGCTACAGTAATTGGAACATCTTTAGCAAATGACATACTATTACTGCTAATATACATTTCAAA
CAAAATCGCTTCCATAATTTTCCTCAATCACAAGATGTAGATGAACAACTAGAATCACAAGAACTTCCAC
ATGAATCACCTGCCCATACATGAACAGGAACATTAGTATCATATGAATCAGAACTAGACTGTGTATTAGA
TGATGCAGTAGGTACTGACCAGCGCCAAGGATTTTTATAATATTCTTGGGCTTCTTCATAAGTCATAGTG
ACTGCTTCTACTGTTCCATCTCCCATATAAACATACTCAACTACAGTTAAAGGAAGGTAGTCATTTGAAA
TAGGAACTACACCTTCCCCAGGAGTCGTAGAGAAAAAATCCGTAAAGAAACTTTTAAACCAATTAAAGAT
AAACATTACAAAAAGCCTCTCTTGAATTCGACTTGCTTCTCGCCATAATCATATCGAATTTCTGCATTAA
ATTCGACAGAACCATCTGCGTACATCATAAATGAATGCACAACAACTTCTGTAGACCATGGTTGTAGTTC
ATATTTCTTCATTACATGCCGTGAAATGATAATATCTAAATCTTCATTTGGTTTAATCCAACGATTTAAC
ATAGTGCTCTCCTCTATAAGATAATTCTATTATACTATACTCATTTTGATATGTAAACTGCAAAAACGAA
AAAATGAACCCCGAAGGATTCCTTAAATTTTAACCACCAATACCTGGAGTTGGCGTAACAGTCCAAAGAC
CTTCATTTGCAGTAATCCAATCAGCACGCTGATTTTCTTCATATACTGTTGAATATGCTGCTTTTTCTGA
ATGAAATGTCTGATAATGAGCCCCAGAAATTTTGTGAACATCAGAATAAAGAGGGAAAGCTACAGAAATT
 
TCTTTACCCTGAATTGTACCACCGTCAACTGCTTGGTCAAATGTTTTAATATTAACATAACCGCGAGTAC
TAGCCATGTAATTCTCCTTTTATTTAAATTACATGACTATTTATAATAAATTCTCGCGGAACGTTAAAAT
ACCCAATTTTAGTTAATATAACAACATCACCATTAATAATTACCGGAGAATAATCTTCTGGAGCATTAAA
TTTCCAATGGGCTCTAATATTATATCCATCAGAACCAAATGCTCGACTAGCCGGTCTCCCGGCTTTTTGC
CATGCATAAAAATCAAATTTCATTTTGAATGCTCTATAATTAATTTTAAAGCTTCTTCACTGACAAAAAT
TTCGTCAGGACCTTCTATAATTTCAACCATACGAAGTTGATTAGTTAAGAGCTCACACACGAGCTCTTTG
TTATAATCTTTAAATAGCTGGTCAATACGTTTCTTATGGGCTTTCTTAAGCTGACGTTTATTTAAACGAG
CCATTAATAGCCTCGGTCTTGACGAGCGAAGTTTTCGGCATTTTTCAGATAATAAAGTTTAAAGATTTCT
TCAGCTGACATTCCAAGAGCAATGAATTTATTCATAAAAAAGTGAAACTGGTCAATCAGTTCAAATTTAA
TTTCAAGCTGATCTTCAGGAGATAAATCTTGAATTTTCTTGGATTGCATTTCAGAATAACGTTTCTTCCA
AGGTTTCCATACAGCAGAAGCTTCTTTTTCACCATTACTCATACCACCAAGAGAAGTATATAGCTCGCGA
GTTTCGTCTGCGATATAATCGTCTTGGTTTCGCAGCCAAGCAAGAACTTCTCCTGCAGTTTCAAGTGAAT
CAGGATGACGATTTGTTTCTGGTTTATCATTAGCCAAACGAATCTGTAAATGCCGCTGCATATCAAGCAT
AACCTGCAGTGGGTCAATATTGTGCATAATATTCTCAGCATATGCACGAGTTGCTTTATCAACACCTTCG
ATCAAATGAGCGCATTCATTAAAGTGAGCCATTATTTTTCCTTTCAATTCATTAATAAGTTAAATAATTA
TATCATTTGAATATGTAAGCAATTAATTAAAAATATATACTTCATCAGTTCCATTCTTTTCTTTGGAATG
ATATATGTTAAAGACGTATTTTTTATTAAGATGCTTAACATTATATTTTTTAGACCATTCTTTAAGAAGA
GTGTTTTCCTTTCCGTGATGCTCTAAAACATTCGACAGTCCAAATTTTATTCCTCTGTCATTTAAAGAAT
CTAAAAGATTTAAAAGGTCTTTTTCTTCTTCTTCTGACCAAAATTTATTATAATCAGCAACTGTTATAAG
ATACGGAGGGTCCACATATACAAAATCGCCGTCTAGAATTTTGACATCTTTAAAATGCAATGAACTAAAG
ATTATTTTATCACAATTTTGTTTAAAGTGATTAAAGCGTTTTTCACTATTTTTGTTTATAGTTCTTTTTC
CAAACGGAGTAGTAAAATTTCCTTTATCGTTTATACGAATCATATTACTAAACCCGTGAAAATGAAGAAC
ATAAAGTAAAAGAGGATCTCTAGTTTTATTATAATCTTCACGTAATTTCAAAAACTCTTCTTTTGATGTT
TTTGATAGTTTGTATTGCTTTATTACTTTTAAAACGTCATCCCATGATACATTAATAAGACGCTTATACA
TTTCAATAATTGGTTCTTGAATATCATTGGCCAATACAGGACCATTGACGTTCAAAGACACTGATAAACC
TCCACAAAATAAATCCACGAATCTGTCATATTTCGGAAAGTGAGGCTTAAGTTCAGGTAATAATGATTGT
TTATTACCCGTATACGCGATAGCTCCTAGCATTATATTCTCTCATTTATTGCAGCAAAAATGAATTATAC
AATTCTTCATCATATGTATTTGATAATAATACTAACACGTTTTGTGATTTAGCATATTCATTAAACCTTG
CTTGGATGGTTGGATTATCAGTAAATGATTCTCTTTTTGTCCATTTATTACGTGGATAATATCCGCATGG
CAAAAAATCTAATATCATCAATGACACAGGGTTTTCAGTTTTTGTGTATTCTTTTAATTTCTCTTTATCT
TCTAAATATATTCCAACCGAATCAATAGCTGTACCTCCACCAACTTGGTGTTTAATTTCAATTATATGCT
TTTTACCGTTGCGAGAAATAACAATATCAAATCTTCTAGTACGCTTTTTAGTTATAAAATTAGAGCAAAA
CAAATTATTTGATTTTTCAGTATCATCAATTAAAACCATTTTTTCTTGATAACTAACTGAACAATCAGTG
AAATATTCTTTTACTTTATTAAGTATGAAATTTTCAACTACGGCAGATATTTTATATGAATCTCCGTTTA
AAGCCATTTCATGCACAATTTTATTAATCATTATATCATCCTCAGCAATTTATTTTATAATACCACAACA
AGCTGAGAAAGTAAACAAATTTATGATAAATATTAATTTAAAGGAGGACATATGGTACAAAAATTAATGG
CACTTGTTAATGCCATCAAAGGTAATAAAAAGCGTATAGCTTTTACTATTTCTGCTATGGTAGGAATTTT
ACTCTGGAACTTTATTTTATCACCTGTTGCAATTGCACATGGTATTAATATTCCAATAGTTACTCTTGAT
ACATTCGTAGATTTAGCATTTGCTTTAGTTGGGTTAATTTAAATCTTAGCATATTTAGATAACCGCATTT
TAGCCATCAACCCCTGGGCAATATTATTTTTCATATATTCCATAATTTGTTCATGGGATGCACCTTCCTT
TCTAATCATATCATTAACATCTTTTGATTTCCAGGGAGATTTATCCCAAAACATAACCCTTTCTCCTGCA
TCAACTAATTTAGTCATTCGTTTGATAGTGTCAGGGTGACGAGGTTCATTATCTAAGACCCACACTCGTC
TATCTTTAAATGGAACAATTTCTAGGTCTAATTGACCACCCGTAATAGCTATACCATTTTCAATAAAAAG
TGAATCTATAGGTCCTTCTAGAACATATACATCACCATCTTTAACTCGTTCGACTCCATAGATTTTTGTT
 
GCCTCAGGATAAGCTTTGATGGTGATATATTTTTGAGGAGCATCTTTCTTTAATGCACGTCCTTGAAAAG
ACTCAGCTTTTCCACTAGCATTATAAATTGGAATAACAAGACGAGGCTCAGGTATTTCCTTTTTATATGT
TCCCGGTGCTATGCTATTAACCAATTTAGGCCATTCGGTTGTAAACCAAAGATATTTCCATTTATCCTTT
GGAATACAACGAGCTTTTACATATTTTATAATTGGATTGTCTTCCGCCAGTTTATCTAATCTAACGCATG
ACGGAAGAGATTTAATTATTTTCTTTTCAGGTTGTTTAGGAAGTTCTTTAGGTTTTTCTACTGGACGACT
TTTACCTTTTTCTTTTCTTATTTCAAAGATATACTCACGATATAAATCAGGTTCAAACTCCTTTAAATAT
ATTCCGATTGGTGCATGATAGTTACAGTTATAACAATGAATGTTTCCTTCATTATTATCACCATAATACC
ATCCACGGGCTTTATTTTGGTCGGTTTTTGAATCTCCACACACTGGGCATCTAAACCGCAATTTAAAAGT
TGAACTATTATTTACTTGTGTGAATTTAGGTAAATGAGCTAATGCACGATACGCAAACTCATTATCAATC
CAAGGTATTGATGACATTTTTACTCTTCTTTTTCTTTAGATTCCTCTTTTTTCTTTTTAGGAATCTGTTC
AGGACCTTTATTTACTACAGCGCCTGACGTTGTTCCAGTAGAGATATTTTCAGGATTACCACCTGAATCT
CCAGCGACCATATCTTCTTTGATAAATTCTTTATATGTTTTCATATTAACCTCTATTCATAAAAGCATTA
AAAATTTGGTCATCAATAGATGGTGCAGTAATATTTTCTGTATCATTTAGCAAATCATTAATTTCATCAG
ATAGCATAACACATGTACCTTTATTTCCTGTAATTGTTACTGCACAATTACGAACGTTTTCTTTATTAAA
TTTTAGGTTAATATTTTCGTAATGAAAATCCTTTAATGCTGAATATATTTTTAAAATAAAATTATCTTTT
ACTACAATAAACCCAACCTCACTATTAAAACGTGGTTCATGGAAACTGTAATAAACCTTTTCATCTATAA
AATCGTATGAAATTTTTTCCATTTTTAAATATGAAAATTCAGAACTATGCATACTAACCTCTAATTATTC
ACCTACCACTTCAGCGATGATTATTTTTATTATTAAAGTTTTTATCGCAATACAGAACATAATTATACTG
CATTACACCACCAGACTTAAGCTGTTTTTGCACTTCAGCTTTCATTTCAGGACGATCACGCTCAACGATA
TTCATAATATCTGCTTCAATTTGGGTTTCAACTTCAGTCTGATCCGCAGTCATAGACCATTCACACAAAT
CTTTATCATAGCCTGCCATAGCAGGCTGAGCAGCACAAGAAGCTAAAGCAAAAATTGTAGCAAAGATAAA
TTTTTTCATGATTAATCTCCTCAGTAGTTTATGTTTATATAGTATCTCAATTTCCAATAAAAGTAAACAG
TTATTTTAAAACTTTTGCGTAATCACATGTTACAAACTGTTTCTCTAGCTTAACGATCTTACGAAAGTAT
CTTTTGCATTGGCGAATCTGCCGCTTCGTAGGGCGAACAGCAAACTTAATAAATTCCACTCGACCAAATG
GAGGACTTTCTTCTGCTGGAATATCTAACACCAATTCCCACGTATCTGCAATAAGTGCTTTGAATTGCGT
ATTTTTCCTGACGTTATACGGAGTAGGTTTAAATAAAACAATATGCATATTATCCTCGGCAATCCACTTC
ACACACTTTCTTATCATCAATGAAAGCTTTAACTAATGCTTTATTAACTTCAGCATATTGAGTAGTAGCC
CATTGAACGTCATCTTTCATCATTGTGGTTTCTTTAGTAAACATGCTTTCATTCTTAAACCACCCCATAA
AAACTACCTTTACCAATTCCATAACAATCTCCTCATTTAACCAACAAGACTACTATACCATAATCTTGTC
AGCTTGTAAACTAAAATTTTAATTCATTCGCCAAAGCATCTAACTGAGCTCGAGTGGATTCATTTCTTTG
ATAGCGATTCTGCTCAGCCTGAATCTGTTGTGAACCTGCTACTTCGTTCACTTCAGTTGGAGTAGAATCT
TGTTCAATTTCTACCCATTTCTGATTTCCTTTTTGAACACCCATCAAAAACTTATTCCATTTGTTTTTAT
CACCATATCGTGATTTGATTTGCTTAATGAGTTGTTGTTCAGCAGCTGCTAGCTCCTCGGTTTCAATGAC
TGCAAGCATAAAATCGGCTGTTGCTGGAAGACCGGCAGATTCTGCAATATCGCTCATGTTAACATCGGAA
GAGTCCCAAGCTTGTTTACCAACCTGTGCTGCAGTCCAAAGAACAGTTTCGGTTTCAACAGCCAGAGCAC
GCAATTCCTCTGCAATAGCTTTAACAGTTGTGTAACTATTTTCTGAATAAACTCTAATGCGGCAAGATTT
ACAAATACCTAGATAGTCGACAATAATGATTGTTGGAACAAAATTCTTTTTGAGCTTCAATTCATTTAAA
AGCGATCGAAATGTATTAGCATCTGCTCCACCGGTAGGATACTGTTTAACGATTAAACGACCGAGAGTAG
ATTTCTCACGCCATTTTTCCATTTTTCCTTTATACTCAGCGTAAGAAATATGCCCATCATCAATGTCATC
AAGAGAAACATCAAGCATATTAGCATCAATACGTTTAGCACAGACTTCTTCTGCCATTTCCATTGAAATG
TAAAGAACATTATGTCCGAGCTGTAAATAATCTGCTGCCAATGAACACAATCCTAATGACTTACCGACGT
TAACGCCAGCCATTAAAACGTTCAGTGTTCCAGTCTCAGCTCCGCCTTTAGTAATTTTGTTTAGAATTCT
GAGTTTAAATGGAACCTTACGAGCTTTATTCATATAAGATAGCCAACGTGCTTCGTAGTCATCCATCCAA
TCATGACCAACGTAGCTATCAAATGAAATTGATAATGCTTGGCGCATGATGTCAGGAATAGCACCAACAT
 
CCGGCATTTTCTTATTTCGTTTTTCCGGAGGAAGCTCAGCATTAGTTTGAATTTCGATTATTTTAGACGT
AGCATTAAACATCGCCCTTTGCTGAACATATTTTTCTGTTTCTTTTACTAACCAGCTGTGGTCTTCCGGA
GAATCAGCTAGTTTTGAAATAAGTGTTTTTACACCAGAATATTCTGTTTCAGTAAATGAACTATTTTCTA
ATGCAACATTTAACGCATTAATAGATGGAACGCTATGGTACTCATTAACATGAGATTTAATTAATTTGAA
TGTATTTTTAGCTGGACCACTTTCAAAATATTCTGAATCCATATATGGCCAAACTTTTGAAAAATAAGCT
TGATCAAATATGAGATGAGAAAGAATAATTTCTACCACACTTACTCCTTAAAAGAATTTAAATTTTTTCT
TTGACCTTTTATTAAATGCATCTTGTAATTGCATTGTAATACATTTTTCTACATGAGGAGCTAACTCAGC
TTTTCTTTCTTGGTCAAGAACAGCAAAGTCCATTACAACCTTTCCATCAACCCAATCCAGTTTTGTTACA
TACACTATATGTGTAGAACCATCTTCTAGTTTAATGACAATCTCCTGGATAACATTTTCCATAGCGGATT
TAATTATCTTAAGAGACTCATTAAAAAGACGTTCTTTTCTTTCTTCTTCCCCCTCCGAAGAGGGGGATTC
ATCGATAATTTCTAGATCTAAATCTAAATCATCTTTATTCATTAAATTCTTCCATATCACTTAACTGTTC
GAGGTCAGTTTCTAAATCAGCTGCTGATTTACTTTTACTTTCTGGAGATTTAAATTTTTCAACCTTTGAG
TTAATCAATTCATCAACTTCAGCTTCAACAATTTCATTACTATCAATAGCACCTAACTGATAAGCACGTT
TAATAGCATCTCGGAATGGTTGATGCTTAAATAAAGGACCCCAGAATGTAGTGCAGTTAGTATCTTTTGC
ACGCCAAGATTTTTCTTCGCGAATCATCTCGCCGGTTTCTTCGTCAAGAAATTCACGAGCATACCAACCA
TTCTTAGGTTTTACCACGAATCCTAATTCTAGAGCCATATCTAACAATCCAGAATAAGGATCGATACCAC
CGTCAAATTTAACATCAATAAAAAATTTACTTTTTTCTTTAACGGTACGAGATTTTTCTACATTTAGAAC
AAATTGATACCCCTGAAGATCAGAACCATCTTTAATCTGACGTTTACCGATAATGAATACAGTATCAGCC
GAATACATTACGCCTGTACCACCTGTCATCACGGTTTTACTAAACATTTCAATTGTTTCAATTGTATGGT
TAACCGCAACACACGGAATATTTTTAATGCTAAAATAAGGAGTAACAATACGGAATAATGACTTCAGTGA
TTTAGCACGAGTCATATCTGCCACAGATTTTTCATTCAAGGCATCTTCCGTTTCTTTCTTAGAAGCCATA
TTACCGATTGAGTCGATGAATACAATAACCTTTTCACCACGCTCAATAGCTTCAAGCTGGTTCACCATAT
CAATTTTCAGCTGTTCAACTGACTGAATTGGCGTATGAATTACTCGTTCCGGGTCAACTCCCATGGATCG
CAAATAAGCTGGAGTAATACCAAATTCGCTATCATAGAATAGACAAACCGCGTCAGGATATTTGTTCAAA
TATGCCGCAACCATAGTCAAAGACATATTTGATTTAAAGTGTTTAGAAGGCCCTGCGAAAATAGTTAAAC
CAGACTGCATACCACCATCAATCGCACCAGAAATAGCAATATTAAGCATTGGGATTTTTGTACGGATTAC
ATCCTTTTCATTAAAGAATTTAGATGTAGTCAGCTCAGCAGTCATTTTAGAAGTGGAAGCTTTAATCAAA
CGGGATTTTAAATCTGCAATAGACATTCATTTTTTCCATAGGCATCATTATATTTTCCTCACTGGTTAAA
GATAGAGTAATTATAACACAATAAATTTAGGCATTAATCAACTGCTATTGGATGAATAGCATTAAACTTA
TGAAATGCTTCTGATTTTTCTTTACGCGAAACACACATGCGAAGAACCTTTAATGACTCGTCTTCTTCAC
CCAACGATTTTCGTTTTTCAATATTAGAAGTTTTCCAACGAGCTTGCTCTGGAAACTCTCTATTGATTTG
CTCAAGCGCTCTATTATGTTTAGAATTACTGCGAATTGAACTGCACCCGCCAGGAGCTTGTGCTTTTCCA
GATACAACTAGATATTTGAACAAGGCCAAATGCGGATAACCTTGATTAATTAAATTGAGAAATGCATACA
TATCTTCGCATAAATCAATTTTTCCATACCCAATTTGTTCTGTAGTAAGTTTTCCGAGGTCATACCATGT
ATTCGTGAATCCATATGAATTTTCACGATAATTACCCCAAGATGAAGTAATTTTAAAAATAGGTAGACGA
GCATGGCCATGATAATACCCACAATCCATGGCATCCTCAACATATTGAATCAATTCATAGAACTGTTCAC
GAGTCAATTGATTGACTTTATCTACGCAACGACGATCATCTCTTTTTCGCATTGAACTCATACGAATAGT
AGTATCATCGTCAATCATCCAGATTCGTTGACCTGCATACATATCAGTAATTGCTTTACGAGTACCGGCA
ATTCCGTTTACATCATCAGGAATAGTTATAATTTTAGCTCTAGACCCATAGGCGTCATAATAAGCTTTTT
CTTCATGTTCACGTACTACGATATGAGGTTCATAATCAGTCGGAAACATATCAAGGGCAGAAACTGCCCC
TACATGTTGATAGCTCGGAATTACGAATTGAATCATTTCCACTCACCGTTATAATCTTTTTTCACAATAT
GAGTTTCGCCAGTATTCCACCAATGATCAACCAAGTAAAAATGACGAGAATATACATGAAGACTTCCAAC
ATTCCATATAATAGAACCTGCTTTATACTGACGAGTTGAATCGCCTGCATTCAAATCAGATACTAATTTA
TCTAGTACGTATTTTTGCCATGCATAATCATTACGGAATCCGAAGACCACGTCATTTGAGCGCATGCTTA
 
CTACTGCATTGACTTTCTTATCACGAATCAGGTATTGTACTGTATTCGTGCACATGAAATCTGACATGCC
GTCTTTGTTGTAGTCAAACTGCATAGATGGACGAGTATAAATCATGATACCACGTCGAGAATCAGGATTT
TGACCAAGTTCAGCTAAACACATGTCATATTGAGCATAGTTATCTTCTGACCAGATAGCCCAACCATAAT
TCGAGTTAATTTCACCTTTAGAAGATGCTACTTGTTGCCAAATCTTCGGTGTTTCACCCGGAATATCTTT
AACGAACAAGCTCTTAGATTTATACCATTCAAGTTCACGCTGAATGTATTCATCATTAAGAGCGCCAAAA
ATAAACGGTTCATCTGCTACAAATGATGCGCCAATAATTTCAATAGTTTTAACACCAGTTTTATCAACTA
CGAAATCTTTTTCTTTTAATGCAAGCCCCAAATGAAGACGGATTTCTTCAACTGTCATAGAGTCACTAAT
CATTTAAACCTCAATTGATGCATTCATATTTAAATTGTAACAGTAATAAACCCCACCCTATGATAAGAGT
TGGAATCATAGCAGGAACTGTTACGCTATAGTATATACTTATTATAACCATCAAGATTAAAAGCAACACT
GCTATAATTTTGCTTTTCATTCTTTCTCTCTGATGATAATTACCTGATTTGGCTGCGCAGACTTTTTAGT
TTCACCAGCAATTGACCAAATAAATGTAATAAACCAACCAATAATTGACCAGTTAAACAGTAAAGATGTG
AAAAAGATTCCTACTGTCGATTTTGACCCACGCATCAAGGCGATAAACCATGGAAGCATGTATATAATAA
TAGCCAACACACCTGAAACTAAAACCATAAAAATTGAACCTGCTACTAAAGTTTCCATGTTTTCCTCACT
TAGTCAAATTTTTTACACATGAATTATAAGAATTCACTACATACTCCATCGGAGCATTTTTACCGGTACG
CCACTGGTAATTATTAGCCCAATTTGCCCAAAGCACGGCGCAGTAGTTTTCAATTTTTTCTTCGCGCGTA
ATTACATCCGAATTACGATATGCTTGAGCAGATTCATCTGGACGAATAGCCTCGTCAAAATTCGCCTGCA
TTTGTTCTACTGTCTGTTTTGGAGCTTCTTTATAACACTTGACATTAGGATTATAAAATTTGCTTGAACA
GTTTACAATTTTTCCTACATCAGACTGATTTACTACCTGTCCTTGAGCTACACAACCAGTAAGACCTAAT
GCAATAACCAAAATAGCGATTTTCATAATAATTTCCTCTTAAAACTTAACAAATAACTTTAAAGCATCTT
CAGTTTTAATTCCTTTTCTTACACCCATAACAGAAATTGGCTGTGACTGAAATTTTCCTTTACCATTATA
AATTTCTAATCCAGTCACCATTCCATTTTGTTCTATAATACCGTAAGTGTTACCTTCATTTTCAAATTCT
TTAACTTTAAAAAGTTTATTCGCTTTTTCTGCGTTAACAACTTTTAAATTTTGAATAAAATTAGTTTGAA
TATTTTTCATTTTGTGTTCCTCCGTAGTTGATAGTTGTATAGTACCACAGAGGAACAGTCTTGTAAACAG
TTTTGTGAAAAAATTTTTAATGAATTCAAAGGGTCCAGAATCATCTGTTTTTCATAAGTATAGATTTATA
TTACTTGTATGAAAAGGGGACCCGGAGGTCCCTAGATTTATTCTATCAGCCAAACAGGAAGTCTAACGAA
GCTTTTTCCTCATAGTCCATACCTGCCGATTCACACATACCCGCAAGCGGTTTAACAAACGATTTTTGGA
ACAAAGTTGAGTAGTCAATCCAAGATAGTACATCAGAACGAATTTCTTTTGGAAGTTCTGTACCCGATGG
CCAAGCAATGCACTTATCACCAAACGGATTTCCTTCACGTAATGGAAGAACCATTACTTTATTTCCATCC
AAAATTGGAGCTACACCCAGACCACTAACAGCTCGACGATAAGTTAGCACACCACGAATATGGAACGGAC
ATTTAAATCCTGGCCAACCTTTATCATCATATTTCGCTATATCGTTCGCAGTTTTTACTTCAGCAATAAC
TTTATAGTCAAGTTGACGATATTCTTTCTCGAAGTTCTTGTAATATTCTTGGACAGACTCTTCGCCTTCC
TGAAGAATACGACGAATACTTTCTTCGAGTGCTTCTTGCACTGCTTTTGGTGTTGAACTCTGCTGAGTTT
CCATACCCATGATTTTTAGATGCGGTTCAGCAAATCGCTTATCTTCCATATCATAAACGTTCAGAGCATA
ACGTTTTTTCGCTTTCCAAAATCCACCAACACCCTTTGAACCAAGCGGAGGGCAAGAAATAGCTTCACGG
TCCATATGCATCAGATGCTCGCGGTTATTCATATAATCACATAACTCACGATATGCAACATCAATCATAG
GTTCCATCTTTTTCTTACCAAACTGATTCATGAATTCAACCAAATCGTTCTGCTCTTTGAATCGGTCAAG
ACCAACTTTTTCAATAACTTTATCTACACAAACATATACCGAATCAGTATCACCTGCTGCGATGAAATCT
TCATCATTAGTTCCGCATACTTTATTCAGATATTCATTAATTTTACGAGCAATCCACTGAATACCAACTT
GACCAAAAATTGTGATAGCAGTAGCATTTCGTAAATCATAGTAACGGAAATGAATATTACCAAGAGCACC
ATAAAGACTGTTAATAAGAATTTTACGGTTCAGCTGATTTGTATTAGCAAGTGTAGCTGCTTTTTCACAT
TCTTCAATCAGACTATTAAGAACAGATTCAGTATAATTCGATAGTTCATTTAAGAAATCATCAGTGAACT
TAACATATCGTTCAACTTCTGGTTTAGTTGAACAAGACCCTGCGCCTTTCATAATAATCTTTTTAATAGC
TTCGGCATTCATTTCTTCAGCGAACATTTTCTTTTTCCAATCTTTACGCTGGAAAAATACTTTAGCGATT
TCCTTTGGAATGATACCTTCTTGATGCTTATCATACATCCATCCATTCGGAGAACAAGAATATTCATCAC
 
TTGGTTTAGGAGCTGTTCCTGCGATATATTCATGAATTGGATGAACTTTAAACTGACCACGAATAGTTTC
AGGACTAATGTTAACCTGGCGAATAATGCTCGGATACAGAGACGTCAAGTCAAAACTCATAATGTATCGA
CGAGCAATTGGTTTAGGTTCAAATACAAATGCACCCGGAAAACTCTGTTTAACGTGCGAACCTTGTTGAG
GAATAACCTTGTGTTCACCTTTCAATGAGTTAAAAATAATAGCATCCCAAGTTTTAATAGGACTCATTAC
ACCAGAAAAAGGCATTTTAGCATAATAAGACATACTTAAAACTAGATCGATAAACCCACGAATTTTATCA
ATTGCTTGAACTGATTCTACGTCAATGATGTTATAACTAATGTATCGTTGATGATTAGTCTCACGAAGTT
TATTAATAGGACCGTCGTATGGTAATTTACCTTTTTTGGTTTCATGTTGAGCAACTGATTCCAAAGAGAA
TGACGGCAAATTAGTAAAAGCGAATTTCTTGTACAAATCTAAATAATCAAGAATAGATACGCCATCAATA
GAATAAATTTCTTTGCTACCGTACATATTTTGAATTAGTTTAGATTTTACCCGACCGATTGGAGAGAAAC
GTTTCATACTGCGTTCACCCAGAATCATTTTAACGCGATTCATGATATACGGAACGTCAAACCCCTCAAT
ATTCCAACCAGTAAAAATAGCAGGTCGTTTCTGTTCCCAGAGATTAATATATTCCATGAGCATATCACGC
TCATTATCAAATGGCATATAAATTACTCGGTCAAGAATTTCTTGAGGAACTTCATCACCACCTTCACAGT
CAAGCTTAGCAGCTAACTTTGCATCCCATTTTGATACTGAACCGTACATTGAATTCAAAAGGTCGAAAAC
ATAAAAACGGTCGTCAATTGAATCATAATGAGTGATAGCATCAATTTCATATTCTGCTTTCATTGGGTCA
GGAAATTTATCACCAGTAACCTCAATGTCACAGTTAGCTACACGAACAAATTTTCGGTCATAAACAATTT
CTGAACCATACGTATCACTGATATAAGCGAGTTTAAAATCGTTCATACCGAGAGCTTCGAGACCGATGTC
TTCCATTCGCTTCATCCAATCTCGAGCATCTTTCATTGATGGAAATTTTTGAGGAGCACAGTTTTTACCA
TAGATGTCTTTGTATTTTGACTCTTCCTTACAATGCCTAAACATAGTCGGAAGATATTCTACTTCACGAG
TACGTTCCTTTCCGTTTTCATCAATATAACGTTCAATAATATTATTTCCGACTGTTTCGATAGAGATATA
AAATTCTTTCATAGATATTCCTTAGTTTATAGCCCGAGTTATTAGGCTCTTGATATATTATACTCCAAAT
AAGGGGCCGAAGCCCCTTGCTTAATTACCAATCGTATATTTAGGAACGAGTTTCCATTCATGTTTTTGTT
TAAAAGAAATAACTCGGAAGTTATTAGTTAAATCTTTCATAAAAGTTCTTTGACCAGGAACGATTTCAAT
TAGTCCCCAATCTTCTAATAGCCATGCAATCGAATCACGACGAACTTCATCTTCTTCTGTCATTTCAACT
TGGCGACCATCCATACGAAGCATTTCTTTAAAATGAACGATATAGTATAGTCCTTTTTTCTGAAGAATAT
GACAGGACTGATACAGAACTTTATCTTTATTATTAGCAATTCCCATACGAGTCAAAGTTTCTTTTACTTT
CAGAAAATCTTCAGGTTTTTTAAGAGTAATTTCAATCATTTTACCATTCCAATGCTAGTTTTTTGAGTTG
TTTCTGTTCTTTTACGTTCTTAGTCACTTCTTTCAAAAAATCATCCGTGACTAAACCTTTTAGTTCTTTT
AATACTAAAGGAAGTTTTCCGTTTTTAGTAAGAATTGATTTATAGTTAATTGCATCATTTGTATTAACTT
GATACCGTTTAGCAAGTAACTTAATAATCAATACTTCGGTGGAATCTTCAACCAGTTTTGCCCATTTACC
ATATCTTTTACCACGAGGAACTGCAGCCATCAGATAATTAAAATGGGCTTCATCACTTAAGCCTGATCCA
ATTAAATTCATAGCATATACAGCTGGCATGCACTCTGGAAATTGTGATAATGCATTTTCGACCATGAATT
TTGAATAATCTTTTTGAGCAATAGAGCATTTAGTTTTATTATTAATAGCTCCAATTATTTCAAAAAATTC
ATTTTCGGCTTTTTCTTTAAAAGAATCAGCAGCGGATTGGACAGCTGTCCAATCTTTTGAATACCAAGCA
ACTTGATGCTCGTTTAATTGAATATCATCTTCAAATAAGCTCATATCACTTCCACTGCATTTCGCATGCT
AATTGAATGAAAAGATAAGCTAAATGTAATTCAGTGTTAGCTGCAATACCATGATACTGATTATTTTCAC
CAACAATTTCGTACATACGAATAATGCTCTGTGGAGTTACACGTGAATAGATTTCTTCGGCAAGTTTACC
CACGAACCACGAATAATCAGCCGCGTATTTTGGTGCTAAAGCTCTGAGTTGTTTAACATCTTTATTTTTG
AGAGACTCAAGAACATCATCAATAGCACCACGATCGTTAGTAACCAGTGATAAAATACCAGCATCCAAAA
CACCTTTAGATGAATAACTATCGAGCTCGCCAATAGTTTTACGAAAATCAGGAAAATTCTTTTTAACCAA
AGCTGCTACAACTTTCATATCAGCTATAGCAATTCCTTCGTGCTTGCAGATTTCAGTCAATCGACGAATC
ATCTGCTTCATCATTTCAATTTTATCTTCATCAGTTGGTTGACCGAATGTAATAACTCGGCAGCGTGACT
GAAGCGGTTTAATAATACCATCAATATTGTTAGCAGTAATAATAATACTACAGTTTGAACTATAAGCTTC
CATAAAGGAACGAAGATGTCGCTGAGACTCTGCTAACCCTGAGCGGTCAAATTCATCAATAACGATTACT
TTTTGACGACCATCAAATGAAGCGGCGCTGGCAAAATTAGTCAAAGGACCACGAACGAAATCAATTTTAC
 
AATCTGATCCATTCACAAACATCATATCAGCATTTACATCATGACACAATGCTTTTGCTACAGTTGTTTT
ACCTGTTCCTGGAGAAGGAGAATGAAGAATAATATGTGGAATCTTACCTTTACTTGTAATAGATTTAAAG
GTTTCTTTATCAAAGGCGGGAAGAATACATTCATCGATAGTAGATGGACGATATTTCTGTTCAAGAATGT
GTTCTTTTTCATTTACGGTAATCATAATTTCCTCATTCAAATTTTAGTGTAAATTATAAAGGGCCGAAGC
CCTCTATTAAAAATCGTGGGTAGAATCAGCTTCAAGAGCTACTACATAATTCGCGTGTTCACCTTCAAAT
TTAGCAGCACCTTGTTTACCTTTTGCCCAGAGCAGAAGTTTATAATTTCCTGGTTGCATTTTCATATTTG
CCATATTGATAATGAAATTAAATGTATTTTCACCATCATAATCACCAAGAGTCAAAGAATATTTAACACG
GGTCAGAGCAGAATCTTCTACTTTATTAAAACCGTTAATTACGATTTTACCTTCTTTTACCGTGATAGCA
ATTGTATCAATTTGCAGACCACGAGATACACGCAACAGTTGTTGAAGGTCTTCAGCTTTAATTTCAGTAA
CAACAGATGCTACCGGGAATGGAATTGGTTTATTAGGAGCAACTACTGTACTCGGATCGGCTGCTGGCCA
AAAAATTGTTGAACGAGCATCAGCAATTTTAATATTTCCATCTTCTGACTGGGAAATTTCTGCATCATCA
TTAACTAGAGACAGAATACCGAGAAAACCGTTCAAATCGTAAATCGCTACATCAAAATCAATAACGTCAG
AAATATTTGCTTCCGCATAAGTTGTACCATTAACTGCGCGAGTCATAATAAATTGACCGGATTTAAGCAT
AATACCAGAGTTAATAGTAGCGAAATTTTTAAGCAGAGCAGTAGTATCTTTAGACAGTTTCATGTAATTT
CCTTCAATTCAAATGAGATTTAATTTTATAACTAATTTAATAAAGCAATTAACGATTAAAATCAGCCGCA
ATTGTTTCCGCAACAATTTGAGCAGCAACAATTAGACGTTCATCTGCATTACCGCAATAATCATCTTCAA
GGCGTTCACCACATGAAGTCATAATAAATTTAGCACCGGCGTTTAGGGATTCTGTAGTATGTTTGCGCAT
TAGTTCAATCCATTTATTACTTACTTCACGATCGATAGCTTCATAATACGCATGACGAGCAGGTGCAGAT
TTAATTTTGTTCTGAATAACTTCCATTGCGTTATCAGAAAGAGACAAAACCCATGCTCGACGAATTTTAT
TTTGGTTTTGTGGATTTGATTCAGAACGCACGTGTTTTGGCTGAATATCTTTTACATCAACAGTATAATT
CACAGTAATTTTAGTCATAATACACCTTTAGTCATAATAATCAGTAACAGTCCAAGCTTCATTTCTATTG
GACATTATTTTTGTATATTCTGCTTTAAATGCATTCCTAAGCATAGATTCAGTAACTATATGCTCTTCAT
TAGAAAAATTATTTCTCAGAATATATCGTTTTATTTCAGGAATAGTTAATAGATGCTGTCCAGTTGAATA
TTCCATGTTTTTCCTCCATAGAGATTATACTCTAATAAATTAAAGCATAATCTCTTATAAATTAAACCAT
TACAGTAAATCGACCAACTTTCTTCATTTGAAGATGCTGACCATATGCTTGCGGGTCATGGTCTCTATGC
GAAATAACAAAAACATTAGTATTTTTTAAACTATCCAAAATAGTTGAAATAGCTTTTACACCTTCAACGT
CGGTCGCTGAATCAAAAACTTCATCAAGAATTAGTGTGTTTATTTTAACACCTGAAACTTTTTCAGCAAT
ATCACGCCAAGTAAATAAAAGAGCAATATCAATTCGTGCTTTTTCACCTTGACTGAATGAAGCATAACTA
AAATCTTCGCGACCACGGGATTTAATTGTCTCATTAAATTCTTCATCTAATGTAAACACATAATCCGCTT
CCATTATTTTAAGATAATGGTTAATCTGCTTATTAAATAATGGAATGTACTTTTTAATAATAGCACCTTT
AATACCAGAATCTTTGAGCATATCAGTCAAAATTCCTCGGTGGTATTTTTCCATTACTAAATTAGTTTTT
GTCTTAACAATTTTATCAAGTTCTTCTTGAAGCAGTGCTATTTCATCAGCATGGTCAATAAACTCAGAAG
ATGCTTTTTCTATAGCCGCTTTAACTTTTTTAGCTTTATCTACTGCTGCGATCAAAGATTGCTTTTTATT
GCGAATATCATTTGCCAACGACTGCTGGGTTTTAATATTATCTCGGTATTCATCAACAAGAACTTTTAAA
TTATCACGATGTGTTGAAAGCTGTTCAAAAGAATGCGTACATTCAGAAACTTTATCTTTAATTTTAGAAA
CAACTTTATCACCGGAACTCAATTGTGACAAACAGGTTGGACATAATCCACCTTCGTGATACATATTAAT
GACTTTATTATACGAGTCAATTTTTGATTTAATTAAAACTGCTTCTTGACCGATTTTATTAAATGCATCA
GTCGGGTCTTCGTCTAAAACAATATTAACTAATCTTTCATTAGCTTCTTCTATTTCCGATTTTAGCGTTC
TAGCTTCTTTTGCCAAATCATCATACATATTTTGTAGACGAGTAAGGTTGTCACCAGTTAATTTTTTCTG
GCGTTCAACGTTATCATTATATATTTTAATTTGTTGGATAATACTATCTTTTTTAACATCAAGCACTTGG
TTTTGTGAATTTAATTCACGTATTAGTGCTTTATTAAGCTTATCCATTTCAGCTAATGTTCCTACCTCAA
GCAGGTCTTCCACAAGCTTTCTTCGTGCAGGGGTCGACAAACCCATGAAAGGGGTATACCCTGCTGTACC
AAGGACAACAATCTGCTTGAAACTGGCATATGACATTCCGATAAGCTGTTCAAATTCTGCTTGGAAATCT
TTACTGCTGGCAGATTCATTAAGACGTGTACCGTTAACGGTGATTTCGAAAACGTTTGGTTTTTGTCCTC
 
TTTTGATATAGTACTTTTTCTCATCATATTCCATCCACAGTTCAACTAAAAGTTCTTTCTTATTTGTGCT
GTTTATTAATTGACCTTTCTTTACATCACGGAATGGCTTGCCAAAAAGCCCAAATGTGATGGCTTCTAAC
ATAGTAGACTTACCACCGCCATTTCGTCCAGTAATAAGAGTTTTTTGAACCTTATCTAATTGAATGTCAA
CTCCATTTTGACCAACTGACATTATATTTTTATATTTTACTCTATTAAGTTTAAATGATTTCATTTTGTA
TCCAATTGTTCACAATGAAGAAAATTCTTTAACTAAAGCCTGTAATGAAGCCTCTATTGGATATCCATTC
TTAACTAACATTTTCCTAAACATCACATGTTTAGGACGATTATATGATATCCACAATTCATACAATTTAT
CTTTATTATCCCAATGATAGCCTTTTCGTCTAGATGCTATATGAGCTTTTCTAATCTCCGGATTTTTCCA
ACTAGCAGCTACTGAATTTGATATAGCTATTTTATATTCAGGTTGATAAAGCAGCACGAACAGCCTTTTG
GTGTTTAATTTTATTTTCTTCGTTATTAAACCATTCCATTGAAGCCATTGAAATTTTTTGTTTTGTTTTA
AAATTATTTAATCTTTCTTTGTTATCTTTCCTATTTTTTGCTACTGAAAAATATTTTAATGCCCTTTCTC
TAGCAGCATTTCTATTTTGTGGATTTGAATAATACTGTTGAAGCCTTTTTGATATTATGTCACTTACATC
CTGTCTTATAAAACTATATTCAGCAGCTATTAAATCAACTTCGGCTAAAGATAAATTTTTATATGTTTTT
CCTGACATCATTCTATTTAAAGCATTTAGCATTTTTCCATTTTTACCTTTTGCTAATAAGAAATGTGCTT
TGAAATGATTAAAATGACTTAAATTAACTAAATTCCATGTAGATTTTAAATATTCAGGAAATATTGATTT
AGGAAGCACGTGGTGTGCTTCCGTTTTACCTGGAGTTTTAACAGTTGAATTCATAGAAATAAAATTAATG
TAAGACTTGGCGTCTTCTTCACTCAACGCTAATTTTTCAACTAATTCCTTTATTAAAAATTCATCATTAA
TATCAAGTTTAAAATTCTTCACAAAAGATTCCTTTTAATGTATCTTTTAGACCATTCTATCATATCATCA
TAATCTAAAAAGTATTCATCAAATTCAGCCATGCAAACAACGCCTTGTGCTGTTTTTGATGTAATATAAA
TTATTCCAACATATCTAGAATCTTCTTCGGTATAATCAATGTTTGCTATGAATTCATCATTAATGTCAAA
TGTCGAAAACTTCACAGTATGCATCCTTAATACAAGATACAGCCATATCTCGCAATGATTTAGGTGTGTC
ATTATCTAAAATGTTGAAGTTAAAGGATACAGCCCAGTCAGTGCAGACCGTAACCTTTTTAATACAATTA
TCTTCAACCTCTAGCGGTTCAAACCAATATTCAATAATTTCTTTATGCCCAATATCATCTTTTACTTCAC
ATTCAAAGTGCTGACTCATCATAACATTTTTAAATTCATCAAAAGTCATTGTGTTGCCTCTACATATAGC
TGATTTGCATATTGAATAAGTGCTTCACGGTCAGAATCAGTGATGTCTGGAATTGCATTAATGTATTCTT
CCATCAATGTCTGAAGCGATTGAACTTCAACTTCTTCACTGTCATCTGACTCGACAGAGTTATCAATCTT
TGACACAACTCGTAATGAATGCACAACTTTCTCTAGTTCAGATTCGAACTTCGTCAGATTTTTGTCTACT
TCAGTTACTATAACACGTACTGATAGATTTGTAAAATCTTTATAGTCAATTTTTCCTTTAAATGGATAAT
GAATTCTACGATGCCAGGTAGTATTGTTTGGAATAAATTCCATTCGTTCTGTTTCTGTATCAAACATCCA
GAACCCACGAGGGTCATTCTCGTCACCTGCAGTTAGTGTCCATGGTGTCCCAATATATCTGACGTTTGCA
GCCTCAGAAATAGTATGGAAGTGACCAGACCACACTTCTTTATAAGTCTTAAGGAAATCAGGTTCAAGAC
CATGAGATTTCATTCCTTTATAAAAATAAAATCCATTCAGTTCCCAGTGACCGACGCAAAAAGAAGCAGA
TGAAGTTTTGATGTGTTCAAGAATTTCACCAGTATTTTCTTCGCACATCCAAGGAATCAAATCAATCAAA
CATCCGTCAAAATCTACTGTAGTAGGCTTATCATATACTTTAACATTAGGATATTTAGCCAAAAGCTCAG
TAGAGGCATTTGGATGCATTACATTTTTATAGTGGAGATCGTGATTTCCTACAATAGTATGTAATGTAAT
TCCAGCATCATCAAGCGTTTGAACTATTTCACGGGCGAACTCCATAGTTTTGTGTGTGATTGCTTTTCGC
ACATCAAAAATATCACCGTATTGAATCCATGTAGTAATTCCATTTTTCTTAGAATATTCTATTGCTTGTT
TAATTCCATCAATTTGAATACCGCGAATCCACTCATCATCAGCTTTAACGCCTAAATGCCAATCACCTAA
ATTTAAAATTTTCATATATCAAGAACCGTCATTGAAATGCAAAATAAAATTATTGAAATAAATCCATCTG
GAGTACTAAAGAACCCAATCCAACATGCTCTAGTGAATAGATAAAATGCAAGAAAAAGTATCACATATCC
AAGAAATATCATTATATCAAACTCCGTATAAAGCTAAAGGGCCGAAGCCCTTTATTTTGTAATAATGTCA
AACTGTTCTTTAAAGCAGAAGCTTGAATCTTGATGCTGATACAAAAATTCATATGCTTTTTCACGTTCGC
GGTCATAAAGAGTTCGGTCAGATGACAGTTCTTTAATACGCTCGAATGTTGATTCCATGTCATTTTCATC
AAACCAAATGATACCGCTATCATGCGAGGTCAAAGGAGTATTATCAACACGGAATTTTAAATTATCACCC
GTTGATTTCCAGAAAACAGGAATTGTTCCACATGCACCAAGCTCGAGATGAGTATATTCTAAAGAACGTT
 
GTAGATATTTTTTGTCCAACTTACTCAACTGATAACCAAATCCGGATTTACTCATGCGTTCAAGCATTTC
GCTATTTACATAACGGTCAAGAATTTGCGTTGGTAAATTAGGAGCAATTTTAATTTGGTCTACTTGATGA
AGACGATAATACTCGTATGGAATTCCTTTTTCTTTAATAGGAATGAACGCTGGAGAACGTTCCAGACCTT
CCATAATAGTACTTAGTCCTGCAGGTTTAAGATGTTTTTCGTGAAAATCAAACATCTGATAAAAACCTTT
CCATGTAGTCGTACGACCAATCCAACGGTTGATATTCATGTTAATTTCAGAAACATCTTTCCAGTAGGTT
GACCGAACCTTCGCAATATCCATAGGAGGCTGAAAGTTATATACTGTCGGCGCTTCTTCAATATCATCAA
ACAGAGAAACAGTTTCTGGATACCATTCTTTCATCAGAACTTTATTAAAATCACCATTATCAGAATGGCT
AAAAATAACATCAGCTCGACGAACAGTTTCTTCTAATCCCAAATTTCGACGCAAAGAAAGAGAAGAATGG
TCATGTTGATAAACTACAACACGAACCGAAGGTTTAATGTTATCAATAATTTTTTTATAGTTATTAATAG
TGTCTTCTTCAACTGAAGTAGCAGGAACTGAATTGATAATTAGAATATCACAATCATTTACCAGCTTAAG
TGTTTTATCGTATTCTTTTGCCAATAAAACCGGAATTGAAAATGATTTATAATCATGCGCACAGTTACGA
GTAAATGATTTATCTTTAGCATAAACCAAAGTTACTTCATGACCATTTTTAATAAACCAATCACGTTGCT
CAAGAGAAAATTTAGTTACACCGCAACCTTCAAGACCTCGAGCCATAAAAATACATACTTTCATTTAATA
TCCTCATTGTTTTGGTTTATTTTACCAAAAATTTATAAAGCAATATAGGAGCCGAAGCTCCTATCCGCAT
AATACGCCATACAAAGGCTCATTAGAGCTTCTAAATTTTATGCGCTTATATGTTATAGTTCCTTCTGCTT
TAGCTTTATCATGAGACTCTTTAAAGCGTCTCATCATTTCCTCTTTAGAGGAACGAATTTTATTATAATC
TATTTCAGAAGTCTGGATGTTCATCTTTCATAACTGCCACCATTTTTTAACTTGATAAGAATCAACCCAC
ACTTTCATATTAGGGTCTGCTCTTACAATAGGAGGATTAACTTCTTTAATTGAACTATCACGGTATTCTT
TTTCAGAAATTTTCACACAAAGATGACCATTCAAAGATTCAGTAAATCCTGCATTAATTTTAAGACGCTT
GAATTTTACGCGTGTAAGCATCAATCATATCCTCAATCTGCGATCTAGTAGTCTTCCAAAGAATACTAAT
GAGTTCATCTTTATATGGCTGTTTTAGAATATCCCGACTTTTCTTGATAGCATATTCGTATTGAGCAAAA
TTATTATTTTCAGCTGCAATCTGAGCGTGCTTATAAAGACGATTCAGTTCGCGTTTGTTTTTAGACAATA
ACTTATTTGCTTTTCTTTCTGCTTCAAGACGGTTCTTTTCTTCAATAGAAGAAATAAGCTTTTCCACTTC
ATCATTAATTTCGGGTTTATCAGTCATATTATTTCTCTAATATAAAATAAAAATCATCATCTGTTAAATG
ATACCGATAGTTTAATTCTACACCATTAGATTTAAAAGCGGTATCATACGGATTTTCTGGATCAATATCA
ATGTCAAGAGATAAAACTTCCCTGAGATACATTTTAAGTAAATAGGGAATAGCTTCAACTTCAGGTATTT
CTTCCAAGAATCCGGAGAGGTTAATCGTTAGCCTCATATAAAAAATCCAAACTAGGAGAATCGTCTACAA
CACTTTTCTTTTCAGCCCCCGGTGTTCTATAGGTTGATTCTTCGTAATGCGTCATTTTATCATAGATGTC
TTGAATAAAAGTTTCATCTACTAACGCAACCATATCGTCGTCACGACTGTCATAGACATTGTGAACGAAG
TAACTATATTTCTTTGCAACTTCCTTACGTTCTTTTTTAATACGTTGGACGAATGCATTAAAACAAGCTT
GAGTTATATACGCATGTGGGTTTTTATATTTCGTTTCATCAAAATTGTGAAGCCCCTTAATAGAAGCTTC
TATACCATCTGCAATCATTTCTTGTTTCCAAGACTGGGTGTATCCTGAAAAGTTGAAACGTTTAGATAAG
CCTTCTGCAATAAGCATAATGGCTAATCCGATAGTATCATTTTGACGAACTACTTTATTTGGGTCTTTAT
TATTTGCTAATTCTGTTTTCCAATCAATAATAGCTTGTAAAAGCTCTTTATTGTTTACGTAATTATATTT
AGGCTTAGTTTCTGACATTTTCACCTCTTAGCTCAATTCATAGATCTATTATATCATAATATTTGAAGAT
CTATCTTAAAGCATAGAGGATGAATCAATTTCAAGAATTCATCAGCTTAGCCGCTCCAAGAGCTGCATCT
AGTGAATCAAATTGGTCAACATATTCAATTAATTCACCGTAATTAGCGTATAACCACCATTGGCTAAATT
CATACTCAAGGATGAATCCGTTTCCTTCAATTTGAATTAAACCAATGCCATTTGTATTTACTTCATATCC
AGCAAGACGCAAATCGTTAATAAGAGCTTCGTTCATAATTATACCTTAGTAATTTTCAGGTCTGCAAATT
TTTTCTTGCGTTGATTTTTCATGCGACGAATAGTTTTATCGGAAATTTCATGCTTTTGATAAGCTTTAGA
TTCTACACCAAAAGCTTTAACATCAAATTCTGACAAGATATATTGAACCAACAATTCACGGACAGTATTG
CGTCCAATCTTCTGATCATTCTGTTTCATCGTCTGATGAAGCTCTTTTTCCCATTTATCCAAAATTTGAG
GAGTTACAATATCGCCTTTTTCTAACAAAGAAACTACTTTATCATATGCGTAAGAATTGATGGCGTTTTT
AATTTGAATAGTCATACATTATCCTCAATTACGTTAAAATTTTATTATCCAAAAAGGGCCGAAGCCCTTA
 
GCTAAACTTTTTGGCACCCTTCCAGCCTTCATACATCATTGCGGCTGATATTAATATTGCTCCTGAGCAA
CTTGAATATTTGTGATTCCAAAACCAGTTTAGAAAAACTTCGTCATCAATACCTTGGGCTATCATATTTT
CAAAGAACTGACGACGAGCTTCGGCAATTCGCTGCGATAATTTAGATTCAGGATTCATTTAAATTTCCCA
ATTTCCATTTTCATCAATAAATTTAATCCAGTCATTTACTGACCACTTGGTCGTATCACCCTTTGGAGTA
ACATTTAAAGTGTATAGCCCTTGCTTAAAAAGCATGCGTTTGATATTCATATTTTCCTCAGCTGTAACGA
TAACACTCGTTTGATTTACGTTTAGCAACTCGTTGAGAAGTATTATAATCAAAATCATCGTCAATGTAAA
CTGATTTTTTCAACTTTCTTACTTCACCGCGTAATTGACGATTTAGCTCATCTTTAACTTCTGAATCAAT
ACCTTTCATTCTACGCCATTTATCTGAGCGAAAAATGTTTTCAACCATATCTTTATGACTTACACCATCA
GGAGCTTTACGCTTTCCAAAATAGTCATAATCACGCACTTTTAAGTCTTTACGACGATACGTTTTACCCA
TGGAGTTTAATTTCCTTAGCAACTGAACTAAATACAGCACGATCACAAATCATACGTTTGTTCCATTTAA
TCACAGTATAATGAACATCTAAATCATCGACTGCTGTAACGATAACGATATGTCCGTTAACACGATAATA
CTGGTCAATTTTAATCTCTTTATCAACCATATCAATTCCTCAAAGGTAATTCATATGTTAATAATACCAT
AGTTTGAACTTGTTGTAAACAACTTTGTGAAAAATATTTTAGGGAATGATAAGAAAGGAACGATAGCTTA
GAATGGTAATATACAGAATGTGAGAAAGAAAGGCCCAGAGGGCCCGTCTTAATCTTCTATGATATCTCTA
TCATATCCAAGTGAAATGAGAGTTTCTTTGAAGTGTTTAATGTTCTTTTGTCTAGAATCATTAATGAAAA
TGACTGGATAACGAATGTTAAGAGATGTGAATCCAGCGCGTTTAGCAAGAGATACAATCAGTGGACGATC
ATACTCAATCTTACCATTATTTGTAAGAACTTTATAGAAAGTAAAAGGAGCATTGAGCTCCTTTAGAAGT
TTTGTAACTGATTGACATCCAGGACAACGACCTACTTCATCTGGAATTCCATAGACTTCAATCTTATTTT
GTTTCACAACTATTCCTTACTAAGAGCAGCATTCAGCTTGTTAGTAATTTTATCCAGGCGCTCATTGAAC
TCACTGGAAGATAAGCCCTTTTCTGGAGAAATTAAACTAATCACGAAAAATATAGCAATAAAAGGAATAA
GGAAAATAGCTCCAACTGCCATAAACAGAAAGAATGTTACAGTTGTAAGAAAATCAGCTAAACCTTTACG
AAATTTATACATATTTTACCCTTAATTAATTAACCAAGCATTGATAAGCACTAAACTATATTGGGAATAA
AATTCTGGACCAAAATGAAAAATCATATCATTTATAGTATCCATAATGTAATTCAATTTAATCATGTTTC
CACACCCCATCGGTATTTGACCAAAGTCGCTGATTATCTGATCCTCGCCACAGCTTTTTAGTCGGAAGAT
TTTTCTCATACTTCCCATCAATAATAACATCAACATATTTAAGCATTTCTAGTTGTTTAATATCTTCAAA
CTTATATCCTGTCCACAACCAAATGCTTTTATTGGGATAAAGATTTTTTACTGTTTGAACAATGCAATGA
ATGACATCTCGATTATCCGGATAGAGAGGGTCTCCTCCAGTTATAGTCAATCCTTCTATATAATCATTAT
TCAAACATTCAATTAATTGTTCTAGTGTTTCACCAGTGAATGGAATACCATTTCTAGCATTCCATGTTGA
TTTATTATAACACCCTTCACATTTATGCAAACAACCTGTAACGAAAAGAACGACCCTGCAACCAGGGCCA
TTCACAAAATCGCAAGGATAAAATCTATCATAATTCATTGATGCTTAACCCTATGCATGATTTCTTTATT
TTTGCCGAGATTAAATCCACGTTCGTTCGGATTTCCCAAATAACCACACGTTCTTCTTATTGTGTTCATC
TTTTTAGGATCAGTTTCTCCACAAATAGAACAAACAAATCCGTTTTCAGTAGGAGTCATTTCATGGGTAC
TTCCACATGTAAAACATTTATCTACCGGCATGTTAACACCAAAATAATCTAAATGTTGTGCAGCATAATC
CCATACGGCCTCAAGACCCTTTAAGTTATTTTTCATATCAGGAAGTTCAACATAAGAAATGTGACCACCT
GTCGCAATGAAATGATATGGCGCTTCACGAGAAATCTTTTCAAACGGAGTAATATTTTCTTCTACTGAAA
CATGGAAACTGTTAGTGTACCAGCCTTTATCGGTAACATCTTTTACGCTTCCATATTTTTCTGTATCGAG
TTTACAGAAGCGATAACAAAGGTTTTCAGCAGGAGTAGAATATAAACTAAAAGCAAATCCGGTTCTTTCA
GTCCACTGTTTAAGATGAGCATTCATTTTAGTTAAAATTTCTCGTCCAATATCACGACCGACAAGAATAT
TCAATTCGTGAATACCAATGTATCCTAAAGACACTGAACTTCTACCGTTTTTAAATAACTCAATTATGTC
GTCATCAGGTTTAAGACGAACCCCGAATGCACCTTCTTGGTAAAGAATAGGAGCAACAGTAGCTTTAACT
CCTTTTAAGGAACTAATTCTACACATCAAAGCTTCAAAACATAAATCCATTCGTTCATTGAACAATTCAA
CAAATTTCTGTTCATTGAACTGTGTTCCAATATAAGAATCTAACGCGATGCGAGGAAGATTCAGTGTTAC
AACACCAAGATTATTACGTCCATCAAGAATTTCATTGCCAGTCGAATCTTTCCATACGCCCAAGAAACTA
CGGCAACCCATTGGAGAAACAGGAACAGATGAACCAGTGATAGCTTTATTGTTCTTAGCTGAAATAATAT
 
CAGGATACATCCTTTTGCTTGCACACTCTAAAGCAAGCTGCTTAATATCATAGTTCGGATCGTCTTTATA
AAGATTAACACCTTCTTCAACGAACATAACAAGCTTAGGGAAAATAGGAGTTATCCCATCACGGCCAAGA
CCTTTAATGCGATTTTTCAGAATTGCTTTCTGAATCATTCGTTCAGTCCAGTCAGTTCCCGTACCAAATG
TAATTGTTACAAAAGGCGTTTGTCCGTTTGAACTAAAGAGAGTATTTACTTCATATTCATAAGCTTGGAA
TGCATCGTATACGTCTTTTTCTGTTTTAGATTGAGCATAATTCAACGCATCAGCGATTTGCCATTTTTCT
GCATCCTCAATATGTTTTGCATATGTGCGTTTAACATAAGGAGAAAGTACTTTATCTACATTGGCAAAAG
TCGTTCCGCCGTATTGGTGAGAAGCAACCTGTGCAGTAATTTGTGCCATAATTGCAGTAGCAACGCCAAT
TGATTTAGGAGTTTCAATCTGTGCATTACCAAGCTTAAATCCGTTTTCAAGCATTCCTTTTAAATCTACT
AAACAGCAATTAGTAAATGGAAGAGCAGGGGAATAATCAATATCATGGAAATGAATAATTCCGCTTTCAT
GCGCTTTCATAATAAAAGACGGGACCATATTTTTGGCAATGTGTTTAGACACAATACCAGCCATAAGGTC
CCGTTGAGTTGGAAAAACACGAGAATCTTTATTAGCATTCTCGTTTAAAAGGTCTTTATTAGTTTTATGA
ATCAATCCTTCAATTTCTTTTTCAATTGTCATTTTAAACTCTTTCTAAGCTGCTTCTTGAATGAAGCTAT
TAACTGTGTTTTGGTGTCAGATTCATTATATTCAAATCCTCTTTGAAGCATCTCGGCCATCATTTCCTCT
TTTCCTAAACGAGAAAATTCCTTTGATTTATCTCCAACAAAGTTAGGGTGAATATTATTTTGGGTGTAAT
CGGATTTTAAGTAAGTAAGTAAATTTTCTAACCATTCAAGATAATCAACCCCTTGTCCCTTTAAGCCAGA
ACGATTAAATTTGTGCTTCATTTGACCTTCTGCGGCATTGCAGAGATTACAAAGCAATCCACGCACCTTT
CCTGCTTTTGGTCCATTTAATTCATGGTCATGGTCGAGGTGATTAGCTTGAACATCAGGATTTAGTTCTC
GTTGGCAAATTAAGCATTTACCGTTTTGTGCATTATAAAATTTTTGTTTTTCTTCTTTGTATAATTTGCC
AGTCAATAACATAATAAACCCTTACCTTAAATAGATAAGGGTATTTATTATTTTCAAGTATTGTAAAACA
TTTGATGCAATCGCTTATATTGCTGAATCATTCGGTCAGAAAAAGAAATTTGAGTTTCAAGCCATTCAAT
GTACTCTGCGGCAGCTTGCATTAAATTTCCTTCATAGCCATCGTTATTTTCTTGTGCAGCTAATTTAGCT
AATGCGTATGAAATACGTTCACCTTGAAAATCGGCTTTAGGCTTCTGAACAACTTGATTAGTTCGCTCTA
CAACTTCTTCAATTTCGCCATTTTCGACTGATTCAGTATTCCACAAACACCAGTATGTAATTGGCTTATC
GTAGATGTTAATAATCTTTCCATCAGAAAGTTCAATTTCAATAATTCCAGTATCAGGTTCTATATCATCT
TCACATTCTTTTGCAAGTTCACGAACTTTAAAGACAGTACCTGCACTAAGTTCCGGCCAGTAATTACATA
GCCCTGTATCAGCACGATTAATTCTAAACCACTTATCTACTGTAATCATGTCCCATCTCCATATCAATTA
AGTCATTTATCGTTGGTTCATTATATACTGTTTCTTCATCAGTGTAAACCGGTTCTTCCGGCTCTGGCTC
TACAGTTTCCCATCTAGCCGCCCACCAAGGTTTTATAGCGTAATCCATTCTCGTACTGTCTGAGTTACTA
CACGTTCTCGAAGCTCAACCAGTTCAACGGTAGGAACTGCATAATACCAGTCAGAATGGTAAGAACCTGA
GCGAGATTCATTTACAGCCATGTACATTATGTTTTGGACTAAAATAAACACACTGACGATATTGGCATTT
ACCATCTTGCACCCAGTCTTCTATTTCGATAGGTTTAAGATAGTCGGAAAAATCGAAATCATAGTTTTCC
GAAAATGCATCATGGTCTTCGATGATTTCGCTCAGAACTGCATCAACATCTAATTTATTTTCAATAGTCA
TTTTTTCACCAATTAATTTCGCAATTTATTAAGTTCTTCATAATCAATCCAGGATTCTAATCCTGATGGC
AAATCAGATTCAATTTCCCAAAGAAGTTCTTCAATTTTATGAAGACGATCTAATTCTTCAGCTGGAATAG
TAACCATTGATGGAGTTCTTGATACACTAATATCGTAAATCATATTTACCCCAGTTTGACCATACAATCG
CCGTATTTCCACTTAGAAATAGACTTTTCACCGTTAGAATAATAAACTTCGAGTTTAGCACGATTATTTT
TAATTTGAATAACCTTGGCTGTCATCAAAGTTCCATACCCATAGTAAACTGCAACTTCATCACCTACATA
TACCGCGCTTCCGCGGTAATCATGGATATAATCAGTTCCTTCGAGAATCATTTAAAGTATTCCCGCAATT
GGTCAAATCCACCAATATGACTTCCATCAGGAGCAAATACCTGAGGCATTGTTAAGCCGATTTGAGTATC
ACGACCTAGTTTAGTCAGAAGCTCAGCAATTTTCTCATCGTCAAAAACACCTTTTTCCGGCATAATGTTG
ATAAATTCAAACGGCTGTTTCTTCACAGCCAAAAGACGTTTTGCATTATCGCAATACACACATTTATGGA
TGTTGCTATCATAACCATATACTTTAAACATATTATTCCTTAATTCCTAGTACTTGTTTAAAAGTCTCAT
CGTAATCAAGACTTTGGCCCGTTTGTTCTTTATGTTTGTATATAATATCACTTACTTCTGATAGCATATT
TTTATATGAACGAGTTAAAGCTGATTTAAGCACGCTGTATCTATCAGGAAATTTACCATGTTCATTATAA
 
TAAGCTATTGCTAATTCACGGACTGCCTTTTCAGCAGTCTCCATATATTCTTTACGCTTTGTCATTTTCT
TCTCGGTCAAATCGGTGTTTACAATGGCGACATTTATAACGAAGATTACTAGTCTGCCAATGGACCAATT
GCACCTGTTCAGTTCCACATTCAGGGCAATTAGGAACGTTTTAGAAGCCCTTTCGCGGCGTTCAACCAT
GACCATTACAGAATCCCAATTAACAGGAGGGCTAATCATCGCAACCATGAATCTTTCCACGCACTTTCAG
ATCGTCTTCTTCACCAGCCATAATAATTTTAATTACTGGAATTACTAGAAGCTGCAATATCTTCTAATAG
ACGCTTTCATTTCAACACCTCAATAGCATTACGTAAACCATTTGCTTTGGCGGTTAAAGCCTTAAGAACT
TTAGTATGCTCTTCAATTTGTTTTTCAACTTCAATCAAACGAGCATTGAGATATTCACGTTCTTCTTTCA
TAGTATCATTCCCATGATTGGGTTTGGTGCAGATTTAAATTCACTAGGGTCCTTTAACTTAATAACAAA
TTTAGTTTCAAACAGTGAAAGACCATAATTCGTATTATCATCAAAGGATTTGACTACATCCATATCAGAT
AATAACACGCGCAAATTATAAGCCAAAGAACTAATAGTTTCATTATGTTTATTAGCTTCGCGTGGTTTCA
TATACGGAAGATTTTATATATCTCGTGCGGAGCACAAACAATAATTGACAATTCAAATTGTCCATTCTG
GGACGATGGAGTGAATTTAGCCGTAACCCAGTCTTTTACATTCACATTAATAGTACGGTTATCACCATGA
CAAAACAGTCTACGAGAATGCTTAAAAAGCATATTCACGTTATTATTGAATTCGTGTGAAAATTCACTAG
CAAATTTTTCTTTACTATCTTTAGAAAACCATTCTTCTAAATAAGTTCTTTTCACGTCGTTAATAATATT
AAATGTAACAACACTAGCATTTGATACATTAGTCTCAATGTAGCTTGGATACATAAATTTTTAACATTA
TTAACAGCTATCACAGAAGATAGTAATCGGGAACGTTTGAACCACTCGAGCAAAGAACCAAGAGAATGTG
AATACGCATTTTGTGTATCTTTACAAATATGGTTTTCCCATCCAATATAATCTAAAAAATCACGAAGAAT
ATTATTGATTACTGCTCTGTGTCCAATCTGATAATTACGGTGTTTAGTAATAAGACTGCCAAAATAATCA
ATATAATGTTTACGAGTTTTCATGTTCTTCTCACTTGGTTAATGATTTATACTCCGAGCCATCCTTGGCT
TTAAATTTTACTTAATTAACTGTAAAGCTTGTTCAAGACGATCCAGGCGATTAACAGATTCTTCCCAAAT
CCTTTTAGCCTGCTCATATTCTTTCTGCGCTTTATTAGAAATTTCTAGAACTTCTTTATAAGCTTTTTCT
AGTGCAATAACTTCTGGACGAATTTCTACCGGTTCAGGTGAATCGTCAAGACATTCCATTAGTTCCTCAA
GGGTAGTTTCTTCTTTAGGAGTATTCACAATTTCATCACATTTTTGTTGGTAAATTTCTTTATCAGTCGG
TGAGTACGCACACTTTACTTCACGAAGACTAACTACAATTTTATATCCTTCCCAACCGCATATATTCTTA
AAAGGATATGTATGAATATTTTCTACTGTTTCCATACAAAGTAATGCGGTCTCTAATTGACGAGTTATAG
TGCTAGCAATTGAGAAAAATTTATTAATATTCTTTTGGTTAGTTTCTGGTGTAAAAAATTCATAATTCAC
AAAAAGCTGCTTTATTTTTATCAAGTTCATATTCTTTTATGATAATCATATCAGAAGCCAAAGGATGAAT
TTGACGATAATCACCATAACATAATTTAGAAGCTGATTTTAGAATTTGCTTCTTAAAAAGTCTGAAATTA
CTAATCCAACGACGAGTAAAAATATTCTCAGGGTCTTCTTTGACATTAAGATGATAAGAATTAACATCAC
CGAACCAATTATATCCTACTACATCTTTGGTTCGTTTAATTTCTTTCCCAAAATTACCAAGAATATCCCG
ATTAACCAAATATGAAAGAAGACGAGACTCTTTAAAGGTTTTCTTGATAACATCTTTATCTACACGGCTA
GAAATTTTACGAATTTCATGAATAAATTTAGCAGAAGAAACAATACCTGCATCAACACTATTGAGCCATT
GATTGATAATAGAAAGAACACTATTTTGGCCAAACATCGGTATAGCTTTATCATCAATACACTATTGAAT
GATTTGATATATTCGTTACGAGTCATAATAATCTCCTCAGTAGAAAGTAAGAACATTATACCACATCCTT
GTGGCAAAGTAAACTAGTTCAGTGCATTTAGTGCATTGTTCAGTTTAGAACGTTGCTTCGTGAGATTTTT
GACTTTTTCTTGTGCTTTTTCTAGCATCTTTTCAGCTTCTAACACTTCATTAGTTGCTTTAACGAGTTCA
TCATCTACTGCCTTGATAGACTTCTCAATCGCATCCGCGTGCCATTTTTCAACAGGTTTAAGACTCGGAT
TTTCAATAGGAAGAAAATTCACTTTATTGAATTTCCATGCATCTTTATTACCTGAACTATACATCCAAAA
TTTAGGATCGTTTGATAAGTAATTAGATAAATTTAAGTTATCTTGCACTTCCTGCTTTTTCTCTTGTACA
ACTTCGTCCTTTCTCAAAAAATCGTATTTAAATAAAACGACCATATCAAGTTCATACGTTGTTGTGCCTA
ATTTAAATCGTTCGAAATGCTGTGAAATCTTAGACTGAACACTAGCAACAACATCCATATACTTAAATGC
TTCTATCAACTGTGATTTAAGGCATTCACAAATTGAAAGAGAATTTTTTGTATTAGGTTTAAAGCTAATT
CGTGCAGTTCTATTATTTTCTTTTAATGGTCTTACTTCCATCTGAAGAGTATAACCATCAAATTTCAGAT
TCTTTAAGTTAATATCAGAACCTTTTAATCGACTAGCAGTCGACAAAATTTGCTTTAATTGTTTACGGAA
 
TAGAGCAATAAATCTCCATTCAAGACGATAATGATTTGGATTAAAAAATCCTGTTGATAAATCAACCTTA
CTCTGACCAACGCCTTGAACAAATAGAGGATTTTTATAATCAATAGTTTTACAGAAATCAACGAGCTGCT
CACGAGCAGTCATTTGTGTAATATACCCTGCTTCATTAAAATTACGCACCCATTCACTGCTGTTCAGATG
TTTAAAGGCGTGAATGGAACAATTAACTTTATTAAGGTCTAAATTATTTTCACACAAAAATGTCATAGCA
TCACGAGTATAGCTGGCATTACGAACCATATCTTCAATTTGAGAACGAGTTTTCATGGTGTTCCTTAAGA
TTTAAGTAAATCAACAATTTTAATTAATTTTTCACGTTCAGATTTAGCTTTACTACTCAATCCAGATAGT
CTAAAGATTTCATCGTCATATTGTTGAATAGAAATATTCAGCTCTTCAATCTGTTTATTAAAATAATCAA
TTTGTTCAGAATGTTTTTCGTTACTACGAACTGGTACAGGTTTTGTAGGCAATTTAGTTGAACTGGATTC
ATTCGGGCGATAAATTAAAATGCAATTTGAACCAATCGGGCATTTAGCACCAGATGAATATTCTAACGTT
CAAGCTTCTTTTAAAACTTCAAAAGCTAAACAGAGATGATGCCCCATATTGACATAATCTGTAGAGCGAG
CTCTGACGTAATAATCATCTCCACGAGTACTAAATTTAAAGCATTTAAGGTCTTCTGTATTATTAGTTTT
AAAAATCATTTGTTTATCTAGACCTTTAGCTAATCGAGCGCCTAATGCTAATAATCGTCGTTGATTTTCC
CACAAATCCGCAATCATTTGATCGAATGATAATTTCGGCATTAGTCGGCCATAAAGGTCATATCCTTTAT
TATAATTACGAAGGATTTCACTCGCGTTAATACGAGACAATGCTCCAATTTTATTAAGGGCTTTCATGAT
GCGATTAGATAAACTCATTCCAGACCCGTTTGTTCGCTGTAAATGTTTTTCTAATCCAAATCCAATATCA
ACTTTAAATTTATCTAAAATTTCAGCATGAACATCTCTGTCAATAACATTCAAATCCAAAGTTGGGTTAA
ATCTATGAAAAAATTTATCTGGCTCTCCACGACGAAGTACAGTCCATTCATTCATCATTTTTTATTAACT
AAAGATTTAATTACTGCATTGACATTATTAATTACTACTGACATATTTTCCTCACTCAATTTTACTAATT
ACGCGGAATAAGATTGAACAGACTATATAAGTACCACATATAGATTGAACTAATACCATTCCAAAGAACC
AAACAATATTATCAAACAATGTCTGTTTTACGTCGAAAGGACGTAAACTTACAGTAATTATCGCCTTTTT
CTATTGATGAATACATCTCTGGCGAAATATATTCGCTAAATCTATAACCGTCTTTGAGTTCATATACGGC
AATAAACGATAAACTAGACCCCTTTCCTTGAGTTCCTGTAAGGGTATTAACTACAGTAACATCATAATCT
TTATAATGCATATAATCATTAATTGCGTAATAACCATATGCAATTACTATACATAAACAACATATCAATA
AATTCAATCTTTTAATTATCAACTGTTTCATAATAATCTCAATTAAAAGGGCTTAGAACCATTATACCAT
CCTTGGTATAAAGCGGTTATGCGAGTACCGTCTTTAACCGTTCTTCAAACTTCCGAAGAGTATTTTGGCG
TTCAGCTCTTTGCTTTTTGTAAGTTTCAATACGCTCTGAAATGAGAGTGTATCGTTCATTTACTGATTCT
TTCATAAAATCAGGAATTTCACGAGAAGCTTTAATCTCGTCAAATTTATCAATAACAGCTTGCTCTTCAG
CAATTAAGTTATCATACATCAAAATATCTTTCTTGATGAACTCAATATCTTCTTGAGTTACACGAGATAA
CTTAGATGCTTTATCCTTTTTGTACTGTTCGTTAGTATCACGAGACCAGTGTAATGTACGATTTTTATTC
GTATTTTTGTAAATTTCTACAATACCAATCTCATCGATAATAACGATCCAATTCCAACGGGATTTGTAAA
TTTGTCCTCCATCAACTGTGATTTCACCTCCGATTGAGATGTCATTAAAGAACTTGCTTTGTGCTTCAGA
TTTAAATTTACCATCGTTGTAATTTACCAGGTTGAAAATATCTTTAGCGTTCATTTTCTGTTCATCCGTA
GTTGATAGTTGTATAGTACCACAGAGGAACGGTCTTGTAAACAACTAAAAGAAACTTCTTTCACAATTTT
TTCCACTGAACCACGCGCTCACTGCTTTCTTAGTCTCAGGAGCAGTGTTATCCATAAACCATTCAAAGGC
AGCCTTTTTATGATTCTGGAGGGCTTCTCGGGCTTTAATCTGCTCACGGTCTATTAACACTAACATATGA
GCCTTTCTTGTCACCAGGGGCTTCTTATGATTTTTTGAATATTCCCAATCATTTGTCCATCGCATCGTTG
TTGCGAATTGAAATACAGCTTCTTTAATCTTAGTTTCGTAAATTTCACGAGCCTTTGAGTATAACATCAT
TACCTCCATTTACCAGTTTAATTCTAGTCATCTTTTTGATGGCAGTCCATATAATCTATTTCTGAACTGC
CTTTTTGTCATAGAAGGCCTCTTATGAATTATTTCAGAAGAGTAACCTCAGCGATTTCTTCCCAACCGTT
TTTGTCGGTCATAATAAAGTCAGCAAGATAAAGAGCAGTACGCAGTGAAACATTGCGTAAACGGTTAACA
TTGACTTTCATCCATGATAATGCTTTATAAGTTTCTTCATCAGAAAGACCGCGCTTTTGCATCATGTCAG
TTGAAAGAATAACATCTTCAACCCTGACCATAATTTCTTCATTAGTGTGAACACCCAAATCCAAATAAAC
TGAGCGGGACACTAATGCTTGTAAATGTGGAGCAAGTTTAGTACCACGGTCTAATTCGCGGTCAATGTCA
ACGTTTGTGATAAAAACAATCGTTCCTTTAAATTCAAACTCACGCTCAATGCCTTTTTCTTCTAAGTAAG
 
AAGATGCAGTGCTCCAGCAGACTTTACGGGTCTCTCCAGTGTCCAGAGCAGCTTTCAGAAGATTAAGAAT
ATCCATATCAGAGAAAACATCCACATCATCAATCAAAAGGACAGAATTCTCTTCACGATTATTCCAGAGC
TGTTCATAAAGACCGATACCAGAGATTTTACCGTTAATGCTTTTATATTCAATGTATCCAATATCATTTG
CTTTATTTAAAGCTTTATCTAAAGAATACGTTTTACCAATACCTGCTGCTCCCGAGATAATTAATGAACG
AATGTTTCCGTTAATAATACCATTCGTCATCATTCCCATAACATTAAATCTTTTATTAATGCGGGTTTTC
ATATCTTCATATGATTCTTTAACTTCTTCAACTTTTACACCATCATATGAAATGTCTGATTTGTAAACCC
AAACACCGCGACGTTTACCGTCAATTTCAACAAAAACTTTACCATCTCCTTGTGCATCTACCGGAGCATT
ATCTGGGAACCATTCACCTAAGAGCTCAAAAGTTCCAGAGATTTCTTTACCGAAGTAGATACCCTTATTG
ATAGTTACAGTTTTCATTTTATTCTCCAAATCCGTATCAGTTGATAGTTGTATAGTACCATAAAGCTTTA
TGCTTGTAAACCATTTTGTGAAAAATTTTTGAAATAAAAAAGGGGACCTCTAGGGCCCCCTTTATGCTTT
TATTCCATTTCTATTATATGCATGCTCTTTTTGCTATCAAAATATATGTTTTTAACTGTTAACTCCGAAT
TAATTGGCAATAAAAACTCTCTTTCATAATCCACCATACAAAGATTATCATAAAGCTTATTAAGCTTATT
ATCATCAGTCTGGCTAGATTCTTCAATTGCGATTAAATTTTCTAAAATTTCAATCATAGCAGTTGAATAG
TCAAAAACGTTTGCATTTTTTAACACTATTAAAACATTAGTCATATAGCGTTCTGCTGCAAATGTTTCAG
CTATATGAGATTCACTACTAAAGGAAGTTATTCTTTTAAATTTGATTCTCCTATTAACTTCAAACTTATA
AGATAGTACTTCCTCTATTGTAAGTCCTCTAAAAAGTACGTCTTTAATCTCAAGTTTGTCTTTAATGTAA
TCATTAACACGCTTATGATTAAAATCATTAATTTTGTTGCCAAGGCATTTCATTAAAATATGCATTGTGT
TTCTCTTCAATTTTTAAAGAACAATTAATTAACTACAAATATATTATAATATGCTTTATGATAGAAGTAA
ACAACTTTGTGAAAAAATTTTTGAAATAAAAGGGGAGCCCGAAGGCTCCCTATCATTTATAATAACTTCG
GTGGTTTTCAAGATAAACCTTCTCAAGGAAGTCATCCCAGAAACCCATGTCTACTTTTTGCTGCATACCG
TTCTTAGAAGCTTCAGTAGATGCTGCTTCTACTTGATCGACCACATCTTCCAAAAACTCTTGAACCGTTT
TAAATGGATGTTTACCCAACTTCACGTCGAGAATAAATGGAGCGTCTTGAAGTGGATAAACCAAGTCACC
AGTTTTGTAAATTTCCAATAGTTGAAGTCCACCACGACAAGCATGGCTCAGAGCTTTCCAGTCAATGCCT
TCGTTGGCTTCGGCCTTACGGGCACGTTCACCATATTCAGCATCTAATTTGTTCAGTGACTGCTTAAGCT
CAATAAGAGAAAGCGTTGTCTGATATTTACGACCCAATACAGTGTAGAACGTTTGTGGGCCTGTTTTCTC
ATGATTATGGAACACCCATTCACAGAATTCGTTTTCTGGAAGACGATGCTTAATATCTTCAACTTTAGTA
CGACGCTGCTTAATAGAACCATCTTCTTGGTAATCAACCCATTGCTCGGGGATTTGATTAACTACTTTCA
ATACATCACGTAATGCAGCCAAACGAGAACCCTTGACACCGTATTTAGAAGCTTGCTTACGGACATATCC
TAAATATGATTTCATGTTAGTCGTATAAAAACGAGAACGGTTGTCTTGAATAAACTTCCACACATCAGGC
AAATCAGATTTAACCACTAGTTCAGGTTGAGTGTGAAGCATATCTAACGCTACAGTTTCACCATCTGCTG
CTAATTTAAAGAAATATTTAAGACTGTATAGTTCATGGTCAATATCATCTTTAGTGTTTTTAGATGATGT
GTTGTTAGTGTTTTTGCTCATGTGCTCTTTGACATTTCCGATAAGAATATCGCGAGCAGGAGGAACAAAA
ATTTCTTTAAAATCTACATCAGATTCTGGGGTAGAAGTTCCATAAAGATGACTACCAAAATAGCTTTTCA
TTACTGTTTTCATCATTCAGCCTTATATTCAATAACAGGACATACTTTAGCTTTACGAACTTTTAAAAAT
TCAACGATAATCGATTTCTTAGGTTGAATAGGAGGTAAACCTTTATACGCCCTATCAATATTTTCTACGT
GTAAAGCATAATCCTTTTTCCATTTATAATCTTTATATTTTTCACGCAAATGTGCAGGAATAACAAATGA
ACCGATAATAAGTAAAACAACCAAAAGCGCAAGTATAAATGCCGATGGAAGAAGAGTCCATAAACTACCG
AAAATAAGCCAACGTTGTAGGCTTTCTGCACAAATAATTGTTAGCCCTACGAGTATAGCACACCCAAAAA
TAGAAACGAAAAATGTAGGAGGCAATAGACCAAAAATATGCGCATAGTGTCTTAGGTCGTTGCCATTCAT
CGTTAAACAGTTTGAATAATTTATAGTGCCAAGAGTTTTCATTAATAATCATAATTATCCTTTCATTGAA
GGCGTAACGGTTGTTAAATACTTAATCATAGCTTCAGCTTCTGATTTTGATAATGAAATTTCTCCATTGT
TTTGACGAATAGAAATAAAATCCGGATGACGGTCACCACCAGCTTTTAAAACACATATAAGTATCAACTT
CGCCTTCAATATTTGAAATGATTGAAGCAGTTCCACATGAATGCGGTTTTTGATAAATCAATACAGCTTT
TCTTCCACCGTTTGACTGTTCATAGGCTTTTAATACATCAAGCAAATCAGTTCTTTTATCAATAGCTAAA
 
ATGTTATTTTCATCTTCAATGTTAAATCCTTCGGATACTTCATAAAGCGTTACCCATAAATCACCAATGT
CAAAACAGCATTTACTTTTCCACGGCTTTCAAGAGTATGACAAATTTTCTTGATTTCGTCATAACTCATT
TTAATAATATCATCTCCTTGACTAAGGAAGATGTCATTTTTAAATTCAACACGAATTAAACCACTTCCAC
TTGAAATAAACATATTTCCTCACTTTGAAATCATAGTTGGAATAACAGAATCAAGATAAGTCTTTAGTGC
AATAGCTTCGTCTTTGTTTAATGTAATGATATGCGATCGAAAATCGTCAATTTGACGAATAGATACTACA
TCTCCCTCTTCATAGCATTTTGAAACATTTAAAATAGTTTCATCATTCTGATTACAGGAGTTAGTAATAA
TAGCATTACATTTTTAAACCATTTTAAATTATGTTTTCTTTTAGTAGAATCATAAAAATATTTAATGTTA
GTTATAAAATTATCCCAATTATTAATTGATAAGCACATTGACTCGCTTTTAATATTAAATCCTGGGCATG
AAGAATAAAAATGAATTTTATGCTCATCATTAATGCTTACAATTTTATCAGTGTAAGCATATTCAATTTG
GGTTAAACGAACAATTTCACTAGGCGTAATATAACATATCATCTTCTTGCGTCAAACGATACATGTTATT
TACTTTTTCGATCGCTAATTCACCAAAAAGCACACTATCTTCTAATACTAATCGTTTCATATTGATTCTC
TTCGACTCACTCATAATTACATACTCCTCTGAATCATATCAATAATGTTATTCACCAAATTATAAGTAAA
CATTGGGTAATTATATTGAATCATCACATACACAACAAACAAAACTTTCATTCTCTTCTCCTTGGCAGTT
GACAAGATTACTATACCATAACCTTGCCAACTTGTAAACCATTAAATGACGTTTTCGATAAAATTCTGAA
GCTTTGTATGAGCATCAACCATGATTTTCATTTCTTCTTTGGCGAAAGCTATACCTCTTTCACGAGAAGA
ACATTCATAGTCAGAAACTGCATTAGCATATTCTTCAATTAGCTTCATTAAAAACATCTGTTTTTCAGTT
TTCATTATTCCACCTAATCATTTCAAGATATTGAACTAACTTAGCTTTAGATTTATCCAAATCCCTTTTA
GCTGCTTCTATACCGTCGTATGAATATCCTTCACAATGCTCAACTGCTAATTGATATGAATCTATTTCAA
TATCACGTGCTAATTTAATGATTTTTTCAAACTGTTCACGTGTTAGCATAATTAAACTCTCGTATTATGA
TCGATAATTTCATCAAGAAACATATCTAACGCTTCTAAAGCATTATCAACTTTAGCTTCAAGTTCTTCAA
TACCTGATAAGGCAAAGTTAATGCGTTCCTCATCTGCTTTACGAATTAAAGCTACCAATTCCTTAATTTT
ATCCGCTTGTTCGATACTAATCATTATTCCACCACATATGAAAGAGAGAATATTGCACACGCCATGTGAG
TTGCAACTTCATCACACATATTATAACGTTTCTTGAGAAGTTCTACAAGTTCTTCACTAGTAACTTCATC
CATGTCGACGAAAAAATCACCATTAATGATGACGTAGATATTTCCTTCTTGATTAAGTGCTTCAATTTTC
ATGATGTTCTCCTCTTTATCCGATAGTTGTATAGTACCACAGCTCAAACGGAAAGTAAACCGGTAAAATG
AAAAAAGTCTCCCGAAGGAGACTAATGTTATTCGAGGGAAAGAAGATACTTACTCTGGTAAAACATTCCA
GTAATATCATCTATCGTGCTTTGGATGGCTGGAGGCATTTCTTTATAAATGCTGTTAGATTGGTCTAGTA
TGCGATCAATCATTTTAATTGTGTCGGTAGGAAGTTTACTGGCATCTGGAATTGAAGGTGTGTATTTTCT
ACCAGAATACCCCAAATATTGCTCACAAAATTTATCAATCAAATCTGGCAACTCGGAAAAAATAAAATCG
TATGCTTTGTGTCTAGCATAACTTTTAGTTTCAAAATGTGCAGAATGAAAATAAGCTTGTGCAGCCATTA
ATAAACCTAAGTATTCATCTGCCTTTGAAGGTTTTCCACTTTGTGAAAAGTCGCTGAATTTCATTCAGTC
TCCAATTTAATTTTCATAATTCTAGCGTATGATTGTGCCATCTCCGCGCCTCGCTCTATACATTCAAAAT
CAGAAGAGCACGGGTCATTTTTATAGGTCGTTCTCATAAAACTATAGAATTGTTCAGACGATTCTACGCT
TTTATTTTCAAAAAGTGTATAAACGTGCCTAATACCAGATTCCATAAATTTATCAAAATGAGGATCGACA
TTCGCTTCAATTGATGGAGATAAAACAAATGACAATCCTAGCATGGCAAATAGTACCGTTGCTTTTAAGG
CCATAAAGGCCTCCTATCATTTTTGTCCTGTATTTACTTTATGCCGATGCACGGCCTTAACTTTATCAAG
GTATTTTTCAAAATTTCGCAATCTAGTATAGTCTGCCGGAGATTGGTTAAGTGATACTTCTCGACGCAAA
GCTGAAATGATATTTCCAACTTCCCTACGAATTTCATCTAATTGAAGCACAGTAAGATTGCGAAGTTGCT
TTTCAGTTAATTGTAGCATATATACCCCTTTAGTTAGATAAACCTATTTATAACTTTTGCACTAACCGAG
CTTTTTAGTTAATTCATTCCAATGTTTTCTACACAAAGAAACATAAATTTCATCACCAATACAGATTTGA
TTACCTTCTTTAACTGGTGTTCCATCTTCCATTAATCGAGCTGTCATAATCGCTTTTTTACCGCAATGAC
AAACTGCCTTTAGTTCAATAAGTTTATCTGCAATCGCTAAAAGTTCTTTAGAACCTTCAAACAATTTTCC
AGCGAAATCAGTCCTTAATCCATAAGCCATAACAGGAACATTATATGTATCAACAATTCGGCTCAATTGA
TGCACCTGTTCAGTTTTTAAAAACTGAGCTTCATCTACAAATACGCAATGAATATCTTTTTGTGCTTCAG
 
CCCATTTATAGAACTCGAAAATATCCATATCATCTGTAATAATATTCGCTTCCTGCTTAATTCCAATGCG
AGAAACGACTTCACAGACAGAATCGCGAGTATCAATAGCAGGCTTAAGAACTAATACACTCATTCCACGT
TCTTTATAATTATGTGCAGCAGTCAAAAGAGAAGCAGATTTTCCAGCATTCATTGCTGCATAAGTAAAAA
TTAAACTCGCCATTTTAGTCCTTAGTTAAATTTTCTAAATATGTTTCTAAATCATTTTCAGCTTTATCGA
TAGATTTTACTAATTCGTAATATGTTTCGGCATCTCCATAATCAGAAGATAATTCAAAAGACAAATCCTT
TTCTAAACTAATAATTTCACCAACTAAAAATAATATTTCGTTCTTTTGTTCGCGAGTAATCATAAGGAAT
TTATATAATTAATAAGTTCTTGTTCTTTATTATCGAATTCTTTAGAAAGTTCTTCGTACTCGTTTGCGCT
AAAAGGACCGCATTCATTACAAACTTTTTCCAATTCACTATTTTTATCCATAACTTCGTGGATAAGAGAA
AAGAGTTTGTCTTTTTGTTCTTTGCTTAAACTCATATATTAATTTCCAGAAGTTTTAAAATAATTCGCTT
TTGCTTAAATATTTCCACAGTTAAATGATCAGTATCTTTTTGACCAATTCCAATGGCAAAACCTTTATTA
ACAGCGAGTTCAATTAAATTATCAAGTTCACGTAACACTTCATATTGTAACTCAGAACTATTCATTTCGG
TTTACCTTCTTTAACGAAATCTGGATAAGGATAAAATCCCGAATAAAATCCTTCACCTTTATGATTGATG
CATCCTTTATTAGAACAATAGCACCAATAATCGAAATCACAAACCATTTCATCGTTGCATAAAGCAAAAA
CAACCGGCCATTTACACTTTTCGCAGTGGGCATTTTTAAGAATACTAGTTTGGCTCATAACCATGTCACC
TTTAAGCAGTATTCTTCTACATGCTGTTTACGACCTTTCTTATCAATAAAGGTATATTCAACGAATGTTC
CAATGTAGTCTTTATCTACATCATGTGGACTATTAATTGGGCATTTAGTACGACAAATGCGTTCCCATTG
ACGAATAATTACTGCCTTATTATTTGGGTCATATGGATGTGGATAATGTATATTCATAATAATGGTTCCC
AATCAACAATCACAATTTCTAATTTAGAGGAATATGTATCTAAAATCCCCTCAATAATATCCCAGTTCCC
TTTACCTATGCCTGCGCCAATCCTAGGCATATAGATTGTAGGTTTAATCAGTTTATTTTCACCAAACTCA
TTTAATTCTAACATACAATTCATTAAAGCGGAATACTCAAAATTTGGCCCTGGTTGAAATTGAGTATAAA
GATTAAAGCAGTAAGCTTTATGAGTCCTAAAGTATTTTTCATAGACTGAGTAAGAACCGAGTTTAGTTAC
ATCACCCCATTCAGTCTGTAATTTATCAGCTTCCAAAATTTTAGGGAAAGCTTTGGTTAATTGACCCGCT
ACGCCTGAACCCATAGTATGAAAACAATTACATCCATGCGCAATATTTTTACCTTCAGCGAAAAGGGCGA
CAATATCGCCCTTGATATATTTTACAATCATCTAGTACTCAATCCTCGATTATAAGAATCTACCAAACGG
TCAACCATTGAATGACAAGCGGCTTTATCTTTCTCCTCCGCAACTGAACATTCTAAGGTATTCCACTTTT
TAGCATATCGTTTTAATAATGTATCGTTTTTGTATCTGCTTGATTTATCTCTTTCTCCGTCTTTATATGC
ATATATTAATTTCTGAGCAAATTCCGCCTGGCATGCTTTATTTTTCCCACAATAATCTGCCGCAGTGCGG
TTTACATATTCTCTAATTTCAGTATATGATGTATCTGCTGACGCAGAAGCAGAAAATGAAATTAATCCTA
TACATAAAACCAAAATTTTAGTCATTTACTATTTCCAAAAGTTTATTATTTTTAAGGTAATTAGCCTTTT
CTAGGACTTCAGAAGCATATTTAGAACCTGCTTTAACATTCCATCCCGAATTATAAGAGGATATTGCTTT
TCTTATATCGCCCTTATGTATATTTAACCAATAAGAAAGTTCAATGTACGCCCAGGAAGCTGAATTGGAT
CGTTTATTCAACATTCTTTTTATTTCAGCATCGGTCATATTATAACCAAGTTCCTTAACTCTTGCTCGCA
TAGTAGGCAAATAATTTTGGAACATTCCGTAGGCGTGATGATTTGGTTTAGATTTTAAATTAACTCCGCC
AGAGCTTTCTTGCCATAAAATGGCTGCCATTATATGACCTAATCCGCTCTTGTGGATGTTTTTGTGTGTT
TTATATTTTCCATCCTTAGAAAATTGTTCCCCGAATTGATACGCGTAACGCATGTTATCGAGTTGGACAT
TACTGAAAGTATGCTCGGAGCTATGTGCCATCATTGAAATGGCCAATAGACCAGCGAGTAGTGCTTTTCT
CATGCTTACCTCATTGAGTTTTAATTACTGCTTTAGAAGCCTTTCCTGGTAAACGACGACTGTTAATAAT
TGCCATTCTACATTGAAGTGACGGGTCTTTGAACTTCGCATTAGGTTTACAAACTGTAAATCCAAGCCAA
AGATTTCCATCTGTGATTTCTAAACGTCCAGGACGATATTCAATTCCATCAATAAAATCCTCGTCAATGT
CAGGACGCGGAGGCATACTCAGGAATTCATTAACTTCTAAAACATGGTCTTTTATTTTATGGAATAATTC
AAAAACGTATGTCTCATCAATCTCCCGTTGAATTGCGCGATCAAGAAGATGTTGAGAATATTTTAGATGA
AACGATGAGACCCCTGCTGCTTTTGATGCCTCACGAATCTCATTGTTAATTTGACGAAACTCCGACTCAA
AGTGACGACGAAGCTTATTTCGACGGATAAAAACTTCTGTATTGATAGTCATGTTGTTCTCCTCTTAACT
GATAGAAAAATTATACCACAGTCAAGAGGAAAAGTAAACAGTTATTCTTTAAATCTAATCAATTTATTCA
 
TAGACTTTGAAACTTCTGCACGAACCTCATGTAGATTTTTGAGCTGTTCAAGACGCTGCGTATAGTAAGC
AATTTCATCTTCTTCGAGACAGTCCTGCGAATCTTCTTTAAGATACCGTGCATAGTCCTGGAAAGCGTTA
CGGACTACTTCCTGGAAGTCATCAAGACTTTGAATTTTCTTAGGAGCAACAGATACACGACGAGGTGCAG
TATAATACTCATAACCAAACCCTGCGCTTAATTGATCCATTAGTATTTTTCCTCTGGTTGGAACACTGCA
CGACAAGCCCACATACTGGCTTCTTTGAGTTTCGTTTTAGCAATAGTTAACTGATCGAGGCTTTCAGCAT
AATTCTTCGCGAATTCACAGTCTTCACAATTATCTAGTGCTTCCCAGAATTCATCATATAAAGCATCAAA
GATAAGTCCTAAACGAACTTCAGCGTCTTTGATAGCATTCACTTTACCGATTTTCTCTTCAGTATGTGGT
TTATCCCTTAATATCTTCAATCATATTTGACTTCCTCACCAGTACATAAATCATATTCAACTAAACGAAT
AGGTTCATGAATGCCATATCCCTGAACAGAAACTTCTGTCGTAGGATAAATTCCATCAATATCACCCATA
TTCCAGGCTTCATTAAATTGCTGTTCGCCTGAATTACTAAACCACTCGGCGAAAGCATTTATCACATCTT
TAGAACCTTCAATAATTATCTTTGCCATTACAAACTCTCGGTAAAGGTACGAGCGATAACGTCGCGCTGC
TGTTCCGGAGTCAGAGAGTTAAAGCGAACTGCATAACCGGATACACGGATGGTCAGCTGCGGATATTTTT
CCGGATGCTTAACTGCATCTTCCAGAGTTTCACGACGCAGAACGTTAACGTTCAGGTGTTGACCACCTTC
AATTTTAACTGTAGGTTGTGGCTCAATTTCAATTTCACGGGCATGCAAACCATAGAAAATTTCTGGGTCT
ACAAAAGAGTCCTCTTTAAAGGTTTTAGAGACAATAATTCGTGCTTGAATACCATCTTCAAAATAAATAG
TACCTTTATGTGTGCCTTCAAGAATTTGATATGCTTTCATATAAACCTCAATTAGAAAATAAATTTATCC
AAGATTGTTCTTTAATTAAAAATGGCTCAGAATCATATGCCATTAAACTGTGGGTAATTAATCCTTTAAA
AGGTCCATCAATAAATTCCATGGTAAAATATGGAATTTTATTCATTAGCCGTGCATTAGGAGCAGTACAC
AAAACTCTGCATCCTTTGAATACGCCTTTTTGTAATTTGTATTGCTTAGGATAAAATTCGCTTAAAATGT
TATTTTTTGCCAAAATTTCAAAATGATTCACCAATTTATTTTTAATAGTTTTTGGCGAAAAATAAAGATA
TTCGAAAAGCTGAGTGTCTGTCATCATTGCATTCCGATTACGAAAAACTGTGGACGAGTAATACCACCAA
TACAACATTTAATATTACAGCAGCAGTGTACAGTGTCAATATGGACACTATAAATCTTATCCATATCAGG
AGATTTGACAGGCTCATCAATTATATACAAAATTCGCGAAAGCGATAAACCTCTGAACTTGCTTCCTTTA
TTACCAATAACTGCGCACAGAATCAGTAAATAAACGAAAACGTATATCATCATTAGAATAACGCGAAAAT
TCCTTTTTGATGTTATTTGCAGAAATTTTAGCGTAAGCTGAAGTATTAGAAAGAACAATAACTGTTCCGC
CATCATACAACCAATTAGCAGCAAAGTTAGTCACAGCAATTGATTTACCAGATTGACGTCCACCATCTAG
TCGAAGTGTGCAATACTGTTTAAGTAAGTCTTCAAATGGCGGGATATTCGTTTTTACAAATTTCTTCTAC
TCTAGCATCAGAATGGTGTGTAAAAGCATTCATCAGGGATAGATAAGGACCAGTTAAAAATGTTCTCATT
TCTTCTCTCTAAATTTGGGCCATTCCATGGCGCATGAATTGTCCATTTCTGTATTTACCCATTACCGCGC
TTGGGCTCGACATTATTACAGGTTGGCGGGAATCCCTCACAGAATCATGAGGTCCAGGTTGTTCCCATGT
TATTTGAACGATTTAACGTACTGAATAGAACCTTTACTATTCACAAAGGCAATTTTACCATTTTTGTTAT
ACATGGTAGCAGTACGTTGATAGCCATAAGTTTGTTTACCAGATTGATAACCAACTAATGCGAAATTATC
TTCACCCATTTTATTCTGTTTAGCGCGAATGCGTTCATGACCATGTGCCATAAGAACCTTAGCATCACCA
GTTGCAATGGATCGACGAGTTTTTTCATCTTCAACTGTATTAACAAAGTCGTCAAATGAATCAAATTTAG
CATGAGATTTATTATACAGTTTTCCATCTACTGCATAATTATATCCCTGTACATCCATCTCAACTTCTGT
ACCATCATCCAATACAGCAGTGATTTTCTGACCTTTTTTAAATTTCAAAAGATCAGTAAAAGATTCAGCA
ATAATTTGAGAATTAATAGCTGCTTCAGTAATAAATTCTTGATAGGTTTTCATGTGTTTCCTTTAAATAT
TTTAATTAGTAATTGTCTATTCAAGTAACTGTGAATATACTATCACAATTCCAAGAGAAAGTAGACAATT
TTATAGATTTTTATACGCGTCACAAGTGCCAGTTCTAAACGTTGCAATGACTCGTTTTGCGCGATTAGGT
GTTTGATTATACCATCTACTTTTAGCTAAGTTAACTGCTGCTTCATCCCAGCGTTTTTGTTGAAGCATAC
GTAAAGAGTTAGTAAATCCTGCCACACCGGTTTCTCCCATTTGGAAGACCATATTAATCAATGCACAACG
ACGAACCGCATCAAGAGAATCATAAACCGGTTTTAATTTAGCATTTCTCAGAATTCCGCGAACAGCAGCA
TCAACATCCTGATTAAAGAGTTTTTCAGCCTCGTCTTTGGTAATTACACCATTGCAATTACGTCCAATAG
CTTTATCTAATTCAGATTTAGCAACACTAAGTGATGGACTTTTTGTAAGCAAATGACCGATGCCAATAGT
 
GTAATAGCCTTCTGTGTCTTTATAGATTTTAAGTCTAAGACCTTCATCTATACGCAACATTTCAAATATA
TTCATAATACCTCCTAAGTATTTATAGAAGGTATTTATAAAATTAAAGAGGTTGTTCATTATTCGGTAAA
GTGAAGGACCCATCACATATTGCCACTGAGTACGAGGAATAAGAGCAAAAGCGTCCATCTCTGGAATCAT
AACGCCATCTTTATTTTCAAAATAAGACTCGCAACGGCAATTTCTGAACATCTCATGCTCTACTGGAATC
GTATAATAAAACAACTGTAGGTCTTTATTACTAGAATATTTAAATACACCTAGGTCTTCTAGAAGGTCTG
GATTATAATTGCCAAAACCAGTCTCTTCTAAACATTCTCTTCGTGCTGCATCTAATGCGCTTAAATCAGA
ATTTTCTACACGGCCCTTTGGAATATCCCAGCGATGTGCCATCATTCCAGTCTTACGAGAACCAGTAACC
CGACCCATAAATAAATCTTTATCTTCGGTCATAAAGATAATACCAGCTGATAGTGTTTTCATTTTAATTT
CCTGCAATCACTCACAAACTCTTTCATTGACTCATTTTCAATATAAGACATGTAGCTATTATATTCCTTT
AATTGTATTTTGTAATCCTTTTTTCTTTGCCAATTTATTTTAAAATTATCATAATGAAAATATAAAATGA
TACCAAAGAATGAAAACAATGAAATAATTTTAGTATAACAAAGCTCACTCCAAACTTCTGTTATATTTAC
TGTGCCGTCGATGTTTAAAATAAGACAGTCAATTAACAATCCAAAAAGACTACCCGCAAGAGCTGCAGCC
AACGCCACAGCAAAAATTAAAAATGACTCAGAAAACGAATATTTGACTTTATTTAGTTTTGGCTTTTGCA
TCGTGATTCCTTAACAAATTTCATAATTTCATTAAATTCATACTCAGCTAGTTTAAGCTGGTGTTCCTTT
TTAATCTTTTTACAATGAGCTTTCCAATCGTGTACACGTTTACGATAATGTCTTCCTTGATACCAGTATC
CTACCCAATTTACAGGTACTAATAAAAATGGAACTACCAATGGAAGAGTTAGCATTAACATAATTATTAC
GCCAGATTCAATATCAGTCATAACATCTAAAACACCTCCAATAATCAATAGAATTACAAATGATATGAGT
ATCACAGGACCTATTAATACGTCAGTAGGAATTATCTGGCGCTTTAATTCATACTTCAAAGGTTTACTTG
GAAGGTATAGTGATGGCTTTGACATATTCTCTACATTCCTTAACAAATTTTTCTAGTAATAAATCACTTT
CAAAATTAGGATTTTTCACTAATTTATCAAAAAGACCATCAACAATAGTTAAAATATTCTTTTTGCTAAG
AATACGTTTATTTTCATGCTTCGTTTCAGAGTCAACTATAAGAGTAAAGAAATATTTCTTTCCCTGAAAT
TTTACCGTAGTATCAATATAAAATAAATTTGACTTTTGTAAGTTACGTTTAAACCATGCGTCACTTAAAC
TATAAACACCGAGATAATCAAAATCGTCGTTTAAATAACAAACTGACCATTCAGGAGAAATGAAATCAGT
AAATTCAACATCAAAATCACATGTCAATAAATGAATTGATTCAATATTGTTAATAAGTATTCCAGGACGT
ATTAAAGACTTTTTACCTCTGGAAAATCTTCCAGAAAGACTTTCATCAGTTTCATATGAAGAACCCCAAT
AATAATTACGTCCTTCTGCCATACGTTTAAGAGCATTTAGTAATTGGTCTGGAACATCAACCTGTCTTTG
GAACTCTTCAAACATTGAATTGAAATCACTTTGCATTTTCATTCCTATTTACTCCAAGTAATAGGGGGCC
GAAGCCCCTTATCATTATTTCAGAGAATTAATATATTCCTGAACATCGGCAGAGGTAGTTTCAACCCCAG
AAATATTGCCATTAAAGGTTTCAACTCGAGCAAGAGTATCTTCAATATCAACCTTAGTCAGTGCTGCAAT
TTCAACTACATCATCAGCAGTACTAATTCCAAGGGCATTTGCTGCACGAGTTTCACGGATATATTCCAAT
TTAACTGCAAGTTCTTGGCGAGCATCATCTAACTCAACTACTTTCTTAGCGATTTCAATTCGCATTTCAG
CATAACCATCAGCTTTAGTAGTCAGCTGTTCAGCTGTTCGACGATATAGCAAACCGAGTTTAGCATGCAT
TGTTACATCTTGACCTTCGGAAAGAAGCTTGCGAATTTCACGCTCTTTTGATTCAGCCTGTTTATTCTTT
TCAACAATAAGTTCACGAATACGTTTTTCTTCATTAATAGATTTAACAGAAGCAGTTTTTAGGTCTTTAA
TTTTATCAAGCAGTTTTGCTGCTGCGGCAGTATACTGTTCTTCAACAGATAGATTTTTAGCCATAGCAGA
ACCAAGTTTAGTGCGAATAAACTCAACAATTTTCTTCAGTGTGTTCATAGTATTTCCTTAGGTTAATTAA
GATTTAAAATCCATGAGAGTATTATACTCCGGATAGTTTGTAAACTATTTCAACATCGCCTTTTGCTGCT
TTATAACCTTCAGCCCAAGATACAACCGAGTTTTTATCTGAAGATTCAAATTCTGGCATTGGGCTTTCGC
CAATGTTCACGCAATATGTTTCTTCATTTTCGTCGAACCAAATAATTAAAGTAGTCATTTATTTCTCCGT
TTCTGTATTTGTTTTTGATAAGTCTATAATAACACCGTCCTTGGTGTTTGTACACAATTATTTCATATTA
TTGAAATATTCTTCTGCGATTTCGTCGTTATCATGGTAAACTTTAGAAGACAGTTTAACATAACCTTCAG
CAGTGAACATGTTAATCACGACCTTTACAGTATACCACTGACCATCTTCATTACCCATTACTGCGTAAGT
TTCAAACATCGGATGGTCAGGACCGATAACTTTAATATCATTCACTGTACGACCGAAATCTTCTGAAACA
CATTTCATAAAGAAGTTGAACAGTTCACCGTAATTATCCATTTTATTCTCCAAGTTGTTTTCTGTATCAG
 
TAGTTGATAGTTGTATAGTACCATGGAAGAACAGGGATGTAAACAGTTTTGTGAAAAAATTTTTAAAAAG
TTTTAGGGAATTCTAGGGCGGGGAGGGGCAATTAAAAGATAGGATAATATATTATAAAGGGTATAAACTA
AATGATGCCTAGAGAGGTCTGGAAAGGCCTAGATACCAAAAAGCCCCAACCTTTCGGTCGGGGCTAAGCC
TTGCGGCAACCTTGTCGGGGTTCCACCTGCTAAGGCAAGTGTTTGTACGAAACGCCGGGATTTGAACCCG
GTTATTAAGTAGTTGACGCTACTCAATATTTTTAAAAGGCCATATCTCGACCATATCCGAACGTTCCGTC
AAAAACGCTACTCGGCTTACAGCAAAGATATTTCCTCGAATCGATAATTCGGTGCGCCGTTTCTGCTGTG
ATAGTAAAGAGCCAGAACACAGTAAAACTGTGGGAGGAACTATACTCCAGGGAACATCAGTCCGACGACT
TACCGGTAGCGACCCGGTTTCTTAATATTCTTTTAAAGCATCAATCTGTTCACGGCGTTTACGCCATAAA
TCAATTGCTTCATACGCGGATTCAGCATTTCGAATTAGCCGGCATTTATGTAAAGACTTGTGATTTTGGT
ATTGTGTAGATAATGGAATTTTATCTAAAAGGTCTTTACGTTCAAAGTATTCGTAACCATCTAAACCAAA
TGAACGATTTTTGAGAAATTGCCAATGCCTAAATTCGTTTTCGACATAAACATAGTCTGCATGCTGATAA
AGCAAATTAATTACACGTTCAGAAATAACAGTGTCATTATGATTAAAATAGAATGTTAAATCATGAACGA
CTAAATAAACATTACCTTTCATATTTTCCTCACTTATAATTGGTCGAGGCAGTAGGGATCGAACCTACGA
CCCAGGACTTAGAAGGTCCTTGCTCTTCCTTCTGAGCTATGCCTCGTATTTGGCGGACGTGATAGGATTC
GAACCTATAACCAATCGCTTAACAGGCGATCGCTCTGCCATTGAGCTACACATCCAAATTGGTGGGGAGT
GATGGAGTCGAACCACCCGAGTCGCAATGACAATGAATTTACAGTCCACACCGCTACCTCTACGGGATAA
CTCCCCCAAATTAATTTGGTGGCCCTGGGTGGAATTGAACCACCATCTGGCGATTATGAGTCGCTTGCTT
GAACCTTCCAGCTACAGGGCCAAATTTTCTTATTTTATGTAATATTTATTTAAAAACTTTCTAGCTTGAG
TATGGCTAACATTTATAGCGTTTGCATAATTTTTAATAAAACCTCGTTCATCCATATTAAAATTTTCTAA
TATTTTTAATCGTCTAGTTATCTCATCTTTGCTTAAGGTACTTGCATTATTTTTAGAAATTTTTTCTAAG
GACTGTGCCGAGTGTTTTCGACCATTAAAATTAAAATTTTTAGATTTTGGAGGAAATCTATCATTTAGTC
TCGCATCTTTTGTATTTTGATTATTAGTTGCGCAATATATGTTATTTAATGAATATGGTCCTTTATCACC
GAAACGGCACATGCAGTATTCATGACCCTTTCTTCCACGCTTATCATATTTTCCCGTTGATTTCCACCAA
TAGCCATTCATCAAAAGTTAAATTCCATTCAATGTTTCTACGTATAGCAGAATTCCGCTGAGAAATATTT
TGCATAATCTTCTTTCATCACAAATTTCTCTACTTATTCAATACACCGAGCTAAATCAGCAAAACTGGCG
GAGGCGATAGGATTTGAACCTATGAGTCGCCGGAGCGACTGCCGGTTTTCAAGACCGGTGCATTAAACCA
CTCTGCCACGCCTCCAGTCTCCATACAAGGATTTGAACCTTGGACCTCCTGATCCCAAATCAGGCGCTCT
ACCAAACTGAGCTACACGGAGTAAATTAAATTGGAGCGGATAATGAGAATCGAACTCACATCATCAGATT
GGAAGTCTGAGGTAATACCATTATACGATATCCGCAAATTTGGTGCGAGAAGTGGGACTCGAACCCACAA
GGAAATCATTCCGCAGCATTTTAAGTGCTGTGCCTTTACCAATTTGACCATTCTCGCGCTGGGAATAAAG
GACTCGAACCTTTGCATCTAGCAGTCAAAGTTCTATGCCTTACCAACTTGGCTAATTCCCAATTATTAAC
AAAGGCTCTCAAATAAGAACCCTTGATGATAGAGGGTATTAATCAATGCGATATGAGTTAATAATAACAA
ATATTTCTTAAAGCAAATCAACCATTTTAACAGTCGGCAAAACAATTTCTTCTTCGATATAAGCAAATTC
TACATGTTCAACTACACGCAAGAAATCATATTCATAGTATTCAGAAGCATGACCAAGAATATCAATGAGG
CCTTCAAATATCTTATTACCATTCACAAAGAATAAGTCAAATACACGTTCTTCATCTTCTTCGGATTTAT
TACCAAGGTCAACTTCTATATCAAAGACCTTTTTAATGAATTCTGGATAAAGTTGGTCTGAGAACAGTCC
TTCAACCAAATATTCATATATAAAAAGCTCTTGCTCGCTGAACAACCCATTACCACAATAACGGTCAGCT
TTAGTGCTAGAATGACGACTCTTAAACTTCTTCAAAAGATATTTAAATTGTTGGAGCTCATGCTCTTCTA
CGCCAAACAGTGTTTTGGTTTTATAGTTATCACCATCATTTTCCCAGGTAGTGACATCAATTACGTAACC
CTTAGGAATTGTGCTGCCTAAACGAATATACATTTGTTCACCATATTAATGTAAGTATATTTAATACATT
CAAGACCCAAAGGATTCTTGAAAATATCATATTCAAGAGGACCTTTTTCTGTTTCAATAAAGAAATCAAA
ATTTACTGTATTAAATTTACGGTCTTCCTTCACTAATTTAACTTGAGAAGATGAACGGTCAATGTAAACC
TTTTCAACTTCAAAACATGTTAAAATGCCATAATCATCAATCAAGGCTTTAGCCGCATCTTGATCATATT
TATATCCATTTTCAACGGACGATACTTTCGCATAAAGAATCATTATTAACCTTTAGAAATTTTATCCATA
 
ACGATAGCGATTAAACCAATTAAAAATGCTACTACAAGTGAAAACACATTTTCTGCTGTAGTCAATAATC
CGCATATAAATCCAACAAACATTGAAAAACTAAAAGCGGAAGCAGAAATTGCAATAGCAACATTTCGAAT
TAATTCACAACGTTTCATTTTATTCTCCTCAGTAGTTGATAGGGTAATAATATCACCACCCTATCTAAAA
GTAAACAGTTTATAAACTATTTTATTCCGCGTGATGCAATTGTATTCTGCCGTTTAGCACCAGTTTGTTT
AAAGTCATGATTGTCGATTTGATCGGTACGAGCAACTCCGAATGATGATTTGATCTTAGCTTTAGATCCT
TTAGTTATAGAAATAAACATCTCGGAAGCCCCTTTCTTAAAGTCCGAGGCGATTACTTCAACGCCGTTCT
TAGCAAGTGACTTCAGAATTGCCTCTGCATACTCATCGTCAAGACTCGTCTGAGTATTAATAACGAATGT
CTTTGGCGCAGCCGCTTCATTGATAAATTCTTTAAATGTTTTCATTTTATTTTCCTAATTAATTTTGATG
AGGTAATAGTATCACAACTAAAACCCTATGTAAACAACTTTGTGAAATTATTTTAAATCTTCTAATCGTT
TCTTCATCTTAGAACCGTTCTTAGCGATTTCGCCTGCATCAGAGCATAATGCTAAAAGCGCTTTAACTTT
AGTTTGGTCTCCTTTAAAAGATGAAGGATCAACAGCAGCAAGTTCTTTAATACGAGAGAAAAGACGCTCA
GCTTCAGCTAAAGCCATTCCAAGTTTTACTTCCATACCATGGGAAGATTCAGTAATAGTAGTTTTTGTAG
CAAACTCTTTGAAAGTTTTCATTTTATTTTCCTAATTAATTTTGATGAGATAATAGTATCACTACCTCAT
CAGTATGTAAACAACTTTGTGAAATTATTTTAAATCATCTGCCCAATCGAGTTTAAGAGGCTCTTTGTAT
TCACGATCTAATACGACCGGAATTTGTACATCACCGCTAAATGATAGGGGCCCAACATTATAAGACAATG
TTATATGCGGTGTGTAATCATCAAAATCATGTGTAGCACCTAATGCCCGCGCATACATGTGTCGACAGCG
CAGATATTCAGAATCTAGCACAAGTACAAGAGTCGATCCATCTTGTGTTTTCCATACTTCTAAATGTCCA
GAAGAAGCTACTTCAAAACTTCCACTCGATGGAACATATGGAACATTTACTCTTGAATAACATATAGTCG
AATGAATTTTTTCTCTAGGAACTGGATTAGGAACACGTAAAGAGCGCTGGAGTTCTTCCAGCGCATCAAG
TGTTAATTCTGAAAACTTAGCTGCTACATAAAGACCCGTTGAAAAGTCTTTAAATTCCATTATTCTTCAT
CTTTTGCTTCATCTGCAGATTCAGCAGTAAGATTCTTGACAGCTTCAACGATTTCTTCAACTTTGATGGT
ATCGCCAGTGATACCTACTGCACGAGCAATTTCAGCCAAAGTTCCTTGCAGAATTTTGGATTCTTCCATC
AGACGAGCCGCTTGGTCCTGTGTATCAAGAATGCGAGATTTCAGAGTTACGATTTCAGCAGACAGTTTTT
GTTCAATAGTTTGTTCAGACATTATAGTACCTTTAGTGTATTTTTAATTTTAGAAAAAAGTTCTTCAATA
GAACCATCGTTTGTAATTACTAAATCGCCATCACGAATTGGCAATCCAGCTTCTGTAATATGTGTATCAT
TGGATTTTTGACCAGGACGAACTACATGAATTACTGTAGCACCCATCGCCCTAGCCGCATCCATTTCATG
ATCTTGACGGGTATCAGGAACGATATAATAATCATAACCTGAGTTAAATTTATCAAGATAATCTAAAGCA
AATAATTTTACCCAGTACATGCGGTCGAAGTTATTAACAATCAAATCCGTACCTAGGGCTTGCATCAGAC
GACGGACTGACCATTGATCTTCAATATTATTTATAACGTCAGTAATCTTGTTAAATGCTACGAAATTAAC
TGATTCTTTTCCTTCGTCATCAAAAACAAACACACCTTTAATTGGGCTTTTACCATTAAGATAGCAAAAT
GCTTGTTCCATAATCGTGATTACTTCTAATTTAGTCAGATTTAAATTAGTCTCACGATCATAGTCAATTC
CTTCAAACTCTTTACGAGTTAAGCAAGGATAGTCAGTGTTTGCTGCAAATACTCCCCATGCATAAGCCAA
TGCATCCTTAATAGGACCAGCAAGTTGGTATTTAACTGCAGAATAATTGCTCATGATAAAATCAGCAGTA
GTATCTTTTCCACTACGCTTTACACCACTTAAAAAGATTAGTTTCATGTGTTTCTCCTCAAATTTAATTA
AGATTATAACACACAAAACTGAAGCATTAAACTTCTGCTATAATTTTTCCATCTTTTTCTACTTGAAAAT
AGGTGTAAGGAATCGTAGCTGTACATACTAAAGCCGGGTCTGAATCTTCTGTGTAGCTAAATTCTACTTC
AGATAGGTCAGAAACCCAAGGCTTATAAAAATTTATTGACATCACGATTTCAGTTTTGCTATTATCTAAG
ATGTAAAGCGTAATGTACTCAGGACCTGTTTTTTGGGCAGTATTTTCACCTGTAAGATAGTTGCTAGTTC
CTAGCATCCATTCATACATTCCTATCCATGACTTAAGTTCTTCATCAACTATAAATCTCACAATGAGTGG
ATCGTACTCAAATGTAGCACCTGGACGTTGTGCTCGGCCCAGTCCAAACGGCCCAGTCACGGTATCAGTA
ACAGGTATTCTAATTCCTGGAATAGGAACTGACTGAGCATTTAAAGTAAAAGCAGATGTAGTATTACTAT
GTGGTATTGATACTACAAAGTTAGTTGTATTTGCTTGGTTAAAAATTTGTTGCAGAGCTTGCGACATATA
TTCCTCATAATGCTTTATAACTGTTGGTGGTATAATGGGTCTAAGTCCCTTCCATTCAATTCCATTTAGA
ACAAACAACAGAAAAGAATGGAAGATAATAGAATTAGATATTTGACCAGACTTTGTTTGCAGAGAAACGT
 
TTTCCTTTTGAAACGAACTGCTGAAGTGGCATCAACACAACGTTCGCCCAGTCTTTCGGGGCGATTTCAA
CAAGGCTACCCATAATATTACCAGGTATATACGCCTTAATCATTTGATCTGCACCTCTAAATCCTTTCAC
TTGACTCCAATCAATTTTTAATTTCGTTTTATTAGTAATAGTAGGTGTATTTGCATATTGCTTTAAAAGC
TCTTCTAGAAATTGCTGACGAGCTTTAGGTGGAATATAGTGCAAGTTCAATCCGTACATTAAATTATGCT
TACCTAAACCAAGGTAAATTATCAAAGGAAATTTATCCCAATAAGGAAGAGTTTCCTTGTGTTTAGCATC
ATAAGCAAAAGCATATATTCGTCCCGGCTGCGGGCGAACAACTTTATGTCCTTTTACTTGCTTAATAGTT
TCGGCAAACCACTTTCTGGTTTTATTATTAATTGCTGCACCTTCATTACGAATTTTATCACGCAATGTTT
GTCTGAATGAATTTATCATAAGCAGTTGTCTTTCTTGCTTATTGAGTTTATTCATTGGTTTTGATTCAAG
TTTTTGAATCTTTTCAGCCGTTTTAATTCCTGAAGCATATTTTGACATTGCTGAAGTAAACGTAGAGTAT
TTGATTCCTCTTTCTTCAGCAAATTGCTTTCCTGTCATTCCTTTTGCTTTGGCCTTTTTGTATTCAAGAC
CTATCTGAATCCATTTCTTTTCGTTTAATGATTGCTTAACCTTTGGAACTTGGGGAGTGCTTTCATTAAT
TATTTGAAAAATAGCCATTATGCCCCCTTAAAGCCAAGAGCTCGTAATCCATCTTCTGTTAGAATTCTAA
ATTTTATTCCACGCTTTTCAGCTAAAGATTGTGCTGCTTTCCATTTGTCGGTATTAACAGAATATGTATA
AATTTCATTCATAAATCTTTTCTTCGCTGCGGTTGTTAGATGTGCTGGTTTAACCGGTGGTTGTGTTTCT
TTTTTAGGTTTTATTTCAATAAAAAATTCTTGCCCAGAAGAATCTTTCATCCAAATATCCATGAAGTATC
TACGTTTTTTCCCTTCTGCATTACAAAAATAAGGAATTACTGCTGTTTCACTACCCCATGCAATAATTTC
TGGATTTTTATCTAACCATTCAAAAAAGAATTTTTCCCAATTTGATCTATACGTAATTTTTTTAGGGTCA
CCTCTATACTTTGATATATTTTTAGGAACCCATTTTCCAGAATATGCCATTGGATTCTCCTTATAAATAG
ATAATATATTTATAAACAGGAGGGCCCATGCTCTTTACATTTTTTGATCCGATTGAATATGCGGCCAAAA
CGGTGAATAAAAACGCGCCGACTATTCCTATGACAGATATTTTTAGAAACTATAAAGACTATTTTAAACG
CGCTCTTGCGGGATACCGCTTACGTACTTATTATATTAAAGGTTCACCACGCCCGGAAGAATTAGCAAAT
ACTATATATGGAAATACGCAGTTGTATTGGGTTTTATTGATGTGTAATGATAATTATGATCCGTATTATG
GATGGATTACTTCGCAAGAAGCTGCTTATCAAGCATCTATACAAAAATACAAAAATGTGGGTGGAGACCA
AATAGTATATCATGTGAATGAGAACGGTGAAAAATTTTATAATTTAATATCATACGATGATAATCCATAT
GTTTGGTATGACAAAGGCGATAAAGCTAGAAAATATCCTCAATATGAAGGAGCACTTGCTGCGGTCGATA
CGTATGAAGCGTCTGTTCTTGAAAATGAAAAACTTCGTCAAATAAAAATAATAGCAAAATCAGACATCAA
TTCATTTATGAACGACCTTATACGTATAATGGAGAAATCTTATGGAAATGATAAGTAATAACCTTAATTG
GTTTGTTGGTGTTGTTGAAGATAGAATGGACCCATTAAAATTAGGTCGTGTTCGTGTTCGTGTGGTTGGT
TTGCATCCACCTCAAAGAGCACAAGGTGATGTAATGGGTATTCCAACTGAAAAATTACCATGGATGTCAG
TTATTCAACCTATAACTTCTGCAGCAATGTCTGGAATTGGGGGCTCCGTTACTGGTCCGGTAGAAGGAAC
TAGAGTTTATGGTCATTTTTTGGACAAATGGAAAACTAATGGAATTGTCCTTGGCACGTATGGTGGAATA
GTTCGCGAAAAACCGAATAGACTTGAAGGATTTTCTGACCCAACTGGACAGTATCCTAGACGTCTAGGAA
ATGATACTAACGTACTAAACCAAGGCGGAGAAGTAGGATATGATTCCTCTTCTAACGTTATTCAAGATAG
TAACTTAGACACTGCAATAAATCCCGATGATAGACCACTATCAGAGATTCCAACCGATGATAATCCAAAT
ATGTCAATGGCTGAAATGCTTCGCCGTGATGAAGGATTAAGATTAAAAGTTTATTGGGATACCGAAGGAT
ATCCGACAATTGGTATTGGTCATCTTATCATGAAACAGCCAGTTCGTGATATGGCTCAAATTAATAAAGT
TTTATCAAAACAAGTTGGTCGTGAAATTACTGGAAACCCAGGTTCTATTACGATGGAAGAGGCGACGACT
TTATTTGAGCGTGATTTGGCTGATATGCAACGTGACATTAAATCGCATTCTAAAGTAGGACCAGTCTGGC
AAGCTGTCAACCGTTCTCGTCAAATGGCGTTAGAAAATATGGCATTTCAGATGGGTGTTGGTGGTGTAGC
TAAATTTAACACAATGTTAACTGCTATGTTAGCAGGAGATTGGGAAAAAGCATATAAAGCCGGTCGTGAT
TCATTGTGGTATCAACAAACAAAAGGCCGTGCATCCCGTGTTACTATGATTATTCTTACGGGAAATTTGG
AATCATATGGTGTTGAAGTGAAAACCCCAGCTAGGTCTCTATCAGCAATGGCTGCTACTGTAGCTAAATC
TTCTGACCCTGCTGACACTCCTATTCCAAATGACTCGAGAATTTTATTCAAAGAACCAGTTTCTTCATAT
AAAGGTGAATATCCTTATGTGCATACAATGGAAACTGAAAGCGGACATATTCAGGAATTTGATGATACCC
 
CTGGGCAAGAACGATACAGATTAGTTCATCCAACTGGAACTTATGAAGAAGTATCACCGTCAGGAAGAAG
AACAAGAAAAACTGTTGATAATTTGTATGATATAACCAACGCTGATGGTAATTTTTTGGTAGCCGGTGAT
AAAAAGACTAACGTCGGTGGATCAGAAATTTATTACAACATGGATAATCGTCTTCACCAGATAGATGGAA
GCAATACAATATTTGTGCGTGGCGATGAAACTAAGACAGTTGAAGGTAATGGAACTATCCTAGTTAAAGG
TAATGTTACTATTGTAGTTGAAGGTAATGCTGACATTACAGTTAAAGGAGATGCTACCACTTTAGTTGAA
GGTAATCAAACTAATACAGTAAATGGAAATCTTTCTTGGAAAGTTGCTGGGACAGTTGATTGGGACGTTG
GTGGTGATTGGACAGAAAAAATGGCATCTATGAGTTCTATTTCATCTGGTCAATACACAATTGATGGATC
GAGGATTGACATTGGCTAATATACTTCCAATGAGCGCTGATTTAGGAGAATCCATGGAAGGTTCTTCTAT
CGACGTCACCTTTACCGCTCAATTAGAAACAGGTGAAACGTTAGTATCTATAAATATAACTAGTTACGAA
GAAACTCCTGGAGTTTTAGTAGAAGAAAATCGCTTATATGGAACATATGAATCTGTATTTGGTTTCGGAA
ATGACGCGTTGAAATATCGTTTAGGCGATGAATTTAAAACTGCTGCTTCATGGGAAGAACTTCCTACTGA
TTCTATACTCAGTTGTATTTGTGGAAAGCTCCTCAAAACCTCCAGAAGACATTCACTTACGAAGTAACAT
TAATATATGACTACCAAGAACAAAGTGAATCTGGAGGTTCTGGCAGTAATTCTAGGTCATCTTCTGATAC
TACTGAACCGACAGATCCTCCTGCTCCAGTAAGAAAAACTCTAGTTAAAAATTATACTAAAACTATAGTT
GGAAATTGGAGTCGTTGGGCTAATAAATTGAGAAGCTATGTGTATGAGAGGCCATAAATGTCAGGATTAA
GTTATGATAAGTGTGTTACTGCTGGCCATGAAGCGTGGCCTCCAACAGTTGTGAATGCTACACAAAGTAA
AGTATTCACTGGAGGAATTGCTGTTCTCGTAGCAGGCGATCCAATTACAGAACATACAGAAATTAAAAAG
CCATATGAAACACATGGCGGAGTGACACAACCCAGAACTTCTAAGGTATATGTCACTGGAAAGAAAGCTG
TTCAAATGGCTGATCCAATATCATGCGGTGATACTGTGGCTCAGGCATCATCTAAAGTATTCATTAAATA
GGATTTAAAATGGCAAATACCCCTGTAAATTATCAATTAACAAGAACAGCAAATGCTATTCCCGAGATAT
TCGTCGGGGGTACGTTTGCTGAAATAAAACAAAACCTCATTGAATGGCTTAATGGCCAAAATGAATTTTT
GGATTATGATTTTGAAGGCTCAAGATTAAACGTTCTGTGTGACCTTTTGGCTTATAATACGCTGTACATT
CAACAGTTTGGTAATGCTGCTGTGTATGAAAGCTTTATGCGTACTGCTAACTTACGAAGTTCAGTTGTTC
AAGCTGCGCAAGATAACGGATATTTACCTACTTCAAAATCCGCTGCGCAGACAGAAATTATGTTAACATG
CACTGACGCATTGAATAGGAATTACATTACGATTCCTCGCGGAACTCGCTTTTTAGCATATGCAAAAGAT
ACTTCTGTTAATCCATATAACTTCGTTTCTACCGAAGACGTTATTGCTATTCGTGATAAAAATAACCAAT
ATTTTCCGCGTTTAAAATTGGCTCAGGGACGTATAGTAAGAACTGAAATCATTTATGATAAATTAACACC
TATTATCATTTATGATAAAAATATTGATAGAAACCAGGTTAAATTATATGTTGATGGAGCGGAATGGATT
AACTGGACGAGAAAGTCAATGGTTCATGCTGGTTCAACATCAACGATTTACTATATGCGTGAAACTATTG
ATGGAAACACTGAATTCTATTTTGGCGAAGGTGAAATTTCTGTTAATGCTTCTGAAGGAGCTTTGACTGC
TAATTATATTGGAGGTCTTAAACCTACTCAGAACTCTACGATTGTTATTGAGTACATCAGTACTAATGGT
GCTGACGCGAACGGAGCAGTAGGATTTTCATACGCAGATACATTAACAAATATAACTGTCATCAATATTA
ATGAAAATCCAAACGATGACCCAGATTTTGTTGGGGCAGATGGAGGCGGTGATCCAGAAGATATTGAGCG
TATTCGCGAATTGGGTACTATTAAACGCGAAACCCAACAACGATGCGTAACTGCGACTGACTACGATACA
TTCGTTTCAGAGAGATTTGGTTCTATTATTCAAGCAGTTCAAACTTTCACTGATTCTACTAAACCTGGGT
ATGCATTTATTGCTGCTAAACCTAAATCAGGATTGTATTTAACTACTGTACAACGTGAAGATATTAAAAA
TTATCTCAAAGACTATAATTTAGCTCCTATTACGCCATCAATTATTTCTCCTAATTATCTTTTTATTAAG
ACTAATTTAAAAGTCACATATGCTTTAAATAAGCTGCAAGAATCCGAACAGTGGCTTGAAGGTCAAATAA
TTGATAAAATTGATCGCTATTATACCGAAGATGTAGAAATTTTTAACTCATCTTTCGCTAAATCTAAGAT
GTTGACATACGTAGATGATGCAGATCATTCTGTCATTGGTTCATCAGCGACTATTCAAATGGTTCGTGAA
GTACAAAACTTCTATAAAACGCCTGAAGCGGGTATTAAATACAATAATCAAATAAAAGATCGTTCTATGG
AATCTAATACGTTTTCATTTAATTCTGGACGAAAGGTTGTAAATCCTGATACTGGTTTAGAAGAAGATGT
ATTATATGACGTTCGTATAGTATCAACAGACCGAGATTCTAAAGGAATTGGTAAAGTTATTATTGGTCCA
TTTGCTTCTGGCGATGTTACAGAAAATGAAAACATTCAGCCGTATACAGGCAACGATTTTAACAAATTAG
 
CAAATTCTGATGGACGTGACAAATACTATGTTATCGGTGAAATAAATTATCCAGCTGATGTGATTTATTG
GAATATCGCTAAAATTAATTTAACATCTGAAAAATTTGAAGTTCAGACTATTGAATTATATTCTGACCCA
ACCGATGATGTTATCTTTACTCGCGATGGTTCACTGATTGTATTTGAAAATGACTTACGTCCACAATACT
TAACTATCGATTTGGAGCCTATATCACAATGACAGTAAAAGCACCTTCAGTCACTAGTCTCAGAATTTCC
AAGTTATCCGCAAATCAGGTGCAAGTACGCTGGGATGACGTTGGTGCTAATTTCTACTATTTTGTAGAAA
TCGCTGAGACAAAAACAAACTCGGGGGAAAAACTCCCGAGTGATCAATATCGTTGGATTAATTTAGGATA
TACAGCAAATAATAGTTTCTTTTTTGATGATGCTGATCCATTAACAACATACATTATTAGAGTAGCCACA
GCTGCGCAAGATTTTGAGCAGTCTGACTGGATTTATACAGAAGAGTTTGAAACTTTTGCTACAAATGCTT
ATACATTTCAAAACATGATTGAAATGCAATTAGCCAATAAATTCATTCAGGAAAAATTTACTCTTAATAA
TTCTGATTATGTTAATTTTAATAATGACACTATAATGGCTGCATTGATGAATGAATCGTTCCAATTCAGC
CCATCATATGTTGATGTTTCATCAATCAGTAATTTTATTATTGGTGAAAATGAGTATCATGAAATACAAG
GTTCTATTCAACAAGTATGTAAGGATATTAACCGAGTTTATTTGATGGAATCAGAAGGGATTCTATATCT
TTTTGAGCGTTATCAACCTGTAGTTAAAGTATCTAATGATAAAGGACAAACCTGGAAAGATGTAAAGCTC
TTCAATGACCGTGTAGGATATCCTTTATCTAAGACTGTATATTACCAATCTGCAAACACAACATACGTTC
TAGGATACGACAAGATTTTCTACGGCCGTAAATCTACTGATGTTAGATGGTCCGCTGATGATGTTAGATT
TAGTTCACAGGATATAACATTCGCTAAACTTGGTGTTCAATTGCATCTAGGATTTGATGTAGAAATTTTT
GTCACTTATGCGACTTTACCTGCGAATGTATATCGAATTGCAGAAGCTATTACCTGCACGGATGATTATA
TCTATGTTGTTGCCAGAGATAAAGTTAGATACATAAAAACGAGTAATGCACCTATAGATTCTGATCCATT
ATCTCCAACATATTCGGAAAGACTTTTTGAACCTGATACCATGACTATAACTGGAAATCCTAAAGCAGTA
TGTTATAAAATGGATTCTATTGGTGATAAAGTTTTTGCTCTTATTATTGGTGAAGTTGAAACATTAAATG
CCAATCCGAGGACTTCAAAAATAATTGATTCCGCTGATAAAGGAATATATGTTTTAAATCATGACACAAA
AACATGGAAAAGAGTTTTCGGTAATACTGAAGAAGAAAGAAGACGCATTCAACCCGGATATGCGAATATG
TCAACTGACGGTAAATTAGTTTCTCTGTCTTCGAGTAATTTTAAATTTTTAAGTGATAATGTTGTTAATG
ACCCTGAAACTGCAGCAAAATATCAGTTAATTGGCGCTGTTAAATATGAATTTCCTCGTGAATGGTTAGC
TGATAAGCATTATCATATGATGGCATTTATAGCTGATGAAAAATCTGATTGGGAGACTTTTACTCCTCAA
CCAATGAAATACTACGCAGAACCGTTCTTTAACTGGTCTAAAAAATCTAACACACGTTGTTGGATAAACA
ACTCTGATAGAGTTGTAGTAGTTTATGCTGATTTAAAATACACCAAAGTTATAGAAAATATTCCGGAAAC
ATCGCCAGATAGATTAGTTCATGAATACTGGGATGATGGTGATTGCACTATAGTAATGCCAAATGTTAAA
TTCACTGGATTTAAAAAATACGCATCAGGAATGCTTTTCTATAAAGCCTCTGGTGAAATAATTTCTTACT
ATGATTTTAACTATCGTGTGAGAGATACAGTAGAAATTATTTGGAAGCCAACTGGAGTATTTTTAAAAGC
ATTTTTACAAAACCAAGAGCATGAGACTCCTTGGTCACCAGAAGAAGAGCGTGGATTAGCTGACCCAGAT
TTAAGACCATTAATTGGCACAATGATGCCTGATTCTTATTTGCTACAGGATTCGAATTTTGAAGCATTTT
GCGAAGCATATATTCAATATCTTTCTGATGGATACGGAACTCAATACAATAATTTACGGAATCTTATTCG
TAATCAGTATCCACGAGAAGAGCATGCATGGGAATATTTGTGGTCAGAGATATATAAAAGAAACATTTAT
TTAAATGCTGATAAACGCGATGCTGTTGCGAGATTCTTTGAATCACGTAGCTACGATTTTTATTCTACTA
AAGGAATTGAAGCATCATACAAGTTTCTTTTTAAAGTTCTTTATAATGAAGAAGTTGAAATTGAAATTGA
ATCTGGGGCTGGTACTGAATATGACATAATCGTTCAATCTGATTCTTTGACTGAAGATTTAGTAGGACAA
ACGATTTATACGGCAACAGGAAGATGTAATGTTACTTATATAGAAAGAAGCTATTCTAATGGTAAATTGC
AATGGACCGTAACTATTCATAATCTTTTGGGAAGATTAATTGCTGGTCAAGAAGTTAAAGCAGAAAGACT
CCCTAGTTTTGAAGGCGAAATTATTCGTGGCGTTAAAGGAAAGGATTTGCTTCAAAACAATATAGACTAT
ATTAATAGAAGTAGATCATACTATGTAATGAAAATTAAATCCAATTTACCTTCTTCCCGCTGGAAATCTG
ACGTTATTCGTTTTGTTCATCCAGTAGGATTTGGATTTATAGCAATTACCCTTTTAACAATGTTTATTAA
TGTTGGTTTAACTCTTAAACATACAGAGACTATAATTAATAAATACAAAAACTATAAATGGGATTCTGGA
TTGCCTACTGAATACGCTGATAGAGTAGCTAAATTGACTCCAACTGGCGAAATTGAGCATGATTCAGTAA
 
CAGGCGAAGCAATTTATGAGCCTGGCCCAATGGCTGGTGTAAAATATCCTCTTCCTGATGACTATAATGC
TGAAAATAATAATTCAATATTTCAAGGTCAATTGCCGTCTGAACGACGTAAATTAATGAGTCCTTTATTT
GATGCATCTGGAACAACATTTGCACAATTTAGAGATTTAGTTAATAAACGTCTAAAAGACAATATAGGAA
ATCCAAGAGACCCTGAAAATCCAACACAGGTTAAAATAGATGAATGATTCAAGTGTTATCTATCGTGCGA
TAGTTACTTCAAAATTTAGAACAGAAAAAATGTTGAATTTTTATAATTCAATTGGAAGTGGTCCGGATAA
AAACACTATCTTTATCACATTTGGAAGATCAGAACCGTGGTCATCAAATGAAAATGAGGTGGGCTTTGCC
CCACCTTATCCTACCGATTCTGTATTAGGTGTAACTGACATGTGGACTCATATGATGGGAACAGTAAAAG
TTCTTCCATCAATGCTTGATGCAGTTATTCCTCGTCGAGATTGGGGAGATACTAGATATCCGGATCCATA
CACATTTAGAATTAACGATATTGTAGTGTGTAACTCAGCTCCTTACAACGCTACTGAATCAGGCGCGGGT
TGGTTAGTGTATCGTTGTTTAGATGTTCCTGATACCGGAATGTGTTCAATAGCATCTTTAACTGATAAAG
ATGAATGCCTTAAATTAGGAGGAAAATGGACTCCTTCTGCTAGGTCAATGACTCCGCCTGAAGGCCGAGG
AGATGCTGAAGGAACAATTGAACCCGGAGACGGGTATGTGTGGGAATATCTTTTTGAGATTCCGCCTGAT
GTATCTATAAATAGATGCACGAATGAATATATCGTGGTTCCTTGGACTGAGGAATTAAAAGAGGACCCGA
CTAGATGGGGATATGAAGATAATCTCACTTGGCAACAAGATGATTTTGGATTAATTTACCGTGTTAAGGC
AAATACTATTCGTTTTAAAGCATATTTAAACTCAGTTTATTTTCCTGAAGCTGCATTGCCAGGAAATAAA
GGATTTAGACAAATATCAATAATCACGAATCCTCTTGAAGCTAAAGTTCATCCAAATGACCCAAACGTTA
AAGCTGAAAAGGATTATTATGACCCAGAAGATTTAATGAGGCATTCGGGCGAAATGATTTATATGGAAAA
TAGGCCACCTATTATTATGGCAATGGATCAAACAGAAGAAATCAATATTCTGTTTACATTTTAAATTAAG
GGAGCCCATGGGCTCCCTTTTTCTTTATAAATACTATAAACTCATAAGGAAACCGCTATGTTCATTCAAG
AACCAAAGAAATTGATTGATACCGGCGAAATTGGTAACGCTTCTACTGGTGATATCTTATTCGACGGTGG
TAATAAAATTAATAGTGATTTTAACGCAATTTATAATGCGTTTGGCGATCAGCGTAAAATGGCAGTAGCA
AATGGCACTGGAGCAGATGGTCAAATTATCCATGCTACTGGATATTATCAAAAACACTCTATTACAGAGT
ACGCAACTCCAGTAAAAGTTGGCACTAGGCATGATATTGATACCTCTACTGTAGGTGTTAAAGTTATCAT
TGAAAGAGGCGAACTTGGCGACTGCGTTGAATTTATTAACTCTAATGGATCAATATCAGTTACTAATCCT
CTAACAATTCAAGCTATTGATTCAATTAAAGGTGTTTCAGGTAATTTAGTAGTAACTAGCCCATATAGTA
AAGTTACTTTACGCTGTATTTCATCTGATAATTCTACATCGGTTTGGAATTATTCTATTGAAAGTATGTT
TGGACAAAAGGAATCACCAGCTGAAGGTACATGGAATGTTTCTACATCCGGATCAGTTGATATTCCACTA
TTTCAACGCACTGAATACAATATGGCTAAATTGCTAGTTACGTGCCAATCAGTAGATGGAAGAAAAATTA
AAACAGCAGAAATAAATATTCTTGTGGATACTGTTAATTCAGAGGTAATTTCTTCTGAATATGCTGTCAT
GCGAGTTGGGAATGAAACCGAAGAAGATGAAATCGCTAATATTGCATTTAGTATTAAAGAAAACTATGTA
ACGGCGACTATAAGTTCTTCAACTGTCGGTATGAGAGCAGCAGTTAAAGTTATCGCTACGCAGAAAATCG
GGGTGGCTCAATAATGAAACAAAATATTAATATCGGTAATGTTGTAGATGATGGTACCGGTGACTACCTG
CGTAAAGGTGGTATAAAAATAAATGAAAACTTTGATGAGCTTTATTATGAACTCGGTGATGGTGATGTTC
CATATTCAGCCGGTGCCTGGAAAACTTATAATGCTTCATCAGGACAAACATTAACAGCAGAATGGGGAAA
ATCATACGCTATTAATACATCTTCTGGAAGAGTGACTATAAATCTTCCAAAGGGTACAGTTAATGATTAC
AACAAGGTAATTAGAGCTAGAGACGTATTTGCTACATGGAACGTCAACCCAGTTACACTAGTAGCTGCTT
CCGGCGATACGATTAAAGGGTCTGCAGTACCAGTTGAAATTAATGTTCAATTCAGCGATTTAGAACTAGT
GTATTGTGCCCCAGGACGTTGGGAATATGTCAAAAATAAACAAATTGACAAAATTACCAGTTCAGACATT
AGTAATGTAGCTCGTAAAGAATTTTTAGTCGAAGTCCAAGGGCAAACAGACTTTTTAGATGTTTTCAGTG
GAACTAGTTATAATGTAAATAACATCAGAGTAAAACATCGTGGTAACGAATTATATTATGGCGATGTGTT
TAGCGAAAACAGCGATTTTGGCTCTCCAGGCGAAAATGAAGGAGAACTGGTTCCTCTTGATGGATTTAAT
ATTCGATTAAGACAGCCTTGTAATATTGGTGACACTGTTCAAATTGAAACATTTATGGATGGTGTATCGC
AGTGGAGAAGTTCATATACAAGACGTCAAATTAGATTGTTAGATTCAAAATTAACGTCAAAAACTTCTCT
AGAAGGAAGTATTTACGTTACTGATTTATCAACAATGAAATCAATTCCATTTTCTGCTTTTGGATTAATT
 
CCAGGAGAACCTATTAATCCTAATTCTCTTGAAGTTAGTTTTAATGGAATTTTACAAGAATTGGCTGGAA
CAGTTGGAATGCCATTATTTCATTGTGTTGGTGCCGATTCAGACGATGAAGTAGAATGCTCTGTTTTAGG
TGGAACTTGGGAACAATCTCATACCGATTATTCAGTTGAAACTGATGAAAACGGCATACCAGAAATTTTA
CATTTCGATAGAGTATTTGAGCATGGTGACATTATCAATATCACCTGGTTTAATAATGATTTGGGTACAT
TATTAACAAAAGATGAGATTATTGATGAAACTGATAATCTCTATGTATCGCAAGGACCGGGAGTAGATAT
TTCCGGTGATGTAAATTTAACAGACTTTGATAAAATTGGTTGGCCAAATGTAGAAGCAGTTCAATCTTAT
CAACGCGAATTTACTGCTGTTTCAAATATCTTTGATACGATTTATCCTATTGGAACTATATATGAAAACG
CTGTTAATCCAAATAACCCTGTTACATATATGGGATTCGGCTCATGGAAATTATTTGGGCAAGGAAAAGT
TTTAGTTGGATGGAATGAAGATATTTCGGACCCTAACTTTGCTCTAAATAACAACGATTTAGATTCTGGT
GGAAATCCTTCGCATACTGCAGGCGGAACAGGTGGTTCTACTTCTGTTACATTGGAAAATGCTAATCTTC
CTGCAACCGAGACAGATGAAGAAGTTCTAATAGTTGATGAAAATGGATCAGTCATTGTTGGTGGATGTCA
ATACGATCCAGATGAATCCGGTCCAATTTATACTAAATACCGTGAAGCTAAAGCATCTACTAACTCTACT
CACACTCCGCCAACATCAATAACTAACATTCAACCATATATTACAGTTTATCGTTGGATAAGGATTGCAT
AATGAGTTTACTTAATAACAAAGCGGGAGTTATTTCCCGCTTAGCCGATTTTCTTGGTTTTAGACCTAAA
ACTGGCGACATTGATGTAATGAATCGTCAATCAGTCGGGTCAGTGACAATATCTCAATTAGCGAAAGGAT
TTTATGAACCAAACATAGAATCAGCTATTAATGACGTTCATAATTTTTCTATAAAAGACGTTGGTACAAT
TATTACTAATAAAACTGGTGTTTCTCCTGAGGGTGTTTCTCAAACTGATTATTGGGCATTTTCTGGAACT
GTAACAGACGATTCTCTTCCTCCGGGTTCTCCTGTTACGGTATTAGTATTTGGTCTTCCAGTTTCAGCAA
CAACTGGAATGACGGCAATTGAGTTTGTTGCAAAAGTTCGTGTTGCCCTTCAAGAAGCTATTGCATCATT
TACTGCTATCAACTCATATAAAGACCATCCAACAGATGGTAGTAAATTAGAAGTTACTTATTTAGATAAT
CAAAAACATGTATTAAGCACATATTCTACATATGGAATAACTATTTCGCAGGAAATTATTTCTGAGTCTA
AACCTGGCTATGGTACATGGAATTTATTAGGCGCACAAACTGTAACTTTAGATAATCAGCAGACTCCTAC
AGTATTTTATCATTTTGAGAGAACAGCATGAGTAATAATACATATCAACACGTTTCTAATGAATCTCGTT
ATGTAAAATTTGATCCTACCGATACGAATTTTCCACCAGAGATTACTGATGTTCAGGCTGCTATAGCAGC
CATTTCTCCTGCTGGCGTAAATGGAGTTCCTGATGCATCGTCAACAACAAAGGGAATTTTATTTCTTGCC
ACTGAACAGGAAGTTATCGATGGAACTAATAATACCAAAGCAGTTACACCAGCAACGTTGGCAACAAGAT
TATCATATCCAAACGCAACTGAAGCTGTTTACGGATTAACAAGATATTCAACCGATGATGAAGCCATTGC
CGGAGTTAATAATGAATCTTCTATAACTCCAGCTAAATTTACTGTTGCTCTTAATAATGTCTTTGAAACT
CGTGTTTCAACTGAATCATCAAATGGGGTTATTAAAATTTCATCTTTACCGCAAGCATTGGCAGGTGCAG
ATGATACTACTGCAATGACTCCATTAAAAACACAACAATTAGCTGTTAAATTGATTGCGCAAATTGCTCC
TTCTAAAAATGCTGCTACAGAATCTGAGCAAGGTGTAATTCAGTTAGCTACAGTAGCACAGGCTCGTCAG
GGAACTTTAAGAGAAGGATACGCAATTTCTCCTTATACGTTTATGAATTCTACTGCTACTGAAGAATATA
AAGGCGTAATTAAATTAGGAACGCAATCAGAAGTTAACTCGAATAATGCTTCTGTTGCGGTTACTGGAGC
AACTCTTAATGGTCGTGGTTCTACGACGTCAATGAGAGGCGTAGTTAAATTAACTACAACCGCCGGTTCA
CAGAGTGGAGGCGATGCTTCATCAGCCTTAGCTTGGAATGCTGACGTTATCCACCAAAGAGGCGGTCAAA
CTATTAATGGAACACTTCGCATTAATAATACGCTTACAATAGCTTCAGGTGGGGCAAATATTACCGGAAC
AGTTAACATGACTGGCGGTTATATTCAAGGTAAACGCGTCGTAACACAAAATGAAATTGATAGAACTATT
CCTGTCGGAGCTATTATGATGTGGGCCGCTGATAGTCTTCCTAGTGATGCTTGGCGTTTTTGCCACGGTG
GAACTGTTTCAGCGTCAGATTGTCCATTATATGCTTCTAGAATTGGAACAAGATATGGCGGAAGCTCATC
AAATCCTGGATTGCCTGACATGCGCGGTCTTTTTGTTCGTGGCTCTGGCCGTGGCTCTCATTTAACAAAT
CCAAATGTTAATGGTAATGACCAATTTGGTAAACCTAGATTAGGTGTAGGTTGTACTGGTGGATATGTTG
GTGAAGTACAGAAACAACAGATGTCTTATCATAAACATGCTGGTGGATTTGGTGAGTATGATGATTCTGG
GGCATTCGGTAATACTCGTAGATCAAATTTTGTTGGTACACGTAAAGGACTTGACTGGGATAACCGTTCA
TACTTCACTAATGACGGGTATGAAATTGACCCAGCATCACAACGAAATTCCAGATATACATTAAATCGTC
 
CTGAATTAATTGGAAATGAAACACGTCCATGGAACATTTCTTTAAACTACATAATTAAGGTAAAAGAATG
ACAGATATTGTACTGAATGACTTACCATTCGTTGACGGCCCTCCTGCAGAGGGCCAGAGCCGCATTTCCT
GGATTAAAAACGGCGAAGAAATATTAGGAGCTGACACGCAGTATGGAAGCGAAGGTTCAATGAATAGACC
TACAGTTTCTGTACTAAGAAATGTCGAAGTTCTCGATAAAAACATTGGAATACTTAAAACATCTTTAGAA
ACCGCAAATAGTGATATTAAAACAATTCAGGGCATCTTAGATGTATCTGGTGATATTGAAGCTTTGGCCC
AAATAGGTATCAATAAAAAGGATATTTCTGACCTCAAAACGCTAACCAGTGAACATACAGAAATATTAAA
TGGACCTAATAGTACAGTTGACAACATTCTTGCTGATATTGGTCCATTTAACTCTGAGGCCAACTCTGTA
TACAGAACAATCAGAAATGATTTACTGTGGATAAAGCGTGAACTTGGACAATACGCAGGTCAAGATATTA
ATGGTCTTCCTGTTGTAGGAAATCCTAGTAGTGGAATGAAGCATCGCATTATTAATAATACTGATGCCAT
TACTTCACAGGGAATACGTTTAAGCGAATTAGAAACAAAATTTATTGAATCTGATGTAGGTTCTTTGACT
ATTGAAGTTGGTAATCTTCGTGAAGAGCTTGGACCGAAACCACCATCATTTTCACAAAACGTTTATAGTC
GTTTAAATGAAATTGACACTAAACAGACAACATTTGAATCTGACATTAGTGCTATTAAGACCTCAATAGG
ATATCCAGGAAATAATTCGATTATTACTAGTGTTAATACAAACACTGATAATATTGCATCTATTAATTTA
GAGCTAAATCAAAGTGGAGGTATTAAACAGCGTTTAACCGTTATTGAAACTTCTATTGGTTCAGATGATA
TTCCTTCGAGTATTAAAGGCCAAATCAAAGATAATACAACTTTAATCGAATCTCTAAATGGAATCGTCGG
TGAAAACACTTCATCTGGTTTAAGAGCGAATGTTTCATGGTTAAACAAAATTGTTGGAACTGATTCTAGC
GGTGGACAACCTTCTCCTTCTGGGTCTCTTTTAAACCGAGTTTCTACAATTGAAACTTCTGTTTCAGGAT
TGAATAACGATGTTCAAAACCTACAAGTAGAGATTGGTAATAATAGCGCAGGAATTAAAGGGCAAGTTGT
AGCGTTAAATACTTTAGTAAATGGAACTAATCCAAACGGTTCAACAGTCGAAGAACGCGGATTAACCAAT
TCAATAAAAGCTAACGAAACCAACATTGCATCAGTTACACAAGAAGTGAATACAGCTAAAGGTAATATAT
CTTCTTTACAAAGCGGTGTTCAAGCTCTCCAAGAAGCCGGTTATATTCCTGAAGCGCCAAGAGATGGGCA
AGCTTACGTTCGTAAAGACGGCGAATGGGTATTGCTTTCTACCTTTTTATCACCAGCATAACATGGGGCC
GCAAGGCCCCAAAGGATTTTAAATGTCAGGATATAATTCTCAGAATCCAAAGGAACTCAAAGATGTCATT
CTAAGACGTTTAGGGGCTCCAATTATTAATGTTGAGTTAACACCCGATCAAATTTACGATTGTATCCAGC
GTGCCCTAGAATTATACGGTGAATACCATTTTGATGGACTCAATAAAGGGTTTCATGTGTTTTACGTAGG
GGATGACGAAGAAAAGTACAAGACCGGAGTCTTCGATTTAAGAGGTTCTAACGTATTTGCAGTAACTCGC
ATTTTACGCACAAATATTGGGTCAATAACATCTATGGATGGAAACGCTACATATCCGTGGTTTACTGACT
TTCTTTTGGGAATGGCTGGTATTAATGGCGGAATGGGAACGTCTTGTAATAGATTTTATGGACCAAATGC
CTTTGGTGCCGATTTGGGGTATTTTACTCAACTTACCAGTTATATGGGAATGATGCAGGATATGCTCTCT
CCTATTCCAGACTTTTGGTTTAATTCAGCAAATGAACAGCTCAAAGTCATGGGAAACTTCCAAAAATATG
ATTTAATTATCGTAGAAAGCTGGACTAAATCATACATTGATACAAACAAAATGGTTGGAAATACAGTAGG
ATATGGAACAGTCGTTCCACAAGATAACTGGTCATTATCTGAACGATATAATAACCCAGACAACAATTTA
GTAGGTCGTGTTGTTGGTCAAGACCCAAATGTTAAGCAAGGTGCTTACAATAATCGTTGGGTGAAAGACT
ATGCAACAGCTTTAGCTAAAGAATTAAATGGTCAAATTTTAGCACGCCACCAGGGAATGATGCTTCCTGG
CGGTGTTACAATTGATGGACAACGCTTAATAGAAGAAGCTCGATTAGAAAAAGAAGCACTGCGCGAAGAA
TTATACTTACTTGACCCTCCATTTGGAATTTTGGTAGGTTAATATGGCTACTTACGATAAAAATCTTTTT
GCTAAATTGGAAAACCGCACAGGTTATTCTCAGACCAATGAAACTGAAATACTAAATCCTTATGTAAATT
TCAATCATTATAAAAACAGCCAAATATTAGCTGATGTATTAGTAGCTGAAAGCATTCAAATGCGAGGTGT
AGAATGCTATTATGTTCCAAGAGAGTATGTTTCCCCTGATTTGATATTCGGCGAAGACTTGAAAAATAAA
TTTACTAAAGCTTGGAAATTTGCTGCATATTTAAATTCATTTGAAGGATATGAAGGAGCTAAATCGTTCT
TTAGTAATTTTGGTATGCAAGTACAGGATGAAGTTACTTTGTCCATTAATCCAAACTTGTTTAAACACCA
AGTAAATGGAAAAGAACCGAAAGAAGGCGATTTGATATATTTTCCTATGGATAACAGCTTATTTGAAATT
AACTGGGTTGAACCATATGATCCATTTTATCAATTAGGCCAAAACGCTATTCGTAAAATTACGGCAGGTA
AATTCATTTATTCTGGAGAAGAAATTAATCCAGTTCTACAGAAAAATGAAGGAATTAACATTCCAGAATT
 
TAGTGAATTAGAATTAAATCCTGTTCGCAATCTTAACGGTATTCATGACATTAATATTGATCAGTATGCT
GAAGTAGATCAAATTAATTCTGAAGCTAAAGAATATGTTGAACCCTATGTTGTTGTCAATAACAGAGGCA
AATCTTTCGAATCTAGCCCATTTGATAATGATTTCATGGATTAATAAATATTATAAACTAATTAAAGCCC
AGATTAGGAGAAGTCATGTTTGGTTATTTTTATAATTCGTCTTTTAGACGATATGCTACCTTGATGGGCG
ATTTGTTTTCAAATATCCAAATCAAACGTCAGTTAGAATCTGGTGATAAGTTTATACGTGTTCCTATTAC
ATATGCATCAAAGGAACACTTTATGATGAAATTGAATAAATGGACATCAATAAATTCACAAGAAGATGTA
GCTAAAGTTGAAACCATTCTACCTCGTATAAATTTACATTTAGTTGATTTTAGCTATAATGCTCCATTTA
AAACAAACATTTTAAATCAGAATTTACTGCAAAAAGGTGCAACTTCTGTAGTATCGCAGTATAATCCATC
TCCTATTAAAATGATTTATGAATTGAGTATCTTTACTCGCTACGAAGATGATATGTTTCAAATAGTTGAA
CAGATTCTTCCATATTTTCAACCTCATTTTAATACAACTATGTACGAGCAGTTTGGAAATGATATTCCAT
TTAAAAGGGATATCAAAATTGTACTGATGTCTGCTGCTATAGACGAAGCTATAGATGGGGATAATTTATC
TCGTCGTAGAATTGAATGGTCATTAACATTTGAAGTAAATGGATGGATGTATCCTCCAGTAGATGATGCA
GAAGGATTAATTCGTACTACTTATACAGATTTTCACGCCAATACAAGAGATTTGCCTGATGGCGAAGGTG
TTTTTGAATCTGTCGATAGCGAAGTTGTTCCTCGAGATATTAACCCAGAAGACTGGGATGGAACAGTAAA
ACAAACTTTCACTAGTAATGTAAATAGACCAACACCGCCAGAACCTCCTGGCCCAAGAACATAGAGGTTA
TTATGGAAGGTCTTGATATAAACAAACTTTTAGATATTTCTGACCTCCCCGGAATTGACGGGGAGGAAAT
CAAAGTATATGAACCTCTGCAATTAGTAGAAGTTAAAAGCAATCCACAAAACCGTACTCCTGACTTAGAA
GATGATTATGGAGTAGTTCGTCGAAATATGCATTTTCAACAACAAATGCTAATGGACGCGGCCAAGATTT
TTCTTGAGACGGCAAAGAATGCTGATTCTCCTCGTCACATGGAAGTATTTGCAACTCTTATGGGGCAAAT
GACTACGACGAACAGAGAAATACTGAAGCTTCATAAAGATATGAAAGATATTACATCTGAGCAGGTTGGC
ACCAAAGGCGCTGTTCCTACAGGTCAAATGAATATTCAGAATGCGACAGTATTCATGGGTTCACCAACAG
AATTAATGGACGAAATTGGTGATGCTTACGAGGCTCAAGAAGCTCGTGAGAAGGTGATAAATGGAACAAC
CAATTAATGCATTAAATGATTTCCATCCGTTAAATGAAGCTGGAAAAATTTTAATAAAACACCCAAGCTT
AGCGGAAAGAAAAGATGAAGATGGAATTCATTGGATAAAATCTCAGTGGGATGGAAAATGGTATCCTGAA
AAATTCAGTGATTACCTTCGTCTACACAAAATAGTAAAAATTCCAAACAACTCTGATAAGCCTGAATTAT
TTCAAACTTATAAAGATAAGAATAATAAAAGATCTCGGTATATGGGTCTTCCTAACTTGAAACGAGCTAA
TATTAAAACACAATGGACTCGTGAAATGGTTGAGGAATGGAAAAAATGCCGAGACGATATTGTTTATTTT
GCAGAAACATACTGTGCTATTACTCATATTGACTATGGTGTCATAAAGGTTCAATTACGTGACTATCAGC
GTGATATGCTCAAAATAATGTCATCTAAACGTATGACTGTTTGTAATCTATCGCGTCAGCTCGGTAAAAC
AACGGTAGTAGCTATTTTCCTTGCACACTTTGTATGTTTTAACAAGGATAAAGCTGTAGGTATTCTTGCG
CACAAAGGCTCAATGTCTGCGGAAGTTTTAGACCGTACTAAGCAAGCAATTGAACTGCTTCCTGACTTTT
TACAGCCAGGTATAGTTGAATGGAATAAGGGTTCAATTGAACTAGATAATGGTTCTTCAATTGGCGCTTA
TGCTTCCTCTCCTGACGCAGTTCGTGGTAACTCGTTCGCAATGATTTACATTGACGAATGTGCGTTTATT
CCAAACTTCCATGATTCCTGGCTTGCTATTCAACCAGTAATTTCATCTGGTCGTCGTTCGAAAATTATTA
TTACTACGACTCCTAATGGATTAAATCATTTTTATGATATTTGGACTGCTGCTGTTGAAGGTAAATCTGG
ATTTGAACCATATACTGCTATTTGGAATTCAGTTAAAGAACGTCTTTATAACGATGAAGATATTTTTGAC
GATGGATGGCAATGGAGCATACAAACCATTAATGGTTCTACTTTAGCTCAATTTCGTCAAGAACACACCG
CAGCGTTTGAAGGGACTTCTGGTACATTAATTTCGGGAATGAAATTAGCTATTATGGATTTCATTGAAGT
AACTCCAGATGATCATGGTTTTCATCGATTTAAAAGCCCTGAACCAGATAGAAAATATATTGCAACTCTA
GACTGCTCAGAAGGTCGTGGGCAAGATTACCACGCTTTGCATATTATTGATGTTACCGATGATGTGTGGG
AACAGGTTGGTGTTTTGCACTCAAACACTATTTCTCATTTAATTCTACCTGACATCGTTATGCGTTATTT
AGTAGAATACAATGAATGCCCAGTTTATATTGAATTAAATAGTACTGGTGTGTCAGTTGCAAAATCGCTT
TATATGGATTTAGAATACGAAGGTGTTATCTGTGATTCATATACTGATTTAGGAATGAAACAAACTAAAC
GCACGAAAGCAGTAGGATGTTCCACGCTAAAAGACCTTATTGAAAAAGATAAGCTTATTATTCATCACCG
 
AGCGACTATTCAAGAATTTAGAACGTTTAGTGAAAAAGGCGTGTCTTGGGCGGCTGAAGAAGGTTATCAC
GACGATTTAGTAATGTCTTTAGTAATTTTTGGATGGTTATCAACACAATCAAAATTTATTGATTATGCGG
ATAAAGATGACACGCGATTAGCATCTGAAGTATTTTCAAAAGAGCTTCAAGATATGAGCGACGACTACGC
GCCAGTTATATTTGTGGATTCGGTTCATTCTGCTGAGTATGTTCCAGTATCTCATGGTATGTCAATGGTA
TAAATATATTAAAGCATATTAAAGAGGATTAAAAATGACTTTATTATCTCCGGGCATTGAGCTCAAAGAA
ACTACGGTTCAAAGCACCGTGGTTAATAACTCTACTGGTACAGCAGCTTTGGCCGGTAAATTCCAGTGGG
GTCCTGCTTTTCAGATTAAACAGGTTACAAATGAAGTAGATTTAGTTAATACTTTTGGTCAACCAACCGC
TGAAACTGCTGACTATTTTATGTCTGCGATGAATTTCTTGCAGTACGGAAATGACTTACGAGTAGTTCGT
GCTGTTGATAGAGATACCGCTAAAAACTCATCACCAATCGCTGGTAATATTGAATACACAATTTCTACCC
CAGGTAGTAACTATGCGGTTGGAGATAAAATCACAGTCAAATATGTTTCAGATGATATTGAAACTGAAGG
TAAAATTACTGAAGTAGACGCAGATGGAAAAATTAAGAAAATTAATATTCCTACTGCAAAAATTATCGCT
AAAGCGAAAGAAGTCGGTGAATATCCAACACTAGGTTCTAACTGGACTGCGGAAATTTCTTCATCTTCCT
CTGGTTTAGCTGCAGTAATAACTCTTGGAAAAATTATTACTGATTCTGGTATTTTATTAGCTGAAATTGA
AAATGCTGAAGCTGCTATGACAGCGGTTGACTTTCAAGCAAATCTTAAAAAATATGGAATTCCAGGAGTA
GTAGCGCTTTATCCAGGCGAATTAGGCGATAAAATTGAAATTGAAATCGTATCTAAAGCTGACTATGCAA
AAGGAGCTTCTGCATTACTCCCAATTTATCCAGGTGGTGGTACTCGTGCATCTACTGCCAAAGCAGTGTT
TGGATATGGACCGCAAACTGATTCACAATACGCTATTATAGTTCGTCGCAATGATGCTATTGTTCAAAGC
GTTGTTCTTTCAACTAAGCGTGGTGAAAAAGATATTTACGATAGTAACATCTATATCGATGACTTTTTCG
CAAAAGGCGGCTCAGAATATATTTTTGCAACTGCACAAAACTGGCCAGAAGGCTTCTCTGGAATTTTAAC
TCTGTCTGGTGGATTATCATCAAATGCTGAAGTAACAGCAGGAGATTTGATGGAAGCTTGGGACTTCTTT
GCTGACCGTGAATCCGTTGATGTTCAACTGTTTATTGCGGGTTCTTGTGCCGGTGAATCTTTAGAAACAG
CATCTACTGTCCAAAAACACGTCGTTTCAATTGGGGATGCTCGCCAAGATTGCTTAGTATTGTGCTCTCC
TCCGCGTGAAACTGTAGTTGGAATTCCTGTAACTCGTGCAGTAGATAATTTAGTTAACTGGAGAACTGCG
GCAGGTTCATACACTGATAATAACTTTAATATCAGTTCAACCTACGCAGCAATTGATGGTAACTATAAGT
ATCAGTATGACAAATATAATGATGTGAATCGTTGGGTTCCATTAGCAGCTGATATTGCTGGTTTATGCGC
AAGAACTGATAACGTATCTCAGACTTGGATGTCTCCAGCTGGTTATAATCGTGGCCAGATTCTTAACGTT
ATTAAACTTGCTATTGAAACTCGCCAGGCTCAGCGCGACCGTTTATACCAAGAAGCTATCAACCCAGTAA
CTGGTACAGGTGGCGATGGTTACGTATTGTATGGTGATAAAACAGCTACTTCTGTTCCTTCTCCATTTGA
TCGTATTAACGTTCGTCGTCTGTTTAATATGTTGAAAACGAATATCGGACGTAGTTCAAAATATCGTTTG
TTCGAATTAAACAACGCGTTTACTCGTTCATCATTCCGCACAGAAACTGCCCAGTACTTGCAGGGAATTA
AAGCTCTCGGTGGAATTTATGAATATCGTGTAGTTTGCGATACAACAAATAACACTCCGTCAGTAATTGA
TAGAAATGAGTTTGTTGCAACATTCTACATCCAACCTGCGCGCAGTATAAATTATATTACTTTGAATTTC
GTCGCAACGGCTACTGGTGCAGATTTCGATGAGTTAACTGGTCTTGCAGGTTAATACGGTGCATTCTAAA
GGCCTGTTTCGGCAGGCCATATAAATACACTATATCCTTAATTCTTTAATTCTATATGCCCTAGGTTAAA
CATAGGGATATAAATACTACAGAGGCTAATATGTTTGTAGATGATGTAACACGCGCGTTTGAATCAGGTG
ATTTTGCGCGACCTAACTTATTCCAAGTAGAAATTTCTTATCTTGGACAAAATTTTACGTTTCAATGTAA
AGCCACTGCTTTACCAGCTGGTATTGTAGAAAAAATTCCAGTCGGATTTATGAACCGTAAAATTAACGTA
GCAGGCGATCGTACATTCGATGACTGGACTGTTACAGTAATGAACGATGAAGCTCATGATGCTCGCCAGA
AGTTCGTTGATTGGCAAAGCATTGCTGCGGGGCAAGGAAACGAAATTACTGGTGGAAAACCTGCAGAGTA
TAAAAAGAGCGCTATCGTTCGTCAATATGCTCGTGACGCTAAAACAGTAACAAAAGAAATTGAAATTAAA
GGTCTGTGGCCTACTAACGTGGGTGAACTTCAATTAGATTGGGATTCAAACAATGAAATCCAAACATTTG
AAGTAACTCTTGCTCTCGATTATTGGGAATAAAATGAATGGGGAGAAATCCCCATCCTGCTTAAAGCAGA
GAAGTCCATTATAAATATAACTATAATTCCCATTTGGAGAATACAATGAAATTTAATGTATTAAGTTTGT
TTGCTCCATGGGCTAAAATGGACGAACGAAATTTTAAAGACCAAGAAAAAGAAGATCTTGTTTCCATTAC
 
AGCCCCAAAGCTTGATGATGGAGCGAGAGAATTTGAAGTAAGCTCGAATGAAGCTGCTTCTCCTTATAAT
GCTGCATTCCAAACAATTTTTGGTTCATATGAACCAGGAATGAAAACTACTCGTGAGCTTATTGATACAT
ATCGTAATCTCATGAATAACTATGAAGTGGACAATGCAGTTTCAGAAATCGTTTCAGACGCTATCGTCTA
TGAAGATGATACTGAAGTTGTAGCGTTAAATTTGGATAAATCTAAATTTAGTCCAAAAATTAAAAATATG
ATGTTAGATGAATTTAGTGATATATTAAATCATCTATCGTTTCAACGAAAAGGTTCTAATCATTTTAGAC
GTTGGTATGTTGATTCAAGAATTTTCTTTCATAAAATCATTGATCCAAAACGTCCAAAAGAAGGCATAAA
AGAATTACGTAGATTAGACCCTCGCCGAGTTCAGTATGTTCGTGAAATTATAACAGAAACTGAAGCTGGC
ACAAAAATAGTTAAAGGTTACAAAGAATATTTTATATATGATACTGCCCATGAGTCATATGCATGTGATG
GTAGAATGTATGAAGCTGGCACAAAAATAAAAATTCCTAAAGCTGCCGTCGTTTATGCCCATTCTGGATT
AGTTGATTGTTGCGGTAAAAATATCATCGGGTATTTGCATCGTGCTGTTAAACCTGCTAACCAATTAAAA
TTATTAGAAGATGCTGTAGTCATTTATCGCATTACTCGTGCTCCTGACCGTCGTGTTTGGTATGTAGACA
CAGGTAATATGCCTGCTCGTAAAGCTGCTGAGCACATGCAACATGTTATGAACACGATGAAAAACCGTGT
AGTATATGATGCATCAACAGGTAAAATAAAAAATCAACAGCATAATATGTCTATGACCGAAGACTATTGG
TTGCAGCGCCGTGATGGTAAAGCTGTGACAGAAGTTGATACTCTTCCTGGTGCTGATAATACTGGCAATA
TGGAAGATATTCGTTGGTTTAGACAAGCTCTTTATATGGCATTACGTGTTCCTCTTTCACGCATTCCGCA
AGACCAACAAGGCGGTGTGATGTTTGATTCTGGAACTAGCATTACACGTGATGAATTAACGTTTGCTAAA
TTTATTCGTGAGTTGCAGCACAAGTTTGAAGAAGTTTTCCTAGATCCGCTTAAAACAAATCTTTTGCTTA
AAGGTATAATTACAGAAGATGAGTGGAATGATGAAATAAATAATATTAAGATAGAATTTCATCGGGATAG
CTACTTTGCTGAGCTCAAAGAAGCAGAAATTTTGGAACGAAGAATTAATATGCTAACCATGGCAGAACCA
TTTATTGGTAAATATATTTCTCACAGAACTGCTATGAAAGATATTTTGCAGATGACTGATGAAGAAATAG
AACAAGAAGCCAAGCAAATTGAAGAAGAGTCTAAAGAGGCTCGTTTCCAAGACCCCGACCAAGAACAAGA
GGATTTTTAATGGAAGGTTTAATTGAAGCTATTAAATCAAACGACCTCGTAGCCGCTCGTAAATTATTTG
CTGAAGCCATGGCTGCAAGAACGATTGATTTAATTAAAGAAGAAAAAATCGCTATCGCTCGTAATTTCTT
AATCGAAGGTGAAGAACCTGAAGACGAGGATGAAGATGAAGATGATGACGAAGATAGCGATGATAAAGAC
GAAGACTCTGACGAAGACGAGGATGATGAATAATGCTTCTGATCCCTGAAACTCATGAATTAGTTCTCGA
AAGTGTCGAAGCACTTATTACTGAAGCACAGGGTCGCTTTGACGAATTGTCTTCTGCTTTAAATAAAGAC
GATATAAATACAATTGTCGAGAATATGCTTGATGATGAAACTGATTTAGCGGTTGCATTAGCTTCTATTA
ATGAAAATATGCCGTTAAATGAATTTATCGTTAAACATGTTTCCGCCCGTGGTGAAATTACTCGCACTAA
AGATCGCAAAACCCGCGAACGAAATGCATTTCAAACCACTGGGCTGTCTAAAGCAAAACGTAGACAAATT
GCTCGTAAAGCTACCAAAACGAAGATTGCCAATCCAGCAGGTCAATCTCGTGCTCAGCGTAAGCGTAAAA
AAGCTCTTAAACGCCGTAAAGCATTAGGATTAAGCTAATGAATGAACCCCAATTACTAATTGAAACTTGG
GGTCAACCTGGCGAAATTATTGATGGCGTGCCAATGCTTGAATCTCATGATGGAAAAGACTTAGGTTTAA
AACCGGGTTTATACATCGAAGGAATATTCATGCAAGCGGAAGTCGTCAATAGAAATAAACGTCTTTATCC
AAAACGTATATTAGAAAAAGCGGTAAAAGACTATATTAATGAGCAAGTTTTAACTAAACAAGCTCTCGGA
GAATTAAATCATCCTCCACGCGCTAATGTTGACCCGATGCAAGCCGCTATCATTATAGAAGATATGTGGT
GGAAAGGAAATGACGTATACGGACGAGCTCGTGTTATTGAAGGTGACCATGGTCCTGGAGATAAATTAGC
AGCTAATATTCGTGCCGGATGGATTCCAGGAGTTTCTTCTCGTGGATTAGGTTCATTGACTGACACAAAT
AAAGGTTATCGTATCGTAAACGAAGGATTCAAATTAACTGTAGGTGTTGATGCAGTATGGGGTCCAAGTG
CTCCAGATGCATGGGTAACTCCTAAGGAAATTACCGAATCACAGACGGCGGAAGCCGATACAAGTGCCGA
TGACGCCTATATGGCTCTCGCAGAGGCCATGAAAAAAGCGTTATAAATATTATTATCTAAACAACAGGAC
TACAAAATGCTTAAAGAACAACTGATTGCCGAAGCGCAGAAAATTGATGCTTCCGTTGCTCTTGATAGTA
TTTTCGAATCAGTTAATATTTCTCCGGAAGCAAAAGAAACTTTCGGCACTGTATTCGAAGCTACCGTCAA
GCAGCACGCCGTTAAATTAGCTGAATCTCATATCGCTAAAATTGCTGAAAAAGCAGAAGAAGAAGTAGAA
AAAAATAAAGAAGAAGCCGAAGAAAAAGCTGAGAAGAAAATCGCTGAGCAAGCTTCTAAATTCATTGACC
 
ATCTTGCAAAAGAATGGCTCGCTGAAAATAAATTAGCAGTTGATAAAGGCATCAAAGCCGAACTGTTTGA
ATCCATGCTTGGTGGATTAAAAGAGCTCTTTGTTGAACACAACGTTGTTGTTCCAGAAGAATCAGTTGAT
ATTGTAGCTGAAATGGAAGAAGAGCTGCAAGAACATAAAGAAGAATCAGCTCGTCTGTTCGAAGAACTCA
ATATGCGCGACGCATATATCAATTATGTGCAGCGTGAAGTGGCATTGAGCGAAAGTACTAAAGATCTGAC
TGAGTCTCAAAAAGAAAAAGTCTCTGCTCTGGTCGAAGGTATGGATTATTCAGATGCATTCTCAAGTAAA
TTGAGTGCCATCGTAGAAATGGTGAAGAAGTCTAATAAAGATGAAAGCACTATTACTGATGGTATAAATA
CTCCTGATACTGAAGCAGCCGGACTGAATTTCGTCACTGAAGCTGTAGAAGATAAAGCTGCACATGGTGC
AGAAGATATTGTAAGTGTATATGCGAAAGTCGCATCTCGTTTCTAATTTTAAAGGTTAACACAAATGACT
ATCAAAACTAAAGCTGAACTTTTGAACAAATGGAAGCCATTACTGGAAGGTGAAGGTTTACCGGAAATTG
CTAATAGCAAACAAGCGATTATCGCTAAAATCTTTGAAAACCAGGAAAAAGATTTCCAGACAGCTCCGGA
ATATAAAGACGAAAAAATTGCTCAGGCATTCGGTTCTTTCTTAACAGAAGCTGAAATCGGTGGTGACCAC
GGTTACAATGCTACCAACATCGCTGCAGGTCAGACTTCTGGCGCAGTAACTCAGATTGGCCCAGCTGTTA
TGGGTATGGTACGTCGTGCTATTCCTAACCTGATTGCTTTCGATATTTGTGGTGTTCAGCCGATGAACAG
CCCGACTGGCCAGGTATTCGCACTGCGCGCAGTATACGGTAAAGACCCGGTCGCTTCTGGTGCTAAAGAA
GCATTCCACCCAATGTATGGTCCAGATGCAATGTTCTCTGGTCAGGGTGCTGCTAAGAAATTCGCTGCTT
TGAAAGCAAGTGATACTCTTGAAGTCGGAACTATTTACACTCACTTCTTCCAGGAAACTGGTACTGTATA
TCTGCAAGCTACAGAAGTTAAACAAATCGATGTCGGTGCTAGTGATGCAGCTAAATTAGATGCTGAAATT
AAGAAACAAATGGAAGCTGGTGTACTGGTAGAAATCGCTGAAGGTATGGCTACTTCTATCGCTGAACTTC
AGGAAGGTTTCAATGGTTCTACCGATAACCCATGGAATGAAATGGGCTTCCGTATCGATAAGCAAGTTAT
CGAAGCTAAATCTCGCCAGCTGAAAGCTGCTTACTCTATTGAATTAGCACAAGACCTCCGCGCTGTTCAC
GGTATGGATGCTGATGCTGAACTGTCTGGTATTCTGGCTACAGAAATTATGCTGGAAATCAACCGTGAAG
TTGTTGATTGGATTAACTACTCAGCTCAGGTTGGTAAATCTGGTATGACCCTGACTCCAGGTTCTAAAGC
TGGTGTATTTGACTTCCAGGACCCAATCGATATTCGTGGTGCTCGCTGGGCGGGTGAATCCTTTAAAGCT
CTGTTGTTCCAGATTGACAAAGAAGCAGTTGAAATTGCTCGTCAGACCGGTCGTGGTGAAGGTAACTTCA
TTATCGCTTCCCGTAACGTAGTTAACGTTCTGGCTTCAGTTGATACCGGCATTTCTTATGCTGCACAGGG
TCTGGCTACCGGCTTCAACACTGATACTACCAAGTCAGTATTTGCTGGTGTTCTGGGTGGTAAATACCGC
GTATATATCGACCAGTATGCTAAACAGGATTATTTCACTGTAGGTTATAAAGGTCCGAACGAAATGGATG
CTGGTATTTACTATGCTCCATATGTAGCTCTGACTCCGCTGCGTGGTTCCGATCCGAAGAACTTCCAACC
GGTAATGGGATTCAAAACTCGTTACGGTATCGGTATCAACCCATTTGCAGAATCCGCTGCTCAGGCTCCG
GCTTCTCGCATCCAGAGCGGTATGCCTTCTATTCTGAATAGCCTTGGTAAAAACGCTTACTTTAGACGTG
TATATGTTAAAGGTATCTAATCTTTGACGATTTAAGGGACCTTCGGGTCCCTTTAGTTGTTTTTATGGTA
TAAATAAATCATATAAACCAAAAGGAAAGCGCAATGGCTAAAATCAACGAACTTCTGCGCGAATCAACCA
CAACGAATAGCAACTCAATCGGTCGCCCAAATCTCGTTGCTTTGACTCGCGCTACCACTAAATTAATATA
TTCTGACATTGTAGCAACGCAAAGAACTAATCAACCTGTTGCTGCTTTTTATGGTATCAAATACCTTAAC
CCAGACAACGAATTTACATTTAAAACTGGTGCTACTTATGCTGGCGAAGCTGGATATGTAGACCGAGAAC
AAATCACAGAATTAACAGAAGAGTCTAAATTAACTCTCAATAAAGGCGATTTATTCAAATATAATAATAT
CGTTTATAAAGTATTAGAAGATACACCATTTGCTGATATTGAAGAAAGCGACTTAGAGCTGGCTCTTCAG
ATTGCAATTGTTCTTTTAAAGGTTCGTCTATTTTCTGACGCAGCGTCAACAAGCAAATTTGAAAGCTCTG
ATAGTGAAATTGCGGATGCTAGATTCCAGATTAATAAATGGCAAACCGCGGTTAAATCTCGTAAACTTAA
AACTGGCATCACAGTTGAATTAGCGCAAGATTTAGAAGCAAATGGATTCGATGCTCCTAATTTCTTGGAA
GATTTGCTTGCAACTGAAATGGCAGATGAAATCAATAAAGATATTCTGCAATCTTTGATTACAGTGTCAA
AACGCTATAAAGTTACAGGAATTACTGATAGTGGATTCATCGATTTGAGTTATGCGTCTGCACCTGAAGC
TGGTCGTTCATTATACCGAATGGTATGTGAAATTGTTTCGCATATCCAAAAAGAATCAACTTATACAGCA
ACGTTCTGTGTTGCTTCTGCTCGTGCCGCTGCGATTCTTGCTGCATCAGGTTGGTTAAAACATAAACCAG
 
AAGATGACAAATATCTTTCACAAAATGCCTACGGGTTATTAGCTAATGGTTTACCGCTTTATTGCGATAC
TAACAGCCCATTAGATTATGTAATCGTTGGTGTAGTAGAAAATATCGGTGAAAAAGAAATTGTTGGATCA
ATTTTCTATGCTCCGTATACAGAAGGTCTCGACTTAGATGACCCTGAACATGTAGGCGCATTTAAAGTTG
TTGTTGATCCAGAAAGCTTACAACCGTCTATCAGTTTATTAGTTAGATATGCTTTATCAGCAAATCCTTA
TACCGTAGCAAAAGATGAAAAAGAAGCAAGAGTAATTGATGGTGGAGACATGGATAAAATGGCGGGTCGT
TCAGATTTGTCTGTTTTATTAGGTGTTAAATTACCAAAAATTATTATTGATGAATAAAACAAAGGGACCG
AAAGGTCCCTTTTTATTTAGCTCACCAACTCAATCCAAGCTGGACGAAGTACATCTTACCATTTTAACTA
ATTCCTTTTTAATCAAAGAAGGATTATCTGCTTGAGTTAGAGTAATACCTTCACGAGAAGTTTCTTCCAA
AATATCTTGAACAGTTAGCCCCATCACCTTTCCAAAATCCTTTGGACCAATTTCGCCAATTTTAGAAATA
ACGTTATTTACACGGTTCAGTGTAACGTAACAAGCTAAAATTCCCACCAATTTGTTATCAGCTTCTGATA
GTTCAACTTTAGCTTTAATAGGCTTATCAGACTTTTTCTTTTCACTAAATTTGGAATTCTTGCATTTAAT
TGCTACACGATTTCCATTATGAAGCCAAGAAGGATAACAAGGTTTCAATACATATCCTTCAGCAGTAAAT
ACTTCGCCTTTTGCTTCGGCATTCCAAACGCATTTATTTGCATCAACTAATCCAGCATGGTCTACTGTAA
AATTATAATCTTGGACGACAGAATCTAAATCATTTGGCAATTTAATAAGCTCTTCAAATTTACCGCGACC
TAAAAGTGGAGCAATTTTAAATTTAAATGTATTACAGAATGATTCCATCATATAATCATCAACATAAGTC
ACATCACCACTTTCTGTAGTGACAATAATGTCAAATACATAAAAATCTTTATCGCCATAATCAACATTCT
TCTGAATGCCAGGTCCAGCGAATTCGCCAAAGACTTGATAAGATACAACCGCTGAGGTTTCCATAATATC
TTGTACTGCTTTAATGGAATCAGCGTAATTCTTTAGAATAATTTCATACCCAAAGAAATCTTCAGCAGGA
AGAATCGGTCCAGTACGTTTAGCACAAGTTACTTTATCACGCTCAATAATCAATGAGAAATTTGTGCCGT
GAATCTTTTCACGAGCTACCCATTCGCCGCCAGTCAATCCCAAGCTGTAAAGTTTTTCAATAAATTTAGA
GTTGTAATGATTTTCAAGACTGCTACTTTTTAAACATAATTAATCCTCAAAATGTAATTTCTAACCAATC
ACCATCACGCTGATCACTATTGACTTTAAAATTAAATCCTTCTTTTCTCAGCCAATCACCAATTTCTTCA
GTAATCAATTTATCGCGAGCAGTATAATAACTAAAATATGTTTTACCTTGTTCAGCTGCTTTATTAGCAA
GTTCCGAAAAATCTTTAATAAAACACTCTAGCTTAAACTGTTTACTTTTTAATGCTTTTTCGCGTAATTG
ATTAGCAAAAGATTCATTTTCATAAAGTTCATACTGTTCCATTTTTCACCTTTTTATTGATATGTCTTTT
TCTATAGACAACTTTTTCTCGAGTTTCAAATTTATTCTTAGCAGTCATAGCTCGAGCCCATAATACAGCC
ACTTCTTTTGCATGTAAGTTTAATTCACGAGCAATTTCAATGAATGACTTTCCAGACTCATGAAGAGTAA
ACACCACAACCTCAGTTCTCATATTCAATCTCATGTTATCGAGTTGGTGCCATTATATACATCATTTTCT
GATTGTGTTTTGTGTGCTTTCAAAATGAAGAAAGGGGCCGAAGCCCCTTATGATTATGGATAGGTATAGA
TGATACCAGTTTCTAAAGCAGTTTTATGAATGATGTATCCATTACGCGATTCTTGGACATCAACTTCTGG
ATAGTCTTTCATCATCTTCTGAAGAGTGTAACGATGCAGGTAGTATTTACTCTGGATCTTCAGTTTTCCA
ATCTTTACCTTCTTCAGTCATTTTTTGGATTTCATCCATAACCCACCAACCGCACCAGATGTAAGCTGAA
GTACGATGTGGAAGAGGATGAACATAAGGTAATTCACCTTCTGGTTCTGGAGCAACTTTATCAGTTACCT
GAACTTGACCTGTTGTTTTAACTGTTTTGCTATCATAATCAGTAGCAGTAACGACGGCAGTCACTTCAAT
AGTTTGACTTCCAACAGATGAAGTATCAACGGTATATACATTAGTTGACCCTTCTACAGGAGAAGAATCT
TTCTTCCATGAATAAGTAATTTGCGCTTCTTCTGGAGCATCAGTAACATTAGCAGTAAATGTAGCCGAAG
CATCTTGTTGAACGTTAATAGAAGGAGGAGTCAATGTAACCTGTGGATTCATTGTCTTCTTATTAACCGT
TAATGACACTTCATTAGAAGTAACGCTTAGTGCATCATAATCTGTCGCGGTTACTTGAGCTACACACTTG
ATTTTTTAACTCCACTTGTAGTTGGAGTATAGCTAAATGTAGAGTTAGTTTCTCCACCAACTTGTGAATC
ATCTACATACCACTGATACGTAGCAGATGCTCCATCAGGTTGAGAAGCTAAGGCAGCAGTAAATTGAACT
GGGGTTCCGATTACTCCAGCGTCAGGGCTATTAGGAGTTACAGCTAAGGTGGTCGTTTGTGTCTTATTTT
GAACTGTGATAGTTGTAGTAGCTTCTGCTGTTTCAGCTTCAGCTTCTGGAATTGTATTTGTTGCAACTAC
TTTAATAGTCTTTTGACCGGCAGGTATAACTAAAAGTTGCTTCAGCTCCATCTTGTGGAACATTATCTAC
GCTCCAAGCATAGGTAATAGTTCCACCTTCAGTTTGACCACTGGGTGCAGCAGTAAACTGCTTGGTTTCA
 
TCAATAACCCCTGTCGGTGTTTTAGGAGTTATATCAACTGTAAAAGTCATAAGTTATCCTTATTTTAATG
TTACGAAAGAAGAATTGCGTGTTTCACGAATTAAAACTGATCCGTCGCGGTTAATGTAATAAATTAAGCT
AAATAAAGTTTGGTGTGCTGACGCATGTTCAAAACTAGTTGGGTGAGATTTCCAATCCGGAGTTTCAGCA
ATCCATTGATAAATCCACCAAGGAACAGTGCAGAATCCTGGATTTTTTCCAATCAGCTGAAGATTCGGAC
TAAAGTTTTCCGGAAGAGTAAATACAGACGGCTTTTCAGATTCAATAATCTCAGCTACAGCTTGTTCAAA
CTTTTCTTCAACAAAAGGAGTATCTTCAATCAAAACATCGGTATTTTCAGGAATTTTATCCGTTTCTACT
ACTTCAATTTTAATGTCAGATTTAACCGGAGAATCAATCAGAAGTGCTGCTTCTGGATTGACTTCTTCAT
CGTCATATTTTAATCCCTCTGCGGCATCAGCAGCATCAATTAAGTCTTTAATAGATAACCCATCAGTCTC
TGGCATAGGTTCACTAGCGAGCTTCTGGAGGGCTTCTTCAATATCAACAACGATATTATCAAAAGATTTA
TTCTTTTTGACCTTTATACCAAACTGTTCAGCATATTCAGCTAATTTAGCTTTAGCTTCTTTGTTATCAT
CAAGAGCCTTCAGCTCTGCAATATAATCTTTATCTATCATAATATTTCCTCAGTATAAATATAGATATAT
TTATTACATGGTATTTAGACATGACTGACATTAAAGTACATTTTTATGATTTTAGTCACGTGAGAATTGA
TTGTGAAGAAAGTACATTTCATGAACTCCGTGATTTCTTTAGCTTTGAAGCTGATGGGTATCGTTTTAAT
CCGAAATACAAATATGGCCACTGGGATGGACGAATCCGTCTTTTAGATTATAATCGTCTTCTTCCATTCG
GCTTAGTCGGGCAAATTAAAAAGTTCTGTGATAATTTTGGCTATAAAGCCTGGATTGACCCACAAATTAA
CGAAAAAGAAGAATTATCAAGAAAAGATTTTGATGAATGGCTTTCTAAATTAGAAATCTATTCAGGAAAT
AAAAGAATCGAACCACACTGGTATCAAAAAGATGCAGTGTTCGAAGGATTAGTTAATCGTCGTAGAATTC
TTAATCTTCCAACATCTGCAGGTAAATCTTTAATTCAAGCTTTGCTTGCGCGATATTATTTGGAAAATTA
TGAAGGTAAAATTCTTATCATTGTTCCAACAACTGCTTTAACAACTCAGATGGCTGATGACTTTGTTGAC
TATCGTTTATTCAGTCATGCAATGATAAAGAAAATTGGTGGCGGAGCATCAAAAGATGATAAATATAAAA
ATGATGCACCAGTCGTTGTTGGTACATGGCAAACTGTAGTTAAACAACCGAAAGAATGGTTCTCACAGTT
TGGAATGATGATGAATGATGAATGCCATCTTGCTACAGGAAAAAGTATTTCATCTATCATATCAGGTTTA
AATAACTGCATGTTCAAATTCGGTTTGTCTGGTTCATTACGTGATGGCAAAGCTAATATCATGCAGTATG
TTGGAATGTTTGGTGAAATATTTAAGCCAGTAACGACTTCTAAATTAATGGAAGATGGACAAGTAACTGA
GCTAAAAATTAATAGTATTTTTCTTCGCTATCCAGATGAGTTCACTACTAAATTAAAGGGAAAAACTTAC
CAAGAAGAAATAAAAATTATTACTGGGCTCAGTAAAAGAAATAAATGGATCGCTAAATTAGCTATTAAGC
TTGCGCAAAAAGATGAAAACGCTTTTGTCATGTTTAAACATGTATCGCATGGTAAAGCTATTTTCGATTT
AATTAAAAATGAATACGATAAAGTTTATTACGTATCAGGGGAAGTTGATACCGAAACCCGCAATATAATG
AAAACCTTAGCTGAAAATGGTAAAGGAATAATTATAGTAGCTAGTTATGGTGTATTTTCTACTGGTATTT
CAGTTAAAAATCTGCATCACGTTGTTTTAGCGCACGGTGTTAAATCAAAAATCATTGTATTACAAACAAT
CGGTCGTGTATTACGTAAGCATGGTTCTAAGACAATAGCAACCGTCTGGGACCTCATAGATGATTGTGGT
GTTAAGCCAAAATCTGCTAATACGAAAAAGAAATATGTTCATTTGAACTATCTTTTAAAACACGGCATTG
ATCGTATTCAGCGCTACGCAGATGAAAAATTTAATTACGTAATGAAAACAGTTAATTTATAAGGGCTTCG
GCCCTTTGGAGAAAAAGATGTTACTAGGATTTAAACAATTTCTTTATGAAGCTTCTATTGATGAATTTAT
GGGTAAAATTGCCTCTTGTCAAACATTAGAAGGTTTAGAAGAACTTGAAGCTTATTATAAGAAAAGAGTC
AAAGAAACTGAATTAAAAGATACTGATGACATCTCTGTAAGAGATGCTTTGGCAGGAAAAAGAGCTGAAT
TAGAAGATTCAGACGATGAAGTAGAAGAAAGCTTTTAAATTAAAAAAGGCCCAACCAAAAAGGAAGGGCC
AAAACTATAGACTAAAGGTCACACTATAGCAAAAGTTGTGTTTCATTTAATTGTTCTTCCGAACTTTCTG
AAACTGGTAGTTCTTTAATGTAATTATAGCAAGGCCCAGGATGTACAGGACCTTTGTCTGTTTCAACAAC
CAATGCAGAATCGATTGGAGTTTTACAGACAACACAAATCTTATCTGACATGATTGTCTCCTCTGAATTA
TATCTATTTATACAACTCTCATATGCATATCAATGCCCATATCTTTAGAATAAAAATATTCATCAAGATA
TCCGGCAAATTTTCCTTTAATATAAAGGACATCTTCACCACACGGGTGATCGGCCAGGATACGAATATCC
TGACGCTTAAGATTATGCTTTTTCATTAAGAATTGAATCTCAGTTTCAAATTCTTCTTCATAATTAAAAG
CATCATCAATGCTATATCTCATTATTTTCCAGCCTCAAATGCCCGCATGTCTTGAATATGCTTAATAGCA
 
AATCCGCGTGATTTGATAGCATCAAGAGCTCCGCTACAGAAATCTAATAAAATCCCCCAATACTGTAACG
AGGTATCAACCTTTAAAACATCCTTATCCGCTGATAGAACTGTCTTCATTTCTGATTTCTCGTAACGATC
CATACTAAATTCATCACCATCTCCTCGTCCCGAGTAGTAGTCTAATTTAGCTTTAAGAGCAACTTTTTTC
TGTGCGTCAATTCTAAGCATTTCCTTTTTAATACTTGAATGCTTATTAAGCCATTTACTGTATAACATCA
CATTATTAGCTGCTTCATACTGTAATTTAGTTGAATCTATAAACACATCTTTCTTCAATTCTTCTTGAAG
ATCTTCTAATCTCATATTGTTCTCTATTCAATTGTTATTGGATGGACTTAGATTCATTATACCACGTTTT
AACGTGAAGCATTATACCCTATTACTCGAAGCCAGTTGCAGTTTTATCTGCTCAATATCATCAGGGTTAT
CGATGACCGAAAAGCGTATTTCTACTATCAGAGTATAATCATCATAAACTGGTATCACATTAACTGATAA
TTTATCAATACGTGGCTCATAGTTTCTTACTGCGCTTTCGATATTGCGCTCAACCGTGTCAGCAGTAAGA
GGAGTCATATTTTCAAAAAGCTGATCTGATAAATCACATCCAAATTCAGGGTCAAACGGTCTTGAACCTT
TTCTTGTTGTAATAATTCCCAAAAGACTGTTTTTAATTGACCTTAATCCAAGTGATCTGGAAACATCTTT
GTTCCAATCCATTTTCATTTCCGGGTCAATGTCAGAATAAAGCTTATTAATATTTGCCATTATAGTAACT
CAAAGAACTCTTTGAGGCCTCTTATTACATGAGCATGGGTTTTTCCACACTCTGGACATTTAATTGGAAC
AGCCAAATAAACGGTAGGCTTTAAAAGCATATCTTTTATAGCTACAATATCTGACTCTGTGATGATAGAA
TATAAATCTTCTAGTTCTTTTTCATTTAAGTCTTCAACTGGAATGCTTTCACCGTTAGCATGAATCGTTT
CTATGCATGATACTATCATGTGGGCTATATTTTTATCATCAAAAATTTTAGGGTATCGGAATTTAATTTT
AATGTCACCCAGTGTATACCAGAGGTCTTCTGGTGCATCTATTTGTGTATGTAATAGATTTATATGGGTT
GGTATTTCAGTTCCACAGGTGCACTTCCAGGAGTTTTCGTGATTAACTTCTCCAAGAGAATGCGCCCATA
AATGAATCAGCAATAGTTCTGATTCTTGGCGGTTTAAATCTTTTGCATTTGTGCAGTCTTTGATTAGCTT
TTTAACAATTACTTCTACGGAACCATTATTTTTGGCAGTAATAAGTTCTAGATATTCTTTAAGCGTGAAT
GCGCGACAATTGATTATTTTAGAACCAACTCTCACATCAAATTTGTATTCATACATATTTAGCTCCTTTA
TTTATCATATTTATAAATAGAATAAAAGGAGCATCTATGGCAAACATTATTCGTTGTAAATTACCAGATG
GTGTTCATCGTTTTAAACCATTTACGGTAGAAGATTATCGAGATTTTTTGTTAGTTCGAAACGATATAGA
ACATCGGTCACCACAAGAACAAAAAGAAATAATTACTGATTTAATTGATGATTATTTTGGAGACTATCCG
AAGACTTGGCAACCATTTATATTTTTGCAGGTATTTGTAGGGTCAATAGGTAAAACTAAAGTACCGGTCA
CATTTGTATGTCCAAAATGTAAAAAAGAAAAGACAGTTCCATTTGAAATATATCAAAAAGAATTAAAGGA
ACCTGTTTTTGATGTAGCTAATGTTAAAATTAAATTAAAGTTTCCTTCTGAGTTTTATGAAAATAAAGCA
AAGATGATTACTGAAAATATTCATTCTGTTCAAGTAGATGAAATATGGTATGATTGGAAGGAAATTAGTG
AATCAAGCCAAATAGAACTTGTTGATGCCATCGAGATAGAAACATTAGAAAAAATTCTCGATGCAATGAA
TCCTATTAATTTAACTCTACATATGTCATGCTGTAATAAGTACATTAAAAAATACACTGATATAGTAGAC
GTGTTTAAGCTGTTAGTTAACCCAGATGAGATATTTACTTTTTATCAAATTAATCACACACTCGTAAAAA
GTAATTATAGCTTAAATTCAATAATGAAAATGATTCCTGCCGAGCGCGGATTCGTATTAAAACTGATTGA
GAAGGATAAACAATAATGAGTATGTTGCAACGCCCCGGATATCCAAATCTCAGCGTTAAATTATTTGATA
GCTACGACGCTTGGAGTAATAATAGATTTGTTGAATTAGCTGCTACTATTACCACATTAACTATGCGGGA
TTCTCTTTATGGACGAAATGAAGGAATGCTGCAGTTTTATGATTCTAAAAACATCCATACAAAAATGGAT
GGAAATGAAATAATTCAGATTTCTGTAGCTAATGCAAATGATATTAATAATGTTAAAACACGAATTTATG
GATGTAAGCATTTTTCCGTGTCAGTAGATTCAAAAGGTGATAACATCATTGCTATTGAATTGGGAACTAT
TCATTCTATAGAAAATCTTAAATTTGGTAGACAATTTTTCCCTGATGCAGGTGAATCTATAAAAGAAATG
CTTGGTGTCATTTATCAGGATCGCACATTATTAACTCCAGCAATAAATGCTATAAATGCTTATGTTCCTG
ATATTCCATGGACTAGCACATTTGAAAACTATTTGTCATATGTAAGAGAAGTTGCTCTAGCTGTAGGAAG
CGACAAATTTGTATTTGTATGGCAAGACATCATGGGAGTTAACATGATGGACTATGATATGATGATAAAT
CAAGAACCATATCCAATGATTGTCGGTGAGCCATCTTTAATAGGTCAATTCATCCAAGAATTAAAATATC
CATTAGCATATGATTTCGTTTGGTTGACTAAATCGAATCCTTACAAACGTGATCCAATGAAAAATGCTAC
TATCTATGCTCATTCATTTTTAGATTCTTCACTGCCAATGATTACTACAGGAAAGGGTGAAAACTCTATT
 
GTAGTGTCAAGATCAGGTGCTTATTCTGAAATGACTTATAGGAATGGATATGAAGAAGCTATTCGTCTTC
AAACTATGGCACAATATGACGGTTATGCTAAATGTTCTACTGTCGGTAATTTTAACTTGACTCCTGGTGT
TAAAATTATTTTTAATGATAGTAAAAACCAATTTAAAACAGAATTTTACGTTGATGAAGTTATCCATGAA
TTATCCAATAATAATTCAGTAACTCATCTATATATGTTCACTAATGCAACGAAACTGGAAACAATAGACC
CAGTTAAGGTTAAAAATGAATTTAAATCTGATACTACCACTGAAGAAAGTAGTTCTTCCAATAAGCAATA
AAGAAGTTTCTATTCCTAAAATGGGTCTTAAACATTATAACATTTTAAAGGATGTTAAAGGTCCTGATGA
AAATTTAAAACTTCTTATTGATTCTATTTGTCCGAATTTATCACCGGCAGAAGTTGATTTCGTTTCTATT
CATTTATTGGAATTTAATGGAAAGATTAAATCTCGTAAAGAAATAGATGGCTATACTTATGACATTAATG
ATGTTTATGTATGCCAAAGATTAGAATTTCAATACCAAGGAAATACATTTTATTTTAGACCTCCTGGAAA
ATTTGAACAATTTTTAACGGTGAGCGATATGTTATCTAAATGCTTGCTTAAGGTCAACGATGAAGTTAAA
GAAATTAATTTTCTTGAGATGCCAGCATTCGTTTTAAAATGGGCAAATGATATTTTTACAACTTTAGCAA
TTCCTGGCCCTAATGGTCCAATAACCGGAATTGGCAATATTATTGGATTATTTGAATGAAAAAGCCACAA
GAAATGCAAACGATGCGTAGAAAAGTTATTTCAGATAATAAACCAACACAGGAAGCGGCTAAATCCGCTT
CTAACACTTTATCTGGACTTAATGATATATCTACGAAATTGGATGATGCTCAAGCTGCTTCTGAATTAAT
AGCTCAAACTGTCGAAGAAAAATCGAATGAAATAGTTGGAGCAATTGGTAACGTAGAAAACGCAGTGAGT
GATACTACTGCCGGTTCTGAGTTAATTGCTGAAACTGTCGAAATTGGCAACAATATTAATAAAGAAATCG
GTGAATCACTCGGAAGCAAATTAGATAAATTAACAAGTTTACTAGAGCAAAAAATTCAGACAGCTGGAAT
TCAACAGACTGGAACTAGTTTAGCCACAGTTGAAAGCGCTATTCCTGTTAAAGTCGTTGAGGATGATACT
GCTGAATCTGTGGGTCCTTTATTACCGGCTCCCGAAGCAGTTAATAATGATCCTGACGCTGATTTTTTCC
CTACCCCTCAGCCAGTTGAACCCAAACAAGAATCGCCAGAAGAAAAACAGAAAAAAGAAGCATTTAACTT
AAAATTATCTCAAGCTTTAGATAAATTAACAAAGACTGTTGATTTTGGATTTAAGAAATCCATTTCAATT
AGTGATAAAATATCAAGCATGTTATTTAAGTACACCATCAGTGCTGCTATTGAAGCTGCTAAAATGACTG
CAATGATATTGGCTGTTGTTGTTGGAATAGACCTGTTGATGGTTCACTTTAAATATTGGTCAGATAAATT
TTCAAAAGCCTGGGATTTATTTAATACTGACTTTACTAAATTCTCTAGCGAAACCGGAACTTGGGGTCCT
TTATTACAGAGCATCTTTGATTCTATTGATAAAATTAAACAACTTTGGGAAGCGGGAGATTGGGGTGGAT
TGACAGTAGCTATTGTTGAAGGGCTTGGAAAGGTTCTTTATAATTTAGGAGAACTTATTCAGCTTGGAAT
GGCTAAATTATCTGCGGCAATTCTTCGAGTCATTCCTGGCATGAAGGATACTGCTGATGAAGTAGAAGGA
AGAGCACTAGAAAATTTCCAAAATTCTACTGGAGCATCTCTCAATAAAGAAGACCAAGAAAAAGTAGCAA
ATTATCAAGATAAACGAATGAATGGAGACCTTGGCCCAATAGCAGAAGGACTAGACAAAATCTCTAACTG
GAAAACTCGTGCATCTAACTGGATTCGTGGTGTAGATAATAAAGAAGCACTGACTACTGACGAAGAACGT
GCAGCAGAAGAAGAAAAATTAAAGCAGCTTTCACCTGAAGAAAGAAAAAATGCTTTAATGAAGGCCAATG
AAGCTCGTGCCGCGATGATTCGTTTTGAAAAATATGCTGATTCAGCTGATATGAGTAAAGACTCAACGGT
TAAATCAGTTGAAGCTGCCTATGAAGACCTTAAACAGCGGATGGATGACCCGGATTTAAATAATTCGCCG
GCAGTTAAAAAAGAACTTGCTTCTAGATTTGCTAAAATTGATGCTACTTATCAAGAGCTCAAGAAAAATC
AGCCTAATGCCAAACCTGAAACTTCTGCTAAATCACCAGAAGCGAAACAGGTCCAGGTTATTGAAAAGAA
CAAAGCACAACAAGCTCCTGTTCAACAAGCATCTCCTTCAATCAATAATACTAATAATGTTATTAAGAAA
AATACTGTCGTTCATAATATGACACCTGTTACGAGCACAACTGCTCCTGGTGTATTTGGCGCGACTGGAG
TTAATTAAGGAATAATATGGCAATTGTTAAAGAAATAACTGCTGATTTAATTAAAAAGTCCGGTGAGACA
ATTTCAGCCGGACAGAGCACTAAATCAGAAGTAGGAATTAAAACATACACAGCCCAGTTTCCAACTGGGC
GTGCTAGTGGTAATGACACTACAGGGGACTTCCAGGTAACAGATCTATATAAGAATGGATTATTATTTAC
TGCATACAATATGTCATCTAGGGATTCTGGAAGTCTTAGATCGATGAGATCTAACTACTCTTCTTCATCT
TCGAGTATTTTACGTACAGCCAGAAACACTATTAGTAGTACAGTATCAAAACTATCAAATGGATTAATAT
CAAATAATAATTCAGGAACAATAAGTAAAGCTCCTGTCGCAAACATTCTTTTACCGAGATCTAAATCTGA
TGTTGATACATCATCACATAGATTTAATGATGTTCAAGAAAGCCTTATCAGTAGAGGCGGAGGTACTGCT
 
ACTGGAGTGCTAAGTAATATTGCTTCAACCGCAGTATTTGGGGCGTTGGAAAGTATAACACAAGGTATAA
TGGCTGATAATAATGAACAGATTTATACGACAGCCAGAAGTATGTATGGTGGTGCTGAAAATAGAACTAA
AGTGTTTACATGGGATTTAACTCCACGTTCAACAGAAGATTTAATGGCTATTATTAATATCTATCAATAT
TTTAACTATTTTTCTTATGGTGAAACGGGTAAATCTCAATATGCTGCTGAAATAAAGGGGTATTTAGATG
ATTGGTATCGTTCTACGTTAATTGAACCTTTATCTCCGGAAGACGCAGCTAAAAATAAAACACTATTTGA
GAAAATGACATCGAGTTTAACTAACGTTCTAGTAGTTTCAAACCCGACGGTTTGGATGGTGAAAAACTTT
GGTGCAACATCTAAGTTTGATGGAAAAACGGAAATATTTGGTCCATGCCAAATACAGAGCATCAGATTTG
ATAAAACACCTAACGGTAACTTTAACGGATTAGCTATTGCTCCAAATCTCCCTAGTACATTTACTCTCGA
GATTACTATGAGAGAAATTATCACGTTAAACCGTGCTTCTTTATATGCGGGGACTTTTTAATGTATTCTT
TAGAGGAATTTAATAATCAAGCAATAAACGCAGATTTCCAACGTAATAATATGTTTAGCTGCGTTTTTGC
AACAACTCCATCAACTAAAAGCTCTTCGTTGATAAGTTCAATTAGCAACTTTTCTTATAATAACTTGGGC
CTAAATTCAGATTGGTTAGGATTAACTCAAGGTGATATTAATCAGGGAATTACAACGCTAATTACAGCTG
GCACACAAAAACTAATAAGAAAATCGGGGGTTAGTAAATATCTTATTGGTGCCATGAGTCAACGTACAGT
TCAAAGTTTATTAGGCTCATTTACAGTTGGTACATATTTAATTGACTTCTTTAACATGGCATATAACTCA
TCTGGATTGATGATATACTCTGTAAAAATGCCAGAGAATAGATTATCCTATGAAACTGATTGGAACTACA
ACTCTCCTAATATTCGTATAACTGGGAGAGAATTAGACCCTTTGGTTATTTCATTTAGAATGGATTCAGA
ATCGTGTAATTACCGTGCAATGCAAGACTGGGTTAATGCTGTTCAAGACCCAGTAACTGGATTACGTGCT
CTGCCACAAGATGTCGAGGCAGATATCCAGGTTAATCTTCATTCTCGTAATGGATTGCCTCATACTGCGG
TGATGTTCACCGGATGTATTCCAGTGTCAGTGAGCGCTCCTGAGTTATCATATGATGGAGATAACCAAAT
AACTACATTTGATGTTACTTTTGCGTATAGAGTCATGCAGGCTGGAGCAGTTGATAGGCAAGCTGCGCTT
GAATGGCTTGAATCTGCTGCTATAAATGGTATTCAAAGCTCTTCTGGAAATAATGGAGGTGTTACTGAAC
TATCTAGTTCGCTTTCACGACTTAGTAGATTAGGAGGAACTGCAGGAAGCATTTCAAACATTAATACTAT
GACAGGGATTGTCAATTCGCAGAGTAAAATATTAGGAGCAATATAACAATGGGGACCTTTCGGTCCCCAT
ATTTTTATTTACGGAATGAAATGAAAGCAGAAACTGAAGCAACTAAACTGTCTTCAATATAAACTTCAAT
TTTTACCGGAGCTTCTGACTCAAATTTACCTGTTACTACACCCTGGAAAATGCTTTCAGTCTGTTCTGGC
TTTGAAAAATTTTCAGAAGGAAAAATTCCGAACTTTTTATCTGTTCCAAAAATTTTAATAAATTCATCGT
AAACCGCTTCGTTAAAAGCATTATCAGCAGGAATAACGCCTTCAACTACAAGTTCTTGACCTAAAAAGCG
CAAAGAAGATTTCATTTTGTGTTCCTCATGTTATGTCAGTAAGATTATTATAACACAACATAAGGAACTT
GTAAACTACATTTTGAACTTTTTAGACATTCCGTTGATGTTAACCAAAGAAGCTAATATTTGAGAAACTT
CGTTTTCCTCTTCTTTTGATTCTTCTACAAGGAATTGATTAAATCCAGCCATTGGACGAACTTCACCAGT
TTCCATTAAATGGTCTCCATCATATACTGATTCTTCTAATTGTTCAGAAGGAGGAACGATTGTACCTTCG
ATTAGATACTTGTTATAAGCAGTTTCTTTCATAAAGTACGTACTTACTTTATTAATCTTCAGTAAAATTC
CACGCGGTAGAATAACTTCAGCTTCTGATGGAAATCCTGATAAATGACCTGGAACGATAACTTTGATTCG
CTCAGCTCCTTTAATTACCATACCCACGCTAGTTTCAGCATCTTCATTAGCATATGTAGAATGACTACCC
ATATGCATCAAATCTTCTGCATCAACGGAACCTTCTCCTTCTCCAGAAAATACTGCACCGGAATCATCTA
GAGCCATATAGTTTTTACCATGCTCGCCAAAGATATTTGGTTTAAGTGATGTCGATACGAAGTTCTTGAA
ATAGAACATTTTGTTTTCAATGTTGTGACGCAAAGTTTTAAAGGTAACTTCTTGTCCGCGATAAAGCGTA
GTTCCTGGAGGAAGAACTGAACCTTTAGCGAATGCAGAATCCAACGTCTCGATGTGCTTAATAGCACGTT
CCATATATTCTTCACGGGTAGAATCTGGTTTACCCAAAAGGAACGAGTTCATTTCAACATATGAATCTGC
ACAATAATCTTCAATTACATCAGACTCAGCCTGAGTCAGTCCTTTTGGAGTTACTTCAGTGCTTGCATAT
TCATATGCCTGGGCAGCAGATTCATTAATCAATTTATAAATGCCATTCATGACAAATTGAATAATAACTG
TCTTTTCATCTGGCGTAGCTTTAGAACCTTTAACCAAATCATTGATAATTTTCTTAATAACATCAAAGCT
ATTAGAAATATTAATATCAGCAAGATCACTTAAAATTTTATCAGTTTTATAATCAAAGAATTCTTTATTC
TTTAAATATTCTTGAGAACCTTGATATGTTATATCGCTCTGTAACTTGATAGATGGATCACGTAAACGTG
 
CATGGGCTAATGCAATATGATTTCGGAAGTTTTCTAGTTCATAATCGCTAGATTTAAAACGAGAAGCACT
TCTCATACCACCGATAGCTCCTTTGCTGAAGTTTGAACCAATCTGAATAACGCCAGAGTTCATTGGTCCT
TCTGCTTTATATACCGGAACTTTAGATTCAAACTCTTGATATTTCTTGGCAGCGAAGCTATCACCCTGAA
GTGAAGCATCGACAGTAGAATATTGAGCTTGTGCTGCTAATCGACGACTAATTTTAGTTTTCTGAATAAC
CGCTTGGTCAGTACGTTTATCATTTTCTAAAGCAATAGAAGCAGCAACAGCGACAGCTTTAGGAACAGCA
TCACCTGTTTTAACATCTACATAAACATCACCGACCTTCGATTCAACTTTTGCGAATAACTCAGTTGAAA
TAGGAGGTACACCTGGAATGTCTTCTAAATTGGCATTTTTACGGTAAACCATAATATATGCATACTTTTT
ATCATAGTCCCAGATTTCCTTTAAAAGAACATATCGACCTGCTCCTTTAGTACGTACTAAACGGTCAGCA
ATAACTTGCATTTGACGAGCTGCGCCATTTGTTTTACTTTTTAGTACGCGGAATAAGCATGCGTCGATTT
TATACTGTTTCATTGTTTGAAGAGCAGTATCAAAAACAGCATTAATGACGCCAATTGGATTTCCACCCAA
GTTTCTAAGCTTAACCAGCGATAATTTCTCGTTGACACCTATCATTACGATATGCATCATTTTATCGCCT
GGCTTAACGTTTTTATTTGTATCACCACCAGAGGTGTATGTACAAAATCTAAATCCAGGAAGCACGCTTT
CTTCGCCGGCTTGAATAGCAAAAATTTGTGGTATTTTATGCTTAGGATTTAAGTTAACAACCGGCGCCGA
AGCGCCGTCAAATAATTCTGTAATAAGTTCCATGATTTATCCTTGAACGAACTTGTAAGGCATGTTTGTA
ATATCTATGCAAGACGCAATAATTCCGAGAGATGATTCTACTTTCTGTTTAACATTTGATGAAACAAATG
ATGCAAAGTCAACTCTGTCTTCTATTTCAATATCACCTGTCATTGCAACTACTTCGCCGGTTTCCATTAA
ACGGTCTCCGTCATAGATTACCGATTCGGTGAGTTCTTCTGTGGTCATAACTTCAGCTTGAATGAGTTTT
GTATTATTAGACGTGGTTCCGTCATTATTAGAAGCATCAGTTATTTTATTAACTTTGACCATCAATCCGC
GCGGTAGAATAACTTCAGCTTCTGTTGCTATTCCGAGACTTCCTGGATATACAACATTAACTTTATGAGC
TCCATCAATTGCCCATCCTATTTGAACTTTAACGTATTCAGGATTTTCTTTATACGCTCTTATTTCGTTT
GGATTAATAGTTATTCCTTCTTCATTTTTATCAACTGTTAATTCATTGCGAGCTTCTGGTTCTAAAAGAC
CAATACCAGCATGTGTAATTCCAAAACGTCCAAAAATGATAGGAGTTAAAGAAGTAGATACAAAATTTCT
GAAATAGAACACTTTATTTTTAACTAGCGCTTCGTATATAGGAGCAGTCATACTTTGAGCACGATAAACT
GTAATGCCTTCCGGTATGCGGTCACCATTTTCAAAAGCTGAATCTAAATTATTTATAGCAGATTCAATTT
CTTTTTCACTCATTACATCATATCGTTCTGGATTGTAACGACCTAAAAGAAGATTATTAATGTCTGTATA
TCCTGAACCACAATACTCTCTAATACCGCGCTTTTGTGATGGTGTATATTTAGAACCATCACAAGCAATA
GTAATATCAATCATTGGACCAGCACCAATTCCAGAAAAACTATAAATCATTCGTTTGAGGAATTTAGTTT
TTGCTGCATTCCAGGCATTTTTAGTTATTTCTCCCCAGACTTCATCTGGCTGCCCTTTACGAGTTTTATT
ATGTTTATCAAGCCAATTATCTTTATATTCTTCAAAGAATTGATTGACAGCTGCCGCGTATTTCTCTAAA
CATTTTATTGAAGTAGTTGGTTCATTTTTAATCGCGTTAAAAATTACATCTTTAATTTTTTCAGAAACTT
CATCAACCTCACTTTTTGAAAAGAATTTATAGTCATAGTGAAGTTCATTCGCAGTTCCTTCGGCAATAAT
TTTTGCTGCCTTTGTTTTAGAAGCTAATGAATTAATTCCAATTTTCATTTCTTCTGCTTCGGGAATTAAA
GGAGCGGTAGCAGGCTTATTGAAATTAGCTGCGGTAGATTCATACTTCTGGAATAATTCACTTTCAAATC
TAGAAGATTCCAATGATTGACTTTGCGCAATTGCTCTACGGGAAATTTTAACTTTAACGATTACAGCTTG
GTCAGTACGTTTATCATTTTCTTGTGCAATAGATGCTGCAATTGCCTCGTTTTTAGTTACTTGAGCTCCA
GTATCTTTATTGATATAAACATCACCGACCTTCGATTCAACTTTAGTAAAGAGCTCGGTACTAATTTCCG
GAACTCCTGGAATGTCTTCTAGTGATACATTTTTGCGATGTATAAGAATATATGCATACTTTTTATCATA
ATCCCAGAGTTCCTTAAGAAGGACGTATCTGCCACCTGAACGACTACGGATAAGTCTATCAGCAATAACT
TGAATTTGTCGAGCTTGACCAGCAGTTTTAGACTTAAGAATACGAAGCATACAAGCATCAATTTTATACT
GACGCATTGTTTGCATTGCAACAGTAAAAACTGAATTGATATAATTAATTGGGCTTGGACCAAGACCTTT
TAATTTAGCAATTGAACCTTTAGCAGTTAATGTAAAAGGAACAATATGCATCATTTTATCGCCCATTTTT
AAATCGCGATTAGTATCACCTCCAGATGTATAGGTACATAAACGAAAGCCTGGTTGTTCAATTGCATCAT
CAACATGAACTGAAAAAATTTGTGGTATTTTCTTCTTTGGATTTAAGTTTGTAATCGGAAGAGTAGTATC
TTCGTCAAATAATTCTGTAATGAGTTCCATCATATCCTCTCTAGTGTTTATTCTATTTATAAAATTAAAG
 
GCCCGAAGGCCTTTAATAATCTATAGGTAAGAGAGTACGATATATTTCAAACTTTGGACCTTTTTCATAA
GCATCAAAAGTTTCTGTGAATTTATTATAAGCATATGCATCTATAAATTCAATCATGATTTGTGATACAG
AAGTAGAAACATCTCCACCTTCTTTTTGAGCTACAACAATTGTTTCTAAGTAAGCTTTCATAGACCAGTT
ACCTCATGAAAATCGCCAAATACATCTTCGAACGTATTAGCTTTAGTTTTATCTTCGCGTAAACGAATCG
CAATCGGAAGAAATAATTTAACGTAATCAGTGCGGCCATCAGATTTTAACCAACCGTTGCATTCGCACTC
TAGAATTTTTCCAATATAATAATTTTGGTTTTCCATAATGCGAGTACGGTCAAGTTCATGCGATTTTACA
CCGGCTTTATCTTTTAAGCCTGAACCAGCATTTACCTTAATTTTTCCGCACTCTGACTCAAGAATAAATC
CACCTGCTTTAGTAGGGTCTTTACGGTGAGGATAAATTCCTACAATTTTTAAATCAACATCAATTACTTC
TTTAAATTTATAGAGATTTTTTGAACGAGCATTTTCCCATAATCCATCGATATTTTTGAGAATAATACCT
TCAAGACCTTGGTCAATATACTTTTTATAAATTACCTTAGCTTCATCTAGGTTATTTACTACCTGGTTTT
CAATTAAAATTACTTTATCATATCCCGATGTCATTTGTTCTAGTTTAGAAAAACGTACATCATACTTCAA
ACGAAATGCAGGAAGACCGTATATTTCTACCAACGGGACATAATCCCAGACCTGAAACTTCATGCATTGA
GCTTCTTTTTCAGAAATGGTTCCCTTTAAAGATTTATTGGCGATGCCATTAGATGCAGTACGTGATTCAG
CTACTTCTGTGAAGTCTTTAACTTTACTGTTTTCAGGATGAGCATCAAAAAGAAAATCTAGGCCTTCTGG
CTCCTTTTTAACTTGCTCATGGTATACCAATTCGCCATCAATTAACACGCCTTCTGGATGAATCTGGCGG
GCTTCAGCGGTCATTTTGATTAATTCTTCCTTAAGAAGATCTAATCCTAGATATTCATTACCGGCTCGTG
ATAAAAGACGAACATCATCTAATTCATCACCTCTGACTTCGGCAAAGCACCGAGCCCCATCAGCTTTTAA
CTGGGCAAAAGCTGGAAATTTGATATTCTTATTAATGCCTTTTTCATCATAAGAACTTGCAAGCATTTGA
GGTTGTTCAGGAATTAAACCTGGCCAAACTTTGTTTGCAATAGATACTGAAGCACCACATTCAAGGTCTC
GCATCATCACTCGACGCAAAACTTCAACATCATCTTTTTTACCATCGGTGATATATCCAGTTAATTCCTC
AATTGCTGCATTTCCAGTCAATTTCCGAGTAGCTAATGTGAATTCAATGAAGTCAAGCATATCGATAAGA
GTCAACATTCCAAAACTCTGGGTAGCAATACCAGGTTTAGGCCATTTCTTGATATAATACTGCAACCCAC
GAGAATAAGTCAGACGATATACTCGTTTAAGCAATTCATTATCTTTATTCTTTTCAAGAATTGCTTGCTT
CTGCTTAGTTGAACCAATAGATGCTATTTCGTTCAGAATTTTAAGAATCATTATTCATCCTTCAGAGTTT
GGTTTACAGCTCTATTATAAATCAATTCATCATTAAGCTCAGTCAAAGACCTATGATATGTGGTTCTAAC
TTTATTTCCTTGCATCCAGTGCTTGACGTAAATGAAACCTTGCTCTACACATTTTTTAAAAATTCGTTCG
TCTTTTTGAGCTCGGAATTCTGGATTGCATCTAAAAAATTGATTTACGTGACCGTAATCACGTGTAGTAT
TACCTTCATTTTCATAAATAGTGTGAACAACAAACATTAGAATGCTCCTTGGAAAATATTATCACCACTA
CCACTAGTAAGTCTATTAAAATACCCTGCGTCGCCAGGCTTAAAACTCCATGTATGGAATGGATGCATCT
GGTGTGAGTGTGTATAATATGGATTATAAGGATTAAATCCAGGAGTTCTCCAGGTAGTGTTCCAAGGAAA
AGAATCGTTTTTAATCATCTTCTCGATTGTATCTTTTATGGCTTTTTCACTATCAAAACTTTCTTTATTT
TCCTTCGGCGAAAAAAGCTTATTTTCTACATCATTCCATGTATAAACCCGCTGAGCAGTTTTTGGAATAT
TGTCACGCTCTCCTCGAGCCATCCAATAAACAGGAACACGTAATATTTCACTCGCATGGTCGCAGTGGTG
AGCGAGATCGTCAATATAACAAATTACGTTATATTTCTCTTTTGCCTTTTTGAACAGCTCTTCTTTTGAA
GAATCATGACCACACATCAATACTTCTGAGAAGGCACCAGGAAAAAGAGCATTCAAATTAAATTGACGAT
TCAGCAGAGCGTCAATAGAATCGCCCAATGCTGTAACAGCTACAAAATTATAATCTTCCTTTAATTTGTT
AATTACACACAGAGCATCTTTATATGGAGACAAGTAACGAATAAAATCTGAACGATTGTATTTTTCAATT
AACTTGACACCAAGTTCTTCATCACAGTTAAAGAGTTTACCAGGTGAAATAAATTTCTCATCTTGGATCA
TTTTTAAAATATGTTCTAACGGAAGATTATATTTCTGAGCAAAATAAGGAAGGCCTGATTGCCAGCTTAA
ACATACTCCATCAATATCAGTTAAAATAGTAGGCTTCATAGAGAGTCTCTTAATAGGTTTAACACATCAA
TAAATTCAGCTTCGGTTAGTATTGTATCATCTTTTGTTAGACCACTAGCCATGCTGTGCTTCAAAACTTT
TCCTTTCGAGGCTAGTAATGCATCACGAAAGCCCTTGTTTTGAATCGCTGCTTCAAAATATGCATTTGTA
TATAATTCTTTCCACGCTGGGGAGTATCTTGAAAACGGAACTCCGAGCCAGAAGAGGGTCCCACGGTCCT
GAGCTCTAGCATAAGACCTTCCAGCTTGTTGGGCGGCAAGCCCGGATAACCCAAATATACGACGTTGTTG
 
TTCAACATTTTTCACCTTGCATCCTTGGAGGAATCCTTCAAGACCTCCGAATTGAATACCATCCATGACG
AAAGGCCATTGGGCGAAATTACTTAATGCGCATGATGGCCATCTAAAATTGCTTCTAATCTCTAACTCAG
ACATCTTTAATACTTTATAATTTCAACATCCGCCCAATGACCATAACTTGGCAGACGGTCTTCAATAGAT
AATCCACCATCATATTTAAGCATTTTAATTGTCATAATATGGCTCTAAATGGTCTTCTGTGAGCGTAGAA
ACGTCAGTCACACGATAAGTAACTTCAACCATTTCCGTACCTAATAATTTCTGAATTAATTTCTTAAACA
TATTATCAGAACCTCATCTGAAAACCGTGTGATTTAACATTACCGCCGCCAATATCAGAAATATTAATTT
CACGCGCAATTGAAGGGTCAATGTCAATTTCGCGATTGAGTTTAGTGATAGCTAAAGTATCACGCCCATT
TACTGTACGGAATTCTAAAGGGCAGACTACATTAACGTAATTTTTAATCGATTCACTGGTAGGTTGCAAA
TGAGCAGGAAGGTCTTTAGGCGCCTTAGAGAAAACCACTTCACAAAAATTTTTGCGGGTATCAAAATAAG
TAGTCATAAACATAACATTTTCCTCAATAGGGGCCAAAGCCCCTCATTTATTTTAAATATCAAATTCGTT
AAGAACTACATCAAAGATTGCTTCAAGATGCTCAGGTTTAGCTCTGTTACTCAGAATATGACGAATCCAA
GTTTTAACCAAAAGCTTGCGATTAGCACCATTCCAGCAAGGATGAGTCCCTAAATCGCGTTGACGAAAAT
CATCATCCAGAGCGATTTTAAAGTTGGAACCTTCCATTGTGATTGAAACCGTGATGCCATTTTCAAATCG
CATATAAACATAGTTAGGAGTCATATACTGTTCAATTTCGCATACTGATCCATTTTGATGTTTCCAAAGG
CAGATAGTATCAATAGAACCTGCAATACCATTAGAAACATATTTACGTTCAAAGTTGATGTAGTTCATTT
TTATTCTCCTTTGTTTGTGTAAGATAATACTATCACAAAGGAACCATACTGTAAACAACTTTATGCAATC
TTTGGAAAATAAAAAAGGACTCCCGAAGGAGTCCTCAACTTATGCTTTCTGCTTACCAAAACGAGAAGCA
TCATCTCGAAGAACCGCACGTGCTCGGCGCATGATCTTCTCAACAGTTTGATTGATACGAGAGTTCGACC
CACGCTTGTAGCCAGCGCGTTTAGAATCACCAACTTTCTTTTCAACTGCTTTCTTTGCTTTAGCTTGTTT
TGCCATTATAAATTCTCTTTTAAACGAAAATGCAGGACTTATTGGCATTGCCTGCGAAAGCCCTCAAAGG
GGTACTTAGGTTTTGAATATTTACCGACAGGATAACCATAAACCCGTCATCATACCACCAAGACTTACAC
AGATAACACTTTTCTGACGCATGATAGTAACATTCAAGAGGTACACCGTAAAACTGCCGGGGTCTTAAAA
CTATAATGATTCGCAAATCATTAATCAGACAGTTCGACGGCTCCTCGATTTTAGCTCACACTAAGGCAGT
GAATCTCCAATAAATTACTTCAATGTTACCACAAAGTGACGAACTGCTTTTCGCGCGGCAGAAGCCAGAG
GCTTAGCATATTTAAGTTCATCTTTTTCCTGAAGCTCGGCAGCTAATGCAGTTTGAGCAGGATTCAGATG
TTTGAAATAACGCAGGATTTCAAGAGCTTCGGCTTCAACATCAATAGATGCGCCATAGTTTTCGTGACCA
TTATTCCATGCGTTTCGTTGCAGTTCAAGAGCGTGTTGTAATTGTTTAATCATTTAAAAATTCTCGTTAG
AGATTAAAACTCGGTAGTCACGTTCTTCTGAATTTCATCTTCTTTCGACAGATCTCTCAGTTGTAGACTA
CCACATAGAATTGTTCGGTTAACTTATTATTCCGACACCCAATTCATATTATTTATATCACTTATAAAGA
CACGGAATAGCTTTATAATGACAGGTAACGAATTTTTGTTTAATTTCTTTTGGCTGCTTAAGACCCAGAG
CTACAAAAGGATGCGGAACGTTTCGAATTTGACCAACTGGAAGAGAAGTCAAATCACCAACTTCACAAAA
ACCTTCAGGAACATCAGGACCGACAGAGTGAATTACACACAGTTCAGGAACTTCACCTTGAACACGTTTA
CCGATAATAAGTCCTGATTCTGTAACTTCTTCATCGCCGGCTTGTGCAGGTTCAGAAACTAAAATAACAT
ATTCACCGACAGCACGAATTGGTAGCTGTTGTACTTCAGACATCGTTTTTCCTTTTTGCTAACAGATGAA
TTAATAATAACAAATAATTCTTAAAGCATTTATTTACCAATAAATTGAAGCAAATGCTCAACTTTCATAC
CATTAACAGAAATCAATTTGTCAATAGAAAAACCTCGCCAAGCGCCAAGTTCAACATCAAATACTGGAAT
CATATCCGTAGATTCTTTCCGAGTAGATTCAGTCAATTTACCGGTTTGCATGGTTGGCATAAAGTCTGCA
TCACGAGTACCTTTCATAGTACGAATAGTACCATCAGACTTTTCAAAAACTACGTTTGAAACACCCATGG
ACAATTTAGTTTTCAAAATTTCACGAATTGCTACTTTCTGCTCAGTTGTCAGTTTCATTTATTTACCTAT
TACAGTTTTAATATGAGTTGTTCCACGTTCTTTAAGGGTGGAAAGTAATTTTTGGAATTTTTCTAAATCA
GATTTCCAACTATATGGTTTATCAATACAAACCCAATTTGTCTTATAATACTGTTTCCATTTAGAGAAAA
AATATTTCTTATACTCTACAGCAAACGAGATGTTCTCATTAGAATAAGAACTAATTGCTGTGAGCTTTAC
CAAACGAAATTTCATTATTCACCGCAGAATTCGTTGATATTTTCCCAGTTTAACTTATTCAAGTTTTTCT
TAGGAACATTAAACACTTCAATACCTGCATTTCGCAGAATATCATCCCAGCCAGGTTTATTTTTATCATA
 
TGTTTCGCAATAAACCAGCTTTTTAATACCAGATTGAGCTATCGCTTTTGCACAATCTGGACAAGGAGAA
AGTGTTACATACATAGTAGCACCTTCAATAGAAGAACCATTTCGTGCAGCAAACAAAATTGCATTTAGCT
CAGCATGAATTTCATTTTTAGACGACCATTCAGAGTGAGCACTACGATGTTCTTTCGCCAAGACAAAACG
ATCAGTTGAACCAAATGATACGCATTCAGGCTTATGGCCTTGAATGATAGTATGTTTAGGCTTATTCAGC
AACCAACCTTGCTCAGCAGCATAATCACAACAGTTCACACCCCCTGCGGGTGAACCATTATACCCAGTAG
AAATAATACGTCCATTCTTTTCAATTACTGCTCCTACCTTCCAGGAGCAACATTTTGATTCCTGCGATAC
TAAATATGCAATTTGAAGTACTGTACTCGCTTTCATTTCATAATCACCAGATAAGCAGATTTAGCAGTTT
CAACACGATAAATTTCGTGACGAAGTTTAGTTATACTTTTAATAACAGAACTAATTATATTCTGCCCATC
TTTAAAGCGGTTTTTCTTATCAATAAAAACTGCGCCAGTCATCTTTTTATGAAGCTCAACTGGATACTTC
GTCACAATAATAGCATCATACACAGAAGGATGAATACTATTCACCAGAGTATCATTCATTAAAGTTATTC
TAATGAACTTTGCTGTTTCAGAGTCAAGCGCTCTATGATCGCCAGTATCATTTTCAAGACAATTATCAAT
TATATCAGTTAAATTCATCATAGTACGCCATACACCCTTTGTGCTTCAACTAATCCATCAAAATCCAGTT
TAAGATGCGATATTTGATCGCCATCACCTGGATTCACAATTACTAATACTGAACGAGGAGTTTCGGTAAT
AACACGAACCGATGTTTCAGGAAATCGTTCAGAAACCTTATTTACCAATTCCTGCGCAAATAGCTTAACT
TTTTCTTGGAATTCTTTAACAGTAATCGGTTTTTCACTTAGCAGCATTTTGTTTTCCTCATTTGTTTGGT
AGAGCTAATATCACAACTCTACCGTAAAGTAAACTATTAAATTGCTTTGAATTCTGCTGTCTGCGATTCA
AAACGAATATCGCCTTTGATGACTAGCTCAGCATCAAGACCGAATACAACAATGATATGCGCGGAACCTG
GATACAGCGTAATGGCAACAGAATCCACCTGGTCTGGAAGCAAAGTGTTCAATACATGAGTCACTTGAGC
ATGGATTCGAAGCTCGGCTGCGTTATCAAGTTTTTCAAACATATTATTAGCGATAATTTGGCTAAACACT
ACTTCTACGATTTTAGAATAAGTAGGAAACATATTTACCTCACATAATTTTCTTCAAGCCAATTAATAAT
ATCCCAAGTTTCATGGTGCAAAGCCATTAAAAATCTCCCGAGGCGACTTGCCAGCATTCAACACCGATGC
GGCGCCACATTTCAACTACTTGAGTTCGGTCATCAATAGCTAATTTCACATCAAAATGTGGTGCAATGTG
TTTCCAGAAAATTTCTTCTTTAACCACATCGTCTTTACGGGTATCGCCTTGTTCGCGCTGACATTGCATA
ACTAATGGAACGCCAGCAATGTCCTCAACCCATTTACGGGTCATACGATAATATTTCGTTGGGTCTTCTT
TAGTTCCACTTTCACGGCCTGAAACGACTACAATGTAATATCCTTGCTTATCGTACATCTTAGCTAGTTC
AACGACCATTGGATTAATAATATCGGTATCGCATTTTTCAAGGTCATAAGGACCACGACCATTCATTTTT
GCTAATGTGCCATCAACATCAAAAATAACTGCTTTTGGTTTACCAGGAGTCCCATTATATACTGGAAGAC
CGAGATATTCCCGCATGCTTTTATACATTGAGCGTAAAACATCAATTGGTACTGCTTTAGTTCCGCGTTT
TGAGTTACGTTTAACCAATTCAGTCCAAGGAACATCAAACACTTTATATTCAATTTGATGCCCAAGCTCT
TTGGCAAACTCTTCCCAGACCTTACGTCGTTCAGGATTTAGATTAGTATCTGAAACAATTACACCCTTCG
TTACGTCTTGGCATAGAATCATGTTAGCAACATCATGCTGCATGTAAGTTACGATGCCTTCTTTCTTTTT
GGTATACTTGTACTCATCGCGTTCTTCATGCGCCATAATAGATTGGCGATAGTCATCACGATTGATATTA
TAAAACCCGGGATTCTTAGCAATAAATTCACGAGCCCAAGTACTCTTACCAGAACCAGGACAGCCAATAG
TCAAAATAATCTTTTTCATCATTTAATTCCTAAGAAAACTTCAAGAATACGAATATTGTGCTCACGCCGT
CCTTTGTACAATTCAGCTGTTGATTGATTCACTGATTTACTCTGAACATTGGATGAAATAAATTTTATCA
TGTGATTTTTAAGCTGATACATATCAAGCCCACGAGCTTTCGCTTCTTTTCGCAGGGCTTTACCAGCATC
ATCTAAAGCTTTAGCAGGGTCTTCATCATTTAATACTATTCCACGATCCAAATCCATGTATACTGCACCA
TGACATGCATCAATGTAACGGTCTGAGCATTCAATATAGCATTAGCAGAGTTTTCATTTATTTTTCTCAA
CCAATGATTGAATATAATCATGCAGGTCTTTAGATGCTTTGCCCCACTTATTTTGATATTCATTTTTGAG
ATTAGCACGTGATTGAGCTAACAAAACATCATTAGTCGGAGGTAAAGATTCTAACCGCTGAATCTGGCGT
CCATAAATCATTGCAGCCATCTCGGATTCATAAATCAATTCTTTGAGATGTTCAAATTGATGCCATGAAA
TCATTTACATTTATCCTCTTTTAACTCTTGACGATAATAACATATCATAGTTTTCTGGTCATGTACATAT
CGTTTTACATCATTAAGCCAAATACGAAATTCCTGGGAATCTTCAAATGGCATACCGACCCAAGCTTTAC
CATCGATAACTTTAACTTGCCAAGATAGTTTAGCTTCATCATATGACTTTATTTGTACAGGCCAATTAGG
 
ATGAACTGTTTCTTTCTTTACTTCTAGAGGCTTTGTCGAACAACCAACTAGAAGACCAATAGATAATATT
ACTGCTGATAGTTTAATCATTTAGAAAGGTCCTGGATGTCTTCTGCGAACTTGTTGAAGGAGTTGTTGAT
TTGTTTTTCAACCAATCCTGGCTTATGAGCCACCACATCCGCCTTCTTTGCGTCTTTGCGCAGTTTTTCA
TTTTCACGCTCAATAGCAGAAATTGCCTCACGATTTTTAGTATTCATCGCATCAATATAATTATACTGAA
TTCGCAAATTATTTAATGCTAAAGCGTTTTCATTGGCCGTTTTTGTAATTTCTACAACAGACGTTTCTAA
TCTTTCAATTTTATTTTTTAAAATTAAAGAAGTTCCGCCTAATGCAATTACAATTAATAGCAAACCTGCT
GTCGTATTACTTAATTGCATAAAGTTTTAATAACCTCTATAATATCGTCTTGAGAAAGACCGTTAATTAA
AATATGATGTTCAGCTGGAGATTTAGAAATTTTAAAGCATGCCTCAACATCTTCTGCCATATCCGATGCA
CTACGATTTGGATTACTAATCCCAAGGCGGTGTTTTCCCGTCAAAGGATTAACGATGATGTAGCATTTAC
AACTATTGATATGAACATTAGGCTGAGTCTGATTAATGAATACTTCGCACTCATACTTAGCGAGTTGATT
TTCTAAAAAGACTTTCATCTCTTCAACCGCATCAGGAAGCATATCACGGGCTTGCTCAAGACGACGATTT
CGATATTCTTTAATGGTCGTTTTCCGCTTGACTTGCTTAGCTAAATCTTTCTTAAGATCAGTGATATATC
CAACTCGACGATTTCCTTTGAATACAGAAATCCCATCTGTAGTATCACCGTATGCTTCAACGACCATTTC
AGTAGTAATAAGCTGCAAATCCATCATAAAGTCCTCATGTTATGTCAGTAAGATTACTATAACACAACAC
GAAGGACTTGTAAACAGCTTAGTATCCTTCCGGGATAAATTTTTTATAATTTTTCAAAAAATTCTGTTCG
ATTTCACACATGACCTTTTCTTGACTATCATACCCCTGATATAGGCTCATGATGATACCGAACAGGTGAT
CCATTCCAGCACCTTTAGCAACACCTTGCGCTTCCATTGCATAAGTCTTTCTATCCTTACCGCAGTTTTT
ATTATGACAGTCAAGAACTAAGAATAGAGCTCGATCTAAGTAATTCAGATAAGTCGTCTCAAATGCTTCA
ATTTTTCTGTATGAATATTCATCGCCGGCATACATTGCTTTAAGATCGTCTGATGCACCGTCAATAATAG
TCTTAAACAATTTTTCTGGATTATCTAATGAACTTTTTGTGCTATGAAGAGACACGTACCAGTCAGACTT
AATTTTAAAATGAGAACCATCTTTCATTACTGCAACATAGCCTTCGATGTTTTCTGCATTTTTAGCTTCT
TCTACACATTTAGGGCTATCGATTTCGAATCGTTCAACTAGATATGGACGAAGAGTAGCATCTTTATAAA
TATCATCGTATGAAATGTATTCACCTGTTTCATTTTCACGAATATTCAATAAAATGATTTTCATCTCTTG
ATAAGCAAGAACGATTCTATTCGTCGGAGCGACGAATTCGAAGTTAGCAGTAAATCCATCTTCAGCTAAT
TCTTTAAGTCTATCACGCAACCGATGGTGATTAATATTCATCAAAATACCATTAGCCATTAAAGCCTGCT
CAGATTTGATTGAACCCTTTGATTTGAACAGAATTTCATCACCGTCTAAATAAGTTGATACCAAAGACCC
ATCTTCTTTTGTTAGAATGTAATCAACATCATTTAAATCGATATTCATCGTGAATGGATTTTCATTCAAG
TTAAAAAACTTTTCCATGGGACGAGAGGCAATTTTTACTGGTTTTTCTCCATCCATTTCAAACATAATTC
CACGACATTCCAATGCGTCTGGAAGTAACCAATCAGAATAAGATGCATAATTATATGAGAAAATTCTGTA
AGTTCTTCCAGATGCACTTACATCATCCGAGTAAAAAAACTTACGCTGTGAATCCTTACATAGTTCCATT
AAATTGTTAAAAAGTTCTTGCATTGTGTATCCTCTTTTGTGTTTTGAATATAGTACCACACTCCATGTGG
AAGCATCATTTTTTCTTATGTTGAATATTCCAAGGCGGGTTAAACAGTTTAATGAATAGTGGCTCCTCTA
AGTCAATCGTTGCGATTGTCATTGTACCTAACTCATTTGTCATAGAAAGATTAAAACATTGGCGGGCGTA
AAATTCAACTTTGCTTCCTTCCTTTAGAGCAGCATGAATTAATGCAGATTTAGTAGAATCAGACGTTTTG
TCTTTACGGTTAATAGCAGTTCTATAATAATTTATTCTTTTACGTAAATTTTTAGTTTTTCCAATATAAA
CAAGCTCATCATTTATGGCAATAGCATAAATTACGTTATACTTGTTTGGAATAGATAATTGTTTTATACT
TCCGTTGTCGTCTAATTCTAGCTCAGTATATTTAATAAATGAATATTCTGTTGCAATTTCTTTCATAATA
AAATGGGCCTTGCGGCCCACTCCTTAAAAATATTTTTTAAAACTCATCATAACTTTATCATCAACATCAT
TATCAATCTGTGCAACAAGGTAAGATGACAGTTCTACTTCTTGTGGCGCGGATTGAACATTATCAGAATT
AAGATATTCACGAATCCAAGGATATGGATGTTTAACCGGAGCATCGGTAATTGGGCATGGAAGACCGCAC
TGTTTCATACGAGATACAGTTAAGTAATCAATAAAGCTCCACATGCTATTTGTATTTAGTCCGGGAACGT
CTCCATCTTTAAATAAATGAACCGCCCAATCTTTTTCTTGGCGGTTAACTTCCATGAAAATATCAACTGC
TTCTTGTTCACACTCTTTGGCAATTTTAACCCATTCATCACCATCAGTGCCAAGTTGAAGTTGACGAATA
ATATATTGTGTACCTTTAAGATGAAGCTGCTCATCACGTGCAATGAACTTCATAATCTTGGCATTACCTT
 
CCATGATTTCCATGTTCTTATGGAAGTTAAAGGTACATGCAAAAGATACATAAAAACGAATAGCTTCCAA
TGCGTTGATTACGTGCAGGCAGAGGTAAAGAGAGCGCATTAGTTCACGTTTCCAATACATTTCATGCTCT
ATGGCATCTTTAATATCATCTTCGTCTGCGTTAATTTCTTTTTTGTACTCAATATCAGCTTTAGCATTCT
GCCAATATCTAGTTTTCTCAATAACATCATCATAATAACGACCAATAGATTCAGCACGTTTCATGATAGC
TTTATCTAATACAATCTCATCAAATACCTTTGATGGATCAGTATAAAGATTTCGCATAATATGAGTGTAT
GAACGACTGTGAATAGTTTCACTAAAAGTCCATGTAGCAACCCATGTATCAAGACTTGGGTCTGAAATTA
ACGACATAAGTACAGCAGACGGTGCACGACCCTGAATGCTATCTAGAAGTGATTGATACTTCAGGTTATT
AGTAAAAATATCTTGCTGATATTGAGGAAGCTTGTTAAATTGCGCAGCATCCATCATTAAGTTTACTTCT
TCAGGACGCCAGAAAAATGACAACTGCTTTTCGGTTAAATCTTCAAAAACTTTATGACGTTGAATATCAT
AACGCGCAATACCAAGACCTGAACCAAAAAACATAGGTTCTTTTAAAACATCAACTGGATTTGTATTAAA
AACTGTGCTCATAAATTTTCCACTTAGTTAATAGATAGTGACTCGTCCATGAGTCAAATTATATCATAAT
TTACAGGATGAACAATCTTCAGCTTTTGGAGTTTCTATTTCATAATCATCAGTACCAGAACCATCGCGGG
TATTATGATAATAGAAATTTTTAATGCCATAATACCATCCGTATAACATGTCATCAATCATTATTGACAT
TGGAACCTTTCCTTTTGGAAAAATCTGCGGGTCATAATATGTATTCGCTGAAGCTGATTGACATACCCAT
TTCAGCATAATAGCTACCTGCGTAAGATAAGGTTTATTACCTTTCTTAGCTAATTTCCATGTATAATCAT
ATAGGTCTATGTTATGTTCAATATTGGGCACGACTTGATTAAAGGAACCCTCTTTTGATTCTTTAACAGA
GACTGGTCCACGTGGAGGCTCGATACCGTTTGTACTGTTAGAAACTTGGGAAGATGACTCACATGGCATA
AGTGCTGATAATGTGCTATTACGGATGCCAAAGAGCTTAAGGTCTTCCCGCAGCGACGACCAGTCACAAA
CGTATTTTGGAGCTGCGATTTGGTCAATCTTTTTATTGTACCAGTCGATAGGTAATTCGCCTCGAGACCA
ACGAGTGTCTGAATAATATTCACAAGGTCCTTTTTCTTTGGCGAGCTTAATGGATGCTTTAATGAGTCCA
TACTGTAATCTCTCAAATAGTTCATGTGTTAAATCGTTAGCATCTTCATAAGAAGCAAAGTTACTTGCCA
GCCAAGCTGCATAGTTAGTAACACCTACACCAAGGTTACGACGCTTTTTAGCTTTTTCTGCTTCAGGAAC
TGGATATCCTTGGTAATCCAACAGATTATCAAGAGCACGAACTTGAACTTCTGCCAATTCATTAATTTTA
TCTTGGTCTTGCCAGTCAAAATTATCTAGTACGAATGCAGAGAGAGTACACAATCCAATTTCAGCATCAG
GACTATTCACATCATTTGTTGGAATAGCAATTTCACAGCACAAGTTACTCTGACGAATAGGTGCCTTTTC
ACGAATAAACGGAGTATAGTTATTCGTATTATCAATGAACTGCACATAAATCCTTGCTGTTCCTGAACGT
TCAGTCATGAGCAATTCAAATAGTTCACGGGCTTTAATACGCTTTTTACGAATATTAGGGTCTTTTTCTG
CTGCTTCGTATAATTCACGGAAACGGTCTTGGTCTTTAAAATAAGAATAATACAGCTCTCCACCCATTTC
ATGCGGACTGAACAAAGTAATGTAATCGTTCTTTCCGAATCGTTCCATCATCAAATCATTCAGTTGAACA
CCATAATCCATATGACGGATGCGGTTTTCTTCTACGCCTTTGTTATTTTTCAAAACGAGAAGATTTTCAA
CTTCCAAATGCCAAATAGGATAATAAGCAGTAGCAGCGCCGCCACGAATTCCACCCTGTGAACATGATTT
AACAGCAGTCTGAAAATGTTTCCAAAAAGGAATAACACCAGTATGGCGTACTTCACCCATGCCAATCTTA
GAACCTTCGGCACGAATCATACCAACGTTAATTCCAATTCCAGCGCGTTTAGAAATATATTCAACAATTG
AAGCAGAAGCTTTATTAATTGACTTTAATGAATCACCAGCTTCAATAACAACGCATGAACTAAACTGCCG
AGTTGGAGTACGGCAACCAGCCATAATAGGAGTTGGCAGTGAAATCTGTCGAGTAGATACTGCTTCATAA
AAACGAATAACATGTTTTAATCTGTCAACAGGTTCATCTTGATGCAATGCCATTCCAATAGTCATAAATG
CAAACTGTGGAGTTTCATAAATTTGACCAGTGGTTTTATCTTTAACGAGATATTTTTCTTTTAATTGCAT
CGCCCCGGAATAAGTAAATTCCATATCCCGCTCGTGCTTAATTTTTGATTCTAAAAATGTAATTTCTTCT
GCAGAATATTTTGACAACAATTCAGGGTCGTATTTACCTTCATTTACACAGTAAGAAATATGGTCAATAA
ATGAACGCGGTTCATACTGCCCATAAACATGTTTACGAAGAGCAAACATTAAACAGCGTGCAGCTACATA
TTGATAATCAGGTTCTTCAACCGAAATAGAATTCGCAGCAGCCTTAATGACAATAGTCTGAATGTCATCA
GTGGTCATTCCATCACGGAGATATGATTTAATATTTTCATATAATTCATAAGGATCTACAGATGTTCCTT
CAGCTGCCCAAGATAAAACTTTAATAATTTTTTGCGGGTCAAAGCTCTGAGAAACACCACTACTTTTGAT
AACATTAATTAATTGCATAAGTCCTCAACTTGAAAATCGTCTTTAAACAATCGGTTAACTATATGAGCTA
 
TTATATCACCATGACACGGCTTTGGTTTACATGTGCATCCTAGCCTCATTCCACGTAAAGGCTCTAAATG
TTCTTTAGTTATTTCTCCGGATTTAATTCGACGTATAAAATCTTTTTTGAATAATTCAATGGCAGCCTCC
CGGCTGCCAGCATCTTTACCGACGTAATTTCCCCAAAATGTACCACGGTGAATATTAACATCAAAGTCGG
ATTTATATTTATTCACTACCTGGCATAGACGGCCCGCGCGGCGATAATTCGTCATATTGTTTTTCCGTTA
AAACAGTAATATCATAGTAACAATCTGAAGAAGTTTTAACTGTAGAAATTTTATTATCAAAATACTCACG
AGTCATTTTATGAGTATAATATTTTTTGCCATAAATGATAATAGGCTGATTTGGTCCTGGAACTTCTAAC
TCGCTTGGATTAGGAAGTGTAAAAAGAACGACACCAGAAGTATCTTTAAATCGTAAAATCATATATCCTC
GCAATTAAATTAAAATTATACCGCCATCTTTCCTTTAATTGGCGGGTGAGATACATAGTTGTTAAGAACG
AAATCTTTAGGCCTAAGTTTAAGAACATATTTTAATTGTTCTTTAGTAGAAAGATATCGGAATTTATAAG
GTAGACCACTTATTACCAGCTCACAAAGCTCTTTAGGTTCACGACGCAAAATTTCTTTACATTGTTCTAC
GTGATTCATATAGATATGAGTATTACCGCCAGAAAATATCAAATCTCCAGGAATAAGATTACACATCTTA
GCTACAATATGAACTAACGCAGCATATGATGCAATATTAAATGGAAGACCAAGAAAAACATCTACTGATC
GTTGATACCACTGCAAATCCAAATAGCCATTACGCACATTAAACTGATAGAACATATGACAAGGCGGTAA
TGCCATATATTTAAGTTCAGCTGGATTCCACGCAGAAACAATTTGTCGCCTATCATTCGGCAGTTTTTTA
ATACGATCAATAACTTCTACAATTTGGTCTACACCGCCAAAATCACGCCACTGTTTTCCATAAATTGGAC
CAAGTTCACCGCTATGGTATCCTAAATCTTTTGCTTGATTTTCGTAATTTTCATCCCAGACTGTTTTGCC
TTGAATTAATGAATCATGCTGAATTAATCGTAAATCATTGACATTTGTGCTTCCTGATAAAAACCAAAGT
AGCTCAGCAATGCAAGCTTTCCAGGCGAGCTTTTTAGTTGTTACTGCAGGAAAACCTTTACTTAAATCCC
AGCGTAATTTAGTACCGAACAAAGCAATTGTTCCTGTGCCTGTTCGATCATCGGTTTCATAGCCATTTTC
AAAAATGTCTTTAATTAAATCTTGGTATTGTTTCATTTAGTACCTTATATAAATTATAATACATTTTATG
AAGCTAAATAATCAATACGGTTTAGCATCAAATTTTCTTTCGATTTCAGGAGTGAAATATAATTCTTTAC
CGCGTTTAGAAAAGCGACCAATAATATGATAATTATTCTCATCAAACGTAATATTGCCAATATCCATGGC
TTTCAACCAAATATCGATATTTCTATCCAGTTTTATATCAAAATTCCAATACGGAGAACTATTACCGAAA
ATACTGCAAGCGGATTCAGTGAAACAACTGCTATAAATCTTGTCTTTAGGAACTACTAATTGATAGACGC
CTTTAATAAACTGAGGACCCTGCCATTTTGGTTGTTTCGCGTCTTTTTCTAATAGAATAGGGTAATAATT
AAAGTTCATATTTTCCGCCACGCGTTATTTCATTTATACACTGATTCCGTAAGGGTTGTTACTTCATCTA
TTTTATACCAATGGGTTTCAACCATTTCACGCTTGCTTATATCATCAAGAAAACTTGCATCTAATTGAAC
CGTTGAATTAACACGATGCCTTTTAACGATGCGAGAAACAACTACTTCATCCGCACAAGGTAATGCAGCA
TATAACAAAGCGGGTCCGCCAATTACGCTTACATTAGAATTCTGATCAAGCATAGCCTCGAATGGTGCAT
TAGGACTTGACACTTGAATTGAACCGCCAGAAATGTAAGTTATGTACTGCTCCCATGTAATATAGAAATG
TGCTAAATCACCGTCTTTAGTTTCAGGATAATCACGCTCGAGGTCACATACTACAATATGGCTACGACCA
GGAAGTAATGTAGACAATGACTGGAACGTTTTAGCACCCATAATCATAATTGTACCTTCAGTACGAGCTT
TAAAATTCTGGAGGTCCTTTTTAACTCGTCCCCATGGTAAACCATCACATAAACCGAATGCTAATTCATT
AAAGCCTTCGACCGTTTTAGTTGGAGAATAAGCGAATACCAATTTAATCATTACGCAAAACCCCTTTCAA
TAAACCATTCAGTGGCTTTATTAGCGTCAAAGAATAATTCTTCATACGTCTTTTCTTTGTTTTCAAAGAC
TGTCACACAAACACGTTGACATTCTCCGCAGTATTCTTCTGACATACTCAAAGCGTCAGAAAACATTTCA
TTAAATTCATTTAAATCAGGATTATGCAATGCGTTAAAAATTGCATAATCGAATTCATCATTCATAAATT
CAAATACAAAAATCATATCATCTCCTTCAAACAGACTTTTTCACAATTTTCCAATCAGTTTTAAACTGCT
CGACGTCAGAATGGTAAATCCAGAATCCTGCGCTTTCTCCGTCTTCATAAAGAGGACATCCATCGCATTC
ATCTTCCCATCCCATATCACGTAAAAGATGTTCAGCTTTTTCAACAAGTTCAGAATCTTTACCGATGATA
TTAAAATACCATTTACCTCTAACTTCTGAATCTTTGATGCTTTGGCGTTGTAATCTCATTTTATTCTCCT
TAGTTGATAGGGTAATAGTATCACACTACTACCCTTCTGTAAACTACTTTTTGAAAGTTTTTCGCAAAAG
TTCAATGATTTCATCTACATTGTTTTCGTCTACAATGCAGTGAATTTTTGTTACGCCAGAAACCTTGTCT
TCGATTTCATCTTCTTCAGAAGTCTGTTCTTTATACTCACGAAAACAATGAAATTCATCTTCACAAATGT
 
TAAAGTAAAAATGCTTTCCATTTGCGCATTCAATGTGTTTTATTGCTCTAAATCCATCAACAAAGAAAGT
TTCTTTAACTTCAAACCACCCGCCATTTTCTTCAATAATTTTAACTATTGATGAATTAGAACGTGGGCGA
TAATTAATAAATGTATCAATAAGTCTTGGAACAAGTTCATACTTTTTGCCAATATACATTACGTTTTCCT
CATTTTAACGGGGCTGTAATAGCCCCTTGATAATTATTGTTCAATCAGTCCCATGTAAAATTCTGCGTCT
TCAGAATCCATACCATCGCAATATTCATTAGCCATAAAGCGGGTGAGGTCTTCAAGAGGACCTTCAATAA
CGATTTGAATACTCCAAAACTTAGAATCTTGCACGCTTGTGATACTAAGTTCAGGATAACGATTACGAAT
AATTTCTTCAATATATTCAAAATCAACGATGTCAATATCAACTTTAGCCATATTATTTTCCTCTTTAATC
ATCAGCAGTATTGCCGATAGTTGTATAGTACCATGGAAGGACAAGGATGTAAACCGTTTTGTGAAAAATT
TTTGAAATAAAAAAGGGGACCCTAGAGGTCCCCAATTAATTAGTAATATAATCTATTAAAGGTCATTCAA
AAGGTCATCCAGGTCCGTGTCATCAGCACTAGATGAACTGCCAGAGCTTGAGCTCATAAAATCATCTTCA
GTTTTTGTATTGAAGTCATCAACATTGAATGCATCCAAATCATCAGCAACTTTATCAGCTTTCTTAGCAG
CAGTCGCTGCGGCACCACCCATAGCAGCAGTTCCCATAACTTGACTAAACTTAGTGCTCAGTTCTTCGAA
CGATTTGAATTTATCTTTAGAAGTCATTTCAGAAAGGTCAACCATTTGTTCGAACAGTTCTTTCTGGAAA
GATTCATCGTCAATGTTTGGAATCGCAGATTGATTCAGGAATTTAGATTCGTCGTAGTTACTAAATCCGG
AAACTTGTTTAACTTTCAGTACAAAGTTAGCACCTTCCCACGGACAAGTTACATCAACCGGAGTTTCACC
CATTTCAACATCAACTGCAATCATTGCATTGATTTTATCCCAGATTTTCTTACCGAAACGGTATTTAAAT
ACTTTACCTTCGTTTTCTGGAGCAGCTGGATCTTTTACTACAAGAATGTTAGCCCAGTAAGAAGTTTTAC
GTTTAACAAGACCGTACTCTTTATTGTCAGTGTTGTACAAATCATTTTTACTGATGTACTGACATACTGG
ACAAGAATCGTAATCACCGTGGGTAGATGAGCAATTTTCGATATACCATTTACCGTTTTTCTTGAAACCG
TGATTTACAAGAATTGCAAATGGTGCTTGTTCATCATTTTTAGACGGAAGAAAACGAATTACTGCTTGAC
CGTTACCCGCATTATCGAGTTTCAGTTTCCACTCGCCTTTATCTTCAGAAGAAAAACCACCTTTATTTCC
AGCCAGTTTAGCCATTTGTGCAGCGAGTTCAGCAGTAGATTTACGTTTAAACATTTTTATTTCCTTTTTA
ATTTAATTAACAGTTGGTGCTATGACGATGTATGACCTCATAGCTGGTCAGTGAGATAATTATAATCTAT
TTATAATAAGCAATTAATACTTGCAAGATTTCACAGTTTCAATGAAAACATTTTTAGCTTTCTGTGAATC
AATATTTAAAATTTTTCTATAAGCCTTTAACTTTATAGAATAATTATTCCAGACTAAATTATCAGTCTGT
TCATCGTGTTTATCAATTATATTTAAAAACGAATCAAGCAAGATAAACGTTTCAAACGAAATTATATTCG
ACTGAAGCAGTTTAAAAATATAACTTGATTGAACCTTTGGATTATATTCAAAGATTTCTTTAAAAGCAGA
AACTTCAACTTTTTTACTAAAATAATAAATGTTGCGAATATCTTCTTCAAACTTAAATTTAATTTGCTTT
AAGCGTCCGATATATTCACGATAAAACACAAGTGCATCAGCGTCAGAGATGTCACCAATCCAAGCATCTT
GGTTAGCAACCAAATTACTTATAAAAATTAAAGCAAGTTCCTTTAATTTATATTTTTCTGATAACTTCTG
GAAAAAATACTTATCCCTTCGCTTTTGATAAGCGGCATCAGACACCCGCATGCACCAATTATACTTAATA
ACATCATACTTTCCATTCATATGCTGTTTTATCATTAAGTATAATTTATAAACTGATTTACCATCAATGT
ATCTTTCACCACCAGCAGGCATGCGGAATTTAATCATAGTAGAAAATCTAATGTATTAGTTTTTTCACAG
CGAACAACAGAAGGACGTAAAAGATTTTCGTCAATAGCTTCTGACTGAATTTTTTCAATTATACCCGAAG
GAATAAATTTAGCAAATTGAGTTTCAGGAATAGAATTTTCTTCTAAGAATGCTGTTGTAGCTTCAAGATA
ACTCATTCCAAACTCTTCTACCATTTTTTCAATAATAAATCCATTTTCTTGACGATCAAGAAGCTTTGCT
ATTTCATCCTTTTCTTTCTTGATTGAAAGTTCTTTTTCTGAAAGACCGGTCTCATCGACCGGACGAATAT
CATTTAGAGAAAACTGTGTCATAAAGTTCAACTACCTCTTCAGTTTCAGCTTCAAACACATCACGGTTAT
CTTTATGATACAAAGCTAATAGACGATTAAACATCTTACCATCAACGCCAAGTTCATCTTTAGCACGAAT
TCGAATATCTTTAATCAGTTCATTATAACCGGAAATTTTCAGTTTATGATCAGATGCTTCTTTAATAAAT
TTAGCCAAGTCTTCGCCATGGATAGCTTCATCAAATTCAACCATTTCTTTTTTAGCCATTATTCACCTCA
AAATTCATTAATGCTATTAGTTAATTTAGAAAGACCCGCTTTTACAAAATATGAATAAATTTTGCCACGC
GGTGGTAATTTATATGAATTATAGTAATTCACAATGTTTGAAGCAATATTATCAGGAATATAATCAAAAT
CAATTAGAACTAAATTTTCTTTATAACGATTATATTCAGATTCTGTGAGAAGCACCTTAGCTTGCTCACG
 
GTCATTAGCAATGGCTTCAACGATTGAAGTTTTCATTGAAGGAGTTCGTTCACCTTCAACTCTGGTAAAC
CAGAAGTCAGATCGTACTTTAACTGAAGCAACGTTATCCTTTTTGTCGCCTTTAAGGATTTTAGTCATAC
AGTCAATTTCAGCAGAACCGCTTTTAATTTTAACCCATTTCTTATGCATTGGAGACCATTGCTTAACATT
TGGATATTTGTGAAGCTGTGTAAAGTCACCATCCGACGAAATGATTAAAATCTTATGTCCTTCTAAAGAG
AACTTTTTAACAAGAACAGCAATATGGTCATCCGCTTCATACTTATCAATATCCATAACAATGTATGGCA
TATAAGCTTTCAATTCATCTATAACTTTATGGCTGGATTCAAAATAACCTTCCCAGTCCCAAGTAGATTC
TTCTCGTGCTTTTCCACGGTTTTTCTTATAATAATAAGCAAAATCACGACGCCAATATCCAGATTTCGCG
TTATCAATACACAACACGATTTTAGTGTATCCAAGCGTTTTTGCTTTTTTGACATTAAACTTAATTGAGT
TCAATATCAAATGACGAACCATTGATAAATTAATTTTTTCTTTATCTGGGAAGTTTACTAAAGCAGTTGA
AAGCGCAATTTGACTAAAGTCAATAAAGCAAATTCCCTCTTTGTAATCTTCATCCAGCATCATTTCTAAA
TCCATATGAACCCCGTTCAATTAGTGAGATTTCTATTATATACCATCCAAATCTTAAAGTAAACAAGTAT
AAATACTTATTATTGAAAACACAATAGGAGCCCGGGAGAATGGCCGAGATTAAAAGAAAGTTCAGAGCAG
AAGATGGTCTGGACGCAGGTGGTGATAAAATAATCAACGTAGCTTTAGCTGATCGTGCCGTAGGAACTGA
CGGTGTTAACGTTGATTACTTAATTCAAGAAAATACAGTTCAACAATATGATCCAACTCGTGGATATTTA
AAAGATTTTGTAATCATTTATGATAACCGCTTTTGGGCTGCTATAAATGATATTCCAAAACCAGCAGGAG
CTTTTAATAGCGGACGCTGGAGAGCATTACGTACCGATGCAAACTGGATTACGGTTTCATCCGGTTCATA
TCAATTAAAATCCGGTGAAGCAATTTCGGTTAATACTGCAGCTGGAAATGACATCACGTTTACTTTACCA
TCTTCTCCAATTGATGGTGATACTATCGTTCTCCAAGATATTGGAGGAAAACCCGGAGTTAACCAAGTTT
TAATTGTAGCTCCAGTGCAAAGTATTGTAAACTTTAGAGGTGAACAAGTACGTTCAGTACTAATGACTCA
TCCAAAGTCACAGCTAGTTTTAATTTTTAGTAATCGTCTGTGGCAAATGTATGTTGCTGATTATAGTAGA
GAAGCTGTAATTGTAACACCAGCGAATACTTATCAAGCACAATCAAACGATTTTATCGTGCATAGATTTA
CTTCTGCCGCACCGATAAATATTAAACTTCCGAGATTTGCTAATCACGGAGATATTATTAATTTCGTTGA
TTTAGATAAACTAAATCCACTTTATCATACAATTGTTACTACATACGATGAAACTACTTCAATACAAGAA
GATGGAACTCATTCTATTGAAGACCGTACATCAATCGACGGTTTCTTGATGTTTGATGATAATGAGAAAT
TGTGGAGATTGTTTGACGGGGACAGTAAAGCACGTTTACGTATCATAACGACTAATTCAAACATTCTTCC
AAATGAAGAAGTTATGGTATTTGGTGCGAATAACGGAACAACTCAAACAATTGAGCTTCAGCTTCCAACT
AATATTTCTGTTGGTGATACTGTTAAAATTTCCATGAATTACATGAGAAAAGGACAAACAGTTAAAATCA
AAGCTGCTGATGAAGATAAAATTGCTTCTTCAGTTCAATTACTGCAATTCCCAAAACGCTCAGAATATCC
GCCTGAAGCTGAATGGGTAACTGTCCAAGAATTAGTTTTTAACGGTGAAACTAATTATGTTCCAGTTTTG
GAGCTTGCTTATATTGAAGATTCTGATGGAAAATACTGGGTTGTACAGCAAAACGTTCCAACCGTAGAAA
GAGTAGATTCTTTAAATGATTCTACTAGAGCAAGATTAGGCGTAATTGCTTTAGCTACACAAGCTCAAGC
TAACGTCGATTTAGAAAATTCTCCACAAAAAGAATTAGCAATTACTCCAGAAACGTTAGCTAATCGCACT
GCTACTGAAACTCGCAGAGGTATTGCAAGAATAGCAACTACTGCTCAAGTGAATCAGAACACCACATTCT
CTTTTGCTGACGATATTATCATCACTCCTAAAAAGCTGAATGAAAGAACTGCTACTGAAACTCGCAGAGG
TGTTGCTGAAATTGCTACGCAGCAAGAAACTAATACAGGTACTGATGATACTACAATCATCACTCCTAAA
AAGCTTCAAGCCCGTCAAGGTTCTGAATCATTATCTGGTATTGTAACTTTTGTATCTACTGCAGGTGCTA
CTCCAGCTTCTAGCCGTGAATTAAATGGTACGAATGTTTATAATAAAAACACTAATAATTTAGTTGTTTC
ACCTAAAGCTTTGGATCAGTATAAAGCTACTCCAACGCAGCAAGGTGCAGTAATTTTAGCAGTTGAAAGT
GAAGTAATTGCTGGAAAAAGTCAGGAAGGATGGGCGAATGCTGTTGTAACGCCAGAAACGTTACATAAAA
AGACATCAACTGATGGAAGAATTGGTTTAATTGAAATTGCTACGCAAAGTGAAGTTAATACAGGAACTGA
TTATACTCGTGCAGTCACTCCTAAAACTTTAAATGACCGTAGAGCAACTGAAAGTTTAAGTGGTATAGCT
GAAATTGCTACACAAGTTGAATTCGACGCAGGCGTCGACGATACTCGTATCTCTACACCATTAAAAATTA
AAACCAGATTTAATAGTACTGATCGTACTTCTGTTGTTGCTCTATCTGGATTAATTGAATCAGGAACTCT
CTGGGACCATTATACCCTTAATATTCTTGAAGCAAATGAGACACAACGTGGTACACTTCGTGTAGCTACA
 
CAAGTTGAAGCTGCTGCAGGAAAATTAGATAATGTTTTAATAACTCCTAAAAAGCTTTTAGGTACTAAAT
CTACCGAATCGCAAGAGGGTGTTATTAAAGTTGCAACTCAGTCTGAAGCTGTGGCTGGAACGTCAGCAAA
TACTGCTATATCTCCAAAAAATTTAAAATGGATTGTGCAGAGTGAACCTTCTTGGAGAGCAACTACTACG
GTAAGAGGGTTTGTTAAAACTTCGTCTGGTTCAATTACATTCGTTGGTAATGATACAGTCGGTTCTACCC
AAGATTTAGAACTTTATGAGAAAAATAATTATGCAGTATCACCATATGAATTAAACCGTGTATTAGCAAA
TTATTTGCCGTTAAAAGCAAAAGCTGTAGATAGTAATTTATTGGATGGTCTAGATTCATCCCAGTTCATT
CGTAGGGATATTGCACAGACGGTTAATGGTTCACTAACCTTAACCCAACAAACGAATCTGAGTGCCCCTC
TTGTATCATCTAGTACTGCTACGTTTGGTGGTTCAGTTTCGGCAAATAGTACATTAACTATTTCTAATAC
TGGTACGACTTCTTCTCGATTTACATTTGAGAAAGGTCCTGCTTCTGGTAGTAATGCTGATTCTGCATTG
TATGTTCGTGTATGGGGTAATAAGTACAGCGGCGGTTCTGATGTAACTCGTGCAACGATTATAGAATTCT
CTGATGCTACCGGCTCTCATTTCTATTCTCAAAGAGATACGTCAAATAATGTGTTGTTCAACATTTCAGG
TACGATGCAATCAGTCAACGCTAGCGTTCGTGGTGTTCTGAACGTTACAGGTGTCTCAACGTTTAATAGT
TCAGTTACAGCCAATGGTGAATTCATCAGTAAATCACCAAATGCTTTTAGAGCAATAAATGGAAATTACG
GATTCTTTATTCGTAATGCTGGTAATGACACCTATTTTATGCTCACTGCAGCAGGTGATCAGAGCGGTGG
ATTTAATGGATTACGTCCATTATCAATTAATAATCAATCCGGTCAGGTTACGATTGGTGAAAGCTTAATC
ATTGCCAAAGGTGCTACTATAAATTCAGGTGGTTTGACTGTTAACTCGAGAATTCGTTCTCAGGGTACTA
AAACATCTGATTTATATACCCGTGCGCCAACATCTGATACTGTAGGATTCTGGTCAATCGATATTAATGA
TTCAGCCACTTATAACCAGTTCCCGGGTTATTTTAAAATGGTTGAAAAAACTAATGAAGTGACTGGGCTT
CCATACTTAGAACGTGGCGAAGAAGTTAAATCTCCTGGTACACTGACTCAGTTTGGTAATACACTTGATT
CGCTTTACCAAGATTGGATTACTTATCCAACGACGCCAGAAGCGCGTACCACTCGCTGGACACGTACATG
GCAGAAAACCAAAAACTCTTGGTCAAGTTTTGTTCAGGTATTTGATGGTGGAAACCCTCCTCAACCTTCT
GATATTGGTGCTTTACCTTCTGATAATGCAACAATCGGAAACTTGACAATAAGGGATTTCTTAAGGATTG
GTAATGTCCGCATTATTCCAGACCCTGTGAATAAATCTGTTAAATTCGAGTGGATTGAATAAGAGGTATT
ATGGAAAAATTTATGGCTAAGTTTGGACAAGGATACGTCCAAACGCCATTTTTATCGGAAAGCAATTCAG
TACGATTTAAATTAAGCATAGCGGGATCTTGCCCGCTTTCTACAGCAGGACCATACGTTAAATTTCAAGA
TAATCCTGTAGGAAGTCAAACATTTAGCGCAGGTCTTCATTTAAGAGTTTTTGACCCTTCCACCGGAGCA
TTAGTTGATAGTAAGTCATATGCTTTTTCGACTTCAAATGATACTACATCAGCTGCTTTTGTTAGCTTCA
TGAATTCTTTGACAAATAATAGAATTGTTGCTATATTAACTAACGGAAAGGTTAATTTTCCTCCTGAAGT
AGTATCTTGGTTAAGAACTGCAGGAACGTCTGCTTTTCCATCTGATTCTATATTGTCAAGATTCGACGTA
TCATATGCTGCTTTTTATACTTCTTCTAAAAGAGCTATTGCATTAGAGCATGTTAAACTGAGTAATAGAA
AAAGCACAGATGATTATCAAACTATTTTAGATGTCGTATTTGACAGTTTAGAAGATGTTGGAGCTACCGG
GTTTCCAAGAAGAACGTATGAAAGCGTTGAGCAATTTATGTCAGCGGTTGGTGGAACTAATAACGAAATC
GCGCGTTTACCAACTTCGGCTGCTATAAGTAAATTATCTGATTATAATTTAATTCCTGGTGATGTTCTTT
ATCTTAAAACACAGCTATACGCCGATGCCGATTTACTTGCTCTTGGAACTACGAATATATCCATTCGATT
TTATAATGCATCAAATGGATATATTTCCTCGACACAAGCTGAATTTACCGGGCAAGCTGGTGTTTGGGAA
TTAAAAGAAGATTATGTAGTTGTTCCAGAAAATGCAGTAGGATTTACGATATATGCACAAAGAACTGCAC
AAGCTGGTCAAGGTGGAATGAGGAACTTAAGCTTTTCTGAGGTATCAAGAAATGGTAGTATTTCGAAACC
CGCTGAATTTGGTGTCAATGGTATTCGAGTTAATTATGTCTGTGAATCTGCTTCACCTCCGGATATAATG
GTACTTCCTACACAAGCATCGTCTAAAACTGGTAAAGTGTTTGGGCAAGAATTTAGAGAAGTATAAATTG
AGGGAGCCTTCGGGTTCCCTTTTTCTTTATAAATACTATTAAAATAAAGGGGCATACAATGGCTGATTTA
AAAGTAGGTTCAACAACTGGAGGCTCTGTCATTTGGCATCAAGGAAATTTTCCATTGAATCCAGCCGGTG
ACGATGTACTCTATAAATCATTTAAAATATATTCAGAATACAATAAACCACAAGCTGCTGATAACGATTT
CGTTTCTAAAGCTAATGGCGGTACTTATCTAAACCGAGTTATCTTTAACCGGGGTATTCAAGTCCCGGGA
GCCGGCAATGGTCTGACCGGGATGTTTGTTGGGCCAGGTGATGGAGCAACTTATGATAATGTTAACCTTG
 
ATATAATATCTTGGTATGGTATCGGATTTAAATCATCTCAAGGAACAGGACCCAGGACAATTGTTTTTAA
CGTTCGTGACGGTGAAATTTCGGCCCGTGGTAATATAAATTCACAGCGTCAGGTGCGAGCCGAAGCCGCA
GCGCCCATTGCCGCGAATGACCTTACTAGAAAGGACTATGTTGATGGAGCAATAAATACTGTTACAGCAA
ATGCGAACTCTAGAGTGTTGCGTTCTGGCGATACTATGACAGGTAATTTAACCGCGCCAAACTTTTTCTC
GCAGAATCCCGCATCTCAACCTTCACATGTTCCACGATTTGACCAAATCGTAATTAAGGATTCTGTTCAA
GATTTCGGCTATTATTAAGAGGACTTATGGCTACTTTAAAACAAATACAATTTAAAAGAAGCAAAACCGC
AGGTGCACGTCCTGCTGCTTCAGTATTAGCCGAAGGTGAATTGGCTATAAACTTAAAAGATAGAACAATT
TTTACTAAAGATGATTCAGGAAATATCATTGATTTAAGCATTTCTGCTGGTGGTAATATCAGTGGAAATA
TCACTCAAACTGGTGATTATACACAGACTGGAAAATTTAATTTAATTGGTCCGCAAATTGTAGCAAGCGG
TGGATATATTGAGTTTAATTATCGTACTACTGGAAGTGGGGCTTGGTCCGGTCAACATACTGCGAAAGCT
CCAATTTTTGTAGATTTAAGCTCAGCTACTTCCACATCTGAATATAATCCTATAATTAAACAGCGCTTTA
AAGATGGAACTTTTTCATTAGGTACTTTAGTTTCTGAAGGTTCGTTGAAGATTCATTATATCAATGAATC
TGGCGATTCGAAATATTGGACGTTTCGACGTGACGGCGGATTTACTGTTGACGGTGGAGGTTTAGGAGTA
TCAGGAGGTAGTATAACTACATCTGGAAATATTGCAGCTCTCGGAAATATTACTTCGCCTCAGATTAATA
CTAAAAATATTATTTTAGATACAAAAGCATTTGGACAATACGATTCGCAATCATTAGTTCAATATGTTTA
TCCTGGAACAGGTGAAGAAAATGGTATAAACTATCTTCGTAAAGTTCGAGCTAAATCAGGCGGAACCATT
TACCATGAAATCGCTTCAGCTCAAACCGGTAAAAATGATGAAATTTCTTGGTGGACCGGAAATACACTTA
CTACTAAATTAATGGGTCTTCGTAATGATGGCGCAATGGTATTACGTCGTTCACTTGCTATTGGCACAAT
TACTGCTGACGAAAATACCAATAACTACGGCTCTCCTACTCCAATGGGAGAAAGATATATTGCTTTGGGC
GATGCTGCCACTGGTTTAAAATACATTAAGCAGGGCGTTTATGATTTAGTAGGTAATTGGAATTCTGTTG
CTTCTATTACTCCTGATAGTTTCCGTAGTACTCGTAAAGCATTATTTGGACGTTCAGAAGACCAAGGCGG
AACCTGGACAATGCCTGGAACGAACGCTGCTCTCTTGTCTGTTCAAACTCAGGCAGATGTGAATAACGCA
GGTGATGGACAAACTCATATCGGTTATAACTCCGGCGGAAAAATGTCCCACTATTTCCGCGGTAAAGGCC
AAACTAATATTAATACTCAAAAAGGCATGGAAGTTAACCCTGGTATTTTAAAATTGGTAACTGACTCTAA
TAACGTACAGTTTTATGCTAATGGAACAGTATCTTCAATACAAAGAATTAAATTTGATAACGGATTAGTT
CTTACTGGTGCTAGACCCGACGGTATTCAACTTGACGCTCCTACAGCAGCAGATGGCACTAAAACTATAC
TGTGGGCTGGTGGTACTCGCGCAGGTCAGAATAAAAGTTATGTATCTATTAAAGCGTGGGGTAATTCATT
TAACGCATCTGGCGATCGTGCTCGTGAAACGGTTTTTGAGGTAGGTGATGGGCAAGGTTTCCATTTTTAT
TCTCAACGTGTAGCTCCTGCGCCTGGTTCGACTGTCGGGCCTATTCAACTCCGAGTTAATGGTGGTTTAT
TAACTGCAGGTAGTATTGTTGCTTCTGGTTCTATTACAACTGAATCTTCTTTAAATGTTAATAATGGATT
GTCTGTAAATGGACAAGCTAAATTTGGTGGAACAGCAAACGCACTGAGAATTTGGAACGCTGAATACGGT
GTTATTTTCCGTCGTTCTGAAAGTAACTTTTATATTATTCCAACCAATCAAAATGAAGGCGAAAGTGGAG
ACATTCACAGCTCTTTAAGACCCGTGAGAATAGGATTAAACGATGGTGCGGTTGGATTAGGAAGAGATTC
TTTTATAGTAGATCAAAATAATGCTTTAACTACGATAAACAGTAACTCTCGCATTAATGCCAACTTTAGA
ATGCAATTGGGGCAGTCGACATATATTGATGCAGAATGTACTGATACTGTTCGACCAGCTGGTGCAGGTT
CATTTGTCTCGCAAAATAATGAAAACGTTCGTGCTCCATTCTATATGAATATTAATCGTACAGATACAAG
TACTTACGTTCCTATTTTAAAACAACGTTATGTTCAAGGAAATAGCTGTTATTCTTTAGGTACATTAATT
AGTACGGGTGATTTTAGAATTCATTATCATGAAGGTGGTGATAATGGTTCAACAGGTCCACAAAAGGCGG
ATTTAGCATGGCAATTTAGACGTGATGGCTCGTTCCGTTCACCTAATAAAATTGAAATTAATGCCGTTAC
AATTGGTACTGATGGCAACATCACAGGTGGTACTGGTAATTTTGCCAACTTAAATACCACGTTAAATCGT
AAAACGACTGTGGGAGGTTGGGCTGGTAGTTCCGTTGTTGGATGGTACAAATTTGCTACAGTAACAATTC
CTCAGAGTACTGGCACAGTAACTTTTAAAATTTCCGGTGGAGCTGGTTTTAATTTTAAAAGCTATAACCA
AGCTTCTATTGCTGAGATAGTTCTTCGCACAGGTAATAATCCTAAAGGCATAAACGCTGTACTATGGAAC
AGGTCAGACCTGAGTTTTAATCAGATAGCTACTATGAATACATCTGATGATACTTATGATGTATACGTAT
 
TCTGTGAAGGTTATACAAACGCATTGATAGTTGAATATTCATGTAGTGAAAACTCTTCAGTAACTGTAGT
CGGTCTTAACGGAGGCATTCAACCAGTTGTTGATGCATTGCCTGAGGGACATGTTGTAGGTAAAACAGTT
CGTCTGTTAAATAATATTGGCGGAACTTTCGCAGCTGGCGAATCTGACATTGTTACTCGTGGTGAATACG
TTGCTGATAATCAAAAAGGCATGCGTATTAAATCTAACGGTAATAATATCGGTTCTAATGCTGCTATACT
TCGAAATGACGGCGGAAGTTTTTATATTTTAGCTACAGATAAAAATACAGCAGAAAAATCCGATGCAGCT
AATGGTGATTGGAATGGCTTAAGACCTTTCGCCATTAATATGGCTGATGGTCGCGTTGGTATGAACCATG
GTCTAAATATTACCGGCGGTGGCTTGAATGTTACTGGTGGCTTGAATGTTACTAGTGGTAATACTAGTTT
AGGTAATATTTCGTCTCGTGTGGTGGCTCGTTGGCGAGGAGCGTCCGGTTGGGCTGATAACTCGGATACA
ATGAAATCTAAAATCACCTTTATGGCAGACCATGGGGATTTATCTAATTCAGACAGTTATTATCCTATTG
TAGGCGCATACAGCAACTATGGTTCAGCGGGTTATCGTCAAACCTTTGAGTTCGGATGGGTCGGTTCCGG
CACTACAGCGGGTTGGCGCGATGGTATTATTCGTATTCGCGGAGATAATGCTAATGGCCAGCAAGCAAGA
TGGCGCTTTACAATGGACGGTACTTTAGATTGCCCTGGTAAAGTACTGCTACCACAAACAGGTGCCTTTG
GTGTCAATACATCAAATGGTCTCGGTGGAAATAGTATAACATTTGGTGATAGCGATACTGGTATTAAGCA
AAATGGCGATGGATTATTAGACATATATGCGAACAGCGTACAAGTATTCCGTTTCCAAAACGGTGATTTG
TACTCATACAAAAATATAAATGCTCCAAACGTTTATATTCGTTCTGATATTCGTTTAAAATCCAACTTCA
AACCTATCGAAAATGCGCTTGATAAAGTTGAAAAACTTAACGGTGTCATTTACGATAAAGCTGAATACAT
CGGTGGAGAGGCAATTGAAACTGAAGCGGGTATTGTGGCTCAAACGTTACAAGACGTTTTACCAGAAGCT
GTCCGTGAAACAGAAGACAGCAAGGGTAATAAAATACTCACTGTTTCTTCTCAAGCCCAGATTGCTCTTC
TAGTTGAAGCTGTGAAAACACTTTCTGCTCGTGTAAAAGAACTTGAATCTAAACTTATGTAAAAGAGGGC
TTCGGCCCTCTAAGGATTTTTAATGGCAATTGTAGGTGTTCCTGGCTGGATTGGAGAATCTGCCGTGAAT
GAAACTGGACAACGCTGGATGGATGCTGCGATGAGAGCTGTTCATGTTTCAGTTCCCGGCTGGATGAGCT
CAATGGCAGGACAATCAAAAGAAATTTATCTATCTATAGGGGCTAACCATAATTATGATAGAAACTCCCT
TATTAACTGGATGAGGGCTCAAGGTGGTGCGCCCGTGGTTATTACAATCACTGGTGACTTAGTATCAAAC
AGCACAGGTAACGCTTGTTTGGAATTTCCTAGTGACCTTCCTAACGCGTATATTCAACTTATCATTAATA
GCGGTGTGACTGTTTATGGCCGAGGAGGTAATGGTTCTACTAATAGTTCGGCAGGTGGAAACGGTGGTAC
AGCTATCCATAATGCAGCCGGAACTAAACTCCGTATTCGTAATAACGGCGCTATTGCCGGTGGTGGCGGT
GGTGGCGGTGCCGCATCATTGAAAAATAGCTATCCAACTAATGGTAGCTGCGGTGGTGGCGGCGGTAGAC
CATTTGGCGTAGGTGGCAAAATAGGCTCTGACTCTATATTGTCCGGTTCGAATGCGTCTTTAACAGATGC
CGGTACAGGTGGTACTACATTCCAATACGGCGCAGGTAACGGCGGTAACGTTGGAGCTGGCGGTGGACGA
GGATGGGGCAAAAATGTTTATACTTCAGAGGGTGGCGCAGCTGGTGCTGCTGTCACAGGCAATGCTCCTA
ACTGGCAAAACGTAGGAACTATTTACGGCTCAAGAGTCTAATTTTTGAATAAATATCTTAAAAGGAGGGT
CTATGGCAGCACCTAGAATATCATTTTCGCCCTCTGATATTCTGTTTGGTGTTCTAGATCGCTTGTTCAA
AGATAACGCTACCGGGAAGGTTCTTGCTTCCCGGGTAGCTGTCGTAATTCTTTTGTTTATAATGGCGATT
GTTTGGTATAGGGGAGATAGTTTCTTTGAGTACTATAAGCAATCAAAGTATGAAACATACAGTGAAATTA
TTGAAAAGGAAAGAACTGCACGCTTTGAATCTGTCATCCTGGAACAACTCCAGATAGTTCATATATCATC
TGAGGCAGACTTTAGTGCGGTGTATTCTTTCCGCCCTAAAAACTTAAACTATTTTGTTGATATTATAGCA
TACGAAGGAAAATTACCTTCAACGATAAGTGAAAAATCACTTGGAGGATATCCTGTTGATAAAACTATGG
ATGAATATGCAGTTCATTTAAATGGACGTCATTATTATTCCAGCTCAAAATTTGCTTTTTTGCCAACTAA
AAAGCCTACTCCCGAAATAAACTACATGTACAGTTGTCCATATTTTAATTTGGATAATATCTATGCTGGA
ACAATAACCATGTACTGGTATAGAAATGATCATATAAGTAATGACCGCCTTGAATCAATATGTTCTCAGG
CGGCCAGAATATTAGGAAGGGCTAAATAATTATTTGTTTGTATACATCTCTAGATATCGATATACACCCT
CAAAACCCTCGTTGAATTCGTCGATGAGGGTTTTCTTATCTTCTTGAGTTAATTCAGAAACAATTTTACG
GAATGAATTCTGATTTAACTTTCTACCTTCATGCGTTACTCCAATCTCATTCAGAAATGCAATAAAATTA
GCACGATTCTCAACAATATCTTCTCTGGAAAATTTAATCAAAATAGACGCAACAGTAATAATTTCACGAA
 
CTGTATCAATATTTTTATTCATTAACTATACCACTCAATTAGTTGACTTTGTTAATATCATCAGACGCTT
GATTTGTAAACTGGTCTGTGTTATTTTCTTCAAAAATTTTTTCTACGAATTCCTTGAACGACTCACGTTC
CTGAGCTACATTGTGCTCGATTACATTTTCAAGATTATGACTCATTCGAAATAATCTTCAATTTCGTAAT
CATGGACATAAAACATTATAGTTTTTAATACATCATCAATACTTTTTCCTGGAGCTGGAATTACGTAAAA
ATACCCTGCTTTTGAGAGGTCTTTATAAGTTCCAATCAAGAAATCATTATTCTCAAGATGTAACTCTTCA
ACTAATTCATTAACAATTGAGTGGTATAAATTTGGTAGAAACTTATATAGCTTTTCTAGAATATCAATTT
TGATTGTGTATTGAACCACAGATTGAGAATCAATAATCATAGACCTTCCCCTTATGTTTCTGTTTGCGAT
TAGATTCTTTAAACGCTTTCTTCTTATCCTTATGAACAGAAGCTTTATTAAAATTATGTTTTGCGACTAA
ATTGTTCATAGTGCTGAATTACCTCTCTCAAACATTTGCATGTGAATGAAAACTTTTTAGCTACACCACA
TTCAAATATATGTTCTCTTAAATCACGTGTATCGGTATATCCCATCTCAACGATAAAATGCCGTATTAGA
TTTTTATCTTTATCATTGAGAGAATTAAAATAATCGGATTTTGAATTAATTTCCCTGGCCAAATTGAATC
ACCTTCAGTTGACGTTTTAATTCTTTTATCATCTCTTCGTTCATCGCAATATAAAGATCGCGTAAAGCAG
GTTTTAACATTCCATTTACTGGAGAACTAAATGGACATACATAATCTTTTCCTACGAGCTTTTTAGTGAA
TTCAATATTACAGAACTGAAATGCCGGCTCATTGGTATAAATTCCCCAATTAGTTGACATCATTTTATTG
GCATATTCCAGTGCCTGGATTTGATTCATAATTCCATCAATTTGAAACTGTTTAATATTCATTAGTAAAG
GTCCTCAGAGTAAAGTTCTTTTTCACTACCACCACGTTCAATACGTACTTGTCCAGCGTAAGTTGCAATA
ATCATTGCTTCTTCACGTGTCCAATAATTACTATATTGGTCAATAAACCCTTGGTCATCATCACAAACTT
GTTGAGTAACTAATTGAGGTTTAACTACATCTAAAACTTCTGCCATATCTTTAGAATAATGACGAGCACC
TGGAATAATAAGAGTTCGTCCATCTTTTAATTTAAAACGATTAGCTGCACAGACAATTCGTCGTTGATAC
TTTTGGTTTTCATCCCAATATGCAGTCTGCCAACAGATTTCAGGAACTTCTTTCAGAATATCTTCTTCTG
TGCATTTATAACCATGCGCTTTAAATTTTTCAACAAGACTTTCTGGAGTTTCACGAGATAAAGGAACATT
CAGCATTTTTAAACGATTAATAAATGGGTTCATTTAAACCATCCTTTAATACGTTGCCACAAAGTTTTCT
GCTGAGCTTTGTTGACACCAATTGAACGAATAACTGGTTGAGATTCCTGGAATTCTTTATAATCGGCAAG
GTAAATTTCGTAAGCTGCATCCGTAAATGAACTTATCGCTGCCATAAAATTATTGCGAATACCTACTGGA
GCATCTTTACTTTCACGAATAATCATGTATTTACCAGTCTTAATCTTTACAATAGTTCCAAGATAAGCTA
TGGTACCAAATATCCCAACCCTCTTGAGTAGGTTCTGCACAACGACGAAGTTCATTAACAATTTCTAACT
TGTTCATTATTTATTCCTCACAGTTCAGATGCTACAGTGATTACAGCTTCAATGTTTTCTGCCGAGCGTT
TAATGTCAAGATACACATTACCGTTTTTAGCGATTTTACATGACATTCCGATGTCAGTAAATTTCTGAAT
ATGATGTTCCATCATTTTATATCCAAAAATTCGCATATTTCCATTGTTGTTAATTTCAAAATTACGAATT
CCGTGAGTGCGTTTTTCTAAAATAGCGAGATAGTTACTGCGATAAGTTTCAACCTTTTTAAGAACAAATC
CATTTTCATCTAAAAGTTTTAACATGAGGTCTTTATCTTCTTCCATATCGGAAGTAATCTCACGAGCTTT
ACGAGTTGCTCGTTTTTTCAGCAGTTCCGGAGCATTTTCTTGTGCGTATAAAGTTGCTGCATTTGAAATA
ATATCCTGAGCTTCACCAGTAATGATTAATCCATCACCAGATTTCTCCACCAGGCCTTTTTTAATCAATA
CCCCAATATTACTATTAACTACTGCGTTACCTAAATCTGGATGCACCTCACGAACTTCTGCAGCTGTAAT
GAAATCTTTCTTAGCAATGGTAATTAAAATCGCAGCAGTTTTTTCATTCAGAACATCGTTAGAAGCTTTG
ATGATGTAAGTTACTTTAGACATTTTCTAATCTCCGTAATTCTGTATCAGTAGTTGATAGTTGTATAATA
CCACAGTATGCTTTGGTTGTAAACCGTTTTGTGAAAAAAATTTTAAAATAAAAAAAGGGAGAGCCTCGGC
TCTCCCTAAAATTACTGCATGACTGTGATAACTGTCATGATAACACGTTGAATTCCGAACGCAAGAAGAC
CTCCTGCTACGGCTGGAACAACGCCTAAACCCGCCAGTAAAATGCTACCGGATACTAATGCAGCGCTTGT
AATACCTATGAATGGACTCATTTGATTTCCTCTAAATCTTTGGTGTATTCGGTAACTACATCAGTAGTTT
TCCAATATTCGTTTTCTTCTTTTTTAGCTTTAGCTTCTTCAGCAAGTTTCTTCGCTTCATCGGAAGTCAT
ATGAAAAATATTCATTCCAACTAGTTTATCAACATAAGAAGAATACATATCGATTTTCGAAAGTTCTTCG
GTCAGTTCTTTACGAGTTTTACCCTGTACAACAATTTCACCTGAAATTACTTTCTTAATGAAATGTGCTT
TGGCAAAGGCTAAACGAAAAGCTGACTCAGTTTCTTTAATTTTGTTATCAATTCGTTTTTGGACATAAGT
 
TTTACGAACTTCAACAAAGTCTTTAATTAAATCAACTACGTTATCGTAAACTTGCAGCTTTCCTTTCTCA
TTAATAACCGTAATATTCTGGGAACGACGTTCAATCAGTCCGAAGTCTTTCATAATTTTTGCATGGCGTT
CTTCTTCGTTATCGCTCAAAGAATATTCTTTACGGAATTTAACTTTGAATCCAAAACCATGCTCACCACA
AGCATCATCCCATGTAATGAAGCCTTTATCTTCAAGTGGGTCTAAAATTTTACTCACGTAAGTTTCACGA
TCATACTTATACGGAATCTCAGTGATATGCATTTGAGTTCGTGAAGTAAACTTATATGTTCCGCGAATTT
CATATTGACCATCAATCTCAACGACTTCACCACGAAATTCTGGGAATTCTACCTTCGGTTTAGTTACTTT
CTTTCCTTGAAGAGCTTGCAGTACAGCTTTCTTGACAGAAGAAACACTATGAGGAAGAATGTAAGTTGCA
TAACCAGTTGCAATACCGGAAACGCCATTAAGAAGAACAGTAGGAATAATAGGCAAATAGAAAGCAGGCG
GAATGTGTTCTTTATCTTGATGTACCGGAGCATATTCAGTATCTTTATATACGTTATAGAAATTTTTACT
TACACGAGCAAAAATATAACGACTTGCCGCTGCTTTTTGAACGGTACGAGAACCGAAGTTTCCTTGACCA
TCTAACAGAGGAAAGTTATTATTCCAAGTGTTAGCCATCAAAGCTCCCGCATCTTGCGCAGAGTTTTCTC
CATGGTGATATCCAAGGTCCGCTACACCACCTGCAATAGAAGCGAGTTTGTGAAACTTATCTTTATTCCC
TCGTGCCAAATCAAGAGCTCGAGCAATAACAAATCGTTGAACCGGCTTAAATCCATCAATCATATTTGGG
ATAGCACGATTTTCAACCGTGTACATAGCATAAGCCAATGCTTCATTATCAATGATACTTTTTAAATCGC
GATTATTCAGTTGCATAAATTTACCATACTAGTGAATGTAGTGCCATAATAACATCAGAAATGAAAAGCA
CGACTTGAATTAATCCGAACATTATTCCGTAATATAACGCTATCAATAAAATAGCAAGGTCTAATGAATA
GCCCAAGATTTTCTTAATCATTAGATAACAACACAAATGTTAAATATGCACACATACCCTGGGCTAAAGC
CTGCGAAAACACACTACTGGCATCGATACAGATAGTTAAAACACATGCTACTATCCAACAAATAAATTAA
ATAACTCCTAATAATTTTGCGATATTCATAGTGTTCTCCTCAACTATTTTTTAAAACCTAAAATACCAAA
GTTTTTAAGATTGTTACGATAGAACTGCATCACATGTTCGTTATGGAAATTACTCATTAATATGCCTGCA
AAACGAATTTAAAATTATCAGCCAACATACGGTTCATTTCTTCAAGTGTTTGATACTCAGAATGATGATT
ACGAGTAAACGCTAAAGCTAACTGTCCTTTTCCAAATCCCGTCGTCAGAGGTTTCATCTTAGAAGCAGGC
AGATAAAATACTGTGTATGGAACATTGTTATTTGCAATAGTACGTGCAAGCTGAGACCGACGTTGACGAA
TATGACTTAGAACTGCACTGAATCCTTGCTTAGAACGCTGATTACCTACATAAAATCGTGCAGACACGCA
TGGATTACTAAATGGACGACCATCTAATTTACTCACTAAAAAGTAAAATCCAGGTTTAGATAAAATATCT
TTATGCGGAGTTCCTAAAAACCATTCACCACCCTTAATTGTACCAATAACAGTAGCGCCTGCATTATTCA
GATCAGTAACAGTCATATATTTCATATTAATTTCCTCTAAATTATTTTCTACTCCAAGGCCGCATGAATA
CACACGGCCATTAAATTAATCGTCGCAGTCGACGCTCAATTCCCAAAACTCTTCAACAGTATAAGTTTCA
GTATCATTTTCAATACAGAAACGTTCATTACTGTTATTTGCTAAAGTAGCGTTAACTGTCATTTTCTCGC
TGGTGCTCTTAAGAGGTGAAATACGAATTAGCTGATCACCACTATCCAAGCAAAAAATTTCGCCAACTTT
TACATCTTTAAAAATTTTCATAATTCACCTCAGGGAGTATAAAACCCAAATGCAGTTGTTGACCATCCCA
TCCAATACGGAAAATTTGCACCGATGTAAAACACGAGAATATAAAACCAAATACCCAGTAAATTCATCAT
TTTACACCATTCCAAATTGTTTCAACTACGGATTTTAAACCATTTTGATGAATATCCATTCCGACTACTT
CCATCAAATAGATTCCAACTACAACTTAACCCAAGGCGAAAATCAGCATGAAAATGAATAAAGCCGGAAA
AATATTATCGAAAAACCATTCCATAAATGTAAAAGCACTGCGTTTACTCTTCATATTTTCCTCACATAAA
TCCAAAGTAAACGTTTAATACATCAATCATTAAAACGATTGGGAATATACTCAAAACTATTAGTATTATA
ACTACATTCCATATAGCTTTAACAATCTTTTTCATTTTCTGTTCCTCCGTAGTTGATAGAGTAATAATAC
CACAGAGGAACAATCTTGTAAACAACTTTTTTAAAAATATTCGTAATAAATGTGAATACCAATCACTACT
GCCGAAACTTGTGCAACCCACCACGCACAAGCAATAAGTACAGAATTAAAAATTTTCATAATAACCTCAT
CACAAAAGTAAATGTTAAACAAATTAATGGAATACTAATTAACCAAACAAAACACCACCATAATGAACTC
ATAGTTCAATCTCAACGATTTTCATTTTACTCAATAATCCGCTAAAAGATCGACGATTGCATCACGAAGA
CACTTATTCGCAATACGTTTAAGAGCAATTTCAGTTTCTGGAGCATTTTGAATAATCATTGAATTCAATT
CAGCCTCAGCAAACTGAAGTTCTTTAACTAATTCAGCTTTATATTTTCCTTGCCCTTTCTTAGCTTTTGC
TCGTTTTAGTTCAGCTGTAGTAAACATTTTATTCTCCAAATCCGTATCAGTTGATAGTTGTATATTACCA
 
CAGTCCTTGTGGTATGTAAACCGTTTTGTGAAAAAATTTTTAAATGGAAAGATACCATCCGTTGTAGTTG
CTTTTTCTTACAACCTTACGAAGGTCTTCTCTATCACCGATGAATTTCGGAGTGTACTGGATGACACCTG
GATGAATTTCTTTAGTGTTGAATATAATTATACAGTCAGCGACTTGATGATTTAGAATGGGCCCTAGATT
TATTCCAGAACCATATGGATACTCTCCGCTGCATCCGGTTGTTACCGAAATCCATCGTGAGTCAGTTTGA
TGTGTTTTGACTTCTACACGAAGCCCACAGTATTTTGGATGCGCCAATACATCCCATGCATATGTATATG
GGTCATTAACATCCTCTTGGCCTTTGTTAACATACCCGCTTAGCCAATCTGCTACAAAAAATTCTGCGTA
CACAGCGATACGACATCTTTCAATAACTTCTGCTTTATCCTGGTTCGGGTTTTGTTTTAAAGAGTATCTT
GCTGTATCAGCAATTTTGACCTTCATTTCACTAGTCAAGTCACTGTTCGATAGGGTAAATGTCGGAATCT
GAAATAGTCTCTGTAAACCCGGATTCGTTTTCTGCATTGAACTTTCCTTTTACGTCGGAATCACTGATAT
TCATATAAATCATAATTTCTCTTAAAACAAAAGGGCCGAAGCCCTTATTTTATTTGAATTGCGCAATTCT
TTTCTCTAGACACTCAGCATAAGATTTCATTGAGATAAACTGCGAAAGTAGCAGTTCTTGCTCAACTGCA
CTAACCGTTAGAAACTTTGTGCTTTCTAAAAATTTACTCAGTGCATTAATTTTGAGCATTAATTGATCGT
ATTCTTCTTTTACTCGTGCTTGATAACCTAACATAATTTTCCTTAATATGATAAGGGCCGAAGCCCTTAT
TTAAATTGTTCAGTAACGTCTTCAACTACTTCGTATTGGCAGGTACGCATTTTAGCATCGTTGTAATCAA
TCGGAATTGATACTACATCACGCGGATGTACTTTAACTTTTACAACTCGACTGGTTGAACTGCCAAAGTG
ACGAATATAAGATTTAGAACACACATGCAGACCACGAGAACAAGTTTGTGTATCATCGTCATTCACACGA
GTACGTGGCATTTTAACTACTTTACCCGGACTGTTATCAAAGGTGTTTGAGTGACAGTCAAAGTAGTTGT
CACGAACTACTTTCCAAGCATAGAAGTAACCATCTTCGGTGATTTCAATATCGTTTGCTACCAAGAAATC
AAAGAGTCGAGATACCGCTTTTTGGCTTGGGTTTTCCAGCAGATTTTCCAAGAACGGAAAATAAAATTCA
AAGTTTTCGCCTTTTTCCATCGAGTCAAGAATACGATCAACCAAACCAGACCGCAATTCAATATTTTGAT
AGAACAAGCTTCCACCTTCAATTCGAACATCGCCGGAAATATATTTTTCAACAGCGCGACGAACATTAAT
TTTTTGCGCAGCTTCTTCCAGCTTATCCGCTACAAGCAGATTAAGAATTTCCTGAAAGTTTGAATGAGTA
TTAGGAGTTGCGTTATAAGTTACACCGTCAACAGTAATTGAAATGAATTTTTTAGATGCATTCCAAATAA
TGTCAGATTTAGCAACTGGAGCAATAACTGCATCGCTATTAACTTTAACTGTAATATCACCGCTAATAGT
AACTTTAGGGCGTTTAGCTTCTTCAGCATTTTTCAAAACACGACGGATTGTGTCAACCGATACACCTTGC
CAATCAGCCAATTCCTGTTGGGTGTAATTACCACTTGAATACAGTTTAACAATTTCAGCTTGTTCGTTTT
TGGTCAGGCATTTAATATTGTACAT
**Enterobacteria phage RB69, complete genome (GenBank: AY303349.1) (SEQ ID NO: 12)** 
TTATTTGGCCGCTTCCACGGCCTTCATGAATTTAACGATTTGAGCGACCTGTTCTTTGGAAAGAGTACCA
CGTCGTCCCATGTATTCTGACACAATATGATAATTGGTTTCAAATTCAATAACCATTTTATCATCATTAT
CGTAAGCATTATCTTTAAGCTTATCGAAAATTTTAGCACACAATTCTAACTTCCTGTTCAGGTCTCTGTT
ATCAGCACCTGTGAATCGATTCCATCCCCAGCGATTATTTACTGTTGAGAGTTTTGCAAAGTCTTTACTG
ATTTCTTTGTTCTTAGAGCTAAAATATTTACTAAGGAAATTAAGCTCTTCATTACGAGTAATATGATTCA
AATAAGGCTGTGCTCTACGACTTGGTGATACATACTCATCATAATCCACTTTATCAATTAGCGTAATATA
GTCATTAATTGATTTTTCGAACAAACATTCTACTTCACCAAGTTTACGAACTTTCTTAACAATCTGCGGA
CGGACTACAGTGAACTCTTTAATACCTGACAAATCGGCAATACGAGTCAAGAAATTAGAATCATAGTTCA
ACCAACCTTTATCTGCGTCAAGACATTCAGCGTTAGAACGATTACGAATTACAACATAACCTTGAATCTC
ATCAGCTTTACCGGCCGGAATAAAGAGATCTTCTGATACCCAAACATCGTTTTCATTCTTAAAGAACCGA
TGAGCACTTGGGGATTTAGGACGAGGCTGCGATGAACGTTCTTTAACTTCAATCCATGGTTTAACTAATG
CAAGAATTTCAGAGGTCTTATAAAATTTAAGGCTATCACCAGCGAAAATAACCTTTAATCGTTCCAGAAG
TTTCATTGCAACTTCATCTTCCGGGTTAATGAATAATACGCTACTACCCCGTCCGGAAGGAACCTTATCA
ACGAGTGTTGATGCATCTGTGGAAGTCAAAGCATCGTTCAGAGCACGCATCATTGGTACGCGGCCTTTCA
CATCGTCAATAACAATATCAATATGCTTACGACTAATACCTAGCAGGCTATTAATTGAAATAGTTGCAGA
 
CGAGCTTCCGCTTTCACGAATACGACGAAGACGTGGGTCTGAAACGATATCATATACAACGCCAAGGTTG
CACCATTCAGGTCCCATTTTAAAACGTTTGTACAACTCAGAATAAGTTAGCTGTTCTTTAGAATATTTCA
GTGATTCAATATTAGAACCAGCTTTTTCCATGTATTTACGAGCAGAGTAACCAAGAGAATCTATTTCACG
GAATACATGACGTGGATATTCACAATCAATCCATTTCTTAGAATCTTCAGCAAATAATTTTGCATCAATT
TCTGCAACACGTTTATGGATATTTGCTACTGTACGTTTATCAAATGACAATGCTTCACGAGACGGGGCTA
CATCTAGTTCACCCATAGGGAATTTAATATAAGCTGTAGTGCATCGCGTCATCATCCATGTTTGTTCTTT
AATAACACTTCCGATTGGATATACAATACCACCATAAACAGCCATGATATTACCACGTTCACCCCATGGA
GCTTCCTTGGCTAAATAAACATCATCGAATTCAGGGAAGTATTTGATATCTTTAACACCACGAACTTCTG
CAATATCGCCGAGAGGACGCATAACATAAGCAATTTCGGATGCGAACTTTTCAAAATCTTCTGGGTTAAC
AGGCACAACTACTTCAACGCCGGTCCGGTCATCAGGACCCATCTTATCTACAAAGGTCGGCTTAATTTGT
GGACCTGTGTCATCTTGGTAGATTACGTAACCACGAACTTCACCATTATGATAAGAAGTAATGTTAAACG
TATCAGTATATGCTAATGGAGCTTTAGAACCGAGCCCGAATCCACCAATGAAATCGTTACTCGATGTTTT
AGTTGAAGCAAAATATGAGTTATAAATTCCTGGCTCTTCGTCATTACCTCGAATGGTGAAATCGCTCATA
CCCGGTCCAAAATCACGAACTACAAAACGTGGGTCTAAACGACCTGGAGCCTGAACAATAAACTTATCAG
TGCAACCGTTAAGAATTTGTCCATCGATACAGTTTGTAATTAATTCACGTACACAAGCCAATTCTTTGTT
TGTATAAAGGTCATTAGACAAAATCTTATAAACTTTGCTATTGCCTTGAATAGTGAATGCTGTGCTCTTA
CCGCCTGAACCAATAATCGTTTCTTTAGCCGTTTCGATAATCATGTTTTTCTCTCATCACAAGTTACGTT
TAAAAATTTCTGCCACTTCGAGAAGTTCTTCTTTAGTAGCTGTGTCGGTTTGTATTTTATATCGAATTTC
TTTAAAGCGTTCTTTAAAGGACTCTGCTGATTCGATATCAAAAAATCTTTGGATAAGCCGGAATTCCCGG
AGCACTGCTTTATCAAAAAGTTCCAAGTCCACGTTATATGTTCTCATAATATTCTCTTCAGTAGATTCCT
AGGGCGCTATTACTAAAGACCATATAATCTATCATCTTATGTGTAATAGTGCCTCAGGCTATTCAGGATT
TACGCTGTGAAGATTTTATCCCACATCGCAACAACCGCATCTCGGTTATACTTCTTCCATACTGCTTCAG
CACGTTTAACTCGAGCTTCGTATGTGTCAGTGAATTCTAATACAGCTTTTATGTAAAGCGCAACTACTTC
TTTTTTAGGAGTAGAGGAATCAATTTCTACACGCACAGTATCTGGGTCGTATTCAAATGTCGCATGAGGA
TAATCCTTAAAAACTAACTGGATTACAGGGACACCTTTAGAGATGGCTTCAATAGAACTATTCTCTACCG
TACCTGTGTCAGGACACGTATTAATAAGTGCTTTAGCTGTTTTAAGACGTTCCATAATTTCACTATGTGG
AGCATCAATAATAACAGGATATTTAGCTGGTGGTGCCCATTTACTAGGACAGTATCCTGCACCATTTTCG
CCTAGTGCTTTAATCGCTTCAAGAGCAACATCAGTACGACGCATTTTAGTGTCATAGCGTTGTGCACTAA
TAATCAGATCAGTCGCCGGAAGAACTTCAGGTTTCTCATTCAAACAAATAACCGGATAATCTTCGGCTTC
AAACAACGGATTTGCTCGCCAATAATCATAATCGATTTTGTCAGTCAGACCTGAAGTCATATAACAATAT
TTGCGTTCAAATTCACGGAAGGCTTTAGTAGGAGCATAACTCTTACCACCATTCTTATGAATTTGAATAG
TCTGGTCCCAAATTTGAACAATGCCACGAGATACCGGTTCAAATGGTGTTGCCATAAAATTCTTATGAAC
TTTAGAGAGACCTGCGTCTTTAATTGCCTGAACGGTTAGTTTAGTCGGTTCCATAGTAACAACAAAATCG
TATTCATGGATGCCGGCTAGTTCAACAATCTTACGAGCCATAGTATAACTCATACCTAGACTGAATTTAG
TCAGATGACCAACAGGATGATGATTAACCTTCCAATCACCAAATGTGTCATTATCAAAGCCAATATAATC
AATATCAGCACCGATGGACACAAGATATTTCAACACATTTAAATGTACTGCTTCAAGACCACCTTGTACA
CGGTCAGGGTTAAATGGGACTGCGCGAGATGGGATAAACATAACTTTCATAATTAAGCCTTATTAAAATT
AAAATGAAGCTCCCGAAGGAGCTTAGGACATCCATTCTTTACGAAGTTCTGAATCATCACCCATCAACAT
TTCAAAGAGTTCTTTCCAATTTTCAGGAAGTTTAACTACGTCGTACACAGGGTTCTGAATCATTTTACGA
TATTCTGACTTTTCAAGGGAGCCTAATCCCTTGATGTATCGAATACTATGCTTAGGCAGTGTATCTTTAG
CTTCTTCATACTCAGCTACAGTATAAAACCATTTTTGAGTTTTGCCAATTTGTGCAATAATTACAGGCGT
TTTAACAAAGCGAATACGTCCTTGTTCAAACAATTCAGGCCAATTACTAAAGAAAGCAAGCAAAGCCGGA
TAAATACTTCCAAGGCCGTCATGATCGGCATCGGTCATAATTGCAATATTACGATAGTTCGTATTCTCTG
CCTTTTCACCAAGAATAAGACCTGTGATAGCACAGATATCAAAGAGCTCTTTGTTCTTCATCATATCAGC
 
ATATGACATACCCCAGCTATTCATCACCTTACCACGTAATGGGAATCCGCCGTGTAGTTCACGGTCACGA
ACATCAATGAGATATCCAATTGCCGAATCACCTTCTGTTAAGAACAATGTCGTATCTGCATCTTTACCGC
ATTGGTTAGCTTTAATATGCTTATGAACCTTAGCTTTAGTTGCCTTTTTAGCTGCTTTAGTTTCTGCAGC
CTTTTCTGCTGCCAATTTACGAGCTAATGCTGCTTCAACAATAGGCATAATCAAGGCTTCGTTTTTAAGA
AGTGCCTGAGCAATTTTCTTAGCATCTAATTGGATATGATTACGAATATCACCATACGTTGACGTTAAAC
GTTCTTTAGTCTGAGAATCAAAACGCATGTTGCTCATATCTCTAATAAACATCAACATTGTGAGACATTC
TTTAACACGAGCTTTAGTGACTTCAATACCCTTATATTTCTTTTTAATACCAGGCAAAAGATGTTCGCAG
ATATCATCCATTACACATTCAACATGATGACCACCATTTTTAGTATGGATGTTATTCACATAAGTTAAAT
GACGGAAGCCATCTGGTGATGTTGTGAATGCTATAGAGACTTTATCATTTTCTTGAATGATGTTATCTTC
GCCAAATTGTTTAGCAAATCGTTTAAAATTACCATCAACCTTTTTACCATTAAATGTGAACTTGATATCA
GGATAAACGACTGCCAATGTTTGAAGTCGGTCTAAAGTAATATCTAAATAAATTTGAGATAAATTATGCT
CTTCAAATGAAGTGAAATCAGGAGTGAAGATTACTTTAGTTCCTTTACCTTTAGATTTCTTAGAAGACCA
TGATTTGTTTTCCATTCCGTTTGAACAGTTAACAGTGATTTCATTTTCGCCATCTGACGTGATGCCTGTA
AACAACGTAGAGAAGATATTAGTTAAACTACTCCCGACGCCGTTCATACCACCGGTCTTGCGTTCAGAGT
CATCGCCAAAGTTACCACCAGCCTTTGGAATAGTCCAGGCTGCTACAGGCCCAGGAATTTGTTCACCCGT
TTGGTCGGTAACTAAACCTTGAGGAATACCACGTCCGTTATCTTCCACCGAAACTTGATTATTTTTAATT
TGCACATCAATTTTATTTGCAAACTTAAATGATGTACGAATAGCTTCATCAACCGAGTTGTCAATAATCT
CATCGATTAATTTAACCAGACCAAGGAACATTTCAACTGGTGAATAATTACCAACCAAAAACGGCTCGTG
GGCTTCTTTAGCAGAAAGAACCAATATACATACCATACGGTTTCTTAATAATGGTTCTACATCTGACAAT
ACCCTAATTTCATTTTTAATCATGTTGTCTCCTCGTTATGAAGGTATTCTATCATCCGAAATCTAAAGCA
AAAAGGGCCGAAGCCCTTATTTAAATTTAATTTTCTGAAGTGCATCAATTGCAGCCAAACGCCCTTGTTC
ACTTTTAACTGTAATGACCACATCGCCTGATACGATCACCGAACCATCAACAAATTCTATAGAGCCTTTT
TTGAAATCGACAGCTGTATCTGGTTCCGGAACTTCTTCAAAGAATTTAAATTCTGCACCATAAAGCAGAA
TATCACCTTGCATACTAATGATTGTACGTCCAGGTCCATCGGTTTCAGCGGCAATCATTGAAACACCGTA
ACCAATCATCTCTTCTACAATAAAATCTCTTGATAATAAAACTGTATGCAGAGTAGTGTTAAGACGTTTA
CCAGTTGTATTAAGAATTCTCAAGAAATTATCTTCTTGACCTTTGATAATACGATATTTCTTACCAACTT
CAAAACCTTTAAAAGCCATAATTAACCCTTCTTAAGTAAGTCGTAAAAACCACCGTTCACATGTTTAGGA
GCAGAAACCCGACGAGTAGACAGACGATGACATTCAGGGCAAACATCGTTTTCTCGTTCAGAAATTCTTT
TAATTTTTTCATATTCATGACCACAATCTTCAGATTGGCATTTATAGTCGTATAACGGCATTATTATTCC
TTAAAATATGCTTTCAACATTCGATACAAAGACCACGCCTGGTCGTTATTTTCGATAGTAATCGTCATCA
CAGGAAATTCAGACAATTCGTCAAGTTCACCAGAATCTTGCTCTTCGGACGCTTGATATGGATTTCCAAC
TTCGTCAAAGAACTCTGCTTCATTCGTGGAGAGCCAGATAAAGTTTTCATCAAGGATATCACCGCCTGTT
GCGTATCCAACCATGCCAGTAGATGTCACAATTTTAGTAGGACGTCCAAGATGGTCAACATCTAATACTT
TAAATGGTTCCATGCCAAGACGTCGTGCGTAAATCCCATTATCAGTGTGGTCTTTAATAAACTCTTCTTG
GGCACGTTTATCTTTAAATTGGTACCATGAGTTAACGCTGAATTTATTAGCCATTAGTAAGTTTCCTTCC
AATAAGTTGTACCATTTTCAGTGATTTCACGGAAGATACCATAGATAGGCATTTCATCACCGAGTTTTTC
GTAAACATAAACCACTTCAGTGTAGCATAAAAATTCAGTCCAGTTTTTCTGTACAACAACTAAATCAGGA
TTAAACAGAACATCTTCAAACTCGTCACGATTCGATTCAGTGGTTACATTTTTAATAATTTGACGTTTCA
TAATATTCTCCTCTTGTTTTGATAGGTCTATAGTATCATAGACCATAGTTGTTGTACATCATTTTAGCAA
AATAAAATCAACTTGTTCTGCTAACGAACGTCTGAACATTAATTGACAACTTCCGAGTATGAAATCAAGT
GCTTCATAGACCGCGATGTAGTATCCTTTAGCTTTGAAGAGTTCAACATCCACCTGGTCATCAAACCATG
CATAAACCGCGTCAACACTTTCAAATCCAAAATAGAACTCTTGAGCCCAATCTTCGTTCAATTCAAATGT
GTGCTCAATTCTATAGTATCTTAAGATATTTTGTTCAAGGAGTTTGTCTTCATTTACTGTAGGACGATTA
GGGACATTATAGTTGTCGCCGTGATGGTAGAAGTTACCTGAGATTATTCCATGTTTATCAAGGAATTCCC
 
ATGTTTCTTCAGAGTTGTCAGTGCCAGACCAACCGTAAGGTGAGCGAGGTTCTTTATTCCAAGAGGTTTC
CCAAATTCTGACGGCTTTACCGTCTTCGATAGCAATTCCTGGTAATGATTCACGTTGAGTACAAAAACTA
CGTTCTACGCGGTAAACTAACATATTATTCTCCTCTGGTTGATATGGGTTATAATATCAAAACCAGAGGA
GAAGTAAACAACTTTATGCGAAAGCAGCTGGGCTATATTTTTTAATTTGGAATTCAGGAACTACCATGAA
CTGTCCAGCGAAAACGATGTTATAAAGCTTAACACCATCTTCTACCCAACGGTCGCTAATAAAACCAGCA
ATTTGCTTAGTTTTCCATGGCGTTCCATCAGTTGCCGCAGCAATAGTGAATTCATGGTCTTTTTCAACAG
AAACTGATCTGCATAGTCCCCATTTGAACGCTTTTTCTTCTGGTTCAATGAATTGGCTTTTAACGTAACC
AATGTTACCATCGGCGTCACGAACTTTAACTTCATTACGTCCAAAAACCGGATCGCTCACAGAAAGAACT
TCAACCAACTTCCCGGCAACTTTAGAGCGAGAAGCATCTGATACGTGTACATAATCACCAATAGAGAGAT
TTGCAATATTCATAATAATTCCTTAAGCTTTACGAGGAGACACATAGCGAGTGATTTCAGATTCTTTAAC
AACTCGGAAGTCACCTAAGAATACGATATTGTATAACTTTTCACCATCTTCAACCCATTGGTCGCTAATG
AAGCCACACATGGTATCAGTTGTGCTTGGATAACCGTATTCGTCGATGTAATCAAATTCTTTATCGGTAA
TAAATTCAACACCTTTGCAAAGGGCCCATTTAAACTGTACATTAGGTTCAGTAGAAGCAAAATGAGCGCT
CACAGGAGTTTCCAAAAATTTAGGACTTACGTAAGCGTACCGAGTTTCAGTTTTCCCAGAACTAGGAATG
ATTCGAACTTTAACTTCTTTAACACGACCAGTACCATTAGTACGAAGTCCTACCACTTCAACTAGACGAC
CTGCAGCTTTAGAACGAGATGCAGTTGATACACGCTTAATTTCACCAATAGTAATCATAATTTTTCTCTC
TTTGTTGTTAAGATTTATACTCCATCCGTCCTTGGATGTACAACATTAATTAAGGATATTAACCACAGCA
CTACGTGGAACTACACTAAAATCACCTGCATGAACAACGTTCAGAAGTTCAACAACCGTCTTCAATCCAC
TGATCAGTTACCCATCCACTCACTGGACCTGCGAAACGGACGTCGAATTGAAACAGCTTTACACAACAAA
TCAGTAGGCTCAATAGGCTTAATCAGAGTACCGTATATACAAGCGATATCCATATCAATACAGATGTCTT
TAACGGGTTCTACTTCTTCAATCAACAGTTCAAAGTCTGTGCTTGGAAGGGCGGTAGTAGAAATTGTACC
ATATGAATTTTTAATTTCTAAGAATAACCAAGGGCCTTCAGCTTGTTTGATGAAGTTAACAATAGTGAAA
CTATTACCTTGAGTGGCGCCTAAAATTTCACAAAACTTTTCATAAGCTCGCACTGAAGCACCGTACCAGT
TAATTTGTTTTTCGTCTTTCAGGATGTAAGTAGCCGTAGTATTGATATTCATAATGTTTTCCTGTTTGGT
ATAAGATAGAAAGATTATACTCCAACCATCCTTGGTTGTAAACTGTTTTATCGCTGTTTTACACTTAACG
GGTCAGGATTCTCAGGGACATCTTCAAGGCCAAGCGCATAACGCTGAGCATATTTTAACATCAAAATATC
TTTAGCACAATCATGAATACTATCATGGGCAACGAATCCTTTTAGCGTACCCATCGGTAATGGACACATA
CTTAATCCTCGTGTCAACGAGTAAGCTTCGATTGCTGTACGAATATCGCGTTGAGCCCAGAATTTAACCG
GTTCTAATTTATCGGTATCAATCTCTGGTTCAAGAACACCTTCATCACGATAGAGGTCACGAATCAAATC
GACCAAGATCGGGAAGTCAAAGCTCATACCACGGCACCACATCTGTGATTTCCATTGGTCAACTTTATTA
GCTCGACAATAATCTAAAAAGATTTTAATGCCTTCAAGCGTAGTGACATCGTCTTCAGACGGGGCCAAGT
TTTTACGTGCTTCAGCCGATTGTTCTTTCCACCATTTCATAGTGGATTTAGCGAAAACTCGCTTCCCTTT
CTGAGAAGCTAAATTAAATTTAATTCGCTTACCTCGTTGAACAAGTTCTTCAAATGATTCAACAACTTCA
GGGTCGGAATTATATGCAATAACCGCAAGGTCAATAACAGCCGCTTTTGATGTATTGCCGAATGTTTCAA
AATCTATAATAACATCAGTGCTCATAATTTTCTCTCAAATAAAATGGACGTCAAAACGACCACGAGTTGT
GCCGACATACAATAACTGTTTAGCTAATTCTGAATCAGCATAATGGATACACGGTGTATAAACAAAGGCC
GTGTCAACAGACATACCCTGGGCCTTATGGAAGGTTGATACTGGTAGTGCCTTCACCTTAGTGAACATAC
GTTTAGCATCCCAGAATTCGCTCCATGGAGCTTTACCACCTTTATTCCAGAATTTGTATGTTTCTGCGGT
CTTAGCCAAAAACAAATTGAATTTATAAAGCTCTTCTTCGGAAGAAATAACCTTTATATCCTCGCGGATG
TATTCTTCATCATCCCCATATGTTTCTACTGTAAGATTCCAATATCGTATCATATACTCGCCCGAAACAC
CACGTGCTTTAACGAATGTAGACGTTTCTACAGCATCTCGAATACGAACGAATTGCCCGTTATTGAAAAG
CATTTCGGTCATATTTTTACCATCTAGCTTGTACGTCTTAGTAAATGGCTCTTGTAGAACAATGACTTCG
TCTTTAATAAATGGTTTTTCTGTTTCAAGAAGTTTTCGGCGAATAATTGAATTTAACTTGTCCACTGACT
TATTTGTGAATGCCATCATCCGATTTTCGAAAAGGTCTTCAGGAGTTTTAACGACATTAAAATAATTCAT
 
CATATATGATTTAAGGTCGGTATATCCATGAACACCATGCCCGTCAACTACTTTATCATAAATCCATTTG
CCATTACGAATATCGGTCGCAACATCAATAATTGGTGCATTGCTACGTTTAACTTCTGTAAGGTCGCATT
GCACGAAGTCTTTGTGAGTGAAGAAAGGACTTAGATATGCAGTGGATTCGCCTGGGTCTACCGGTCGAAT
CTGACATCGGTCGCCAATTCCAATTACTGTGCACCATTTAGGGATACTAGCTAACATTATTTTAAACAGA
GAACGGTCGCACATTGACGCTTCATCAAATAACAGTACACGACACAAGGCTAAGTCGGGAACCTTTTTCT
GTTCAAACAATACGTTTTCTTCGTATGTAGTTGGGTTGATTTTAAGAATGCTATGAATAGTCGATGCTTC
TTGACCAGCAAGTTTGCTTAGAACCTTTTTAGCGGCATGTGTTGGTGCTGCTAAAATAATACCTGATTCT
CCAGAAGAAATAAGAGCTTCAACGATAAATCTAGTAAGGGTTGTTTTACCTGTACCGGCAGGCCCATTAA
TAGTTACGTGATGTTTCTTTTCTTTAATAGCTTTCATAACAACGTTGAAGGCGTTTTTCTGGCCTTCAGT
CAAATCATCAAATGTCATCGTAAAGTCCCTGCAATTGGAATACTAACAATACGGCCTGTGTCCAGGATTC
GTTGATACAATCTTTGCGTCTCCGCATCTAGTTTAACGTGTTTCATTTCAAGCTTATTAAACCAGAACTT
ACGCGGAGTTTCGACCATTCTTAGAACTCCCATTTTTAAATCTGTTTTATACTGTGTTTTCAAAGTATCG
AAAACTTTATCTGCAATATCCTTTCTAAAAAATACTGCAGGACGATGCTCATCAAGAGGAACCGGGACAG
TAAATCCTTCCATATCTCGATAAACTATTGCATATACATAAACCATAATTAACCTTTAATCAGTTGGAAT
AATGATGCGCTAAGTGGTAAGCCACGTTTCAATTTTAATTTACCAATGAAACCAATCTTTTCAAAACGTT
GCAGACCTTGGATAACTTTATCATATGTATCGAAAAGCGATTCAGCGTCAGTTAATTTAGTTTTAGTTTT
CCAGTCAGAGCCTTTAAGAAGAACGTTGAGTTTAGTAAGTTCATGGCCTTCTGCTTGATGTGAGCCATTA
ATGCGTGCTTTCAAAGCAACAATTCTAAGTTTAGATTCGTTAGATAAAGGTTGATTTTTCATCATGTTTG
CCATATGCTTAACACGGTTTTCACGTTTTGCTCGGATTTTTAAAATAGTACCATAAACCTTTTTAATTTG
GGCCCATTTCTTCTCGTCGAGATTAAGTTTCTCTACTTTACGAGCAGAAGGATTAGCGATACGTCTTGCA
ATAGCATCACGTTTCTCAATAAGTTCTTCTAATGTTAAATCAGAACGAGCCGTGTTATATTTTTTCTTAA
CTGGGATAACATTACCTTTAATGTAGCCTATATCATTATTAAAACGTTCTAAGGATAATTTCTCACCTTC
AACAGAGTTATTGAAAGATTCACCTGAGTAAGCGCAGACCTTTTGATCAAGAATGTTTTTAAGGTAATTA
AAGTCTAAGTTAAAATCCTTAGAGCGTCTCTTCGCAGAAGCTCTGGTGTGGTCAAGACGACGATTGATTT
TGTTAATTTTATTATTTGATAACATAATATTTTCCTCTGTCTTACGGACGTTAAGGTCATTTAGCTGATA
AGTCTATAATATCATGCCCTCAGGTAGTGTACACTCTTTTTTCATAAAATGCTTTAATTTTTTATGTGTT
ATAATGGTTCTGAGTTCCATCCGGTTATTCCAATTCAGTCAGTAACTGACAATAGACAGATTCCAGATTG
TATAATGTCCATCTTGAATAATAGAATCTACACGCAGTGTAGTCCCTATAGGGAGCATAAATTCACATTC
GTCAGATATAAGTCTAGCGTTAGACCGTCTTGTCGCTTCAGGGAAAGCTCCATTGAACTCACAAGATGGC
GCAGCCAGAATCATGTTAAGCATATGTTCACGGAAGTTGAAAGCAAATGGTGCATCTTTGATGCAGAACA
TATTAAATGTTCCGTAAAAATTATACGAAGCAAAGTTACGTGCTACACCGAACACTGGACTGAAGCTCAT
TACTCTGTCAAATTTAATAATACTACCAATGCCTTTATCATCAAGTGTGGCCATAGTTTTCTTAGAGATT
CCACGATACATTGTCTCAGGAACAGAAGACGACCAATTTCTACGAACAATTGCACTTAAATCTTGTTGGA
TGCTATCAGGCTTGTTTTCCATACACTGCCATAGTACACTTTGTTCAATGTCACTGAATTGCTCATCGAT
TTTGTACTGAAGCATATCTTGATAATCCGGGCATTGAGCACGACGAACCATTTCTTCAATTTCACACTCG
TTATACGGCATAAACATTTAGGTTTCTCCTATGGTTGATAAAGGTATAGTATCATCATCGGAAGAGTTTG
TACACTAAAAATTCAACATTTTCAAATGAATGATTTTGTATAATCTAGATAAAGGGTCATATTCTAAGTC
TTCAATTTCCACAATCTCAAATCTAGAGATACCTGAGACCATGTACTCTTGTTCAGATTCAATCATATCT
AGGACATCAACACGTGTTTCTTCCATAACATGCTTAGGGTTCTTTCCGGCAAGTACAATGTTAATCATGT
TTTGGTAGTAATCAAAAATGAATGGAGCATTCCTGAGACTAAGAATAGTGTACGTCTGATATTCCCATCT
CCCGGAGAATTGACGAGCTGTACTGAAATCAGTTGTGAACGATGTTACTCGTCCAGGAGACCAATGGCAT
CCAACAGAAAGAAGTGAAAGACGTTCTACTTCTTCTGGAGTTACACCACGGTAGAGTTCTACTGGTACTG
TAGAAGTCATATGCTTACGAACAAGATGGTCTAATTCATAGTGGAAATCCGGGTCTTCCTTAGAATCGGT
GCAAGCCCAGAGAATGTTTTGTTCTTGGCGGGTGAATTTGCTATCAATCAGATTTGTTAGGTATTCGCGT
 
TCTTGAAGTTCAGATTGACATGTCATGATAGACCTCCATTTAAAGATAAGCCTATCATAACATGAGAGGA
GGGAATGTAAACTACTTTTTAAGCTTTCCTACTAAAGGTCCATCAGGACCGGGTTGTTCTAACATGAATA
AGGACGCTTTAAAAAGCAATGTCAACCCTTTCACCATTCGATACCATGTCTCAAAGTCTTTTTCCTTGAT
TTCCAGTAAGAATTTCTCTTTCAATTTCTCTTTCATTTCCAAGGACTCCATGGTGGAGGGTCGTCATAAT
AAGAGGCAAGGTCTTTCAGGGCTTCGACAGGCAAACTAAAGCCGTGTCGTTGTATAATACTATCTAGTTC
ATCAACAAGTTCACTACGATGCACTTTAGGAGCCTCAACTTCAGGCTCGTCAAACATACTCATAAGGTCC
ATATTATTCACCTAAATAAAAACTAGGCTGCTCGGAGAATGTTTCTAGCGCTACACCTTTTGTCTTAAAA
AATTTCCAAATAGCTCGTTGCAAATGGATTAAATCTGCTAATGATGTAATAAAGTATTTTTTGGCAAAAT
ACTTATGGTCTCGCTTTTCAAACAAATCTAAACGCTTTTCTGGATTCACATAAACGGCATTTTCCGTAGA
TTCATAACCTAAGTCCTTTATGCCTTCTTCAAGACTAAACGGAGAATTTTTAGAATGAGTAATAGCTCCA
ACTCGCTCTACACGAATTTTTGTAACCTTTAGGAATCTGCAATTTGCAGAATTCTTCTACGATATATTGC
GGATTATTTAGACTTTTGTTAATGAGAATAGCTATTTCTAGTGGAGTCTTATCTTCCACTGGTATATGTT
TAAATGACTTGGCTTCAAGCAAATACATTTTAAATAACATAGCATCCATTATAATTTCCTCGATGGTTCA
CCCATATAAAGTTTTACAATACGTTCGCCTTCATCTTCGACGAGCTTTTCTTCATGTTTCTGGAGTTCTT
TGACCATTTTCTCTTCAAAAACATCTAACCAATCTTCTGGTCCGACTCCATCATTCAAGCTTTTAAACCA
TTTTACTACTCTGTTCATTCACAGCTCCCTGAGCTTGAACCACAATCAGAATAACTTGACCCAGAATCAC
CGGACCAAGACGATACGTAAGAATGATTATAACTTGAAGAGCTTGAAGCATTCGCCTGGTTTGTTTTTAG
ACGAACAACTTCTTCCATCATTCCATCACCAATATATTCCCAACTTTTTTGATGTGTATCAGGAATACGA
ATCATCCCTTCGCCGGGTTTAACAACAAACAACTTGTTGAGCATCGGAAGCATTAATTGTTGCGTCGAAC
AAAACGAACACCACCATCATCCCCAACGGATTTGGGCATTAAATTTGACGTCTTCACCATCCATCATGAA
TGAGTGTACGAGTACGTCATCATAATATCCAAGACCGTATTTCAACGAAACTTCTTGAGCAATCAAAGCA
CCTAATGTGTTATTAGGTTTAATCCAACGATTTAACATATTGTTCTCCTCTTGAGTAAGTAAGAATATCT
TAACATAGTTTTTAGTGGTTGTAAACTCTAGAAACGAAAAAAGGAATCCCGAAGGATTCCTTAAATTATT
TAACCACTTACTGGTGTAGGGGTAAACATTGCAGCGTTCTTAGTACGCCAATCTGCTGCGTCAGTCACCA
CAGTAGTGTAAGCAGCTTTTTCAGATGGGAAAGTCTGATAATGAGCATCAGCAATCCGATGTTCGTTTGA
ATAAATCTCAAACGGTACAGAAACTTCCATACCTTTAACTTCTTTACCTTCACCAGCAGGATGCGTAAAG
GTTTTGATGTTTACATAACCACCCATAATTAACTCCTTTGTTGTTTAATTACAGGTGTATTTATATCAAT
AATCGTCGTAGAAATCACTCAAAACATCGTATAGCTCTTTCTCATTATTGCAGATTAGCGACTTAATTGC
GTCAGGGTGAGACTCCAGTGCACAAAGCTATATTGGCCGCGTGATGAGAATGTTTCTCAGATTCATATCA
TCATTAGGGATGGCAATAACCAATCGGGTCTTTCTCGTAAAGGTCGTTCATTAGTAACCTCGGTCTTGGC
GAGCGAAGTTTTCAGCATTCTTCAGGTAATACAGTTTGAAGATTTCTTCAGCAGTGAGTCCAAGACCCTG
GAACATATTCAGAACAAAGTGCAGAATATCAATCATTTCGAATTTAATTTCGAGCTGGTCTTCAGGAGAC
AGTTCATCGATACGACGATTTCGATATTCAGCATGTTGTGCCTTCCACGGCTTCCACACAGAAGAGGCAG
CTTTCTCGCCGTTGCTCATACCGCCAAGTGAAGTCAGCAGTTCACGAAATTCATCATCAATATAATCTTT
CTGATTACGCAACCAATCAACAACATCGCCAGCAGTAGCCAATTCATCAGGATGTTTGTTATGTTCAGGC
TTGTCATTTGCTAAGCGAACCTGTAAAGATTTCTGCATATCAAGCATTACCTGCAGTGGGTCTTTATCTT
CGTGAATCAGAGCATTAAAATAAGCTTCTTCTGCTTTATCAACACCAGAAATAAGTTGTGAACATTCGTT
AAAGTGTGCCATTATTTTTCCTTTCAATTCAGTATAAGAGAGTTAATTCTAACATTAATTTTATAAAGCG
TAATAAATAATAGGTAACTAAGGAGATATTATGTTTAAACTTCCATCGTTAATCAACGCTATTAAAGGCA
ATAAAAAGAAAACTGCTATCGCTGCAATAGTTGTAGGTGGTATTATTTCATGGAACTTCGGGATTGCACC
ATTGTTAGTTGCCCATGGTATTATAGTTCCTTCTGTTCCATTGGATACTGTTGTTGACCTAGCTTTTGCT
GTTATAGGGCTTGTTTAAATCTTCGAATAACGAGATAATCTCATTTTAGCATGCAACCCACTTGAAATAT
TCTGTTTCATATATTCTAAAATTTCTTGTGGTGTTGCATTTTCTTTCATTACCATATCATTCACATCCTT
TGAACTCCATGGAGCTCGGTCCCAGAACATGACACGTTCGCCTGCATCAACTAATCGCTTCATGCGAGCT
 
ATAGTATCCGGATGCCTAGGTTCATTATCCATTACCCATACCCGACGTTCTTTAAATGGTACCACATCTA
AATCTATCGAGCCGCCTGTAATAGCGATTGCATTAGGTATGAATAAACTATCAATAGGGCCTTCCATTAC
CCAGACGTCTCCTTCCTTAACACGTTCAACACCATAAATTTTTGTTGCAGACTCAAACGCTTTAATAGTG
ATATATTTCTGAGGAGCATCTTTACGAAGTGCACGTCCTTGGAATGACTCGGCCTTCCCGTCTTTATTAA
AAATAGGAATCACTAAACGTGGTTCGGGAATCTCTTTCTTATACGTACCAGGTTGAATCTTGTTGACCAA
TTTGGGCCATTCTAATGTGAACCATAATCTGTTCCATGATTCTTTAGGAATACACCGAGACTTAACATAT
TTCACAATAGGATGCTCTTCAGGCAACTTGTCCAGACGAGAACAAGACGGCAAAGAATTTATAGTCTTTT
TCTCTGGCTCTGGCTTAGCTTCAGGAACCTTTTCAATCTTTGGTTCCATTCTAGCTTTTTCTTTACGAAC
TTCCATTAGATATTCGCGATAAAGCTCTTCATCATACTCCTTTAAGTATCCGGAAATTGTATTGTGGTAT
TGACAGTTATAGCAATGTACATTACCAGGAGTACCGCCATAATACCAACCGCGAGCTTTCATTGCATCTG
TCTGGGAGTCGCCACAAACTGGACAACGAAACGTTAATTTAAATGTGCTTGAATTATTGATTTGTCTAAA
ACGAGGGAGGTGAGAAAACGCACGATAAGCAAATTCATTATCTATCCATTCAGCCATAGTATTCTCCAGA
GGCCTTATTAATTTAAGGCCATCATATCACAACTTATTAATATTTACTTTAATTCGCTTTCTTTTCTTGG
AAGGAATTTGTTCTGGACCTTTATTTACGACTGCACCTGTAGTTGTTCCTGATGCAATAGCTTGAGGACT
GCCGCCTGAATCACCAGCAACCATATCCTCAAATAAAGGGAGTTCTTTAAATATTTCTTTCTGTTGTTCT
TCTGTAATGTTATAGCGTGAAGCTACAGCACTCCAGGCGGACATCATTGAGGCCATTCCATTAAGACCTG
GAACAGTGCTCATCATTCGCTTCATTGAACGAACTGAAGCATGAAAAGGTGTATAAGCCGCTTCTCTTCA
GGAGTGGAAGGACGTTTAAGAACACTTCCACGTTCATCGATGATTTTGGCTTCGTATGCTTTCCATTCGG
TAAAAGGTTTTTGCATTAGTCGAATAAACTTATATGCATAAACAGAATCTATTGCAGTGTTAATAATACC
CATAGGTACCTCCTGAGTATATTTAATCAATTACAAAGATTGCTTTGTCACCATTTATATCTGTGACTTC
ATATGTGGTGCCTTCATACTGTGGAATATTCCAAATTACACCGTCATTAGCTTTTACGCTAATATGGCAT
TGGTGTTTTGAAACAACTTCATAATCTTCAAAACTTGTAAAAACACTAATTTTAGAACCAGTGCAAATTG
CAGTAGTCATTTTAACCTCGATTCATAAATGCATTAAATATTTGGTCATCAACAGAAATTTCTAATTCGA
CTTCAATTGCTTCAGCCTTTTCTTCAGGTGGTAATTCGTATCCACACACTACAATTTTTTCATCGATATC
AAAATATACAGAAGCAATACGATTAATGTTATTTTCAGTTAAGTCCAAATCAACATCAACATCTTTTTGG
CTTACACTTAGAGGATAACTAATACGGGTTATTTTATTATCTTTAACAAAGAGTCCACCAACATACTCTG
TACTACGTTTGAAGTTTACACGTTTCCGAATTGAAAGATATGATGCATCTTTACCGTGAGCTCGGCTCAT
AGGACATAATGAATAGCTAGAATAAGAGATGTCAAATCCTACACCTTCAATATGAGCCAACGTGTCATGA
TTAAACCAATTATAATCATATGCCAAATCCATTATATTGTAATATGTGAAAGGCACAGGATTAACATCAT
ATGTTGTACCTTTAAAATCTGCCCAGGCAACGATTTCGTTATCTTTAACAATTACAAAAATTACACCGAT
ATGGTATCCGTTATTATTTTCATTCGGCGTTATATTATAACATTGTGCATTAGGAAATTTACCATATTTA
AATGGCGCGTTTTTGTGCATGTAATAAATCATATTATTCACCTGTGATTTCGGTTATGATATCTTTGTTA
TCGAAATTAGGGTCACAATACAAAACATAGTTGTACTGCATGATACCGCCAGACTTAAGTTGTTCAACAA
CTTTATTAACCATATTAGGACGGTCTCGTTTAGTGATGTTCATGATATCAGAGAAGATTTGTTGTTCAAC
ATCTTCTTGATTTGCAGTCATTGACCACTCACAGAGGTCTTTATCATAACCTGCCGTAACTGGAGCCGGT
GCACATGATACTGCAAGTAAGATTGCTGCCAATGATAACTTTTTCATTTTAACTCCTTAGTTGATTTGAT
AGGACTACTATACTATAGTCCATCTATAATGTAAACAAGTTTATTCTTTTTCGCGAATAATAATTACTTG
TTGAGGCTGCTGAGCCGATTTCTTTTCGGCAACGATAGACCAAATTAAAGTTCCAATCCAACCAACCATC
GTCCAGTTAAAGAGTAAAGATACAAAGAAGATTGCTGTTGTGCTTTTTGTGCCACGAATAAGTGCGATTA
CCCAAGGCAACATGTAAATGATGATTGAACCAATACCTGCGATTAAAACTGCTACTGAACTAGCTACGAA
TAATTCCATGTTAATTTCCTCTTGATTTGTTATGTTTATAGTATCTTGTTTTAACTTTGGTGTAAACCAA
TTAATTAAATTTGTTTAAACGTTCAATACACTGGACGTAGTTATTCGCGATTTGTTTCTGGGTAGGCTCT
TTACCAGTTGTTTCAACATAGCGAATGAACGCGATTTCAGTAGCCTGTAAGCAATGTTCTTTAACGCGTT
GTTCTTTTGACAATGCTACAGTATCAGAATCACGATAACGTTGAGCATGGGCATCAGCTTGAATCATTCG
 
ATTACCTACAGTAACCGGGTCTTCCACTTCAACTTTAGGTTGGTTGTACTGTTTAACATTCGGATTGTAA
ATCAGTTGGCCAACGTTATTAGGGATAAAGCCATGGGCACCACATCCAGTTAATCCGAATACCATTGCTA
AAGCGATTAATTTTTTCATTTCAATTTCCTCTGTAGTTGATAGAAAGATAGTAACACAGTATGTGGTGCT
TGTAAACAGCTATTTTAAACTTTTGCGAAATTTGCGTTTTTCTTGACGAATCTGTCGTTTAGTCGGACGT
ACAGCAAATTTAACGAAGTCCACTTTGCCGTGTGGAAACTTTTCAGATGCTTCACAGTCCATCATTAGTT
CCCATCCTTCCAGAATAAGAGCTTTCCATTGTGTAGTTGATGCAAATTCGTATGTTGATGGTTTAAACAC
AGTTGAATACATATTATCCTCTGAAATCAGCTTCAAAAACTTTCTCGTCATTAATAAATGCTTTAACTAA
TGCATTGTTCACTTCAGCAAGTTGTACCGTAACCCACTCGATTTCTTCGCGATCACCTGATAACATGATA
TCTTTAGTGAACATCTCACCGCTTTTAAACCAACCGATAAAAGTAACTTTAGTTAATTTGCTCATATTTC
CTCGCAAAATGCCCATTCAGCGTGATAAACTGTAGCACCTTTAGTATTATTCATTTGAACTACGTCTACC
GGAGAAATTACTCGATAGAGACGTGGTTCACCTCCATAAGAGCGTGCTGCTCGTCCTGCATAAATCTTTG
CTAAACCGATATCTTCAGTAAAGAATACACGATCCAAGTTTTTCTTACGTCCAGTTTCAGACAGAACTCC
TGTTTCCTCAGGAGGACAAAGCATGTTTTCGATGTTAGCTGAACTACTTGAACCGTGATAAAAAATCTTA
AATTCTGCTTTACAATCGATGGTTTTCATTTTGTTCTCCAGTAGGTGATAGGTCTATAATACACAACTAC
TGGAGAAGTAAACTTAAAAACGTAATTTAGATATATCTGCGTTGATTTCGTTTTGGTTTGCGTCGTATGC
AATTAAACGAACCGCTTTAGATGGGTCATTCAATGCATAGAAATGATGGAACATTGCTCGAACGATATCA
CCATTTACGAGTTCTACCTGGATAGGAGAACCTTTTTCGAGTTGGAAATGTTCATAAGCTTTGATGGTTC
GCAGATAATCAAGTTTTTGATTTCCGGCTCCAACAATTTGTACGATACGTTTGCCCATGTTTTCCATACG
AGATACAATAGTTTTAGGAGCAAGAATAACGTAGGTTGCCAGCGGAGAAACTACTTCAACAGTTGTGTTT
TCGGTGATTGCAGTAAAAATTGCTTGAGACATATTCATAATGTTTTCCTCTTGGTTGGTGTAAGTACATC
ATATCATGTCTAGAGGAAGATGTAAACCCCGAAAGGAGACCGAAGTCTCCTTTTTATTAAAATTTTAACT
CATTGGCTAAGCTATCTAGGTCAGCACGTGTTGCTTTGGCTTTACCTACGCGTTCCGTACGATTCACCTC
TGCCTGACGCATCTGAGCACCTGCGTTTTCATTTACTGTGTTAGGTGTGTTCATGCCTTCTTGCTCAATC
TCAACCCATTTCTGATTGCCTTTACGTACACCCATCAGGAATTTATTCCACTTATTCTTATCGCCGTATC
GTGATTTGATTTGCTTGATAAGTTGTTGTTCAGCTTGTGCAAGTTCTTCGGTTTCAATTACAGCCAACAT
GAAATCTGCCGTTGCTGGTAAACCTGCTGATTCTGCGATATCACTCATGTTCATGTCTGAAGCATCCCAT
GCTGCACGTCCTACTTGAGCTGCAGTCCATAGAACTGTTTCAGATTCTACTGCTAATGCACGAAGTTCTT
CTGCGATAGCTTTAACTAATGTGTAGCTATTTTCAGTGTAAACTCTGATACGACAAGACCCACAAATACC
TAAGTAATCAACCATGATTACAGATGGCACAAAGTTCTTCTTAAGTTTTAACTCATTAAGCAATGCACGG
AATGTATTAGCATTAGCTCCACCAGTAGGATACTGTTTAACTACTAAACGTCCTAATGTTGTTTTTGAAC
GCCATTTTTCCATCTTGGCTTTATATTCAGCATAAGATACGTTACCATCATCAATATCATCAAGTGATAC
GTCAAGCATGTTTGCATCTATACGCTTAGCGCAAACTTCTTCAGCCATTTCCATAGAAATATAAAGAACG
TTATGACCTGTTTGAAGATAATCTGCAGCAAGTGAACAAAGGCCTAAAGATTTACCGACGTTAACACCAG
CCATTAAAACGTTTAGTGTACCGGTTTCAGCACCACCTTTGGTTATTTTATTCAAAATGTTTAATTTGAA
CGGGACCTTACGTGCTTTATTTAGATAAGAAAGCCAGCGTGCTTCGTAGTCTTCCATCCAGTCATGTCCA
ACATAACTATCGAACGAAATTGAAAGTGCTTGACGCATAATATCAGGAATAGCTCCTACGTCAGGCATTT
TCTTGTTACGTTGTTCTGGAGGTAGTTCAGCATTAGACTGAATTTCAATGATTTTGGACGTAGCATTATA
CATCGCTTTTTGCTGGACATATTTCTCAGTTTCTTTAACCAACCATTCGTGGTCTTCAGGAGTATCAGCC
AATTTTTCAATTAGGTCTGAAGTTCCTTTATATTCGGCTTCAGTTAAAGAACTGTTATCTAAAGCCACCT
TTAATGCATTGATAGATGGCATTGCATTATATTCGTTAACGTGGCTCTTTATAATTTTAAAGACGTTTTT
TGCCGGTCCACGCTCAAAGTAATCAGAATCCATATAAGGCCAGACCTTAGAAAAATAAGCCTGGTCATAT
ACTAGATGAGACAAAATAATTTCTACCACGATAACTCCTTAAAAGAATTTAAATTTTTTCTTGGCCCGTT
CATTAAAAGCTTGTTGTACTTGCATTGTAACACATTTTTCAACATGTGGTGCAAGTTCTGCCTTTTCTGT
CCTGGTCAAGAACTGCAAAGTCCATTTCGATTTTACCATCAACCCAGTGCTAGTTTTGTAATATAGACTT
 
ATATGATGGGAACCGTCTTCAAGTGTAATCAGAATCTCTTGAATGACATTTTCCATTGCCGAACGGATAA
TTTTAAGAGATTCATTAAACACTCGTTCTTTGCGAATGTCTTCCCCTTCCGAAGAAGGGGATTCATCAAC
GATTTCTAGATTTAAATCATCGAGATTATTCATCGAAATCTTCCATTTCGTCAAGGTCGTTTTCTATATC
TGCTGCAGACGGAGCATCAGAAGTTTTAACCGGAACTTTAGTTGTTGCTTTGCAATTAATAAGGTCGTTA
ACAGCGTCATCAACTTCTTTAATAGAAGAGATAGCACCTAGTTTATATTTAGTTTCAATTGCATCTCGGA
AAGGTTTGTGTTTAAACAACGGTCCCCAGAATTCTACACAATCAGTAGCTTTAGCACGCCACGATTTCTC
TTCACGAATCATTTCACCTGTTTCTTCGTCAAGGAATTCACGAGCATACCACCCGGCTTTAGGCTTAACC
ACAAAACCGATTTCAGTTGCCATTTCTAACAAGCCACTGAATGGGTCGATACCACCATCAAAATTAACCG
TGATTGGGAAAGTAGATTTTTCTTTAACGGTGCGAGATTTTTCTGCTTTCAACGTGAAGTCATAACCGGT
CAATTCTGTACCTTCTTTAACCTGACGTTTAGAGATAAAAAATACCGTGTTGGCAGAATAAAGAATACCT
GTACCACCACCCATAATCTCTTTAGGATACAATCCGCCAATTTCCATTGCTGTATGGTTGATTGCAACAC
ATGGAATATCTTTAATAGTCAGATAAGGAGTCACAATGCGGAACAGAGATTTAAGTGCCTTAGCACGAGA
CATATCACCTACAACTTTCTCGTTCAATGCATCTTCAGTTTCTTTCTTAGACGCAGTATTACCAATCGAG
TCAATAAAGATAATAACTTTATCACCGCGTTCAATAGCGTCAAGCTGATTAGTCATATCAACTTTAAGTT
GTTCGACAGATTGAATCGGAGTATGAACTACACGGTCTAAATCAACACCCATTGAACGAAAATAAGATTC
TGAAGCACCGAATTCTGAGTCATAAAACAAACAGATTGCATCTTTATATTTCTTCATATACGCTGCAACC
ATAGTTAGTCCAAACAACGTTTTAAAGTGTTTAGAAGGAGCAGCAAAAATAGTTAAGCCTGATTGCAACC
CTGCATTCAGTGCACCACCTAATGCAATATTCAACATCGGAATACGAGTAGGGACTTCATCGCGATTATT
AAACAGCTTAGACTTAGTCAAGTCTGCAGTCATTTTAGAAGTAGAAGCTTTAATCAGACGAGATTTTAAA
TCAGACATTGTATTTTTCCATAGGCATCATTATATTTTACTCATGTTTAAAAGATAAGATGATTATATAC
TAAGGGTTATACAGCATTTAAAACTTTAAATGGCTCTTAACGAGCCCATTTGATGATTTCTGTAATCAAA
TCATTACGTTCGCTTGCATTAAATCCTATAACTTTGTTAGCACCATCATGATTAAGTAATTGATACGTTT
GGATTTCGTTATGAATTGCAAACTCTTCAAAATGTGAAATGACTGTATTAAAACCGAATGAAGACAAAGT
AATAAGGTCGTCTTCAAAACTTTCAGTCATTTCGTTACGTGTTAAAATAATTGTGAACACATCATGGTTT
TGGCAAAGACGAACATAATCCTGTTCATAAACGTCAAGAACCGAGTCCGCATCTTGTCGAATACGTCCAT
AAACCCAGTTACTAATATATCCACGATCTAACAGATAAATCTTAGTAGGGTCTAAATAATTCAACATCGT
TTCGAAACAGCCAACTTCATTTCGAGACTTAACATCAAAACGTCCATCAACTGTTCGTTTAGGGAAATCA
ATCTTTACATATCTTTCACTAATTTCCATTATGTCATTAATGAAAGTTGTTTTACCAGCATTATCCGGGC
CATCTACTAAAATAATTTTAGCCATTTTATTCCTCGATTACGACATCTTCATATTTAGGTATATTATACG
GCGAAGAAATTAAAGCGTTAAAGTAGATGAACTCACCAAGACCTGGATGGAATACTTCTTCAGGATTTTC
GAAATATTGAATCTTCGGACGTCCTTTAAGAATTTGATAGTACTCAGTTTTAGTTGGTTTAAAAATTCGA
ATCAGCGCTTCTTCAGGATACTTATTTCTGTCGAAGCTATTATTTGGATCGGTTATATAAAGCGGTTGGC
CTAATTGAATTGCACATTCAACCACTTGTTCAAAACGATAGCACGGGTCACTAATTCGAAATGGATGAAA
AATACCATCTAGATGAATAGGAGCCAAATCACACTACAAGACGCTGCATGATGGAAGGACGTATAACTTT
CTGCGTTACGATAATCTTATTACCATCTGCACCTGCGTTAACCAGCACTTCTTTTTGACGTTCATTCAAC
ACAGTAGTTTGAAGTGAACGATTAACCGACAACATATCGGTTTTTAAGAATTCGTCAATATAAAAACGTG
GTTTATTAGGGTCTGCAGTGATATTGAAAATGAATTCCACATCAAAAGCGCCGTCATATCCAGTGATATC
TGTGATTAGAAAATCACAATCATAATAAGCAATTAATGAATCAACCACAAATTCATTTTCAGACCAGAAA
TATTTACGAGTGTTATACGCGTTATCTTTATACCATAATGGAACAAACTTAAATTCCGGGAAAAGACGTT
CGCATTCTTTAATATCAGATGCATTACGTGGATATGTAATCACATCACCTGGATTAGCACGGTGCAAATG
AAGTTGGAAGTTGCCATCCTTAAGGATAGAATACTGTCCAGTTTCATAAGACCGCATACTCAAGATAGGT
ACGCGGAAAATAGCCATTAGTCAAGGAATCCTTTAACATAATCAAAATCACGTTCAAACACATGGGCACT
TACCATGGTATGAGTATAATCACCAACTTCTACACCACATTCGTTAGCGATATAATGAAGTAATTTTCCT
TGGAGATAAAAATCCAACTGCATAACAACAGCACAGTTCTGGGAACGCATATGGCAGTGGGTATAAAGCT
 
TACCATCACGAATATAATAAGTCACTGAGTCAGTACATGGATATTCAAGCGTTTCGTCCGAATCAAGCAA
AGCCTGATCAGAACTTTCTAGAATTTGGAACACAACTCTTCGGGAGTTAGGCTTCTCTTTAAGTTCTTTT
AGAAGAGCTGGTAATTGGGCCGCAATGCGAGGACCGTAGAAAGTGTTGAAGTTTGCAGGCAAAGCGTCTG
ATTTAGGTTTAGAAATAAATTTAGCAACGTTAGGATATGCCTTAAACGCTTCTTCTGCATCAGTACCACC
AGAAATCATAAACTTCCAGAAGTCTTCGGCATATTCATAAGAGATGCGGTTAATACGTGGGTCGGTCATT
TTGAATGATGATGGAGTGTCAACAACTACTGTCATAGACCCAATTTCATTACAACGTCCAATACGAGAAT
CAGTTACGAATTCCGGGTTTTCTAAAATTTCACGATTGACTGCTTTAAATGCGGTTTGAAAATCTGCGGC
TTTGATATATTTCATTGTGTTATGTTTTCCCATGTTTACCTCAATACAATTTATTATATACTCAATTCAT
AAAGCTTATTTGAATTTCTTAGCGATGTTGATATAACGTAAAGCTTCAGCATCGATATTTCCAAGAACAC
TATGAAGAAATGCATCAGCGTAGTATTCCAATTGAACAGGTTCTTTAATCACTTTAGCCGCTTTAGTTTC
AAACAGGCCCATCAGGGCCTTTTTAAGAATTTGTTCGTTGCTGTTAATTTTCATAGTCAAAATCTCTTCA
TTAAGTTGGTGGTTAAAAGTTTTTCTAGAAATCATTCTGGGTACCTCAAAGCGTCATCAGAGTTAGTTAC
ATCAATAATAGATATAGAATTTTCTGATAACCCATTTTTAGATAACTGACGTTTATAATATCTTTCACAA
TATGTACACGTCGTCCCGCAGATTTCATTAGCACAATCGAAATCTGGATGTCTGAACCACCTTTTTTCTA
AAAAGCCATCTAACAAAGAGGTTTCAATATTTACTGTATGTGAAAACTCAGTTTCGTTTTCAAGAGTGAA
AATAGTCTCTAAAGGCTTCCACAGTTCTAACAAATTACCTTCAAACTTTTCAGAAGCGTATGCTTCAAGA
ACTTTCAAAATGTAGTCTGTGCTACCTGTACGTCCTGAAACTTTGAATTGAGTGATACCAATATCATTAT
AAAGTTTCAAATCTTGAGGACGAACAAAACGAGTTCTCAACCAGTTAAATGGGTCTGTATCACGAGCTTT
AATACAGTGCTGCATAGGATATCCGTTTAAGGATTTAGCATCATCAGCTGTAACATCAGTACTATGGAAA
ATATAGCAAGAGTCACGATAGCTACAATGTGTTGTATACCCTTTACCAGCATTAGAACAGAACTCATTTA
CAAGAACTTCAAATATTATATTGTTCTTATTGCAAAACTCTGCTGCACGCTTCAGGAAAGAAACTGAACG
GTTTTTATGAATTCCACAGCAGACCTTTTTGATATTATATTGGTCATGCAAGTATTTGATTTGAGTTACA
GCATCGACATGTAGAATTGTTGAAACTTCAATCTCAATGTCTTTATTGACTTCACGAACAATTTCCATAA
CAACAGGGTTAGCGATAGTAATACGCCATACGCCTATTGACCAAAGATACTGAACATAATCCTGAATAGC
TTTCTTCTTCCACTCAACTAGTTCTCTTTTTGAACCAGGATTTATCGTGTTGAGTGTATAGTTAAAGCAA
ATACCTAATTCGTTACAACGTTTAACGTAATCTTCTAACATCTTTTTGTCAACGTCAGGAAGCCTAAAAT
CAGGTCTGGCTGCTACAAAAGCCATTTCTCTTGTAGAGCCGTATACTTCGTTTATCAAGCTATTAGGATA
AGTTTTATTGAGCTCTACGATTTTATCAAGAAGAGCTAAATCAAAATTCGTTCCGATTTTAAAAGTATTC
ATTTTTTGCATCTCGCGTTAATTGATGCGATGGTTTTTGCAGGTCTTACTTAAATTATTATAGATTCGGT
TTATATGATTTAAATGTTTCGAATTCGCCTTTATTCATAAGAGCTTTGAATTCGTCAGGACGCATAGCTT
TGTCGTCGATAATCCAATCATATACTGGTTTATGTGTTAGCAAATTATGGTACTTCAAGCCAATGTTTGC
CAAGTTCTGAATCAGACTTGGGAGAATGTCAATTGCTATACGTCCAGGACCTACTGACTTCATACCGCGA
GCAGTGTAAAGCGTTATGGTATGCCCTGCATCATACAGAGCATTAATGGCAGACACCATTTCGACGTCTG
GTTTGAAGTTCACGTAATCACGATTGTTATTCCACTCTGTTATACAATCATCGACATCAAAACAAAGATT
AAGCATGTTTTCTACATGGTGCATTAGATTCGTTCCCATTGATGAGTGATATCGCCACCGATTGGACGAT
AAGAAACACCAGTTTCTTTATCGGTTAGTACCCATTTACCAGGATTTTTTGTAATCAGAGTAGCCGTTAC
AGGTTCTGCTACGTCATAACTAATAGAGCCGTCTTGAAGTACACGGCCCATGTTACGTAATTGCTTAGTC
AGAAGTTCCATACCTTTATAATACGCGGCTACCGCTTTAAGTGGATTATTCTTGAAATAACCACCTAATG
CAATCCAGATAATTGCTACAGCCAAATGATGATACTCGTTAAACGAACTCATCTTTCCATATTTGTAAAC
TAATGGATTGATGTTAATAGTCACATTACAAGTTGTTTCGTCAATAGTAAACTGTCCCCAGGTCGGATTA
GCGTTGAATTCATCATAACCTGAAACGGCATAAAGCACTTTAGCTTCATCGTACAATTTTGGTCCGTAAA
TCTTGGTTTCGCCAAAATATCCACGAGGGTCGATAAACTTGACTTCGCCGTTATCAGTAATCATAGTATT
ACTGAAGTTCGGGTCACCGTGAATAACAGAGTACTGTCCCTGAGAACGGTTATGATAACGAATAAGGTGT
TCTAAAGCTTGTTTCAGCATTGGCTTCAGACGTCCAATCTTAGTGTAGTTCACATGTGTGATTTTACCAA
 
ATGAATCAATCAATGGTTGAATGCTTTCACAACGATCAATAACTTTCGTATAGAATTCTTTAGTGAAATC
ACGACGAACAGTTTCAGGACTTACAAAATAAGTGTCTGTGCTGAATTTCAGTGCATCTAAAATTGCATCA
ACAATTTGTGGACGAGCAAGACTTGATTTGGATTTAATATATTCAAATGCCGGCTTACCTTTAATGCGTT
CCATTTCAAAGTTATTTTCATTAACAGAAACTAACTGAGGAACTGAATCTGATTTCACTTTAAGATACCA
TGAAATCTCATCTTTCTGAAGAGCTCGACCATGTTCGGTTAATGCGAATTTAGTTACAGTTTCTTCACCA
ATTTCGAACTGCATTAAAACTACGATTAAGTTCACGAACTTCATGAGCTTTTTCAAGTTTAGGCATATCA
CCTAAATCAATTAGATTCATAAGCTCTGATTTATTGAATGCACTGCCGTAAAGGAATTCTACGAAATCAC
GTCCTTTGCAGTATTCGTGAATTTCCTCATCAGTAGAACCCATGTAGAATTCCCAGTCTTTGAACTGATA
AATTCCTACAACGTTACCGCCTGTTGAACCTACGTTAGTGATATTTTCACCATCGAAATTATATCGACAT
TCATCACCCTTAACATAGATTGCGTTAACATTCCAAGACCATGAACCAAAATCAGGAATAATATCACACC
AGTTAACAATTACGTTATGGCCATCTAAATCTTTGTATAATTTAGCAAGAGCATAAGCAGAACCATACGC
TTCATCAACTGTACGAATAGTCACATTGAACCCTTCTTGCTCACAATAAGCTCGAACAGTTTCAGCAAAT
TTTGAATGAACTACTACAATAATTTCATCTGCGCCAAGGTCAGAATAAATTGTATACAAATTGCTCAGAA
TAGTGTCTTGTTTATAGTTAACAAGCACCTTAGGAATATGATGCGTTATTGGATACAAACGAGTTGCTAA
TCCAGCACCAAGAATAACAGCTTTTTTCATGTTGAAGATCCTCATTGGTAAGTAGGTGTATTGTACACTA
TTCTAGTTAAAGCATTCATACTTTGCTTGTAGTCCAATCATAATCCATCCAATTACAAGTGTTGGAATCA
TTACTGGAGCCGTTAAGCTCCAGTAGATAGTCATGACAAGCATCAATAGTAAAAGTCCTATTGTTATTGC
TTTCATTGTTCGCCTCGTAACATAGCACCTAGTGCACCGCCTGGATGGAACTTAAGGAAGTCATTAGAGG
TAAATCCACGTTCAGACGAAAGGTTGATAGCAAACGTATCGATAAGCGCTAACAACAATGTAGTGGACAT
AGTCGGAGCCAGAGAATTCTCATCAACCTCTACCGCGATGCCTGTACAGAAAGAGTAATCAAAAAGGGCT
TCATTTTCTTGTGGAATGTCTGGGTTACAATGCAACAGAATCTGTTTGACGTTCGGTCGAATCATTTTAA
GATGCTTAGCCACGCCTATCATTTCTTCAGTTTTACCAGAACGACTAATATGAATCAACACGTCATTAGG
CGCAATAAATCCTGCATCACCGTGAGAATAATGCCCAGTGTTCAAGTACATACTCGGGATACCGAGTGAA
GCAAATGTCTCAGAAGCTTTAGTTGCAATATTAGCATTCTTACCAACACCTGTAATAATTACTCGGCTTT
CATAATTACTAAGTCCCGGGCGTCGAAGAGTTTCTAGAATAGCATTATAACGAGCAGGGTTTTGACTAAT
TACTTTGGCCATAGCGGCCAAAGAAGAAGCTTGTTTTATGATTGCGTCAATAGCGATAGTAATAGGAGTA
GTTTTCATATTTCTCTCATTTAATTAGTTTACCGTCCATGACATTATATCAAAAATCGAACATATCGAAC
AGAGATGCTTTCTTCTCGTAATCGAGTTTCGCTGCTGATGTGAATCCTTCAAGTGGTTTAATAAATGTCT
TCTCAAGGAGAACAGTGTAGTCCATCCAATGAAGTACGTCGTCTTTAATTAAATCTGTGATTTCAGTACC
AGAAGGCCATGCGATACATTTATCACCGAATGGGTTTCCTTCACGTAAAGGCAGAACATATACTTTTTCA
CCTTCTACAACTTGTGGTGCATCAATATTACCTTTGATAGCTCGGTTATATGTCAGAATTCCACGAATAT
GGAACGGGCATTTGGGACCAGGGAATCCACCTACGTCATATTTAGCAATGTTATTCGCAGAAGATACCGA
CGCGATGCTAATATAATTCAATTGACGGAATTCTTTTTCAAACTCTTTAAAATATTCTTGTAATGATTCT
TCACCTTCTTGAAGCATACGACGAATACATTCTTTAAGAGCTTTCTGTACTGCTTTAGGAGTCGAAGATT
TCTGAGTCTCTAGACCCATGATTTTGAGTTTAGGCTCAGCGTAACGAGTACCTTCCATATCCCACACGTT
TAATGCATAACGTTTCTTACCAGTCCAAAATCCGCCAATACCTTTAGAACCAAGCGGAGGCCCAGCGATA
GCTTCTCGGTCCATGAACATTAAGTGTTGTTTATTGTTCATGTATTCACACATTTCACGGAAACCTCTAT
CAATAGCTGGTTCCATACGTTCACGTGCAAACTTATCTAAGAAGTCTACCCAATGGTTGGTATCACGGAA
TTTAGATTCACCAACCTTATCGATAATTTTATCAGCAGATACGTAAATAGAGTCTGTATCACCATAAAGA
ACGAAAGCTTCACCTTCTGTACCACAAACTTCATTCAGATATTCATTAACTTTACGTTCAATCCACTGTA
AAGCCATTTGCCCGAATGTTGTGATTGCAGTAGCATTACGCAAATCATAATAACGGAACCAAACGTTGCC
AAGTGCACCATAAAGTGAGTTGATAAGCAATTTACGGTTAATTTGTGCAGTCATACCTGCAACTTCAGTA
CGTTGAGCTCTAAACAACATTTCATTAAGAGATTTAGCAGACAACTTTTTAATCTTTTCTTTAATCTCAT
CGCTGAAGTCGAAACGATAATCAACATCTAATGGTTCGTCAACAGAAAGATTAGGATTATGCAATGCCTC
 
TTTAATTATTTCACCATTACGTTGAGCTGCAAGCATGTAACCTTTATGTTCTTTACGTTGATTAAAGACC
TTAGTGATTTCAGTTGGAACTACACCATCACGGTCTTTATAATACATCATGCCGTTAGGAGAACAACTGT
ACACATCAGAAGGACGTTCAGCAACAGCGTTAATATAATCATGCAATGGAGCTACTTTAAACGTTCCTGC
TATTGTTTCTGGGCTAATATTCACTTGGCGAATAATACTTGGATATAGAGATGTAAGGTCGAAACTCATT
ACATATTTGTATCGATTTGGAATAGGTTCCTTAACAAAAGCGCCGGGATAAGGTTGAACCGGGTGAGAAC
GACCTTGTGGAATCACCTTGTTCTGCTCTTTAAGGCTATTAAAAATAATAGCATCCCATGTTTTAATTGG
GCTAAACACAGATTGAATCTGTATCTTAGCATAATAACCCATGTCCAAACTCAAGTTGATGAACTGACGC
TTAGCATCAATTTGCAATACACGATACACGTCGATAATGTTATAAGAAATATATCGTTGGTGATTGCTTT
CACGAAGCTTAGAAATAGGGCCGTCATATTTCAGTTTACCAACGTTCAATTCAAATTCTGAAATGTAATC
CAGAGAATACGACGGTTGATTGGTAAAAGAGAATTTTTTGTAAAGGTCAATGTAATCAAGAACAGAGATA
CCGAACAATGTAATGATTTCACGAGAACCATACATGTTTTCGATAACTTTAACACGAGTTTTACGATGTG
GTGATAAACGTTTCGCAGTTGATTCGCCAAAAATATTCTTGATTCGGTTATACACGTACGGAATATCAAA
TGACTCAACGTTCCATCCAGTCAAAATGACAGGAGTTTTCTGTTGCCAGAAGTTGAGATATTCCATCAAC
AATTCTTTTTCGTTATCGAACGGCATATAAATGATTTTATCAATAATTTCAGATGGAACTTCATCACCAC
CTTGTTCTTGAAGCTTAGCAGCGATTTCAATAGACCATTCTTCTACATTACCATATGGAGAATTCAATAG
ATCAAATACGTAGAACCTGTCGTCAATTGAGTCATAATGGGTGATAGCATCAATCGGATGTTTTGCTTGT
GACGGCTCAGGGAACCCATCCGGAGATGTTACTTCGATGTCGAAGTTAGCCACACGAATTTTTGTATGGT
CGTATTTGATTTCATAGTTATAAGTGTCAGACAAATACGCCAATTTGAAATCGTCCATGCCAAGTGCTTC
AAGTCCGATATCTTCCATGCGTTTAATCCATTGGGAGGCATCACGCATATTAGCGAACAACTTACGAGTA
CACGGTTTACCGTAGATATCGAAATATTTCGTAGCCTGACTTTCTGGACAATGAGCAAACAGTGATGGTT
TATATTCTACTTCACGAGTACGTTCACGGCCATTAGAATCGATGTAACGTTCAAAAATTGAATCACCAAT
CTGTTCAACCGTTAAGTAAAATTCTTTCATTTAATTTCCTTTAGACGAGTTATTGTCTTTTGTTTGTTGA
TGAACTTATTATACTCCAAATGGGACCGAAGTCCCATTGCTTTAATTACCAATTGTATATTTTGCTTTCA
AAGTCCAGTCATCTTTCTGTTTAAAAGAGATAACACGGAAGTTATTAGTCAGGCCAAACAGATCTTCACG
AGCAGAGTCTTCAATAACAATCAATCCCCAGTCTTCAAGCAACTGAGCAATCGAATCACGTCGCTGATAG
TCTTCACCATCAATATCAACCTGACGTCCGTCCATACGCAGCATTTCTTTAAAGTGGACGATGTAATATT
TACCTTGCTTTTGGAGGATATGACAGGACTGATAAAGAACTTTATCCTTGTTATTAGCGATGCCCATACG
GGTCAGAGTCTCTTTGACTTTCAGAAAATCTTCAGGGTTCTTCAATTTAATTTCAATCATTTTTACCATT
CCAATGCTAATTTTTTAAATTGTTTTTGTTCTTTCACATTTTTTGTAACTTCTTTGAGAAACTCATCAGT
GACCATGGCCTTCAGTTCTTTAAGGACTATTGGCAACTTGTTATTTTTTTCAAGGAGCCGTTTATACTCG
GTGGCATCATTCATGTTGATTGTGTAATGTTTCATCAACAATTTGATAACTAGTAACTCTGAAGTGTCTT
CGACTAGTTTAGCCCATTTAGAGAATCGACGACCACGCGGAATTGCCGCCATCATATAGTTGAAGTGCGC
TTCATCACTTAATTCAGAACCGACTAAGTTCATCATGTAAACGGCTGGCATACATTCTGGGAACTGGGAT
AATGAGTTCTCAACCATAAATTTTGAGTAGTCGACCTGAGCAATTGACATAGATTTCTTTTCATTAATAG
CATTAATGATTTTAAAGAATTCATTCTCAGGTTTTTCTTTAAACATATCAGCACATTTTTGAATCGCATC
AGCGTCATTAGACTTCCATGCAATTTGATGCTCGTTAAGTTGGACGTCATCATCAAATAAGTTCATATTA
TTTCCAAGTCAGTTCGCAGGTCAGTTGAAGAAGCATATACATAACATGTAATTCAATATTACTTGCTAGT
CCATGAAACTTATTATTTTCGCCTGCAATTTCGTAAAGCGAAATAATACTTTTACCAGGTGCAACTTGGT
CATAACATTCTGAAACTAATTTATCGATGAACCATGAATAATCTGCAGCATATTTTGGAGCTAATGCACG
AAGTTGTTTGATATCTTTGTTCTTCATTGCTTCGATAACATCAGAGACTGTGCCACGGTCATTAGTTACG
ATAGACAGAATACCTGCATCAAGAACACCTTTAGACGAGTACTGGTCTAATTGGCCAATTGTACGACGAA
AGTCCGGGAAGTTCTTTTTAACCAGAGCTGCAACAACTTTCATATCAGCGATTTCGATATTTTCATTCTT
ACAGATTTCAACCATACGATGAATCATTTTCTTCATCATCGAGATTTTATCTTCTTCGGTTGGACGTCCG
AATTCGATAACTCGACAACGACTACGTAACGGTTCAATAATTCCATCGATGTTGTTAGCAGTAATAATAA
 
TAGAACAGTTACTAGAGAATTCTTCCATAAACGTACGAAGGTGACGTTGTGATTCGGCGAGGCCACTACG
GTCAAATTCATCAATAACAATAACTTTCGGCTTGCCTTCCATTGAGACTGAACGAGCAAATGCAGTTAAC
GGACCGCGTACAAAATCAATTTTACAATCAGAACCGTTGACGAACATCATTTCAGCGTTGATATCGTTAC
ATAATGCTTTTGCCACGGTTGTTTTACCGGTACCAGGAGATGGAGAGTGTAAAATAATATGAGGAAGTTT
TCCTTTAGATACTAAAGACTTGAATGTTTCATGGTCATACGCAGGCAGAATACATTCGTCAATGGAAGAA
GGACGATATTTCTGTTCTAGAATGTGTTCTTTGGAATTGATAGTAATCATGTAATTTTCCTCATTATCAT
TAAACTGGAGGATTTCTCCTCCATAATCGTACTACATTAAACGTAAAGCTTTGACTTAGAAGTCGTGGGT
AGAATCGGCTTCCATCGCAATAACATAACTTACTTGAGAGCTTTCAAACTTAGCTGCAACTTTATCACCA
GCTCCCCACAACATTACTTTATAGTTACCTGGCTGAATTTTCATATTAGCCATATTGATTACGAAGTTAA
AGTTGTTAGAACCATCATAATCGGTAAGAGTCAAAGAATACTTAGGACGAGTTAGTCCGGAATCTTCAAC
TTTATTATAGCCATTGATAACAATTTTACCATCTTTATTTGTAATAGCAATTGTATCAATCTGAAGACCA
CGTGAAACTCGAAGCAATTGTTGAAGGTCTTCGGCTTTAATTTCAGTAATCACAGAAGCCACTGGGAATT
GAATTGGCTTATTAGGAAAAACAATGGTGCTCTTGTCAGCGGCAGGCCAATAAACTGTGGAACGTGTATC
TGCAATTTTAATATTACCATCAGTGTGCATTGAAATTTCAGCATCGTCAGACACTAGACTCAAAATGCTT
AAGAAGCTATTCAAATCATAAAGTGCTACGTCAAAATCAATTTCATCAGAGATATTTGCTTCCGCATAAG
TGGTGCCATTAACTGCACGAGTCATAATAAATTTACCTTGACTCAGCAGAATGCCTGAGTTAATAGAGGC
AAAGTTTTTCAGAATAGCGATAGTATCTTTAGACAGTTTCATTTTATTTCCTTTCAAGACAATTCAAAGT
TTATAACAAATTTATTATGCAGCAACCATCTGCTTTTTAATAGCTTCAGGCAGTGATTCAATATATTCTG
GAATTTTAGAAGCAACAATTTCGCCAGCTGCAATTAGTTGTTCGTCGGCATCAACACAATAGAAATCTTC
TAGACGCTCAGAACCGTCTTTATCAAGAACGATTTTTCCGCCAGCTTCAATAGAATCTTTATAGTGCTTA
CGCATCAGATGAATCCACATATCAGATACATCGTTATCAATAAAACCATAAATCATAAAACGAGTTTCTT
GTGGCAAAGATTGAAGTTTTTTAGCACTTGCGTCATCACAATGCAGGACCCATGCTTTACGAACACGATT
ATGGTTATGTGGGTTAGCGTCAGTACGGATAGAAGTAACTTTCAGATCAGCAGCAGTGAAACCTTTAGCA
GTAGTAAACATATTATTTCTCTCTTATTCGTAGAAGTCGGTAACTGTCCATGCGGAATTGCGATTAGACA
TTATTTTATTATACTCATCTTTAAAAGCAGATTTGAGCATTTGTTCAGTAACAATATGTTCTTCACCTGA
AAACGTATTGGTCAGAACATATTTTTTAATTTCAGGGACAACAAGAAGATGTTGACCGGTTGAATACTCC
ATGTTGTTCTCCATTTAATTTGATGTGGTCATTATAACCACATCGTGTTAAAGCGTTTATGAAACAGTCA
TTACAGTAAATCGTCCAACTTTGCTCATTTGAAGATGTTGTCCATAAGCTTGTGGGTCATGGTCTCTGTG
ACTAATTATAAACACGTTTGCATCGGTTAGAGAGTTAAGAATTGTTGAAATAGCTTTAATACCTTCAGAA
TCAGTCGCTGAATCCATGACTTCGTCCATAATTAATGTATTGATTCTAACACCAGAGACCTTTTCGGCAA
TATCTCGCCATGTAAACAACAATGCGATATCGATACGTGCCTTTTCACCTTGACTAAATGAAGCATAACT
AAACTCTTCACGGCCACGTGATTTTATAGATTCATTGAATTCTTCATCAATAGAAAACACGTAATCAGCT
TCCATGATTTTAAGGTAATGGTTAATCTGCTTGTTGAACAGAGGCACATATTTTTTAATGATAGCACCTT
TAATACCAGAATCTTTTAACATATCTGTGATAATACCACGATGGTATTTTTCAAGAACGATATCCGATTT
TGTTTTAATGATTTTATCAAGTTCTTTTTGAAGCAAAGCTATCTCATCGGCATGGTCGATAAATTCCGCC
GAGGCTTGTTCTATTGCAGCTTTAACTTTCTTAGCTTTATCAATAGTGGCAATCATTGCTTGTTTCTTGG
TACGAATATCAGATGCAAGGTCCTGTTTAGTCTTAACATTAGCTCTATATTCGTCCACTAGGACACTCAG
ATTATCTCTGTGACAGGTAAGTTGCTCGAATGAGTGATTACATTCATGAAGCTTATCGGTAATCTTAGAG
ACAATAGGATCACCCTGGTGCAACTGTGATGCACAAGTAGGACATGTACCACCATCATGATACATCTTAA
TAACTTTGTTATATGAGTCAATTTTAGATTTAATCAAGAATGCTTCTTGTCCGATCTTGTTGAATGCCTC
AGTAGGGTCTTCATCAAGAACGATATTAAGTAGTCGCTCATTAGCTTCTTCGATTTCTGCTTTTAATGAA
CGAGCTTCTTTGGCTAGGTCGTCATACATATTCTGTAGACGCGCTACATTATCACCAGATAATTTCTTTT
GACGTTCGATATTCTCATTATAAATCTTAATTTGTTGGATAACACCATCTTTCTTGGCATCGAGAACTTG
TCCCTGGGAATTAAGTTCTCGAACTTGAGACTTATTGATTTTATCCATTTCAGCGAGTGTGCCAACTTCT
 
AAAAGGTCTTCGACAAGTTTTCTACGAGCAGGGGTGCTAAGAGCCATGAAAGGGGTATACCCTGCCGTCC
CAAGCACAACGATTTGTTTAAAAGACGCGTATGACATTCCGATGCTACGTTCAAATTCTTCTTGGAAGTC
ACGACTGCTGGCAGATTCATCAAGACGAACACCGTCAACGGAGATTTCGAAAATATTGGGCTTTTGGCCA
CGTTTAATGAAGTATTTTTTATCATCGAATTCCATCCAAAGCTCAACTAAGAGCTCTTTCTTGTTCGTGC
TATTAATGATTTGTCCTTTCTTAACATCACGAAATGGTTTACCGAAAAGTCCGAAAGTAATGGCTTCTAA
CATCGTAGATTTACCACCGCCATTTTTACCAGTGATTAATGTCTTTTGGACTTTATCTAATTGGATATCA
ATAGGATTCCCACCTACCGACATGATGTTTTGATAACGAACACGATTTAATTTAAATGATTTCATATGTA
TTCTCTTCGAATGAAGTCCTCTACATATTTTAACATGTCATCTCTATCAGCAAAATACTCGTCTAGTTCT
GCATACATAACAACACCCGGTGCGTACTCACAGGTAACATACATTGTTCCAAAGAAACGAGGAGTTGTCT
CCTCGTAATTCTCAGTCCATTCAACGATACCAATATAACCGTCACCAATACTAAATTCATGACGATGGAT
TTCATAATCAGTCTTCGTCATTATACGCATCCTTGATACATGCTTCAGCCATTTTAAGAATAGAGCCAGG
AGTATCGTTGTCCATTATATTAAAGCTAAATTCAATAGCCCAGTCGGTGCAGACATGAACTGCTTTAACT
TCATCGCCTTCAACGCGTAATGGCTCAATCCAATACTTGATAGTGTTCTCGAATCCATCATCATCTTTAA
CGTCCATTTCAACTTCAAGCCTGAGTCCAGCATTGTTAAATTCATCTAAAGTCATTGTGTTGCCTCAATA
TAAAGTTCGTTGGAATATTTGACCAGCGCTTCTCTGTCTTCGTCTGAGATATCTGGAATCGCGTCAATAT
ATTCTTTAATGATATCTTGAAGTGATTTAATCTCGATTTCCTGGTCGTCATCAGATTCAACACTGTTATC
AACTTTAGAAACCATACGAAGAGAGTGAACTACTTTTTCAAGTTCAGATTCAAACTTAGTCAACTCTGAA
TCAATTTCTGATACTATAACACGTACGGCTAGATTGGTATAATCAGAATAATTGATTTTGCCTTTAAACG
GATAAGTGATTCGTCGATGCCATGTTGTTTCATTAGGAATAAATTCCATACGTTCAACATCAGTGTCAAA
TACCCAGAATCCGCGCGGGTCGTTCTCATCACCTGCAGTAAGAGTCCATGGTGTCCCAATATATTTGACG
TTAGCAGCTTCAGAGATTGTGTGGAAGTGGCCGGACCAGACTTGTTTATAAGTCTTTAAGAAATCAGGTT
CAAGCCCATGAGACTTCATCCCTTTATAGAAATAAAAGCCATTCAGTTCCCAATGACCAACACAATACGC
GGCAGAAGAAGTCTTGATGTGCTCTAAGATTTCACCTGTATTTTCTTCACACATCCATGGAACGATATCA
ATCAGACATCCGTCAAAATCTACAGTAGTTGGATGCTCGTAAATAGTAAAGTTTTCAAATTGAGTTAACA
ATTCAGAAACAGTGTTAGGTGTTATTGTATTCTTAAACGCCATGTCATGGTTTCCTATCGTGACATGAAC
GTGAATACCTGCTTTAGAAATCATATCAACGATTTCACGATTGAATTCCATTGTACGATGTGTTATTGCT
TTACGGACATCAAACCAATCGCCGTACTGAATCCAAGTTTTAATCCCGTGTTTCTTAGAGTATTCAATTG
CTTGACGAATACCATCACGTTGAATATCTTGAACCCATGGGTCGTCTTGTTTGACTCCGATATGGAAGTC
GCCTAAGTGCAAAATTTTCATATTATTTCCTCATTAGTTAATTCATTTTATCATCCGTTTATAAAGCAAA
AAAGGAACTCCGAAGAGTTCCTATCCACATAGTACACCGTACAGAGGCTCATTTGAACTTTTAAATCGAA
TGGGCTTGTATGTAATAGTTCCTTCGGCTTTAGCTTTATCATAAGACTCTTTAAAACGTCTCGTTCTTTC
TGCTTTGCCTTTACGGATTTTATCGAAATTTTCAGAGGTCAGGTCGTTCGTCATGCATAATTATCTCTCA
GAAAATACCTAAACCGATTTCTCTCATCGTTTCCACCATTTCTTAACTTGAAATGAATCAACCCACACGA
GTTGTTTTGGGTCTGCCTTTACAATAGGCGGACAAATTTCTGTAATAGAACCGTCGTGATATTCCTTTTC
AGAAATTTTAACACAAAGAGGACCTCCCAATTTAAGGGTGAACCCTGCGTTCAGCTTCAGACGTTTAAAC
TTTGTTTGAACTAGCGCCATTAATGATGTCCTCAATAGCTTGACGAGTAGTAACCCACTGGATTTTAATT
AACTTGTCATTGTATGGTTGTCGTAGAATATCCCGAGATTTCTTAATAGCATATTCGTAAGCTTCGAAGT
TGTTTTGAATTGCTGCTTCTTGAGCATGAGCATAAAGACGGTTTAGTTCAAAACGATTCTTTTTAAGAAT
CTTTTCGGATTTTTTACGTGCTTCAGCTTCATTTTTCTTTTGGATTTCTTGTAGCATTTTAGCTACTGCA
TCTTCTGCTTCTTTATTTTCAATAATCTCGGTAGTCATATTATCTCATTAAATTAAAATAAAAACGTCCA
TCCTCTTCAATACAGAACTCGTACTCAACTGGAGTCCCATTTATTGAAAATGACACGTCATGCGGGTTCA
AAGGGTCGATGTCAAAATTAATTTTAAATTCACGACCCAACAGTTTTACCAAATAAGGGATTGCTTCGTC
AAAAGGTTCTTCTAAGTAGTTAGAGATGTCAATTGTCATCCTCATATAAAAAATCCAAACTAGGCGAATC
GCTAACAACTTCCTTCTTTTCAGCCCCAGGAGCTTTATAAGCGGATTCTTCATAATGCGTCATTTTATCG
 
TAGATGTCTTGAATAAACGTTTCATCTACCAACGCTACCATATCATCGTCATGACTGTCGTAGACATTAT
GAACGAAGTAACTATATTTCTTTGCAACTTCCTTACGTTCTTTTTTGATACGTTGGACAAAAGCATTAAA
ACAAGCTCGAGTTATGTATGCATGTGGGTTATTGTACTTAGTTTCGTCGAAATTATGAAGTCCCTTAATA
GAGGCTTCAATCCCATCTGCAATCATCTCCTGCTTCCAAGACTGGGTATATCCTGAAAAGTTGAAACGTT
TGGACAGGCCTTCGGCGATAAGCATAATTGCTAAACCGATGGTATCATTCTGACGAATAATTTTGTTTGG
GTCTTTATTATTTAAGAGCTCCTTTTTCCACTCGATTATCCCGGCGAGAAGCTCTTTATTATTCACATAG
TTATTTTTAGGTTTTTGTTCAGTCATAAGTACCTCTTGGCTCAATTCATAGGTCTATTATATCATAATAT
TTAGAGACCTATCTTAAAGCACGAGATACTATCGGAGCAAACAATAAGCCCGAAACACTTTTTCCATTGA
ACGTTTGAACTCATTGATATCGATATAAAGCTCAGTAAGACCGGCATTATAAAGAAAATCTAAAAAGTTC
TCATGTGTTTCGTTATAGTCGCCTTCGTTTAATTGAGATACATGAGTTTCCCAAAAACCAACACCTTTAA
CGAATGGAAAATACGTGACTTTCATGTATTCGTCAACATAAAATTCGCCCAGCTTTTCTTCAGTTATACT
GATTCGAGTTAATTTCATTCTATTTCCTTTGCTGCTTCAAGAGCAGCATCCAGCGAATCAAATTGGTCGA
CGAAATCAATATTCAACGCATCGTCAACCGTAGCATATAACCACCATTGTTTGAATTCATATTCTAGGAT
AAATCCAGGCCCTTCAATCTGAGGAAGACCTAGGCTATTTGTACCAACTTCGTAACCAGCTAGACGCAGG
TCATTAATCAAAGTTTCTGTTAATACGCTCATTTTACCGCCTTAACAATTTTCAAGTCAGCGAATTTTTT
CTTACGTTGATTTTTCATACTACGGATAGTCTTTTCAGAGATTTCATGTTTCTGATAAGCTTTAGATTCA
ACACCAAATGCAGCACCGTCAAATTCAGAAAGAATATATTGTACTCGAATTTCACGAATAGAATTACGTC
CAATTTTCTGGTCCGTGTTTTTGCATGATTTCCATAAAGACGGAAATTCCCACGCGTCAAGAACTTGAGG
AGTAACAATATTGCCAGATTCCAGAAGAGCAACAACTTTATCGAAAGCGAAAGAATTAACTACGCTTTTG
ATTTTAGTAGACATATTATTTTCCTCGGTTATTTTATTATCCAACGAGAGAATTATACTCATCTCTCGAG
AGTTTGTATACTATTTTTGAAGCATGGAAAGTAAGTAATTTGTATCACGTGGAGTAATATCAACACCCAT
ATCAAAGATGCCTTTATAGATTGGGTATTTGTTTTCGGTTGTATGGAAGCTGAAACTATTTCCATCACAA
CTAACAGTAATAGCATCTGTTAAAATGTCATACTCAATCCTAACAATTGAATCCCCAATTTTAACGTCGA
GGTGTTTTTTGAATTTGATAAAATCTAATTCTTGGGCCATGATATTTTCCTCATTTTAAGACGATAGGAA
GATAATAACATGTCTTCCCTATCGTTGTACATTATTAACCGAACTTTTTTGGCGCCTTTCCAGCCTTCGT
ACATAACTGCACAAGTCAGTAGTACAGCGTGCTGGCATTCTTTGTATTTGTAAATCCAGAACCAGCGTAA
GAAGTCTTCATCCTCAACGCCGGTGGATTTCATATTGGCAAAGAATTCGGCACGTTCTTCACGAAGCTTT
TGCGATAACATAGATTCAGGATTCATTTTGTGTTCCACACCCCATTTTCATCGATGAACTTAATCCAGTC
ATTTACAGACCATTTAGTTGTATCACCCTTTGGATTAACATTTAAAGTATATAATCCCTGCTTAAAAAGC
ATTCGTTTGATATTCATAATTTCCTCAACTGTAACGATAACATTCGTTAGCTTTACGTTTGGCTTCAGTA
TGAGAAGTATTATAATCATAATCTTCTTTACTGTAAACATCTTTTTTCAACTTACGAACTTCTTTGCGGA
GTTGTCGATTCATTTCATTCTTGACTTCAGAATCAACGCCTTTCATACGTTTCCATTTATCTGAGCGGAA
AATGTTTTCAACCATGTCTTTGTGGCTAACACCGTCAGGAGCTTTACGTTTACCAATGTAATAAAAATCT
TTAGAAGACAAATCTTTACGACGATACGTTTTACCCATAAGTACCTCAGAGATTGATTTCAATGATACGT
TTGATATACTGCACATCACTTACTGAAAGGTCTTTAAGACCAATTGATTCTAAACGTTGTGCAAATGTTA
ACATATGGGTGAAGTCCAATTGCGTTTTAGTCATTTGATAAGTGTGAATGCCATTAGCAAATTCAACCTT
CAAATCGTTAATGGTGACGAAAATCATTCGTTCATCATTCATGTTAAGAACTAGAACAACTTTCTTTGAG
TCTAAACTAATCATAGTTTAATTTCCTTCGCAATTGAACAGAAGATGCCACGGTCACAAATCATTTCACC
GATTCCAGGATACTTGATTGAGCGATACCAAACATCTAGAGTTGAAACTTTGGTTATTATAATAACATTG
CCGTTAACGGAATAATACTGATCAACCTTAATTTCTTTATCTTCAATTGTCATTTTGTTTTCCTCTGTGG
TAGATGATGCTAATCTTAACATATCTATCGAGGATGTAAACTGTTTTATGAAAATATTTTTGATTTGAGA
GGGGCACAAGGAGATAGTGAAGTGAAGATTCCAATGGATTATATTAGAAATGAGATAAAGAAGACCAGGC
GGCACCTGGTCAATCTTCTATGATATCAGAGTCGTATCCTAATGAAATCAAGGACTTTTTAAACTCTGCA
ATATCTTTGTTTCTTATCATTTAAAAATATGACAGGATAACGAATATTAAGTGAAGTGAATCCTGCACGT
 
TTAGCAAGAGATACGATTAAAGGACGGTCATATTCAATCTTACCATTATTAGTGAGGACTTTATAAAATG
TATAAGGAGCATTGAGCTCCTTTAAAAGTTTTGTAACAGTATAACATCCTGGACAACGTCCAACTTCTTC
AGGGATGCCATATATTTCAATCTTGTTCTTTAAGTTCGAGTTTTGTTCCACGAGAAATAATTCCTTGGTA
AGCCCAATAAGGAGGATTAACAGAATCCTCACCAGAGTTTTCTTCAGGAAAATAAACCTGGATAACAAAT
CCAGATGCATCAAAGGTAAAATAAACCGTAGGCAGTTCACCTAACAATTCGGCACGAGCATTAATTTCTT
CGAGATCCCGTTCACGTCCAGCCCAAAAACTAGGATTTAGATTACATTCGTAAAAATAATAGTCATTACC
GAACATATCAAACCCCTTCCAACTCTCTACCACATACCGCTCAAAACTAATCATAATTAAGCCTTTTTAT
CAAGAACAGAATTCAGTTTATTAGTAATTTTGTCCAAACGTTCATTGAATTCGCTGGAGGATAAACCCTT
TTCCGGAGAAATTAAACTAATCACGAAAAATACACCAGTAAAAGGAATAAGAAAAATAGCTCCAACTGCC
ATAAACAGAAAGAATGTTACAGTTGTAAGAAAATCAGCTAAACCTTTACGAAATTTATACATATTTACCC
TTAATTAATTAACCAAACATTGATAAGCACTAAACTATATTGCGAATAAAATTCTGGGCCAAAATGAAAA
ATCATATCATTTATAGTGTCCATAATGTAATTCAATTTAATCATGTTTCCACACCCCATCGGTATTTGAC
CAAAGTCGCTGATTATCTGATCCTCGCCATAGCTTTTTGGTCGGAAGATTTTTCTCATACTTCCCATCAA
TAATAACATCAACATATTTAAGCATTTCTAGTTGTTTAATATCTTCAAACTTGTATCCTGTCCACAACCA
AATGCTTTTATTGGGATAAAGATTTTTTACTGTTTGAGCAATGCAATGAATAACATCTCGATTGTCAGGA
TAGAGAGGGTCACCTCCGGTTATAGTCAATCCTTCTATATAATCGTTATTCAAACATTCAATTAATTGTT
CTAGTGTTTCACCAGTGAATGGAACACCATTTCTAGCATTCCATGTTGATTTATTATAACACCCTTCACA
TTTATGCAAACAACCTGTAACGAAAAGAACGACCCTACAACCAGGGCCATTCACAAAATCGCAAGGATAA
AATCTATCATAATTCATTGGTGTTTAACCCTATGCATGATTTCTTTATTTTTACCGAGATTAAATCCGCG
TTCGTTCGGATTTCCCAAATAACCGCATGTTCTTCTTATGGTGTTCATCTTTTTAGGATCAGTTTCTCCA
CAAATAGAACAAACAAATCCGGTTTCAGTAGGAGTCATTTCATGGGTACTTCCGCATGTAAAACATTTAT
CTACTGGCATATTAACACCAAAATAATCTAAATGTTGTGCAGCATAATCCCACACGGCCTCAAGACCTTT
TAAGTTATTTTTCATATCAGGAAGTTCAACATAAGAAATGTGACCACCTGTCGCAATGAAATGATATGGC
GCTTCACGAGAAATCTTTTCAAACGGAGTAATATTTTCTTCTACTGAAACATGGAAACTATTAGTGTACC
AGCCTTTATCAGTAACATCCTTTACACTTCCATATTTTTCTGTATCAAGTTTACAGAAGCGATAACAAAG
GTTTTCAGCAGGTGTAGAATACAAGCTAAAAGCAAACCCAGTTCTTTCAGTCCATTGTTTAAGAAGAGCA
TTCATTTTAGTTAAAATTTCTTGTCCAATATCACGACCGACAAGAATATTCAATTCGTGAATACCAATGT
ATCCTAGGGACACTGAACTTCTACCATTTTTAAATAACTCAATTATGTCATCATCAGGTTTAAGACGAAC
CCCGAATGCACCTTCTTGGTAAAGAATAGGAGCAACAGTAGCTTTAACTCCTTTTAAGGAACTAATTCTA
CACATCAAAGCTTCAAAACACAGGTCCATTCGCTCATCGAACAATTCAGTAAATTTCTGTTCATTGAACT
GTGTTCCAATATAAGAATCTAACGCAATACGAGGAAGATTCAGTGTTACAACGCCAAGATTATTGCGTCC
ATCAAGAATTTCATTGCCAGTTGAATCTTTCCATATGCTCAAGAAACTACGGCAACCCATTGGAGAAACA
GGAACTGAAGAACCGGTAATAGCTTTATTATTCTTAACCGAAATAATGTCAGGATACATTCTTTTGCTTG
CGCACTCTAACGCAAGTTGTTTAATATCATAGTTCGGATCATCTTTATAAAGATTAACACCTTCTTCAAC
AAACATAACAAGTTTAGGAAAAATAGGAGTTATTCCATCACGACCAAGACCTTTAATGCGGTTTTTAAGA
ATTGCTTTCTGAATCATTCGTTCAGTCCAGTCAGTTCCCGTACCAAATGTAATTGTTACAAAAGGCGTTT
GTCCGTTTGAACTAAAGAGAGTATTTACTTCATATTCATAAGCTTGGAATGCATCGTATACGTCTTTTTC
TGTTTTAGACTGGGCATAATTCAACGCATCAGCGATTTGCCATTTTTCTGCATCCTCAATATGTTTTGCA
TAGGTGCGTTTAACATAAGGAGAAAGTACTTTATCTACATTAGCAAAAGTCGTTCCGCCGTATTGATGGG
AAGCAACCTGTGCAGTAATTTGTGCCATAATTGCAGTAGCAACGCCAATAGATTTAGGCGTTTCAATTTG
AGCATTGCCTAATTTAAATCCATTCTCAAGCATCCCCTTTAAATCGACTAAACAGCAATTGGTAAACGGA
AGAGCAGGAGAATAATCAATATCATGCATGTGAATGATTCCGCTTTCATGCGCATGCATAATAAAAGACG
GGACCATATTTTTGGCAATGTGCTTAGACACAATACCAGCCATAAGGTCCCGTTGAGTTGGAAAAACACG
AGAATCTTTATTAGCATTCTCGTTCAAAAGATCTTTATTAGTTTTATAAATTAATCCTTCAATTTCTTTT
 
TCAATTTTCATTTTAAACTCTTTCTAAGCTGCTTTTTGAATGAAGCTATTAATTGTGTTTTAGTATCAGA
TTCATTATATTCAAATCCTCTTTGAAGCATCTCAGCCATCATTTCCTCTTTACCTAGTCTTGAGAACTCT
TTAGTTTTGTCTGGAACAAAATTAGGATGGATATTATTATCAGAATAATCTTGTTTCAAATAAGCAAGAA
GAGCTTCAAGCCATTCTAAGTAGTCAACGTTTTGTCCTTTCAAACCGGAACGGTTGAACTTATGCTTCAT
TTGACCTTCTGCTGCGTTGCACAGATTACAGAGTAATCCACGAACTCTACCGGCTTTTGGCCCGTTTAAT
TCATGGTCGTGGTCAAGATGGTTACCTTGAACATCAGGGTCTAATTCACGGTGGCAAATAGGACACTTAC
CGTTCTGTGCATCATAAAATTTTTGTTTTTCTTCTTTATATTTTGCGCCTGATAATAACATGATAAACCC
TTACCTTAAATAGATAAGGGTATTTATAATTTAGTAAACGTTATAAAAGTCTCGATGAAAACGTTTATAT
TGACGAATCATTTTCTTAGTAGGACGTGTTACTGAAATAATTGAAGTAGTCATAGTCGGATTGTTTGGAG
CGCCAGTCATAACAAATTTCCAGGATTTTTGGCCATTTTTTCTTTCACCATAAAATAATTCAGTTTTCAT
AATAAACCTTTAACTTAAACGTCTTTCTAAATATTCAATGTATTCCGCTGCGGCTTGCATCAGATTACCT
TCATAACCGTCATTATTTTCTTGGGCGGCTAATTTAGCTAAAGCATAAGAAATACGTTCACCTTCGAATT
CGCCAAGTCCACCATCACAAACTACTTGGTCAATTTCTTCGAGTTCATCTATTGAATGGGATTCCCAGAA
GCACCAAAGAGAAGTATCACGGTCATAGATGCAAAGTTTCTCACCTGTGTTCAACATCACTTCGATAATG
CCATGGTCGCCATAACCATCTTCGGTAGTAGTACCAATTACCTTGATGATTGTTCCTTTCTTTAATTCAG
GAAACTTGACTTGTAGTTCTTCATCTTCTTTAACAATCTTGAACCATTTACCGCGAGTTTCAGAAATATT
CATATCAATATTCTCTATAAAAGTTACGATGAGAACGTTTAGCTTTGCGAAGCTGACGTTTAGTAGGACG
AACTGTAAATGGTTGTCCAGAGAAAAAATTCGGATCGGTTTTAATAAGCAAATACCAATCATGTTTGCCA
TTTTTTTAATAAACGGTACAATAATTTAACTTGCATAATAATCTCTTCCCATTTCCATATCAATTAATTC
ATTTATTGAAGGTTCATCATAACATGGTTCTTCGTCGTTGTAAACCGTTTCGACGTCTGGCTCGACAGTC
TCCCATCGAGCAGAATACCACGGTTTAACCCCGGAGTTTATTGAGCTCTTCATAATCAATCCAGGATTCT
AGCCCTGACGGCAGGTCACATTCAATGTTCCACAGAAGCTCTTCAATGGCTTGCAGGCGATTATATTCTT
CAACGGTAATGGTAACCATTGGTGCAACCGGTTCTGCCTTTTTAACCATCATATCGTTCATCAGGACGTA
AAGGTCATCACGAACGACAGTGTCCGTTCCAACCCCATGATTTGGCTTCGTAAGCTAATGATGAAGGGAG
CACATTTAAAACTTCAAAAATAGTTGGAGCATCAGGACCAAGTTCATTGGCACAAACTTCGTATACATCT
TGGAAATCTTTAAGATTTAATGAATATGTCATTTAAAATATTCTCGCAGTTGGTCAAATCCACCAATGTG
ACTTCCGTCAGGAGCAAACACTTGAGGCATAGTCAATCCGACTTGTGATTCACGTCCAAGCTTAACCAGA
AGCTCAGCAATTTTCTCATCATCAAAAACACCTTTTTCTGGCATAACATTAATGAATTCGAACGGCTGTT
TCTTAACAGTCAGCAATCGTTTCGCATTATCACAATAAATGCATTTATGAATAGTAGAGTCATACCCGTA
CACTTTAAACATTATTTTTCCTTTCATCGATTAAATATGCAATGCCAAATACAATACATGTAATAAAACC
TGTGATACAGATAGAAACCAACAACACTGTACCAAAGAACCAAATCAAGTTATCTTTAGTAGTTTGTTTG
ATATCCATCTCACGAATTTCTATAGCTATCGTATCACCTTTATTCAGAAGCGTATAAAACGATGGTGATA
CACGCCTGTCAAAAACTCGTCCTTGCTCATCTTTATAGACTGCAATAAATTCTAATTTAGAATATTTTCC
TGTAGATTGGCCTGAGATTAATTCCACTACCTCCACAGGAATTACTTTAGTTTCCATGTAAGTATTCTGG
CTGTTCCAAATGAATGGAATAATAGCAAAAACTAAAGCTGCGATAAAACAACGAAGGCTATATCTCATTA
TAATGTTCTCTCAAATTTGTGGAAGCATTTACGGCATTTCAATTTCAAGTTATCAGTGGTCCAGTTAACC
AGTTGGACTTGGATAGTTCCACATTCCGGATATGGATGGTTTGATGCCGATTTGGCAGCATTTTCACGTC
GTTCAACCATTTTCATTATTTCGTTCCATGCTGGAGGAACGAACTCTTCTTCACCATGAATTTTATCTCG
CTGAACAAATTCAATCTTTGAACTATCGTCAATAAACTTAACCAAGCCATGATTAGTGCTCATATCAATA
TCCTCTAAAAGACGTAGTTTCATTATAACACCTCAATGGCATTTCGTAAACCATCCGCTTTCGCATTCAG
AGCTTTTAACGATTTAGTTTGTTCTGCAATCTGGGCTTCGACTTCTTTCAGACGAGATTTGAGATATTCG
CGTTCTTCTCTTAAGTTATCAACCTTTTCTGCTTTAGGTTCTTTAATAGGCTTAAACACATTAGGGTTCT
TCAGTTTAATTTTAATTCGAGAACTGAATAACATTGAACCGTTATCTTCGGTTTCACTGTGCATTTCTAC
CACGTCCATGATTCTTAACATCATGCGAAGTTTATAAGACAGCGAACGAACGGTTTTATTGTGCTTCTTA
 
ATTTCTTCAGCTTTCATGGTGCCGAGATTACGTGAGTAATAATAAGGAACCTGAATATCAATTGATAATG
ATGCGGTACCATTACCGGTTGGTTTGAACACTGCAGTAACCCAATCTTTAACATGAACTTTATTTGTACG
ATCATCGCCGCCAGTGAATAATTTACAAGAATGTTTAAAAAGCATATTCACTTGGTTATTGAATTCACAA
GCAAATTTATCAGCAAATTTAGTTTCAGGAATGGTAGAGAACCAATCAGCTAAATGAGTCTTTTTAACAT
CATTAACAATAGTGAATTCAACCAAATCGGTTTTAGATGTCACAGATTTAATAAAGCTCGGCTTCATAAA
GTTTTTGATGTTATCACGAGCTACAGTAGAAGATAATAAACGAGAACGTTTGAACCATTGAAGCAGTGTC
CCTAATGAATGAGAATACGCATTCTGAGTATCTTTACAGATATGATTTTCTTGGCCCGTGTATGTACCAA
AATCGCGAAGAATACCACTTACTACATCTCGGTGTGGAACATCAAATCCTAGTGCACCAACAGCGCGCTG
GGCATGAACGTTAGCGATAAGACCGGCAAAGTATTCAATGTAATGTGAACGTGTCATAATGTTTTCCTCT
CGTTAAGATAGAAAGATTATACTCCAACCATCCTTGGTTGTAAACTGTTTTTATGATTTTAATGCATTCA
TTGCGTTAAAAAGTTTAGAATACTGCAACTGGAGATTCGTTACAACACTTTGAGCATCACGGAGCTTAGC
TTGTGCCTTGTCCAGTTCAACAGACAGTTGGTCCACGGGGGCTTTGATTTGGTTGTAATGGTAATCTTCG
GCCGGAAGAAGGCATTAGAACATTTAGTTTTACTGCCGACAGTTTGCACACATTTGGCAACCTTTAACAT
ACCAGGTTGTGCCGGAGCTGGAGAATATGCACCTAAGACTTCGGCATTACCAACTGGTGTTGCAGTAATT
GACGCTTCGAACGTTGCATTTTCCTTTTTAAGATTTTGATAGTATTCAGAGTCTTTGTGCCACCATGGCG
CAGGAACCTTTTTACCAATAGATACACGAGCCTGGATAGCAATATGACCCTTATTCTTGATGGGGTCGAT
AATATTAAGCTCACGAGAATAAATTGCTTTAATCTGGTGGATATTATCACCTAACACTTCCAACGCTTGG
TGGAATACACGAGCAACGTTATCATTCTTAGGTTTATACGTAAGAGTGAACTCATTCTCACTGATAACCA
TGATTTCCAACTTATCGTAAGTATATTTCAAATGTTTCATGTTAATTGCTTGAGGACGAATACGAGAAGC
AATGGCCAACAGATTGTTAAGGTTATTTCTGAACTGTTTAGCAAAATATTGTCCGTAGCCGCCGATACAA
AAATTACGAAGTTTCTGTTTGGCTAATAAACGGCTGAACTCACCATTTTTAATGAACTCTTTAACGGGTG
TGTTAGTAACGCATTCAAATTGAGTAAACTTACGAGAATTAATCCCATAATATGAACAGATTTCCTCAAT
CTCTTCAGGAGTCTTTACAGGCATACGGATAGGATTAGGTCCTGGAGTATATGCGTTCGAGGTAAATTCT
TTGATAAGTTCTCTACGTGTAATAGAAGCCATTTTATATTCCTCATGTTGAAAAATTTATTATACTCCAA
CCATCCTTGGTTGTACATCATTCCTTAAGAAGTTTTACTACTTGAATAAGATTAGCTCGTTCTGTTTTAT
TTTTACCGTTTAAGCCTGCTAATCGTGAGATTTCATCGTCAGTCTGTTGAATAGTTACATTAAGTTCGTT
AATCAAAGCTTCAAAGTATTTGATTTGCTCAGCATGTTTTGATTCAAATTTCTGAACCGGAATAACGTCT
TGGTTTAGTTCGTCAATAAGGTCTTTAAGAGCATCTTCTTGGTTGATATCATATTCAACTTCTTTTATTT
CTTCAACGAGCTCGTCTTTATATCTAACGTCTTTTAGTCCTGGTGCTGGTCCTGAAATCTCTTCTTTAAG
AGTACAAACATACCCAATACGGTATCCAATGCCGTCTCGCTTGATATCAACTTCGTCGATAAATCCACAA
TGTTTAAGACCAGAGCCGATTAGGTTAAAATATCCTTGACGTCGTCCTTCAAACGTTACACGAACTACTA
AGACGGTTTTGCCACCGACCTTTTGAAGCTCACATTTATAAAAGTCAGTTGAACCTGAATAACGTGGATG
AGTTTTAAAGTTCATCTGACGATCTAGGCCCATTTGTAAACGTGCTGATACGGCCAGAAAACGTGTTTGG
TTCTCGTTAAGATGAGCAACAATCTGTTTCAAAGGCATTTTACCCTGTTCACGTCCGAACAAATCAGTAC
GTCCTAGATTACGCAGGATGTTGTTAGAGTTAATCATCGACAGAACGTTAAGTTTATTCAGCGCGCTGAA
GATTCCATCAGCCATTGATTTTCCATAACCTTTACACGCTTTAATAAGTGCTCGCGGCAGATTAGTATAG
ATTTCAGACGGCATATGAAAATCAAATTTACTAGTCAGTAATTGATGAACTTGGATAGCAATTTGCTCAT
AATTAAGTGCTCTGCGATCGTTATGGTCTTTAAACCACACTTTAATCTGTGAAGCACATAAACCGTTAAT
TTCATGTTTTACCGCATCAGATGTAATGCTCATAACTATTTCCTCAGATTGAATTGGAACTCAAAACCAT
TATAACCCGTCCTTGGGTAAAGCGTTTATGCGTGAACCGTCTTTAAACGTTCTTCAAATTTCCGAAGAGT
ACTAATACGTTCTGCTTTCTGTTTTGTATAGTTTGCAATACGTTCTGAAGTGAGAGCGTATCGTTCGTTT
ACTGTTTCTTTCATGAAGTCAGGGATTTCACGAGAATCTCCAATTTCATCAAACTTATCGAGTACTGCCT
GGTCTTCAGCAATTAAATTTTCGTACATCTCGATATCTTTCTTAATAAACTCGATATCTTCCTGGGTTAC
TTTGGATAAACGAGATGCTTTATCCTTTTTGTGCTGTTCGTTAGTTTCACGAGACCAGAATAATGTACGT
 
TTTTTGTTAGTGTTTTTGTAGACTTCCACAATTCCAACTGAATCGATGATAACAATCCAATTCCAGCGAG
ATTTGAAAATCTGACCTCCGTCCACGGTAATCTCGTTACCATAAGCAACGTCATTAAAGAACTTGCTTTG
TGCTTCAGATTTAAATTTACCGTCGTTATAATTTACCAGGTTGAAAATATCTTTAGCGTTCATGTTTTAC
CTCCAGTAGTTGATAGGTCTATAATAACATAACCATAGACCTTGTAAACAACTAAAAGAAACTTTTTTCG
GTATTTTTTCCACTGAACCAAACTGACTTCGGGATTTTTTGTAATTTTTCAGATTTAAGTTTCTCTTCCT
GTTCTTTCTTATATACACCCTTGTCGTACTGGAGCATCTCTAGGGCATTCTTTTGCAAACGATCTAAGGT
AATTAAGGCTTTAGTTTTTCCTTTCAAGGAAGAGTCTTGGCGAACCTCCTCGCTATATTGTTCTGTTGCA
CTAATCATACGATGGGCATATAATTCACGACGGGCTTGGATATTCATAATTTCCTCATTTCAGTAAAGTA
ACTTCCGAGATTTCCTCCCAACCATCTTTATCCAGTTGAAATAAAATCTGCTACATAAAGAGCCGTACGT
AATGATACATTGCGAAGACGAGATACGTTAGCCTTCATCCAGGACAATGCTTTGTACGTTTCTGCATCCG
TCAGGCCACGTTTCTGCATCATGTCAGTAGATAATATTACATCTTCAACTCTGACCATAATCTCTTCATT
AGAGTGTACTCCAAGGTCCAAATACACTGAACGAGATACTAAAGCTTGAAGGTGTGGAGCAAGTTTCGAA
CCGCGTTCTAACTCTTTATCGATATCAACGTTTGTAATGAAAACGATAGTACCTTCAAACTCAAATTCTT
TATCGATACCCTTATCATCAAGATAAGATGATGCAGTGCTCCAGCAAACTTTGCGTTTATCACCAGTATC
TAATGCGGCTTTAAGAAGGTTCAAGATATCCATATCTGAGAATACGTCAACGTCGTCGATTAAGAGAACA
CTGTCTGCATCACGGTTGAGCCAAAGTTGTTCGTAAAGGCCAATACCAGAGATTTTGCCATTAATGCTTT
TGTATTTGATGAAACCATTATCATTAGCATTATTAAGCGCTTTATCCAGAGAATACGTCTTACCAATACC
TGCTGCACCTGAAATAATGAGTGAACGAATTTTGCCATCAATAATGCCATTCGTCATCATGTTCATTACT
TTAAAGCGTTTGTTGATACGAGTTTTCATATCTTCAGCGCTTTCAACAACTGCAACTGGTGATGTCTCAA
CTCCTTCCATTACGATGTCTGATTTGAAGACCCATACTCCACGTTCAGAACCATCAATCATACCGAAAAT
TTTACCGTCACCTAAGTGGGAACATTCAGGATTAAGCTTTGCCGGGAACCATGTCTTTGTGGCAACATAA
GAACCAGAGATTTCGTTATTACGATAGATACCTTTTTTGATTTGAACGTTGAACATTTTATTCTCCAAAT
TTACTCATTTCGTTTCGATAGGTCTATAATAACATGTTTAAAGCAAAAGTAAACAATTATTTGCGACGAG
ATTTTTTGGTAAGAGGTTGAAAGAACACTGCTTTACCAGCAGGAGTCAAAGTGACTCGTAATGCATTACC
GATTCCCAAGGATGCTTTAACCAGACCTTTAGATTTCAGTTCACCGAATGCTTGACGTTGGTCTGCATCA
CGAGTGTTAACCATAAGACCTTTATCACCAGCTTCACGAATCATTTCTAACATTTTGGTTTTCATTTTGT
TCTCCTCTTGTTTTGATAGGTCTATAATAACATATCCTAAAAGCATGTACACTACTTTTTAAAAATATTT
TGACCCCCTAAAGGGCCCGAAGGCCCTTATCATTTATAGTACGAATTGTGGTTTTCTAAGTAGACTTGTT
CAAGGAAGTCGTCCCAGAAACTCATATCTACTTTTTGCTGCATACCGTTCTTAGCAGCTTGTTCTGCCGC
ATGTTCAACTTGGTCAACGATATCTTCTAAGAATTCCTGTACGGTTTTAAACGTATGTTTACCAAGCTTA
ACATCGAGAATAAATGGAGCATCCTGAAGCGGGTAAACCAGGTCGCCGGTTTTGTAAATTTCTAAGAGCT
GAAGTCCACCACGGCATGCATGACTCAATGCTTTCCAGTCAATACCTTCGTTAGCTTCTGCCTTACGAGC
ACGTTCACCATAGTTCTCTTCCAGTTTAACCAGGATGGCATTGAACTCTTTATTGGTTAACGTATCTTGG
AATTTACGGTCTAACAGATTATAGAATGTTTGCTTACCTGTTTTCTCGTTTTCAGTTTCAACCCACGAAG
CAAATTCACCTACAGGCAGAACATTCTTCATATGTGATAAACGAATTACTGCACCATTATCAAAGTATTG
TCCCCATTCATTAGAACGTTTAAGGGCTTGACGAAGAACAGCCAGTCGAGAACCCTTGACACCGTATTTA
GAAGCTTGCTTACGGACATACCCTAAATAAGATTTCATGTTAGTGGTATAGAAACGAGACCTGTTATCTT
GAATATACTTCCATACATCAGGCAAATCAGATTTAACTACTAGACTTTGTGGAGTGTGAAGCATATCTAA
TGCGACGGTTTCACCATCTGCAGCTAGTTTAAAGAAATATTTGAGACTATACAGCTCATGGTCCACGTCG
TCTTTTGTGTTCTTAGAAGCAGTGTTATTCGTGTTCTTGCTCATGTGTTCTTTAACGTTACCGGTCAAGA
TATCACGAGCATGAGGGACATAAATTTCTTTAAAATCAACGTCAGATTCAGGAGTAGACGTTCCATAAAG
GTGGCTACCAAAGTAGCCTTTCATAATTGTTTTCATCATTTAGCCTTATATTCAAGAGTAGGACAGACTT
TTTCTTTACGAGCTTTAAGGTATTCACCTATTAATGATTTCTTACGTTCAATAGGCTCAAGTCCATTTTT
AATTCGTTCTTGGTTTTGTGCAATGAGCTCAATTTCTTTATAATATTTTCTGTCGTTATATTTGTCTAAC
 
AACCAAGACGGACCAATTACCAACCCGGTAGAAATAAAAGCCATGACTGCTACAATTAAAACGCCTACAC
CAATTGAGGCTGGAACTAATGTCCATAGACTGCTAAAAACAAAGAATTTAGTTAAAATTTCATGGCCAAT
GATTGTGACTGCTGCTAAAATCGCAAATCCAATAATCGAAACAGCCAATACCGGGAGAAATGTTTTCCAA
AAATAAGAACACAACGTTTTCGGGCGATTCCATGTACTATTGAATGCGGCATAAATTTTATAGTGCCAAG
AGTTTTCATTGATAATCATAATAATTCCTTATTTCAAAGTCGGGATAATAGAATCAAGATAAGCTTTAAG
AGCAACTGCTTCATCTTTGTCTAATGTAATGATATGTTGACGAAGGTCATCAATTTGGCGAATAACTACG
ACATCACCGTCTTCATAAGAAGGATTAACGCCTAAGACTGTTGTATAGTCAGGGCGGTCCTGATTTGTAA
TAAAAGCATTACGGCATTTAGCCCATTGAAGTTTGGCTTTTTTGACTGTTTCAGATTCCATGAATTCTTT
CACCTTAGTGACGAATGTTTTCCAATTATTAATAGAAAAACATTCGTCTTCAGTTTCGATATTAACCCCA
CTATAAAGTCCGTGATAAAAACGAACTTGAAGGTCTTCGTCAATGTTAATTACAGATGCATGGCCTGGTG
GATTGAGTTTCAAAATCTTGTCCAGTTCCATAGGAAGAATAGTAACAGCATCATCTTCTTGTGTCAGAAC
GTAAGCATAACCACGTTTTTCGATACATAAATCGCCAAACAACTCGGTTGTTTCAATTACAGTTTTCATA
TTATTCACCAATTAAAGTTTCAACAAAATTCACCAAATCATTCAGACAAGCATCAACCTGATTTTCATAA
AAGTCTTCGTTATCATTTACTGCATGATAAGCTACATCATTCAAATCTGATTCGGCCTCACGTAAGTGAG
GGATAAGTTCCATCAATTTATTAGCTTGTTCACGGGTCATTTTACCATACTCGCTTCAATAAGATTAATT
ACACCATTTACCATGTTATACGTAAACATCGGATAATTGTATTGTACTATAACATAAGCGATAAGCAAAA
GTTTCATTTTGTTCTCCATTTGTTTTGATAGGACTACTATAACATAGTCCTACCAAGATGTAAACTACCG
ATTGTGTTTTAAATGTTGATAAAGTTCTTCAACTTGTTTAGGGTCCAAAACAATTAAGTCATTTTCTTGG
CGAAGATAATTCCATCCCTTATCCCCAGGGCCATTTTTACAAAACATCACCGTCGTCATATCATCATCAG
CGCTTTCAGGTAAAAGTTTAAGTACACCGTCTTCAGATTTTCCAGTATAATCGTAAAGCATAATGTCCTC
ACATAATCTCGTTAACATACATTTGCAATTCGGTGTGGGCGTCGGCCATCTTTTTCATAGCTCTGTTAAT
GTCGATGTCTCCATCTTCGCAACCATGAGCATTTTCCCAATCGGATACAGAATTAGCATACTCTTCAATC
AAAAACATCAACTTAGAAATCTTTTCTTTACGTGTCATAACGTTCTCCTCTAGTAAGTAAGGACTACTAT
AACACAACCTGAGGAGAAGTAAACTGATAGATGCCAAAAAGCCCCAACCTTTCGGAAGGGGCTAGTAAAT
TAGATAGATGTAGGCTCTAATTTTATTTTCATGAACTCAGCAAATTTTCTGGCAGTTTTTATGCCTTGTT
CATGACATTCAAATGCATCATCACAGGGGCTATTTTTGTAATGCTTTGCCATATACTTATAAAATTTTTC
AGAATTCTCAACATTTTGAATTTCGAACAAAGTATATACGTTTTTAATCCCTGATTCCATAAAGTTATCA
AAGTACGGGTCAACATATGCAGTGCCAACAATAGGAGTCGTTGCTGCAAAAAACCCCAGCATCGCCACAA
TGGCTATCGCTCTTAAGGCCATATAGGCCTCCTGTTAATTTTGGCCAGTATTTATTTTTCGCCTTTGGAC
GGCCTTAACATCTGCGAGGTATTTTTCCAGCTTACGCTTACGAGTATAATCTGCTTTAGAACCAGTTTGA
CGGATATCCTCATTTAAATGAGAAATAGAATGTCCTAATTCTCTTCGGATTTCATCCAATTGGGTTACAG
TAAGATTACGAAGTTGTCTTTCGTTTAATTGCTGCATATATACCTCTTGGTTAGATATTAGTATTTATAA
CAACCCTGTCAAATCGTTCCAATGCTTCCGGCATAATGACACGTATCTATCGGTATCACCAATTTCAATT
TGACTGCCATCTGTAATTGGAAGTCCGTCTTCCGTGACTCTAGCTACCATTGTGGCCTTTTTACCGCAAT
GACAGACACCTTTAAGTTCAACAAGCTTATCGGCAATAGCCATTAATGCTTGAGAACCTTCAAATAGATT
ACCTTTAAAATCTGTTCGTAGTCCGTATGCCATTACCGGCACATTGTATACGTCAACAATACGACTAAGT
TGATACACCTTTTCAGTAGTTAAGAACTGAGCTTCGTCAACAAATACACAGTGAATATCCTTTTGTGCTG
CAGCCCATTTATAGAATTCAAAGATATCCATGTCTTTTGTAATTATATTGGCGTCCTGACGAATACCAAT
ACGAGAGACTATTTCACCTTTTGAATCGCGAGTATCAATAGCCGGTTTAAGAACTAATACACCCATGCCA
CGTTCTTTATAGTTATGTGCGGCAGTAAGTAATGAAGCAGACTTACCAGCGTTCATAGCGGCATATGTAA
AAATTAAACTAGCCATAAGAGTCCCTTATAGTGAATTCACGTAGTCAATGAGTTCTTGTTCTTTCCTGTC
AAATGATTCCATCAATGCGTGATATTCTTCATCTGTTAAAGGAGCATATTCATCGTACGCATTGGTGGCT
GCTTGTTCATCCAATAGGTCATGGATAAGTTCGAACAGTTTAGCTTTCTCTTCTTTAGTTAATTTCATTT
AGGTTCCCAATCAACAATCACAATTTCTAGTTTAGTGGAATACGTATCTAAAATCTCTTCGATGACATCC
 
CAGTTTCCGCCACCGATACCTGCACCAATTCTTGGCATATAAATTACAGGCTTTGTTATCTTATTCTTTC
CGAATTCATTTAATTCTATTATACAATTCATTAAAGCAGAGTACTCAAAATTAGGTCCCGGCTCAAACTG
CGTATAAAGATTAAAGCAATAAGCTTTATGGGTCTTAAAATATTTTTCATAAACCGAATAAGTCCCGAGT
TTAGACTCGTCTCCAAATTCGGTTTGAAGTTTATCGGCTTCCAGAATTTTTGGATAAGCCTTTGCTAATT
GTCCTGCCACACCACCGCCCATTGTATGAAAACAATTACATCCATGAGCAATATTATTACCTTTAGAAAA
CAGGGCGACAATATCGCCCTTGATATAGTTTTTAATCACTTGCTGCAAGTCCTCTAGTATAAGAGTCAAC
TAGACGGTCCACCATAGAATTGCATGCTGCCTTATCTTTAGGTTCCGCCACCGAGCATTCTAATATTTGC
CATTTTTTATAGTATCTTTTAATTAAGGTAGACGCCTTGAACCTACTAGACGCCCCATCTTTTTCTCCGT
CTTTATACGCATATAACAATTTTTGTGAAAAATCTACTTTACACTCTTGACTTTTACCGCAATAATCAGA
CGCCGTTCTGTTTGTATACTCCGTTACATCAGTATATGTAGTATTAGCAAGAGCGTTAAATGAAACCAAG
CCAACTATTAAAGCCAACATTTTGGTCATTCTTTTATTTCCAGTAGTTTATTTGATTTAAGGTAATTAGC
CTTTTCTAGGACTTCAGAAGCATATTTACTTCCAGCACTTGTCTTCCATCCCGCATTATATGAGGATATA
GCTTTTCTTATATCCCCGTTATGTATATTTAACCAATAAGAAAATTCGATATAAGCCCATCTGGCTGAAT
TAGACCGCTTAGATAGCATTTTCTTAATTTGGGCGTCAGTCATATTATAACCAAGCTGGTTAATTCTTGC
CCTCATTGTCGGTAGATAATTTTGGAACATTCCATACGCATGGTGACCTTTCTTATCTTTAAGATTAATA
CCGGCAGATGATTCCTGCCATAGTATTGCGGCCATTATGTATCCAAGTCCTTTCTTATGCATATTTTCAT
GCGTTTTAAATTTTCCGTCCTTAGAAAACTGTTCCCCAAACTGATAAGCATAATTCAAGTTATCGAGTTG
GACATTACTAAAAGTATGCTCGGAGCTATGTGCTGATAACGAAAGAGCAAATAGACCAGCGAGTAGTGCT
TTTCTCATGTTTACCTCTAGATTTTAATCACTGCTTTAGAAGCTTTTCCTGGTAGTCGTTTACTGTTAAT
AATAGCCATTCTGCATTGAAGAGTCTTCATTTTGAATGCTGGATTTTCGCGACAAACTGTAAGTCCTAAC
CAAAGATTTCCATCAGTTATTTCTAAACGGCCCGGGCGATATTCGACACCTTCGACAAAATCTTCATCAA
CATCTGGACGTGCCGGCATTGATAAAAATTCTGCAACTTCTTTAACATGATTTTTTACTTTATGAAATAA
TTCAAAAACGTAATTCTCATCTATTTCTCTCTGGATGGCACGATCTAAGAGATGTTGAGAATACTTGATA
TGAAAGCCTGGTAGTCCAAGAGACTTTGCAGCATCTTTTATACTGGCATTTATTTTGCGGAACTCCGTTT
CAAAGATTCGACGGAGTTTATTACGGCGGATAAAAACGTCTGAATTGATAGTCATTTTGATTTCTCCTCT
AGTTGACAGGTCTATAGTAACACAACTAGAGGAGAAGTAAACTACTTTATTGATAAAATGAGAGTTAAGG
CCCAAGATTCTTTAGATACTATATTGGCGAAGTGCCTCATCTCTTCGATTTGGCGACGACGCTGGTCAGC
ATAGTCTTCTTCGCCTTCTTCCTTACAATCTTCAAGGTCTTCTTCAAGATACTTTAAGTATTCACTACAA
ATTTTGTTGACCTTTTCCTGGAAGTCCAACAACCCAGTAATAGTAGAAGCCATTAGTATTTTTCCTCAGG
TCGAAATACTGCACGACATGCCCACATGCTGGCTTCTTTTAGATGTTGCTTAGCATACTTAAGAGCCTGC
ACTGCATCCATATCTTCTTTGGTTTGTTGCTCTGTTTCATTTTCTTTCAAGATATATGCTTCTTCAATCA
GAGCATCAAACATCAATCCAAGACGGACTTCTGCATCTTTAATAGCGTTCACTTTACCGATTTTATCATC
GGTATGCGGCTTGTAGCCTTTGATGTCTTCAATCGACATAAGGAATTTCCTCATCGGTGGCAAGGTCATA
TTCGACCAATTGGATAGGCTCGTTAATACCATATCCACGTACAGTGATATCGGTTGTCGGATATTTTCCA
GTAATATCACCCATTCTAAATGCTTCAGTGAATGATTGCTCGCCTGAGCAACTAAACCAGGCGGCAAAAC
ATTTTAAAACATCTTCAGACCCTTCTATAATCAATTTAGCCATTATAGACTCTCAGTAAAGGTACGAGCG
ATAACGTCGCGCTGCTGTTCCGGAGTCAGAGAGTTAAAGCGAACTGCATAACCGGATACACGGATGGTCA
GCTGCGGATATTTTTCCGGATGCTTAACTGCATCTTCCAGAGTTTCGCGACGCAGAATGTTAACGTTCAG
GTGTTGACCACCTTCAATTTTAACTTTAGGTTGATGTTCAATTTCAATTTCACGGGCATTCAAACCGTAG
AAAATTTCCGGGTCTACAATAGAGTCTTCTTTAAAAGTCTTAGAGACAATAATCCGGGCTTGAGTACCGT
CTTCAAAATAAATAGTGCCTTTATGTGCACCTTCAAGAATTTGATATGCTTTCATATAACCTCAATTAGA
AAATAAATTTATCCAAGTTTTTTCTTTAATCAAAAATGGGTCGGAATTAAACGCCATTAAACTTTGAGTG
ATTAATCCCTTAAATGGCCCATCAATAAATTCCATTTTGAAATGCGGAATACTAGTCATCAACCGTGCAT
TAGGAGCAGTACAAAGAACCTGCTGCCCTGCGAACGGACCTTTAGTCAAACGGTATCGTTTAGGTTTAAA
 
ATGGTAAATCTCGTAATTTTCTACAAGAGCCCTTTTATTGCATACTGGGCTGTTATAACAATATTCAAAA
ACTTCAATAGTGGTCATCATTGCATTCCAATCACAAAAAATTGAGGACGAGCAATACCGCCGATGCAATA
TTTGCTTTTGCAACAGTGTTGGACTGTTTTAATATGTATGTCGTAAATCTTATCCATATCAGGAGCTTTA
ATAGGCTCGTCAATTATATACAGAATACGAGAAAGCGTTAATCCACGGAATTTAGAACCTTCATTGCCAA
TAAAACTACGAACAGAATCAGTAAATAAACGGAATCGAATATCGTCGCTAGAATAACGAGAAAATTCTTT
TTCAATGTTGCCAGCAGAAATTTTAGCATAAGCTGAAGTATTAGAAAGAACAATAACTGTTCCACCATCA
TACAGCCAATTAGCAGCAAAGTTAGTCACAGCAATTGATTTACCTGATTGTCGGCCGCCGTCTAAACGAA
GTGTACAGTACTGCTTGAGTACGTCTTCAAATGGTGGGACATAACCGTTCTTACAAATTTCTTCTACTCT
AGCATCAGAATGGTGTGTAAAAGCATTCATCAGGGATAGATAAGGTCCAGTTAAAAATGTTCTCATATTA
TTTCTCTTAAGGTTTTGGCCATTCCGTGGCACATGTTAGGTCCTTAGACTCGCTATGTAAACCCATTACC
GCATTGCGCTCGACCTTATTACAGGTTGGAAAAGGTACATCACTCAGAGGACTCGACGTTCATAAGGAGA
GGTTGCGTTTCCCTTTATTAGGCAAGAATTATACTCTAGGCTTTTGAGCTTGTTTGGTATTGGATTCCCA
TGCGGCTTTGGTAATTTTGTTAGCGGCATAACCAAAGGCATTAGATAGAACACCTTTAGAGTTGAATACA
TCCAATCCAATAAGTACACCGGCTGTGTTAAATTTAGGAGCAAAGTACGCATTAGTTTTAGAAAGAGCTT
TTTTCAATTCAGGGTCTTGAACCAAGCCATCAAAAACGCCTTTACTATTTACTTTGTATGTTACTTTGGG
ATTTTTACCTTCTTCGATAGCAGCTTTCTGTTCCGCAGATAATGTAACTGCAGCGATAAACTCCTGATAT
GTTTTCATTTTGTTCCTATTGTTTTAATTTATGTTATTCTTTAGGGAAGTTTTTAGAGTATGCATCATAA
AGTTTATTACCAATACGACGGAAGACTTTCAGCTGCAAAACGGTTTTCAGGGTTTTTAATCATGATTTGC
ATCATGTAAGTTCCTGCATCCGAAAGAGCTTCTTCATAGTACCCATTAATTTCTTTAAGAGTCCTACGTT
TACCGCCAGGACCGTCAACAGTAATTCTCCATTTACCTGTAGAAGTGCGGCTCGGTGGCTCAACTTGTTC
AATCCAAATATAACCACCTTCAACGCGTTGGTTGGCGGATTCTGCAATAAATTCTTTATACGTTTTCATT
TTTGTTTCCTTGTTTGTTTTGATAGAGTAATTATACCATATTAATACTTAAGCGTAAACCGTTTAAGCTT
GACCGCCAGAAATAATTCCGAAGTTGTTAAAATCGTATCCAATATCGTCAACATCTACTTTAGGACTTAC
TACAACATAAGCAATTTCACTACGTTTTGCATAGGTGTTGATTTCTTTACCATCGCCACCTAAGAAAGAA
TACCCATTACGTTTTAATGAGCCAAAATCTTCCGCATCACCAATGAAAGCTTTAACTTTAAATTTAGGGT
CTTTGGCATCTGCTTCAATGATAAATTCTTGATACATTTTCATTTTTCCTTAGTAAATTGATTTACCATA
GTATTTGTACCAAGTCGGACGTTGAGCAATTTTTTCATCTAAGCGTGCTTGACTTATCGCAATGGACGCT
TCGGACGGGACATAATTATTACGAAATTCGGCAGGGATGTCGGAAATATCCTGGACAGTCGTGTCCTTGA
TTTTGAAACCACGTTTCAAGCATTCAGCGATTAGTTCGATTTGACGTAAACGTAAAAATTCGAGTTTGTC
GTAAAAGAATGTTACGTGACCTGTGCCTAAGATAAATGTTGGAGAGATTTTAAAATCCTTTACACGTTTA
CCGTTTTGAACATGTTTACGGACTGCACCAAATACGCGTGGTAATTCACGATACTCAGCCATAAGATGTT
GGTCGGTCAGTTCAGAAACAAGTGTTAAATTGATACGAGTCATAATTTCCTCCAAGTAGTTGATGAATGT
ATAGTATCAAAGTCCTTGGAGGAAGTAAACACTATTTTTCTGAAGCGCCTGAAATATATTTTTCAAGACG
TTGTTTCATTAGCGAGCCGTTCTTAGCGATTTCTTCCGGTTTATCGCACATACGCAACAAGGCTTCAAGC
TTAACGATATCACTGGTGCGCTCAAATTTGCGAGTATCTATCTTGCTAGCAAGTTCAGAAATACGTGCTA
ATAGACTTTCAGCTTCAACCAAAGCTGCACCTAGCTTCGTGGCAAATGTGTCAGTAGCTTCGTTAAGAGA
TAGATTCTGATAAAAATTCATGTATGTTTTCATATATACGCTTTCCAAGTTCCTGTTTTAAATGTTGAGA
TTACGCGTTTCGCGCGATTAGGTGTCTGACGATACCATTTAGATTGGGCTAGATTTACTGCCGCTTCGTC
CCAACGTTTCTGTTGAAGCATACGAAGAGAATTAGTAAAACCGGCGACGCCTGATACACCCATTTGGAAG
ACCATATTGACCAATGCACACCGACGAACTGCATCTAAAGAATCATATACCGGTTTAAGTTTAGCATTAC
CCAAGATTCCGCGAACGGCTTTATCAACGTCTTCATTAAATAGCTTTTCAGCCTCATCAAGGGTAATTGT
ACCGTTGCATTTACGGCCGATCATTCTGTCAAGTTCAGCTTTAGCTACGGCTAAAGACGGGTTCTTTGTT
ACTAAATGGCCTATGCCAATCGTCCAAAAGCCTTCAGTGTCTTTGTATAAAGTCAGTCTAAGACCTTCGT
CATTACGAAGCATTTCAAATATATTCATAATACCTCCCACGTTTATAGGAGGTATTTATTACTTATAGGA
 
GGGTAGTGAGCACCTTATATAATGATTTGCCCATTACATATTCCCATTGAGAAGGTTTAATTAAAGCGAA
TGCATCAAATTCAGGAATCTGTCGTCCGTCTGGGAATGTATGATAGGAATTACAAACACTGTTTCTGAAT
TGCTCGTGCTCTACAGGGATGGTATATAGGAACAGATGAATATTCTTATTGTCTGAGTAATGATGTTCAC
CTAGGTCTTTCAGGAAGGCAGGGTCATATTGTGTAAACCCAGTTTCTTCTTCGCATTCACGAATAGCGGC
TTCAATAGGAGATTCGCCAGGTTCGACACGGCCTTTAGGAATATCCCACCGATGAGCTAATGCACCTTTA
GGACGAGAACCAGTTACACGCCCCATGAATAATTCTTTATCTTCGGTAAGGAAAATAATACCGGCTGATA
GTTCTTTCACTTTATTTTTCTTACTCATCATCTCGGTCCCATGATTTGGCCATTAATTCGGACATAAATT
CTTCAATGCGCGTTTCTTTAGATGGACTTTCACTATTAGAGACCGCAAGTTTGTAATCTGCAATAGGACG
GAACAAATCAAGGTCCATATCAAGGTCGTCTTTGATTTGTTCTTCAGGTACATCTGTATCATACGTATAC
AGATAACTATCAAGTGATTCTGATTTAACGTAAATTGCTATACTCATCTCAAATTCCTGCATTCGGTAAT
AAAACGTTCAACTTCATTATTCTTGGCCTTTATCTTGTCTTTACGTTCCTGTAAACTACTTTTAACAAGA
TTAATAATGATGCCTACAGTACCTCCACCAATAATACCACCAATAATACTTACAATAGTAGATGCTAATA
AGATAGCGATAATATCTGGTACAGCAAAGGCACTTACTTTAGTACTAAAAAACCATACCGACGGTACAAA
TATAATCCATCCTGTTATCCATTGGCACTTATTGAAAAACTTTTCAAGCTCAGCTTGGGTTGTTCGTATT
ACTTCATCGATAGGTTGATATCCATTAATGTATCTGTTCACGATAATACCTGCTGGTTTGTCGATTTTAA
ATGCTTGACAATGGTGTTTCACTAAGTAGGATGGACATCCTGTCCAATCTACTGAATAGTATGAAATACC
TTCGTCGCCACCGTCCATGTTTGACCAGTGAATTTTCTGTATCATTACCAACCTTCCATAGGGAATTCCA
GATAAACTTGAGCGTTGACACGAATTTCATTGGTAATCTTTTTAACTCGGTCCGTATTACGAGATACACG
TCCGAACCAACGCCATCCATTACTAATTGCACGAGAACCAGTGTGCCATGTTTGCCAATCAAACTGAAGA
AGAGTACGGTCTGGAGCTTCCCATTTCTCTAGTTCGCCTTTTTCGATTTTTTCGAGGACTTCTTTGTGCC
ACTGGCGATAGATAAGTTCGCCATCAGGAATCTGACTGAACTCTGCAGTTCCTGTTGCAAAATGAGTAGG
ACACACATCAGCGTTAACAAGACCAAGAATATGTTCAGACAAATAGCGAGGATTGTCATAGTCAGGTGGT
CCTGCAGTAATAAAGTGTTGACCAACCGGAATATCTGGGCGTGGTACATCGTCATGGTGATAACCTGGGA
TAGCAGGATACCAACCAGGCATCAGCATGTGGACTCGTGAATCAAATACTACATCCGAGTCTTTCCAATC
GTCAGGGAGATTTTGGATAAAGCTACGAGTAATTGGACCGCCGTTATCAAAAGCAAAATTCAAATCGCAA
TTAAAGAACATAGGCTCGTTTTTAATCTGGTCGTTTTTAATCGCATGAGCAAAACCACCTACTGCACGAG
CTTGACTATTAAACACTTTAGAACTATTCATATTATTTTCCTCGACATTCTTTAATGAAATTTTCTAGAT
TGGTAGATTCAATAGCTTTTTTACGAGCTTCTGCGTGTTCTTCTTCAGACAATTTACGTTTATAATTGAC
CCAGACATTATGGTCGAAACTAGTCGTGGCATATAATAACAATGCTCCAACGAATAGCAAAAACGGCGAT
AAGAATACAAAACCAGGAACACCATCCATATCGTAAGCCTTTTGTAATACTGCAACAGAAAGGGCTGCGT
AAAAAAGTGATGCAAATATTGTTAAAAGACCAATTCGGTTGGTTTCGCCTAATTTCATAATATTCTCCTC
ATGTTTAATAGGACTACTATAATATAGTCCTATAATTGTGTAAACAGTTTTGCGAAATTATTTGAAAGGT
TTTACCATATGGAAATTTTGTTTGAAACGTTTGACGTAATCGTCATGTCCTAAACTTTGGTACATACTTA
CTACTTTGCTGTAGTTTTCCCAGGCATACTCACGGAACCAACCAGAGGTGATGCTTGTACAGACTTTTTC
CAATGCCATCATAAAACTGGCTTCAGGGTTAATTTTAAAGTCATTAGGAATTTGAGAACGTTCTAATGCT
AGTACACATGATTCTTCGTATACACCAGCCAATTTGACCATTTCTGGTAATGATTCGAACTTCTCGCGTG
AGGTCATGACTTCAGAGCCGTCTTTCATATAAAATGTATACGCTGGACGGTCTGTTAGTGCAATGGCTTC
ATGAATAGTATCATGGTCATATGTATAGATGTCATCTTTAAAGAACGCACTTTTAGAAGTGTCAAGAACC
GGGTGAGCATAGTTCAAGGTTTCTTTCTGGCGTTTCAGCATAATTTCAGTAAGTTCATCGTTCAGTGTAA
CACCTTTACCACGAAGATACTGAATATGGTTCATAGTCTTTAAAAAGAATGGATTATTTTTCTTGAAACG
ATGAGACATCTTAATTGCAAGACATATCTCAGGAGTTGCCCAGTAAAACCCGTTCAAACGGTCTTTAGTC
AAATTTTTCTTGGCGTATTTTAAAAGGTCATAAGAAGACGTGAAATCTTCAGGAGTGAGTTCACCGATTA
ATTCACGGACATTAGTGATTGCCGGAACAATATATGCTTCAAAATGAGTTTCGCGTCCATTATGGAAGCA
TTTGAAAACTTGGACATCAGGGTTATCTTGGTGAACATATACACCCATCATTTGATTTTTGAATGCATTC
 
CAAGAACATTGGTCCGCAATAAAGTCCCAATCGCTATTTTTGATAGCGCTAGAATCAATAAGACCGTGAT
AGTGAAGTGCACGAGAACCAATAACCAACATCATAATATTTTCCTCTGATTAATTCATTAACTTAAATTT
CACGACATGCGATATCATATTCATCCCAGAGGTCATCGTGTGAAGCCTCTACCAATGGAACGATTGTGCC
GTTTATGTCTTCATTATAAGGGCAAATTGTACAGTGCGCATCTTCGAATCGGATGCCTTTGTGTTCAGAT
TTTTTATAACGCATATCGAATATCTTTGCAAACTTTTCACGAGTAATGGTTTTAGCAACCACTTTGGTTC
CGATACTTTCGTAATAAATCATTGTGGACTCCATTTAATTTGATAGGACTACTATAACATAGCCCTATCT
TGTTGTAAACTGTTTTAGCAAGAATAACTAGATGATACCCAGCCTTGAACAGTAATACTATTATTTGATA
ACGAGATTGCGTCCTGGAGATTCAGACCTGCGTTATCTTCAATCCATAATGTAACTTCTTCGCGTTCTTC
TGTTGCAATTTGGAACAGGTTTTCCAGAGCAGCTACTGCCGTACGTACAGCACAATCTAATTCGCGAGAC
ATATTGTTTCCTTAGCAAGTGTGAGAAGATGCGACCCAACCACCAGTTTCGAGGCTCAGGTTGTAGTCGT
ATTGGTAAACGTCAGCCCATTCAGCATAGCCGTTTTTTGTACAACGTTCAAGATCGGCTTTAAGATGTCC
GGGTGAATGATAGGTTCCACCCATACCATAAGCTGGGTACAAGTCGAATGTTGTGTTTTGTTTATCAGCA
ATTTCTTTGGCAGCCTCAATAGCTGCATAGATTTTTGCTACAGCTTCAGTCAGTTCGGTTTGTTCTTTAG
ACATAATGATTCCTTAGCACAACGCAGGAGACGAAACCCAAGCACCTTCAGTAATAACGTAACCATTACC
TTCACGCTTAACATCGCCACCATTATTTTCAATTTCATTTTTCATACCACTGGGATACGATTTCACCCTT
AGGATAATAAGTACGACCGCTACCATAATCACCTACAGAAAATGATACGGCATATTGGTCTGCGATCTTT
GTTGCTTCTTCTTCGATACGTTCAGCTTGATTCAGAAGTTTATAAATTGCATCTGAAGCGGATTTGGCAT
CATTAAATTCTGGTACTTTAATTTCGATAATCTTACTCATTTTCTACATTCCTTAATAAAGTTTTCTATT
GTTTCATTTTCACGTTTAGCTAGGAGTTCATTATAACTAATCATTTGAGAAGCGTAAACCTTTTTATTTT
TCTTGTTATGGTTTATAAAATCGTAGTAAAAATAGACTACAGTACAAAATGATGCGATGGATGCAAAGGC
TTGGGTATACCGTAAGTCCTGCCATTGGTCTGTGAATCCGCCAGTGTTTCCTATAAATCCTAACACAACG
CCGTCAATAAGAAATCCAACTAATCCAAAGGCAAATCCCATGCCAATACTCATAATAAAAATTAGAATGT
TCTCACCAAGAGAAGGCCATACGGTTAAGAGTTGAGGCTTTTGCATCTTATTTCCTTAACGAATTCCATA
GCTTCTTCATATTCATACTTTTTAACAGCTTCATCATACTGAGCTTGGGCTTTTGCGCACTGAACTTTCC
ACTCACGAACTCGAGCTCTGTGATGACGGCCTTGGTGCCAATATCCTACCCAATTAATAGGCATTAATAA
AAACGGTACAATAAGTGGTGCGACGAACATTAATGAAAATGCTGCATCAGACGATATAACTTCACTAGAA
TCTAAAACAGCTCCTATAATCATAAAGATTAAAAATGCTAAAGCTAAAATAGGGCCAAATAGTACTTCAT
TAGGAATTAATTTACGTTTGTATTCGTATGCCCAGGGCTTACTTGGCATGTATAGGGTTGGCTTTGACAT
ATTCTCTACATTCCATAATAAATTGCTCAAGGATTAAATTGTTAGTGAAATTAGGACTAACAACCATAGT
TTCATAAAGCGCATCAATAGCACTTAAAGCATCAACTCTTTTAAGCAAAGGCTTGCCTTCATACATAGTT
TCAGAATCGATGATTAACGTAAAGTAATATTTTTTATTATCGACTAGAACTTCTGTATCGATATAAAACA
TATTAGACCTCAGAAGCTTATCTCTAAAACCATAATAATCTTCAGAGCGTTCGCTGATATATTCCCATTC
AGTGTTTAAACAACAAACAGACCATGTAGGAGCAACGAAATCAGTGAATCTAATATCATGGTCTGCAACT
AGTTTAGCAATAGCTCCGATATCATGAATCATAATACCTGGGGCTAATAAAGATTTTCTGCTATGGCTAG
CCGTTTTCTCGGTGGTCTCATAAGAAGACGACCAGTACCATTTACGTCCGGTTGCAACATATCGTAATGA
ATCAACTAAAGACGTAGGTGGATTTACCTTATCTCGGAACTCTTCAAACATTGAGTCAATATCATTATTT
TGGTTTCGCATGTTAACTCCTTTATTATCCAAAAGGAGGACCGGAGTCCTCCGTATTATTTTATTTCAAA
GAGTTAATATATTCTTGTACATCAGCAGAAGTGGTTTCGACACCAGCTGGAGTGCCATTGAACGTTTCAA
CACGAGTCAGAGTGTCTTCGATATCAACCTTAGTCAGTGCAGCGATTTCAATTACATCATCGGCCGTAGA
GATACCAAGGGCATTGGCTGCTCGAGTTTCACGAATGTATTCGAGTTTGATTGCCAGATTCTGACGAGCA
TCATCAAGTTCAACCACCTTACGAGCAATTTCAATTCGCATAGCGGCATACCCGTCAGCTTTTGTATTAA
GTTGTTCGGCAGTTCGACGATACAGAAGACCAAGCTTAGCATGCATGGTAACATCTTGTCCTTCAGCCAA
GAGCTTACGAATTTCACGTTCCTTAGAAGCGGCTTGCTTGTTCTTTTCATTAACAAGTTCGCGAATACGT
TTTTCTTCATTAATAGATTTAACAGAAGCCGTTTTTAGTTCTTTGATTTGGGTAATCAACTTATCTGCAG
 
CTGCGTTATACTGTTCTTCGACAGTCAGATTTTTAGCCATAGCAGTACCCAGTTTAGTGCGGATGAATTC
AACAAGTTTCTTCAGTGTGTTCATATTATTACCTTTCAATTGGTGGTTTATTATCCAACGAGAGCATTAT
ACTCTGCTCTCAAGAGTTTGTAAATTATTTTTTAATCAAATTGACATAAAACTTGGCGTCTTCGGGGTGC
ATGCCTTCGCAGTATTCTTCAACCATGAACTTCTTAACAGCTTCATAAGGACCACTCAGATTAAGCTCAG
TTGTCCAGAACTTGGAATCTTGGACTGAATCGATACGAACTTCAGGGTGTCGGTTGCGAATAACTTCTTC
GGTGTATTCATAATCAACGACATCAATACTAACTTTAGCCATTTTGTATTCCTCGTGTTTTGATAGGACT
ACTATAACATAAACTTACTTAGATGTAAATTATTTCAACGTCACCTTTAGCTGCTTTATAACCTTCAGCC
CAAGATACAACAGAGTTTTTATCTGAGGATTCAAATTCAGGCATTGGACTTTCACCAATATTCACACAGT
ATGTTTCTTCATTTTCATCGAACCAAATAATTAAAGTAGTCATTTTGTTTACTCCTCTGTAGTTGATAAG
TCTATAGTATCACCATCCTTGGTGCTTGTACATCATTATTTTTAATTTTTCCAACTTAACAACTTGTAGT
GTCCTTCATTCATACGTGGTTTATCCCAAGAAATACTAATATATGATTCACTGTCAATAGAAGTCGCCGG
GTTTTTCTCTACCGTAAGACCTTCACTTTCCAGCCATTCAATAGTATTTGCGCATAAATCAGAAGAACAC
CTAATCGGAAACCGGCAAGATTTTTCACCTTTAGCTGCAGCGTTATACATCTTTTCTAAAATACGCTTTT
GCACGTTTTTAAAAATAGTTTCAGCAGATTCTTCAGATTTTTTATTCAGTTCAGTATAAAGACTCATAAC
ATTCTTCTCATTAATAGGGCTCATAATATCTCAATCATAAGCCCATGTACGCGTTTATTTCATATTATCG
AAATATTCTTCAGCGATTTCGACATTATTATGGTAAACTTTAGAAGACAGTTTAACATAACCTTCAGCAG
TGAACATGTTAATCACAACCTTTACAGTATACCACTGACCATCTTCATTACCCATTACTGCGTAAGTTTC
AAACATCGGATGGTCAGGTCCAATAACTTTAATATCATTCACCGTACGACCGAAATCTTCTGGAACGCAT
TTCATAAAGAAGTTGAACAGTTCACCATAATTATCCATTTCATTCTCCAAGTTTTTTCTGTATCGGTAGT
TGATAGTTGTATAGTACCACGGAAGAACAGGGATGTAAACAGTTTTGTGAAAAAAATTTTTAAAAAGTTT
TGTGGAATTCTAGGGCAGGGAGAGGAAATTAAAAGGTAGGATAATATATTATAAAGGGTATAAACTAAAT
GATGCCTAGAGAGGCCCGGAAAGGCCTAGATACAAAAAAGCCCTATCATTTAGATAGGGATTTAAAATTA
TTTACCTAGTTTAGTTATTATAGCTTCGGCAGCAGCTTTTAGCTTAGATGCAGTGTTAATACGATTTTTA
ATTTCGGTATACGCATCGCCAAGAACATCAAGGCTATCAGAATAAACACTTACACTATTGCGTTCGCTAT
TTTGAATAGAACCTTTAGTTTTACGATCAAAATCAGTGTACACGTCTTGAAGACACGGGCTGCCTTCTTT
GCATCATCCAATGCATCAAGAGCTTTATTCAAAGCATTGACATATTTTTTAGTGTCAAACATATTCTTAG
AAATTTTAACAGGTGCACGTTCAGGAGTAGCTAAGGCGGATTTAGGAACCTGCGTGCCAGATGCCAGTTT
CTTAGAAATTTTGAAAAACGCTTTATTGAAACTATTTTCTTGACTCATCGTAAAAGAACTAGTATCGATG
TTATACATTTCAATAGCTTTAGCTTTCCACTTAAGAAAATCGCCACGGTCTTTTGGATCCAAGTGGAAGA
ATACTTTAGTAAGATCTGCTTCAGATGGTAATTTTGCTGCTTCGGTTAAAAATTCGGCATAGGTTTTCAT
TTAAAATCCTTGAAATAATTTATCGGTTGGTTATTAATTATTTATTACTTTGTTATTATCAGTAACAGCA
TTCTCTGAGTGATTCAAACTAAAAAGCCCCAACCTTTCGGAAGGGGCTAAGCCTCGCGGCAACCTTGTCG
GGGTTCCACCTGCTAAGGCAAGTGTTTGTACGAAACGCCGGGATTCGAACCCGGTTATTAAGCAGTTGAC
GCTACTCAATATTTTTAAAAGGCCATATCTCGACCATATCCGAACGTTCCGTCAAAAACGCTACTCGACT
TACGGCAAAGATATTTCCTCGAATCGATAATTCGGTGCGCCGTTTCTGCTGTGATGTAAGGGGATATTAA
CGAATCATAAAGATTTATTAAGACCAGTCCTTACTCAGGGAACATCAGTCCGACGACTTACCGGTAGCGA
CCCGGCTTCTTATTTTGACATATTATCAATTTGCTCTCGACGCCGTCGCCATAGATTAATCGCTTCATTA
ACACATTCGGCGTTTGGTATATGACGGCAATAATATAATGATTCGCGTTTCTGGAACCGATGTTGCAATG
GGATTTCGTTTAAAAGGTCTTTACGGTCAAAGTATTCAAAACTAGTTAACCCGGAACGGTTTTTAAGAAA
CTTCCAATGTCCGAATTCATTTTGAAAATAAACATAATCAGCATGTTGATATAATGAATTAATTAACCTA
TCGGAAATAACTGTTTCACGTTCGTTAAAATAAAATGTTAAATCATGAACTACTAAGTAAACATTGCCTT
TCATATTTTCCTCACTTAAAGTTGGTCGAGACAGAAGGATTCTAACCTTCAACCTACGGATTAGAAGTCC
CCGTTGCTCTATACAATTGAGCTATGCCTCGAATATATGACCCAGACCAGATTCGAACTGGTAACCTTTC
CCTTATGAGGGGACTGCTGCTAACCGTTGAGCTACAGGGCCTAAATAACTTTCTATTATGAAAGGCACTT
 
TAGAGTACCTTTGGTAATAGAGGGTATGAATTAATAATAACACATAATTCTTAAAGCAAATCAATCATTT
TAACAGTTGGCAAAACGATTTCTTCTTCAATATAAGCAAATTCAACACATTCAACTACACGCAAGAAATC
ATAATCATAATATTCAGAAGCATGACCAAGAATACCAATGAGACCTTCTGATACCAAATATCCATTCTCA
CGAAACAGGTCGAATACACACGATTCGTCTTCTTCAGATTTATTACCAAGGTCGACTTCAATATCAAAGA
CCTTTTTAATGAATTCTGGATAAAGCTGGTCTGAGAACAGTCCTTCAACCAAGTATTCATAGATTAAAAG
CGAGCAATCACTGAATGGTGTATTACCGCAATAACGTTCGGCTTTGGTGCTAGAATGACGGCTCTTAAAT
TTCTTCAAGAGATATTTAAACTGTTGGAGCTCATGCTCTTCCACTTCAAACAATGTTTTGGTTTTATAGT
TGTCACCATCATTTTCATAGGTAGTGACATCAATTACGTAACCCTTAGGAATTGTACTGCCTAAACAAAT
ATTCATTTACTCACCGTACCATATTAATGTAATTATATTCAATGCAACGTAATTTCAAAGGATTCTTGAA
AATATCATATTCAAGAGGGCCTTTTTCTGTTTCAATAAAGAAATCAAAGTTAACTGTGTTAAATTTATCG
TCTTCACAGAGTAATTTAACTTGCGAAGACGAACGGTCGATATAAACCTTTTCAACCTCAAAGTTAGTCA
GAATACCATAGTCATTAACTAGTGCTTTAGCTGCACGTTGGTCGCTTTCATATCCGTTCTCAATGGACGA
TACTTTAGCATAAAGAATCATGAATGTTCCACCACTTCAAAGGTATGAGCATCAACAATATGATACCAGT
CAAAATTTTCTTCTGCGTTTTTATAAATTTCGCGAAGAGCTTCAACAGTTTTTCCTGTAGCAACAAGATC
GTCAATACCGCCAAGAGGATAACAGTCGTAACCAGCAAACAGAAGGAAATTAATAGTTTCTGTAGGTTCT
GGATGCAGTGTACCATTGTCATCGACTACTTCAAAGAACATATAACCAGGCGATTTAGATTTAATCCAAG
CCCATGCTTGAGCTGCTGAATCAAATGGTTTGCGTATCAAACGACAATCAGGGTCTTTAGAAGGATTTGC
TTCAAAATCCGCATATACGTGATATTTCATTTTACTCACCTTTTGCAATTTTATCCATAACAATAGCGAT
TAAACCGATTAGAAATGCTACTACCAATGAGATAGAGTTTTCAGTTGTTGTCAGTAGTCCACAAATGAAT
CCTACAAACATCGAAAAACTAAAAGCCATTGAAGCAATTACTACAGCAACATTACGAATCAGTTCAGCGC
GTTTCATTTTATTTCTCCATTTACTCATTTTGTTTTGATAGGACTACTATAACATAGTCTTATCAGCATG
TAAACTACTTACAGAAGAGTTTTTGCAATAATTTTAAGCTGAGCTACAGAAATATGCTTAGCTTTAAACA
GTGCAGTCTCTTCAGCCGGAGAGATAACAATAGTAGCGCCATCTTTCTTAGCAGACCATCCATCCCCTAA
GTAAACAGTACCTTTTACTTCTTCGCCGTCAACCAGACTAATCATTGGTTTACCCTCACGTCCTTTATTT
GCTTTAATAACTTCGGAAGTAAGGGTTGCTTCGGTCAGAGTAGAAACGTTAGCAACAGGTGATATAAATT
CTTTATAGGTTTTCATTTTAGGTTCCATTTTGTTGTTTTGATAGGACTACTATAACATAGTCCTATCGTG
TTGTAAAATATTTTATGCCTTTAGATGAAATAGTATTCTGGCGTTTGACCCCTGTTTGTGAAAAATCATG
GTTATCAATTTGGTCAACTCGCATAACACCAAATGCAGATTTAATTTTAGCCTTTGAACCTTTTTTGATA
CTAATTACCATATAAGTATCATTTTTCTTATATTCGAAATTTGTTACTTCAACGCCTTGATTAGAAATTG
ACTTAATAATAGCATTAGCGTATTCATCATCTAACGGACCGTTAGTGTTAATAACAAAATTTTTATCTGC
TTTAGTAGCTTCCGCGATGAATTCTTGATAGGTTTTCATTTTATTTCCTTGAGGTTGTTTATTTGATAGG
ATGATAGTAACACACCATCATCCTTATGTAAACTGTTTTGTGAAAATTATTTTAGATCTTCGGCCCAATC
TAGTTTTAAGGGTTCTTTGTATTCACGGTCGAGAATAACCGGAATAGCTACATCACCACTGAATGATAGT
TGGCCAACGTTATATGATAACGTTATATGTGGAGTGTAGTCGTCGAAATCATGAGTAGCACCTAAAGCTC
GTGCGTACTGATGACGACATCTAAGATATTCGGAATCAAGAACTAAAACCAGTACAGCGCCGTCATCGGT
TTTCCATACCTCAAGGTGTCCTGATTCGGCAACAGAATAACTACCACTAGATGTAGTATATGGTATGTTT
ACACGTGAATAACAAATTGTTGAATGAAGCTTTTCACGTGGTACTGGATTAGGAATTCTTAATTTGCGCT
GGAGGTCTTCCAGCGCATCAAGTGTTAATTCCGAAAATTTAGCTGCGACGTAGAGGCCTTGTGAAAAGTC
CTCAAATTTCATTATTCTTCTTCTGTGGTTTCAGCTTTAACAAGTTCACGTACTGCTTCTACCAGGTCTT
CCAGTTTAACAGATTCACTTGTAATACCGACCAGTTGAGCAATCTGAGCTAGGGTATTCTGAAGAAGTTT
AGAATCGTCAGAAATACGAGCGGCGGCATCTTGGGTATCAAGAATACGAGATTTCAGAATTACGATTTCA
GACTGCAGTTTTTCAATAGTTTCAGTGGACATTTTATTACCTTAAGATTTTTTCAATTTTAGAATAAAGC
TCTTCTAACGAGCCGTCGTTTGTAATAACTAAGTCGCCTTCTTCAATAGGCAACCCAGCTTCTGTAATAT
GTTTATCAGTGGATTCCGTACCAGAGCGAACCACATGAATAACTGTAGCACCCATCGCTCTAGCGGTTTC
 
CATTTCATGGACTTGGCGTGTATCGGTCACAACGTAGTAATCGAAATCACTTCCAATATAATCCATATAG
TTTAATGCAAAAAGCTTAACCCAATACATACGGTCAAATAAATTAACGACAACATCCGTACCGAGGGCTT
GCATAAGACGGCGGATTGACCATGTGTCGTTGATATTATTTATAGCTTCACGCAGGGCTTCATATGGTTT
AATATCTAGATATGAATACCCACCTTCTAATGATGATAATATGTTAACACCATTAATTGGCAGGTATTGT
CGTTGTAAATAAATTAGCGTTTCTTCCATTAATTCAATAACATCCAATTTATTGAGATTTAATGGTGTTT
CACGGTCGTATCCAATGCCTTCAAAGTATTCATATTTCAACTCGGTGAATACATCAGGGTTTTCGGCATG
TTTACGCTCCCAAGCTATTGCTAGAGCGTCTTTAATTGGATATGCTAATTGATATTTTATGGATTTAAAG
TTTGAATTGATGTAATCAGCAGTAGTGTCTTTACCACTACGTTTGATGCCGCTTAAAAAGATTAGCTCCA
TAGGTTTTTCCTCATATAATTTATGAACGATTATAACATATGGAGCTAGAAGCGATTAGCGCAAGGAAGG
GTGTCTAGAAATGGATTTAGATTCGACCGCTTGACGCGGTTGTTGAGATACAATGACTTCACCATCCTTT
TCTATCGTCATATACGCATAGTGAATAGTTGCTACGCACGTAACGGCAGGGTCGGAGTCTTCAGTGTAAC
TAAATTCAATTTCGCTCAAATCGGAAATCCATGGTTTATAGAAATTTGCCGTCAAAACCACTTCAGTCTT
ATTGTTGTTTAAAATGTGCAAAGTAACAAACTCAGGACCATCGGCATGGGCTGTATTATGACCAGTTATA
TAGTTATTAGTGCTGAGCATCCATTTATACATGTTCATCCAGGACGTCATGTCTTCATCAACTATAAATC
GTACAATAAAAGGGTCATATTCAAATGTAGTGCCTGCACGCATTGCACGTCCTAACCCCATTGTGCCTGT
CACAGTTTCAACAACAGGAATTCTCACACCTGGCAATGGTGCCGTTTGGGCATTGAGCACAAAACCTGAA
GTTCGTGTACTATCTGGGATATCCACAACAAAGTTTGTTATATTTGTTTGGTTAAAAATCTGTCTCGTTG
GACGAGTCATAAGCACCTCAAAAACGCTTTAATAAAAATGCAGTATAATGGTTTTAGGTCTCTTCCATTA
ATCTGGTTCAACAGAATATCAGAAGAACGGAAGAGAATAGAATTAAGATTTCCAAACAGTATTCGCAGAG
AAACGTTTTCCTTTTGACATAAACTGCTGAGTAGGTAACATGATTATGTTAGCCCAGTCTTTAGGAGCTA
TTTCTGTTATTGTTCCTCTAATGTGGCCTGGCAAGTATGCTTTAATCATTTTGTCCGCACCTTGAAATCC
TTTCACCTTGCTCCAGTCTATCTTCAACTTTGTGTTATTAGTAATAGTAGTTGTGCTAGCATATTGCTTC
AGCAATTCTTCAAGAAACTGTTGTCGTGCTTTAGGTGGGATATAGTGCAAGTTTAACCCGTACATCAAAT
TATGTTTACCTAATCCTAAATAAATAATCAAAGGATATCGGTCCCAGTATGGCAGAGTATCTTTATGTTT
AGCATCATACACGTAAGTATAAATTTTGCCTGGCGCAGGCTTGACTACTTTATGACCTTTTACACTTTTC
TTAATTGTTTCAACAAACCACTTAGATGATTTGTTGTTTACTGCTGCGCCTTCATTTCTAATCTTTTCTC
GTATTGAACTGCGAAACGAGTTTATCATCAGAAGTTGACGTTCTTGCTTATTGAGTTTATTTTTAGGTTT
AGATTCAAGCGTTTCAATTTTCTGTGCAGTCTTAATAGATGATGCATATCTAGACATTGCAGATGTAAAC
GACGAGTATTTTATTCCTTTTGATTCTGCAAATGCTTTTGCAGTGATTCCTTTCTCTTTAGCTTTCTTGT
ATTCTAATCCTATCTCAATCCATTTTCTTTCATTCATAGACTGAGCAATCTTAGGGGCTTGTACGCCTTC
AGATATAATTTGGAATATGCTCATAATTACCCCTTGTATCCGAGTTTCCTAAGTCCGTCTTCGGTTAGAA
CTCGAAATTTTATATTTCTCTTTTCAGCAACAGCTGAGGCAGCTTTCCATTTGTCACAGTTCACAGACCA
CACGTAGATCTCATTCATATAACGTTTCTTTGCTGCGGTAGTAAGATTTGCAGGCTTAATAGGTGGTTGA
GTTTCTTTCTTTGGTTTTATTTCTACAAAGAACTCTTGACCTGATGCATCTTTCATCCATATATCCATAA
AGTATCGACGTTTTTTACCTTCTGCGTTACAGAAATATGGAATTACTGCTGTTTCGCTGCCCCATGCAAT
AATTTCTGGATTTTTATCTAACCATTCGAAAAAGAATTTTTCCCAGTTTGAACGATACGTTATTTTGGTA
TGGTCTCCACGGTATTTTTTTAAATTTTTTGGGACCCATTTACCAGAGTAGGCCATGTTGTCCTCCTTAT
AAATAGTATTATTATTTATACCAATTTCATATTGGAAGGAGACTATTTTGTTATTTACATTCTTCGACCC
AATAGGATATAGTGCTAAAACCATTAATAAGAATGCACCTACTATTCCTATGACAGACATATTTCGTAAC
TATAAAGAGTACTTTAAACGTGTTGCGACCAACTATAGATTACAAACTTATTATATTAAAGGTTCTCCGC
GTCCTGAAGAACTAGCAAACATAATTTATGGCAACCCTCAATTATACTGGGTCCTTTTGATGTGTAATGA
TAACTATGATCCGTATTATGGTTGGATCACATCTCAGGAAGCAGCGTATCAAGCTTCTATTCAGAAATAT
GCAAATGCCGGCGGAGACCAAATTCTTTATCATATTAACGAAAAACGCGAGAAATTTTATAACTTAGTTT
CATATCCAGATGAGCCATTAGTATGGTATGATAAAGGCGACAAAGCTCGTAAGTATCCGCAATATAAAGG
 
ACCTTTAGCTGCAGTAGATACTTATGAAGCGGCTGTACTAGATAACGAAAAACTTCGTAAAATAAAAATT
GTTGCAAAGGAAGATATAAACTCGTTTATCACTGATTTGATTCGTGAGATGGAGATTGCATAATGGAAAT
GATTAGTAGTAGCCTTAATTGGTTCGTTGGTGTTGTTGAAGACAGAATGGACCCATTGAAACAAGGGCGT
GTTCGAGTACGAGTCGTAGGCCTGCATCCAGCACAGAGAGCACAAGGTGATGTACAAGGTATTCCTACAG
AAAAACTTCCATGGATGACTGTTATTCAACCTATTACATCTGCATCAATGTCGGGTATTGGTGGTTCTGT
TACAGGCCCTGTTGAAGGAACTCGAGTATATGGTCATTTCTTAGACAAATGGAAAACAAACGGTATTGTA
CTCGGTACATATGGTGGAATTGTACGCGAAAAGCCTAATAGACTCGAAGGTTTTTCCGACCCGACAGGTC
AGTATCCGCGACGTTTAGGTAATGATACGAACGTATTAAACCAAGGTGGTGAAGCTGGCTATTATTCGAA
CTCTAACGTAATTCAAGATAACAACTTAGATTATGGTATAAACCCAGATGATACAGATTTAGCAAATATT
CCTGAAGATGATGACCCTAATTTTACAATAACAGAAATGTTACGTCGTGACGAAGGTCTTCGCGATAAAG
TATATTGGGACCATCTAGGGTATCCTACAGTAGGTATTGGACACCTTATCGTAATGGAAAAGACCCGAGA
CATGTCTCGAATTAATAAATTGTTATCCGATCAAGTAGGGCGTGAAGTAACAGGAAATCCCGGGACTATT
ACATTAGAAGAAGCAACAGCTCTATTTGAGAAGGACCTTGCTAAGATGCAGAAAGACATTCGTTCTAATT
CTAAAGTAGGTCCTGTTTATGCTAAAATGAACAGGTCTCGCCAAATGGCATTAGAAAACATGTGCTTCCA
AATGGGCGTTGGCGGTGTAGCAAAATTCAATACTATGCTTAAAGCAATGGCCACAGGCGATTGGAAAACT
GCATACAAATCTGGACGCGATTCGTTATGGTTCCAACAGACTAAAGGGCGTGCTTCACGTGTAACAACAA
TTATTTTAACTGGTAACATGGAATCATATGGCGTTCCTGTTAAAACACCACCTTCACCAGGAGTCGGTGC
CGATTTAGTTACTAGAAATACCGATCCAGAAGACCCGGCTGGTCCTCCTGTTCCATTAGATTCACGTATC
CTTTTTAAAGAACCGGAATCAAGTTATCGTGGTGAATACCCATATGTTCATGCTATGGAAACAGAAAGCG
GTCATATCCAAGAGTTTGACGACACTCCAGGTAACGAACGTTATCGCTTAGTTCATCCAACAGGGTCATA
TGAGGAAGTTTCTCCGTCTGGTCGTCGCACTCGTAAAACTGTAGAAGACCTTTTTGACATCACTAATGGC
GATGGGAATTTCCTGGTTTCTGGTGATAAACTTGTTAACGTTGGTGCTAACGAAATATATTATAACATGG
CGGACCGCCTCCATCAAATAGATGGCAGCGACACAATCTTTATTCGTGGCAACCAAGTTAAAACTGTTGA
AGGTGATGGAACTCTTTACGTTAAGGGCAATATCAAAATAGTCGTTGACGGGAATGCAGATATTCTTGTT
AAAGGTGATGCTAAGTCTCAAGTTGAAGGGAATCATGACTATACTGTCAATGGTAATGTTAAATGGACTG
TCAATGGTAATGTTGATATGACTGTTGCTGGCAATTGGTCGGAGACAATGACTACAATGAGCTCAATAGC
TTCAGGACAATATACTGTTGACGGATCTCGTATTGATATTGGGTAATATATGGCACAAATACTTCCTTTA
AATACCGACTTAGGAGAAGACATGGAAGGGGCGTCTATTGACGTCCTTTTTACTCCACAGTTAGAAACTA
CCGAAACTTTGGTGTCAATAAATATAATAGATTATGAACCAACACAAGGCATTACAGTTGACGGTAATCA
CTTATATGGAACATATGAAAGCGTATTTAGCTTTTCTTCAGATGCATTGAAATATCGATTAAACGATGAT
TTTAAAACTGCATCTTCATGGGAAGATTTACCGCAAGACCAAAGCACTCAACTATATTTGTGGAGAGCTC
CTCAGAACCTCCGTAAAGTATTTAGTTATACGGTTGAAATGATTTATAACTACCAAGAAGAAGGTTCATC
AGGCGGCTCAAGAAGTGACTCTAGTACAGAACCTCCGCCGGCTCCGGTACAAAAAACTCTTACTAAGGTT
TATACAAAAACTATAGTAGGAAATTGGAGCAAATGGGCTCAACAATTACGAAACTATGTTTATGCGAGGC
CATAAAAATGTCTGGCTTAAGTTACGACCAGTGCGTTACAACAGGTCATGAAGCATGGCCTCCTACAATA
ATTAATGCTTCTCAATCTAAAGTATTCACCGGCGGTATTCCTGTTCTTGTAGCAGGAGACCAAATAACAC
CTCATACAGAAATTAAAAAACCGTACGAGACTCATGGAGGTGTTACTGAACCTCGTACATCTAAAGTATA
TGTCACAGGTAAAAAGGCCGTTCAAATGGCTGACCCAATTTCATGCGGGGATACTGTTTCTCAGGCCTCG
TCAAAAGTCTTTATAAAATAGGAATTAAAATGGCAAACACTCCTGTAAATTATCAGTTAGTCAGAACGGC
GAATGCTATTCCCGAAATTTTTATCGGGGGCACATTTGCCGAAATTAAAACAAATATAATAGAATGGCTC
AATGGACAGAACGAATTTCTTGATTATGATTTTGAAGGTTCGCGTTTAAACGTATTGTGTGACCTTTTAG
CATATAATACTCTTTATATCCAACAGTTCAGTAATAGTGCAGTGTATGAAAGCTTTATGCGTACTGCTAA
CTTGCGCAGTTCCGTTGTACAAGCGGCTCAGGATAATGGATATCTGCCTGCATCTAAATCAGCAGCACAA
ACAGAAATTATGCTCACATGTACGGATGCATTAAATAGAAACTATATCACTATTCCACGTGGTACTCGTT
 
TCCTGGCGTATGCTAAAGGTACTTCTGTAAATCCATATAACTTTGTTTCCACCGAAGACGTCATTGTAGT
TAAAGACAAAAATAATCAATATTTCCCACGTCTTAGGTTAGCTCAGGGACGTATTGTTCGTACTGAATTA
ACATTTGATAAATTAAAACCTATCATTATTCGTGATAAGAACATCGACCGTAATTTGGTTAAATTGTATG
TTGACGGAGCAGAATGGATTAACTGGACTCGCAAATCGATGGTTCATGCTGGTTCTACTTCGACCATTTA
TTATATGCGAGAAACTGTTGACGGGAACACTGAATTTTATTTTGGTGAAGGTGAAATCTCTATTAGCACA
TCAGAAGGTGCTTTAACATCCAACTACATTGGTGGCCTTAAACCTGTCCAGGGTTCTAAAATCGTAATTG
AATATATTTCTACTAATGGTGCTGAAGCAAATGGTGCAGTAGGATTCTCTTATGCTGATACATTAGCTAA
TATCACAGTTATTGGAATTAATGAAAACCCAAGTAATAACCCTGACTTCGTTGGTGCGGATGGCGGTGGC
GACCCTGAAGATATTGAACGTATCCGTGAATTAGGTACAATTAAACGTGAAACTCAACAACGATGTGTGA
CTGCGACAGATTACGACACATTCGTCTCTGAACGATTTGGTTCTATTATTCAAGCGGTTCAGACATTTAC
GGATTCATCTAAGCCTGGGTACGCATTTATTGCTGCTAAACCTAAGTCTGGGTTATATTTGACTTCCGTC
CAACGAGATGATATTAAAAATTATCTTAATGAGTATAACTTAGGGACGATAACTCCTGTTGTTATTTCTC
CTGATTATTTGTTTATTAAAATGAATATTCGCGTTACGTATGCATTGAACAAATTACAAGAATCTGAACA
ATGGTTAGAAGGCCAAATTATTAATAAAATCGACCGGTATTATATTGAAGACGTGGAAATTTTTAACTCG
TCATTCGCAAAATCTAAAATGCTAACGTATGTCGATGACGCAGATATTTCTATCATTGGTTCGTCTGCGA
CTATTCAAATGGTTCGCGAGGTACAGAACTTCTACAAAACACCTGAAACAGGTATTAAATACAATAACCA
GATTAAAGATAGAACATTAGAATCTAACGTGTTTTCATTTGATAGTTTACGCGTTGACCCTGAAACGGAA
GCTACTATTAAATATGACGTTCGCATTGTAGGTTCAGATAGAAATGACCGTGGCATTGGACAAGTTATCA
TTGGTCCGTTTGCAGATGGCGACGTTATAGAAAATGCTTATATTCAACCATATACTGGTGACGATTTTAA
TAAACTTCATGTTACGGACGGAAGAAACAAATATTACTCTATAGGTGAAGTAAATTATCCGGCTGATTCA
ATTTATTGGAATATAGCTAAAATTGATTTAACTTCTGATAGGTTTGAAGTTCAAACTATTGAACTCTATT
CAGACCCAGCTGATGATGTTATCTTTACTAAAGACGGGTCATTAATTGTATTTGAAAATGACTTACGTCC
GCAATATTTAACCATAGATTTGGAGCCTATTTCACAATGACAGTAAAAGCGCCAGCAGTCACGAGTCTCA
GATTATCTAAATTGTCCGCAAACCAAGTTCAAATTCGCTGGGATGAGGTTGGCGCTAATTTCTACTATTT
TGTGGAAATAGCTGAGACCAAAACAGCTAATGGGGAAGCAATTCCCCGTAATAGCTATAGATGGACTAAT
TTAGGATATACCGCTGATAACAATTTCTTTGAGTCTGGTTTAAATTCATTAACAACATATATGATGCGTG
TGGCTACAGCAGCGGAGGGATTTGAGCAGTCTGATTGGAGATACACCGAAGAGTTTGAAACATTTGAAAT
AAACGCTTACACATTCCAACATATGATTGAAATGCAATTAGCTAACGAGTTTATTTCTGAAAAATTTACA
AAAAATAATACAAACTACGTTGATTTTAATAATGACACGATCATGGCAGCACTGATGAGCGAGTCGTTCC
AATTTAGTCCTGCATACACCGATGTGTCATCGATTAGAAATTTTATAATTGGTGAAAATCAGTATCATGA
AATCCAGGGGCATATCCAGGACGTATGTAAAGATATAAATCGAGTATATCTAATGGAATCTGAAGGAATT
CTTTACCTTTTTGAAAGATTCCAACCTATTGTAAAAGTGTCTAATGATAAAGGCCAAACATGGAAAGCGG
TTAAATTGCTTAATGACCGTGTAGGATATCCACTTTCACGAACTGTATATTACCAATCAGAATATACGAC
GTATTTGCTCGGCTATGATAAGATTTTTTACGGGCGTAAATCATCCGATATTCGTTGGTCTGCTGATGAT
GTTCGTTTCAGTTCACAAGATGTAACATTTGCTAAAATCGGGGACCGATTAAATTTAGGTTTTGATGTAG
AGATTTTCGGTACATACGCGTCATTACCAGGAAACGTATCACGTATTGCAGAAGCTATTACTTGTAATGA
TGACTATGTTTATATTGCGGCCAGAGATAAAGTTCGCTTCGTTAAAACCAGTAACGCTCCTATTGATTCA
GATCCATTATCTCCGACCTATTCCGAACGGTTGTTTGAACCTGAGACGTTTACAATAACAGGCAACCCTA
AAGCAGTTTGTTATAAAATGGATTCTGTTGGTGGAAGAATATTCGCTCTTATTATTGGTGAAGTCGAAAA
CGTTAATGACGACCCAAGAACAAAGCCTATTTTAGATTCCGTTGATAAAGGTGTATACGTTTTAGACCAT
GACGCTGGAACATTTAAAAGGATATTTGGTAATACCGAAGAAGAACGCCGTCGTATAGAACCGGCTTTCA
CCAATATGTCTACTGATGGTGTTGAGCTTTATATCTCTTCTAGTAATTTTAAATTTTTAGAATCTGATAT
TGTTGACGACCCAGAAACGCAATCTAAGTATGGACTTTTAGGAGCAGTTAAGTATGAATATCCTAGAGAA
TGGCTCGCAGATAAACATTACCATATGATGATTTTCGCATCAAACGAAGAAAGTAATTGGGAAACTTTTT
 
CACCAACTCCTATGCAGTATTATGCAGAACCTTTCTTTAGTTATTCTAGAAAATCAGGCACACGTTCTTG
GATTAATAACTCTGATAGAGCGGTAGTGATTTATTCTGATTTGCTTTACACAAAGGTAGTGGAAAGCCAC
CCTAGCACTTCACCTGATCGTAAAGTTCATGAATACTGGAATGATGGTGATTGTAAGATTGTTATGCCGA
ATATAGAATTCACTGGATTTAAAAAATACGCATCTGGTATGTTGTTTTATAAATCATCAGGTGAAATAAT
TTCATATTACGATTTTAGTTATCGTGTTAGAGATAACGTTTCTATAATTTGGAAGCCCACTAACGTATTT
TTGACCTCCTCATTACAAAACCAAGAAAAAGAAACTTCTTGGGTTCCTGTTGAAGAGACAGGTCTAGCTG
ACCCGGATTTACGCCCATTACTCACTACAATGATGCCAGAGTCTTATCTGTTGGATAATACAAACTTTGA
AGCATTTTGTGAAGCGTACATTCAATATCTTTCTGATGGATACGGGACTCATTATAATAACTTATTGAAT
TTGATTAAAAATAAATACCCACGAGAAGAACACGCATGGGAATATCTTTGGTCTGAAATTTATAAACGGA
ACATTTATTTGAATGCTGAGAAACGTGATTTAGTTTCTAGGTTCTTTGAGTCCAGAAGTTACGATTTTTA
TTCTACTAAAGGAACTGAAGCGTCGTATAAGTTTTTGTTTAAAGTTCTTTATAACGAAGACGTTGAAGTG
GAAATTGAGTCATCTGCTGGAACAGAGTACGATATCATAATTCAATCCGATTCTATAAGTGAAGATTTGG
TTGGACAAACTATATACACTGCTACAGGTAGATGTAATGTTACATATTTAGAAAGAAGCTATTCTAAAGG
TAAATTACAATGGACTGTGACTATTCATAACCTTTTAGGTAGGCTTTTAGTCGGTCAAGAAGTAAAAGCC
GAAAGACTAGCAGATTTTGAAGGTGAAATAGTTCGCGGTATTAAAGGTAAAGAACTTGCCCAAAACACTA
TCGATTATTTTAATCGTGGTAGAGCTTATTACGTCATGAAAATAAAGTCAAATCTTCCGTCCTCTCGTTG
GAAATCTGACGTACTACGTTTTGTACATCCGGTAGGATTTGGATTTATTGCAATAACCTTATTAACAATG
TTTATTAATACAGGATTAACGCTTAAACATGTTGAAACTATTATTAATAAATATAAAAATTATAAGTGGG
ATTCCGGACTGCCTACGGAATATGCAGACCGCGTCGCTCGTTTAGACCCTCAAGGTAATGTAGAATTCAA
TCCTGCTACAGGTGAAGTAATTTATGATGCCGGTCCTTATGCTGGTATCGAATACCCTTTACCTGCAAAT
TATAACGAAGAAAATGATAACTCTATTTTCCAAGGACAATTACCGTCCGAACGACGCAAGCCAATGAGCC
CATTATTTGATGCATCAGGTACAACGTTCTCTAGATTTAGAGAGCTTGTTAATGAAAGATTGTTAGATAA
CGTGGGAAATCCTAGAGACCCGATTAATCCACCACAGGTTAAATTAGATGAATGATTCAAGTGTTGTCTA
TCGTTCGATAGTTACTTCAAAATTTAGAACTGAAAAAATGTTGAATTTCTACAATTCAATAGGTGATGGT
GATAATAAAAACACCATCTTTATAACGTTTGGTCGGTCTGAACCATGGGCTGCTAATGAAAATGAGGTGG
GCTTCGCCCCACCGTATCCAACAGACTCGGTTTTAGGTGTAACAGATATGTGGACTAACATGATGGGTTT
AGTTAAAGTTATCCCATCTATGTTAGACTCGGTTATTCCTAGACGTGACTGGGGAGATATTCGTTATCCG
GACCCTTATACATTCAAAATAAATGATATAGTCGTATGTAATACAGCCCCATATAATGCCACTGAAGTCG
GAGCTGGATGGTTAGTATACCGTTGCGTAGATGTTCCTGATGTTGGGATGTGTTCTATTGAGTCTCTCGA
CAACAAAGAAGAATGTCTTAAATTAGGCGGTAAATGGACTCCATCTGTTAGGTCTTTAACCCCTCCTGAA
GGCCGTGGAGATGCTGAAGGGATTATCGAAGTTGGTGATGGATATATATGGGAGTATCTTTATGAGATTC
CACCTGATGTGTCTATAAACCGATGTACTAATGAATATATAGTCGTTCCATGGCCGGAAGAAGTTAAAGA
AGACCCTGCTCGTTGGGGATATGAAAACAATCTAACCTGGCAACAAGATGATTTTGGCCTTGTGTATAGA
GTTAAAGCAAACATAATTCGTTTTAAAGCCTATTTAGACTCAGTTTATTTCCCTAATGCAGCACTGCCTG
GTAATAAAGGATTTAGACAAATTTCTATTATCACAAACCCCTTGGAAGCTAAACTCCGTCCTAATGACCC
TAATATAAAGGCTGAAAAGGATTATTATGACCCCAGAGATTTACAGCGTCATTCTGGTGAAATGATTTAT
ATGGAAAACCGTCCACCTATTATTATGGCAATGGACCAAACCGAAGAGATCAATATTTTGTTTATGTTCT
AATTTAGGGAGACTTCGGTCTCCCTTTCGTGTATAAATAGTATAAACTATTAAGGATTAGCCACATGTAT
ATTCAAACTCCAAAACAATTGATTGACGTTGGCGAAATTGGTAACGCTTCTACAGGCGATATCCTTTTTG
ACGGCGGTGTTAAAATAAACAATGATATAAATGCTATTTACAATGCGTTTGGTGACCAACGAAAAATGGC
AACTGCTAATGGGACAGGACCAAATGGACAAATAATCCATGCCACTGGATACTACCAAAAAGGAGGCCCT
ACTGATTATTTCACCCCTGTTCCAGTAGGAAGTAGACACGACATAGATGCTTCTACCGGTGGTGTTATTG
TTACTGTTGCTAGAGGTGAACTCGGCGATTCCGTGGAATTTATAAATTCAAACGGGTCAATTTCGGTAAA
CAACCCATTAAGTATCCAAGCATTAGATTCTATTAAAGGTGTTGCAGGTAATTTAGTTATAACCACACCT
 
TATACTAAAGTTACTCTTCGTTGTATTTCTTCTGGTGCAGGTGGTTCAATATGGGATTATTCTACAGAAA
GTATGTTTAGTCATACCGAAATTCCTGTAGACGGGACATGGAATATTATTTCAGACTATGTCAATATTCC
TCTATTTTATAAAACTGAATATAATGCTGCTAAACTTTTGGTTACATGCCAATCTGCCAACGGCAGAAAA
ATTAAATCATGCGAAATAAATATTCTAATAGATACAATTAATTCAAGAGTTATTTCAACCGAATACGCAG
TGATGCGCGTTGGTAATGATAACGAAGAAGATGAAATTGCGAATATTAGTTTTTCGATAATTAACAATTT
TGCCACTATGACAGTTTCTTCACATATAAATGGTCTTCGAGTGGCAGCTAAAGTTATTTCAACTCAGAAA
ATTAGGGTCGCTCAATAATGAAACAAAATATTAAAATTGGGAACGTCGTTGATGACGGACAAGGCGATTA
CCTTCGTCGTGGTGGTGCAAAAATTAATGAAAACTTTGATGAGTTGTATTATGAATTAGGCGATGGCGAA
GTTCCATATTCCGCTGGTGCCTGGAAAACATACCATTCTTCAGATGGACTTAATTTAGCTGCTGAATGGG
GTAAATCATACGCAATTGATACTTCAACAGGGCGTGTTTCTATAAATTTACCTAAAGGAACAGTGGAAGA
TTATAATAAAGTAATTAGAGCCCGTGATGTATTCGCCACATGGAATATTAACCCTGTAACATTAATTGCT
GCCAGTGGTGATACTATTAAAGGTTCTTCTAGCCCAGTAGAAATCAATGTGCAGTTTTCAGACTTAGAAT
TAGTTTACTGTTCTCCTGGGCGCTGGGAATACGTTAAAAATAAACAAATAGACAAAATTACTAGTTCTGA
TATTAGTAGTGTAGCTCGTAAAGAATTTTTAGTCGAAGTTCAAGGTCAAACTGATTTTCTTGATGTATTT
GGGTCTGTTTCTTATAATGTGAATAATATCCGTGTAAAACATCGTGGTAACGAATTATACTACGGTGATG
CGTTCAGTGACAATAGTGACTTTGGTTCTCCAGGAGCTAATCCTGGAGAAATCGTTGCATTAGATGGTAA
AAACATCCGTTTAAGACAACCATGTAATATTGGCGACACCGTACAAATTGAAACCTTTTTGGATGGGATT
ACACAATTACGCAGTTCTTATTCTCGCCGCCAAATACGTATTTTAGATTCGAAATTAACAACCCGTCCTT
CCTTAGAAGGAAGTGTATATGTCGCTGATTTGTCAACTTTGAAATCTATTCCTTTTTCGGCATTTGGAGT
TAATCCTTCAGAACCGGTCAATACTAATTCTTTAGAAGTTCGTTTTAATGGTATTCTTCAAGAATTAGCC
GGTACTGTTGGTTTACCTATGTTCAGATGTGAAGGTGCTGATGCAGAAACTTCTACCGAGTGTTCAGCTT
TAGGTGGGACTTGGGTTACATCTAACACCGATTATTCTATTGAATATGATGATAACGATGCAACTATTGC
ACGTGCTTTAGTCTTTGACCGTAAGTTTGAAGACCAAGACATTATTGATATTACATGGTTTAATAACGAT
TTAGGCGCTTTATTAAGTATCGATGACATTTTAGATACAACTGATGAAAGGTATGTCGCACAAGGTGCAA
CCGTAAATGTGACCGGCGATGTGGCTTTGACCGATTTCAATAATATCGGATGGCCTAATGTAGAATCTGT
TCCGGCATATAGCAGGGAATTCTCATCTATTGCAAATATTTTTAACACTATTTACCCTGTCGGTACTATT
TACGAAAATGCAGTGAACCCTAATAACCCAGCCACGTATTTAGGATTTGGTTCTTGGAAATTATGGGGAC
AAGGAAAAGTATTAGTTGGATGGAACGACGATATTAGCGATCCTAATTTTGCATTAAATAATAACGACCT
TGATTCTAGCGGGAATCCTACACATACCGCCGGTGGAACAGTCGGAACAACGTCTAACACACTAACTAAT
TCTGATTTACCTCCTACTCAAACTGACGAGAAAGTTCTTATTTCCGATGAGAACGGTACAATTATTATTG
GTGGTTGTCAATACGACCCTGATGCAGAAGGTCCTATATACACCAAATACCGTGAAGGCCACGCCACAAC
GAATAGTACACACGTACCGCCTCAAGCCGTAAATAATATTCAACCGTCAATTACCGTATACCGTTGGGTA
AGGATTGCATAATGACCATACTTAATACTAAAGCGGGAGTAATATCCCGCGAAGCAGATTTTTTAGGATT
TAGAAAAAATCCTACTCAAATTGATATTTTAAACAACCAAGGCGTAGGTTCTGTAACCATTTCTCAATTG
GCAAAAGGATTCTACGATGATAACGTAGAATCTGCAATAAACGATGTTCATAATATTGCTAGAGCAGATG
TAGGAACAGTTACAATTAACACCTCAGGTGTATCTCCTGAAGGAACTTCACAGGTAGATTATTGGTCCTT
TAGTGGAAGTGTAACTGACCCTAGTTTGCCCGATGGAAGCCCTGTTATTGTAAAAGTTTACGGGCTTCCT
GTTAATGCTACTGTAGGTATGACAGCCGACGAATTTGTTGTACAATTACGTACTACTCTCCGGAATGCGA
TTTCCGACCAATTGGCTATTGCAGAGTATAAAGATGACCCTACTGTTGGGACTAAATTACAAATAACATA
TTTAGATAACCAAAGACACGTACTCCCAACGTATTCCTCTTATGGTATCACGGTTTCTCAAGAAATAGTG
TCTCAGGCTAAGTCCGGGTACGGCACTTGGAATTTACTAGGTGCGCAAACCATTACTCTTGACAGCCATA
CTTCACCTACAACCTTTTATTATTTTGTGAGAACCGCATGAGTAACAATACATATCAACACGTTTCGAAC
GAATCAGTATATGTTGAATTTGACCCTACCGGGTCAAATTTTGATAGTTCAATAACCAATGTCCAGGCAG
CCTTAGCCTCAATTAGCGCATATGGTGTTAAAGGTGTTCCGGACGCAAGCGAAGCAGAAAAAGGTGTAAT
 
TCAATTAGCGACAGAACAAGAAGTTCTTGATGGATTTAATAGTACTAAAGCCGTTACACCCGCCACATTA
AATGCAAGACTTCAGTATCCGAACGCGTCAGAAACACAGTACGGCGTAACTAAATATGCAACACAAGAGG
AGGCCATTGCTGGCACTTTAGACACTGTTTCTATTACTCCGCTTAAATTAAACCAAACTATTGATAACAC
GTTCTCTACTCGTTATTCAACAGAAACAACTAACGGTGTTATTAAAATTGCAACTCAAACCGCTGCACTT
GCCGGGTCTGATGACACCACAGCAATGACTCCGCTTAAAACGCAGCAATTAGCAATAAAATTAATTTCTC
AAATAGCTCCTAATAACGACCCGGCTTCAGAATCTATTACCGGTGTTGTGCGTTTAGCTACTGTGGCTCA
AACACGTCAAGGAACTCTTCGCGAAGGATATGCAATTTCCCCGTATACCTTTATGAATTCTGTTGCAACA
CAAGAATATAAAGGTGTTATACGTCTAGGAACACAAGCGGAAATTAATAGTAATTTGGGAGATGTTGCTG
TAACAGGTGAAACGCTAAATGGTAGAGGAGCTACAGGTTCTATGCGTGGAGTCGTAAAATTAACGACGCA
GGCCGGTGTTGCACCCGAAGGCGATAGCTCTGGAGCATTAGCATGGAACGCAGATGTAATTAATACACGC
GGTGGGCAAACTATTAATGGTTCTTTAAATTTAGATCACCTCACAGCAAATGGAATTTGGTCGCGTGGTG
GAATGTGGAAAAACGGTGACCAGCCAGTTGCAACAGAGAGATATGCATCTGAACGTGTTCCTGTTGGAAC
AATTCAAATGTTCGCAGGTGATAGCGCTCCTCCAGGTTGGGTTTTATGTCATGGTGGGACCATTTCTGGA
GACCAATTTCCGGACTATCGAAATGTGGTTGGAACAAGATTTGGCGGTGATTGGAATAATCCTGGAATTC
CGGATATGCGCGGCCTTTTTGTTAGAGGAGCTGGTACTGGCAGTCATATTTTAAATAATCGTGGCCAAGA
CGGATATGGAAAGGATAGATTAGGTGTAGGATGTGACGGCATGCATGTTGGTGGTGTCCAAGCACAACAA
ATGTCATATCATAAACATGCCGGTGGATGGGGCGAATTTCAACGTCATGAGGCACCGTTCGGAGCATCCG
TATATCAAGGTTATTTGGGTACTAGAAAATACTCTGATTGGGATAACGCATCATATTTCACAAATGACGG
CTTCGAATTAGGTGGTCACCGCGACGCAACAGGCACTCTTAACCGTGAAGGACTTATCGGATATGAGACT
CGTCCATGGAATATATCTTTAAATTATATTATTAAAGTCCATTACTAAGGGTAAAAAATGATCGAATTAA
AAAGTCTACCATATGTTGATGGCCCTCCTGACGAGGGTCAAAAACGTTTAAACTGGATTAAAAATTCAGA
AGAAATAACTGGCGCTGATACGTTATACGGTTCTGAAGGAGTAATGAACCGTCCAATAACAGAAGTTCAA
CGAAATGTAGAAACAATTAACGATAACGTTAAGACTATCGCAGAATCTTTAGATACCGCTAATGCTGATA
TTGTCACAATTAAAAGTATCTTGGATGTTTCTGGTGACGTGGATGCTTTAGCACAAATTGGACATAACAC
TGATGATATTGAAGTATTAAAACACACTGTAAATTCACACGGTGTTGATATTTTAAACACTGAAGAAAAA
CTAGATGATACTATTGCTAATATCGGAGTAGTTAATCCAGAAACAGATTCGGTGTATCGTACGGTGCGTA
ACGACCTTCTTTGGATTAAAACCGAATTAGGGCAATATACTGGACAAGATATTAACGGTGTCCCAACCGA
AGGTAATGAAAGCACCGGGATGAAACGACGTATTATTACTAACAGTTCAGTGTTGGTTGACCAAGGCGTT
CGTTTAACCGAGTTAGAAAATAAATTTGCCGATTCTGATGTTGGGGCTCTGACAACCGAAGTTGAAAACC
TCCGTCAAGAAATAGGTCCTAGACCATCGTTAACAGTACCGGTTTACACTCGTTTATCCGGTATTGATTC
ATCCATTTCTATCCAAACTAGAGATATTGCGGCATTAAAAGATTTTGTTGGATATCCTAATTCAACCGCT
ATTAAAACTCAAGTCGAGGCCAATCGTCTTTCTATATCAACAATAAATTCAGACATTAACTCGCCTGGCG
GTATTAAACCACGTTTGACAACGTTAGAAACTACTATAGGCTCTCCAGATTTACCAACTACTCTTCAGGG
TAAGATCAAATTAAATACCGATTCCATTTCAGGTATTAACACAGTTTTAGGTGTTGATTCTTCTAGTGGT
TTACGTTTTAACGTAGCATGGCTTAATCAGGTGGTTGGCGTTGATTCAAATGGTGGACAACCTGAACCGG
CTGGTTCACTTTTATATAGGACTCGTATTCTTGAAACTGGTGTTACTGACCTAGGCAATAACATTCAAAA
CGTTCAAACCGAATTGGGAACTAATTCGTCCGGTATTAAAGGACAAGTGACTAGTTTAAATAAACTTATA
AGCGGCACTAACCCAAACGGTCAAACCATAGAAGAACGTGGTATATTGCCAACAGTTAAAAACCATGACA
CATCAATTATGGCTTTAACTACGCGTGTTACCACTTTAGAAACCGACCTTGCTGCAGCAGAAGCAGAAAT
TCAGGCCCTTAAAGAGGCTGGATATATCGAAGATGCTCCTTCAGACGGTAAATTCTATGTTCGTAAAGAT
GGTGCTTGGGTTGAACTTCCTACAGCTTAATGAAAAGGGCCTTCGGGCCCTAAAGGATTTATATGTCAGG
TTATAATGCACAAAACCCTAAAGAATTAAAGGATGTAATATTACGTCGTTTAGGCGCTCCTATTGTAAAT
GTTGAATTAACTACAGACCAAATTTATGATTGTATTTCCAGAGCATTAGAACTTTATGGCGAATACCATT
TTGACGGGTTGAATAAAGGTTTCCATGTATTTTTTGTTGATGACGAAGAAAAATACCGTCACGGCGTATT
 
TGATATGCGTGGTTCTAATGTATTTGCTGTAACAAGAATTATAAGAACAAACGTTGGCTCTATTACTTCA
ATGGACGGTAATGCAACTTATCCTTGGTTCACTGATTTCCTTTTAGGAATGGCCGGTGTTTCTGGTGGTA
TGGGTAGTAATTGTGGTAAATTCTACGGTCCGAACGCTTTTGGTGCCGACCTCGGGTATTATTCTCAATT
AGTATCTTATATGGGTATGCTTCAAGATATGATTGCTCCTCTTCCGGATTTTTGGTTTAACTCTGCAAAC
GAGCAATTAAAAGTAATGGGTAACTTTAGACAGAAAGATGTCATTATTGTTGAATCATACGTTCGTTCTT
ATATTGAGACCCATAAGATGGTAGGAAATACAGTAGGTTATGGGCAGGTCGGTCCTCGTGATTCATGGTC
TATTTCTGAGCGGTACGACAACCCGGACCATAATTTGGTCGGACGACGAGTTGGTGAAGATCCTGCAACT
AAACAAGGCGCATATAACAACCGCTGGGTTAAAGATTATGCGTCTGCATTAGCTAAAGAGCTTAATGGAC
AAATTCTTGCTAAACATCAAGGTATGATGTTACCAGGCGGTGTCACTGTTGATGGTGTTCGATTAATCGA
AGAAGCTCGATTAGAGAAAGAAGCTTTACGAGAAGAGTTATATTTACTAGACCCTCCTGCAGGTATTTTG
GTAGGTTAATATGGCGACTTATGATAAAAATCTTTTCGCCAAACTAGAAAATCGTGGCGGGTATTCTCAG
ACAAATGAGACCGAAATCCTCAATCCATTTGTCAATTTTAATAACTATGAGAATAGTCAGACATTAGCTG
ATGTTCTAGTGGCAGAAAGCATACAAATGCGTGGTATTGAATGTTTTTATGTTCCACGTGAATATGTTGC
TGTGGATCTTATCTTTGGTGAGGACCTTAAAAATAAATTTACTAAAGCATGGAAATTTGCTGCGTATCTG
AACTCTTTTGAAGGTTATGAAGGAGCGAAATCGTTCTTCAGCAACTTCGGAATGCAAGTTCAAGATGAAG
TTACATTATCTATTAACCCAGGTCTTTTCAAACACCAGGTCAACAATCAAGAGCCTAAGGAAGGTGATTT
AATATACTTCCCAATGGACAACAGTTTGTTTGAGATTAACTGGGTTGAACCATACGACCCATTCTATCAA
GTAGGTAAAAATGCGATTCGTAAAATTACTGCAGGCAAGTTTATTTACTCTGGCGAAGAAATCAATCCGG
TGCTTCAGAAAAATGAAGGTATCAATATACCTGAATTTAGTGACTTGGAACTTAACCCTGTTAGAAATCT
TGACGGTATCCATGACATTAATATCGATGAATATTCTGAAGTTGAGCAAATCAATTCAGAGGCAAGTGAA
TACGTTGAACCATATGTAGTAGTTAACAACCGCGGTAGACAAAATTCACCATTTGACGATGGGTTTATGA
ATTAATAAATAAATAAGATAAAGCAAACACCGGTCGAAAGGCCGGTTAGGAGAAATGATGTTTGGTTATT
TTTACAACTCGTCGTTCAGGCGATATATCACAATGATGGGCGATTTGTTTTCAAATATCCAAGTTAACCG
TCAATTGTCTACAGGCAATAAATTAATTAGAGTTCCTATTACGTATGCATCTAAAGAGCACTTTATGATG
AAACTTAATAAATGGACCTCTATAAACTCGCAAGAAGATGTGGCTAAAGTCGAAACTATTCTACCACGTA
TAAATCTCCAAATGGTAGATTTCGTTTATAACCCAACGTTTAAAACGAATATATTGAATAACTCTCTTTT
GAGTAAATCGACTAAGGATATTGTAGACCAATACAACCCATCACCAATTAAAATGATTTTTGAACTGAGC
ATCTTTACTCGTTATGAAGATGATATGTTCCAAATCGTTGAGCAAATTATTCCATATTTTCAGCCGCATT
TTAATACGACTATGATTGAGCAATATGGACAGGATATTCCATTTGAAAGGGATATTAAAGTCGTATGGAT
GGCAGCAGCAATGGATGAACAAATAGACGGTGATAATTTATCTCGTCGACGTTTAGAGTGGTCATTAACA
TTTGAAGTAAATGGATGGATGTATCCTCCGGTAGGCGCAGCTGAAGGCCTTATTAAAACGACTTATCTTG
ATTTTCATGCTAATGAACGAGACCTTCAAACAGCCGCCAGTGTTTTTGAATCAGTTGATACAGAAATTAA
ACCGAGAGACGTTGAAGCACAAGATTGGAATGGTGAAGTTGAACAAACCTATACTCATGATATTCCAATC
CCGACTCCACCAACCCCTCCTGGTCCTAGAAAACAATAAGAGGTAAATATGGAAGGTCTAGATATTAATA
AGCTTTTAGATATCTCTGACCTCCCTGGAATATCTGGGGAGGAAGTAGAGGTATATGCTCCTTTACAATT
GGTGGAAGTTCAAAGTAATCCTCAAAACCGCACCCCGGATTTGGAAGATGACTATAGCGTTGTTCGTAAG
AATATGCACTTCCAACAACAAATGTTAATGGACGCTGCCAAGATTTTTCTTGAGACGGCTAAAAACGCCG
ATTCTCCTCGTCACATGGAAGTATTTGCAACTCTTATGGGGCAAATGACTACGACGAACAAAGAAATACT
GAAGCTTCATAAAGATATGAAAGAAATCACATCTGAACAAGTTGGCACTAAAAGTGCTGCTCCATCTAGT
CAGATGAATATCCAAAATGCTACAGTGTTCATGGGCTCTCCAACAGAATTAATGGAAGAAGTAGGAGATG
CTTACGAGGCTCAAGAAGCTCGTGAGAAGGTGATTAATGGAACAACCAATTAACGCATTGAATGATAATC
ACCCATTGAATGAAGGTGACAAAGTTGTTATTCTACCGCCTCATTTAGCTGAACGTAAAGAAGAAGATGG
TATCCATTGGATTAAATCACAATGGGACGGTAAATGGTACCCAGAAAAATTTAGTGACTATCTACGTATA
AACAAAATAGTTAAAATTCCTAATAACTCAGATAAGCCTGAATTATTTCAAACATACAAAGATAAGAATA
 
ATAAACGTACACGGTATATGGGTTTACCAAACCTTAAAAGAGCAAATATAAAAACTCAATGGACCTATGA
AATGGTCGCTGAGTGGAAAAAATGCCGCGATGACATTGTATATTTTGCTGAGACATATTGTGCTATTACC
CATATCGACTACGGTACAATTAAAGTACAACTTCGTGACTATCAACGCGATATGCTTAAAATAATGTCTT
CTAAGCGTATGACTGTTTGTAACTTGTCACGCCAGCTAGGCAAAACGACTGTAGTAGCTATTTTTCTTGC
CCATTTTGTTTGCTTTAACAAAGATAAAGCAGTAGGTATTCTTGCACATAAAGGCTCAATGTCGGCGGAA
GTATTAGACCGTACAAAACAAGCAATTGAATTACTCCCTGATTTTTTACAACCAGGTATTGTTGAATGGA
ACAAAGGGTCTATTCAGTTAGACAACGGTTCCTCAATCGGTGCTTACGCTTCATCACCTGATGCGGTTCG
TGGTAACTCGTTCGCTATGATTTATATTGATGAGTGCGCGTTTATCCCAAACTTCATTGATTCATGGCTT
GCTATTCAACCTGTTATTTCTTCAGGTCGTCGTTCAAAAATTATTATTACAACCACACCAAATGGATTAA
ATCACTTTTATGATATTTGGACTGCAGCTGTTGAAGGTAAATCAGGATTTGAACCTTATACTGCAATTTG
GAACTCAGTTAAAGAACGACTATACAACGATGAAGATATTTTTGATGATGGATGGCAGTGGTCTAAACAG
ACTATTTCAGCCTCTTCATTAACACAGTTCCGTCAGGAACACACGGCGGCTTTCGAAGGGACATCAGGCA
CACTTATTTCCGGTATGAAATTAGCTATATTAGATTATATTGAAGTTACACCTGATAGCCATGGATTTCA
CCAGTTCAAAAAGCCTGAGGAAGGTCATAAATATATTGCGACGTTAGACTGTTCTGAAGGACGTGGACAG
GATTATCATGCAATGCATATTATTGATGTTACGACAGATAAATGGGAACAAGTTGGTGTTCTACACTCTA
ATACTATTTCACACCTTATACTCCCAGATATTGTGTTTAAATATCTAATGGAATATAACGAATGTCCAAT
TTATATTGAATTGAACTCAACAGGTGTTTCTGTCGCTAAATCGCTTTATATGGACCTTGAATATGAAAAC
GTTATTTGCGATTCAATGAATGATTTAGGCATGAAGCAAAGCCGTAGAACTAAGCCTGTAGGTTGCTCTA
CATTAAAGGACCTTATTGAAAAAGACAAACTCAAAATAAACCATAGGGCTACTATTCAAGAATTCAGAAC
GTTTAGTGAAAAGGGTGTATCTTGGGCTGCTGAAGAAGGTTACCACGATGACTTAGTGATGGGTCTTGTT
ATTTTCGGATGGTTGTCAACGCAGCAGAAATTTGCAGACTATGCGGATAAAGATGACATGCGCCTTGCAT
CAGAAGTATTCTCAAGAGAATTACAGGATATGAATGACGACTACGCCCCGGTTATATTTGTTGATTGTGC
AAGTAATTCGGCAGAATATAATCCATCAGCACATGGCCTGTCAATGGTATAAATAAAATAAAGCAAATTA
AGAGGAATTAAAATGGCACTTCTCTCTCCGGGCGTTGAGCTCAAAGAAACTACCGTACAGAGTACGGTAG
TTAATAACTCCACTGGTACTGCTGCCCTGGCTGGAAAATTCCAATGGGGACCTGCATTCCAGATTAAACA
AATCACCGATGAAGTTGCGTTGGTTGATATGTTTGGTACTCCTAACACAGACACCGCAGATTATTTCATG
TCTGCTATGAACTTCCTTCAATATGGTAATGACCTTCGTGTTGTACGCGCTGTTGATCGTGATACTGCTA
AGAACTCATCTCCTGTGGCAGGAAACATTAATTTTACTATTTCTTCGGCTGGCACCAACTACAGAGTTGG
TGATAAAGTTGTAGTTAAATATTCTACAGATATTATCGAACCAGACGGTGAAGTTACTTCAGTTGATTCT
GACGGTAAAATTTTGAATATCTTTATTCCTTCAGGTAAAATTATCGCTAAAGCTAAAGAAATCGGCGAAT
ATCCTGAATTAGGTTCAAACTGGACTGCCGAAATGTCTGGGTCTTCTTCTGGTCTGTCTGCAGTAATTAC
TATTGATTCCGTTGTAATGGATTCTGGTATTCTGTTGACTGAAGTTGAAACTTCAGAAGAAGCTATTACT
TCTCTAACTTTCCAAGAATCTATTAAAAAATATGGTGTCCCGGGTGTAGTTGCTCTTTATCCAGGTGAAC
TTGGAGACCAGCTCGAAATTGAAATCGTATCTAAAGCAGACTATGACAAAGGTGCTTCAGCCCAGTTAAA
AATTTATCCTGATGGTGGCACTCGTTATTCTACTGCTAAAGCAATCTTTGGGTACGGTCCGCAGACTGAT
GACCAATATGCAATTATCGTTCGTCGCAACGACTCGGTAGTCCAGAGCGTAGTTCTTTCTACTAAACGTG
GTGAACGAGATATTTATGGTAGCAATATCTTCATTGATGATTTCTTTGCTAAAGGCGCAAGCAATTATAT
TTTCGCTACGGCACAAGGTTGGCCAAAAGGTTTCTCTGGCGTAATCAAACTGAATGGTGGCTTATCATCT
AATGAAACGGTTGAAGCCGGTGATTTAATGGAAGCTTGGGATTTGTTTGCTGACCGTGAATCGGTTAATG
CACAGTTGTTTATTGCTGGTTCTTGTGCTGGTGAATCTTTAGAAGTTGCTTCTACAGTTCAGAAACATGT
TGTAGCAATTGGTGATTCTCGTCAAGATTGCTTGGTTCTTTGCTCGCCTCCACGTGCAGCTGTAGTTGGT
ATTCCTGTTAATCGTGCAGTTGATAATCTGGTTGATTGGCGTACAGCGTCAGGAACTTACACTGATAATA
ACTTTAATATAAGTTCTACTTATGCTGCTATTGATGGTAACTATAAATATCAATATGACAAATATAACGA
TGTGAATCGTTGGGTTCCATTAGCTGCTGATATCGCCGGTCTTTGTGCTCGAACAGACAATATTTCTCAG
 
CCTTGGATGTCTCCAGCCGGTTATAACCGTGGTCAAATTCTTAACGTTATTAAGCTTGCAATTGAAACTC
GTCAAGCACAAAGAGATCGTTTGTATCAGGAAGCTATTAACCCTGTTACTGGTACCGGTGGTGATGGATA
TGTTCTGTATGGTGATAAAACTGCCACTTCTGTTCCTTCGCCATTTGACCGTATTAACGTACGTCGTTTG
TTTAACATGGTTAAAACGAACATTGGTTCGGCATCCAAATATCGCCTATTTGAATTGAACAACGCATTTA
CCCGTTCTTCTTTCCGTACAGAGACTTCTCAATATCTACAGGGTATTAAAGCTCTTGGTGGTGTATATAA
CTTTAAAGTTGTTTGTGATACTACCAATAACACCCCAGCAGTTATCGACCGTAATGAATTTGTAGCTACT
TTCTACTTACAACCTGCCCGCTCGATAAATTATATTACGTTGAACTTTGTAGCAACAGCAACTGGCGCTG
ATTTCGACGAACTGATTGGTGCTGTAGGTGGCTAATAAGACGCCTGATGGGCTATTTCGGTAGCCCATAT
AAATATACTATATCGTTTAAAAATTAATTCTATGGGCATCCGGTTAATCCTAAGCCCATATTACTACAGA
GGCTAATATGTTTGTAGATGACGTAACTCGTGCCTTCGAATCTGGTGATTTCGCTCGCCCTAACCTGTTC
GAAGTAGAAATCTCTTATCTTGGGCAAAACTTTAGCTTCCAGTGTCGTGCTACTGCTCTTCCTGCTGCGA
TCGTTGAAAAGGTTCCTGTTTCTTATATGAACCGTAAAATTAACGTGGCCGGTGACCGTACATTCGATGA
TTGGACTATTACAGTAATGAACGACGATGCTCATAGTATTCGTCAGAAATTCGTTGATTGGCAAGGTATT
GCAGCAGGACAAGGTAATGAAATTACCGGTGGTAAACCTGCTGAATATAAAAAGTCTGCTATCGTTCGTC
AATTTGCTCGTGATGCTAAAACTGTCACTAAAGAAGTTGAAATTGTTGGCTTGTGGCCTACTAACGTTGG
CGAAGTATCTCTTGACTGGGACAGCAATAACGAGATTGAAACGTTCGAAGTAACTCTTGCTCTTGATTGG
TGGGAATAATCTGGTTGGGGAGAAATCCCCAGCTTCCCGTGATGACGGAGAAGTCCATATAAATATAACT
ATAATTCCCATTTGGAGAATACAATGAAATTTAATATCTTAAGTTTGTTTGCTCCATGGGCAAAAATGGA
CGAACGTGATTACAAAGACCAAGAAAAAGAAAATTTAGAATCGATCACCTCACCAAAATTAGACGACGGC
GCTAAAGAATACGAAGTATCTGAAAATGAAGCACAGCAAACATATAATGCTATGTTTCAGAGAATGTTCG
GTAGCCAAGAACCAGGGCTCAAATCAACGCGTGAATTAATCGATACGTATCGAAATTTGATGACGAACTA
TGAAGTGGATAATGCAGTCTCTGAAATAGTTTCCGATGCTATCGTATATGAAGATGATACAGAAGTCGTT
TCTATAAATTTAGACAACACAAAATTTAGTCCAAATATCAAATCAATGATGTTGGATGAATTTAACGAAG
TGTTAAATCATTTATCTTTCCAACGTAAAGGTTCAGATCATTTTAGACGTTGGTATGTGGATTCTCGAAT
TTTCTTCCATAAAATTATTGACCCTAAACGCCCTAAAGAAGGTATTAAAGAGCTTCGTCGTTTAGACCCA
CGTCAAGTTCAATATGTCCGTGAAGTTATTACAACCACTGAAGCCGGTGTTAAAATAGTCAAGGGTTATA
AAGAATATTTCATTTATGACACATCACACGAATCTTATGCTTGTGATGGTCGCATATATGAAGCTGGCAC
AAAAATAAAAATCCCTAAAGCCGCGATTGTTTATGCCCATTCTGGTTTAGTTGATTGTTGTGGTAAAAAT
ATCATTGGTTATTTGCATCGGGCTATTAAGCCGGCGAACCAATTAAAACTTCTTGAAGATGCCGTCGTAA
TTTATCGTATTACTCGTGCTCCTGATCGTCGTGTATGGTATGTTGATACAGGTAATATGCCTTCAAGAAA
AGCAGCAGAACATATGCAACATGTTATGAATACGATGAAAAACCGTATTGCATATGATGCTACCACAGGC
AAGATTAAAAACCAACAGCATATTATGTCGATGACCGAAGATTATTGGTTACAACGACGTGATGGTAAAG
CAGTAACAGAAGTTGATACATTACCAGGTGCAGATAATACTGGAAATATGGAAGATGTACGTTGGTTCCG
TAATGCGCTTTATATGGCTTTACGTATTCCTATTACCCGTATTCCAAGTGACCAAGGTGGTATACAGTTT
GATGCTGGTACTTCTATTACACGAGATGAATTGTCATTTGGTAAATTTATTCGTGAGCTGCAACATAAAT
TTGAAGAGATATTCCTAGACCCGCTTAAAACTAATTTAATTCTTAAAGGAATTATTACAGAAGATGAGTG
GAACGATGAAATAAATAATATTAAGATAAAATTTCATCGGGATAGTTATTTCTCGGAATTAAAGGATGCT
GAGATTCTGGAGCGTCGAATTAATATGCTTCAGATGGCCGAACCATTTATTGGTAAATATATTTCTCACA
GAACAGCTATGAAAGATATTCTTCAGATGTCTGACGAGGAAATTGAACAAGAGGCTAAGCAAATTGAAGA
AGAGTCGAAAGAGGCTCGTTTCCAAGACCCCGACCAAGAACAAGAGGATTTTTAATGGACGATTTAATTC
AAGCTATTAAATCAAACGACCTCGTTGCTACTCGAAAGTTTTTTGAAAGTGCAATGGCAGAAAAAACGGT
TCGTTTGATTGAAGCACGCAAAGCAGAAATCGCTTCTCAATTTTTGATTGAAGGCGAAGAACCTGAAGAA
GAAAAGAAAGCTAAAGCTTCGGAAGACGACGCTGATGAAGGCGATGACGACGAAGACGAAGATGATGAGG
ACGATGAATAATGTATCTTATCCCTGAATCTTATGAATTGGTACTCGAAAATGTCGAAGCACTTATTCCT
 
GAAGCACAGGGCCGAATCGATGCATTGTCTTCTGCTCTCGATATTGACGATATAAATACTATTATCGAGA
ACATGCTTGAAACTGAAACGGATTTAGCTGTTGCAATGGCTTCCATTATTAATGAAGAACAGTTAAATGA
GTTTATCGTTAAACATGTTTCTTCTCGTGGTGAAGTCACCCGCACTAAAGACCGTAAAACTCGTGAACGT
AACGCGTTCCAAACAACCGGTCTTTCTAAAGCAAAACGTAGACAAATCGCTCGTAAGGTCGTTAAAGCTA
AAAAAGCTAACCCTTCAGGCCAAGTTAAAGGCTTGCGTAAGCGTAAAAAGGCTCTTAAACGTCGTAAAGC
ATTAGGATTAAGCTAATGAATAAACCCGAGTTACTTATTGAAACTTGGGGTCAACCCGGTGAGATTATTG
ATGGCGTTCCAATGTTGGAATCTCATGATGGAAAAGATTCTGGCTTAAAACCAGGGCTTTATATAGAAGG
TATTTTTCTTCAGGCAGAGGTTGTTAACCGAAATAAACGTTTATATCCTAAACGCGTTTTGGAAAAAGCA
GTAAGTGACTATATTAAAGAACAAGTCGCAACCAAACAAGCCCTTGGAGAATTAAACCATCCACCTCGCG
CAAATGTTGACCCTATGCAAGCCGCTATCATTATTGAAGATATGTGGTGGAAAGGAAACGATGTATATGG
ACGCGCTAGAATTATTGAAGGCGACCATGGTCCCGGCGATAAATTAGCTGCTAATATACGAGCTGGTTGG
ATTCCAGGTGTTTCCTCTCGTGGTCTTGGTTCTTTAACCGACACCAATAAAGGGTACCGTATCGTGAACG
AAGGATTTAAATTAACCGTCGGCGTAGATGCCGTATGGGGACCTTCTGCTCCAGATGCTTGGGTTACTCC
TAAACAAATATCAGAATCAGAAAATTCGGTGGAAATCACCAAAAACAGTGCTGACGAAGCATTTAAAGCT
CTCGCAGAGAGTTTAAAAGCATTATAAATAATAATGTAATCTAAACCACAGGACACTAAAATGATCAAAA
GAACAATTTTGAAAGAAGCTCAGAATATCGAGACTTCAGTTGCTCTTAATAGTGTTTTCGAATCAGTAGA
ACTGTCTCCGGACGTTAAAGCTAATTTCAGCACTGTATTCGAAGCCACCGTTAAGCAGGAAGCAGTCAAA
CTGGCTGAATCTCATATCAAAGCCATCGCCGAAAAAGCCGAAGAAGTTGAAAAGGCTAAAGAAGATG
CTGAAGAGAAAGCTGATAAAAAATTGGCTGAACAAGCTTCTAAATTCCTGGACCATCTTGCAAAAGAATG
GCTCGCAGAAAACCAAATCGCAGTTGATAAAGGCATTAAAGCTGATTTGTTCGAATCCATGCTTGGTGGT
CTGAAAGAACTGTTTGTTGAACATAACGTTGTTGTTCCAGAAGAATCTGTAGATGTTGTAGCAGAAATGG
AAGAAGAGCTGGCCGAACATAAAGAAGAAACCGCTCGTTTGTTCGAAGAAGTTACCAAACGTGATGCATA
TATCAATTACGTTCAGCGTGAAACCGCTATTAATGAAAGCGTGAAAGACCTGACTGAATCTCAGAAAGAA
AAAGTTATTGGCCTGGTTGAAGGTATGGATTATTCTGACGCATTTGGTACTAAGTTAACTGCTATTGTAG
AAATGGTTAAAGGTTCTACTAAAGAAGAAGCCGCTATTACCGAAAGTATAAATACAGTTGACAATGACGC
TGCCGGTCTTAATTTTGTTGCCGAAGCAGTTGACACTACCACAACTCAGGTAGAACAGAATTCTAATGTA
AGTTTATATGCGAAAGTCGCATCTCGTTTCTAATTTAAAGGTTAACACAAATGACTACTATCAAAACTAA
AGCTCAGCTCGTAGACAAATGGAAAGAACTGCTGGAAGGTGAAGGCCTGCCGGAAATCGCTAATAGCAAG
CAGGCTATTATCGCTAAAATCTTCGAAAACCAGGAAAAAGATTTCGAAGTATCCCCTGAATATAAAGACG
AGAAAATCGCTCAGGCATTTGGTTCTTTCTTGACCGAAGCTGAAATTGGTGGTGACCACGGTTATAATGC
TCAGAACATCGCAGCAGGTCAAACCTCTGGTGCAGTAACCCAGATTGGGCCGGCAGTTATGGGTATGGTT
CGTCGTGCTATTCCTAATCTGATCGCTTTTGATATTTGTGGTGTTCAGCCTATGAACAGCCCTACCGGTC
AGGTATTTGCTCTCCGTGCAGTATATGGTAAAGACCCTATCGCTGCTGGCGCTAAAGAAGCTTTCCATCC
GATGTATGCTCCGGATGCAATGTTCTCTGGTCAGGGCGCTGCTAAGAAATTCCCAGCGCTGGCTGCTAGC
ACACAAACTAAAGTAGGTGATATCTATACTCACTTCTTCCAGGAAACTGGTACTGTATATCTGCAAGCTT
CTGCTCAAGTAACAATTAGTTCAAGTGCTGATGATGCAGCTAAATTAGATGCAGAAATTATCAAACAAAT
GGAAGCTGGCGCACTGGTAGAAATCGCTGAAGGTATGGCTACTTCTATCGCTGAACTTCAGGAAGGCTTC
AATGGTTCTACCGACAACCCGTGGAACGAAATGGGCTTCCGTATCGACAAACAAGTTATTGAAGCTAAAT
CTCGTCAGCTGAAAGCAGCTTACTCCATCGAATTGGCACAAGACCTCCGTGCAGTACACGGTATGGATGC
TGATGCTGAACTGAGCGGTATTCTTGCTACTGAAATTATGCTGGAAATCAACCGTGAAGTTGTTGACTGG
ATTAACTACTCCGCTCAGGTTGGTAAATCTGGTATGACTAATATCGTTGGTTCTAAAGCTGGTGTATTTG
ACTTCCAAGACCCGATTGATATTCGTGGTGCTCGTTGGGCCGGTGAAAGCTTTAAAGCTCTGTTGTTCCA
GATTGATAAAGAAGCAGTTGAAATTGCTCGTCAAACCGGTCGTGGTGAAGGCAACTTTATTATCGCTTCC
CGTAACGTAGTTAACGTACTGGCATCAGTCGATACTGGTATTTCTTACGCTGCACAGGGTCTGGCTTCTG
 
GTTTTAATACCGACACTACTAAGTCTGTATTTGCCGGTGTACTTGGTGGTAAATACCGCGTATACATCGA
CCAGTATGCTAAACAGGATTACTTCACTGTTGGTTATAAAGGCGCTAACGAAATGGATGCAGGTATTTAC
TACGCTCCTTACGTTGCACTTACCCCACTGCGTGGTTCCGATCCTAAGAACTTCCAGCCGGTAATGGGCT
TTAAAACTCGTTATGGTATCGGTGTTAACCCGTTTGCTGAAAGTTCCCTGCAGGCTCCAGGTGCTCGCAT
CCAGTCCGGTATGCCGTCTATCCTGAACAGCCTTGGTAAGAACGCTTACTTCCGCCGTGTATATGTAAAA
GGCATCTAATCTTAATTGATTAAATCTTATTTTGGGAGACTTCGGTCTCCCATTTTTGGTTTTATTTCTG
CGCAATTTCGATGTAAGTTTTCAAAATATTTTGCCAATTAGGCATACCTTCTGAACATATGCCGGAGAAC
CGTCTTTATTTTTGAATGATGCAAGTTTATCAGTGGCCCAATAACCTTTAGTTCGAGCTTCAGAATCAAC
TTTCATCCATACTTTGTTGAAGGCATTACCATTAACTTTTGCACCATATTTTTGTACTTTCGGTTACAGA
TCCAGCTTCCAGAACTTTTACAGTATCTCGTAAGAAACGTGCTTTAGTCATTTTGTTTACTCCTCTGCAG
CTGATAAGTCGATAGTATCACATACCAAATACGTTGTAAACAATCTTTATAAATAATCTATATCACATAA
GGGAAAAATGCAATGAGTAAAATCCAAAAATTATCGCGTGAATCTACAACGTCTACCAGCAACTCAATTG
GTCGCCCAAATCTCGTTGCTTTGACTCGCGCTACGACTAAATTAATATATTCTGACATCGTAGCAACACA
AAGAACTAATCAACCTGTTGCTGCTTTTTATGGTATCAAATATCTTAACCCAGATAACGAATTCACATTT
AAAACTGGTGCTACTTATGCTGGCGAAGCTGGATATGTAGACCGAGAAAAAATTACTGAGCTCACAGAAG
AATCAAAAGCCACACTAAATAAAGGCGATTTTTTCAAATATAATAATATCGTCTATAAAGTGTTAGAAGA
TACACCATTTGCAGATATTCAAGAAAGTGATCTAGAATTAGCTCTTCAGATTGCAGTCGTTCATTTAAAG
GTACGGTTGTTTTCAGATGCAGCGGTAACAAGCAAATTTGAAAGCTCTGGTAGCGAAATTTCTGATGCAA
GATTCCAGATTAATAAATGGCAAACTTCAGTCAAATCTCGTAAACTTAAAACCGGCCTCACGGTTGAATT
AGCGCAAGATTTGGAAGCGAATGGATTTGATGCCCCTAACTTCTTAGAAGATTTGCTTGCAACTGAAATG
GCTGATGAAATTAATAAAGATATTCTTCAGTCTTTAATTACTGTATCGAAACGTTATAAAGTTACTGGAA
TTACTGACACCGGATTTATTGACTTGAGCTATGCATCCGCTCCAGAAGCAGGTCGTTCGTTATATCGAAT
GGTGTGTGAAATGGTATCACACATTCAAAAAGAGTCAACTTATACTGCGACGTTTTGTGTAGCTTCAGCT
CGTGCAGCTGCTATTCTTGCTGCTTCCGGTTGGTTAAAACATAAACCAGAAGATGATAAGTATCTTTCAC
AAAATGCTTACGGGTTCTTAGCTAATGGTTTACCGCTTTATTGCGATACTAACAGTCCATTAGATTATGT
GATCGTTGGCGTAGTAGAAAATATCGGTGAAAAAGAAATTGTTGGATCAATTTTCTATGCTCCGTATACA
GAAGGTCTCGACTTAGATGACCCGGAACATGTTGGTGCATTTAAAGTTGTAGTTGATCCAGAAAGCTTGC
AACCGTCTATTGGTTTATTGGTTAGATACGCTTTATCAGCAAATCCTTACACAGTAGCAAAAGACGAAAA
AGAAGCGAGAATAATTGATGGCGGGGACATGGATAAAATGGCAGGACGTTCAGACTTGTCTGTTTTACTC
GGTGTTAAATTGCCTAAAATTATTATAGATGAATAAACAAAAAAAGGGACCTTACGGTCCCTTTTTTCGT
TATGATACTAATTCAATCCATGCAGGACGCAGAACATCTTGCACCATTCGGACTAATTCTTTCTTAACAA
GATTCGGATTATCAGATGTTGTTAATACAATACCTTCACGAGAAGTTTCTTCCAGAATATCTTGAACAGT
CAATCCCATCACTTTGCCAAAATCTTTTGGCGTTACAGTCCCAATCTTAGAAATAACATTATTAACACGA
TTTAATGTAACATAACAAGCTAGAACATCAAGCAAGTTTTTGTCAATTTCGGTTAATGGAACCTGAGTTT
TAATAGGCTTATCAGACTTTTTCTTTTCACTGAATTTAGAGTTCTTGCACTTAATCGCTACACGAGTACC
GTTTGGCAGCCATTTAGGGAAACAAGGCTTCAGAACATAACCTTCTGCAGTATTATCACCAATAACATTA
GCATCAAATACACAATTATTTGCTTCTACAAGGTCTTCTGACGCAGTAGCATTATAAGCGGCTAAAACGG
AATCAAGGTCATTTGGAATCATGATAAGAGAATCAAATGTTCCACGACCAAGCATTGGTGCCATTTTAAA
ACCGAATTCATTACAGAAATCTTGCATTTCGTAATCGGTCAAATACGTATTATCACCACTTTCTGTATTG
ATAAGGATATCGAACACATAAAAATCTTTTTCACCGTAGTCAACACCTTTCTGGATTCCACCACCAGCGA
ATTCACCGAATACTTGATATGATACGGCTCGAGCTGTGTACATGAATTTCTGAACGGTTTTAATAGCTTT
ATCATACTTCTTGAGTACGATTTCATAACCATAAAAATCTTCTGCTGGAAGAATAGGGCCTGTGCGCTTG
GCGCATGTGACGTTGTCACGTTCAATTATTAACGAGAAGTTGGTCCCATGGATTTTCTCACGTGCTACCC
AAACACCAGTAGTCAAACCATTAGTGTAGAGTTTTTCAATAAACTTAGAGTTGTAGTGGTTTTCAAGACT
 
GCTGTATTTCTTAAACATAAATCACCATTATAAATAATAATATCAAAATAATTAGCAGAGACTCTATCTC
CGACCCGATGTTTAATTTGAATGGATAGAACCTAGATTCATTATACCATCAAAAAATGTAAAGCATATTA
GAACTTGATTTCTATCCAATGACCATCACGTTGGTCGTGATTAATTTTGTACTGAAAGCCGTTATCACGA
AGCCATTGACCGACTTGTTCAGTTATAGTTAAATCGTTCTTTGACGGGTAGTACGTGAATTGTGTCTGAC
CTTTGTCAGCGGCTTTCTCTGCCATCGCAGAGAAGTTAGTAATAAAATCACTCAACAAATTAGCTTTAGT
GGCCAGAGCCCTTTCTCTTAATTCCTGTGCAAAACTCATTTCTTCACCTTTTTGTTAATCAAACGTTTAC
GGTATACAACTTTTTCTTTTGCTTTAAACTTTGACCGAGCTGTCTCGGCTTTAGTCCATAGTAATCCAAC
TTCTTTAGGTTGTAAACCGATTTTTTGAGCAATTTCTACGAATGAAAATCCCGCCTCATGTAATGTATAA
ACTTCAACTTCAGCTCTCATAATCAATCCCGTATTATTTGGTTTGTGCCATTATATACATCAATCTCACG
TTGTGTTTTGTATGCTTTAAAAACGAAGAAAGGGGCCGAAGCCCCTTACGATTATGGATATGTATAGATG
ATACCAGTTTCCAAAGCAGTTTTATGGATGATGTATCCATTACGTGATTCTTGAACATCAACTTCTGGAT
AGTCTTTCATCATCTTCTGAAGAGTGTAACGATGCAGGTAGTATTTACTATCTGGATCTTCAGTTTTCCA
ATCTTTACCTTCTTCAGTCATTTTTTGGATTTCATCCATAACCCACCAACCGCACCAGATGTAAGCTGAA
GTACGATGTGGAAGAGGATGAACATAAGGTAGTTCACCTTCTGGCTCAATTGGAGTCTGTTCAGTAATAA
CAACTTCAGCGGTTGAACTAATTGTCTTTGAAGTATAGTTATCTTTAGTTAATACTGCTTCAACTTTAAT
TGTATGAGTACCAGCTGAGGAGGTATCAATACTGTAAGTATTAGTAGAATTACTTTCTGCTACATTGTCT
TTCTTCCAAGAATACACAATATTAGTATCACCTGGAGCACCAGTAACAGATGCTGTTAAAGTTGCAGTTC
CGCCAACAGTAGTATTAACTGATGGCGGAGATAAAGAAATGGTGGCACTATTAAATGTAGCATTATTAGT
TACTACTGTTGCTTCTGCTGAAATTTCTTTTGTAACATAATTTGTTGCAGAAACAGATACGGTGCATTTA
AGAGTTTTACTTCCGACCTCGGTTGGAGTGAATTTATACGTAGCAGAAGTTGCATCCAGAATATTACTGT
CATCCATTGACCATTGGAATGATGTAGTAGCACCTGAAGGTGCACCAGAGACATTTGCAGTTAAAGTAAT
AGGAACACCAAAGACCGTAGTCGGAGACTCAGGCGTAATTTTTAGTGTTGAAGAATTAGCCTTTTTATTT
ACAGTCAAAGAAACCGCTGATGATTCTACTGTTTTATCAACGTAATCAGTTGCAGTCACTGTGACAGAAC
AAGTTATAGACTTAGTTCCTTCAGATGTTGGTGTATATTCAAAGGTTGATTGCTTTTGACCATCAACAGC
GACCCCGTCAACTTTCCATGTATAAGCAATAGCAGCACCACTAGGTTGATTGCTTACATTAGCAGTGAAT
GTTGTTGCTGTTCCAATTTCTACTGCCGCCGGAGAAGCCGGAGTAACAGCGACGGTAGAATTATTTTCTT
TCTTATTAACAGTGATAGTTGTTGACGCTTCAGCTATTTCCGGATCTCCTGCGGAAAGTGTATTGGTTGC
AACCACTTTAATTTTTTTTGTTCCTGCCGGAGAAGCAAGAGTATAATCCATTGTAGCAGCCGTAGACTCC
TGTGCTATATCATCTACAGTCCAGACGTATGTAATAGTACCATCTGGAGTTTCACCTGCAGGTGTAGCAG
TGAATGTTTGTGAACCTCCAATAACCCCTGTAGGAGTTAAAGGAGATAACGTGACAGTGAATGCCATAAG
TTATCCTTATTTTAATGTTACGAAAGAAGAATTACGAGTTTCACGAATTAGAACTGATCCATCGCGATTA
ATATAATAAATTAAGCTAAATAATGTTTGGTGAGCAGAAGCATGCACAAAACTCGTTGGGTGAGATTTCC
AATCTGGGGTTTCGGCTATCCATTGATAAATCCACCAAGGAACAGTAACATATCCTGGGCCTTTGCCTAA
TTTAATGAGTGTAGGACTAAAATTTTCAGGCAGAACAAAATTTACTTCTTTATTTACTTCTGGCTCAGGC
TCTTTTTCAGATTCAACAACCTGAGTAATTGCTTCTTCGAATTTATCATGGTCGAATTCTTTTTCTTGTT
CGACAACTTCTAATACTTCAACTTGTTCTGTTGGAGAATCAAACAACAGAATAGCTTCTTCTTTAACTTC
TTCGTTGGTGAAATTAACACCGTCCACATCATCTGCGGCAGTAATTAAATCAGTAATAGATAATCCATCA
GTTTCAGGTAATGGTTCATCAGCCAGTGCGTTAAGAGCTTCTTCGATATCAATAACAATATTATCGAACG
ATTTTGTTTTCTTAACACTAATGCCAAATTGTTCTGCATATTCAAACAGTTTAGCTTTAGCTTCCTTTTT
ATCAGAAAGAGCACGTAACTCTTCAATATAGTCTTTATCGATCATGGTATTTCCTCATTATAAATATAAC
TATATTTATTACATGGTAATTTGACATGACTGACATTAAAGTACATTTTTATGATTTTAGTCACGTGCGC
ATTGAATGCGACGAAAGTACGTTTTACGAACTTCGTGACTTTTTTAGCTTTGAAGCTGACGGGTATAAAT
TTAACCCAAAATATCGCTATGGGCAATGGGATGGTAGAATTCGACTTTTAGATTACAATCGTTTGTTACC
ATATGGACTTGTAGGACAAATAAAAAAGTTCTGTAACAATATGAGTTATTCTGTATGGATTGACCCTAAA
 
ATCTTTGAGACCGAAGACCTTACCAGGGAAGATTTTGACGCGTGGCTTTCTAAACAAGAAATTTATTCAG
GCAACGCCAAAATCGAACCACATTGGTATCAAAAAGATGCCGTATATGAAGGTCTGGTTAATCGTCGTCG
AATTTTAAACCTTCCGACATCAGCAGGTAAATCACTAATCCAAGCACTTTTAGCTCGATATTATCTAGAG
AACTATGAAGGTAAAATTCTCATTATCGTCCCTACAACAGCATTGACAACCCAAATGGCTAATGATTTTG
TTGATTATCGCTTGTTTAGTCATTCGATGATTAAGAAAATTGGCGGTGGTGCTGATAAAGCGGACAAATC
TAAAAACGACGCTCCAATTATTGTTGGCACATGGCAAACGGTAGTTAAGCAGCCTAAAGAATGGTTCTCT
CAGTTCGGTATGATGATGAACGATGAATGCCACTTAGCAACAGGCAAAAGTATTTCTTCAATCATCTCAG
GATTAAATAATTGCATGTTTAAATTCGGTTTATCTGGCTCATTAAGAGACGGAAAAGCCAATGTAATGCA
GTACGTAGGTATGTTCGGCGAAATCTTCCGTCCTGTTAGCACGTCTAAATTAATGGAAGACGGACAGGTA
ACTGAGCTTAAGATTAATAGTATTTTCCTTCGTTATCCAGACGAATTCGCCACCAAATTAAAAGGTAAAA
CATACCAAGAAGAAGTTAAAATTATTACCGGACTAAAACGTCGTACAAAGTGGATTGCTCAACTTTCGGT
AAAATTGGCTAAGAAAGACGAGAACGCCTTTGTAATGTTTAAACACGTTACACACGGTAAAGAAATATTT
GAAGCTATTAAAGAGCTTGGATACGAAAAAGTTTATTACGTATCAGGTGAAGTTGATACTGAAACACGTA
ACGCTCTTAAAGTCATGGCAGAAAATGGTAAAGGCATTATTATTGTCGCATCATATGGAGTATTCTCTAC
CGGTATTTCAGTCAAGAATCTTCATCATGTTATTTTTGCTCATGGTGTTAAATCAAAAATTATCGTACTT
CAAACCGTAGGTCGTGTGCTTCGTAAGCACGGTTCTAAAGCTGTTGCTACAGTTTGGGATCTCATCGATG
ACGCAGGTGTAAAACCTAAATCATTAAATACTAAAAAGAAGTATACTCATCTTAATTACTTGTTAAAACA
CGGTATTGATCGTATCCAACGTTACGCTGATGAGAAATTTAACTATGTAATGAAAACAATTAATTTATAA
GGGCTTCGGCCCTTAGGAGATAAAAATGGTACTTGAATTTAAACAATTTTTATACGAAGCATCTATTGAC
GAATTCATGGGTAAAATTGCTAGTTGTCATACCTTAGAAGGGCTTGAAGAATTGGAAGCTTATTATAAGA
AACGTATAAAAGAAACGGAATTAAAAGATACAGATGATATTTCTGTCAGGGATGCCTTAGCAGGTAAGCG
TGCCGAACTTGAAGGCGACGATGAGGATGCGGAAGAAGATTTCTAATTAAAAAAAGGCCCGACCTTTTTG
GAAGGGCCAAAAACCATAGGCAAAAAGGTAACACTATAGTAAAAGTTGGGTTTCGTTCAATTGCTCTTCT
GAACTTTCTGAAACCGGCAATTGTTGTACATAATTATAACAAGGACCAGGATGTGCTGGACCTTTGTCTG
TTTCAACAACCAATGCAGAATCGATTGGAGTTTTACAGACAACACAAATCTTATCTGACATGATTGTCTC
CTCTGAATTATATATATCTATTTATAACCCCATCTTAAAGCGCATTTCTTGTACACGCTTTAAACATGCT
TCCATTTCTGCTAAAAGTCTAAATACTTCTTTATAATTTGTCATATTTACCCCATATCTCTCATATGCAT
ATCAATGCCCATATCTTTAGAATAAAAATATTCATCAAGATATCCGGCAAATTTTCCTTTAATATAAAGG
ACATCTTCACCACACGGGTGGTTCGTCAGAATACGAATATCCTGACGTCGTAGACGATACTTTTCCATTA
AGAATTTAATTTCTGTTTCAAATTCTTCATCATTATTAAAGGCATCTTCAATTGAATAACGCATTATTGC
CCCGCTTCAAATTCTCGCATATCTTGAATATGTTTAAGTGCAAAACTACGAGACTTAACCGCATCAAGTG
CACCACTACAGAACTCAAGTAAAATTCCCCAGTATTGTAAAGTAGTCTCTATTTTAAGCACATCTTTATC
TGCGGCCAGGACAGTTTTCATTTCAGATTTCTCGTATCGGTCCATACTGAATTCATCACCATCACCTCGT
CCCGAGTAGTAGTCTAATCTTGCTTTTAGAGCAGTTTTCTTCTGAGTCTCTATACGCAACATTTCTTTAC
GAATAAATGAGTGCTTACGTAGCCATTTGCTGTATAACTTAACATTATTCGCCGTCTCATATTGCAACTT
TGTCGTATCAATAGCTAAGTCTGCGTCTAACTCTTCCTGTAAATCTTCCAGCTTCATAATATTCTCTCTT
TTAACTAATAATCTCCCGGCTGTATCGGAAGGACCTAAAACCATTATACATAGGTTGCCTTGAAGCATTA
CACTCTATTGCTTGACGCTAACTGAAGTTTAATTTGTTCAATATCGTCAGGGTTGTCTACGACCGAAAAT
CTAATCTCTACTATAACCGTATAGTCGTCATATACCGGCGTTACACTTACAGAAAGTTTGTCGATGCGTG
GCTCATAGTTACGAACAGCTGCCTGGATATTACGCTCAATCGTATCGGCTGTAAGTGGTGTCATATTCTC
GAAAAGTTCATCAACCAAATCGCATCCAAATTCAGGGTCGAAGGGGCGTGAGCCTTTTCTAGTTGTCACT
ATGCCAAGAAGACTATTTTTAATAGAACGAAGTCCTACAGAACGAGCAACGTCCTTGTTCCAATCCATAC
GCATTTCTGGGTCGATATCTGAATAAAGTTTATTGATATTAGACATTATAGTAACTCAAAGAATTCTTTG
AGGCCTCGTATTGTGTGAACGTGTGACGCCCCACATTTGGGACATTTAATAGGGGTTGCCAGATAAATTG
 
ATGGCGACACCAAAAGATTTTTAATATTGATCATGTCATCTTCTGTTATAAGGCCGTAAAGGTCGTTTAT
TTCTGCTTCAGTTAAATCTTCAACAGGGATGGACTCTCCATTGACAAAAACAGCCTCAATACATGAAGCA
ATCATTAATGCGATATTTTTATCATCAAATAATTTAGGTTGACGGAACTTAATTTTAATACCGCTGAACG
AATACCAGGGATCGGCCTGTTCATCTAATTGAGTGTGCAATAAATTCATATAGACTTGAAATGAATGCCC
ACAGGAACAATTCCAGGTGTGTTCTTGATTCACTTCACCTAATGAATGTGCCCACAGATTTACCAACAAT
AACTCGGCTTCTTGTTTAGTCAAGTTTTTAGCGTTAGAGCAATTAGTGATAATATTGTTCACTGCTGATT
CAATAGTACCGGTAGCCCGCGCACCTATGAGTCCTAAATATTCTTTTAAAGTAAAGGCTCTACAATTCAC
TTCTTTATCGCCTAAACGTACGGTAAACGTATATTTGTACATGTTAACTCCTTTATTGTGTATTTATAAA
TAATAGAAAAGGAGTAATTATGGCTAATATAATTCGTTGTAAATTACCGGATGGTGTTCATCGTTTTAAG
CCGTTCACTGTGGCGGATTATAGAGACTTTTTACTAGTTAGAAATGATATCGAACATCGTTCGCCGCAAG
AACAAAAAGAAATAATTGCCGATCTGATTGATGATGATTATTTTGGCGAGTACCCTAAAACATGGCAACC
ATTTATATTCCTGCAAGTTTTTGCAGGCTCTATAGGCAAAACAAAAGTTCCCGTTATTTTCACTTGCCCT
AAATGCTCTAAAGAAAAAACTGCACCATTTGAGATATATCAAAAAGAACTAGTCGAACCTGAACTTGATG
TTGCTGGTATAAAAATACGTTTTTCTTTCCCTGAAAAGTTTTATGACAATAAAGCATTAATGATAAGTGA
AAACATAAAAGAAATATATTATAATGATGAATGGTACCCATGGAATGATTTGACTGAAGAGAACCAAATC
CAAGTAATAGAAGCAATAGACATTGACTCGCTCGAGAAGGTTATTGCTTCTATGAATCCTATTAATTTGA
CCCTTAGATTAGGTTGTTGTGAACGTCATGTTAAAACATATACTGATATCTTAGAGGTTTTTAAATTGTT
AGTTAACCCTGATGAGATTTTTACATTTTATCAGATAAACCATTCACTAGTAAAGAGTCAATACACATTG
GATTCTATTATGCAAATGATTCCTGTTGAACGTGGTATTGCTTTAACATTAGTAGAAAAGGACCATAAAA
AATGACAGTATTCCAACGTCCAGGATATCCTAACTTAAGCGTTAAATTATATCAAGATTATTCGGCTTGG
CAAGAAAATAGATACGTTGAACTCGCGGCAACAATAACAACTTTAACCATGCGAGATTCGCTATATGGAA
GAAATGAAGGGATTCTTCAATTTTATGACACTAAAAACATCCACACTAAAATGGATGGTAGACAGATAGT
CCAAATCTCTGTTTCAAATTCAAACACACCTCTCCAAGTTAGAACTCGAATTTATGGATGTAGACATTAT
TCTGTGTCTGTAGACTCAAAAGGTGACAATATTATTGCAATTGAGCTTGGAACAATACATTCTATAGAGA
ACCTTAAATTTGGACGGCCATTCTTCCCAGACGCAGGAGAATCTATACGTGAAATGTTAGGTGTCATATA
CAAAGACCGTACGTTAATTACTCCGCCAATAAACACTATTAATGCTTATGTTCCTGATATTCCTTGGACA
AGCACATTTGATGATTATTTGGCATATGTCAGAGAATTAGGACTTGCTACAGCGAGTGATAAGTTCGTAT
TCGTATGGCAAGATATTTTAGGCGTAAACATGATTGACTACGATACTCTTATTGGCCAGGAAGGCATTAA
GATGGTTGTAGGCGAGCCAAATACCGTAGGTCAGTATATTCAAGAGCTTGAGTATCCATTAGTATGGGAT
TTTACCTGGATGACTAAAGCTAACCAATTTACAAGAGACCCTATTAAAAACGCCACGGTATTCGCCCATT
CATTTTTAGATACATCTATTCCGGTTATTGTTACAGGTGATGGTGATAACGCTATTGTTGTGTCAAGATC
CGGTGGATATTCCGAAATGACCTATCGTAATGGATTTGAAGAAGCAAGTCGTCTTCAGACAATGGCTCAA
TATGACGGATATGCTAAATGCACTACTACAGGTAATTTTAACATAACGCCTGCTACTAAAATTATATTTG
TTGACCAAAAGAACCAATTCAAATCTGAATTTTACGTAGATGAAGTTATTCATGAGTTATCAAATAACAA
CTCACAAACTCATCTGTACATGTTTACCAACTCAATGGTGTTAGAACCTGTTAACCCAGTTAAGGTTAAA
AATGAACTTAAATCTGATTCTACCTCTAAAGAAAATAATTCTACCACAGTCTAATAAAAAGGTAATGATT
CCCAAATTAGGGCTCAAACATTATAATCTTTTAAAGGACGTTAAAGGTCCTGACGAAAACATGAAAATCT
TAGCTGATTCTATTTGTAAAAATATGAGTCCCGCAGATTTTGATTTTGCATGTCTACACATATTGGAATT
TAATAACAAACTTAAGTCTGAAGTTGAAAAAGACGGATTCACATATAAACTAGATGACGTCTATGTATGC
CAACGAACAGAATTCCAATTCCAAGGCAATACTTTTTATTTTAGACCCCCAGGCAAATTTGAACAATTCG
CTACAATCAGTGAAATGCTATCAAATTGCTTAATAAAAGTTAATGACGAAGAGAAAGAAATTTCTTTCCT
TGAGATGCCTGCTTTTGTAATCAAATGGGCAGAAGACCTTTCGACAACTATTGCTATTCCAGGCCCTAAT
GGCCCTATTAAGGGTATAGCAGAAATTATTGGATTGCTTGAATGAAACCTGAAGAAATGAAATCCATGCG
TAGAAATAAGGTCATTGCCGATAATAAACCACAAAAAGTAGCTGCAACAGCAGCTACCGATTCATTAGAA
 
GCATTGAATAACATTTCATCAAAACTAGACGATGTCCAAGCTGCGTCTGAACTCACATCACAAAGTGTAG
AAGATAAAGGCAATGGAATTATTGAATCAATTGGAGACTTAAAGAATTCGACAGATAATACTGCTGAAGG
CACAGAGCTTATCGCAGAAGTTATTGAAAAACAAACTGAAGTCACTAAAAGTATAAACGAAGTTTCTAGT
GCAATAAGCTCTAAGTTAGACCGATTAGCTACATTATTAGAGCAAAAATTACAAACATCTACCGCTATTC
AAAATACAGGAGGCACCTCTCTTGAAGTAATTGAGAATGCAATACCTGTTAAAGTGGTTGAGAATGAAAC
TTCTGATGAGTTGTTTAAAGCATTCCCTACACCTGAAAAGATCGATAATAAGCCAGACGAAGATTTCTTC
CCAACGCCGGTTCAAGAAAGTGCTAATTCTACTTCCGATTCTAAAGGCGGGATTAGCTTTAAATTGAGCG
ATAAAATTGCAATGCTCACCAAAACAGTTCAAACCGGGTTTAATAAATCAATATCAATTTCAGACAGAAT
TGCTGGAATGCTTTTCAAATACACTATAACGGCTGCTATTGAAGCAGCAAAAATGGCCGCACTTATTCTT
GGTATTGTTATCGGTATTGATTTATTGATAGTCCATTTCAAATATTGGACAGACAAGTTCACTTCTGCCT
GGGATTTATTTGATGAAAACTTTACTAAATTCTCCGATGAAGCTAAAGAGTGGGGTAAATTCTTAAGTGA
TATATTCACTTCTATTGACTCGATAAAACAATTGTGGGAAGCTGGAGACTGGGGTGGTTTAACCGTTGCT
ATTGTCAAAGGTGTAGGAACAGCATTAATGAACTTAGGTGAACTAATTCAATTAGGAATGGCTAAACTCT
CCGCATCTATATTGCGTGCTATAGGTTTTGGTGATACCGCGGACGAAATAGAAGGACGTGCTTTAGAAGG
ATTCCAAGAAACTACTGGGAACAAATTAAAGAAAGAAGACCAAGAGAAAGTCGCCAAATATCAAATGAAG
CGTGACGACGGGGAATTAGGAACCGTTTCTAAAGGTCTTGATATGTTACAACGCGGCAAAACATTCGTTA
CTAACTGGGTTCGTGGTAATGATAATAAAGAAGAGTTTAGTACAAGCGATGAGCGTGCTGCGGAATCGGC
AAAATTAAAAGAGTTACCTGAAGAGGAACGTAAAGAGGCATATATAAAAGCTAATGAAACTCGGGCAGCC
TTAGTTAGATTTGAGGATTATATCGATAAAATCGATATGACTAATCCGGAAAATGCTAAAAACGTTGAGA
AGTCTTATGCCGATTTAAGTAAACTTATTAAAGACCCTGAACTTAATAAAACTCCTGTTGTTAAAAAGGA
ATTAGATGCTAGATTTGAAAAGCTAAATAATAAAATGGCTGAAGCCAAAAAGGCTCAGACAACAGTTAAG
CCTGAATCTTCGTCTAAATCTCCGGAAGCAAAACAAGTTCAGTCAATAGAGAAAGGACGTGCTTCTGAAT
CTAAACAACAGCAACCGGTAGCGACGATTAGTAATACAAATAATGTCGTCAAAAAGAATACCGTAGTCCA
GAATATGACCCCTGTAACAAGTACAACGGCTCCTGGAATTTTCCATGCCACCGGTGTTAATTAAGGATTA
TTATGATTAGTGTAAAAGAAATAGTAGTAGATGCAAAGGATTTAAATAAAGCAATCCCAGTCTCAGAAAG
TGCTGGGCAAAGCACTAAAACCGAAACAACTACTAAAACTTATGTTGCTCAATTTCCTACAGGAAGAGCA
GCCGGAAATGACTCCACAGGAGACTTCCAGGTAACAGACCTTTATAAGAATGGATTATTATTTACCGCGT
ACAATATGTCTGCACGCGATTCAGGATCCCTAAGAAATTTACGTCCGGCATACGCCGGGACTTCATCTAA
TGGTATCATATCAGATTTAACTGATAATGTGAAAGATGCAGTGACTAAATTCTCAAATGGATTGCTTCCT
GCCGGAGCTAATAAATCTACTATAAATAAAACTCCTGTTGCTAATATACTTTTGCCACGTTCCAAATCTG
ACGTTGATACAACTTCACATCGTTTTAATGATATCGGTGATAGCCTTATTACTAAAGGTGGAGGTACTGC
TACGGGCGTTTTAAGTAACATTGCTTCTACTGCAGTTTTTGGTGCATTAGATTCTATTACTCAAGGGCTT
ATGGCTGATAATAACGAACAGATTTATACGACTTCTCGTAGTATGTATGGCGGTGCTGAGAACCGTACTA
AAGTCTTTACATGGGATTTAACTCCACGTTCAACTGAAGACCTTATGGCTATTATAAACATTTACCAGTA
TTTTAACTATTTCTCATATGGTGAGACCGGTAAATCACAATACGCCCAAGAGATTAAAAGTTATTTAGAC
GAATGGTACCGTTCAACGTTTATTGAGCCTATGACACCAGACGATGCTGTAAAAAATAAGACATTGTTTG
AAAAGATTACTGCGTCATTAACAAACGTATTAGTTGTAAGCAACCCTACGATTTGGATGGTTAAAAACTT
TGGACACACATCTAAATTTGACGGGTTAACTGATGTATTTGGCCCATGTCAAATTCAAAGCGTACGTTTT
GATAAAACTCCAAATGGACAATTTAATGGTCTTGCTGTTGCTCCTAACTTACCGTCAACATTTACGTTAG
AGATAACAATGCGAGAAATTATTACATTAAACCGTTCTTCACTTTATGCGGGGACTTTCTAATGTACTCT
TTAGAAGAATTTAATAACCAAGCTATAAATGCAGATTTCCAACGAAACAATATGTTTAGTTGTGTATTTG
CTACTACACCGTCGACCAAATCGTCTTCACTAATTTCGTCAATTGGTAGTTTTGCTTATAATAACTTAGG
GTTGGATTCAGATTGGCTAGGATTGACTCAAGGTGATATCAATCAGGGCGTAACAACCCTGATTACAGCA
GGAACACAGAAATTAATTCGAAAATCAGGTGTAAGTAAATATCTGATTGGAGCAATGAGCCAACGTACGG
 
TACAAAGCTTGTTAGGTGAGTTCACAGTAGGAGCTTATCTGATAGACTTCTTCAATATGGCATATAATAA
TACAGGTTTAATGATTTATTCTGTAAAAATGCCTGAGAACCGATTATCTTATGAAACGGATTTCAACTAT
AACTCCCCGAACATCCGAATTACCGGTCGAGAAATGGACCCGTTAGTAATAAGCTTTAGAATGGATTCCG
AAGCATCTAACTTTAGAGCAATGCAGGACTGGGTAAACTCAGTCCAAGACCCTGTAACAGGTCTTCGCGC
TTTACCACAAGACGTCGAAGCAGATATTCAGGTTAATTTACATGCGCGAAATGGTTTACCACATACCGCT
GTCATGTTCACTGGATGTATTCCTGTTAGCGTCTCTTCTCCTGAGTTAACGTATGATGGTGATAACCAAA
TAACAGTTTTTGACGTAACATTTGCTTATCGCGTTATGCAATCAGGTGCTGTAAATAGACAGGCTGCTTT
AGAATGGCTGGAATCAGGACTTATTAGTAGTGTTTCAGGAATGTTTGGAAATAATCAGAATGATTCTGGA
CTAGGAAGTGCAGTATCAAGACTTTCTAGATTAGGTGGGACTGCTGGTGGTGTGTCTAATATAAACACAT
TGACAGGTGTGGTGAATTCTGCGTCAAGAGTTTTAGGACTATAAGAAAAAGGGAGACAACGTCTCCCTTT
AGGGGTTTATTTACGGAATGAAAGGAATGCTGGTGCTCGGAATGGAGTATCATCTTTAGAGTATTTAACT
AATACGACCATTGGTACCTCTGTTTCTAAATGACCTGTCACTACACCTATAAAAATTTGGTCGGTGTGCT
CAGGGTCACGAAAGTTTTTAAGAGGTTCCATAGCAATATGGAATGCACCTGGGAATACTCGATTCAATTC
GTCAATCACAGCTTCGTTAAAAACATCATCTGCTGGAATAACATTTTCAACTACTAGTTCTTGACCATTA
AAGCGAAGCATAGATTTCATAACGGTTTCCTCATGTTGTTGATGGGATGATAGTATAACACATCCATGTG
CTTGTAAACTATCCTTGGACAAATTTAAACGGAACTGATTCTAAATCCATTAAAGATGCTAACAATTTCA
AACCTTTAGGTGCGCTAGTTTTTTCTACAAATGAAGAGAACGATACCGGCTCATCTGATTCGATTTCACC
TGTCATAGCAACCACCTCACCTGTTTCCATGAGAACATCACCATCATAAATGACAGCTTCATCTAACTGG
TCTGAGGACATGACTTCAGCTTGAATAAATTTAAGGTTGGATTCTAAACCTGCACCAGTGTCAGATGACG
CATCTGTAATTTTATTTATCTGCAAGAGAATGCCTCTAGGCAAAATAATTTCTTGCTCATTAGGGTGATT
ACTCAACTGTCCTGGATAAATCACGTTAACTTTATGAGCACCATCAATAGCCCATCCAACGTTAACACGG
ATTTGTTCTGGTGCATGCATAGCTGTTCTTACTTGACTAGGAGAAATAGTAACACCTTCGTCATTATTGT
CAATGTTAAGCTCTTTGCGAACGTTCTTCAGGAGCAAGTCCAAGGGCAACGTTCTCTTTAAACCCACCGA
AAATAATAGGAGCTTGTGAAGTGGAAACATAGTTTCTGAAGTAGAATACCTTATTTTTAACCATTGCTTC
GTAAATAGGCATTCTGATAGACTGAGCTCTATATAACGTTTGTCCTTCAGGTAACGGTCTCCATTTAAAA
ACGCCGAATCAAGACCATCAATAGCGCGTTTAACTTCATCTTCATCTGCAACATCGTAAAAATCTGGTTT
ATAACGACCTAACAACATATTATTGATATCGGTATACGCAGAAGAGGCATATTCACGAATAGCTCGTTTT
TCAGCAACAGAGTATTGTTTAGGGTCCCGATTCATTGTTATATTAAAGGTGCCTCTAGCCGATTCTCGTG
CATAAGTATACATCACATTAGACAAGAACGCTGATTTACGTTGTTTCCAAGTTTTTTCAGATACTTCAGC
TAAAACGTCGTCGGCCAGTTGGAAATTATCACGTCTAAAATCTTCAAACCATTCGTCTTTAATAGAGTCT
GCAATCTCATTTGCAGCTTCAACAAAAGCAGCTAATGCTTTAACAGATGTTGTTTTTTCAGTTTTAATTT
TCTTAGCAAACTTTTCTAAAGTTGGCTTAATAACACCACCATAAGAAACTTCATCAGCAAATTTAAAGGA
TTCATCAATACGATTATAAATCTTATTTTCGATATCAGCAACTAAAGTGCCTTTTGCTGTGGACGATGCT
TGGGCTAGTACTATTTCATATGCTTCAGGAAGTAATTCGGCTGTAGCCGGGCCACTAACTTCTTTAGCAG
AATTTTCGTATTTCTGGAATAACTCACCTTCTTGGCGGTCCGCTTCTAAAGATTGACTTGCTGCAATAGC
ACGACGAGAAATTTTAGTACGAGCAATAACAGGCTTATCTTTACGTTTTTCTTCTACAGCAGCAATAGAA
CCAGCGATAGCCGTTTCTTTGGTGACCTTTTCGCCAGTCTTTTTACTCACATAAACTTCGCCAACATCAG
AATCTACTTTCGTGTACAACTCTGTGTTAATGTTAGGGATGCCTTTAATATCTTCAATGTTGGCATTTTT
ACGAACCATCATCACATAGGTGTGTTTACCTGTGAATTGATACATTGCCGACAATACTTTAAAACGACCA
CCGGTTTTAGTAGAAACTAAACGAGCAAGAAGAGTTTGAAGCTGCTGTCCACGTCCTTTCAATTTCTTGG
TAGGGAATCGGAACAGAACTGCATCCATACGAAGAGCTTTAACCTGTTCATATACTGTATTGAAAATGGT
GTTGATTGCATCAATAGGAGCAGGACCAAGGCCATTTTTCAATTCAGCGAGATTACCCTTTTCAGATAGA
CTCATAATAACAACATGAGCGTATTTGTCGCCAAGTTTAACTTGTTTTACAGCATCACCTTCGGAAAGGT
AAGAGAATAAGCGAACTACGATGTTTGTATCGATATCACCAATTTTCCAGACTTGTGGAACTTTAGCTTT
 
AGGGTTCAAATTGACGACAGGAAGACTGCCTTCAGATTCAAACACTTCATTTAATTGTTCAGACATAGTG
TTTTCCTTTTAGTAATAATTTATATTTATGCCAAAAAGAGCCCGAAGGCTCTTAATCCACAATCTGGAGC
AAAGTACGATAAACTTCAAAATTAGGGCCTTTTTCGTACTTATCAAAATTGTCCATAAAAATATGATAGG
AATCGTTATTAGGAAAATCTAATACAATTTGGGAACAAGAGGTGGAAACATCACCACCATCTTTATTAGC
GACAACTACTGTTTCTAAATAAGCTTTCATTATACACCCGTCTGGTCCCAGGACCCAAATACGTCTTCAA
ACGAATCCGCTTCTGTTTTATCAAAACGGAACCCTTTAATAATTGGAAGGAAAATACCTACAGTACCTTC
ACGGCCTTTAGAATGAACCCAACCATTACATTCACAATCAGCGATACGTCCAATCAATTTACCTTCACGA
GCTTCGGACATTAATCGTTCACGGTCCAAATCATGACGCTCGTCTAAAGGAATAAGAACCTTTACACCGT
CAACTGTTTTATGAGTTGTATCTTTAAAACCTGAACCACAATCGGTTGTAATACGACGACAACGTGATAC
GAGTTCGACACCACCCAATTTGTTAGGGTCTTTAGAGTGTTCATAGTATCCTACAACTTCTAAAGCTATA
TCAATAACTTCTTTAAATTTAATTAGGTTCTTAGAGCGTTTGTTTTCCCAATATGAATCACGGTTTTTAA
GGATAATACCTTCAAGACCTTGGTCAACATATTTTTTATAAACCACTTTAGCTTCGTCAAGATTACGAAC
TAACTGATTTTCAATTGGCTCAATTCGTTTAAAGCCTTCAGCCATATTTTCAAGAGCAGCAAAACGAACA
TCGTATTTCTGACCTTTAATTTTGCCATCAGAATAGACTTCATCTAAAGGAACATAGTCCCAGGCTTGAA
GAACCATACCTTCAGCTTCTTTAGGAGAAATAGTCCCTTGAAGAGATTTATTTGCTAAACCGTTTGAAGT
GCTACGGTCTGCAACTTGTACTTCTTCGGATTCTTCATTATCACCAAATAAGAACGACAGGTCATTTCCT
GAGCTCACAGCCTTTTTAATATCAAATGAATGATAAACTAATTCGCCGTCGATTAAAACCCCATTAGGGT
GTCTTTCACGAGCTTCTTTAGTCATTTCCATTAGTTCATCTGCTAATAGGGTAAGACCATGGTATTCATT
ACCAGCACGAGAAAAGAATTGAACACCATCATCGCGAACTTCAGCGAAACAACGAGCACCATCTGCTTTT
AATTGAGCAAATGCAGGCCATTTAATATTTTTAGTAATCAATTTCTCATCATATGCAGATGCTAACATCT
GTGGTTGCAATTGAATAAGTCCTGGCCACACCTTATTCGCGATAGACACTGAAGCACCTACTTCAAGGTC
GCGCATCATTACACGACGAAGGACTTCTACATCATCAGGTTTACCGTCAGCGATATAACCCATGAGCTCT
TTAATTGCAGCATTACCAGTGAGTTTACGAGTGGCTAATGTGAACTCAATAAAATCCAACATGTCATCTA
ATTCAAGAAGACCATATGCCTGAGACCTTTCACCTGGCCCAGGCCATTTCTTAATATAATACTGAATGCC
ACGTGCATATGTTAAACGATACACGCGTTCAAGTAATTTGTTGTCCTTATTTTTCTTAAGGATTTCCTGT
TTTGTTTTAGTAGAACCGATCGCAGCTATTTGGTTTAAAATATCTAAAATCATTTGATATCCTCGATTTT
GGTTACGGTTCTATTATAAGCCACTTCATCTAGAAGCGATTTCAGCGTTTTCTGATAAGTGGTAATCCGT
TTACCGAGTCCTTCAACATAATATTCTGAAATGTAGATGAACCCTTGTTCAACACACTCTTTAAATATTT
CTGCGTCTTTTACTGAACGAAACAATGGCCGGTCTCGAAAGAACATGTTAACGTGACCGTAGTCACGCGT
TGTGTTTTTGCCATCTGTAATATCATGAACTACAAACATAATTAACCCTTAACAATTTATAGACCAAGTC
CATTTAGACGAATCTAAAGGAAAGTTTTGAATCGTCCACCACGGCTGCATATCGGTTGGTCCTGGAGTTC
GCCAGGTGTACGGATTTGACGGAGCGCCAATTTGATTAGGCCATGCGCCTGGACTTGTTGCACCAGATTC
TTTAAGCAAATCCGCCAATCGTTTTAAAAGGTCTTCACGATCTGAATCTGAATTATGCTCGGGCGGAACC
AAACGAGATTCAATATCATCCCACGTGTGTACACGTTGTGCAGTGCGAGGAATATCATCACGTTCACCAC
GAGCCAACCAATAAATCGGTACATTAAGAATTTCTGCCGCATGGTCACAGTGATGAGCTAAATCGTCAAC
GTAACAAATCACGTTATATTTTGTTTTAGCTAGTTCGAATAATTGTTCCTTTGAAGAATCATGACCACAC
ATCATTACTTCAGAGAAAGCGCCTGGGAACAAAGCATTCAAATTAAATTGTCTATTCAATCGAGCATCAA
TTGAATCACCTAGCGCCGTCACGGCTACAAAATTATAGTCTTCTTTTAGTTTATTGATAACTCGAAGAGC
ATCCGAATAAGGCGCTAAGTAACGAATAAAATCAGAGCGATTATATTTTTCTATTAATTGTTTACCAAGG
TTATCGTCGGTATTGAACAGTTTACCAGGAGAAATAAATTTTTCGTCCTGAATCATTTTTAAAATATGTT
CTAATGGAAGATTATATTTTTGGGCAAAATAAGGAAGACCAGATTGCCAGCTTAAACATACACCATCAAT
ATCAGTCAAAATAGTAGGCTTCATAAAGAGTCTCTTAATAGGTTTAACACATCAATAAATTCAGCTTCAA
GTTAGTATTGTATCATCTTTTGTTAGACCACTAGCCATGCTGTGCTTCAAAATTTTTCCTTTAGAGGCTT
GTAAGGCATCACGAAACCCCTTGTTTTGGATCGCGGCTTCAAAATATGCATTTGTGTATAATTCTTTCCA
 
CGCTTCGGAGTATCTTGAAAATGGAACTCCTAACCAGAAGAGAGTCCCACGGTCCTGAGCTCTTGCATAA
GACCTTCCTGCTTGCTGTGCAGCCAATCCAGACAACCCAAATATACTATGTTGTTGTTCAACATTTTTCA
CCTTACATCCTTGGAGAAATCCTTCAAGACCTCCGAATTGAATACCATCCATAACAAAAGGCCATTGAGC
AAAGTTACTTAATGCACATGACGGCCACTTGAAATTACTTCTAATCTCTAACTCAGGCATCTTTAACACT
TATAATTTCAACATCAGCCCAATGACCATAACCAGGTATACGGTCTTCGATAGATAAATTTCCGTCATTT
TTAGGCATTTTAATTGACGTAACGTATGGTTCTAAACGATTTTCTGCGTGTGGAGAAACATCTGTCACAC
GATAAGTAACTTCAATCGTTTCCATACCAAACAATTTCTGGATTAATTTTTTAAGCATTTTATTAGAACC
TCATCTGAAAGCCATGTGATTTAACATTACCGCCGCCAATATCAGAAATGTTAATTTCACTTGCAATCGA
AGGATCGATGTCAATTTCACGATTAAGTTTAGTGATAGCCAAAGTATCATGCCCATTTACTGTACGAAAC
TCCAAAGGACATACTACGTTGACATAGTGTTTAATTGACTCGCTAGTAGGTTGCAAATGGACCGGAAGGT
TTTTAGGTGCTTTAGAGAAAACAACTTCACAAAAGTTTTTGCGGGTATCAAAATAAGTGGTCATAAACAT
AATATTTTCCTCAATTGGGAAGCCGAAGCTCCCAGTTAAATTAGATATCGAATTCATTAAGAACTACATC
AAAGATTGCTTCGAGATGTTCAGGTTTAGCTCGGTTACTCAGGATATGACGAATCCAAGTTTTAACCAAA
AGTTTACGATTAACACCGTTCCAGCAAGGATGAGTACCTAAATCACGCTGACGGAAATCATCATCTAATG
CAATTTTGAAGTTGGAACCTTCCATCGTGATTGAAACCGTGATGCCATTTTCAAATCGCATATAAACGTA
ATTAGGAGTCATGCACTGTTCGATTTCACAAACTGTACCGTTTTTGTGTTTCCATAAGCAAATAACTTCA
GAAGAACCAGCGATACCGTTAGAAACATATTTACGTTCGAAGTTGATGTAGTTCATTTTATTCTCCAGAT
GTTTAAGTTGTTTGTTGGTACAGGTCTATAATAACATATCCTGTACCAAAGTAAACATTTTTTATTTGAT
TTTTGTCCACTTAGCTGAATCAATAACACCTTTTGCTTTAATCTTCTCGATTAATGACTCTGCGCGATTT
CTGGCATTTTCGTACCATACAACGTCTTCAGAGTAACCAGTTTCTTCATCTTCGTAAGTTTTAGAACGAA
TACAGCCATAAACTGTAGAATTCAGACGGTAAATTTCACCAGTGAAAAAGTTTTCGATTTCGATATGATA
ACAAAAATCGTCTGAAGGGCCATCAGGAGTTGTACAATAAACCACATCACCTTGAACTGAACGAACCATC
CACTGTAACTCGTTAACAACTTGAGATTTATATCCATCATGGTCACGGCATTTAATTGTAGCATTTAAAT
TGATAGTGTTCATTTTATTCTCCGGTTGTTTTATTGATTAGGTGGTACAAGTCTATAGAAACACATCCTA
CAGGAGAGTAAACATCTTTTTAAACTTTTTCTGCCCTAAACGCACAAAAGGGAGACCGAAGTCTCCCTAA
AATTATGCAACAACCTTTCCAAAACGAGAAGCATCATCTCGAAGAACTGCACGTGCTCTGCGCATGATCT
TCTCAACGGTTTGATTAATACGAGAGTTCGACCCACGCTTGTATCCAGCGCGTTTAGAGGTACCATCAAC
TTTCTTTTCAACTGCTTTCTTTGCTTTAGCTTGTTTTGCCATTATAAATTCTCTTTAAATGAAAATGCAG
GACTTATTGGCGTTGCCTGCGCAAGCCCTCAACGGGAACATAGGTTTTGGATATTTAACGACAGGATAAC
CATAAACCTCGTCATCATACCAACCACGACTTAGACAGATAACCTTTTTCTGACGCGTGATAGTACAAAA
TACATTCAAGAGGTACACCGTAAAACTGCCGGGGTCTTAAAACTATAATGATTCGCAAATCATTAATCAG
ACAGTTCGACGGCTCCTCGATTTACTTACTTCAGGGTAATAACAAAATGACGTACTGCTTTACGAGCCGC
AGAAGCCAGAGGCTTAGCAAATTTCAGTTCATCTTTAGCTTCCAGTTCAGCAGCCAGAGTAGCCTGAGCA
GGATTCAGATGTTTGAAATAACGCAGGATTTCCAGTGCTTCGGCTTCAACATCAATAGATGCGCCGTAAT
TGTCATGGCCGTTATTCCATGCATGGCGTTGCAGTTCAAGAGCGTGTTGTAATTGTTTAATCATTTTAAA
TTCTCAATTCGAGATAATATTTAGTAGCCACGTTCTTCTGAATTTCACCTTCTTTCGACAAGTCTCTCAG
TTGTAGGCTACCGCTTAGAAGAGTGTTCTCTTCATATAGTTATTTATATCACTTATAAAGACACGGAATA
GCTTTATAGTGGCATGTTACGAATTTCTGTTTAATTTCTTTAGGTTGTTTAAGTCCCAAAGCCACAAATG
GATGTGGAACATTTCGAATTTGTCCAACCGGTAGTGGAGTCAAATCACCGACTTCAACAAAACCTTCAGG
AACATCAGGACCTACAGAATGAACTACACATAGTTCAGGAACTTCACCTTGAACACGTTTACCAATCACA
AGTCCTGATTCAGTAACTTCTTCATCACCTGCTTGAGCGGGTTCAGAAACCAGAATGACATATTCACCAA
CAGCACGAATTGGGAGTTGTACTTCAGACATCTTATTTCCTTGTTGTTTGACGATAGATTAATAATACCA
TACTATTCGTAAAGCATATTACATAAGAAGCAAACGTTCGACCGGAGTACCATCAACCGAAATTACTTTA
TCAAGGCGGAAAGAACGCCACTGACCTAACTTGGTATCGAATGCTCGAACTGATTCACGAGGTTCAGAAC
 
GAACTTCTGTTGCCGGATTGTTATTTTCATATTTGACTTCAAGTTCATCGCGCGAGGCAAACATCGTACG
AATATCACCGTTAGCCTTTTCAAATACAACACAATGAACGCCTACAGACAGAATAGTTTTAATACGTTCA
CGAAGAACCATAGTTTCTTGTTCAGTTAAAATCATATTATTCACCACAAAAGTTTCGGATTTGTTCCCAA
TTCAGAGAACGAAGATTATTACCGAGAAGTACTGAATTACTTCAATACCAGCTTGGTTTTAGAATGTCTG
CCCAATTTTCAGGAGATCGGTTGTATAATTCTGCGTATACAACCTTTTTAATACCTGACTGCGTAATAGC
TTTAGTGCAATCAGGACACGGAGAAAGCGTAGTATATAACGTAGCGCCTTCAATAGAATTACCTTTACGA
GCTGCAAACAAAATTGCATTCAATTCCGCATGGATTTCGTTATTAGCAGACCATGCACTATGAGCGGCCC
GATGTTCTTTAGCCAAAACAAAATTATCTGCCCGTCCAAATGCTGTAGTGCCTTCTTTATGTCCAGCAAC
GATGGCCGGGCTTGATTTATTCTTCAACCAACCATTTTCGGATGCATGTTCGCAACAGTTAACGCCACCT
GCGGGTGAACCATTATATCCAGTAGAGATAATGCGTCCATCTTTTTCAATGACCGCACCAACTTTCCATG
AACAACATTTTGATTCTTGAGAAATCAAATAAGCAATTTGAAGATATGTGCTAGCTTTCATTATAGTGTT
CCGTATACGCGAGTTGCTTCAACTAAACCGTCACTGCCTAATTTAAGGCTAGTAATCTGGTCACCATCTT
TAGGATTAACGATTATGAGAAAAGAACGTGGAGATTCTTGAATAACTCGTAATGTTGCATCTTGGAAACG
TTCAGACACCTTATTGATGAGTGCTTGAGCAAATTCTTGTACTTTAATCTGAAATTCGTGTACTGTAATT
GGGGTTTCACTTAGCCTTTCGATTTCCCTCATTGTGTTGGTAGAGTTATAGTATCACAACTCCACCATGT
TGTAAACCATTAAATTGCTTTGCATTCAATTTTTTGTGTACCGAAATTGATATCACATTGGATAACTAAA
TCACCGTCAAGATCAAATACCACTATGACGTGTGCAGACCCAGGGAGAAGAGTAATAGCAATAGAATCTA
CTTGGTCTGGGAGCAAAGCATTGAGTACATGAGTTAGTTGAGCATGAACTCTGAGTTCGGCTGCATTATC
AAGCACTTCAAACATATTATTAGCAATAATCTGACTGAAGACTACCTTTACAATTTCAGAATAAGTTGGG
AACATAATTACCTCAGTGTACAGTATTTACTTTAACTCGAACAACAAATCGCTCGGTCGGTTCACCTACA
GGAACAAACTCTACATGATATTCGCCTTTGTAACGAGTATTCAGATCATTACGAATTCCTGCGAGTTTGT
TGATTAATGTAGGACTCATATTAAGTCCCACTAGTTTACGAAGCATTTTGTATGCTTCATCTTCGATTTC
ATGATGTTTATCGTACATAGTTTTCATTTAACCATTCAATTGCCTGGTCGACGTTATCAAATATACCGCC
GTCCAAACGTTCTTCATGCTCATAGTCCAGCACATCAATACCGAACTGTCCATTTACTAAAGGCCAGGCT
ACAAACAAATATTCTTTATGCTGTTCGATGGCCTCAATCACTTCATATAATCGTTTCATTAAAAATCACC
ATGGTTAACTTGCCAACATTCTAAACCAATACGACGCCACATTTCCACAACCTGGTTACGGTCATCAACG
GCTAATTTCACATCATAATAAGGAGCAATACAGTTCCAAAAGAGTTCTTCCTTAACAACATCGTCCTTAC
GAGTGTCACCTTGATTACGTTGAATATGCATTTCCCATGGAATAGAAAACGAATCCATCCATTTTTTAGT
TGCTTCGTAATAACACATTGAATCTTCTTTAGTTCCAGATTCACGACCGCTTACAGTAATAATTGTATAC
CCGGCCTGATGAAGCATCTTAACATATTCGACAACCATAGGGTTCGGGGCGTCTGTGGATAGTTTATCTA
ACTCATACGGACCGCGAGCAACATGTAGAGCTAAAGTCCCGTCAAGGTCGAAGATAACCGCTTTAGGTTT
ACCTGGAGTTCCTTTATAAACTGGAAGACCTTTGTATTCACGCATACGGGAATACATCGAACGTGGAACA
TCGATAGGAACGGTCTTTGTGCCGCGCTTGGCATTACGTTTAACCAATTCGGTCCATGGAACATCGAACA
CTTGATATACAACTTCATGACCCCACTGGTTAGCATATTTTTCCCAGCCAAGACGACGTTCAGGATTTAA
ATTGGTGTCAGAAATAATAACACCCTTAGTTCCATCTTGACAGAGAATCATATGTGCCGCATCGTGTTGC
ATATAAGTAACGATACTTTCTTTCTTTTTAGTATATTTGTACTCGTCACGTTCTTCGTGGCCCATGATGG
ATTGACGATAATCGTCGCGGTTGATATTAAAGAACCCAGGATTCTTAGCAATAAATTCACGAGCCCAAGT
GCTTTTACCAGACCCCGGACAACCTACAGTCAAAATAATCTTTTTCACAGTTTTACTACCTCTTCAGCAA
ATTCAAATTGTCCACCAAGAATTTTAGCAATTTCACGCCCTAGTTCAGCACCTTTCTTGCAATCTTGCAT
AGTCCAAGAATCACCATTCTGTGATTTACGAAGAGCCCGTGTATTGATATCTGCTACAAGCGTATGGATT
AACTTATCAATTTTGGCTTGGTCCATTATTTTATTCCTAAGAAAGTTTCAAGAATACGAATGTTATGCTC
GCGTCGGTCTTTATTAATTTCAGCAACGTTTTTATTAATTGACGTACTTCTAACGTTTGAAGAAATAAAT
TTAATCATATGACTTTTGAGTTGTTGCAAATCCAAACCTTTTTCTTTAGCAGCTTTACGAAGAGCTTTAC
CTGCTTCATCTAGGGCCTTTGCTGCATCTTTATCATAATTGACAAGTGACATTCCACGGCAGATATCAAT
 
ATACTCATCCGACCATTTGATGTAATTTTCTAGTAATATTTTCATTTAGATTTCCTCAATTATCCATAGG
AGCATTATACTCTGCTCCCGAGAGTTTGTAAACTATTTTACTAAAGATTCGATATACTCATGAAGAGCTT
TAGTCGCTACTGCCCACTTGTTCTGATATTCATTTTTCAGATTAGCACGAGACTGAACTAACAAGCGGTC
ATTAGTCGGAGGCAAAGATTCTAATCGTTGAATCTGACGGCCATAAATCATAGCCGCCATTTCAGATTCA
TAGATAAGCCCTTTAATGTATTCATGCTGTTCTTTAGTCATTGGCATTTTTCCTCTTTTAAACCACTACG
ATAATAACATAACATAGTCTTCTGGTCATGTACATATCTTTTTACGTCGTTTAACCAGATACGAAATTCT
TGAGAGTCTTCAAATGGCATTCCAACCCATGGATTACCATCAATAACCTTTACTTGCCATTTCTGGTTAT
ATTCAACAATAGATTCAGGCCATTGCGGATGTAAAGTTTCTTTAGGGCTTAAGGGCACATCCTGGGCACA
TCCAGCCAAAATGCCAATAGATAATACAACTGCGGTTAATTTAATCATTCTGAGAGCTTCCTGAGGTCTT
CTGCAAAGGAATCGAAGGACCTGTTGATTTGTTTTTCGACCAATCCTGGTTTACTTGCCACCACGTTCTT
CTTCTTCGAATCCTTACGGAGCTTTTCATTTTCGGCCTTAAGTTGGTCAACTAAAACACGGTTCTTATAA
GTCATTTCTTCGATTGACTGATATTGAACCTTGAAGTCGTCTAATACTTTAGCATTGTTTTTAGCGGTCT
CTTTGACAGAAGCTAATTCAGTATTCAACGAATCAACTTTGTTGTTTAAGACAACTATTGTTACCGTAGA
AACTAGAGCAAAAGCTGCTACTATAGCATAAATCGGATTTACTTTAAGCATGTTTTAATAATCTCAATGA
TTTCATCTCGGGATAATCCATTGATAAGAACGGTTTTCAAACCATCACACTGGATTTTGTACGATTCAAA
CAATACACACAACTCTTCTGCTGTATGATACGGGCTTGAAATTCCGAGGCGGTTGAATTTGTCGGTAAGA
GGGTCGCAAATGATGTAGAATTTTATACCTGCGATATGTACGTTTGGTTGACTAATATTAATGAACACAT
TAGCGTCGTATTTAGTCAAATTATTTTGAAGAAATTCTACCATGGCATTAACTGCTTCCGGCATCGCTTC
GCGTTTCTCTTCAGCGTATCGGGTGGAATACTCTTTTTGTTTTACTTTCTTTTTAAGATTTTTGGCCAGT
GTAACACGGAGGTCAGTAAGATAACCAACTGGACGTCCTTTCTTAAAGACTCGGATTCCGTCAGTTGTGT
CTCCAAAAGCGGTTACGACCATATCGTTAGTAATCAATTGCATATTCATAATTTTCTCCTCGTGTTGTTG
ATAAAAGCCATAGTAACACCTTCCTTGGTGTTTGTAAACCATTAATATCCTTCAGGGATGAAATTTTTAT
GATTTTTAATAAAGACAGATTCTAGAGCAGTCATCATTTGCTCTTTTGAACCACCTTGGTAAAGATTCAT
AATGATGCCAAATAGCCAAGGCGACTGTGCGCCCTTACAAATGGCCTGGGCTTCTATAGCATATGTTTTA
CGATCTTTTCCTTTGTGTTTGTCGTATGCGTCTAGGCAGATATAAAATGATCGGTCTAAGAAATCGAGAT
AGGCCTTTTCGAAGAGCTCAACCTTTTTAAATGAAAACTCGTCATCCGCATACATTGCTTTAAGGTCATC
TGAAGCACCGTTCATAATAGCAAGAAACAACTTTTCCGGGCTAGAGATTGAGTCCCGAGTTGTATGAAGA
GCAACGTACCAGTCGGTCTTCAATTTGAAATGAGAACCGTCTTTCATTACGGCTACATATCCTTCGATAT
TTGTAGAAGACTTAACACCTGGGACCCAGTCACCTTCCGGTGCTTCAAATCTGTCTACAAGATATTTACG
AAAAACAGGGTCTAAATAAATGTCGTCATACTCGATATATTTGCCGGTATTGTTATCTCGAATGTTCAAA
AGAATCAATCGTTTTTCTGGATATGTTAGAACAATTTTATTTGATGGGGCAACGTATTCAAAGTTCGCAG
TAAAACCGTCATTACAAAGTTCTAATAAACGATCTGCTAAGTCTTTGTGGTTAATATCTAGTAAAATACT
TGTAGCGGAAGCGGCTTGATCGGATTTGATAGACCCTTTAGATTTGAAACGAACCATATTGCCGTCAAGG
TATGTCGAGACTAATGAACCGTCTTCTTTAGTCATCAGATATTTTACATCGTTAAGGTCAATTGACAATG
TAAACGGGTTCTCATTTAGATTAAAGAATTTTTCCATTGGACGAGAAGCAATACGTAGCGGAGTTTCACC
ATCCATTTCGAACATAATACCACGACACTCTAACGCATCAGGCAGTAACCAATCTGAATATGAAGCAAAG
TTATATGAGAAAATTCGATACTTTTTACCGATTGGACTTACGTCGTCCGAGTAGAAAAATTTACGGTCGG
ATGATGATTTACACAGAGATAACAGATTATAATAAAGCTTTTCCATTTTGTATCCTCAGTTAGTTTTTTT
TAAATTGTACCACAATCCTTGTGGTATGTAAACTACTTTTTCTTATGTTGGATATTCCAAGGCGGGTTGA
ACTTTTTGATAAAAAGTGGTTCTTCAAGGTCCATTGTTGCGATGGTCATTGAACCAAGTTCGTTGGTGAC
TGAAAGGTCAAAACATTGGCGGGCCCAGAATTCAACCTTTTTGCCTTGCATTAAGGCATCCAAAATCATA
AGTGATTTTCTTGAATCTGAAGTCTGGTCTTTTCGATTTATGGCTGTTCGATAATAGTTAATGCGTTTCT
TTAAATTCTTGGTTTTACCAATATACACAAGTTCGTCATCTATAGCAATAGCATATATAACATTTTTCTT
ATTAGGGGCATCAATGGTTTTAATTGTTGCATCTTCTAATAGCTCCAATTCTATATATTTAATAAAGGAA
 
TATTCGGCGGCAATATCTTTCATAATAAAGTGGGACCGAAGTCCCGTCCTTAGAAATATTTTTTGTATGA
ATTGATTACATTTTGGTCTACATCATTATCAATTTGTGCGACAAGATAAGAACTGATTTCAACTTCCTGA
GGAGCTGCTTGAACTAAATCAGAGTTCAGATATTCACGAATCCAAGGATACGGATGCTTGGTCGGAGCAT
CAGTAATTGGACATGGCAAACCGCACTGTTTCATACGAGACACAGTCAAGTAATCAACAAAAGCACACAT
ACTCTGTGTATTGATACCTGGACATGTACCGTCTTTAAAGAGGTGTGCAGCCCATTCCTTGTACTTGACT
ATTAACTTCCATAAAAATGTCAACTGCTTCTTGTTCACATTCTTTAGCAATTTGAACCCACTCATCGCCA
TCAGTACCAGATTGAAGTTGACGGATAATGTACTGAGTACCCTGAGATGGAGTTGTTCGTCACGAGCAAT
GAACTTCATAATCTTTGCATTACCTTCCATAATTTCCATATTTTTATGGAAGTTGAAAGTACATGCAAAT
GATACGTAAAAACGAATAGCTTCTAAGGCGTTGATAACATGCAAACAGAGATAAAGAGACTTCATTAAAT
CGCGCTTGGCGTTAAACATTATTTCTTCGTACATCTCAATATCATTCTGCTGGCATGAGCCTATTGAACA
CTCATAAACTTCCTTAGCATTTTCCCAATTACGAGTCTTGACTAGAACATCGTCATAATATTTACCTATT
GATTCAGCTCGTTTCATGATTGCTTCGTCTAATACAATCTCATCAAATACTTTGGATGGATCGGTATAAA
GATTTCGCATAATATGAGTATATGAACGAGAGTGAATAGTTTCACTGAATGTCCATGTTGCAACCCACGT
ATCTAATGAAGGGTCGGAAATCAGTGCCATAAGTACCGCAGATGGGGCCCTGCCTTGGATAGAATCCAAT
AATGATTGATACTTAAGATTGTTTGTACAGATGTCCTGTTGGAACTGAGGAAGCTTATTGAACTGTGCAG
AGTCCAACATCAAGTTTACCTCTTCGGGGCGCCAGAAAAATGACAGGATTTCTATAAGTTCTTCAAAAAC
TTTATGACGCTGAATATCATACCGTGCAAGACCAAGACCAGAACCAAAGAACATCGGTTCGTTCAAAACA
TCGACTTGTTGTGTATTAAAAACTGTACTCATTATATTTTCTCACTTAGTTATTAACTCATCCATGAGTA
AATTATAATCAAAAGTCTTAAAGCTTACAAGCAGAGCAATCTTCAGCTTTAGGTGTTTCTAATTCATAGT
CATCTGTACCTGACCCATCACGAGTGTTATGATAGTAGAGATTTTTTCCGCCAAAGTACCAGAAGTAAAG
CAGATCGTCAAGCATTACCGACATTGGCACTTTACCTTTCTCAAATACAGCAGGGTCATAATAAGTATTA
GCAGAAGCAGATTGGCACACCCATTTCAACATGATAGCAACTTGGGTCAAATAAGGTTTATTACCTTTCT
TAGCAAGTTTCCACGCGTAGTCGTAAAGACTCATGTTATGTTCGATATTAGGAACAACCTGTCGGAAGTT
GCCTTCTTTAGATTCTTTAATACTTACAGGTCCACGAGGTGGTTCTATACCATTTGTGCTATTTGAGACT
TGACTGCTTGATTCACATGGCATGAGAGCACTAAGTGTACTGTTACGAATTCCATGTTCTTTTGAGTTCA
GCTCGAAGTTCTTCCAGTCACAGACGTAGTTCGGGGCTGCGATTTGGTCAATCTTTTTATTGTACCAGTC
GATAGGTAATTCGCCTCTAGACCATTTAGTGTCTGAATAATACTCACAAGGTCCTTTTTCTTTGGCGAGT
TTGATTGAAGCTTTGATAAGGCCATATTGCAATCTCTCGAACAATTCATGAGTCAAATAGTTAGCGTCTT
CATATGAAGCAAAGTTAGAAGCTAACCATGCAGCATAGTTCGTCACACCGACACCGAGATTTCGACGCTT
TTTAGCTTTTAAAGCTTCTGGAACCGGATAATCTTGATAGTCTAACAGGTTATCAAGTGCACGTACTTGT
ACTTCAGCAAGTTCATTAATTTTATCTTGGTCTTGCCAATCAAATTTATCTAATACAAAAGCAGATAACG
TGCATAAACCAATTTCAGCATCAGGACCATTCACATCATTAGTTGGAATAGCGATTTCACAACATAAGTT
ACTCTGACGAATTGGAGCTTTTTCACGAATAAACGGAGTGTAGTTATTCGTGTTATCAACAAACTGTGGA
TAAATTCGAGCAGTACCAGAACGTTCAGTCATGAACAATTCAAAGAGTTCTAGTGCTTTGATTCTTTTCT
TTCTGATTGTTGGGTCTTTTTCAGCTTTCTCATAAAGCTCACGGAATTTATCTTGATCTTCAAAGAATGA
GTAATATAGTTCGCCTGACATTTCGTGAGGGCTAAATAAAGTAATATAATCATTTTTACCAAGGCGTTCC
ATCATGAGGTCATTAATCTGAATTCCGTAGTCCATATGTCGAATACGGTTTTCATCCACGCCTTTGTTAT
TTTTCAGAACTAATAGGTTCTCTACTTCTAAGTGCCACATTGGATAATACGCTGTAGCAGCACCACCACG
AATACCACCTTGAGAACAAGATTTTACTGCAGTTTGGAAATGTTTCCAGAATGGAATAACACCCGTATGT
CGAACTTCACCCATACCGATCTTAGAACCTTCTGCACGAAGCATACCAACGTTGATACCGATACCAGCTC
GTTTAGAAATATATTCAATAATTGATGCTGAAGTTTTGTTAATAGATTTTAACGAATCACCGGCTTCAAT
AACTACACAACTAGAGAACTGGCGAGTTGGAGTACGAGCACCTGCCATAATTGGGGTTGGCAATGAAACT
TGATGAGTAGAAACGGCATCATAAAAACGAATAATATGTTTCAAACGGTCATGAGTTTCGTCTTGGTGTA
AGGCCATGCCAATAGCCATGATAGCAAACTGGGGAGTTTCATAAATCTTACCAGTTGTTTTATCTTTAAC
 
CAGGTATTTTTCTTTAAGCTGCATAGCACCAGCATAAGTCAGATCAAAATCTCGCTCATGTTTAATCCGA
GAATCAAGATAAATGATTTCTTCTGCAGAATATCTAGACAACAACTCAGGATCATATTTGCCTTCATTAA
CACAATAAGAAATATGGTCGATAAAAGCAGGCGGTTCAAACTGCCCATATACATCTTTACGAAGTGCAAA
CATTAGCCCTTTGGCGGCGACATACTGATAATCAGGTTCTTCGACAGAAATCGAGTTAGCTGCAACTTTA
ATACAAATCTTCTGGATATCTTTGGTGCTCATCCCGTCGACAAGATGGGTCTTAATTTCTTCGTACAATT
CATATGGGTCGATTTGTGTTCCTTCACATTGCCAAGTTAAGACTTGAATAATTTTCTGTGCATCAAAATT
TTGGGATACACCACTGCTCTTAGTTACTTGCATATATTCCTCAGTATAGGTTGATAGACATTACTCCAAA
CGGGATAGTATTTATCCCGTTAATTTATTATACACTTTTAAACTGAAGCGTTAATTATTCTTAGCAATAA
TTTCTTTGTATTCTTTAGGTGTCAAGACTGTGATATCGAAATAAACCAATGGTTCTTTTTCCATACCGTA
ACCACCACAAAATTCACGATATTCATAAATTCGTTCATGTGAATAACAAATGAATCGTTCACCTTTAACT
GTGATTTCACTATTCGGTGAAGGGATATCTAGTTCGGTAGGATTTTTCATTTCAAAAATTGTGGTGCCAT
ATGAATTAATAAAACGTAAAATCATTTATACCGCCATTTTAGCTTTGATAGTTGGATGAGATTCGTAATT
TTTAAGAACGAAATCAGAATGTGTCATCGTTCCAGTAACCCAGTCCATTTGGATTTTTGTAGGCCAATTC
TCAAAATTACTAGGCCAATTAATTTCAAGAGAACACAACTCTTTAGGTTCACGACGAAGAACTTCTTTGC
ATTGTTCTACATGATTGCTGTAGATATGCGTGTTGCCGCCTGAGAACACTAAATCACCCGGGATAAGGTT
ACACATCTTCGCTACAATATGCGTCAAGGCAGCGTACGAGGCGATGTTGAACGGCAGGCCCAAGAAAACA
TCTACACTGCGTTGATACCATTGTAAATCAAGATGTCCATTACGCACATTAAATTGATAGAACATATGAC
AAGGAGGAAGAGCCATTTGGTCAATTTCGGCAGGGTTCCATGCAGAAACTATTTGTCGACGATCATTAGG
AAGAGCTTTAATACGGTCGATAGTTTTGACCAATTGGTCTACACCACCAAAATCACGCCATTGCTTACCA
TAAATAGGACCAAGTTCGCCGCTATGATATCCAAGGTCTTTTGCCTGATTTTCATAGTTGTCGTCCCAAA
TAGTTTTACCTTCAATTAGACTACCATGAGTACGAATACGCAAATCATTAACATTGGTAGAACCAGACAT
GAACCACAGTAGTTCGGCAATACAAGCTTTCCATGCAAGCTTCTTGGTTGTTACTGCAGGGAATCCTTTA
GTCAGGTCCCATCGCAATTTAGTTCCGAACAGGGCAATAGTGCCTGTGCCTGTACGGTCGTCTGTTTCGT
AGCCATTTTCTAGGATATCATTAATCAGGTATTGGTATTGTTTCATTTATACACGCTCTCAGTAATTTCG
GTGAGTTCATCGATTCTGTAGTAATGAGATTCTACCATAGAACGTTGAAGCATAATATCTTGGACGAAAT
CTTTATCAAGTTGAACATCGGAATTAACACGACATTTCTTAATGATTTTAGTCATCACGATTTCATCGGC
ATAAGGCAGTGCTTGTTTTAATAATGCAGGACCACCAATAACCGAGACATCAACATGTTCAGTAGAAAGT
TTAAACGGTACGTCGGTTGAAGGGCTAAACAGGTTAATTTCACCACCCATTACCAAGGTAATAAATTCTG
CTTGGGTGATATAGAACCGTGCAAGTTCATCAGTTTTAGTCCTAGGCAGTTTTCTGGCCATATCAGTGAC
AACCATATGAGTACGATCTGGTAGTAGACGAGGTAATGATGCAAAAGTCTTAGCACCCATAACCATTACA
GTGTTTTTAGTGCGAGCAACAAAGTTACTCATATCTTGTTTGATATGCTTCCATGGAAGTCCATCACCTA
ATCCAAAAGCATATTCCAGTTTATCATCAACTGTTTTAGACGGTGCGCATGCGAATACTAATTTAAGCAT
TTTTTATTTCCTCAGATTTTTCTTAATAACTTTCCAATCAGCTTTGAACATTTCAACGTCGGAGTGGTGA
ATCCAGAATCCTGCGCTTTCACCGTCTTCGTAGAGTGGACATCCATCACATTCTTCGTTCCAACCCATTT
CACGTAAAGCTTCTTCAGCTTGTTCAAGGAGTTCAGCGTCTTTACCGACGATGTTGAAGTACCATTTACC
TCTAACTTCTGAATCTTTGATGCTCTGGCGTTGCAATCTCATTTTATTCTCCTCTTCACTTTTATTTGAT
AGGGCTACTATAAAATAGCCCTATCTTGTTATAAACTACTTTTTAAAAGTTTTTTGCAAAAGTTTGATGA
TTTCGTCGACGTTGGTTTCATCTACGATACAATGGATTTTTGTTACACCTAAAGTTATACCTTCGTTAGT
AGGTTGAGGCTTTACTAAATCTACCTCAGTAAAGTATTTGAACTCATCATAATAAATCTCAAAATAAGTT
TCAATTTCACCACCGGCAGAACATACAGTACCATCTTCCATAGTTACACTTACTACGTTTGACGTATCGC
TATCATAATCAGGTTCCATTCCGTTTACAACAAAACTAGGCCCGTGGAGTTCAATCAATTTTAACATCTT
AGCGTTACTGTGTGGTGCTTCTTGAACAAAGTCGTCTTTAAATAAAGGGTTAATGATATAAGATTTTCCA
ATTTCCATTTTGATTTCCTCATTTGTTGATGTGTTGTAATAAAGATTCTGCAAACGTCTCATATGATTCC
AGGTGTTCTTCTTTAATGCGAACTGCATGATTATCACCCGAAGAATAGAACATCTGTACGATAAGAGCTT
 
CGTAATCAGACGAAATACATGCTACGTTTTCCGGCTTCAGGCATTCATTGTATGTCGCAACTGGGTCAGT
GAAAGCTGCAACGTTTTTGCTACCGCAGTAATCCATACTTCTTTGAATAATAAAAATCATTTTATTTTCC
TCTCATTTGTTGATAAGTCTATAGTATCATGTTTAAAGGAAAAGTAAACAACTTTTTTTGGCAATTTACA
GATACTCAAAAGGGAGACCGAAGTCTCCCTAATTTATTATAGACCAGCTAACAGGTCATCGAGGTCGCCA
TCGTCAGAAGACGAGGAACTCATGAAGTCATCTTCAGTTTTTGCAGAACTAAAGGCTTCCATGTCTTTAT
CAAAATCGTCGAGGTCAGAAGCAACTTTATCTGCAACAGAAGCTGCTGCGGCTGCTGCACCACCCAGAGC
GGCAGTACCAAGAACTTGATTAAATTTAGTATTCAATTCTTCAAACGATTTGAACTTATCTTTAGAAGTC
ATTTCAGAAAGGTCAACCATTTGTTCGAACAATTCTTTCTGGAAAGATTCATCATCAATGTTTGGAATCG
CAGATTGATTCAGGAATTTAGATTCGTCATAGTTACTGAAACCAGAAACCTGTTTAACTTTCAGCACAAA
GTTAGCACCTTCCCATGGACAAGTTACATCAACAGGAGTTTCACCCATTTCAGTATCAACTGCAATCATT
GCATTGATTTTGTCCCAAATCTTTTTACCAAAACGGTATTTGAATACCTTACCTTCATTATCTGGAGCTT
GTGGGTCTTTAACAACCAGAATATTAGCCCAATAAGAAGTTTTACGTTTCAGTTGAGAATATTCAGTTTT
GTTGGTATTGTACAGGTCATTTTTACTAATGTACTGACATACAGGACAAGAGTCATAATCGCCGTGTGTA
GATGAACAGGTTTCAATATACCATTTGCCATTTTTCTTGAACCCGTGGTTAACAAGAATTGCAAACGGAA
GTGCGTCATCTGTTTTTGCCGGCAGGAAACGAATTACCGCTTGACCATTACCGGATGCATCGAGTTTCAG
CTTCCATTCACCTTTATCTTCTGAAGAGAAACCTTTGTTACCATTCAGTTTAGCCATCTGAGCTGCGAGG
TCTGCGGTACTTTTACGTTTAAACATTATATTTACCTTTATTTAGATTATTTAATTTATTTACAGTTGGT
TGCTACGACACTTATACCTCGTAGCTGGTATGATTTATTTATAAAGTAATTTTAACACAGATAAAATAAA
GCTTAATACTTACATGATTTTATTGTTTCTATGAACAATTTCTTAGCAGCATTTACATCGATTTGAAGTA
TTTTTCGATATGCCTTTAATTTGGTAGAATAATTTTGCCAGACTAAATTGTCAGTAAGTTCGTCATGCTT
ATCTATAATATTCAAGAATGAATCCAACAACAAGAAGGTTTCAAACGAAATTATATTGCTTTGTAGCAAT
TTAAATATGTAGCTCGACTGAACTTTATTATTATAATCAAATATCTCTTGAAGCGCACTAACTTCCACTT
TCTTACTGAAATAGTAGATGTTACGTACATCTTCAGCAAACGTCTCCTTGGCTTGTTTGAGACGTCCAAT
ATATTCCCTATAGAAAACTAATGCATCAGCGTCTGATATCTCACCAATCCATGCATCTTGGTTAGCAACT
AAGTTACTGATGAATATCAAAGTGAGTTCCTTTAACTTATATTTCTCTGCTAGTTTCTCAAAGAAATATT
TATCTCGGCGTTTTTGATATGCATTATCCGAAACACGCATGCACCAGTTATATTTGATTACGTCATACCG
ACCATTAAAATGTTGTTTGCACATCAAGTATAGTTTGTATACCGATTTTCCATTAATATAACGTTCTCCG
TCGGGAGGCATGCGTATTTTAATCATAATAAGAAATCTAACGTATTAGTTTTTTCACCACGAGCAACTGA
TGGGCGAAGCATGTTTTCGTCAATTGCTTCGCTTGTAATTTTTTCAACAATTCCTGTAGGAATAAATTTA
GCAAATTGTGTTTCTGGAATAGAATTTTCTTCCAAGAAAGCAGTTGTTGCTTCTAAATAAGACATACCAA
ATTGTTCAACCATGGATTCGATAATGAATCCGTTTTCTTGACGGTCAAGCAATTTAGCGATATCATCTTT
ATCATGTTTTACTGCTAATTCTTGTTCAGATAAACCGGTCTCATCAACCGGTTTAATATCACTTAGTGAA
AACTGTGTCATAAAGCTCGACTACCTCTTCGTTTTCAGCTTCAAATTGGTCACGAGCATCTTTATGATAA
AGAGCTAACAAACGATTAAACATTTTTCCATCAACGCCAAGCTCTTCTTTAGCACGAGTACGAATGTCTT
TAATCAGTTCATTATAACCAGAAATTTTAAGTTTGTTATCGGAAGCTTCCTTGACTAGTTTAGCCAGTTC
TTCACCATGCACATCTTGATTAAATTCAACTGCTTCTTTCTTAGCCATGTTTACCTCAGAATTCATTAAT
TACACTTGTTAATTTAGAAAGACCGGATTTTACAAGTATGAATAAATCTTGCCTTTAGGTTGTGGTTGAT
ATGAGTTATAATACTCTATAATGGTTGAAGCAATATTATCAGGGATATAATCAAAATCAATGAGAACCAA
ATTTTCTTGGTACCGTTTATATTCTTCTGCACTTAATAATACTTCAGCTTGAGAACGATCATTGGCAAGT
GCTTCAATGATCGTTGTTTTCATGCTTGGAGTTCGTTCGCCTTCGACTCGAGTAAACCAGAAATCACCAC
GAACTCGAACAGACGCAACACCATCTTTACGGTCGCCTTTAAGAATTTTAGTCATGCAATCAATTTCGGC
AGAACCATTCTTAATTTTAACCCATTTTTTCTGCGGTGGCGACCACTGTTTAACGTTAGGGTATTTGTGT
AATTGTGTAAAGTCACCATCTGATGCAACAATACACACCTTATGACCAGCTAATGACAAATATTTAGTTA
ATACGCCGATATGGTCATCCGCTTCGTATTTGTCAATATCCATTACAACGTATGGCATATATTTCTTAAT
 
CTCATCAACGACTTGATGAAGTGCAGTAAAATATCCTTCCCAATCCCACTTTGAAGCTTCACGATCAGTT
TTACGATTTTTCTTGTAGTAGTAAGCAAAGTCGCGTCGCCAATACCCAGAAGTAGCGTTATCCATGCACA
ATACAAATTTTGTATAACCTTGCTTACGGAACATCACTACGTTTTTACGAATTGAATTCAAGACTACATG
ACGAACCATCGGAACGGTAATTTTATCACCATCTTCAAAGTTGTTTAATGCAGCTGCCAATGCAATGTTA
CTAAAGTCTGCAAGCGCAATACCTTCTTTGTAATCTTCATCCAACATCATTTCTAAATCCATATGAACCT
CTTGTTCAGTTAGTCAACTTAGACCATAGTAACATACTAGTTCGAAAGCGTAAATAGTCTTTTACTCATT
TATAAATAGTAAAATAGAACACATAATGATAAGAGGACTACCATGGTTGACATCAAACGTAAGTTCAGGG
CCGAAGATGGTCTTGACGCAGGCGGTGATAAGATAGTTAACGTTGCGCTTGCTGACCGCACAGTTGGCAC
CGATGGCGTGAACGTTGACTTTTTGGTGCAAGAAAACACCGTACAGAATTATGACGAGACGCGCGCGTAT
ACAAAAGATTTTATTGTTCTTTATGATAATCGTTTTTATCAAGCTTTAAGCGATATTCCAGCTCCGGCAG
GCCCTTTCTCTTTGGCTAAATGGAAGGCAACTCGTACAGATGCAGAATGGATTACAGTTCAAGGCGGAGA
TTTCCAATTATCAGTAGGTAACTCTATTGCGGTTGATACTTCAGCTGGCATTGATATTAATTTCACTTTG
CCTCAAAATCCTTTAGATGGCGATACAGTTATTTTGTCTGATATTGGCGGTAGAGTAGGCTATGTAAAAG
TTCAAATTACTGCAGAGGCTCAAAGTATTGTAAACTTTAGAGGACAACAGGTTCGTTCAGTTTTAATGAC
GCACCCAAAATCTAAAATAGTATTCATTTTTAGCAACCGCCTATGGCAAATGTATGTTTCTGATTATGAA
CGTAATGCAGTCACAGTAACTCCTGCTAAGCCATATCAGGCGCAACCTAACGATTTTATCATTCGACGTT
TTACATCAGCCGCTCCTATTAATATTACATTGCCTCGCAATGCTAATAATGGCGATATTATTAATTTAGT
TGATTTAGACAAATTAAACCCGTTATATCACACAATTGTCAAAACTTACGATGATACAACTTCTATACGA
GAAGCCGGTGTTCATGTAGCAGAAGGACGTAATACTGCAGAAGCGTTCTTTGTTTATGATTCTGCTAATA
GCTTATGGCGTGTTTGGGAAGGTGACCAGAAATCTCGTTTACGTATAGTTCGTAATGATACTGATTTACG
CCCTAATGAAGAAGTTCTTGTTTTTGGAACTAATAATGATACAATCTCGACGGTAAATCTTACTTTGCCT
ACAGATATTTTGTCTGGTGATACTGTTAAAATATCGTTGAATTATATGCGTAAAGGGCAAACTGTAAAAA
TTAAAGCTGCTGAAGGCGATACAATTGCTAGCAGTATTTCTTTATTACAGTTCCCAAAACGTTCAGAGTA
TCCACCTGATGCACAATGGGTTTCTGTTAGTGAGCTGGAATTTAATGGCGATACTTCGTATGTCCCTGTA
CTTGAGTTGGCTTATATAGAAGATTATTCATCTGAAACAAAATATTGGGTAGTTCAACAAAACACTGTAA
CCGTCGAACGAGTCGATGCATCGAGCAATACAACTCGAGCTCGTTTAGGTGTTATTGCTCTTGCTTCTCA
AGCACAAGCAAATGTTGATTTAGAAAATGCTCCTGGTAAAGAACTGGCTATTACTCCGGAAACATTAGCA
AATCGTACAGCAACTGAAACTCGTCGCGGTATTGCTCGTATTGCAACAACTGCACAAGTTAACCAGAATA
CCGATTTTGCATTCCAGGACGATTTGATTATTTCTCCTAAGAAATTGAACGAACGTACAGCAACTGAAAC
CCGTAGAGGTGTTGCTGAAGTTGCAACTCAGGCTGAAACTAATGCTGGAACAGATGATACCACTATTATT
ACTCCTAAGAAATTGGATGCCCGTCAAGGTTCTGAAGTTTTATCTGGTATTGTAAAATACACATCTACGA
CTGGTACTACAGCTGCTACTGTTCGTGGTAATGCTGGGACTAACGTTTATAACAAAGCCGTAGATAATTT
AACTATTTCTCCAAAGGCTCTTGACCAGTATAAAGCTACCCATACTCAACAGGGCGCAGTAATTCTTGCT
ATTGAAAGTGAAGTTATCGCTGGCGAATCACAAGCCGGTTGGGCTAATGCTGTAGTGACCCCTGAAACTC
TGCATAAGAAAACTTCTACTGATGGACGTATTGGTTTAATTGAAATTGCTACGCAAGCAGAAACTAACAC
TGGAACTGATTATACTAGAGCAGTAACGCCTAAGACGTTAAATGATAGGAAAGCTACGGAAGGATTATCC
GGCATAGCCGAAATTGCTACGCAAGTTGAATTTGATACTGGAACTGACGATACTCGTATCTCGTCTCCAC
TGAAAATTAAAACTCATTTTGATTCTTCTGACCGTACCAGTGTTAATTCTGATTCCGGACTTATTGAAGA
AGGAACCTTGTGGAACCATTATACTCTTGATATTTCTAAAGCAAATGAAACACAACGCGGTACACTTCGC
GTAGCGACCCAGGCAGAATCTAATGCAGGAACTTTAGATGATGTTCTTATTACTCCTAAAAAGCTTTTAG
GGACTAAGTCCACTGAAACGTCTGAAGGCGTGATTAAGGTTGCTACTCAGGCTGAAACTGTAACAGGAAC
TTCTGCTAATACTGCTGTATCACCTAAGAATTTAAAATGGATTGTTCAATCAGAACCATCATGGACTGCT
ACTACGGCAATTCGTGGATTCGTTAAAACTTCATCTGGTTCTATTACATTTGTCGGTGACGATACTCAAG
GTAATACCCAGGATTTGAATCTTTATGAGAAAAATAGTTATGCTATTTCTCCATATGAATTAAACCGTGT
 
ACTTGCTAACTACTTACCGTTGAAAGCTAAAGCAGCTGATAGTAATTTGTTAGATGGTCTAGATTCTCTT
CAGTTCATCCGTAGAGACATCGACCAGACGGTTAATGGTTCTTTAAGTCTTACTAAACAGACCAACCTGA
GTGCTCCTTTAGTATCTACAAGCACCGCTTCTTTTGGTTCCGAAGCATCTGTTACTCGTAGATTAACTCT
TAATGACTCTAGCGGTTCCGAAATAGTTTTCACTAAAGGAACCCAATCTCTTAGTAATAAAGAGAATTTC
GTTGTTAGAGCATGGGGTAATAGCGCTACAGCTGGTGACCGTGATACAGTATTTGAAGCGGGTGACGAAA
CCGGATACCATTTCTATTCTCAGCGCGCTGCTGATAATAAAGTATCATTTAATATTAATGGAACACTTTA
TTCAACAGGTATTGTTTCTACAAATGGATTAAATGTTACAGGTGTTTCTACCTTTACCGGGCCTATTAGT
GCTACAGGCGAAATTGTTTCTAGTTCTCCTATTGCATTCAGAGCTATTAACGGTAACTATGGTGTTATGC
TTTATAACGCTGGTAACAGTTCTTATATTGCATTAACTAACTCAGGTGACCAGACCGGGACGTTTAATAA
CTTACGCCCGATCACGATTAACAATGCCACAGGATTAGTTCGTCTTGACAACGGTGTTCAAATCACAAGT
GGTGCAACAATAACTACCGGTGGGTTAACTGTAAATAACAGGATTATTTCTAACGGCGTTAAAACTGCCA
CGGTTTATACCGATAAACCAACAGCTTCTACTGTAGGTTTTTGGTCTATTGACATTAACGATTCTGCTGT
ATATAGCCAATTCCCTGGATACTGGACGCGTGATAACAAAGGTAACCGTGACCAAGAAATTAAATATCCT
GGTACTTTGACTCAATTCGGCAATAGCTTAGATTCGCTTTATCAGGATTGGATTTGTTATCCTACAGGTG
CAAATGGTGGTAGTATTCGATATACTCGTACCTGGCAGAAAAATAAAGACGCTTGGACCTCATTTGCAAT
GGTATTTGATAGTGGCAACCCACCTTCACCTAGCGATGTCGGTGCTATCCCATCCGATAATGCTGTGATT
GGAAACCTTACTGTTCGAGACTTCTTACAATTAGGAAATGTGAGAATTGTTCCAGACCCGGTTAACAAAA
CCGTTAAATTTATATGGGTTGAATAAGAGGTTATATGGAAAAATTTATGGCAAGGTTTGGACAGGGATAC
GTCCAAACGCCGTTTTTATCGGAAAGCAATTCAGTTAGATTTAAGCTAAGTATAGCGGGTTCATGCCCGC
TTTCAACAACTAATCCGTATATTTTGTTCCAAAACGAGCCTTTAGGGTTGCAGTCTTTTGGTATTGGACT
AAATGTTAGGGTGATAAATCCGGAAAATGGAACTATAGTTGATAGTAAATTATACAATTTTGCGCCTACA
AATAATGCAACTTCAGCTGCATTTATTTCGTTTGTTAATACATACGCAGATAATTTCATTTTCGCATTTA
TTTCTAATAACAAATTTAATTTGCCGCCAGAAATAATTGAATGGTTTAAAGCTGCTGGGAGTTCAGTTAT
ACCGTCAATTGAAGTTGCTAGTCTTGTTGATATTTCATATTCAGCGTTTTATGTTTCAGGTAAAAATACT
ATTGCATTAGAACACATAAAATACAGTAATAAGAAAACAATTTCTGATTACGCAACTCCATTAGATATTG
TATATGACTCTATTGCTGATATAGGTGCTACCGGCTATCCTAGACGTACATATGAAGCTCTAGAAACGTT
TTTATCTCCTGTTGGAGGAACTAACAATGAAATAAAAAGGGTGCCTACATCATCCCTTGTTACTCCTATC
GCAAACTATGGATTAAAGCCTACAGATTTTCTTTATTTGAAATTTCAATTAATGGCTGATGAAGAATTAT
TAGAGGAAGGAACCACAAGACTTTCAATTCGTTTCTTTAAGTCTCCATCTAGCTCTCCTATATCATCTAA
AGATATAAACTTTGATGGAACTGCCGGGGAGTGGAAGCTATATGAAGAATACGTTGAAATACCAGCTGAA
GCTGATGGCTTTACAGTATATTGTTACCGTACGGCCTCAGTCGGTCAAGGTGGACTAAGAAATGTTATTT
TCACTGAAGTATCATGTAACGGAAGTATAGCAAAACCCGCCGAGTTTGGTATAAACGGTATTCGTGTTAA
TTACATTGATGAATCATTAACCGGTAATGATATAATGGACTTGCCTACTCAGTTATCTAACGACACAGGT
AAGGTATTTGGGCAGGAATTTAAAGAGTACACAGAATAAAGGAGACTTCGGTCTCCTTTTCGCGTATAAA
TACTTTAATATTAATAAGGAGACACGAAATGGCCGATTTAAAAGCCGGAAGTACAGTTGGTGGTTCCGTC
ATATGGCATCAAGGCAATTTTCCTTTAATGCCAGCTGGCGACGACATCCTATATAAAACATTTAAATTAT
ACACTGAATATAACAAGCCAAAAGCGGCTGATAACGATTTCGTATCTAAAGCAAATGGTGGGACATATCT
CAAAAAGGTAATCTTTAACGAAGGTGTTGCAGTCAAAACAGCAGATGACCAAACGAATGGTATTTTTTCT
GGTGGTGGTGACGCCGCGACATTCGACCAAACAAACATGGATATTGTTTCGTGGTATGGTATTGGGTTTA
AATCATCTCAAGGTACAGGTGAAAGAACTGTTGTAATTGATACTCGCACCGGTAATATAAGTTCTAAAGG
CGTTATTGAAGCATCTCAATTCAGAGCCACAACGTTAGCACCTTTAAATAATTACGACCTTACTCGCAAA
GATTATGTTGATAGTCAAATAAATACAGTTAATGCTAATGCAAACAGTCGTGTACTCCGTTCAGGAGATA
CGATGACCGGCGCGCTAACCGCCCCAAACTTTTTCTCACAGAATCCTGCATCTCAACCCTCGCATGTTCC
ACGATTTGATCAAATTGTTATTAAGGATTCTGTTCAAGATTTCGGCTATTATTAAGAGGACTTATGGCTA
 
CTTTAAAACAAATACAATTTAAAAGAAGCAAAACTGCAGGAGCACGTCCTGCCGCTTCAGTATTAGCCGA
AGGTGAATTGGCTATAAACTTAAAAGACCGTGTACTTTTTACTAAAGATGACCAAGGAAATATTATTGAT
CTGGGTTTTGCTAAAGGTGGTAGTATTGACGGGAATGTTATTCATACTGGCAATTATAACCAAACTGGCG
ATTATACTTTAAATGGCACCTTCACTCAGACAGGTAATTTTAATTTAACCGGTATTGCTCGAGTAACTCG
TGATATTATTGCCGCTGGGCAGATTATGACTGAAGGCGGTGAACTTATTACAAAAAGTTCAGGTACAGCA
CATGTTCGTTTTCATGATTCGGCTGATCGTGAACGTGGCATTATTTATGCACCGGCTAATGATGGATTAA
CTACGCAAGTAGTTAATATCCGCGTTAAAGACTACGCCGCTGGTGATGAAAGCACCTATGCATTTTCGGG
CAGTGGACTATTTACTTCACCTGAAGTGTCAGCATGGAAATCTATGTCAACTCCTCAGATTTTGACAAAT
AGAGTTATTACTAATAATAAATCTACGAGCGATTATGACATTTATTCTATGGCAGACAACGTTCCATTGT
CTGAAAGCACTACTGCTATTAATCATCTCCGTGTTATGCGCAATGCTGTAGGCGCTGGTGTATTCCATGA
AGTTAAAGATAATGATGGAATAACTTGGTATACTGGAGATGGCTTAGACACTTATCTTTGGTCGTTTACC
TGGTCCGGTGGATTGAAAGCAGGCCATTCTATTTCTGTTGGTACTCCTGGTGGCAATAAAGGATACTCTG
AATTAGGGACTGCTTCAATTTCTCTTGGCGATAATGATACAGGCTTAAAATGGCATCAAGATGGATATTA
TTTCAGCGTTAATAATGGAATAAAAACGTTTTTATTTAGTCCCAACGAAACAACTAGCCTAAGAAAATTT
GTAGCTGGATATTCTACCACCGGAACCGATTTAACTACTCCTCCAACTGAAAACTATGCTTTGGCCACTG
TTGTTACTTACCATGATAATAACGCGTTTGGAGACGGTCAGACTCTTTTAGGATATTATCAAGGCGGTAA
CTATCATCATTATTTTCGCGGTAAGGGTACTACAAACATTAATACTCACGGCGGTTTGTTAGTTACTCCA
GGTAATATTGATGTTATTGGCGGTTCTGTTAATATCGATGGTAGAAATAATTCTTCTACACTGATGTTTA
GAGGTAACACGACCGGAGCTAGTTCAGTTGATAATATGACAATTTCCGTTTGGGGCAATACGTTTACTAA
TCCTAATGTAGGTAATCGTAAAAACGTCATGGAAATTTCTGATGCAACTAGCTGGATGAGCTATATTCAA
AGACTTACTACTGGTGAAGTAGAAATGAACGTTAATGGTTCATTTGAATCATCTGGTGTTACTGCTGGAG
ATAGGGGAGTTCACACAACAGGCGAAATTTCATCTGGGGCTGTGAATGCGCTTCGTATTTGGAATAATGA
TTATGGAGCCATTTTTAGACGTTCAGAAGGCAGTCTTCATATTATTCCAACTGCTTATGGTGAAGGTAAA
CATGGTGATATCGGTCCACTTCGCCCGTTTAGTATGGCTTTAGATACCGGTAAAGTTACTATTCCAGATT
TACAATCAAGTTACAATACGTTCGCTGCGAACGGCTATATTAAATTTGTTGGTCATGGCGCTGGCGCTGG
TGGTTATGATATTCAATATGTTCAAGCGGCTCCTATTTTCCAGGAAATCGATGATGATGCTGTAAGTAAA
TATTATCCTATTGTTAAGCAGAAGTTTTTAAACGGTAAAGCAGTTTGGTCTTTAGGTACTGAAATTAATT
CGGGTACATTTGTTTTACACCATATCAAAGAAGATGGATCACAAGGCCATACATCTCGTTTTAATCAAGA
CGGCACAGTTAATTTCCCTGATAATGTTCAGGTTGGTGGTGGTGAAGCTACTATTGCTCGCAACGGTAAC
ATCTGGTCTGATATCTGGAAAACGTTTACTTCTGCCGGTGATGTAACTAATATCCATGACGCTATTGCTA
CTCGTGTTGCCAAAGAAGGTGATACGATGACAGGCCGCTTAACTCTTAATGCAAACTCGGATGTTATTGT
TATTAACAGTGCTGCAACCGAATCTGGCTATTTGAAAGGACAAAAAGCCGGCGTTGATAACTGGTATGTA
GGTAATGGTGGGGCTGATAATGCCATATCGTTTTATAGTTTCCGAAATAATTCAGGCATAAATGTTAATA
ATGGTGGCGATATTGCTTTAAACCCAGAAGGGTCCGCCACTTTTAATTTTAATAAGGACCGACTTTATAT
AAATGGTTCGGTTTGGACCTCACACCAGGCCGGCGATTGGGGAAATCAATGGCGCCAGGAAGCTCCTGTA
TTTGTGGATTTTGGCTACGTCGGTAATGATAGTTATTATCCAATTATCAAAGGAAAATCTGGTATTACTA
ACGAAGGATATTTTTCAGGCGTTGATTTTGGTATGCGACGCATTCCTAATAACTGGGCACAAGGTATTAT
TCGTGTTGGTAACCAGGAAAATGCTAGCGATCCGCAAGCTATCTTCGAATTCCACCATAATGGAAACATG
TACGTTCCTGACATGGTTAAAGCTGGAGTAAGATTATCAGCTGGTGGAGGTGACCCTGCATGGACAGGCG
CATGTGTTGTTATTGGTGATAATGACACTGGGTTAGTTCATGGCGGTGACGGTCGTATTAACATGGTTGC
AAATGGAGTGCATATTGCTTCGTGGTCAGCTGCTTATCAGATCCATGAAGGTCTTTGGGATTCAACTGGT
GCTTTATGGACTGAAACAGGAAGAGCTATTATTTCTTATGGGCATTTAGTCCAACAAAGTGATGCCTATT
CCACATATGTCCGCGATGTTTATGTCCGCTCTGATATTCGTGTTAAAAAGAACCTCGTTAAATTTGAAAA
TGCTTCTGAGAAGCTTTCTAAAATTAACGGTTACACTTATATGCAGAAGCGAGGCCTAGATGAAGAAGGC
 
AATCAGAAATGGGAACCTAACGCCGGTTTAATTGCTCAAGAAGTTCAAGCTATTTTACCAGAATTAGTTG
AAGGCGACCCGGATGGTGAAGCTTTACTTCGTTTGAACTATAACGGTGTAATTGGTTTAAATACAGCTGC
AATCAATGAGCATACTGCAGAAATTGCAGAACTTAAGTCAGAGATTGAAGAGCTTAAAAAATTAGTTAAA
TCATTGTTAAAATAATAAGAGGGGCTTCGGCCCCTTGGAGGTGTATATGGCAGTTGAAGGACCGTGGGTA
GGATGGTCTGCAAGGGTACAGACTGGAGATCCTTGGATGGGTGGTGCCGCACAGAGATTGAGAATGGGAA
TTCCGTTTTGGATGAGTAATATGATAGGTCGATCTGTTGAAGTAATTCACACGCTTGGAGCAGACCATAA
CTTCAATGGTCAATGGTTCCGAGATAGATGTTTTGAGGCAGGTAGTGCACCTATAGTGTTTAATATCACC
GGAAATTTGGTATCATATAGTAAAGATGTTCCATTATTCTTTATGTATGGCGATACACCAAATGAATATG
TTACTTTGAATATTCATGGTGGAGTTCATATGTGGGGACGAGGTGGTAACGGTACTGTAAACGGAAACCC
AGGCACAAATGGCGGCGATGTAATCCAAAATGATATCGGCGGAAGACTTCGTATTTGGAACTATGGCGTT
ATTGCATCAGGCGGCGGTGGTGGTGGTGCAGTGTCATTACAGAATAGCTGGGCGCCAAATGCTACAGCAG
GCGGCGGTGGTGGTAGACCATTTGGCATCGGCGGAGGCGGCGTTAATTGGCCGGGTGGTAATGCTAGTTA
TGACGCCCCGGGCGGTGCCGGTTACACTTCCCAATTCGGCGGAGGCAATGGCGGTGATGCTGGAGGTCGG
GGCGGAGATGGATGGGGTAATCACTTATCTAGATCTGGCGGTGGTGCTCCTGGCAGAGCAGTATTCGGAA
GTTCACCGTCATGGGGTGCTACAGGCACAATCTACGGCTCCTGGATTTAAACGATAATACCTCTCAATTA
TAAATATTGATAAATGGGAGGTAATATGGCAGCACCAAAAGTATCATTTTCGCCTAGTGATATTTTATTT
GGGTTGCTAGACCGCATTTTCAAAGATAACGCCTCTGGAAATATTCTTATTTCAAGAGTTGCTGTTGTAG
TTCTTTTGTTCCTAATGGCACTAATATGGTACAAAGGGAATATTTTTATGGATTATTACGTGAGGTCGAA
ATATGATACTTACACAGAAGTAATTCAAAAAGAAAGAAATACACGATTTGAATCTGCGGCTTTAGAACAA
CTACAAATAGTCCACGTCACATCAAGGGCGGATTTTAGTTCGGTGTATTCTTTCAGACCTAAAAATCTAA
ATTATTTCGTCGACCTTATTGCGTATGAAGGTAAGTTACCTAGCACAGTAACTGAAAAATCTATGGGAGG
ATTTCCTGTCGATAAAACGACAGCAGAATATTCGGTCCATTTAAGTGGACTTCACTTTACTTCTAAAACA
GATTTTGCTTTCTTACCTACCAAATCAAAAACTCCGGAACTGGCATATATGTATAGTTGTCCTTACTTCA
ATTTGGATAACATATATGCGGGAACTGTTTCTATGTATTGGTATAAGGGGTCGGATGTGTTAAATGAAGA
ACGCTTGGCTGCAATATGCAACCAAGCAGCAAGGATATTAGGGCGGGCTAAATAATTAGTTGTTAGAATA
CATCGTCAAATGGCGATAAATATTTTCAAAACCTTCATTGAATTCACTAATCAGTGTTTTCTTTTCTTCG
GCAGTTAAATTTGTAATTAGTTTTCTGAACGAATTCTGATTTAGCTGCCGACCATCTTTTTTAATTCCAA
TCTCATTCAAGAATCCGATGAAATTTGCTCGGTCTTCAACAATATCTTCCCTAGAGAATTTAATCAAAAT
AGAAGCAATAGTAATAATTTCACGTACGATTTCAATGTCTTTATTCATAACGTTTTATTCATTCCATTTG
GTTCGATATGAGTATAGTACCATTATCAGTGTTGATTGTAAACAGCTTTTTCTACACTTTTAACGTAATC
AGTGTAGTCTTCCTGTTCATACGAGTGTAAGGCCAAGAACGTGGCTAATAGTTCATTATAGACTAAAGCC
TCACGCTCACGTTCGGCCTCTACTACGTTCTGAAGATTATGACTCATATTGTTCCTTATAACTATCAATG
TAGTCCTGTATGTCGAAGAATGACATTTCATATTCTAGGACATTAAGAGCCCGTTCTTCAGTATAGTCAG
AACCAAATACGATAGTGATTTCACCAGGCTTCATACGATCGAAATCAACACTAAAATGAATACGAGAACA
TACTTCGTCTGCCATATCTGCAACAACACTACGATACACTTCTCGGTTAACCTGATAAAGCTCTGCAAGT
TCTTCACATCGAATAACAAGTTCAGTGGCAGGATTTTCTTTTATTTTAGTAAGCATAGTCTTTACCCTTA
TGTTTTTGTTTGCGTTTAGATTGAAGTTCCGATTTCTTCTTGTCCTTATGGACGGAAGCCTTATTGAAAT
CATGTTTTGCTACAAGATTGTTCATAATGATTTATAACCTCATTTAAACATTTATCGGTAAAAGAAAACT
TTTTAGCCACAGAACATTCGATAATGTGTTCTTTCAAATCTTTAGAGTCAGTATAACCCATCTCAGTAAT
AAAGTGGCGAATTAAAGCTTTATTCTTATCATTAACAGAATTAAAATAATCTGCTTTAGAATCAATTATT
CTCATCATCTAATTTCCTTAATTGATATTGCAACGAGCTAATAATTTCTTCGTTCATTTTAATATACATG
TCACGCAAGAAAGGTTTTAACCCTTCACGAATAATTGCTAATGTATCACCTTTACATAAATTAACCAAAT
ACGGATGATCTAAAGCGTCCCAACCAGGCTGATTAGCATACATGCCATAGTTTTCTTCCATCATATGATT
AAGACGTTTGATATCTTGGATACGTGATTCAATAGCATCTTTCTGGAGTTGTTTAATATTCATTAATAAA
 
GGTCCTCTGAATAAAGTTCTTTCTCACTTCCACCACGTTCAATACGTACCTGTCCAGCATACGTTGCAAT
AATCATTGCTTCTTCACGCGTCCAGTAATTAGAGTATTGGTCAATAAAGCCTTGGTCGTCACCACAAACT
TGTTGCGAGACTAATTGAGGACCAACTATATCAAGTACTTCAGCCATATCTTTAGAGTAATGACGAACTC
CAGGAATAATCAGAGTTCCGCCGCTCTTCAATTTAAATCTATTAGCTGCACATACTATAACACGTTGAAC
TTTTTGGTCATTGTTCCAATAGCACGTTTGCCAACAGATTTCAGGAACTTCATTCATTATATCATCAGCA
GAATAATTATAACCGTAGGACTGGAATTTTTCTGCTAGACTTTCAGGAGTCTCACGAGACAAGGCCAAAT
CTAATAGCTCTAAACGTTCTTTAAATGATTTCATTTAAGCCATTCCTTAATACGTTGCCAAATAGATTTT
TGTGATTGTTTATTTACACCAATCGAACGAATAACCGGTTGTGACTGCTGATATTCTTTATAATCTTCTT
TATAGATTTCGTAAGCTGCATCAACAAATGAACTGATCGCTGCCATGTAGTTTTTACGAAGGCCAAATGG
CGCGTCATTATTTTCACGAATAACCGTATATTGGCCTGCTTTAATCTTTACAATTGTCCCTAAATAAGCC
CCGTGGTACCAAATATCCCATCCTTCCTGAGTAGGTTCTGCACATTTACGAAGTTCGTTAATAATATTCA
GCTTATTCATTATGTATTCCTCACAGTACGTTAGTTACAAGGGTAATGATTTCTTTGGACACATTAGGGA
AGTCAATGTAAGTATATTTACCACCGATCTTAATCTTAACATCATTTTCAAGAGAAGTAAACAGCTTTGA
TTCAGTTTCTGACATGTTGTATGCAAAGATTCTAAGAGCGCCATCTCGTCGAATTTCAATTTGGCGAATG
CCTAGAACTCGTTTAGCGAATCGAACTTCTAAGTTACTACGGTTTTCACCAATTTCTTTAACTTCAACTT
TATCTTTAATATTTTCAAAAACAAAGTTTGCTAATTCATGCATTTCAGGAGTAACTCCACGTGCTTTTCT
GGTGTTACGTTTCTGAAGAAGTTCTGGAGCATTTTCTTGGGCAAAGAGGTCTGCAGCTTTTTGGATAATA
TCCATTGCTTCGCCGGTTGCCACAAGTCCATCACCAGATTTTTCGACTAAACCTTTCTTAATCAATACAC
CAATGTTAGAGTTAACAACTGATGCATTGTATTCTGCTTCCAGAGCTTCACGGACATTGGCAGCAGTGGT
GAAGTTGTTCTTAATGATATAAACCATTATTGCAGCAGTTTTTTCATTCAGAGCGTTTTCAGAAGCTTTG
ATGATGTAAGTAACTTTAGACATTTTATTTCTCCAAATTAGTATTTGTTTTGATAGGTCTATAATATCAT
GTTTGAAGCATATGTAAACTGTTTTGTGAGAAAAAAATGCACAAAAGGGAGCTTCGGCTCCCTAATCATG
TTTGTCCAACCAATACAGCCAAATCACTAATGCAGCTATTGCACCGAGAAATCCTAAGGCTAGGGCACTC
AAAGTGCCTCCAAGTCTTTAGTGTACTCTGTCACAACATCAGTAGACTTCCAATACTCATTTTCTTCTTT
CTTAGCTTTAGCTTCTTCTGCCAGTTTACGTGATTCATCGGAAGTGATATGGAAGATGTTAAGACCTACC
AGTTTATCGACATGTTCTTTATACATTTCGATTTGTGCTAGCTCTTCAGTCAAAGCTTTACGAGTCTTGC
CCTGAATCACAATTTCGCCACTGATAACTTTTTTGATGAAGTGGGCCTTAGCAAAAGCAAGTTTAAATGC
TTTTTCAGATTCAAGGATTTTGTTGTCAATACGTTTCTGAACGTAAGTCTTACGGACTTCAACAAAATCT
TTGATTAAGTCCACTACGTTATCGTAGACAGCTAATTTACCCTTTTCATTAATCACTGTGATGTTTTGTG
AACGACGTTCGATGAGACTAAAATCTTTCATGATTTTTTCATGACGTAGCTCTTCATCATCAGGAAGCGA
GTACTCTTTACGGAACTTCACCTTAAACCCAAAGCCGTGTTCACCGCAATCATCTTCCCATGAGATTAGA
CCTTTGTCTTCCAACGGGTCAAGAACCTTACTCACATACGTTTCACGGTCGAATTTATATGGGATTTCAG
TTATCTGCATTTGAGTGCGAGATGTGAACTTATATGTTCCGTGAATCTCATAACGCCCATCAACTTCATG
TACTTCACCACGGAATTCCGGGAATTCAACTTTTGGCTTAGTGACATTTTTACCTTGAAGAGCTTGTAAT
ACGGCCTTTTTGACCGATTTAAAACTATGAGGCAGAATATTTGTTGCGTAACCGGTTGCAATACCGGAGA
CACCATTCAAAAGAACTGTTGGAATAATAGGAAGATAGAATTTTGGCGGAATGTGTTCTTTATCTTCATG
AGCTGGAGCATATTCAGTATCTTTATATACGTTATAGAAATTTTTACTTACACGAGCAAAAATATAACGA
GATGCTGCAGCCTTTTGGACTGTTCTAGAGCCAAAGTTGCCTTGGCCATCTAACAGTGGATAGTTGTTAT
TCCATGTGTTAGCCATCAATGCACCTGCATCTTGTGCAGAAGATTCACCATGGTGATAACCTAAATCTGC
AACACCGCCTGCAATTGACGCAAGTTTATGAAATTTTTCTTTATTACCACGAGCCAAATCTAACGCACGA
TGAACCACAAAACGTTGAACCGGCTTAAATCCATCAATCATGTTCGGGATTGCACGGTTTTCAACGGTGT
ACATCGCATATGCTAAGGCTTCGTTATCAATAATACTTTTTAGATTACGAGAATTCAATTGCATATTTTC
ACCATATTAATGAATGAATAACCATTATAACATCTGAAATGAAAAGCACTACTTGGACTAGTCCAAACAT
CACACCATAGTACAGAACCGTAAGCAACAACAAGAGCCCTAAGGCTCCTGCTAAGATTTTCTTAATCATT
 
ACTCGCCTTTTAATACGTTAAGAAGAGCTAAGACTGCTAAGGTACCTTGTGATACTGCTTGTTGAAATAC
TGTTGTTTCGCCTGTAACTAAAGTGTTAACGAATAAAAACGACCAACACAGCAATGCAATAATCCATGCT
ACCATTTTAATTACCTCACAAATAAATTAAAGGCCTATTAAGCTAGGCCTATATAAATTAGTAACTCTGC
AGAATGAATCTGAAATTATCTGCCAGAAGACGGTTCATTTCAGTGAGTGTTTGATATTCTGATGAATGAT
TGCGAGTGAACGCCAGAGCCAATTGACCTTTACCGTAACCAGTAGTCAGAGGTTTCATGTTCTTAGCTGA
AACAAAGATTACATCATATTCAATATTGTTAGTGCCCATGGTACGGGCTAGTTGCGAACGGCCTTGACGA
ATGTGAGAAAGAATTGCATCCAACCCTTGTTTAGAACGTTGACGTCCTACAAAGAATCGTGCTGCTACTT
GTCGCCAATCAGATGAACCTTTAACAAAGAAGTAAAAACCTTCACGAGAACGAAACTCTTTAGAAACTTC
AGAACCGTATTCGCCATTACGAACTACTGCAACAACTTCACCGCCAACTGCCAGAAGGTCTTTACGAGTT
AAATATTTGGACATGCTAATTTCCTCAATTGATTAATGTTTTTGTAATCCATGAGAGCATTATACTCTGC
TCTCAAGAGTTTGTACAATTTTATTCGGTCATATCAGAACGTTCAAAAAGCTCTTCAACAGTTAAACATT
CGGTGTCATCCTCAATAAAGAATCGGGTCCAACACGTTGGGGCGATGCAGTTATGAGTTTGTCCGGCTAT
AGAAGCTACACGAATAAGCTGCTGACCATTATTCAGGACAAATTTTTCACCAATTTCAACATCTTTAAAA
AGCTTAGCCATTTTGTATTCCTCGTGTTTTGATAGGACTACTATACCATAATCCTACCTTGATGTAAACA
ATTAAATTACATTAAAATATGTTAAAGCCAACCATGTTCCGGTGAACGAACATGCGATGTAAAAACCTGT
ATCGCAAATTGTTCTTAGTACAATAAGAAGTGCATTTTTCATAAGTACCTCAATACAATTGAAAATATTA
TACAAATCATAGGGATACTAAGCAACCATATTCCGATCCACATTTTAGAGCTCATATCTCAACTTCCGCG
AGTTCCATATATCCTTTGTTGATAACCTCTTTAAGGTCTTTAGAAAGAATAACACTAAAATGGTCGTTCA
TATGTCCATTAACTAAACGAGTTATTTCGGCTTCAGATGACATATAAGGCACGTCTGAGTCCCAATGAGA
TAGACCTAAATGGGTATAAATCATCCCTTCGTCGTCTGCAGTGTTGTACATTGAAACAAATTCGCCTGTT
ACTTTATGTTTTGCGTAGAAAAATTTAAATTTCATGATGTTATCCTCTTTTAGATAGGACTACTATATCA
TAGTCCTACTTAGTTGTAAACACTTTTATGAAATTAAAGATGAAGATACCACCCGTTGTAGTTGCTCTTG
CGGACGATTTTCTTTAGGTCTTCATGGTCTCCCACGAACTTTATAGAATAAGAGAAGACGTCAGGTGAGG
TTTCTTTGACATCTAATATAATAATGCAATCTGCGACTCGATGGTTCAAGAAGGGCCCTATATTGATTCC
ATTACCACCAGGATAATCACCCCTGTATCCTGTGGTAACCGAAATCCATTTACAATCTATCTGGTGCGTT
TTGACTTCGACACGCAACCCGCAGTATTTAGGATGAGCTAACACGTCCCATGCATACGTGTACGGGTCTT
CTACGTCTTCATTACCTTTATTAACATATCCGTCTAGCCAATTTGCTACTACATACTCTGCAAAGACTGC
AACCTTACAACGTCGAACAACGTCTTCTTTTATTTGACCTGGATCTTGTTTCAACGAGTACCGAGCAGTA
TCAGCGATCTTAACCTTCATTTCGCTAGTCAAAAACTCATTCGACAGGATAAATGTCGGAAGACTCTTCA
GCCTCAACAGTCCCAAGTTCGTCTTTACCATAGATACCTCTGATGTGTAGTTCACCAAAATAAATGCGGT
CACCTTCTTCAAGATATTCTGCATCTACAGGCGGAATTTCAATAACAACTTCGTCGCATTCTAACCAGCC
GTAGTGTGCATCGCCAGGATGTTTTACCAATTTAGCACAATAAACTGATTTACACTTATGATGACCACGA
AGATTAGCAATTTTGGATTCTGATACGTCAAATGGATGAATCATAATTACCCCTAAAGAAAAAAGGGGAC
CGAAGTCCCCAATTATTATTCTGCAAATAATTCAGTAACGTCTGAAATTACTTCATAACGACAAGTACGG
GTCGTAGCATCGCCATAGTCAACTGGGATGGATACAACATCACGTGGATGAACTTTAACCTTAACAACTT
TATCTGAGCTAGAACCAAAGTAACGAATATATGATTTAGCGCAAACATGTAGACCACGGGAGCAGGTTTG
TGTATCATCATCGTTAACACGAGTACGAGGCATTGAAACAACTTTACCAGGCGAGTTATCAAATGTGCCA
CTATAGCAATCTAAATAGTCTTTACGAACTACTTTCCACGCATAGAAATGTCCGTCTTCAGTGATTTCAA
TATCGTTTGCAACCAAGAAGTCGAATAAACGTTGTACAGCTTTTTCGCTTGGGTTTTCTAACAGGTTTTC
AAGGAACGGCAGATAGAATTTAAAGTCTTCGCCTTTTTCCATAGAATCAATAATACGATTAACCAGTCCT
GAACGAATTTCTATACCTTGATAAAATAAACTACCACCTTCAATAGTAACATTACCGTCAACAAACTTAG
TAATAGCTTTCTTGATGTTAATAAGTTCGACAGCTTTACTAAGGTTTCCTGCGACCAAATTAGCTTTAAT
TTCTTCAAAATTTGCATGACTTGATGGGGTCGCATTGTATGCTACGCGTCCTTCAATGATTGAAATAAAT
TTAGATGATGCGTTCCATACGATTTCTGGACGATGACCTGTAATAATAGGTTCATTATTTTCTGGGACCT
 
GTTCTTCGTCTTCAAATTCAGCTGCTTTCTCTTCAGCTTTAAAATTCTTAACAATCTTACGAAGAGTATC
TGTTGAAATGTCGAAGTAATCGGCAATTTCTTTACGAGTCCATTTGCCTGATTCAACCAGGCCGTAAGCT
TCTAACTGTTCTTCTTGGGACAAGCATTTAATATTGTACATAATGTTTCC
**bolA promoter sequence (SEQ ID NO: 13)** 
AACCTAAATATTTGTTGTTAAGCTGCAATGGAAACGGTAAAAGCGGCTAGTATTTAAAG
GGATGGATGACATCTCAGCGTTGTCG
**P_{J23100} promoter sequence (SEQ ID NO: 14)**
   TTGACGGCTAGCTCAGTCCTAGGTACAGTGCTAGC
**Repeat Sequence (SEQ ID NO: 15)**
   CGGTTTATCCCCGCTGGCGCGGGGAACTC
**Promoter sequence (SEQ ID NO: 16)**
   TTGACGCGTAGCTCAGAGGTAGGTATAATGCTAGAAC

## Claims

1. A composition comprising at least 4 different types of transduction particles, wherein each of said particles comprises a nucleic acid and said particles are capable of contacting *E coli* cells and introducing therein the nucleic acid, wherein
(a) said nucleic acid of each particle comprises a nucleotide sequence encoding a product of interest (POI), wherein the nucleic acid is capable of expressing the POI in an *E coli* cell; and wherein the POI comprises:
(i) a Cas nuclease for targeting and cutting the DNA of the *E coli* cell, thereby modifying or killing the cell; and
(ii) at least one crRNA or guide RNA each comprising a spacer sequence that is complementary to a protospacer sequence comprised by a gene selected from B2 phylogroup *E coli* genes *fimH, bolA, rpoH, lptA* and *murA* and each of which is operable with the Cas nuclease for DNA targeting in the *E coli* cells;
(b) each particle comprises:
(iii) an adhesion moiety for recognising and binding to a cognate moiety selected from a LPS and Tsx displayed on the surface of *E coli* cells; and
(iv) a phage capsid containing the nucleic acid; and
(c) said at least 4 different types of transduction particles comprises
A: particles which comprise a LPS adhesion moiety and a LamB adhesion moiety;
B: particles which comprise a LPS adhesion moiety and a Tsx adhesion moiety; and
C: particles which comprise a Tsx adhesion moiety but are devoid of LamB and LPS adhesion moieties;
and wherein the particles of the composition target all of *E coli* genes *fimH, bolA, rpoH, lptA* and *murA.*

2. The composition of claim 1, wherein the capsid of each particle comprised by the composition is a T-even phage capsid; optionally a capsid of a T2 phage, or a RB69 phage.

3. The composition of any preceding claim, wherein each particle is a phage (optionally a lytic phage) or packaged phagemid.

4. The composition of any preceding claim, wherein the nucleic acid of each particle comprises at least one nucleotide sequence (N1) encoding said POI, wherein each particle is a synthetic T-even phage comprising an insertion of N1 into a Modification Permissive Region (MPR) of the genome of the phage, wherein the MPR is immediately after *gene* 49 and to *gene E* when compared to a reference wild-type T2 phage;
optionally wherein
(a) the nucleic acid comprises a DNA deletion of phage DNA in the MPR;
(b) the insertion comprises up to 8000bp of DNA and/or the deletion comprises up to 8000bp of DNA;
(c) the MPR comprises contiguous DNA between said *gene* 49 and *gene E,* wherein the contiguous DNA is at least 1000bp in length; or wherein the MPR comprises at least 100bp of DNA between said *gene* 49 and *gene E;* and/or
(d) the synthetic phage genome comprises said insertion between coordinates 9000 and 21000, wherein the coordinates are the nucleotide positions counted from the nucleotide (coordinate number 1) immediately after *gene* 49 towards *gene E* when compared to a reference wild-type T2 phage.

5. The composition of any preceding claim, wherein the nuclease is a Type I, II, III, IV, V or VI Cas nuclease, optionally a Cas9 or a Cas3.

6. The composition of any preceding claim, wherein each protospacer sequence is of strain ST131.

7. The composition of any preceding claim, wherein the nucleotide sequence encoding the POI comprises an *E coli bolA* promoter or comprises SEQ ID NO: 13.

8. The composition of any preceding claim, wherein the *E coli* cells comprise a strain of *E coli* that causes sepsis, septicaemia or diarrhoea in humans or animals.

9. The composition of any preceding claim for use in treating or preventing infection by *E coli* cells in a human or animal subject, wherein the use comprises administering the composition to the subject.

10. The composition of any preceding claim for use in treating or preventing sepsis, septicaemia or diarrhoea in a human or animal subject, the use comprising administering to the subject the composition, wherein the *E coli* cells comprise a strain of *E coli* that causes sepsis, septicaemia or diarrhoea in humans or animals.

11. The composition for use of claims 9 or 10, wherein the subject is a transplant or cancer patient (optionally a haematological cancer patient), or wherein the patient is suffering from or at risk of a urinary tract infection (UTI); and optionally wherein the transplant is a solid organ or stem cell transplant (optionally a haematopoietic cell transplant) or wherein the transplant is a transplant of a medical device; optionally wherein the administration is carried out prior to the subject receiving a transplant.

12. The composition for use of any one of claims 9-11 for preventing haemolytic uremic syndrome (HUS), a UTI infection, sepsis, septicaemia or diarrhoea in a human subject.

13. The composition for use of any one of claims 9-12, wherein at least 1 x 10⁷ PFU of particles are administered to the subject; or a composition according to any one of claims 1-8 wherein the composition comprises a dose of at least 1 x 10⁷ PFU of said particles.

14. The composition for use of any one of claims 9-13, wherein the particles are administered to the subject at an MOI (multiplicity of infection) of at least 0.01.

15. The composition for use of any one of claims 9-14, wherein the *E coli* cells comprise at least one strain that is an antibiotic-resistant or MDR strain; and/or at least one B2-I strain; optionally wherein the antibiotic is fluoroquinolone (optionally levofloxacin), carbapenem or vancomycin; and/or wherein the *E coli* cells comprise beta-lactamase (ESBL)-producing *E coli.*

## Patentansprüche

1. Zusammensetzung, die mindestens 4 verschiedene Arten von Transduktionspartikeln umfasst, wobei jedes der Partikel eine Nukleinsäure umfasst und die Partikel in der Lage sind, *E.-coli-*Zellen zu kontaktieren und darin die Nukleinsäure einzuführen, wobei
(a) die Nukleinsäure jedes Partikels eine Nukleotidsequenz umfasst, die für ein Produkt von Interesse (POI) kodiert, wobei die Nukleinsäure in der Lage ist, das POI in einer *E.-coli-Zelle* zu exprimieren; und wobei das POI umfasst:
(i) eine Cas-Nuklease zum Abzielen und Schneiden der DNA der *E.-coli-*Zelle, wodurch die Zelle modifiziert oder abgetötet wird; und
(ii) zumindest eine crRNA oder Guide-RNA, die jeweils eine Spacer-Sequenz umfasst, die zu einer Protospacer-Sequenz komplementär ist, die durch ein Gen besteht, das aus den *E.-coli-*Genen der B2-Phylogruppe *fimH, Bola, rpoH, lptA* und *murA* ausgewählt ist, und von dem jedes mit der Cas-Nuklease für DNA-Targeting in den E.-coli-Zellen betriebsfähig ist;
(b) jedes Partikel umfasst:
(iii) eine Adhäsionseinheit zur Erkennung und Bindung an eine verwandte Einheit, ausgewählt aus einem LPS und TSX, die auf der Oberfläche von *E*.-*coli*-Zellen angezeigt werden; und
(iv) ein Phagenkapsid, das die Nukleinsäure enthält; und
(c) die mindestens 4 verschiedenen Arten von Transduktionspartikeln umfassen
A: Partikel, die eine LPS-Adhäsionseinheit und eine LamB-Adhäsionskomponente umfassen;
B: Partikel, die eine LPS-Adhäsionseinheit und eine Tsx-Adhäsionseinheit umfassen; und
C: Partikel, die eine Tsx-Adhäsionseinheit umfassen, jedoch keine LamB- und LPS-Adhäsionseinheiten umfassen;
und wobei die Partikel der Zusammensetzung auf alles der *E.-coli-*Gene *fimH, Bola, rpoH, lptA* und *murA* abzielen.

2. Zusammensetzung nach Anspruch 1, wobei das Kapsid jedes durch die Zusammensetzung umfassten Partikels ein T-even-Phagenkapsid ist; optional ein Kapsid eines T2-Phagen oder eines RB69-Phagen.

3. Zusammensetzung nach einem vorstehenden Anspruch, wobei jedes Partikel ein Phage (optional ein lytischer Phage) oder bepacktes Phagemid ist.

4. Zusammensetzung nach einem vorstehenden Anspruch, wobei die Nukleinsäure jedes Partikels mindestens eine Nukleotidsequenz (N1) umfasst, die für das POI kodiert, wobei jedes Partikel ein synthetischer T-even-Phage ist, der eine Insertion von N1 in eine Modification Permissive Region (MPR) des Genoms des Phagen umfasst, wobei die MPR unmittelbar nach dem *Gen* 49 und dem *Gen E* folgt, im Vergleich zu einem Referenz-Wildtyp-T2-Phagen;
optional, wobei
(a) die Nukleinsäure eine DNA-Deletion von Phagen-DNA in der MPR umfasst;
(b) die Insertion bis zu 8000 bp DNA umfasst und/oder die Deletion bis zu 8000 bp DNA umfasst;
(c) die MPR zusammenhängende DNA zwischen dem *Gen* 49 und *dem Gen E* umfasst, wobei die zusammenhängende DNA mindestens 1000 bp lang ist; oder wobei die MPR mindestens 100 bp DNA zwischen dem *Gen 49* und *dem Gen E* umfasst; und/oder
(d) das synthetische Phagengenom die Insertion zwischen den Koordinaten 9000 und 21000 umfasst, wobei die Koordinaten die Nukleotidpositionen sind, die von dem Nukleotid (Koordinate 1) unmittelbar nach *dem Gen 49* in Richtung *Gen E* gezählt werden, im Vergleich zu einem Referenz-Wildtyp-T2-Phagen.

5. Zusammensetzung nach einem vorstehenden Anspruch, wobei die Nuklease eine Typ-I-, -II-, -III-, -IV-, -V- oder -VI-Cas-Nuklease ist, optional eine Cas9 oder eine Cas3.

6. Zusammensetzung nach einem vorstehenden Anspruch, wobei jede Protospacer-Sequenz vom Stamm ST131 ist.

7. Zusammensetzung nach einem vorstehenden Anspruch, wobei die Nukleotidsequenz, die für das POI kodiert, einen *E.-coli-bolA-*Promotor umfasst oder SEQ ID NO: 13 umfasst.

8. Zusammensetzung nach einem vorstehenden Anspruch, wobei die *E.-coli-*Zellen einen Stamm von *E*. *coli* umfassen, der bei Menschen oder Tieren Sepsis, Septikämie oder Durchfall verursacht.

9. Zusammensetzung nach einem vorstehenden Anspruch zur Verwendung bei der Behandlung oder Prävention von Infektionen durch *E.-coli-*Zellen in einem menschlichen oder tierischen Individuum, wobei die Verwendung das Verabreichen der Zusammensetzung an das Individuum umfasst.

10. Zusammensetzung nach einem vorstehenden Anspruch zur Verwendung bei der Behandlung oder Prävention von Sepsis, Septikämie oder Durchfall bei einem menschlichen oder tierischen Individuum, wobei die Verwendung das Verabreichen der Zusammensetzung an das Individuum umfasst, wobei die *E.-coli-*Zellen einen Stamm von *E. coli* umfassen, der bei Menschen oder Tieren Sepsis, Septikämie oder Durchfall verursacht.

11. Zusammensetzung zur Verwendung nach den Ansprüchen 9 oder 10, wobei das Individuum ein Transplantations- oder Krebspatient ist (optional ein Patient mit hämatologischem Krebs), oder wobei der Patient an einer Harnwegsinfektion (UTI) leidet oder ein Risiko einer solchen besteht; und optional wobei die Transplantation eine Transplantation eines soliden Organs oder von Stammzellen (optional eine Transplantation von hämatopoetische Zellen) ist oder wobei die Transplantation eine Transplantation eines medizinischen Geräts ist; optional wobei die Verabreichung durchgeführt wird, bevor das Individuum ein Transplantat erhält.

12. Zusammensetzung zur Verwendung nach einem der Ansprüche 9 bis 11 zur Prävention des hämolytisch-urämischen Syndroms (HUS), einer UTI-Infektion, von Sepsis, Septikämie oder Durchfall bei einem menschlichen Individuum.

13. Zusammensetzung zur Verwendung nach einem der Ansprüche 9 bis 12, wobei dem Individuum mindestens 1 x 10⁷ PFU von Partikeln verabreicht werden; oder Zusammensetzung nach einem der Ansprüche 1 bis 8, wobei die Zusammensetzung eine Dosis von mindestens 1 x 10⁷ PFU der Partikel umfasst.

14. Zusammensetzung zur Verwendung nach einem der Ansprüche 9 bis 13, wobei die Partikel dem Individuum bei einer MOI (Multiplizität der Infektion) von mindestens 0,01 verabreicht werden.

15. Zusammensetzung zur Verwendung nach einem der Ansprüche 9 bis 14, wobei die E.-coli-Zellen mindestens einen Stamm umfassen, der ein antibiotikaresistenter oder MDR-Stamm ist; und/oder mindestens einen B2-I-Stamm; optional wobei das Antibiotikum Fluorchinolon (optional Levofloxacin), Carbapenem oder Vancomycin ist;
und/oder wobei die *E.-coli-*Zellen Beta-Laktamase (ESBL) produzierende *E coli* umfassen.

## Revendications

1. Composition comprenant au moins 4 types différents de particules de transduction, dans laquelle chacune desdites particules comprend un acide nucléique et lesdites particules sont capables d'entrer en contact avec des cellules d'E. *coli* et d'y introduire l'acide nucléique, dans laquelle
(a) ledit acide nucléique de chaque particule comprend une séquence nucléotidique codant pour un produit d'intérêt (POI), dans laquelle l'acide nucléique est capable d'exprimer le POI dans une cellule d'*E*. *coli ;* et dans laquelle le POI comprend :
(i) une nucléase Cas pour cibler et couper l'ADN de la cellule d'E. coli, modifiant ou tuant ainsi la cellule ; et
(ii) au moins un ARN cr ou ARN guide comprenant chacun une séquence d'espacement qui est complémentaire d'une séquence protoespaceur constitué d'un gène choisi parmi les gènes *fimH, bolA, rpoH,* lptA et *murA* du phylogroupe B2 d'E. *coli,* et chacun d'entre eux est fonctionnel avec la nucléase Cas pour cibler l'ADN dans les cellules d'*E. coli* ;
(b) chaque particule comprend :
(iii) un fragment d'adhérence pour reconnaître et se lier à un fragment cognitif choisi parmi un LPS et un Tsx présentés à la surface des cellules d'*E. coli ;* et
(iv) une capside de phage contenant l'acide nucléique ; et
(c) lesdits au moins 4 types différents de particules de transduction comprennent
A : des particules qui comprennent un fragment d'adhérence de LPS et un fragment d'adhérence de LamB ;
B : des particules qui comprennent un fragment d'adhérence de LPS et un fragment d'adhérence de Tsx ; et
C : des particules qui comprennent un fragment d'adhérence de Tsx mais qui sont dépourvues de fragments d'adhésion de LamB et de LPS ;
et dans laquelle les particules de la composition ciblent tous les gènes d'E. *coli fimH, bolA, rpoH, lptA* et *murA.*

2. Composition selon la revendication 1, dans laquelle la capside de chaque particule constitué par la composition est une capside de phage T-pair ; éventuellement une capside d'un phage T2 ou d'un phage RB69.

3. Composition selon l'une quelconque des revendications précédentes, dans laquelle chaque particule est un phage (éventuellement un phage lytique) ou un phagemide encapsulé.

4. Composition selon l'une quelconque des revendications précédentes, dans laquelle l'acide nucléique de chaque particule comprend au moins une séquence nucléotidique (N1) codant pour ledit POI, dans laquelle chaque particule est un phage T-pair synthétique comprenant une insertion de N1 dans une région permissive de modification (MPR) du génome du phage, dans laquelle la MPR se trouve immédiatement après le *gène 49* et jusqu'au *gène E* par rapport à un phage T2 de type sauvage de référence ;
éventuellement dans laquelle
(a) l'acide nucléique comprend une délétion d'ADN de l'ADN de phage dans la MPR ;
(b) l'insertion comprend jusqu'à 8000 pb d'ADN et/ou la délétion comprend jusqu'à 8000 pb d'ADN ;
(c) la MPR comprend de l'ADN contigu entre ledit *gène* 49 et le *gène E,* dans lequel l'ADN contigu a une longueur d'au moins 1000 pb ; ou dans laquelle la MPR comprend au moins 100 pb d'ADN entre ledit *gène 49* et le *gène E* ; et/ou
(d) le génome phage synthétique comprend ladite insertion entre les coordonnées 9000 et 21000, dans laquelle lesdites coordonnées sont les positions nucléotidiques comptées à partir du nucléotide (coordonnée numéro 1) immédiatement après le *gène* 49 vers le *gène* E par rapport à un phage T2 de type sauvage de référence.

5. Composition selon l'une quelconque des revendications précédentes, dans laquelle la nucléase est une nucléase Cas de type I, II, III, IV, V ou VI, éventuellement une Cas9 ou une Cas3.

6. Composition selon l'une quelconque des revendications précédentes, dans laquelle chaque séquence protoespaceur est issue de la souche ST131.

7. Composition selon l'une quelconque des revendications précédentes, dans laquelle la séquence nucléotidique codant pour le POI comprend un promoteur bolA *d'E. coli* ou comprend la séquence SEQ ID NO: 13.

8. Composition selon l'une quelconque des revendications précédentes, dans laquelle les cellules *d'E. coli* comprennent une souche *d'E. coli* qui provoque une sepsie, une septicémie ou une diarrhée chez l'homme ou l'animal.

9. Composition selon l'une quelconque des revendications précédentes destinée à être utilisée dans le traitement ou la prévention d'une infection par des cellules *d'E. coli* chez un sujet humain ou animal, dans laquelle l'utilisation comprend l'administration de la composition au sujet.

10. Composition selon l'une quelconque des revendications précédentes, destinée à être utilisée dans le traitement ou la prévention de la sepsie, de la septicémie ou de la diarrhée chez un sujet humain ou animal, l'utilisation comprenant l'administration de la composition au sujet, dans laquelle les cellules coli comprennent une souche de *E. coli* qui provoque une une sepsie, une septicémie ou une diarrhée chez l'homme ou l'animal.

11. Composition destinée à être utilisée selon les revendications 9 ou 10, dans laquelle le sujet est un patient transplanté ou atteint d'un cancer (éventuellement un patient atteint d'un cancer hématologique), ou dans laquelle le patient souffre d'une infection des voies urinaires (IVU) ou présente un risque de celle-ci ; et éventuellement dans laquelle la transplantation est une transplantation d'organe solide ou de cellule souche (éventuellement une transplantation de cellule hématopoïétique) ou dans laquelle la transplantation est une transplantation d'un dispositif médical ; éventuellement dans laquelle l'administration est effectuée avant que le sujet ne reçoive une transplantation.

12. Composition destinée à être utilisée selon l'une quelconque des revendications 9 à 11 pour prévenir le syndrome hémolytique et urémique (SHU), une infection des voies urinaires, une sepsie, une septicémie ou une diarrhée chez un sujet humain.

13. Composition selon l'une quelconque des revendications 9 à 12, dans laquelle au moins 1x107 PFU de particules sont administrées au sujet ; ou composition selon l'une quelconque des revendications 1 à 8, dans laquelle la composition comprend une dose d'au moins 1 x 107 PFU desdites particules.

14. Composition destinée à être utilisée selon l'une quelconque des revendications 9 à 13, dans laquelle les particules sont administrées au sujet à une MOI (multiplicité d'infection) d'au moins 0,01.

15. Composition destinée à être utilisée selon l'une quelconque des revendications 9 à 14, dans laquelle les cellules *E. coli* comprennent au moins une souche qui est une souche résistante aux antibiotiques ou une souche MDR ; et/ou au moins une souche B2-I ; éventuellement dans laquelle l'antibiotique est une fluoroquinolone (éventuellement la lévofloxacine), un carbapénème ou la vancomycine ;
et/ou dans laquelle les cellules *E. coli* comprennent des *E. coli* productrices de bêta-lactamase (ESBL).
